(19) Europäisches Patentamt
European Patent Office
Office européen des brevets

(11) EP 2 222 709 B1

(12) EUROPEAN PATENT SPECIFICATION

(45) Date of publication and mention of the grant of the patent:
**23.11.2016 Bulletin 2016/47**

(21) Application number: **08854122.2**

(22) Date of filing: **28.11.2008**

(51) Int Cl.:
*C07K 16/46* (2006.01) *A61K 39/395* (2006.01)
*A61P 35/00* (2006.01) *A61P 29/00* (2006.01)
*C12N 15/13* (2006.01) *C12N 5/10* (2006.01)
*C07K 16/22* (2006.01) *C07K 16/24* (2006.01)
*C07K 16/28* (2006.01) *C07K 16/32* (2006.01)

(86) International application number:
**PCT/EP2008/066438**

(87) International publication number:
**WO 2009/068649 (04.06.2009 Gazette 2009/23)**

(54) **ANTIGEN-BINDING CONSTRUCTS**

ANTIGENBINDENDE KONSTRUKTE

PRODUITS DE CONSTRUCTION DE LIAISON À UN ANTIGÈNE

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.11.2007 US 991449 P**
**12.02.2008 US 27858 P**
**21.04.2008 US 46572 P**
**16.07.2008 US 81191 P**
**29.07.2008 US 84431 P**

(43) Date of publication of application:
**01.09.2010 Bulletin 2010/35**

(60) Divisional application:
**13154223.5 / 2 641 919**
**13154451.2 / 2 615 115**

(73) Proprietor: **Glaxo Group Limited**
**Brentford, Middlesex TW8 9GS (GB)**

(72) Inventors:
- **ASHMAN, Claire**
  **Stevenage Hertfordshire SG1 2NY (GB)**
- **BATUWANGALA, Thil**
  **Cambridgeshire CB4 0WG (GB)**
- **BURDEN, Michael, Neil**
  **Stevenage Hertfordshire SG1 2NY (GB)**
- **CLEGG, Stephanie, Jane**
  **Stevenage Hertfordshire SG1 2NY (GB)**
- **DE WILDT, Rudolf, Maria**
  **Cambridgeshire CB4 0WG (GB)**
- **ELLIS, Jonathan, Henry**
  **Stevenage Hertfordshire SG1 2NY (GB)**
- **HAMBLIN, Paul, Andrew**
  **Stevenage Hertfordshire SG1 2NY (GB)**
- **HUSSAIN, Farhana**
  **Stevenage Hertfordshire SG1 2NY (GB)**
- **JESPERS, Laurent**
  **Cambridgeshire CB4 0WG (GB)**
- **LEWIS, Alan**
  **Stevenage Hertfordshire SG1 2NY (GB)**
- **ORECCHIA, Martin, Anibal**
  **Stevenage Hertfordshire SG1 2NY (GB)**
- **SHAH, Radha**
  **Stevenage Hertfordshire SG1 2NY (GB)**
- **STEWARD, Michael**
  **Cambridgeshire CB4 0WG (GB)**

(74) Representative: **Hitchcock, Lucy Rose et al**
**GlaxoSmithKline**
**Global Patents (CN925.1)**
**980 Great West Road**
**Brentford, Middlesex TW8 9GS (GB)**

(56) References cited:
**EP-A1- 2 050 764**
**WO-A1-2007/066106**
**WO-A1-2007/085814**
**US-B1- 6 492 123**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 222 709 B1

- SHEN JUQUN ET AL: "Single variable domain antibody as a versatile building block for the construction of IgG-like bispecific antibodies" JOURNAL OF IMMUNOLOGICAL METHODS, ELSEVIER SCIENCE PUBLISHERS B.V.,AMSTERDAM, NL, vol. 318, no. 1-2, 1 January 2007 (2007-01-01), pages 65-74, XP002500843 ISSN: 0022-1759 [retrieved on 2006-10-26]
- SCALLON BERNARD ET AL: "Addition of an extra immunoglobulin domain to two anti-rodent TNF monoclonal antibodies substantially increased their potency" MOLECULAR IMMUNOLOGY, PERGAMON, GB, vol. 41, no. 1, 1 May 2004 (2004-05-01), pages 73-80, XP002424159 ISSN: 0161-5890

SEQUENCE LISTING

<110> GLAXO GROUP LIMITED
ASHMAN Claire
BURDEN Neil
ELLIS Jonathan Henry
CLEGG Stephanie Jane
HAMBLIN Paul Andrew
DE WILDT Rudolf Maria
BATUWANGALA Thil
HUSSAIN Farhana
JESPERS Laurent
LEWIS Alan
STEWARD Michael
ORECCHIA Martin A
SHAH Radha

<120> Antigen binding constructs

<130> PB62748

<150> US60/991449
<151> 2007-11-30

<150> US61/027858
<151> 2008-02-12

<150> US61/046572
<151> 2008-04-21

<150> US61/081,191
<151> 2008-07-16

<150> US61/084,431
<151> 2008-07-29

<160> 270

<170> FastSEQ for Windows Version 4.0

<210> 1
<211> 108
<212> PRT
<213> Homo Sapiens

<400> 1

```
Asp Ile Gln Met Thr Gln Ser Pro Ser Ser Leu Ser Ala Ser Val Gly
 1               5                  10                  15
Asp Arg Val Thr Ile Thr Cys Arg Ala Ser Arg Pro Ile Ser Asp Trp
            20                  25                  30
Leu His Trp Tyr Gln Gln Lys Pro Gly Lys Ala Pro Lys Leu Leu Ile
        35                  40                  45
Ala Trp Ala Ser Thr Leu Asp Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Glu Gly Trp Gly Pro Pro
                85                  90                  95
Thr Phe Gly Gln Gly Thr Lys Val Glu Ile Lys Arg
            100                 105
```

<210> 2
<211> 108
<212> PRT
<213> Homo Sapiens

<400> 2

```
Asp Ile Gln Met Thr Gln Ser Pro Ser Ser Leu Ser Ala Ser Val Gly
 1               5                  10                  15
Asp Arg Val Thr Ile Thr Cys Arg Ala Ser Arg Pro Ile Ser Asp Trp
            20                  25                  30
Leu His Trp Tyr Gln Gln Lys Pro Gly Lys Ala Pro Lys Leu Leu Ile

        35                  40                  45
Ala Trp Ala Ser Ser Leu Tyr Glu Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Glu Gly Trp Gly Pro Pro
                85                  90                  95
Thr Phe Gly Gln Gly Thr Lys Val Glu Ile Lys Arg
            100                 105
```

<210> 3

<211> 108
<212> PRT
<213> Homo Sapiens

<400> 3

```
Asp Ile Gln Met Thr Gln Ser Pro Ser Ser Leu Ser Ala Ser Val Gly
1               5                   10                  15
Asp Arg Val Thr Ile Thr Cys Arg Ala Ser Arg Pro Ile Ser Asp Trp
            20                  25                  30
Leu His Trp Tyr Gln Gln Lys Pro Gly Lys Ala Pro Lys Leu Leu Ile
        35                  40                  45
Ala Trp Ala Ser Ser Leu Gln Gly Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Glu Gly Trp Gly Pro Pro
                85                  90                  95
Thr Phe Gly Gln Gly Thr Lys Val Glu Ile Lys Arg
            100                 105
```

<210> 4
<211> 116
<212> PRT
<213> Homo Sapiens

<400> 4

```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Arg Asn Phe
            20                  25                  30
Gly Met Gly Trp Val Arg Gln Ala Pro Gly Lys Gly Leu Glu Trp Val
        35                  40                  45
Ser Trp Ile Ile Ser Ser Gly Thr Glu Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ser Leu Gly Arg Phe Asp Tyr Trp Gly Gln Gly Thr Leu Val
            100                 105                 110
Thr Val Ser Ser
        115
```

<210> 5
<211> 118
<212> PRT
<213> Homo Sapiens

<400> 5

Gly Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ala Trp Tyr
20 25 30
Asp Met Gly Trp Val Arg Gln Ala Pro Gly Lys Gly Leu Glu Trp Val
35 40 45
Ser Ser Ile Asp Trp His Gly Glu Val Thr Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Thr Ala Glu Asp Glu Pro Gly Tyr Asp Tyr Trp Gly Gln Gly Thr
100 105 110
Leu Val Thr Val Ser Ser
115

<210> 6
<211> 5
<212> PRT
<213> Artificial Sequence

<220>
<223> Linker

<400> 6

Gly Gly Gly Gly Ser
1 5

<210> 7
<211> 6
<212> PRT
<213> Artificial Sequence

<220>
<223> Linker

<400> 7

Thr Val Ala Ala Pro Ser
1 5

<210> 8
<211> 7
<212> PRT
<213> Artificial Sequence

<220>
<223> Linker

<400> 8

Ala Ser Thr Lys Gly Pro Thr
1 5

<210> 9
<211> 7
<212> PRT
<213> Artificial Sequence

<220>
<223> Linker

<400> 9

Ala Ser Thr Lys Gly Pro Ser
1 5

<210> 10
<211> 15
<212> PRT
<213> Artificial Sequence

<220>
<223> Linker

<400> 10

Glu Pro Lys Ser Cys Asp Lys Thr His Thr Cys Pro Pro Cys Pro
1 5 10 15

<210> 11
<211> 18
<212> PRT
<213> Artificial Sequence

<220>
<223> Linker

<400> 11

Glu Leu Gln Leu Glu Glu Ser Cys Ala Glu Ala Gln Asp Gly Glu Leu
1 5 10 15
Asp Gly

<210> 12
<211> 455
<212> PRT
<213> Artificial Sequence

<220>
<223> Humanised

<400> 12

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys
450 455

<210> 13
<211> 219
<212> PRT
<213> Artificial Sequence

<220>
<223> Humanised

<400> 13

Asp Ile Val Met Thr Gln Ser Pro Leu Ser Leu Pro Val Thr Pro Gly
1 5 10 15
Glu Pro Ala Ser Ile Ser Cys Arg Ser Ser Gln Asn Ile Val His Ile
20 25 30
Asn Gly Asn Thr Tyr Leu Glu Trp Tyr Leu Gln Lys Pro Gly Gln Ser
35 40 45
Pro Arg Leu Leu Ile Tyr Lys Ile Ser Asp Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Asp Asp Val Gly Ile Tyr Tyr Cys Phe Gln Gly
85 90 95
Ser His Val Pro Trp Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190
Lys His Lys Val Tyr Ala Cys Glu Val Thr His Gln Gly Leu Ser Ser
195 200 205
Pro Val Thr Lys Ser Phe Asn Arg Gly Glu Cys
210 215

<210> 14
<211> 451
<212> PRT
<213> Artificial Sequence

<220>
<223> Humanised

<400> 14

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450
```

<210> 15
<211> 218
<212> PRT
<213> Artificial Sequence

<220>
<223> Humanised

<400> 15

```
Asp Ile Val Leu Thr Gln Ser Pro Ser Ser Leu Ser Ala Ser Val Gly
1               5                   10                  15
Asp Arg Val Thr Ile Thr Cys Lys Ala Ser Gln Ser Val Asp Tyr Asp
            20                  25                  30
Gly Asp Ser Tyr Met Asn Trp Tyr Gln Gln Lys Pro Gly Lys Ala Pro
        35                  40                  45
Lys Leu Leu Ile Tyr Ala Ala Ser Asn Leu Glu Ser Gly Ile Pro Ser
    50                  55                  60
Arg Phe Ser Gly Ser Gly Ser Gly Thr Asp Phe Thr Phe Thr Ile Ser
65                  70                  75                  80
Ser Leu Gln Pro Glu Asp Ile Ala Thr Tyr Tyr Cys Gln Gln Ser Asn
                85                  90                  95
Glu Asp Pro Pro Thr Phe Gly Gln Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215
```

<210> 16
<211> 565
<212> PRT
<213> Artificial Sequence

<220>
<223> Humanised

<400> 16

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Asp Ile Gln Met Thr Gln Ser
    450                 455                 460
Pro Ser Ser Leu Ser Ala Ser Val Gly Asp Arg Val Thr Ile Thr Cys
465                 470                 475                 480
Arg Ala Ser Arg Pro Ile Ser Asp Trp Leu His Trp Tyr Gln Gln Lys
                485                 490                 495
Pro Gly Lys Ala Pro Lys Leu Leu Ile Ala Trp Ala Ser Thr Leu Asp
            500                 505                 510
Ser Gly Val Pro Ser Arg Phe Ser Gly Ser Gly Ser Gly Thr Asp Phe
        515                 520                 525
Thr Leu Thr Ile Ser Ser Leu Gln Pro Glu Asp Phe Ala Thr Tyr Tyr
    530                 535                 540
Cys Leu Gln Glu Gly Trp Gly Pro Pro Thr Phe Gly Gln Gly Thr Lys
545                 550                 555                 560
```

Val Glu Ile Lys Arg
565

<210> 17
<211> 565
<212> PRT
<213> Artificial Sequence

<220>
<223> Humanised

<400> 17

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Asp Ile Gln Met Thr Gln Ser
    450                 455                 460
Pro Ser Ser Leu Ser Ala Ser Val Gly Asp Arg Val Thr Ile Thr Cys
465                 470                 475                 480
Arg Ala Ser Arg Pro Ile Ser Asp Trp Leu His Trp Tyr Gln Gln Lys
                485                 490                 495
Pro Gly Lys Ala Pro Lys Leu Leu Ile Ala Trp Ala Ser Ser Leu Tyr
```

```
                    500                     505                     510
Glu Gly Val Pro Ser Arg Phe Ser Gly Ser Gly Ser Gly Thr Asp Phe
        515                     520                     525
Thr Leu Thr Ile Ser Ser Leu Gln Pro Glu Asp Phe Ala Thr Tyr Tyr
    530                     535                     540
Cys Leu Gln Glu Gly Trp Gly Pro Pro Thr Phe Gly Gln Gly Thr Lys
545                 550                     555                     560
Val Glu Ile Lys Arg
                565
```

<210> 18
<211> 565
<212> PRT
<213> Artificial Sequence

<220>
<223> Humanised

<400> 18

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
```

Leu Ser Leu Ser Pro Gly Lys Gly Ser Asp Ile Gln Met Thr Gln Ser
450 455 460
Pro Ser Ser Leu Ser Ala Ser Val Gly Asp Arg Val Thr Ile Thr Cys
465 470 475 480
Arg Ala Ser Arg Pro Ile Ser Asp Trp Leu His Trp Tyr Gln Gln Lys
485 490 495
Pro Gly Lys Ala Pro Lys Leu Leu Ile Ala Trp Ala Ser Ser Leu Gln
500 505 510
Gly Gly Val Pro Ser Arg Phe Ser Gly Ser Gly Ser Gly Thr Asp Phe
515 520 525
Thr Leu Thr Ile Ser Ser Leu Gln Pro Glu Asp Phe Ala Thr Tyr Tyr
530 535 540
Cys Leu Gln Glu Gly Trp Gly Pro Pro Thr Phe Gly Gln Gly Thr Lys
545 550 555 560
Val Glu Ile Lys Arg
565

<210> 19
<211> 573
<212> PRT
<213> Artificial Sequence

<220>
<223> Humanised

<400> 19

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys

```
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Val Gln Leu Leu Glu Ser
    450                 455                 460
Gly Gly Gly Leu Val Gln Pro Gly Gly Ser Leu Arg Leu Ser Cys Ala
465                 470                 475                 480
Ala Ser Gly Phe Thr Phe Arg Asn Phe Gly Met Gly Trp Val Arg Gln
                485                 490                 495
Ala Pro Gly Lys Gly Leu Glu Trp Val Ser Trp Ile Ile Ser Ser Gly
            500                 505                 510
Thr Glu Thr Tyr Tyr Ala Asp Ser Val Lys Gly Arg Phe Thr Ile Ser
        515                 520                 525
Arg Asp Asn Ser Lys Asn Thr Leu Tyr Leu Gln Met Asn Ser Leu Arg
    530                 535                 540
Ala Glu Asp Thr Ala Val Tyr Tyr Cys Ala Lys Ser Leu Gly Arg Phe
545                 550                 555                 560
Asp Tyr Trp Gly Gln Gly Thr Leu Val Thr Val Ser Ser
                565                 570
```

<210> 20
<211> 568
<212> PRT
<213> Artificial Sequence

<220>
<223> Humanised

<400> 20

Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Gly Gly Gly Ser Asp Ile Gln Met
450 455 460
Thr Gln Ser Pro Ser Ser Leu Ser Ala Ser Val Gly Asp Arg Val Thr
465 470 475 480
Ile Thr Cys Arg Ala Ser Arg Pro Ile Ser Asp Trp Leu His Trp Tyr
485 490 495
Gln Gln Lys Pro Gly Lys Ala Pro Lys Leu Leu Ile Ala Trp Ala Ser
500 505 510
Thr Leu Asp Ser Gly Val Pro Ser Arg Phe Ser Gly Ser Gly Ser Gly
515 520 525
Thr Asp Phe Thr Leu Thr Ile Ser Ser Leu Gln Pro Glu Asp Phe Ala
530 535 540
Thr Tyr Tyr Cys Leu Gln Glu Gly Trp Gly Pro Pro Thr Phe Gly Gln
545 550 555 560
Gly Thr Lys Val Glu Ile Lys Arg
565

<210> 21
<211> 568
<212> PRT
<213> Artificial Sequence

<220>
<223> Humanised

<400> 21

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
```

```
            275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Gly Gly Gly Ser Asp Ile Gln Met
    450                 455                 460
Thr Gln Ser Pro Ser Ser Leu Ser Ala Ser Val Gly Asp Arg Val Thr
465                 470                 475                 480
Ile Thr Cys Arg Ala Ser Arg Pro Ile Ser Asp Trp Leu His Trp Tyr
                485                 490                 495
Gln Gln Lys Pro Gly Lys Ala Pro Lys Leu Leu Ile Ala Trp Ala Ser
            500                 505                 510
Ser Leu Tyr Glu Gly Val Pro Ser Arg Phe Ser Gly Ser Gly Ser Gly
        515                 520                 525
Thr Asp Phe Thr Leu Thr Ile Ser Ser Leu Gln Pro Glu Asp Phe Ala
    530                 535                 540
Thr Tyr Tyr Cys Leu Gln Glu Gly Trp Gly Pro Pro Thr Phe Gly Gln
545                 550                 555                 560
Gly Thr Lys Val Glu Ile Lys Arg
                565
```

<210> 22
<211> 568
<212> PRT
<213> Artificial Sequence

<220>
<223> Humanised

<400> 22

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220

Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Gly Gly Gly Ser Asp Ile Gln Met
450 455 460
Thr Gln Ser Pro Ser Ser Leu Ser Ala Ser Val Gly Asp Arg Val Thr
465 470 475 480
Ile Thr Cys Arg Ala Ser Arg Pro Ile Ser Asp Trp Leu His Trp Tyr
485 490 495
Gln Gln Lys Pro Gly Lys Ala Pro Lys Leu Leu Ile Ala Trp Ala Ser
500 505 510
Ser Leu Gln Gly Gly Val Pro Ser Arg Phe Ser Gly Ser Gly Ser Gly
515 520 525
Thr Asp Phe Thr Leu Thr Ile Ser Ser Leu Gln Pro Glu Asp Phe Ala
530 535 540
Thr Tyr Tyr Cys Leu Gln Glu Gly Trp Gly Pro Pro Thr Phe Gly Gln
545 550 555 560
Gly Thr Lys Val Glu Ile Lys Arg
565

<210> 23
<211> 576
<212> PRT
<213> Artificial Sequence

<220>
<223> Humanised

<400> 23

Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His

```
                    165                     170                     175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                     240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                     320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                     400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Gly Gly Gly Ser Glu Val Gln Leu
    450                 455                 460
Leu Glu Ser Gly Gly Gly Leu Val Gln Pro Gly Gly Ser Leu Arg Leu
465                 470                 475                     480
Ser Cys Ala Ala Ser Gly Phe Thr Phe Arg Asn Phe Gly Met Gly Trp
                485                 490                 495
Val Arg Gln Ala Pro Gly Lys Gly Leu Glu Trp Val Ser Trp Ile Ile
            500                 505                 510
Ser Ser Gly Thr Glu Thr Tyr Tyr Ala Asp Ser Val Lys Gly Arg Phe
        515                 520                 525
Thr Ile Ser Arg Asp Asn Ser Lys Asn Thr Leu Tyr Leu Gln Met Asn
    530                 535                 540
Ser Leu Arg Ala Glu Asp Thr Ala Val Tyr Tyr Cys Ala Lys Ser Leu
545                 550                 555                     560
Gly Arg Phe Asp Tyr Trp Gly Gln Gly Thr Leu Val Thr Val Ser Ser
                565                 570                 575
```

<210> 24
<211> 571
<212> PRT
<213> Artificial Sequence

<220>
<223> Humanised

<400> 24

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
```

Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Thr Val Ala Ala Pro Ser Gly Ser Asp
450 455 460
Ile Gln Met Thr Gln Ser Pro Ser Ser Leu Ser Ala Ser Val Gly Asp
465 470 475 480
Arg Val Thr Ile Thr Cys Arg Ala Ser Arg Pro Ile Ser Asp Trp Leu
485 490 495
His Trp Tyr Gln Gln Lys Pro Gly Lys Ala Pro Lys Leu Leu Ile Ala
500 505 510
Trp Ala Ser Thr Leu Asp Ser Gly Val Pro Ser Arg Phe Ser Gly Ser
515 520 525
Gly Ser Gly Thr Asp Phe Thr Leu Thr Ile Ser Ser Leu Gln Pro Glu
530 535 540
Asp Phe Ala Thr Tyr Tyr Cys Leu Gln Glu Gly Trp Gly Pro Pro Thr
545 550 555 560
Phe Gly Gln Gly Thr Lys Val Glu Ile Lys Arg
565 570

<210> 25
<211> 571
<212> PRT
<213> Artificial Sequence

<220>

<223> Humanised

<400> 25

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
```

```
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Thr Val Ala Ala Pro Ser Gly Ser Asp
    450                 455                 460
Ile Gln Met Thr Gln Ser Pro Ser Ser Leu Ser Ala Ser Val Gly Asp
465                 470                 475                 480
Arg Val Thr Ile Thr Cys Arg Ala Ser Arg Pro Ile Ser Asp Trp Leu
                485                 490                 495
His Trp Tyr Gln Gln Lys Pro Gly Lys Ala Pro Lys Leu Leu Ile Ala
            500                 505                 510
Trp Ala Ser Ser Leu Tyr Glu Gly Val Pro Ser Arg Phe Ser Gly Ser
        515                 520                 525
Gly Ser Gly Thr Asp Phe Thr Leu Thr Ile Ser Ser Leu Gln Pro Glu
    530                 535                 540
Asp Phe Ala Thr Tyr Tyr Cys Leu Gln Glu Gly Trp Gly Pro Pro Thr
545                 550                 555                 560
Phe Gly Gln Gly Thr Lys Val Glu Ile Lys Arg
                565                 570
```

<210> 26

<211> 571
<212> PRT
<213> Artificial Sequence

<220>
<223> Humanised

<400> 26

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Thr Val Ala Ala Pro Ser Gly Ser Asp
450 455 460
Ile Gln Met Thr Gln Ser Pro Ser Ser Leu Ser Ala Ser Val Gly Asp
465 470 475 480
Arg Val Thr Ile Thr Cys Arg Ala Ser Arg Pro Ile Ser Asp Trp Leu
485 490 495
His Trp Tyr Gln Gln Lys Pro Gly Lys Ala Pro Lys Leu Leu Ile Ala
500 505 510
Trp Ala Ser Ser Leu Gln Gly Gly Val Pro Ser Arg Phe Ser Gly Ser
515 520 525
Gly Ser Gly Thr Asp Phe Thr Leu Thr Ile Ser Ser Leu Gln Pro Glu
530 535 540
Asp Phe Ala Thr Tyr Tyr Cys Leu Gln Glu Gly Trp Gly Pro Pro Thr
545 550 555 560
Phe Gly Gln Gly Thr Lys Val Glu Ile Lys Arg
565 570

<210> 27
<211> 579
<212> PRT
<213> Artificial Sequence

<220>
<223> Humanised

<400> 27

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Thr Val Ala Ala Pro Ser Gly Ser Glu
    450                 455                 460
Val Gln Leu Leu Glu Ser Gly Gly Gly Leu Val Gln Pro Gly Gly Ser
465                 470                 475                 480
Leu Arg Leu Ser Cys Ala Ala Ser Gly Phe Thr Phe Arg Asn Phe Gly
                485                 490                 495
Met Gly Trp Val Arg Gln Ala Pro Gly Lys Gly Leu Glu Trp Val Ser
            500                 505                 510
Trp Ile Ile Ser Ser Gly Thr Glu Thr Tyr Tyr Ala Asp Ser Val Lys
        515                 520                 525
Gly Arg Phe Thr Ile Ser Arg Asp Asn Ser Lys Asn Thr Leu Tyr Leu
    530                 535                 540
Gln Met Asn Ser Leu Arg Ala Glu Asp Thr Ala Val Tyr Tyr Cys Ala
```

```
545                 550                 555                 560
Lys Ser Leu Gly Arg Phe Asp Tyr Trp Gly Gln Gly Thr Leu Val Thr
                565                 570                 575
Val Ser Ser
```

<210> 28
<211> 574
<212> PRT
<213> Artificial Sequence

<220>
<223> Humanised

<400> 28

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Ala Ser Thr Lys Gly Pro Thr
    450                 455                 460
Gly Ser Asp Ile Gln Met Thr Gln Ser Pro Ser Ser Leu Ser Ala Ser
465                 470                 475                 480
```

```
Val Gly Asp Arg Val Thr Ile Thr Cys Arg Ala Ser Arg Pro Ile Ser
                485                 490                 495
Asp Trp Leu His Trp Tyr Gln Gln Lys Pro Gly Lys Ala Pro Lys Leu
            500                 505                 510
Leu Ile Ala Trp Ala Ser Thr Leu Asp Ser Gly Val Pro Ser Arg Phe
        515                 520                 525
Ser Gly Ser Gly Ser Gly Thr Asp Phe Thr Leu Thr Ile Ser Ser Leu
    530                 535                 540
Gln Pro Glu Asp Phe Ala Thr Tyr Tyr Cys Leu Gln Glu Gly Trp Gly
545                 550                 555                 560
Pro Pro Thr Phe Gly Gln Gly Thr Lys Val Glu Ile Lys Arg
                565                 570
```

<210> 29
<211> 574
<212> PRT
<213> Artificial Sequence

<220>
<223> Humanised

<400> 29

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
```

```
                    420                     425                     430
Cys Ser Val Met His Glu Ala Leu His Asn His Tyr Thr Gln Lys Ser
            435                     440                     445
Leu Ser Leu Ser Pro Gly Lys Gly Ser Ala Ser Thr Lys Gly Pro Thr
        450                     455                     460
Gly Ser Asp Ile Gln Met Thr Gln Ser Pro Ser Ser Leu Ser Ala Ser
465                     470                     475                     480
Val Gly Asp Arg Val Thr Ile Thr Cys Arg Ala Ser Arg Pro Ile Ser
                485                     490                     495
Asp Trp Leu His Trp Tyr Gln Gln Lys Pro Gly Lys Ala Pro Lys Leu
            500                     505                     510
Leu Ile Ala Trp Ala Ser Ser Leu Tyr Glu Gly Val Pro Ser Arg Phe
        515                     520                     525
Ser Gly Ser Gly Ser Gly Thr Asp Phe Thr Leu Thr Ile Ser Ser Leu
    530                     535                     540
Gln Pro Glu Asp Phe Ala Thr Tyr Tyr Cys Leu Gln Glu Gly Trp Gly
545                     550                     555                     560
Pro Pro Thr Phe Gly Gln Gly Thr Lys Val Glu Ile Lys Arg
                565                     570
```

<210> 30
<211> 574
<212> PRT
<213> Artificial Sequence

<220>
<223> Humanised

<400> 30

```
Gln Val Gln Leu Val Gln Ser Gly Ala Glu Val Lys Lys Pro Gly Ser
 1               5                  10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
```

```
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Ala Ser Thr Lys Gly Pro Thr
    450                 455                 460
Gly Ser Asp Ile Gln Met Thr Gln Ser Pro Ser Ser Leu Ser Ala Ser
465                 470                 475                 480
Val Gly Asp Arg Val Thr Ile Thr Cys Arg Ala Ser Arg Pro Ile Ser
                485                 490                 495
Asp Trp Leu His Trp Tyr Gln Gln Lys Pro Gly Lys Ala Pro Lys Leu
            500                 505                 510
Leu Ile Ala Trp Ala Ser Ser Leu Gln Gly Gly Val Pro Ser Arg Phe
        515                 520                 525
Ser Gly Ser Gly Ser Gly Thr Asp Phe Thr Leu Thr Ile Ser Ser Leu
    530                 535                 540
Gln Pro Glu Asp Phe Ala Thr Tyr Tyr Cys Leu Gln Glu Gly Trp Gly
545                 550                 555                 560
Pro Pro Thr Phe Gly Gln Gly Thr Lys Val Glu Ile Lys Arg
                565                 570
```

<210> 31
<211> 582
<212> PRT
<213> Artificial Sequence

<220>
<223> Humanised

<400> 31

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
```

```
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Ala Ser Thr Lys Gly Pro Thr
    450                 455                 460
Gly Ser Glu Val Gln Leu Leu Glu Ser Gly Gly Gly Leu Val Gln Pro
465                 470                 475                 480
Gly Gly Ser Leu Arg Leu Ser Cys Ala Ala Ser Gly Phe Thr Phe Arg
                485                 490                 495
Asn Phe Gly Met Gly Trp Val Arg Gln Ala Pro Gly Lys Gly Leu Glu
            500                 505                 510
Trp Val Ser Trp Ile Ile Ser Ser Gly Thr Glu Thr Tyr Tyr Ala Asp
        515                 520                 525
Ser Val Lys Gly Arg Phe Thr Ile Ser Arg Asp Asn Ser Lys Asn Thr
    530                 535                 540
Leu Tyr Leu Gln Met Asn Ser Leu Arg Ala Glu Asp Thr Ala Val Tyr
545                 550                 555                 560
Tyr Cys Ala Lys Ser Leu Gly Arg Phe Asp Tyr Trp Gly Gln Gly Thr
                565                 570                 575
Leu Val Thr Val Ser Ser
            580
```

<210> 32
<211> 582
<212> PRT
<213> Artificial Sequence

<220>
<223> Humanised

<400> 32

Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240

```
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Pro Lys Ser Cys Asp Lys
    450                 455                 460
Thr His Thr Cys Pro Pro Cys Pro Gly Ser Asp Ile Gln Met Thr Gln
465                 470                 475                 480
Ser Pro Ser Ser Leu Ser Ala Ser Val Gly Asp Arg Val Thr Ile Thr
                485                 490                 495
Cys Arg Ala Ser Arg Pro Ile Ser Asp Trp Leu His Trp Tyr Gln Gln
            500                 505                 510
Lys Pro Gly Lys Ala Pro Lys Leu Leu Ile Ala Trp Ala Ser Thr Leu
        515                 520                 525
Asp Ser Gly Val Pro Ser Arg Phe Ser Gly Ser Gly Ser Gly Thr Asp
    530                 535                 540
Phe Thr Leu Thr Ile Ser Ser Leu Gln Pro Glu Asp Phe Ala Thr Tyr
545                 550                 555                 560
Tyr Cys Leu Gln Glu Gly Trp Gly Pro Pro Thr Phe Gly Gln Gly Thr
                565                 570                 575
Lys Val Glu Ile Lys Arg
            580
```

<210> 33
<211> 582
<212> PRT
<213> Artificial Sequence

<220>
<223> Humanised

<400> 33

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
```

```
                    165                     170                     175
Thr Phe Pro Ala Val Leu Gln Ser Ser Gly Leu Tyr Ser Leu Ser Ser
            180                     185                     190
Val Val Thr Val Pro Ser Ser Ser Leu Gly Thr Gln Thr Tyr Ile Cys
        195                     200                     205
Asn Val Asn His Lys Pro Ser Asn Thr Lys Val Asp Lys Lys Val Glu
    210                     215                     220
Pro Lys Ser Cys Asp Lys Thr His Thr Cys Pro Pro Cys Pro Ala Pro
225                     230                     235                     240
Glu Leu Leu Gly Gly Pro Ser Val Phe Leu Phe Pro Pro Lys Pro Lys
                245                     250                     255
Asp Thr Leu Met Ile Ser Arg Thr Pro Glu Val Thr Cys Val Val Val
            260                     265                     270
Asp Val Ser His Glu Asp Pro Glu Val Lys Phe Asn Trp Tyr Val Asp
        275                     280                     285
Gly Val Glu Val His Asn Ala Lys Thr Lys Pro Arg Glu Glu Gln Tyr
    290                     295                     300
Asn Ser Thr Tyr Arg Val Val Ser Val Leu Thr Val Leu His Gln Asp
305                     310                     315                     320
Trp Leu Asn Gly Lys Glu Tyr Lys Cys Lys Val Ser Asn Lys Ala Leu
                325                     330                     335
Pro Ala Pro Ile Glu Lys Thr Ile Ser Lys Ala Lys Gly Gln Pro Arg
            340                     345                     350
Glu Pro Gln Val Tyr Thr Leu Pro Pro Ser Arg Asp Glu Leu Thr Lys
        355                     360                     365
Asn Gln Val Ser Leu Thr Cys Leu Val Lys Gly Phe Tyr Pro Ser Asp
    370                     375                     380
Ile Ala Val Glu Trp Glu Ser Asn Gly Gln Pro Glu Asn Asn Tyr Lys
385                     390                     395                     400
Thr Thr Pro Pro Val Leu Asp Ser Asp Gly Ser Phe Phe Leu Tyr Ser
                405                     410                     415
Lys Leu Thr Val Asp Lys Ser Arg Trp Gln Gln Gly Asn Val Phe Ser
            420                     425                     430
Cys Ser Val Met His Glu Ala Leu His Asn His Tyr Thr Gln Lys Ser
        435                     440                     445
Leu Ser Leu Ser Pro Gly Lys Gly Ser Glu Pro Lys Ser Cys Asp Lys
    450                     455                     460
Thr His Thr Cys Pro Pro Cys Pro Gly Ser Asp Ile Gln Met Thr Gln
465                     470                     475                     480
Ser Pro Ser Ser Leu Ser Ala Ser Val Gly Asp Arg Val Thr Ile Thr
                485                     490                     495
Cys Arg Ala Ser Arg Pro Ile Ser Asp Trp Leu His Trp Tyr Gln Gln
            500                     505                     510
Lys Pro Gly Lys Ala Pro Lys Leu Leu Ile Ala Trp Ala Ser Ser Leu
        515                     520                     525
Tyr Glu Gly Val Pro Ser Arg Phe Ser Gly Ser Gly Ser Gly Thr Asp
    530                     535                     540
Phe Thr Leu Thr Ile Ser Ser Leu Gln Pro Glu Asp Phe Ala Thr Tyr
545                     550                     555                     560
Tyr Cys Leu Gln Glu Gly Trp Gly Pro Pro Thr Phe Gly Gln Gly Thr
                565                     570                     575
Lys Val Glu Ile Lys Arg
            580
```

<210> 34
<211> 582
<212> PRT
<213> Artificial Sequence

<220>
<223> Humanised

<400> 34

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
```

```
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Pro Lys Ser Cys Asp Lys
    450                 455                 460
Thr His Thr Cys Pro Pro Cys Pro Gly Ser Asp Ile Gln Met Thr Gln
465                 470                 475                 480
Ser Pro Ser Ser Leu Ser Ala Ser Val Gly Asp Arg Val Thr Ile Thr
                485                 490                 495
Cys Arg Ala Ser Arg Pro Ile Ser Asp Trp Leu His Trp Tyr Gln Gln
            500                 505                 510
Lys Pro Gly Lys Ala Pro Lys Leu Leu Ile Ala Trp Ala Ser Ser Leu
        515                 520                 525
Gln Gly Gly Val Pro Ser Arg Phe Ser Gly Ser Gly Ser Gly Thr Asp
    530                 535                 540
Phe Thr Leu Thr Ile Ser Ser Leu Gln Pro Glu Asp Phe Ala Thr Tyr
545                 550                 555                 560
Tyr Cys Leu Gln Glu Gly Trp Gly Pro Pro Thr Phe Gly Gln Gly Thr
                565                 570                 575
Lys Val Glu Ile Lys Arg
            580
```

<210> 35
<211> 590
<212> PRT

<213> Artificial Sequence

<220>
<223> Humanised

<400> 35

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
```

```
                20                      25                      30
Tyr Met His Trp Val Arg Gln Ala Pro Gly Gln Gly Leu Glu Trp Met
            35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                      80
Met Glu Leu Ser Ser Leu Arg Ser Glu Asp Thr Ala Val Tyr Tyr Cys
                85                      90                      95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                     160
Pro Val Thr Val Ser Trp Asn Ser Gly Ala Leu Thr Ser Gly Val His
                165                     170                     175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                     240
Glu Leu Leu Gly Gly Pro Ser Val Phe Leu Phe Pro Pro Lys Pro Lys
                245                     250                     255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                     320
Trp Leu Asn Gly Lys Glu Tyr Lys Cys Lys Val Ser Asn Lys Ala Leu
                325                     330                     335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                     400
Thr Thr Pro Pro Val Leu Asp Ser Asp Gly Ser Phe Phe Leu Tyr Ser
                405                     410                     415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Pro Lys Ser Cys Asp Lys
    450                 455                 460
Thr His Thr Cys Pro Pro Cys Pro Gly Ser Glu Val Gln Leu Leu Glu
465                 470                 475                     480
Ser Gly Gly Gly Leu Val Gln Pro Gly Gly Ser Leu Arg Leu Ser Cys
                485                     490                     495
Ala Ala Ser Gly Phe Thr Phe Arg Asn Phe Gly Met Gly Trp Val Arg
            500                 505                 510
Gln Ala Pro Gly Lys Gly Leu Glu Trp Val Ser Trp Ile Ile Ser Ser
        515                 520                 525
Gly Thr Glu Thr Tyr Tyr Ala Asp Ser Val Lys Gly Arg Phe Thr Ile
    530                 535                 540
Ser Arg Asp Asn Ser Lys Asn Thr Leu Tyr Leu Gln Met Asn Ser Leu
545                 550                 555                     560
Arg Ala Glu Asp Thr Ala Val Tyr Tyr Cys Ala Lys Ser Leu Gly Arg
                565                     570                     575
Phe Asp Tyr Trp Gly Gln Gly Thr Leu Val Thr Val Ser Ser
            580                 585                 590
```

<210> 36
<211> 585
<212> PRT
<213> Artificial Sequence

<220>
<223> Humanised

<400> 36

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Ser Glu Leu Gln Leu Glu Glu Ser
450 455 460
Cys Ala Glu Ala Gln Asp Gly Glu Leu Asp Gly Gly Ser Asp Ile Gln
465 470 475 480
Met Thr Gln Ser Pro Ser Ser Leu Ser Ala Ser Val Gly Asp Arg Val
485 490 495
Thr Ile Thr Cys Arg Ala Ser Arg Pro Ile Ser Asp Trp Leu His Trp
500 505 510
Tyr Gln Gln Lys Pro Gly Lys Ala Pro Lys Leu Leu Ile Ala Trp Ala
515 520 525
Ser Thr Leu Asp Ser Gly Val Pro Ser Arg Phe Ser Gly Ser Gly Ser
530 535 540
Gly Thr Asp Phe Thr Leu Thr Ile Ser Ser Leu Gln Pro Glu Asp Phe
545 550 555 560

```
Ala Thr Tyr Tyr Cys Leu Gln Glu Gly Trp Gly Pro Pro Thr Phe Gly
                565                 570                 575
Gln Gly Thr Lys Val Glu Ile Lys Arg
            580                 585
```

<210> 37
<211> 585
<212> PRT
<213> Artificial Sequence

<220>
<223> Humanised

<400> 37

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Leu Gln Leu Glu Glu Ser
    450                 455                 460
Cys Ala Glu Ala Gln Asp Gly Glu Leu Asp Gly Gly Ser Asp Ile Gln
465                 470                 475                 480
Met Thr Gln Ser Pro Ser Ser Leu Ser Ala Ser Val Gly Asp Arg Val
```

Thr Ile Thr Cys Arg Ala Ser Arg Pro Ile Ser Asp Trp Leu His Trp
485 490 495
Tyr Gln Gln Lys Pro Gly Lys Ala Pro Lys Leu Leu Ile Ala Trp Ala
500 505 510
Ser Ser Leu Tyr Glu Gly Val Pro Ser Arg Phe Ser Gly Ser Gly Ser
515 520 525
Gly Thr Asp Phe Thr Leu Thr Ile Ser Ser Leu Gln Pro Glu Asp Phe
530 535 540
Ala Thr Tyr Tyr Cys Leu Gln Glu Gly Trp Gly Pro Pro Thr Phe Gly
545 550 555 560
Gln Gly Thr Lys Val Glu Ile Lys Arg
565 570 575
580 585

<210> 38
<211> 585
<212> PRT
<213> Artificial Sequence

<220>
<223> Humanised

<400> 38

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
```

Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Ser Glu Leu Gln Leu Glu Glu Ser
450 455 460
Cys Ala Glu Ala Gln Asp Gly Glu Leu Asp Gly Gly Ser Asp Ile Gln
465 470 475 480
Met Thr Gln Ser Pro Ser Ser Leu Ser Ala Ser Val Gly Asp Arg Val
485 490 495
Thr Ile Thr Cys Arg Ala Ser Arg Pro Ile Ser Asp Trp Leu His Trp
500 505 510
Tyr Gln Gln Lys Pro Gly Lys Ala Pro Lys Leu Leu Ile Ala Trp Ala
515 520 525
Ser Ser Leu Gln Gly Gly Val Pro Ser Arg Phe Ser Gly Ser Gly Ser
530 535 540
Gly Thr Asp Phe Thr Leu Thr Ile Ser Ser Leu Gln Pro Glu Asp Phe
545 550 555 560
Ala Thr Tyr Tyr Cys Leu Gln Glu Gly Trp Gly Pro Pro Thr Phe Gly
565 570 575
Gln Gly Thr Lys Val Glu Ile Lys Arg
580 585

<210> 39
<211> 593
<212> PRT
<213> Artificial Sequence

<220>
<223> Humanised

<400> 39

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
```

```
            340                     345                     350
Glu Pro Gln Val Tyr Thr Leu Pro Pro Ser Arg Asp Glu Leu Thr Lys
        355                     360                     365
Asn Gln Val Ser Leu Thr Cys Leu Val Lys Gly Phe Tyr Pro Ser Asp
    370                     375                     380
Ile Ala Val Glu Trp Glu Ser Asn Gly Gln Pro Glu Asn Asn Tyr Lys
385                     390                     395                 400
Thr Thr Pro Pro Val Leu Asp Ser Asp Gly Ser Phe Phe Leu Tyr Ser
                405                     410                     415
Lys Leu Thr Val Asp Lys Ser Arg Trp Gln Gln Gly Asn Val Phe Ser
            420                     425                     430
Cys Ser Val Met His Glu Ala Leu His Asn His Tyr Thr Gln Lys Ser
        435                     440                     445
Leu Ser Leu Ser Pro Gly Lys Gly Ser Glu Leu Gln Leu Glu Glu Ser
    450                     455                     460
Cys Ala Glu Ala Gln Asp Gly Glu Leu Asp Gly Gly Ser Glu Val Gln
465                     470                     475                 480
Leu Leu Glu Ser Gly Gly Gly Leu Val Gln Pro Gly Gly Ser Leu Arg
                485                     490                     495
Leu Ser Cys Ala Ala Ser Gly Phe Thr Phe Arg Asn Phe Gly Met Gly
            500                     505                     510
Trp Val Arg Gln Ala Pro Gly Lys Gly Leu Glu Trp Val Ser Trp Ile
        515                     520                     525
Ile Ser Ser Gly Thr Glu Thr Tyr Tyr Ala Asp Ser Val Lys Gly Arg
    530                     535                     540
Phe Thr Ile Ser Arg Asp Asn Ser Lys Asn Thr Leu Tyr Leu Gln Met
545                     550                     555                 560
Asn Ser Leu Arg Ala Glu Asp Thr Ala Val Tyr Tyr Cys Ala Lys Ser
                565                     570                     575
Leu Gly Arg Phe Asp Tyr Trp Gly Gln Gly Thr Leu Val Thr Val Ser
            580                     585                     590
Ser
```

<210> 40
<211> 457
<212> PRT
<213> Artificial Sequence

<220>
<223> Humanised

<400> 40

Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser
    450                 455

<210> 41
<211> 466
<212> PRT
<213> Artificial Sequence

<220>
<223> Humanised

<400> 41

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
```

```
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Ala Ser Thr Lys Gly Pro Thr
    450                 455                 460
Gly Ser
465
```

<210> 42
<211> 474
<212> PRT
<213> Artificial Sequence

<220>
<223> Humanised

<400> 42

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350

Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Ser Glu Pro Lys Ser Cys Asp Lys
450 455 460
Thr His Thr Cys Pro Pro Cys Pro Gly Ser
465 470

<210> 43
<211> 477
<212> PRT

<213> Artificial Sequence

<220>
<223> Humanised

<400> 43

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
```

```
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Gly Ser Glu Leu Gln Leu Glu Glu Ser
    450                 455                 460
Cys Ala Glu Ala Gln Asp Gly Glu Leu Asp Gly Gly Ser
465                 470                 475
```

<210> 44
<211> 332
<212> PRT
<213> Artificial Sequence

<220>
<223> Humanised

<400> 44

```
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Asn Ile Val His Ile
            20                  25                  30
Asn Gly Asn Thr Tyr Leu Glu Trp Tyr Leu Gln Lys Pro Gly Gln Ser
        35                  40                  45
Pro Arg Leu Leu Ile Tyr Lys Ile Ser Asp Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Asp Asp Val Gly Ile Tyr Tyr Cys Phe Gln Gly
                85                  90                  95
Ser His Val Pro Trp Thr Phe Gly Gln Gly Thr Lys Leu Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys Gly Gly Gly Gly Ser
    210                 215                 220
Asp Ile Gln Met Thr Gln Ser Pro Ser Ser Leu Ser Ala Ser Val Gly
225                 230                 235                 240
Asp Arg Val Thr Ile Thr Cys Arg Ala Ser Arg Pro Ile Ser Asp Trp
                245                 250                 255
Leu His Trp Tyr Gln Gln Lys Pro Gly Lys Ala Pro Lys Leu Leu Ile
            260                 265                 270
Ala Trp Ala Ser Thr Leu Asp Ser Gly Val Pro Ser Arg Phe Ser Gly
        275                 280                 285
Ser Gly Ser Gly Thr Asp Phe Thr Leu Thr Ile Ser Ser Leu Gln Pro
    290                 295                 300
Glu Asp Phe Ala Thr Tyr Tyr Cys Leu Gln Glu Gly Trp Gly Pro Pro
305                 310                 315                 320
Thr Phe Gly Gln Gly Thr Lys Val Glu Ile Lys Arg
                325                 330
```

<210> 45
<211> 332
<212> PRT
<213> Artificial Sequence

<220>
<223> Humanised

<400> 45

```
Asp Ile Val Met Thr Gln Ser Pro Leu Ser Leu Pro Val Thr Pro Gly
```

```
Glu Pro Ala Ser Ile Ser Cys Arg Ser Ser Gln Asn Ile Val His Ile
1               5                   10                  15
Asn Gly Asn Thr Tyr Leu Glu Trp Tyr Leu Gln Lys Pro Gly Gln Ser
            20                  25                  30
Pro Arg Leu Leu Ile Tyr Lys Ile Ser Asp Arg Phe Ser Gly Val Pro
        35                  40                  45
Asp Arg Phe Ser Gly Ser Gly Ser Gly Thr Asp Phe Thr Leu Lys Ile
    50                  55                  60
Ser Arg Val Glu Ala Asp Asp Val Gly Ile Tyr Tyr Cys Phe Gln Gly
65                  70                  75                  80
Ser His Val Pro Trp Thr Phe Gly Gln Gly Thr Lys Leu Glu Ile Lys
                85                  90                  95
Arg Thr Val Ala Ala Pro Ser Val Phe Ile Phe Pro Pro Ser Asp Glu
            100                 105                 110
Gln Leu Lys Ser Gly Thr Ala Ser Val Val Cys Leu Leu Asn Asn Phe
        115                 120                 125
Tyr Pro Arg Glu Ala Lys Val Gln Trp Lys Val Asp Asn Ala Leu Gln
    130                 135                 140
Ser Gly Asn Ser Gln Glu Ser Val Thr Glu Gln Asp Ser Lys Asp Ser
145                 150                 155                 160
Thr Tyr Ser Leu Ser Ser Thr Leu Thr Leu Ser Lys Ala Asp Tyr Glu
                165                 170                 175
Lys His Lys Val Tyr Ala Cys Glu Val Thr His Gln Gly Leu Ser Ser
            180                 185                 190
Pro Val Thr Lys Ser Phe Asn Arg Gly Glu Cys Gly Gly Gly Gly Ser
        195                 200                 205
Asp Ile Gln Met Thr Gln Ser Pro Ser Ser Leu Ser Ala Ser Val Gly
    210                 215                 220
Asp Arg Val Thr Ile Thr Cys Arg Ala Ser Arg Pro Ile Ser Asp Trp
225                 230                 235                 240
Leu His Trp Tyr Gln Gln Lys Pro Gly Lys Ala Pro Lys Leu Leu Ile
                245                 250                 255
Ala Trp Ala Ser Ser Leu Tyr Glu Gly Val Pro Ser Arg Phe Ser Gly
            260                 265                 270
Ser Gly Ser Gly Thr Asp Phe Thr Leu Thr Ile Ser Ser Leu Gln Pro
        275                 280                 285
Glu Asp Phe Ala Thr Tyr Tyr Cys Leu Gln Glu Gly Trp Gly Pro Pro
    290                 295                 300
Thr Phe Gly Gln Gly Thr Lys Val Glu Ile Lys Arg
305                 310                 315                 320
                325                 330
```

<210> 46
<211> 332
<212> PRT
<213> Artificial Sequence

<220>
<223> Humanised

<400> 46

Asp Ile Val Met Thr Gln Ser Pro Leu Ser Leu Pro Val Thr Pro Gly
1 5 10 15
Glu Pro Ala Ser Ile Ser Cys Arg Ser Ser Gln Asn Ile Val His Ile
20 25 30
Asn Gly Asn Thr Tyr Leu Glu Trp Tyr Leu Gln Lys Pro Gly Gln Ser
35 40 45
Pro Arg Leu Leu Ile Tyr Lys Ile Ser Asp Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Asp Asp Val Gly Ile Tyr Tyr Cys Phe Gln Gly
85 90 95
Ser His Val Pro Trp Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205
Pro Val Thr Lys Ser Phe Asn Arg Gly Glu Cys Gly Gly Gly Gly Ser
210 215 220
Asp Ile Gln Met Thr Gln Ser Pro Ser Ser Leu Ser Ala Ser Val Gly
225 230 235 240
Asp Arg Val Thr Ile Thr Cys Arg Ala Ser Arg Pro Ile Ser Asp Trp
245 250 255
Leu His Trp Tyr Gln Gln Lys Pro Gly Lys Ala Pro Lys Leu Leu Ile
260 265 270
Ala Trp Ala Ser Ser Leu Gln Gly Gly Val Pro Ser Arg Phe Ser Gly
275 280 285
Ser Gly Ser Gly Thr Asp Phe Thr Leu Thr Ile Ser Ser Leu Gln Pro
290 295 300
Glu Asp Phe Ala Thr Tyr Tyr Cys Leu Gln Glu Gly Trp Gly Pro Pro
305 310 315 320
Thr Phe Gly Gln Gly Thr Lys Val Glu Ile Lys Arg
325 330

<210> 47
<211> 340
<212> PRT
<213> Artificial Sequence

<220>
<223> Humanised

<400> 47

Asp Ile Val Met Thr Gln Ser Pro Leu Ser Leu Pro Val Thr Pro Gly
1 5 10 15
Glu Pro Ala Ser Ile Ser Cys Arg Ser Ser Gln Asn Ile Val His Ile
20 25 30
Asn Gly Asn Thr Tyr Leu Glu Trp Tyr Leu Gln Lys Pro Gly Gln Ser
35 40 45
Pro Arg Leu Leu Ile Tyr Lys Ile Ser Asp Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Asp Asp Val Gly Ile Tyr Tyr Cys Phe Gln Gly
85 90 95
Ser His Val Pro Trp Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190
Lys His Lys Val Tyr Ala Cys Glu Val Thr His Gln Gly Leu Ser Ser
195 200 205
Pro Val Thr Lys Ser Phe Asn Arg Gly Glu Cys Gly Gly Gly Gly Ser
210 215 220
Glu Val Gln Leu Leu Glu Ser Gly Gly Gly Leu Val Gln Pro Gly Gly
225 230 235 240
Ser Leu Arg Leu Ser Cys Ala Ala Ser Gly Phe Thr Phe Arg Asn Phe
245 250 255
Gly Met Gly Trp Val Arg Gln Ala Pro Gly Lys Gly Leu Glu Trp Val
260 265 270
Ser Trp Ile Ile Ser Ser Gly Thr Glu Thr Tyr Tyr Ala Asp Ser Val
275 280 285
Lys Gly Arg Phe Thr Ile Ser Arg Asp Asn Ser Lys Asn Thr Leu Tyr
290 295 300
Leu Gln Met Asn Ser Leu Arg Ala Glu Asp Thr Ala Val Tyr Tyr Cys
305 310 315 320
Ala Lys Ser Leu Gly Arg Phe Asp Tyr Trp Gly Gln Gly Thr Leu Val
325 330 335
Thr Val Ser Ser
340

<210> 48
<211> 571
<212> PRT
<213> Artificial Sequence

<220>
<223> Humanised

<400> 48

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Ser Gly Val Gln Leu Leu Glu Ser Gly Gly Gly Leu
450 455 460
Val Gln Pro Gly Gly Ser Leu Arg Leu Ser Cys Ala Ala Ser Gly Phe
465 470 475 480
Thr Phe Ala Trp Tyr Asp Met Gly Trp Val Arg Gln Ala Pro Gly Lys
485 490 495
Gly Leu Glu Trp Val Ser Ser Ile Asp Trp His Gly Glu Val Thr Tyr
500 505 510
Tyr Ala Asp Ser Val Lys Gly Arg Phe Thr Ile Ser Arg Asp Asn Ser
515 520 525
Lys Asn Thr Leu Tyr Leu Gln Met Asn Ser Leu Arg Ala Glu Asp Thr
530 535 540

```
Ala Val Tyr Tyr Cys Ala Thr Ala Glu Asp Glu Pro Gly Tyr Asp Tyr
545                 550                 555                 560
Trp Gly Gln Gly Thr Leu Val Thr Val Ser Ser
                565                 570
```

<210> 49
<211> 574
<212> PRT
<213> Artificial Sequence

<220>
<223> Humanised

<400> 49

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Gly Gly Gly Ser Gly Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Ala Trp Tyr Asp Met Gly Trp Val Arg Gln Ala
```

```
                        485                     490                     495
Pro Gly Lys Gly Leu Glu Trp Val Ser Ser Ile Asp Trp His Gly Glu
                500                     505                     510
Val Thr Tyr Tyr Ala Asp Ser Val Lys Gly Arg Phe Thr Ile Ser Arg
            515                     520                     525
Asp Asn Ser Lys Asn Thr Leu Tyr Leu Gln Met Asn Ser Leu Arg Ala
    530                     535                     540
Glu Asp Thr Ala Val Tyr Tyr Cys Ala Thr Ala Glu Asp Glu Pro Gly
545                     550                     555                     560
Tyr Asp Tyr Trp Gly Gln Gly Thr Leu Val Thr Val Ser Ser
                565                     570
```

<210> 50
<211> 577
<212> PRT
<213> Artificial Sequence

<220>
<223> Humanised

<400> 50

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
```

His Glu Ala Leu His Asn His Tyr Thr Gln Lys Ser Leu Ser Leu Ser
435 440 445
Pro Gly Lys Thr Val Ala Ala Pro Ser Gly Ser Gly Val Gln Leu Leu
450 455 460
Glu Ser Gly Gly Gly Leu Val Gln Pro Gly Gly Ser Leu Arg Leu Ser
465 470 475 480
Cys Ala Ala Ser Gly Phe Thr Phe Ala Trp Tyr Asp Met Gly Trp Val
485 490 495
Arg Gln Ala Pro Gly Lys Gly Leu Glu Trp Val Ser Ser Ile Asp Trp
500 505 510
His Gly Glu Val Thr Tyr Tyr Ala Asp Ser Val Lys Gly Arg Phe Thr
515 520 525
Ile Ser Arg Asp Asn Ser Lys Asn Thr Leu Tyr Leu Gln Met Asn Ser
530 535 540
Leu Arg Ala Glu Asp Thr Ala Val Tyr Tyr Cys Ala Thr Ala Glu Asp
545 550 555 560
Glu Pro Gly Tyr Asp Tyr Trp Gly Gln Gly Thr Leu Val Thr Val Ser
565 570 575
Ser

<210> 51
<211> 580
<212> PRT
<213> Artificial Sequence

<220>
<223> Humanised

<400> 51

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
```

```
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Ala Ser Thr Lys Gly Pro Thr Gly Ser Gly Val
    450                 455                 460
Gln Leu Leu Glu Ser Gly Gly Gly Leu Val Gln Pro Gly Gly Ser Leu
465                 470                 475                 480
Arg Leu Ser Cys Ala Ala Ser Gly Phe Thr Phe Ala Trp Tyr Asp Met
                485                 490                 495
Gly Trp Val Arg Gln Ala Pro Gly Lys Gly Leu Glu Trp Val Ser Ser
            500                 505                 510
Ile Asp Trp His Gly Glu Val Thr Tyr Tyr Ala Asp Ser Val Lys Gly
        515                 520                 525
Arg Phe Thr Ile Ser Arg Asp Asn Ser Lys Asn Thr Leu Tyr Leu Gln
    530                 535                 540
Met Asn Ser Leu Arg Ala Glu Asp Thr Ala Val Tyr Tyr Cys Ala Thr
545                 550                 555                 560
Ala Glu Asp Glu Pro Gly Tyr Asp Tyr Trp Gly Gln Gly Thr Leu Val
                565                 570                 575
Thr Val Ser Ser
            580
```

<210> 52
<211> 588
<212> PRT
<213> Artificial Sequence

<220>
<223> Humanised

<400> 52

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285

```
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Glu Pro Lys Ser Cys Asp Lys Thr His Thr Cys
    450                 455                 460
Pro Pro Cys Pro Gly Ser Gly Val Gln Leu Leu Glu Ser Gly Gly Gly
465                 470                 475                 480
Leu Val Gln Pro Gly Gly Ser Leu Arg Leu Ser Cys Ala Ala Ser Gly
                485                 490                 495
Phe Thr Phe Ala Trp Tyr Asp Met Gly Trp Val Arg Gln Ala Pro Gly
            500                 505                 510
Lys Gly Leu Glu Trp Val Ser Ser Ile Asp Trp His Gly Glu Val Thr
        515                 520                 525
Tyr Tyr Ala Asp Ser Val Lys Gly Arg Phe Thr Ile Ser Arg Asp Asn
    530                 535                 540
Ser Lys Asn Thr Leu Tyr Leu Gln Met Asn Ser Leu Arg Ala Glu Asp
545                 550                 555                 560
Thr Ala Val Tyr Tyr Cys Ala Thr Ala Glu Asp Glu Pro Gly Tyr Asp
                565                 570                 575
Tyr Trp Gly Gln Gly Thr Leu Val Thr Val Ser Ser
            580                 585
```

<210> 53
<211> 591
<212> PRT
<213> Artificial Sequence

<220>
<223> Humanised

<400> 53

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
```

```
     210                     215                     220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Glu Leu Gln Leu Glu Glu Ser Cys Ala Glu Ala
    450                 455                 460
Gln Asp Gly Glu Leu Asp Gly Gly Ser Gly Val Gln Leu Leu Glu Ser
465                 470                 475                 480
Gly Gly Gly Leu Val Gln Pro Gly Gly Ser Leu Arg Leu Ser Cys Ala
                485                 490                 495
Ala Ser Gly Phe Thr Phe Ala Trp Tyr Asp Met Gly Trp Val Arg Gln
            500                 505                 510
Ala Pro Gly Lys Gly Leu Glu Trp Val Ser Ser Ile Asp Trp His Gly
        515                 520                 525
Glu Val Thr Tyr Tyr Ala Asp Ser Val Lys Gly Arg Phe Thr Ile Ser
    530                 535                 540
Arg Asp Asn Ser Lys Asn Thr Leu Tyr Leu Gln Met Asn Ser Leu Arg
545                 550                 555                 560
Ala Glu Asp Thr Ala Val Tyr Tyr Cys Ala Thr Ala Glu Asp Glu Pro
                565                 570                 575
Gly Tyr Asp Tyr Trp Gly Gln Gly Thr Leu Val Thr Val Ser Ser
            580                 585                 590
```

<210> 54
<211> 338
<212> PRT
<213> Artificial Sequence

<220>
<223> Humanised

<400> 54

Asp Ile Val Leu Thr Gln Ser Pro Ser Ser Leu Ser Ala Ser Val Gly
1               5                   10                  15
Asp Arg Val Thr Ile Thr Cys Lys Ala Ser Gln Ser Val Asp Tyr Asp
            20                  25                  30
Gly Asp Ser Tyr Met Asn Trp Tyr Gln Gln Lys Pro Gly Lys Ala Pro
        35                  40                  45
Lys Leu Leu Ile Tyr Ala Ala Ser Asn Leu Glu Ser Gly Ile Pro Ser
    50                  55                  60
Arg Phe Ser Gly Ser Gly Ser Gly Thr Asp Phe Thr Phe Thr Ile Ser
65                  70                  75                  80
Ser Leu Gln Pro Glu Asp Ile Ala Thr Tyr Tyr Cys Gln Gln Ser Asn
                85                  90                  95
Glu Asp Pro Pro Thr Phe Gly Gln Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys Gly Ser Gly Val Gln Leu
    210                 215                 220
Leu Glu Ser Gly Gly Gly Leu Val Gln Pro Gly Gly Ser Leu Arg Leu
225                 230                 235                 240
Ser Cys Ala Ala Ser Gly Phe Thr Phe Ala Trp Tyr Asp Met Gly Trp
                245                 250                 255
Val Arg Gln Ala Pro Gly Lys Gly Leu Glu Trp Val Ser Ser Ile Asp
            260                 265                 270
Trp His Gly Glu Val Thr Tyr Tyr Ala Asp Ser Val Lys Gly Arg Phe
        275                 280                 285
Thr Ile Ser Arg Asp Asn Ser Lys Asn Thr Leu Tyr Leu Gln Met Asn
    290                 295                 300
Ser Leu Arg Ala Glu Asp Thr Ala Val Tyr Tyr Cys Ala Thr Ala Glu
305                 310                 315                 320
Asp Glu Pro Gly Tyr Asp Tyr Trp Gly Gln Gly Thr Leu Val Thr Val
                325                 330                 335
Ser Ser

<210> 55
<211> 341
<212> PRT
<213> Artificial Sequence

<220>
<223> Humanised

<400> 55

Asp Ile Val Leu Thr Gln Ser Pro Ser Ser Leu Ser Ala Ser Val Gly
1 5 10 15
Asp Arg Val Thr Ile Thr Cys Lys Ala Ser Gln Ser Val Asp Tyr Asp
20 25 30
Gly Asp Ser Tyr Met Asn Trp Tyr Gln Gln Lys Pro Gly Lys Ala Pro
35 40 45
Lys Leu Leu Ile Tyr Ala Ala Ser Asn Leu Glu Ser Gly Ile Pro Ser
50 55 60
Arg Phe Ser Gly Ser Gly Ser Gly Thr Asp Phe Thr Phe Thr Ile Ser
65 70 75 80
Ser Leu Gln Pro Glu Asp Ile Ala Thr Tyr Tyr Cys Gln Gln Ser Asn
85 90 95
Glu Asp Pro Pro Thr Phe Gly Gln Gly Thr Lys Val Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys Gly Gly Gly Gly Ser Gly
210 215 220
Val Gln Leu Leu Glu Ser Gly Gly Gly Leu Val Gln Pro Gly Gly Ser
225 230 235 240
Leu Arg Leu Ser Cys Ala Ala Ser Gly Phe Thr Phe Ala Trp Tyr Asp
245 250 255
Met Gly Trp Val Arg Gln Ala Pro Gly Lys Gly Leu Glu Trp Val Ser
260 265 270
Ser Ile Asp Trp His Gly Glu Val Thr Tyr Tyr Ala Asp Ser Val Lys
275 280 285
Gly Arg Phe Thr Ile Ser Arg Asp Asn Ser Lys Asn Thr Leu Tyr Leu
290 295 300
Gln Met Asn Ser Leu Arg Ala Glu Asp Thr Ala Val Tyr Tyr Cys Ala

305 310 315 320
Thr Ala Glu Asp Glu Pro Gly Tyr Asp Tyr Trp Gly Gln Gly Thr Leu
325 330 335
Val Thr Val Ser Ser
340

<210> 56
<211> 344
<212> PRT
<213> Artificial Sequence

<220>
<223> Humanised

<400> 56

Asp Ile Val Leu Thr Gln Ser Pro Ser Ser Leu Ser Ala Ser Val Gly
1 5 10 15
Asp Arg Val Thr Ile Thr Cys Lys Ala Ser Gln Ser Val Asp Tyr Asp
20 25 30
Gly Asp Ser Tyr Met Asn Trp Tyr Gln Gln Lys Pro Gly Lys Ala Pro
35 40 45
Lys Leu Leu Ile Tyr Ala Ala Ser Asn Leu Glu Ser Gly Ile Pro Ser
50 55 60
Arg Phe Ser Gly Ser Gly Ser Gly Thr Asp Phe Thr Phe Thr Ile Ser
65 70 75 80
Ser Leu Gln Pro Glu Asp Ile Ala Thr Tyr Tyr Cys Gln Gln Ser Asn
85 90 95
Glu Asp Pro Pro Thr Phe Gly Gln Gly Thr Lys Val Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys Thr Val Ala Ala Pro Ser
210 215 220
Gly Ser Gly Val Gln Leu Leu Glu Ser Gly Gly Gly Leu Val Gln Pro
225 230 235 240
Gly Gly Ser Leu Arg Leu Ser Cys Ala Ala Ser Gly Phe Thr Phe Ala
245 250 255
Trp Tyr Asp Met Gly Trp Val Arg Gln Ala Pro Gly Lys Gly Leu Glu
260 265 270
Trp Val Ser Ser Ile Asp Trp His Gly Glu Val Thr Tyr Tyr Ala Asp
275 280 285
Ser Val Lys Gly Arg Phe Thr Ile Ser Arg Asp Asn Ser Lys Asn Thr
290 295 300
Leu Tyr Leu Gln Met Asn Ser Leu Arg Ala Glu Asp Thr Ala Val Tyr
305 310 315 320
Tyr Cys Ala Thr Ala Glu Asp Glu Pro Gly Tyr Asp Tyr Trp Gly Gln
325 330 335
Gly Thr Leu Val Thr Val Ser Ser
340

<210> 57
<211> 345
<212> PRT
<213> Artificial Sequence

<220>
<223> Humanised

<400> 57

Asp Ile Val Leu Thr Gln Ser Pro Ser Ser Leu Ser Ala Ser Val Gly
1 5 10 15
Asp Arg Val Thr Ile Thr Cys Lys Ala Ser Gln Ser Val Asp Tyr Asp
20 25 30
Gly Asp Ser Tyr Met Asn Trp Tyr Gln Gln Lys Pro Gly Lys Ala Pro

```
                35                      40                      45
Lys Leu Leu Ile Tyr Ala Ala Ser Asn Leu Glu Ser Gly Ile Pro Ser
    50                      55                      60
Arg Phe Ser Gly Ser Gly Ser Gly Thr Asp Phe Thr Phe Thr Ile Ser
65                      70                      75                  80
Ser Leu Gln Pro Glu Asp Ile Ala Thr Tyr Tyr Cys Gln Gln Ser Asn
                85                      90                      95
Glu Asp Pro Pro Thr Phe Gly Gln Gly Thr Lys Val Glu Ile Lys Arg
            100                     105                     110
Thr Val Ala Ala Pro Ser Val Phe Ile Phe Pro Pro Ser Asp Glu Gln
        115                     120                     125
Leu Lys Ser Gly Thr Ala Ser Val Val Cys Leu Leu Asn Asn Phe Tyr
    130                     135                     140
Pro Arg Glu Ala Lys Val Gln Trp Lys Val Asp Asn Ala Leu Gln Ser
145                     150                     155                 160
Gly Asn Ser Gln Glu Ser Val Thr Glu Gln Asp Ser Lys Asp Ser Thr
                165                     170                     175
Tyr Ser Leu Ser Ser Thr Leu Thr Leu Ser Lys Ala Asp Tyr Glu Lys
            180                     185                     190
His Lys Val Tyr Ala Cys Glu Val Thr His Gln Gly Leu Ser Ser Pro
        195                     200                     205
Val Thr Lys Ser Phe Asn Arg Gly Glu Cys Ala Ser Thr Lys Gly Pro
    210                     215                     220
Thr Gly Ser Gly Val Gln Leu Leu Glu Ser Gly Gly Gly Leu Val Gln
225                     230                     235                 240
Pro Gly Gly Ser Leu Arg Leu Ser Cys Ala Ala Ser Gly Phe Thr Phe
                245                     250                     255
Ala Trp Tyr Asp Met Gly Trp Val Arg Gln Ala Pro Gly Lys Gly Leu
            260                     265                     270
Glu Trp Val Ser Ser Ile Asp Trp His Gly Glu Val Thr Tyr Tyr Ala
        275                     280                     285
Asp Ser Val Lys Gly Arg Phe Thr Ile Ser Arg Asp Asn Ser Lys Asn
    290                     295                     300
Thr Leu Tyr Leu Gln Met Asn Ser Leu Arg Ala Glu Asp Thr Ala Val
305                     310                     315                 320
Tyr Tyr Cys Ala Thr Ala Glu Asp Glu Pro Gly Tyr Asp Tyr Trp Gly
                325                     330                     335
Gln Gly Thr Leu Val Thr Val Ser Ser
            340                     345
```

<210> 58
<211> 353
<212> PRT
<213> Artificial Sequence

<220>
<223> Humanised

<400> 58

Asp Ile Val Leu Thr Gln Ser Pro Ser Ser Leu Ser Ala Ser Val Gly
1 5 10 15
Asp Arg Val Thr Ile Thr Cys Lys Ala Ser Gln Ser Val Asp Tyr Asp
20 25 30
Gly Asp Ser Tyr Met Asn Trp Tyr Gln Gln Lys Pro Gly Lys Ala Pro
35 40 45
Lys Leu Leu Ile Tyr Ala Ala Ser Asn Leu Glu Ser Gly Ile Pro Ser
50 55 60
Arg Phe Ser Gly Ser Gly Ser Gly Thr Asp Phe Thr Phe Thr Ile Ser
65 70 75 80
Ser Leu Gln Pro Glu Asp Ile Ala Thr Tyr Tyr Cys Gln Gln Ser Asn
85 90 95
Glu Asp Pro Pro Thr Phe Gly Gln Gly Thr Lys Val Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205

Val Thr Lys Ser Phe Asn Arg Gly Glu Cys Glu Pro Lys Ser Cys Asp
210 215 220
Lys Thr His Thr Cys Pro Pro Cys Pro Gly Ser Gly Val Gln Leu Leu
225 230 235 240
Glu Ser Gly Gly Gly Leu Val Gln Pro Gly Gly Ser Leu Arg Leu Ser
245 250 255
Cys Ala Ala Ser Gly Phe Thr Phe Ala Trp Tyr Asp Met Gly Trp Val
260 265 270
Arg Gln Ala Pro Gly Lys Gly Leu Glu Trp Val Ser Ser Ile Asp Trp
275 280 285
His Gly Glu Val Thr Tyr Tyr Ala Asp Ser Val Lys Gly Arg Phe Thr
290 295 300
Ile Ser Arg Asp Asn Ser Lys Asn Thr Leu Tyr Leu Gln Met Asn Ser
305 310 315 320
Leu Arg Ala Glu Asp Thr Ala Val Tyr Tyr Cys Ala Thr Ala Glu Asp
325 330 335
Glu Pro Gly Tyr Asp Tyr Trp Gly Gln Gly Thr Leu Val Thr Val Ser
340 345 350
Ser

<210> 59
<211> 356
<212> PRT
<213> Artificial Sequence

<220>
<223> Humanised

<400> 59

```
Asp Ile Val Leu Thr Gln Ser Pro Ser Ser Leu Ser Ala Ser Val Gly
1               5                   10                  15
Asp Arg Val Thr Ile Thr Cys Lys Ala Ser Gln Ser Val Asp Tyr Asp
            20                  25                  30
Gly Asp Ser Tyr Met Asn Trp Tyr Gln Gln Lys Pro Gly Lys Ala Pro
        35                  40                  45
Lys Leu Leu Ile Tyr Ala Ala Ser Asn Leu Glu Ser Gly Ile Pro Ser
    50                  55                  60
Arg Phe Ser Gly Ser Gly Ser Gly Thr Asp Phe Thr Phe Thr Ile Ser
65                  70                  75                  80
Ser Leu Gln Pro Glu Asp Ile Ala Thr Tyr Tyr Cys Gln Gln Ser Asn
                85                  90                  95
Glu Asp Pro Pro Thr Phe Gly Gln Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys Glu Leu Gln Leu Glu Glu
    210                 215                 220
Ser Cys Ala Glu Ala Gln Asp Gly Glu Leu Asp Gly Gly Ser Gly Val
225                 230                 235                 240
Gln Leu Leu Glu Ser Gly Gly Gly Leu Val Gln Pro Gly Gly Ser Leu
                245                 250                 255
Arg Leu Ser Cys Ala Ala Ser Gly Phe Thr Phe Ala Trp Tyr Asp Met
            260                 265                 270
Gly Trp Val Arg Gln Ala Pro Gly Lys Gly Leu Glu Trp Val Ser Ser
        275                 280                 285
Ile Asp Trp His Gly Glu Val Thr Tyr Tyr Ala Asp Ser Val Lys Gly
    290                 295                 300
Arg Phe Thr Ile Ser Arg Asp Asn Ser Lys Asn Thr Leu Tyr Leu Gln
305                 310                 315                 320
Met Asn Ser Leu Arg Ala Glu Asp Thr Ala Val Tyr Tyr Cys Ala Thr
                325                 330                 335
Ala Glu Asp Glu Pro Gly Tyr Asp Tyr Trp Gly Gln Gly Thr Leu Val
            340                 345                 350
Thr Val Ser Ser

    355
```

<210> 60
<211> 324
<212> DNA
<213> Homo Sapiens

<400> 60

```
gacatccaga tgacccaatc accatcctcc ctgtctgcat ctgtaggaga ccgtgtcacc 60
atcacttgcc gggcaagtcg ccccattagc gactggttac attggtatca gcagaaacca 120
gggaaagccc ccaagctcct gatcgcctgg gcgtcctcgt tgtacgaggg ggtcccatca 180
cgtttcagtg gcagtgggtc ggggacagat ttcactctca ccatcagcag tctgcaaccc 240
gaagatttcg ctacgtacta ctgtttgcag gaggggtggg gtcctccgac gttcggccaa 300
gggaccaagg tggaaatcaa acgg                                        324
```

<210> 61
<211> 324
<212> DNA
<213> Homo Sapiens

<400> 61

```
gacatccaga tgacccaatc accatcctcc ctgtctgcat ctgtaggaga ccgtgtcacc 60
atcacttgcc gggcaagtcg ccccattagc gactggttac attggtatca gcagaaacca 120
gggaaagccc ccaagctcct gatcgcctgg gcgtccagct tgcagggggg ggtcccatca 180
cgtttcagtg gcagtgggtc ggggacagat ttcactctca ccatcagcag tctgcaaccc 240
gaagatttcg ctacgtacta ctgtttgcag gaggggtggg gtcctccgac gttcggccaa 300
gggaccaagg tggaaatcaa acgg                                        324
```

<210> 62
<211> 129
<212> PRT
<213> Homo Sapiens

<400> 62

```
His Lys Cys Asp Ile Thr Leu Gln Glu Ile Ile Lys Thr Leu Asn Ser
1               5                   10                  15
Leu Thr Glu Gln Lys Thr Leu Cys Thr Glu Leu Thr Val Thr Asp Ile
            20                  25                  30
Phe Ala Ala Ser Lys Asn Thr Thr Glu Lys Glu Thr Phe Cys Arg Ala
        35                  40                  45
Ala Thr Val Leu Arg Gln Phe Tyr Ser His His Glu Lys Asp Thr Arg
    50                  55                  60
Cys Leu Gly Ala Thr Ala Gln Gln Phe His Arg His Lys Gln Leu Ile
65                  70                  75                  80
Arg Phe Leu Lys Arg Leu Asp Arg Asn Leu Trp Gly Leu Ala Gly Leu
                85                  90                  95
Asn Ser Cys Pro Val Lys Glu Ala Asn Gln Ser Thr Leu Glu Asn Phe
            100                 105                 110
Leu Glu Arg Leu Lys Thr Ile Met Arg Glu Lys Tyr Ser Lys Cys Ser
        115                 120                 125
Ser
```

<210> 63
<211> 112
<212> PRT
<213> Homo Sapiens

<400> 63

Gly Pro Val Pro Pro Ser Thr Ala Leu Arg Glu Leu Ile Glu Glu Leu
1 5 10 15
Val Asn Ile Thr Gln Asn Gln Lys Ala Pro Leu Cys Asn Gly Ser Met
20 25 30
Val Trp Ser Ile Asn Leu Thr Ala Gly Met Tyr Cys Ala Ala Leu Glu
35 40 45
Ser Leu Ile Asn Val Ser Gly Cys Ser Ala Ile Glu Lys Thr Gln Arg
50 55 60
Met Leu Ser Gly Phe Cys Pro His Lys Val Ser Ala Gly Gln Phe Ser
65 70 75 80
Ser Leu His Val Arg Asp Thr Lys Ile Glu Val Ala Gln Phe Val Lys
85 90 95
Asp Leu Leu Leu His Leu Lys Lys Leu Phe Arg Glu Gly Arg Phe Asn
100 105 110

<210> 64
<211> 19
<212> PRT
<213> unknown

<220>
<223> Mammalian Signal Sequence

<400> 64

Met Gly Trp Ser Cys Ile Ile Leu Phe Leu Val Ala Thr Ala Thr Gly
1 5 10 15
Val His Ser

<210> 65
<211> 449
<212> PRT
<213> Artificial Sequence

<220>
<223> Humanised

<400> 65

Gln Val Thr Leu Arg Glu Ser Gly Pro Ala Leu Val Lys Pro Thr Gln
1 5 10 15
Thr Leu Thr Leu Thr Cys Thr Val Ser Gly Phe Ser Leu Thr Ser Tyr
20 25 30
Ser Val His Trp Val Arg Gln Pro Pro Gly Lys Gly Leu Glu Trp Leu
35 40 45
Gly Val Ile Trp Ala Ser Gly Gly Thr Asp Tyr Asn Ser Ala Leu Met
50 55 60
Ser Arg Leu Ser Ile Ser Lys Asp Thr Ser Arg Asn Gln Val Val Leu
65 70 75 80
Thr Met Thr Asn Met Asp Pro Val Asp Thr Ala Thr Tyr Tyr Cys Ala
85 90 95
Arg Asp Pro Pro Ser Ser Leu Leu Arg Leu Asp Tyr Trp Gly Arg Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys

<210> 66
<211> 220
<212> PRT
<213> Artificial Sequence

<220>

<223> Humanised

<400> 66

Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Gly Asn Gln Lys Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Asn
                85                  90                  95
Val His Ser Phe Pro Phe Thr Phe Gly Gly Gly Thr Lys Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr
            180                 185                 190
Glu Lys His Lys Val Tyr Ala Cys Glu Val Thr His Gln Gly Leu Ser
        195                 200                 205
Ser Pro Val Thr Lys Ser Phe Asn Arg Gly Glu Cys
    210                 215                 220

<210> 67
<211> 456
<212> PRT
<213> Artificial Sequence

<220>
<223> Humanised

<400> 67

Gln Val Gln Leu Val Gln Ser Gly Ala Glu Val Lys Lys Pro Gly Ala
1               5                   10                  15
Ser Val Lys Val Ser Cys Lys Val Ser Gly Glu Ile Ser Thr Gly Tyr
            20                  25                  30
Tyr Phe His Trp Val Arg Gln Ala Pro Gly Lys Gly Leu Glu Trp Met
        35                  40                  45
Gly Arg Ile Asp Pro Glu Asp Asp Ser Thr Lys Tyr Ala Glu Arg Phe
    50                  55                  60
Lys Asp Arg Val Thr Met Thr Glu Asp Thr Ser Thr Asp Thr Ala Tyr
65                  70                  75                  80
Met Glu Leu Ser Ser Leu Arg Ser Glu Asp Thr Ala Val Tyr Tyr Cys
                85                  90                  95
Thr Thr Trp Arg Ile Tyr Arg Asp Ser Ser Gly Arg Pro Phe Tyr Val
            100                 105                 110
Met Asp Ala Trp Gly Gln Gly Thr Leu Val Thr Val Ser Ser Ala Ser
        115                 120                 125
Thr Lys Gly Pro Ser Val Phe Pro Leu Ala Pro Ser Ser Lys Ser Thr
    130                 135                 140
Ser Gly Gly Thr Ala Ala Leu Gly Cys Leu Val Lys Asp Tyr Phe Pro

```
145                 150                 155                 160
Glu Pro Val Thr Val Ser Trp Asn Ser Gly Ala Leu Thr Ser Gly Val
                165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile
        195                 200                 205
Cys Asn Val Asn His Lys Pro Ser Asn Thr Lys Val Asp Lys Lys Val
    210                 215                 220
Glu Pro Lys Ser Cys Asp Lys Thr His Thr Cys Pro Pro Cys Pro Ala
225                 230                 235                 240
Pro Glu Leu Leu Gly Gly Pro Ser Val Phe Leu Phe Pro Pro Lys Pro
                245                 250                 255
Lys Asp Thr Leu Met Ile Ser Arg Thr Pro Glu Val Thr Cys Val Val
            260                 265                 270
Val Asp Val Ser His Glu Asp Pro Glu Val Lys Phe Asn Trp Tyr Val
        275                 280                 285
Asp Gly Val Glu Val His Asn Ala Lys Thr Lys Pro Arg Glu Glu Gln
    290                 295                 300
Tyr Asn Ser Thr Tyr Arg Val Val Ser Val Leu Thr Val Leu His Gln
305                 310                 315                 320
Asp Trp Leu Asn Gly Lys Glu Tyr Lys Cys Lys Val Ser Asn Lys Ala
                325                 330                 335
Leu Pro Ala Pro Ile Glu Lys Thr Ile Ser Lys Ala Lys Gly Gln Pro
            340                 345                 350
Arg Glu Pro Gln Val Tyr Thr Leu Pro Pro Ser Arg Asp Glu Leu Thr
        355                 360                 365
Lys Asn Gln Val Ser Leu Thr Cys Leu Val Lys Gly Phe Tyr Pro Ser
    370                 375                 380
Asp Ile Ala Val Glu Trp Glu Ser Asn Gly Gln Pro Glu Asn Asn Tyr
385                 390                 395                 400
Lys Thr Thr Pro Pro Val Leu Asp Ser Asp Gly Ser Phe Phe Leu Tyr
                405                 410                 415
Ser Lys Leu Thr Val Asp Lys Ser Arg Trp Gln Gln Gly Asn Val Phe
            420                 425                 430
Ser Cys Ser Val Met His Glu Ala Leu His Asn His Tyr Thr Gln Lys
        435                 440                 445
Ser Leu Ser Leu Ser Pro Gly Lys
    450                 455
```

<210> 68
<211> 214
<212> PRT
<213> Artificial Sequence

<220>
<223> Humanised

<400> 68

Asp Ile Gln Met Thr Gln Ser Pro Ser Ser Val Ser Ala Ser Val Gly
1               5                   10                  15
Asp Arg Val Thr Ile Thr Cys Leu Ala Ser Glu Asp Ile Tyr Thr Tyr
            20                  25                  30
Leu Thr Trp Tyr Gln Gln Lys Pro Gly Lys Ala Pro Lys Leu Leu Ile
        35                  40                  45
Tyr Gly Ala Asn Lys Leu Gln Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Leu Gln Gly Ser Lys Phe Pro Leu
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205

Phe Asn Arg Gly Glu Cys
    210

<210> 69
<211> 577
<212> PRT
<213> Artificial Sequence

<220>
<223> Humanised

<400> 69

Gln Val Gln Leu Val Gln Ser Gly Ala Glu Val Lys Lys Pro Gly Ala
1 5 10 15
Ser Val Lys Val Ser Cys Lys Val Ser Gly Glu Ile Ser Thr Gly Tyr
20 25 30
Tyr Phe His Trp Val Arg Gln Ala Pro Gly Lys Gly Leu Glu Trp Met
35 40 45
Gly Arg Ile Asp Pro Glu Asp Asp Ser Thr Lys Tyr Ala Glu Arg Phe
50 55 60
Lys Asp Arg Val Thr Met Thr Glu Asp Thr Ser Thr Asp Thr Ala Tyr
65 70 75 80
Met Glu Leu Ser Ser Leu Arg Ser Glu Asp Thr Ala Val Tyr Tyr Cys
85 90 95
Thr Thr Trp Arg Ile Tyr Arg Asp Ser Ser Gly Arg Pro Phe Tyr Val
100 105 110
Met Asp Ala Trp Gly Gln Gly Thr Leu Val Thr Val Ser Ser Ala Ser
115 120 125
Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro Gly Lys Gly Gly Gly Gly Ser Glu Val Gln
450 455 460
Leu Leu Glu Ser Gly Gly Gly Leu Val Gln Pro Gly Gly Ser Leu Arg
465 470 475 480
Leu Ser Cys Ala Ala Ser Gly Phe Thr Phe Arg Asn Phe Gly Met Gly
485 490 495
Trp Val Arg Gln Ala Pro Gly Lys Gly Leu Glu Trp Val Ser Trp Ile

```
                    500                     505                     510
Ile Ser Ser Gly Thr Glu Thr Tyr Tyr Ala Asp Ser Val Lys Gly Arg
                515                     520                     525
Phe Thr Ile Ser Arg Asp Asn Ser Lys Asn Thr Leu Tyr Leu Gln Met
    530                     535                     540
Asn Ser Leu Arg Ala Glu Asp Thr Ala Val Tyr Tyr Cys Ala Lys Ser
545                     550                     555                     560
Leu Gly Arg Phe Asp Tyr Trp Gly Gln Gly Thr Leu Val Thr Val Ser
                565                     570                     575
Ser
```

<210> 70
<211> 337
<212> PRT
<213> Artificial Sequence

<220>
<223> Humanised

<400> 70

```
Asp Ile Gln Met Thr Gln Ser Pro Ser Ser Val Ser Ala Ser Val Gly
1               5                   10                  15
Asp Arg Val Thr Ile Thr Cys Leu Ala Ser Glu Asp Ile Tyr Thr Tyr
            20                  25                  30
Leu Thr Trp Tyr Gln Gln Lys Pro Gly Lys Ala Pro Lys Leu Leu Ile
        35                  40                  45
Tyr Gly Ala Asn Lys Leu Gln Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Leu Gln Gly Ser Lys Phe Pro Leu
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Gly Gly Gly Ser Gly Val Gln Leu Leu
    210                 215                 220
Glu Ser Gly Gly Gly Leu Val Gln Pro Gly Gly Ser Leu Arg Leu Ser
225                 230                 235                 240
Cys Ala Ala Ser Gly Phe Thr Phe Ala Trp Tyr Asp Met Gly Trp Val
                245                 250                 255
Arg Gln Ala Pro Gly Lys Gly Leu Glu Trp Val Ser Ser Ile Asp Trp
            260                 265                 270
His Gly Glu Val Thr Tyr Tyr Ala Asp Ser Val Lys Gly Arg Phe Thr
        275                 280                 285
Ile Ser Arg Asp Asn Ser Lys Asn Thr Leu Tyr Leu Gln Met Asn Ser
    290                 295                 300
Leu Arg Ala Glu Asp Thr Ala Val Tyr Tyr Cys Ala Thr Ala Glu Asp
305                 310                 315                 320
Glu Pro Gly Tyr Asp Tyr Trp Gly Gln Gly Thr Leu Val Thr Val Ser
                325                 330                 335
Ser
```

<210> 71
<211> 570
<212> PRT
<213> Artificial Sequence

<220>
<223> Humanised

<400> 71

```
Gln Val Thr Leu Arg Glu Ser Gly Pro Ala Leu Val Lys Pro Thr Gln
1               5                   10                  15
Thr Leu Thr Leu Thr Cys Thr Val Ser Gly Phe Ser Leu Thr Ser Tyr
            20                  25                  30
Ser Val His Trp Val Arg Gln Pro Pro Gly Lys Gly Leu Glu Trp Leu
        35                  40                  45
Gly Val Ile Trp Ala Ser Gly Gly Thr Asp Tyr Asn Ser Ala Leu Met
    50                  55                  60
Ser Arg Leu Ser Ile Ser Lys Asp Thr Ser Arg Asn Gln Val Val Leu
65                  70                  75                  80
Thr Met Thr Asn Met Asp Pro Val Asp Thr Ala Thr Tyr Tyr Cys Ala
                85                  90                  95
Arg Asp Pro Pro Ser Ser Leu Leu Arg Leu Asp Tyr Trp Gly Arg Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Gly Gly Gly Gly Ser Glu Val Gln Leu Leu Glu Ser Gly Gly Gly
    450                 455                 460
Leu Val Gln Pro Gly Gly Ser Leu Arg Leu Ser Cys Ala Ala Ser Gly
465                 470                 475                 480
Phe Thr Phe Arg Asn Phe Gly Met Gly Trp Val Arg Gln Ala Pro Gly
                485                 490                 495
Lys Gly Leu Glu Trp Val Ser Trp Ile Ile Ser Ser Gly Thr Glu Thr
            500                 505                 510
Tyr Tyr Ala Asp Ser Val Lys Gly Arg Phe Thr Ile Ser Arg Asp Asn
        515                 520                 525
Ser Lys Asn Thr Leu Tyr Leu Gln Met Asn Ser Leu Arg Ala Glu Asp
    530                 535                 540
Thr Ala Val Tyr Tyr Cys Ala Lys Ser Leu Gly Arg Phe Asp Tyr Trp
545                 550                 555                 560
Gly Gln Gly Thr Leu Val Thr Val Ser Ser
                565                 570
```

<210> 72
<211> 343
<212> PRT
<213> Artificial Sequence

<220>
<223> Humanised

<400> 72

```
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Gly Asn Gln Lys Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Asn
                85                  90                  95
Val His Ser Phe Pro Phe Thr Phe Gly Gly Gly Thr Lys Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr
            180                 185                 190
Glu Lys His Lys Val Tyr Ala Cys Glu Val Thr His Gln Gly Leu Ser
        195                 200                 205
Ser Pro Val Thr Lys Ser Phe Asn Arg Gly Glu Cys Gly Gly Gly Gly
    210                 215                 220
Ser Gly Val Gln Leu Leu Glu Ser Gly Gly Gly Leu Val Gln Pro Gly
225                 230                 235                 240
Gly Ser Leu Arg Leu Ser Cys Ala Ala Ser Gly Phe Thr Phe Ala Trp
                245                 250                 255
Tyr Asp Met Gly Trp Val Arg Gln Ala Pro Gly Lys Gly Leu Glu Trp
            260                 265                 270
Val Ser Ser Ile Asp Trp His Gly Glu Val Thr Tyr Tyr Ala Asp Ser
        275                 280                 285
Val Lys Gly Arg Phe Thr Ile Ser Arg Asp Asn Ser Lys Asn Thr Leu
    290                 295                 300
Tyr Leu Gln Met Asn Ser Leu Arg Ala Glu Asp Thr Ala Val Tyr Tyr
305                 310                 315                 320
Cys Ala Thr Ala Glu Asp Glu Pro Gly Tyr Asp Tyr Trp Gly Gln Gly
                325                 330                 335
Thr Leu Val Thr Val Ser Ser
            340
```

<210> 73
<211> 214
<212> PRT
<213> Artificial Sequence

<220>
<223> Humanised

<400> 73

Asp Ile Gln Met Thr Gln Ser Pro Ser Ser Leu Ser Ala Ser Val Gly
1 5 10 15
Asp Arg Val Thr Ile Thr Cys Arg Ala Ser Gln Gly Ile Arg Asn Tyr
20 25 30
Leu Ala Trp Tyr Gln Gln Lys Pro Gly Lys Ala Pro Lys Leu Leu Ile
35 40 45
Tyr Ala Ala Ser Thr Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Val Ala Thr Tyr Tyr Cys Gln Arg Tyr Asn Arg Ala Pro Tyr
85 90 95
Thr Phe Gly Gln Gly Thr Lys Val Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 74
<211> 562
<212> PRT
<213> Artificial Sequence

<220>
<223> Humanised

<400> 74

Glu Val Gln Leu Val Glu Ser Gly Gly Gly Leu Val Gln Pro Gly Arg
1 5 10 15
Ser Leu Arg Leu Ser Cys Ala Ala Ser Gly Phe Thr Phe Asp Asp Tyr
20 25 30
Ala Met His Trp Val Arg Gln Ala Pro Gly Lys Gly Leu Glu Trp Val
35 40 45
Ser Ala Ile Thr Trp Asn Ser Gly His Ile Asp Tyr Ala Asp Ser Val
50 55 60
Glu Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Lys Val Ser Tyr Leu Ser Thr Ala Ser Ser Leu Asp Tyr Trp Gly
100 105 110
Gln Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400

```
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Ser Thr Gly Asp Ile Gln Met Thr Gln Ser Pro Ser Ser
    450                 455                 460
Leu Ser Ala Ser Val Gly Asp Arg Val Thr Ile Thr Cys Arg Ala Ser
465                 470                 475                 480
Gln Trp Ile Gly Asn Leu Leu Asp Trp Tyr Gln Gln Lys Pro Gly Lys
                485                 490                 495
Ala Pro Lys Leu Leu Ile Tyr Tyr Ala Ser Phe Leu Gln Ser Gly Val
            500                 505                 510
Pro Ser Arg Phe Ser Gly Ser Gly Tyr Gly Thr Asp Phe Thr Leu Thr
        515                 520                 525
Ile Ser Ser Leu Gln Pro Glu Asp Phe Ala Thr Tyr Tyr Cys Gln Gln
    530                 535                 540
Ala Asn Pro Ala Pro Leu Thr Phe Gly Gln Gly Thr Lys Val Glu Ile
545                 550                 555                 560
Lys Arg
```

<210> 75
<211> 570
<212> PRT
<213> Artificial Sequence

<220>
<223> Humanised

<400> 75

```
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
```

```
                340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Ser Thr Gly Glu Val Gln Leu Leu Val Ser Gly Gly Gly
    450                 455                 460
Leu Val Gln Pro Gly Gly Ser Leu Arg Leu Ser Cys Ala Ala Ser Gly
465                 470                 475                 480
Phe Thr Phe Lys Ala Tyr Pro Met Met Trp Val Arg Gln Ala Pro Gly
                485                 490                 495
Lys Gly Leu Glu Trp Val Ser Glu Ile Ser Pro Ser Gly Ser Tyr Thr
            500                 505                 510
Tyr Tyr Ala Asp Ser Val Lys Gly Arg Phe Thr Ile Ser Arg Asp Asn
        515                 520                 525
Ser Lys Asn Thr Leu Tyr Leu Gln Met Asn Ser Leu Arg Ala Glu Asp
    530                 535                 540
Thr Ala Val Tyr Tyr Cys Ala Lys Asp Pro Arg Lys Leu Asp Tyr Trp
545                 550                 555                 560
Gly Gln Gly Thr Leu Val Thr Val Ser Ser
                565                 570
```

<210> 76
<211> 569
<212> PRT
<213> Artificial Sequence

<220>
<223> Humanised

<400> 76

Glu Val Gln Leu Leu Val Ser Gly Gly Gly Leu Val Gln Pro Gly Gly
1               5                   10                  15
Ser Leu Arg Leu Ser Cys Ala Ala Ser Gly Phe Thr Phe Lys Ala Tyr
            20                  25                  30
Pro Met Met Trp Val Arg Gln Ala Pro Gly Lys Gly Leu Glu Trp Val
        35                  40                  45
Ser Glu Ile Ser Pro Ser Gly Ser Tyr Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Pro Arg Lys Leu Asp Tyr Trp Gly Gln Gly Thr Leu Val
            100                 105                 110
Thr Val Ser Ser Ala Ser Thr Lys Gly Pro Ser Glu Val Gln Leu Leu
        115                 120                 125
Glu Ser Gly Gly Gly Leu Val Gln Pro Gly Gly Ser Leu Arg Leu Ser
    130                 135                 140
Cys Ala Ala Ser Gly Phe Thr Phe Ser Ser Tyr Ala Met Ser Trp Val
145                 150                 155                 160
Arg Gln Ala Pro Gly Lys Gly Leu Glu Trp Val Ser Ala Ile Ser Gly
                165                 170                 175
Ser Gly Gly Ser Thr Tyr Tyr Ala Asp Ser Val Lys Gly Arg Phe Thr
            180                 185                 190
Ile Ser Arg Asp Asn Ser Lys Asn Thr Leu Tyr Leu Gln Met Asn Ser
        195                 200                 205
Leu Arg Ala Glu Asp Thr Ala Val Tyr Tyr Cys Ala Lys Ser Tyr Gly
    210                 215                 220
Ala Phe Asp Tyr Trp Gly Gln Gly Thr Leu Val Thr Val Ser Ser Ala
225                 230                 235                 240
Ser Thr Lys Gly Pro Ser Val Phe Pro Leu Ala Pro Ser Ser Lys Ser
                245                 250                 255
Thr Ser Gly Gly Thr Ala Ala Leu Gly Cys Leu Val Lys Asp Tyr Phe
            260                 265                 270
Pro Glu Pro Val Thr Val Ser Trp Asn Ser Gly Ala Leu Thr Ser Gly
        275                 280                 285

Val His Thr Phe Pro Ala Val Leu Gln Ser Ser Gly Leu Tyr Ser Leu
290 295 300
Ser Ser Val Val Thr Val Pro Ser Ser Ser Leu Gly Thr Gln Thr Tyr
305 310 315 320
Ile Cys Asn Val Asn His Lys Pro Ser Asn Thr Lys Val Asp Lys Lys
325 330 335
Val Glu Pro Lys Ser Cys Asp Lys Thr His Thr Cys Pro Pro Cys Pro
340 345 350
Ala Pro Glu Leu Leu Gly Gly Pro Ser Val Phe Leu Phe Pro Pro Lys
355 360 365
Pro Lys Asp Thr Leu Met Ile Ser Arg Thr Pro Glu Val Thr Cys Val
370 375 380
Val Val Asp Val Ser His Glu Asp Pro Glu Val Lys Phe Asn Trp Tyr
385 390 395 400
Val Asp Gly Val Glu Val His Asn Ala Lys Thr Lys Pro Arg Glu Glu
405 410 415
Gln Tyr Asn Ser Thr Tyr Arg Val Val Ser Val Leu Thr Val Leu His
420 425 430
Gln Asp Trp Leu Asn Gly Lys Glu Tyr Lys Cys Lys Val Ser Asn Lys
435 440 445
Ala Leu Pro Ala Pro Ile Glu Lys Thr Ile Ser Lys Ala Lys Gly Gln
450 455 460
Pro Arg Glu Pro Gln Val Tyr Thr Leu Pro Pro Ser Arg Asp Glu Leu
465 470 475 480
Thr Lys Asn Gln Val Ser Leu Thr Cys Leu Val Lys Gly Phe Tyr Pro
485 490 495
Ser Asp Ile Ala Val Glu Trp Glu Ser Asn Gly Gln Pro Glu Asn Asn
500 505 510
Tyr Lys Thr Thr Pro Pro Val Leu Asp Ser Asp Gly Ser Phe Phe Leu
515 520 525
Tyr Ser Lys Leu Thr Val Asp Lys Ser Arg Trp Gln Gln Gly Asn Val
530 535 540
Phe Ser Cys Ser Val Met His Glu Ala Leu His Asn His Tyr Thr Gln
545 550 555 560
Lys Ser Leu Ser Leu Ser Pro Gly Lys
565

<210> 77
<211> 328
<212> PRT
<213> Artificial Sequence

<220>
<223> Humanised

<400> 77

Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Tyr Leu Asn
            20                  25                  30
Leu Asp Trp Tyr Gln Gln Lys Pro Gly Lys Ala Pro Lys Leu Leu Ile
        35                  40                  45
Asn Phe Gly Ser Glu Leu Gln Ser Gly Val Pro Ser Arg Phe Ser Gly
    50                  55                  60
Ser Gly Tyr Gly Thr Asp Phe Thr Leu Thr Ile Ser Ser Leu Gln Pro
65                  70                  75                  80
Glu Asp Phe Ala Thr Tyr Tyr Cys Gln Pro Ser Phe Tyr Phe Pro Tyr
                85                  90                  95
Thr Phe Gly Gln Gly Thr Lys Val Glu Ile Lys Arg Thr Val Ala Ala
            100                 105                 110
Pro Ser Asp Ile Gln Met Thr Gln Ser Pro Ser Ser Leu Ser Ala Ser
        115                 120                 125
Val Gly Asp Arg Val Thr Ile Thr Cys Arg Ala Ser Gln Ser Ile Ser
    130                 135                 140
Ser Tyr Leu Asn Trp Tyr Gln Gln Lys Pro Gly Lys Ala Pro Lys Leu
145                 150                 155                 160
Leu Ile Tyr Ala Ala Ser Ser Leu Gln Ser Gly Val Pro Ser Arg Phe
                165                 170                 175
Ser Gly Ser Gly Ser Gly Thr Asp Phe Thr Leu Thr Ile Ser Ser Leu
            180                 185                 190
Gln Pro Glu Asp Phe Ala Thr Tyr Tyr Cys Gln Gln Ser Tyr Ser Thr
        195                 200                 205
Pro Asn Thr Phe Gly Gln Gly Thr Lys Val Glu Ile Lys Arg Thr Val
    210                 215                 220
Ala Ala Pro Ser Val Phe Ile Phe Pro Pro Ser Asp Glu Gln Leu Lys
225                 230                 235                 240
Ser Gly Thr Ala Ser Val Val Cys Leu Leu Asn Asn Phe Tyr Pro Arg
                245                 250                 255
Glu Ala Lys Val Gln Trp Lys Val Asp Asn Ala Leu Gln Ser Gly Asn
            260                 265                 270
Ser Gln Glu Ser Val Thr Glu Gln Asp Ser Lys Asp Ser Thr Tyr Ser
        275                 280                 285
Leu Ser Ser Thr Leu Thr Leu Ser Lys Ala Asp Tyr Glu Lys His Lys
    290                 295                 300
Val Tyr Ala Cys Glu Val Thr His Gln Gly Leu Ser Ser Pro Val Thr
305                 310                 315                 320
Lys Ser Phe Asn Arg Gly Glu Cys
                325

<210> 78
<211> 574
<212> PRT
<213> Artificial Sequence

<220>
<223> Humanised

<400> 78

```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Gly Ala Tyr
            20                  25                  30
Pro Met Met Trp Val Arg Gln Ala Pro Gly Lys Gly Leu Glu Trp Val
        35                  40                  45
Ser Glu Ile Ser Pro Ser Gly Ser Tyr Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Pro Arg Lys Phe Asp Tyr Trp Gly Gln Gly Thr Leu Val
            100                 105                 110
Thr Val Ser Ser Ala Ser Thr Lys Gly Pro Ser Glu Val Gln Leu Val
        115                 120                 125
Glu Ser Gly Gly Gly Leu Val Gln Pro Gly Arg Ser Leu Arg Leu Ser
    130                 135                 140
Cys Ala Ala Ser Gly Phe Thr Phe Asp Asp Tyr Ala Met His Trp Val
145                 150                 155                 160
Arg Gln Ala Pro Gly Lys Gly Leu Glu Trp Val Ser Ala Ile Thr Trp
                165                 170                 175
Asn Ser Gly His Ile Asp Tyr Ala Asp Ser Val Glu Gly Arg Phe Thr
            180                 185                 190
Ile Ser Arg Asp Asn Ala Lys Asn Ser Leu Tyr Leu Gln Met Asn Ser
        195                 200                 205
Leu Arg Ala Glu Asp Thr Ala Val Tyr Tyr Cys Ala Lys Val Ser Tyr
    210                 215                 220
Leu Ser Thr Ala Ser Ser Leu Asp Tyr Trp Gly Gln Gly Thr Leu Val
225                 230                 235                 240
Thr Val Ser Ser Ala Ser Thr Lys Gly Pro Ser Val Phe Pro Leu Ala
                245                 250                 255
Pro Ser Ser Lys Ser Thr Ser Gly Gly Thr Ala Ala Leu Gly Cys Leu
            260                 265                 270
Val Lys Asp Tyr Phe Pro Glu Pro Val Thr Val Ser Trp Asn Ser Gly
        275                 280                 285
Ala Leu Thr Ser Gly Val His Thr Phe Pro Ala Val Leu Gln Ser Ser
    290                 295                 300
Gly Leu Tyr Ser Leu Ser Ser Val Val Thr Val Pro Ser Ser Ser Leu
305                 310                 315                 320
Gly Thr Gln Thr Tyr Ile Cys Asn Val Asn His Lys Pro Ser Asn Thr
                325                 330                 335
Lys Val Asp Lys Lys Val Glu Pro Lys Ser Cys Asp Lys Thr His Thr
            340                 345                 350
Cys Pro Pro Cys Pro Ala Pro Glu Leu Leu Gly Gly Pro Ser Val Phe
        355                 360                 365
Leu Phe Pro Pro Lys Pro Lys Asp Thr Leu Met Ile Ser Arg Thr Pro
    370                 375                 380
Glu Val Thr Cys Val Val Val Asp Val Ser His Glu Asp Pro Glu Val
385                 390                 395                 400
Lys Phe Asn Trp Tyr Val Asp Gly Val Glu Val His Asn Ala Lys Thr
                405                 410                 415
```

Lys Pro Arg Glu Glu Gln Tyr Asn Ser Thr Tyr Arg Val Val Ser Val
420 425 430
Leu Thr Val Leu His Gln Asp Trp Leu Asn Gly Lys Glu Tyr Lys Cys
435 440 445
Lys Val Ser Asn Lys Ala Leu Pro Ala Pro Ile Glu Lys Thr Ile Ser
450 455 460
Lys Ala Lys Gly Gln Pro Arg Glu Pro Gln Val Tyr Thr Leu Pro Pro
465 470 475 480
Ser Arg Asp Glu Leu Thr Lys Asn Gln Val Ser Leu Thr Cys Leu Val
485 490 495
Lys Gly Phe Tyr Pro Ser Asp Ile Ala Val Glu Trp Glu Ser Asn Gly
500 505 510
Gln Pro Glu Asn Asn Tyr Lys Thr Thr Pro Pro Val Leu Asp Ser Asp
515 520 525
Gly Ser Phe Phe Leu Tyr Ser Lys Leu Thr Val Asp Lys Ser Arg Trp
530 535 540
Gln Gln Gly Asn Val Phe Ser Cys Ser Val Met His Glu Ala Leu His
545 550 555 560
Asn His Tyr Thr Gln Lys Ser Leu Ser Leu Ser Pro Gly Lys
565 570

<210> 79
<211> 328
<212> PRT
<213> Artificial Sequence

<220>
<223> Humanised

<400> 79

Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Trp Ile Gly Asn Leu
            20                  25                  30
Leu Asp Trp Tyr Gln Gln Lys Pro Gly Lys Ala Pro Lys Leu Leu Ile
        35                  40                  45
Tyr Tyr Ala Ser Phe Leu Gln Ser Gly Val Pro Ser Arg Phe Ser Gly
    50                  55                  60
Ser Gly Tyr Gly Thr Asp Phe Thr Leu Thr Ile Ser Ser Leu Gln Pro
65                  70                  75                  80
Glu Asp Phe Ala Thr Tyr Tyr Cys Gln Gln Ala Asn Pro Ala Pro Leu
                85                  90                  95
Thr Phe Gly Gln Gly Thr Lys Val Glu Ile Lys Arg Thr Val Ala Ala
            100                 105                 110
Pro Ser Asp Ile Gln Met Thr Gln Ser Pro Ser Ser Leu Ser Ala Ser
        115                 120                 125
Val Gly Asp Arg Val Thr Ile Thr Cys Arg Ala Ser Gln Gly Ile Arg
    130                 135                 140
Asn Tyr Leu Ala Trp Tyr Gln Gln Lys Pro Gly Lys Ala Pro Lys Leu
145                 150                 155                 160
Leu Ile Tyr Ala Ala Ser Thr Leu Gln Ser Gly Val Pro Ser Arg Phe
                165                 170                 175
Ser Gly Ser Gly Ser Gly Thr Asp Phe Thr Leu Thr Ile Ser Ser Leu
            180                 185                 190
Gln Pro Glu Asp Val Ala Thr Tyr Tyr Cys Gln Arg Tyr Asn Arg Ala
        195                 200                 205
Pro Tyr Thr Phe Gly Gln Gly Thr Lys Val Glu Ile Lys Arg Thr Val
    210                 215                 220
Ala Ala Pro Ser Val Phe Ile Phe Pro Pro Ser Asp Glu Gln Leu Lys
225                 230                 235                 240
Ser Gly Thr Ala Ser Val Val Cys Leu Leu Asn Asn Phe Tyr Pro Arg
                245                 250                 255
Glu Ala Lys Val Gln Trp Lys Val Asp Asn Ala Leu Gln Ser Gly Asn
            260                 265                 270
Ser Gln Glu Ser Val Thr Glu Gln Asp Ser Lys Asp Ser Thr Tyr Ser
        275                 280                 285
Leu Ser Ser Thr Leu Thr Leu Ser Lys Ala Asp Tyr Glu Lys His Lys
    290                 295                 300
Val Tyr Ala Cys Glu Val Thr His Gln Gly Leu Ser Ser Pro Val Thr
305                 310                 315                 320
Lys Ser Phe Asn Arg Gly Glu Cys
                325

<210> 80
<211> 14
<212> PRT
<213> Mus Musculus

<400> 80

Trp Ile Leu Tyr Tyr Gly Arg Ser Lys Trp Tyr Phe Asp Val
1               5                   10

<210> 81
<211> 17
<212> PRT
<213> Mus Musculus

<400> 81

Asn Ile Asn Pro Asn Asn Gly Gly Thr Asn Tyr Asn Gln Lys Phe Lys
1 5 10 15
Asp

<210> 82
<211> 5
<212> PRT
<213> Mus Musculus

<400> 82

Asp Tyr Tyr Met Asn
1 5

<210> 83
<211> 16
<212> PRT
<213> Mus Musculus

<400> 83

Arg Ser Ser Gln Ser Ile Val Gln Ser Asn Gly Asp Thr Tyr Leu Glu
1 5 10 15

<210> 84
<211> 7
<212> PRT
<213> Mus Musculus

<400> 84

Arg Ile Ser Asn Arg Phe Ser
1 5

<210> 85
<211> 9
<212> PRT
<213> Mus Musculus

<400> 85

Phe Gln Gly Ser His Val Pro Tyr Thr
1 5

<210> 86
<211> 7
<212> PRT
<213> Mus Musculus

<400> 86

Arg Val Ser Asn Arg Phe Ser
1 5

<210> 87
<211> 571
<212> PRT
<213> Artificial Sequence

<220>
<223> Humanised

<400> 87

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220
Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Glu Val Gln Leu Leu Glu Ser Gly Gly
450 455 460
Gly Leu Val Gln Pro Gly Gly Ser Leu Arg Leu Ser Cys Ala Ala Ser
465 470 475 480
Gly Phe Thr Phe Arg Asn Phe Gly Met Gly Trp Val Arg Gln Ala Pro
485 490 495
Gly Lys Gly Leu Glu Trp Val Ser Trp Ile Ile Ser Ser Gly Thr Glu
500 505 510
Thr Tyr Tyr Ala Asp Ser Val Lys Gly Arg Phe Thr Ile Ser Arg Asp
515 520 525
Asn Ser Lys Asn Thr Leu Tyr Leu Gln Met Asn Ser Leu Arg Ala Glu
530 535 540
Asp Thr Ala Val Tyr Tyr Cys Ala Lys Ser Leu Gly Arg Phe Asp Tyr
545 550 555 560
Trp Gly Gln Gly Thr Leu Val Thr Val Ser Ser
565 570

<210> 88
<211> 577
<212> PRT
<213> Artificial Sequence

<220>
<223> Humanised

<400> 88

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Thr Val Ala Ala Pro Ser Glu Val Gln
    450                 455                 460
Leu Leu Glu Ser Gly Gly Gly Leu Val Gln Pro Gly Gly Ser Leu Arg
465                 470                 475                 480
Leu Ser Cys Ala Ala Ser Gly Phe Thr Phe Arg Asn Phe Gly Met Gly
                485                 490                 495
Trp Val Arg Gln Ala Pro Gly Lys Gly Leu Glu Trp Val Ser Trp Ile
            500                 505                 510
Ile Ser Ser Gly Thr Glu Thr Tyr Tyr Ala Asp Ser Val Lys Gly Arg
```

```
        515                     520                     525
Phe Thr Ile Ser Arg Asp Asn Ser Lys Asn Thr Leu Tyr Leu Gln Met
    530                     535                     540
Asn Ser Leu Arg Ala Glu Asp Thr Ala Val Tyr Tyr Cys Ala Lys Ser
545                     550                     555                     560
Leu Gly Arg Phe Asp Tyr Trp Gly Gln Gly Thr Leu Val Thr Val Ser
                565                     570                     575
Ser
```

<210> 89
<211> 578
<212> PRT
<213> Artificial Sequence

<220>
<223> Humanised

<400> 89

Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445

```
Leu Ser Leu Ser Pro Gly Lys Ala Ser Thr Lys Gly Pro Thr Glu Val
    450                 455                 460
Gln Leu Leu Glu Ser Gly Gly Gly Leu Val Gln Pro Gly Gly Ser Leu
465                 470                 475                 480
Arg Leu Ser Cys Ala Ala Ser Gly Phe Thr Phe Arg Asn Phe Gly Met
                485                 490                 495
Gly Trp Val Arg Gln Ala Pro Gly Lys Gly Leu Glu Trp Val Ser Trp
            500                 505                 510
Ile Ile Ser Ser Gly Thr Glu Thr Tyr Tyr Ala Asp Ser Val Lys Gly
        515                 520                 525
Arg Phe Thr Ile Ser Arg Asp Asn Ser Lys Asn Thr Leu Tyr Leu Gln
    530                 535                 540
Met Asn Ser Leu Arg Ala Glu Asp Thr Ala Val Tyr Tyr Cys Ala Lys
545                 550                 555                 560
Ser Leu Gly Arg Phe Asp Tyr Trp Gly Gln Gly Thr Leu Val Thr Val
                565                 570                 575
Ser Ser
```

<210> 90
<211> 578
<212> PRT
<213> Artificial Sequence

<220>
<223> Humanised

<400> 90

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
```

```
        370                     375                     380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430
Cys Ser Val Met His Glu Ala Leu His Asn His Tyr Thr Gln Lys Ser
        435                 440                 445
Leu Ser Leu Ser Pro Gly Lys Ala Ser Thr Lys Gly Pro Ser Glu Val
    450                 455                 460
Gln Leu Leu Glu Ser Gly Gly Gly Leu Val Gln Pro Gly Gly Ser Leu
465                 470                 475                 480
Arg Leu Ser Cys Ala Ala Ser Gly Phe Thr Phe Arg Asn Phe Gly Met
                485                 490                 495
Gly Trp Val Arg Gln Ala Pro Gly Lys Gly Leu Glu Trp Val Ser Trp
            500                 505                 510
Ile Ile Ser Ser Gly Thr Glu Thr Tyr Tyr Ala Asp Ser Val Lys Gly
        515                 520                 525
Arg Phe Thr Ile Ser Arg Asp Asn Ser Lys Asn Thr Leu Tyr Leu Gln
    530                 535                 540
Met Asn Ser Leu Arg Ala Glu Asp Thr Ala Val Tyr Tyr Cys Ala Lys
545                 550                 555                 560
Ser Leu Gly Arg Phe Asp Tyr Trp Gly Gln Gly Thr Leu Val Thr Val
                565                 570                 575
Ser Ser
```

<210> 91
<211> 569
<212> PRT
<213> Artificial Sequence

<220>
<223> Humanised

<400> 91

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Val Gln Leu Leu Glu Ser Gly Gly Gly Leu Val Gln
450 455 460
Pro Gly Gly Ser Leu Arg Leu Ser Cys Ala Ala Ser Gly Phe Thr Phe
465 470 475 480
Ala Trp Tyr Asp Met Gly Trp Val Arg Gln Ala Pro Gly Lys Gly Leu
485 490 495
Glu Trp Val Ser Ser Ile Asp Trp His Gly Glu Val Thr Tyr Tyr Ala
500 505 510
Asp Ser Val Lys Gly Arg Phe Thr Ile Ser Arg Asp Asn Ser Lys Asn
515 520 525
Thr Leu Tyr Leu Gln Met Asn Ser Leu Arg Ala Glu Asp Thr Ala Val
530 535 540
Tyr Tyr Cys Ala Thr Ala Glu Asp Glu Pro Gly Tyr Asp Tyr Trp Gly
545 550 555 560
Gln Gly Thr Leu Val Thr Val Ser Ser
565

<210> 92
<211> 575
<212> PRT
<213> Artificial Sequence

<220>
<223> Humanised

<400> 92

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
```

```
                    245                     250                     255
Ile Ser Arg Thr Pro Glu Val Thr Cys Val Val Val Asp Val Ser His
            260                     265                     270
Glu Asp Pro Glu Val Lys Phe Asn Trp Tyr Val Asp Gly Val Glu Val
        275                     280                     285
His Asn Ala Lys Thr Lys Pro Arg Glu Glu Gln Tyr Asn Ser Thr Tyr
    290                     295                     300
Arg Val Val Ser Val Leu Thr Val Leu His Gln Asp Trp Leu Asn Gly
305                     310                     315                     320
Lys Glu Tyr Lys Cys Lys Val Ser Asn Lys Ala Leu Pro Ala Pro Ile
                325                     330                     335
Glu Lys Thr Ile Ser Lys Ala Lys Gly Gln Pro Arg Glu Pro Gln Val
            340                     345                     350
Tyr Thr Leu Pro Pro Ser Arg Asp Glu Leu Thr Lys Asn Gln Val Ser
        355                     360                     365
Leu Thr Cys Leu Val Lys Gly Phe Tyr Pro Ser Asp Ile Ala Val Glu
    370                     375                     380
Trp Glu Ser Asn Gly Gln Pro Glu Asn Asn Tyr Lys Thr Thr Pro Pro
385                     390                     395                     400
Val Leu Asp Ser Asp Gly Ser Phe Phe Leu Tyr Ser Lys Leu Thr Val
                405                     410                     415
Asp Lys Ser Arg Trp Gln Gln Gly Asn Val Phe Ser Cys Ser Val Met
            420                     425                     430
His Glu Ala Leu His Asn His Tyr Thr Gln Lys Ser Leu Ser Leu Ser
        435                     440                     445
Pro Gly Lys Thr Val Ala Ala Pro Ser Gly Val Gln Leu Leu Glu Ser
    450                     455                     460
Gly Gly Gly Leu Val Gln Pro Gly Gly Ser Leu Arg Leu Ser Cys Ala
465                     470                     475                     480
Ala Ser Gly Phe Thr Phe Ala Trp Tyr Asp Met Gly Trp Val Arg Gln
                485                     490                     495
Ala Pro Gly Lys Gly Leu Glu Trp Val Ser Ser Ile Asp Trp His Gly
            500                     505                     510
Glu Val Thr Tyr Tyr Ala Asp Ser Val Lys Gly Arg Phe Thr Ile Ser
        515                     520                     525
Arg Asp Asn Ser Lys Asn Thr Leu Tyr Leu Gln Met Asn Ser Leu Arg
    530                     535                     540
Ala Glu Asp Thr Ala Val Tyr Tyr Cys Ala Thr Ala Glu Asp Glu Pro
545                     550                     555                     560
Gly Tyr Asp Tyr Trp Gly Gln Gly Thr Leu Val Thr Val Ser Ser
                565                     570                     575
```

<210> 93
<211> 576
<212> PRT
<213> Artificial Sequence

<220>
<223> Humanised

<400> 93

Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Ala Ser Thr Lys Gly Pro Thr Gly Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Ala Trp Tyr Asp Met Gly Trp Val Arg
485 490 495
Gln Ala Pro Gly Lys Gly Leu Glu Trp Val Ser Ser Ile Asp Trp His
500 505 510
Gly Glu Val Thr Tyr Tyr Ala Asp Ser Val Lys Gly Arg Phe Thr Ile
515 520 525
Ser Arg Asp Asn Ser Lys Asn Thr Leu Tyr Leu Gln Met Asn Ser Leu
530 535 540
Arg Ala Glu Asp Thr Ala Val Tyr Tyr Cys Ala Thr Ala Glu Asp Glu
545 550 555 560
Pro Gly Tyr Asp Tyr Trp Gly Gln Gly Thr Leu Val Thr Val Ser Ser
565 570 575

<210> 94
<211> 576
<212> PRT
<213> Artificial Sequence

<220>
<223> Humanised

<400> 94

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
```

130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Ala Ser Thr Lys Gly Pro Ser Gly Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Ala Trp Tyr Asp Met Gly Trp Val Arg
485 490 495
Gln Ala Pro Gly Lys Gly Leu Glu Trp Val Ser Ser Ile Asp Trp His
500 505 510
Gly Glu Val Thr Tyr Tyr Ala Asp Ser Val Lys Gly Arg Phe Thr Ile
515 520 525
Ser Arg Asp Asn Ser Lys Asn Thr Leu Tyr Leu Gln Met Asn Ser Leu
530 535 540
Arg Ala Glu Asp Thr Ala Val Tyr Tyr Cys Ala Thr Ala Glu Asp Glu
545 550 555 560
Pro Gly Tyr Asp Tyr Trp Gly Gln Gly Thr Leu Val Thr Val Ser Ser
565 570 575

<210> 95
<211> 578
<212> PRT
<213> Artificial Sequence

<220>
<223> Humanised

<400> 95

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80

```
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Ala Ser Thr Lys Gly Pro Ser Gly Val Gln Leu
    450                 455                 460
Leu Glu Ser Gly Gly Gly Leu Val Gln Pro Gly Gly Ser Leu Arg Leu
465                 470                 475                 480
Ser Cys Ala Ala Ser Gly Phe Thr Phe Ala Trp Tyr Asp Met Gly Trp
                485                 490                 495
Val Arg Gln Ala Pro Gly Lys Gly Leu Glu Trp Val Ser Ser Ile Asp
            500                 505                 510
Trp His Gly Glu Val Thr Tyr Tyr Ala Asp Ser Val Lys Gly Arg Phe
        515                 520                 525
Thr Ile Ser Arg Asp Asn Ser Lys Asn Thr Leu Tyr Leu Gln Met Asn
    530                 535                 540
Ser Leu Arg Ala Glu Asp Thr Ala Val Tyr Tyr Cys Ala Thr Ala Glu
545                 550                 555                 560
Asp Glu Pro Gly Tyr Asp Tyr Trp Gly Gln Gly Thr Leu Val Thr Val
                565                 570                 575
Ser Ser
```

<210> 96
<211> 578

<212> PRT
<213> Artificial Sequence

<220>
<223> Humanised

<400> 96

Gln Val Thr Leu Arg Glu Ser Gly Pro Ala Leu Val Lys Pro Thr Gln

```
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Ala Ser Thr Lys Gly Pro Thr Gly Val Gln Leu
    450                 455                 460
Leu Glu Ser Gly Gly Gly Leu Val Gln Pro Gly Gly Ser Leu Arg Leu
465                 470                 475                 480
Ser Cys Ala Ala Ser Gly Phe Thr Phe Ala Trp Tyr Asp Met Gly Trp
                485                 490                 495
Val Arg Gln Ala Pro Gly Lys Gly Leu Glu Trp Val Ser Ser Ile Asp
            500                 505                 510
Trp His Gly Glu Val Thr Tyr Tyr Ala Asp Ser Val Lys Gly Arg Phe
        515                 520                 525
Thr Ile Ser Arg Asp Asn Ser Lys Asn Thr Leu Tyr Leu Gln Met Asn
    530                 535                 540
Ser Leu Arg Ala Glu Asp Thr Ala Val Tyr Tyr Cys Ala Thr Ala Glu
545                 550                 555                 560
Asp Glu Pro Gly Tyr Asp Tyr Trp Gly Gln Gly Thr Leu Val Thr Val
                565                 570                 575
Ser Ser
```

<210> 97
<211> 11
<212> PRT
<213> Mus Musculus

<400> 97

```
Gly Tyr Ser Ile Thr Ser Asp Phe Ala Trp Asn
 1               5                   10
```

<210> 98
<211> 14
<212> PRT
<213> Mus Musculus

<400> 98

```
Gly Tyr Ile Ser Tyr Ser Gly Asn Tyr Asn Pro Ser Leu Lys
 1               5                   10
```

<210> 99
<211> 9
<212> PRT
<213> Mus Musculus

<400> 99

```
Val Thr Ala Gly Arg Gly Phe Pro Tyr
 1               5
```

<210> 100
<211> 11
<212> PRT
<213> Mus Musculus

<400> 100

```
His Ser Ser Gln Asp Ile Asn Ser Asn Ile Gly
 1               5                   10
```

<210> 101
<211> 7
<212> PRT
<213> Mus Musculus

<400> 101

```
His Gly Ile Asn Leu Asp Asp
 1               5
```

<210> 102

<211> 9
<212> PRT
<213> Mus Musculus

<400> 102

Val Gln Tyr Ala Gln Phe Pro Trp Thr
1 5

<210> 103
<211> 16
<212> PRT
<213> Homo Sapiens

<400> 103

Cys Gly Ala Asp Ser Tyr Glu Met Glu Glu Asp Gly Val Arg Lys Cys
1 5 10 15

<210> 104
<211> 6
<212> PRT
<213> Mus Musculus

<400> 104

Ser Asp Phe Ala Trp Asn

1 5

<210> 105
<211> 13
<212> PRT
<213> Mus Musculus

<400> 105

Tyr Ile Ser Tyr Ser Gly Asn Tyr Asn Pro Ser Leu Lys
1 5 10

<210> 106
<211> 7
<212> PRT
<213> Mus Musculus

<400> 106

Ala Gly Arg Gly Phe Pro Tyr
1 5

<210> 107

<211> 14
<212> PRT
<213> Mus Musculus

<400> 107

Tyr Ile Ser Tyr Ser Gly Asn Tyr Asn Pro Ser Leu Lys Ser
1 5 10

<210> 108
<211> 577
<212> PRT
<213> Artificial Sequence

<220>
<223> Humanised

<400> 108

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Asp Tyr
20 25 30
Tyr Met Asn Trp Val Arg Gln Ala Pro Gly Gln Gly Leu Glu Trp Met
35 40 45
Gly Asn Ile Asn Pro Asn Asn Gly Gly Thr Asn Tyr Asn Gln Lys Phe
50 55 60
Lys Asp Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Trp Ile Leu Tyr Tyr Gly Arg Ser Lys Trp Tyr Phe Asp Val
100 105 110
Trp Gly Arg Gly Thr Leu Val Thr Val Ser Ser Ala Ser Thr Lys Gly
115 120 125
Pro Ser Val Phe Pro Leu Ala Pro Ser Ser Lys Ser Thr Ser Gly Gly
130 135 140
Thr Ala Ala Leu Gly Cys Leu Val Lys Asp Tyr Phe Pro Glu Pro Val
145 150 155 160
Thr Val Ser Trp Asn Ser Gly Ala Leu Thr Ser Gly Val His Thr Phe
165 170 175
Pro Ala Val Leu Gln Ser Ser Gly Leu Tyr Ser Leu Ser Ser Val Val
180 185 190
Thr Val Pro Ser Ser Ser Leu Gly Thr Gln Thr Tyr Ile Cys Asn Val
195 200 205
Asn His Lys Pro Ser Asn Thr Lys Val Asp Lys Lys Val Glu Pro Lys
210 215 220
Ser Cys Asp Lys Thr His Thr Cys Pro Pro Cys Pro Ala Pro Glu Leu
225 230 235 240
Leu Gly Gly Pro Ser Val Phe Leu Phe Pro Pro Lys Pro Lys Asp Thr
245 250 255
Leu Met Ile Ser Arg Thr Pro Glu Val Thr Cys Val Val Val Asp Val
260 265 270

```
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                     320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                     400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Thr Val Ala Ala Pro Ser Gly Ser Glu Val Gln
    450                 455                 460
Leu Leu Val Ser Gly Gly Gly Leu Val Gln Pro Gly Gly Ser Leu Arg
465                 470                 475                     480
Leu Ser Cys Ala Ala Ser Gly Phe Thr Phe Lys Ala Tyr Pro Met Met
                485                 490                 495
Trp Val Arg Gln Ala Pro Gly Lys Gly Leu Glu Trp Val Ser Glu Ile
            500                 505                 510
Ser Pro Ser Gly Ser Tyr Thr Tyr Tyr Ala Asp Ser Val Lys Gly Arg
        515                 520                 525
Phe Thr Ile Ser Arg Asp Asn Ser Lys Asn Thr Leu Tyr Leu Gln Met
    530                 535                 540
Asn Ser Leu Arg Ala Glu Asp Thr Ala Val Tyr Tyr Cys Ala Lys Asp
545                 550                 555                     560
Pro Arg Lys Leu Asp Tyr Trp Gly Gln Gly Thr Leu Val Thr Val Ser
                565                 570                 575
Ser
```

<210> 109
<211> 571
<212> PRT
<213> Artificial Sequence

<220>
<223> Humanised

<400> 109

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Asp Tyr
20 25 30
Tyr Met Asn Trp Val Arg Gln Ala Pro Gly Gln Gly Leu Glu Trp Met
35 40 45
Gly Asn Ile Asn Pro Asn Asn Gly Gly Thr Asn Tyr Asn Gln Lys Phe
50 55 60
Lys Asp Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Trp Ile Leu Tyr Tyr Gly Arg Ser Lys Trp Tyr Phe Asp Val
100 105 110
Trp Gly Arg Gly Thr Leu Val Thr Val Ser Ser Ala Ser Thr Lys Gly
115 120 125
Pro Ser Val Phe Pro Leu Ala Pro Ser Ser Lys Ser Thr Ser Gly Gly
130 135 140
Thr Ala Ala Leu Gly Cys Leu Val Lys Asp Tyr Phe Pro Glu Pro Val
145 150 155 160
Thr Val Ser Trp Asn Ser Gly Ala Leu Thr Ser Gly Val His Thr Phe
165 170 175
Pro Ala Val Leu Gln Ser Ser Gly Leu Tyr Ser Leu Ser Ser Val Val
180 185 190
Thr Val Pro Ser Ser Ser Leu Gly Thr Gln Thr Tyr Ile Cys Asn Val

```
                195                     200                     205
Asn His Lys Pro Ser Asn Thr Lys Val Asp Lys Lys Val Glu Pro Lys
    210                     215                     220
Ser Cys Asp Lys Thr His Thr Cys Pro Pro Cys Pro Ala Pro Glu Leu
225                     230                     235                     240
Leu Gly Gly Pro Ser Val Phe Leu Phe Pro Pro Lys Pro Lys Asp Thr
                245                     250                     255
Leu Met Ile Ser Arg Thr Pro Glu Val Thr Cys Val Val Val Asp Val
            260                     265                     270
Ser His Glu Asp Pro Glu Val Lys Phe Asn Trp Tyr Val Asp Gly Val
        275                     280                     285
Glu Val His Asn Ala Lys Thr Lys Pro Arg Glu Glu Gln Tyr Asn Ser
    290                     295                     300
Thr Tyr Arg Val Val Ser Val Leu Thr Val Leu His Gln Asp Trp Leu
305                     310                     315                     320
Asn Gly Lys Glu Tyr Lys Cys Lys Val Ser Asn Lys Ala Leu Pro Ala
                325                     330                     335
Pro Ile Glu Lys Thr Ile Ser Lys Ala Lys Gly Gln Pro Arg Glu Pro
            340                     345                     350
Gln Val Tyr Thr Leu Pro Pro Ser Arg Asp Glu Leu Thr Lys Asn Gln
        355                     360                     365
Val Ser Leu Thr Cys Leu Val Lys Gly Phe Tyr Pro Ser Asp Ile Ala
    370                     375                     380
Val Glu Trp Glu Ser Asn Gly Gln Pro Glu Asn Asn Tyr Lys Thr Thr
385                     390                     395                     400
Pro Pro Val Leu Asp Ser Asp Gly Ser Phe Phe Leu Tyr Ser Lys Leu
                405                     410                     415
Thr Val Asp Lys Ser Arg Trp Gln Gln Gly Asn Val Phe Ser Cys Ser
            420                     425                     430
Val Met His Glu Ala Leu His Asn His Tyr Thr Gln Lys Ser Leu Ser
        435                     440                     445
Leu Ser Pro Gly Lys Gly Ser Glu Val Gln Leu Leu Val Ser Gly Gly
    450                     455                     460
Gly Leu Val Gln Pro Gly Gly Ser Leu Arg Leu Ser Cys Ala Ala Ser
465                     470                     475                     480
Gly Phe Thr Phe Lys Ala Tyr Pro Met Met Trp Val Arg Gln Ala Pro
                485                     490                     495
Gly Lys Gly Leu Glu Trp Val Ser Glu Ile Ser Pro Ser Gly Ser Tyr
            500                     505                     510
Thr Tyr Tyr Ala Asp Ser Val Lys Gly Arg Phe Thr Ile Ser Arg Asp
        515                     520                     525
Asn Ser Lys Asn Thr Leu Tyr Leu Gln Met Asn Ser Leu Arg Ala Glu
    530                     535                     540
Asp Thr Ala Val Tyr Tyr Cys Ala Lys Asp Pro Arg Lys Leu Asp Tyr
545                     550                     555                     560
Trp Gly Gln Gly Thr Leu Val Thr Val Ser Ser
                565                     570
```

<210> 110
<211> 453
<212> PRT
<213> Artificial Sequence

<220>
<223> Humanised

<400> 110

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys
    450
```

<210> 111
<211> 343
<212> PRT
<213> Artificial Sequence

<220>
<223> Humanised

<400> 111

Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Ile Val Gln Ser
            20                  25                  30
Asn Gly Asp Thr Tyr Leu Glu Trp Tyr Leu Gln Lys Pro Gly Gln Ser
        35                  40                  45
Pro Gln Leu Leu Ile Tyr Arg Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Phe Gln Gly
                85                  90                  95
Ser His Val Pro Tyr Thr Phe Gly Gln Gly Thr Lys Leu Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser

        195                 200                 205
Pro Val Thr Lys Ser Phe Asn Arg Gly Glu Cys Thr Val Ala Ala Pro
    210                 215                 220
Ser Gly Ser Glu Val Gln Leu Leu Val Ser Gly Gly Gly Leu Val Gln
225                 230                 235                 240
Pro Gly Gly Ser Leu Arg Leu Ser Cys Ala Ala Ser Gly Phe Thr Phe
                245                 250                 255
Lys Ala Tyr Pro Met Met Trp Val Arg Gln Ala Pro Gly Lys Gly Leu
            260                 265                 270
Glu Trp Val Ser Glu Ile Ser Pro Ser Gly Ser Tyr Thr Tyr Tyr Ala
        275                 280                 285
Asp Ser Val Lys Gly Arg Phe Thr Ile Ser Arg Asp Asn Ser Lys Asn
    290                 295                 300
Thr Leu Tyr Leu Gln Met Asn Ser Leu Arg Ala Glu Asp Thr Ala Val
305                 310                 315                 320
Tyr Tyr Cys Ala Lys Asp Pro Arg Lys Leu Asp Tyr Trp Gly Gln Gly
                325                 330                 335
Thr Leu Val Thr Val Ser Ser
            340

<210> 112
<211> 337
<212> PRT
<213> Artificial Sequence

<220>
<223> Humanised

<400> 112

Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Ile Val Gln Ser
20 25 30
Asn Gly Asp Thr Tyr Leu Glu Trp Tyr Leu Gln Lys Pro Gly Gln Ser
35 40 45
Pro Gln Leu Leu Ile Tyr Arg Val Ser Asn Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Phe Gln Gly
85 90 95
Ser His Val Pro Tyr Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190
Lys His Lys Val Tyr Ala Cys Glu Val Thr His Gln Gly Leu Ser Ser
195 200 205
Pro Val Thr Lys Ser Phe Asn Arg Gly Glu Cys Gly Ser Glu Val Gln
210 215 220
Leu Leu Val Ser Gly Gly Gly Leu Val Gln Pro Gly Gly Ser Leu Arg
225 230 235 240
Leu Ser Cys Ala Ala Ser Gly Phe Thr Phe Lys Ala Tyr Pro Met Met
245 250 255
Trp Val Arg Gln Ala Pro Gly Lys Gly Leu Glu Trp Val Ser Glu Ile
260 265 270
Ser Pro Ser Gly Ser Tyr Thr Tyr Tyr Ala Asp Ser Val Lys Gly Arg
275 280 285
Phe Thr Ile Ser Arg Asp Asn Ser Lys Asn Thr Leu Tyr Leu Gln Met
290 295 300
Asn Ser Leu Arg Ala Glu Asp Thr Ala Val Tyr Tyr Cys Ala Lys Asp
305 310 315 320
Pro Arg Lys Leu Asp Tyr Trp Gly Gln Gly Thr Leu Val Thr Val Ser
325 330 335
Ser

<210> 113
<211> 219
<212> PRT
<213> Artificial Sequence

<220>
<223> Humanised

<400> 113

Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Ile Val Gln Ser
20 25 30
Asn Gly Asp Thr Tyr Leu Glu Trp Tyr Leu Gln Lys Pro Gly Gln Ser
35 40 45
Pro Gln Leu Leu Ile Tyr Arg Val Ser Asn Arg Phe Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Phe Gln Gly
85 90 95
Ser His Val Pro Tyr Thr Phe Gly Gln Gly Thr Lys Leu Glu Ile Lys
100 105 110
Arg Thr Val Ala Ala Pro Ser Val Phe Ile Phe Pro Pro Ser Asp Glu
115 120 125
Gln Leu Lys Ser Gly Thr Ala Ser Val Val Cys Leu Leu Asn Asn Phe
130 135 140
Tyr Pro Arg Glu Ala Lys Val Gln Trp Lys Val Asp Asn Ala Leu Gln
145 150 155 160
Ser Gly Asn Ser Gln Glu Ser Val Thr Glu Gln Asp Ser Lys Asp Ser
165 170 175
Thr Tyr Ser Leu Ser Ser Thr Leu Thr Leu Ser Lys Ala Asp Tyr Glu
180 185 190
Lys His Lys Val Tyr Ala Cys Glu Val Thr His Gln Gly Leu Ser Ser
195 200 205
Pro Val Thr Lys Ser Phe Asn Arg Gly Glu Cys
210 215

<210> 114
<211> 122
<212> PRT
<213> Artificial Sequence

<220>
<223> Humanised

<400> 114

Gln Val Gln Leu Lys Gln Ser Gly Pro Gly Leu Val Gln Ser Ser Gln
1 5 10 15
Ser Leu Ser Ile Thr Cys Thr Ile Ser Gly Phe Ser Leu Thr Ser His
20 25 30
Gly Ile Tyr Trp Leu Arg Gln Ser Pro Gly Lys Gly Leu Glu Trp Leu
35 40 45
Gly Val Ile Trp Ser Gly Gly Ser Ala Asp Tyr Asn Ala Ala Phe Ile
50 55 60
Ser Arg Leu Ser Ile Ser Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Arg Ser Pro Tyr Tyr Tyr Arg Ser Ser Leu Tyr Ala Met Asp Tyr Trp
100 105 110
Gly Gln Gly Thr Ser Val Thr Val Ser Ser
115 120

<210> 115
<211> 109
<212> PRT
<213> Artificial Sequence

<220>
<223> Humanised

<400> 115

Asn Ile Val Leu Thr Gln Ser Pro Lys Ser Met Ser Met Ser Ile Gly
1               5                   10                  15
Glu Arg Val Thr Leu Ser Cys Lys Ala Ser Glu Asn Val Gly Thr Tyr
            20                  25                  30
Val Ser Trp Tyr Gln Gln Lys Ala Glu Gln Ser Pro Lys Leu Leu Ile
        35                  40                  45
Tyr Gly Ala Ser Asn Arg His Thr Gly Val Pro Asp Arg Phe Thr Gly
    50                  55                  60
Ser Gly Ser Ser Thr Asp Phe Thr Leu Thr Ile Ser Ser Val Gln Ala
65                  70                  75                  80
Glu Asp Leu Ala Asp Tyr His Cys Gly Gln Ser Tyr Ser Asp Pro Leu
                85                  90                  95
Thr Phe Gly Ala Gly Thr Lys Leu Glu Leu Lys Arg Ala
            100                 105

<210> 116
<211> 577
<212> PRT
<213> Artificial Sequence

<220>
<223> Humanised

<400> 116

Gln Val Gln Leu Gln Gln Pro Gly Ala Glu Leu Val Lys Pro Gly Ala
1 5 10 15
Ser Val Lys Met Ser Cys Lys Ala Ser Gly Tyr Thr Phe Thr Ser Tyr
20 25 30
Asn Met His Trp Val Lys Gln Thr Pro Gly Arg Gly Leu Glu Trp Ile
35 40 45
Gly Ala Ile Tyr Pro Gly Asn Gly Asp Thr Ser Tyr Asn Gln Lys Phe
50 55 60
Lys Gly Lys Ala Thr Leu Thr Ala Asp Lys Ser Ser Ser Thr Ala Tyr
65 70 75 80
Met Gln Leu Ser Ser Leu Thr Ser Glu Asp Ser Ala Val Tyr Tyr Cys
85 90 95
Ala Arg Ser Thr Tyr Tyr Gly Gly Asp Trp Tyr Phe Asn Val Trp Gly
100 105 110
Ala Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Thr Val Ala Ala Pro Ser Gly Ser Gly Val Gln Leu Leu
450 455 460
Glu Ser Gly Gly Gly Leu Val Gln Pro Gly Gly Ser Leu Arg Leu Ser
465 470 475 480
Cys Ala Ala Ser Gly Phe Thr Phe Ala Trp Tyr Asp Met Gly Trp Val
485 490 495
Arg Gln Ala Pro Gly Lys Gly Leu Glu Trp Val Ser Ser Ile Asp Trp
500 505 510
His Gly Glu Val Thr Tyr Tyr Ala Asp Ser Val Lys Gly Arg Phe Thr
515 520 525
Ile Ser Arg Asp Asn Ser Lys Asn Thr Leu Tyr Leu Gln Met Asn Ser
530 535 540
Leu Arg Ala Glu Asp Thr Ala Val Tyr Tyr Cys Ala Thr Ala Glu Asp
545 550 555 560
Glu Pro Gly Tyr Asp Tyr Trp Gly Gln Gly Thr Leu Val Thr Val Ser
565 570 575
Ser

<210> 117
<211> 213
<212> PRT
<213> Artificial Sequence

<220>
<223> Humanised

<400> 117

Gln Ile Val Leu Ser Gln Ser Pro Ala Ile Leu Ser Ala Ser Pro Gly
1 5 10 15
Glu Lys Val Thr Met Thr Cys Arg Ala Ser Ser Ser Val Ser Tyr Ile
20 25 30
His Trp Phe Gln Gln Lys Pro Gly Ser Ser Pro Lys Pro Trp Ile Tyr
35 40 45
Ala Thr Ser Asn Leu Ala Ser Gly Val Pro Val Arg Phe Ser Gly Ser
50 55 60
Gly Ser Gly Thr Ser Tyr Ser Leu Thr Ile Ser Arg Val Glu Ala Glu
65 70 75 80
Asp Ala Ala Thr Tyr Tyr Cys Gln Gln Trp Thr Ser Asn Pro Pro Thr
85 90 95
Phe Gly Gly Gly Thr Lys Leu Glu Ile Lys Arg Thr Val Ala Ala Pro
100 105 110
Ser Val Phe Ile Phe Pro Pro Ser Asp Glu Gln Leu Lys Ser Gly Thr
115 120 125
Ala Ser Val Val Cys Leu Leu Asn Asn Phe Tyr Pro Arg Glu Ala Lys
130 135 140
Val Gln Trp Lys Val Asp Asn Ala Leu Gln Ser Gly Asn Ser Gln Glu
145 150 155 160
Ser Val Thr Glu Gln Asp Ser Lys Asp Ser Thr Tyr Ser Leu Ser Ser
165 170 175
Thr Leu Thr Leu Ser Lys Ala Asp Tyr Glu Lys His Lys Val Tyr Ala
180 185 190
Cys Glu Val Thr His Gln Gly Leu Ser Ser Pro Val Thr Lys Ser Phe
195 200 205
Asn Arg Gly Glu Cys
210

<210> 118
<211> 571

<212> PRT
<213> Artificial Sequence

<220>
<223> Humanised

<400> 118

```
Gln Val Gln Leu Gln Gln Pro Gly Ala Glu Leu Val Lys Pro Gly Ala
1               5                   10                  15
Ser Val Lys Met Ser Cys Lys Ala Ser Gly Tyr Thr Phe Thr Ser Tyr
            20                  25                  30
```

Asn Met His Trp Val Lys Gln Thr Pro Gly Arg Gly Leu Glu Trp Ile
35 40 45
Gly Ala Ile Tyr Pro Gly Asn Gly Asp Thr Ser Tyr Asn Gln Lys Phe
50 55 60
Lys Gly Lys Ala Thr Leu Thr Ala Asp Lys Ser Ser Ser Thr Ala Tyr
65 70 75 80
Met Gln Leu Ser Ser Leu Thr Ser Glu Asp Ser Ala Val Tyr Tyr Cys
85 90 95
Ala Arg Ser Thr Tyr Tyr Gly Gly Asp Trp Tyr Phe Asn Val Trp Gly
100 105 110
Ala Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Ser Gly Val Gln Leu Leu Glu Ser Gly Gly Gly Leu
450 455 460
Val Gln Pro Gly Gly Ser Leu Arg Leu Ser Cys Ala Ala Ser Gly Phe
465 470 475 480
Thr Phe Ala Trp Tyr Asp Met Gly Trp Val Arg Gln Ala Pro Gly Lys
485 490 495
Gly Leu Glu Trp Val Ser Ser Ile Asp Trp His Gly Glu Val Thr Tyr
500 505 510
Tyr Ala Asp Ser Val Lys Gly Arg Phe Thr Ile Ser Arg Asp Asn Ser
515 520 525
Lys Asn Thr Leu Tyr Leu Gln Met Asn Ser Leu Arg Ala Glu Asp Thr
530 535 540
Ala Val Tyr Tyr Cys Ala Thr Ala Glu Asp Glu Pro Gly Tyr Asp Tyr
545 550 555 560
Trp Gly Gln Gly Thr Leu Val Thr Val Ser Ser
565 570

<210> 119
<211> 339
<212> PRT
<213> Artificial Sequence

<220>
<223> Humanised

<400> 119

```
Gln Ile Val Leu Ser Gln Ser Pro Ala Ile Leu Ser Ala Ser Pro Gly
1               5                   10                  15
Glu Lys Val Thr Met Thr Cys Arg Ala Ser Ser Ser Val Ser Tyr Ile
            20                  25                  30
His Trp Phe Gln Gln Lys Pro Gly Ser Ser Pro Lys Pro Trp Ile Tyr
        35                  40                  45
Ala Thr Ser Asn Leu Ala Ser Gly Val Pro Val Arg Phe Ser Gly Ser
    50                  55                  60
Gly Ser Gly Thr Ser Tyr Ser Leu Thr Ile Ser Arg Val Glu Ala Glu
65                  70                  75                  80
Asp Ala Ala Thr Tyr Tyr Cys Gln Gln Trp Thr Ser Asn Pro Pro Thr
                85                  90                  95
Phe Gly Gly Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys Thr Val Ala Ala Pro Ser Gly Ser Gly Val Gln
    210                 215                 220
Leu Leu Glu Ser Gly Gly Gly Leu Val Gln Pro Gly Gly Ser Leu Arg
225                 230                 235                 240
Leu Ser Cys Ala Ala Ser Gly Phe Thr Phe Ala Trp Tyr Asp Met Gly
                245                 250                 255
Trp Val Arg Gln Ala Pro Gly Lys Gly Leu Glu Trp Val Ser Ser Ile
            260                 265                 270
Asp Trp His Gly Glu Val Thr Tyr Tyr Ala Asp Ser Val Lys Gly Arg
        275                 280                 285
Phe Thr Ile Ser Arg Asp Asn Ser Lys Asn Thr Leu Tyr Leu Gln Met
    290                 295                 300
Asn Ser Leu Arg Ala Glu Asp Thr Ala Val Tyr Tyr Cys Ala Thr Ala
305                 310                 315                 320
Glu Asp Glu Pro Gly Tyr Asp Tyr Trp Gly Gln Gly Thr Leu Val Thr
                325                 330                 335
Val Ser Ser
```

<210> 120
<211> 451
<212> PRT
<213> Artificial sequence

<220>
<223> Humanised

<400> 120

Gln Val Gln Leu Gln Gln Pro Gly Ala Glu Leu Val Lys Pro Gly Ala
1 5 10 15
Ser Val Lys Met Ser Cys Lys Ala Ser Gly Tyr Thr Phe Thr Ser Tyr
20 25 30
Asn Met His Trp Val Lys Gln Thr Pro Gly Arg Gly Leu Glu Trp Ile
35 40 45
Gly Ala Ile Tyr Pro Gly Asn Gly Asp Thr Ser Tyr Asn Gln Lys Phe
50 55 60
Lys Gly Lys Ala Thr Leu Thr Ala Asp Lys Ser Ser Ser Thr Ala Tyr
65 70 75 80
Met Gln Leu Ser Ser Leu Thr Ser Glu Asp Ser Ala Val Tyr Tyr Cys
85 90 95
Ala Arg Ser Thr Tyr Tyr Gly Gly Asp Trp Tyr Phe Asn Val Trp Gly
100 105 110
Ala Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys
450

<210> 121
<211> 333
<212> PRT
<213> Artificial Sequence

<220>
<223> Humanised

<400> 121

```
Gln Ile Val Leu Ser Gln Ser Pro Ala Ile Leu Ser Ala Ser Pro Gly
1               5                   10                  15
Glu Lys Val Thr Met Thr Cys Arg Ala Ser Ser Ser Val Ser Tyr Ile
            20                  25                  30
His Trp Phe Gln Gln Lys Pro Gly Ser Ser Pro Lys Pro Trp Ile Tyr
        35                  40                  45
Ala Thr Ser Asn Leu Ala Ser Gly Val Pro Val Arg Phe Ser Gly Ser
    50                  55                  60
Gly Ser Gly Thr Ser Tyr Ser Leu Thr Ile Ser Arg Val Glu Ala Glu
65                  70                  75                  80
Asp Ala Ala Thr Tyr Tyr Cys Gln Gln Trp Thr Ser Asn Pro Pro Thr
                85                  90                  95
Phe Gly Gly Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe

        195                 200                 205
Asn Arg Gly Glu Cys Gly Ser Gly Val Gln Leu Leu Glu Ser Gly Gly
    210                 215                 220
Gly Leu Val Gln Pro Gly Gly Ser Leu Arg Leu Ser Cys Ala Ala Ser
225                 230                 235                 240
Gly Phe Thr Phe Ala Trp Tyr Asp Met Gly Trp Val Arg Gln Ala Pro
                245                 250                 255
Gly Lys Gly Leu Glu Trp Val Ser Ser Ile Asp Trp His Gly Glu Val
            260                 265                 270
Thr Tyr Tyr Ala Asp Ser Val Lys Gly Arg Phe Thr Ile Ser Arg Asp
        275                 280                 285
Asn Ser Lys Asn Thr Leu Tyr Leu Gln Met Asn Ser Leu Arg Ala Glu
    290                 295                 300
Asp Thr Ala Val Tyr Tyr Cys Ala Thr Ala Glu Asp Glu Pro Gly Tyr
305                 310                 315                 320
Asp Tyr Trp Gly Gln Gly Thr Leu Val Thr Val Ser Ser
                325                 330
```

<210> 122
<211> 1713
<212> DNA
<213> Artificial Sequence

<220>
<223> Humanised

<400> 122

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ccagcgccag caccaagggc cccagcgtgt tccccctggc ccccagcagc 420
aagagcacca gcggcggcac agccgccctg ggctgcctgg tgaaggacta cttccccgaa 480
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaagaccgt ggccgccccc 1380
tcgggatccg acatccagat gacccagagc cccagcagcc tgagcgccag cgtgggcgac 1440
agggtgacca ttacctgcag ggccagcagg cccatcagcg actggctgca ctggtaccaa 1500
cagaagcccg gcaaggctcc caagctgctg atcgcctggg ccagcagcct gcagggaggc 1560
gtgcccagca ggtttagcgg cagcggcagc ggcaccgact tcaccctcac catctcttcc 1620
ctgcagcccg aggacttcgc cacctactac tgcctgcagg agggctgggg gccccctact 1680
ttcggccagg gcaccaaggt ggagatcaag agg                             1713
```

<210> 123
<211> 607
<212> PRT
<213> Artificial Sequence

<220>
<223> Humanised

<400> 123

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
```

```
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Gly Ser Asp Gly Gly Gly Ile Arg Arg Ser Met
    450                 455                 460
Ser Gly Thr Trp Tyr Leu Lys Ala Met Thr Val Asp Arg Glu Phe Pro
465                 470                 475                 480
Glu Met Asn Leu Glu Ser Val Thr Pro Met Thr Leu Thr Leu Leu Lys
                485                 490                 495
Gly His Asn Leu Glu Ala Lys Val Thr Met Leu Ile Ser Gly Arg Cys
            500                 505                 510
Gln Glu Val Lys Ala Val Leu Gly Arg Thr Lys Glu Arg Lys Lys Tyr
        515                 520                 525
Thr Ala Asp Gly Gly Lys His Val Ala Tyr Ile Ile Pro Ser Ala Val
    530                 535                 540
Arg Asp His Val Ile Phe Tyr Ser Glu Gly Gln Leu His Gly Lys Pro
545                 550                 555                 560
Val Arg Gly Val Lys Leu Val Gly Arg Asp Pro Lys Asn Asn Leu Glu
                565                 570                 575
Ala Leu Glu Asp Phe Glu Lys Ala Ala Gly Ala Arg Gly Leu Ser Thr
            580                 585                 590
Glu Ser Ile Leu Ile Pro Arg Gln Ser Glu Thr Cys Ser Pro Gly
        595                 600                 605
```

<210> 124
<211> 541
<212> PRT
<213> Artificial Sequence

<220>
<223> Humanised

<400> 124

Gln Val Gln Leu Val Gln Ser Gly Ala Glu Val Lys Lys Pro Gly Ala

1 5 10 15

Ser Val Lys Val Ser Cys Lys Ala Ser Gly Tyr Thr Phe Thr Asp Tyr

20 25 30

Tyr Met Asn Trp Val Arg Gln Ala Pro Gly Gln Gly Leu Glu Trp Met

35 40 45

Gly Asn Ile Asn Pro Asn Asn Gly Gly Thr Asn Tyr Asn Gln Lys Phe

50 55 60

Lys Asp Arg Val Thr Met Thr Thr Asp Thr Ser Thr Ser Thr Ala Tyr

65 70 75 80

Met Glu Leu Arg Ser Leu Arg Ser Asp Asp Thr Ala Val Tyr Tyr Cys

85 90 95

Ala Arg Trp Ile Leu Tyr Tyr Gly Arg Ser Lys Trp Tyr Phe Asp Val

100 105 110

Trp Gly Arg Gly Thr Leu Val Thr Val Ser Ser Ala Ser Thr Lys Gly

115 120 125

Pro Ser Val Phe Pro Leu Ala Pro Ser Ser Lys Ser Thr Ser Gly Gly

130 135 140

Thr Ala Ala Leu Gly Cys Leu Val Lys Asp Tyr Phe Pro Glu Pro Val

145 150 155 160

Thr Val Ser Trp Asn Ser Gly Ala Leu Thr Ser Gly Val His Thr Phe

165 170 175

Pro Ala Val Leu Gln Ser Ser Gly Leu Tyr Ser Leu Ser Ser Val Val

180 185 190

Thr Val Pro Ser Ser Ser Leu Gly Thr Gln Thr Tyr Ile Cys Asn Val

195 200 205

Asn His Lys Pro Ser Asn Thr Lys Val Asp Lys Lys Val Glu Pro Lys

210 215 220

Ser Cys Asp Lys Thr His Thr Cys Pro Pro Cys Pro Ala Pro Glu Leu

225 230 235 240

Leu Gly Gly Pro Ser Val Phe Leu Phe Pro Pro Lys Pro Lys Asp Thr

245 250 255

Leu Met Ile Ser Arg Thr Pro Glu Val Thr Cys Val Val Val Asp Val

260 265 270

Ser His Glu Asp Pro Glu Val Lys Phe Asn Trp Tyr Val Asp Gly Val

275 280 285

Glu Val His Asn Ala Lys Thr Lys Pro Arg Glu Glu Gln Tyr Asn Ser

290 295 300

Thr Tyr Arg Val Val Ser Val Leu Thr Val Leu His Gln Asp Trp Leu

305 310 315 320

Asn Gly Lys Glu Tyr Lys Cys Lys Val Ser Asn Lys Ala Leu Pro Ala

325 330 335

Pro Ile Glu Lys Thr Ile Ser Lys Ala Lys Gly Gln Pro Arg Glu Pro

340 345 350

Gln Val Tyr Thr Leu Pro Pro Ser Arg Asp Glu Leu Thr Lys Asn Gln

355 360 365

Val Ser Leu Thr Cys Leu Val Lys Gly Phe Tyr Pro Ser Asp Ile Ala

370 375 380

Val Glu Trp Glu Ser Asn Gly Gln Pro Glu Asn Asn Tyr Lys Thr Thr

385 390 395 400

Pro Pro Val Leu Asp Ser Asp Gly Ser Phe Phe Leu Tyr Ser Lys Leu

405 410 415

Thr Val Asp Lys Ser Arg Trp Gln Gln Gly Asn Val Phe Ser Cys Ser

420 425 430

Val Met His Glu Ala Leu His Asn His Tyr Thr Gln Lys Ser Leu Ser

435 440 445

Leu Ser Pro Gly Lys Gly Ser Glu Val Val Ala Ala Thr Pro Thr Ser

450 455 460

Leu Leu Ile Ser Trp Arg His Pro His Phe Pro Thr Arg Tyr Tyr Arg

465 470 475 480

Ile Thr Tyr Gly Glu Thr Gly Gly Asn Ser Pro Val Gln Glu Phe Thr

485 490 495

Val Pro Leu Gln Pro Pro Thr Ala Thr Ile Ser Gly Leu Lys Pro Gly

500 505 510

Val Asp Tyr Thr Ile Thr Val Tyr Ala Val Thr Asp Gly Arg Asn Gly

515 520 525

Arg Leu Leu Ser Ile Pro Ile Ser Ile Asn Tyr Arg Thr

530 535 540

<210> 125
<211> 613
<212> PRT
<213> Artificial Sequence

<220>
<223> Humanised

<400> 125

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Thr Val Ala Ala Pro Ser Gly Ser Asp Gly Gly
    450                 455                 460
Gly Ile Arg Arg Ser Met Ser Gly Thr Trp Tyr Leu Lys Ala Met Thr
465                 470                 475                 480
Val Asp Arg Glu Phe Pro Glu Met Asn Leu Glu Ser Val Thr Pro Met
                485                 490                 495
Thr Leu Thr Leu Leu Lys Gly His Asn Leu Glu Ala Lys Val Thr Met
            500                 505                 510
Leu Ile Ser Gly Arg Cys Gln Glu Val Lys Ala Val Leu Gly Arg Thr
        515                 520                 525
Lys Glu Arg Lys Lys Tyr Thr Ala Asp Gly Gly Lys His Val Ala Tyr
    530                 535                 540
Ile Ile Pro Ser Ala Val Arg Asp His Val Ile Phe Tyr Ser Glu Gly
545                 550                 555                 560
Gln Leu His Gly Lys Pro Val Arg Gly Val Lys Leu Val Gly Arg Asp
```

```
                    565                 570                 575
Pro Lys Asn Asn Leu Glu Ala Leu Glu Asp Phe Glu Lys Ala Ala Gly
                580                 585                 590
Ala Arg Gly Leu Ser Thr Glu Ser Ile Leu Ile Pro Arg Gln Ser Glu
            595                 600                 605
Thr Cys Ser Pro Gly
        610
```

<210> 126
<211> 513
<212> PRT
<213> Artificial Sequence

<220>
<223> Humanised

<400> 126

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Gly Ser Val Asp Asn Lys Phe Asn Lys Glu Leu
    450                 455                 460
```

```
Arg Gln Ala Tyr Trp Glu Ile Gln Ala Leu Pro Asn Leu Asn Trp Thr
465                 470                 475                 480
Gln Ser Arg Ala Phe Ile Arg Ser Leu Tyr Asp Asp Pro Ser Gln Ser
                485                 490                 495
Ala Asn Leu Leu Ala Glu Ala Lys Lys Leu Asn Asp Ala Gln Ala Pro
            500                 505                 510
Lys
```

<210> 127
<211> 519
<212> PRT
<213> Artificial Sequence

<220>
<223> Humanised

<400> 127

Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Thr Val Ala Ala Pro Ser Gly Ser Val Asp Asn

```
    450                     455                     460
Lys Phe Asn Lys Glu Leu Arg Gln Ala Tyr Trp Glu Ile Gln Ala Leu
465                 470                     475                 480
Pro Asn Leu Asn Trp Thr Gln Ser Arg Ala Phe Ile Arg Ser Leu Tyr
                485                     490                     495
Asp Asp Pro Ser Gln Ser Ala Asn Leu Leu Ala Glu Ala Lys Lys Leu
            500                     505                 510
Asn Asp Ala Gln Ala Pro Lys
        515
```

<210> 128
<211> 578
<212> PRT
<213> Artificial Sequence

<220>
<223> Humanised

<400> 128

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Asp Tyr
20 25 30
Tyr Met Asn Trp Val Arg Gln Ala Pro Gly Gln Gly Leu Glu Trp Met
35 40 45
Gly Asn Ile Asn Pro Asn Asn Gly Gly Thr Asn Tyr Asn Gln Lys Phe
50 55 60
Lys Asp Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Trp Ile Leu Tyr Tyr Gly Arg Ser Lys Trp Tyr Phe Asp Val
100 105 110
Trp Gly Arg Gly Thr Leu Val Thr Val Ser Ser Ala Ser Thr Lys Gly
115 120 125
Pro Ser Val Phe Pro Leu Ala Pro Ser Ser Lys Ser Thr Ser Gly Gly
130 135 140
Thr Ala Ala Leu Gly Cys Leu Val Lys Asp Tyr Phe Pro Glu Pro Val
145 150 155 160
Thr Val Ser Trp Asn Ser Gly Ala Leu Thr Ser Gly Val His Thr Phe
165 170 175
Pro Ala Val Leu Gln Ser Ser Gly Leu Tyr Ser Leu Ser Ser Val Val
180 185 190
Thr Val Pro Ser Ser Ser Leu Gly Thr Gln Thr Tyr Ile Cys Asn Val
195 200 205
Asn His Lys Pro Ser Asn Thr Lys Val Asp Lys Lys Val Glu Pro Lys
210 215 220
Ser Cys Asp Lys Thr His Thr Cys Pro Pro Cys Pro Ala Pro Glu Leu
225 230 235 240
Leu Gly Gly Pro Ser Val Phe Leu Phe Pro Pro Lys Pro Lys Asp Thr
245 250 255
Leu Met Ile Ser Arg Thr Pro Glu Val Thr Cys Val Val Val Asp Val
260 265 270
Ser His Glu Asp Pro Glu Val Lys Phe Asn Trp Tyr Val Asp Gly Val
275 280 285
Glu Val His Asn Ala Lys Thr Lys Pro Arg Glu Glu Gln Tyr Asn Ser
290 295 300
Thr Tyr Arg Val Val Ser Val Leu Thr Val Leu His Gln Asp Trp Leu
305 310 315 320
Asn Gly Lys Glu Tyr Lys Cys Lys Val Ser Asn Lys Ala Leu Pro Ala
325 330 335
Pro Ile Glu Lys Thr Ile Ser Lys Ala Lys Gly Gln Pro Arg Glu Pro
340 345 350
Gln Val Tyr Thr Leu Pro Pro Ser Arg Asp Glu Leu Thr Lys Asn Gln
355 360 365
Val Ser Leu Thr Cys Leu Val Lys Gly Phe Tyr Pro Ser Asp Ile Ala
370 375 380
Val Glu Trp Glu Ser Asn Gly Gln Pro Glu Asn Asn Tyr Lys Thr Thr
385 390 395 400
Pro Pro Val Leu Asp Ser Asp Gly Ser Phe Phe Leu Tyr Ser Lys Leu
405 410 415
Thr Val Asp Lys Ser Arg Trp Gln Gln Gly Asn Val Phe Ser Cys Ser
420 425 430
Val Met His Glu Ala Leu His Asn His Tyr Thr Gln Lys Ser Leu Ser
435 440 445

Leu Ser Pro Gly Lys Gly Ser Asp Leu Gly Lys Lys Leu Leu Glu Ala
450 455 460
Ala Arg Ala Gly Gln Asp Asp Glu Val Arg Ile Leu Met Ala Asn Gly
465 470 475 480
Ala Asp Val Asn Ala Lys Asp Glu Tyr Gly Leu Thr Pro Leu Tyr Leu
485 490 495
Ala Thr Ala His Gly His Leu Glu Ile Val Glu Val Leu Leu Lys Asn
500 505 510
Gly Ala Asp Val Asn Ala Val Asp Ala Ile Gly Phe Thr Pro Leu His
515 520 525
Leu Ala Ala Phe Ile Gly His Leu Glu Ile Ala Glu Val Leu Leu Lys
530 535 540
His Gly Ala Asp Val Asn Ala Gln Asp Lys Phe Gly Lys Thr Ala Phe
545 550 555 560
Asp Ile Ser Ile Gly Asn Gly Asn Glu Asp Leu Ala Glu Ile Leu Gln
565 570 575
Lys Leu

<210> 129
<211> 584
<212> PRT
<213> Artificial Sequence

<220>
<223> Humanised

<400> 129

Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
            260                 265                 270
Ser His Glu Asp Pro Glu Val Lys Phe Asn Trp Tyr Val Asp Gly Val
        275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala

```
        370                     375                     380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Thr Val Ala Ala Pro Ser Gly Ser Asp Leu Gly
    450                 455                 460
Lys Lys Leu Leu Glu Ala Ala Arg Ala Gly Gln Asp Asp Glu Val Arg
465                 470                 475                 480
Ile Leu Met Ala Asn Gly Ala Asp Val Asn Ala Lys Asp Glu Tyr Gly
                485                 490                 495
Leu Thr Pro Leu Tyr Leu Ala Thr Ala His Gly His Leu Glu Ile Val
            500                 505                 510
Glu Val Leu Leu Lys Asn Gly Ala Asp Val Asn Ala Val Asp Ala Ile
        515                 520                 525
Gly Phe Thr Pro Leu His Leu Ala Ala Phe Ile Gly His Leu Glu Ile
    530                 535                 540
Ala Glu Val Leu Leu Lys His Gly Ala Asp Val Asn Ala Gln Asp Lys
545                 550                 555                 560
Phe Gly Lys Thr Ala Phe Asp Ile Ser Ile Gly Asn Gly Asn Glu Asp
                565                 570                 575
Leu Ala Glu Ile Leu Gln Lys Leu
            580
```

<210> 130
<211> 574
<212> PRT
<213> Artificial Sequence

<220>
<223> Humanised

<400> 130

Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Ser Gln Val Gln Leu Val Glu Ser Gly Gly Gly Leu
450 455 460
Val Gln Ala Gly Gly Ser Leu Arg Leu Ser Cys Ala Ala Ser Gly Tyr
465 470 475 480
Ala Tyr Thr Tyr Ile Tyr Met Gly Trp Phe Arg Gln Ala Pro Gly Lys
485 490 495
Glu Arg Glu Gly Val Ala Ala Met Asp Ser Gly Gly Gly Gly Thr Leu
500 505 510
Tyr Ala Asp Ser Val Lys Gly Arg Phe Thr Ile Ser Arg Asp Lys Gly
515 520 525
Lys Asn Thr Val Tyr Leu Gln Met Asp Ser Leu Lys Pro Glu Asp Thr
530 535 540
Ala Thr Tyr Tyr Cys Ala Ala Gly Gly Tyr Glu Leu Arg Asp Arg Thr
545 550 555 560
Tyr Gly Gln Trp Gly Gln Gly Thr Gln Val Thr Val Ser Ser
565 570

<210> 131
<211> 566
<212> PRT
<213> Artificial Sequence

<220>
<223> Humanised

<400> 131

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met

```
                245                     250                     255
Ile Ser Arg Thr Pro Glu Val Thr Cys Val Val Val Asp Val Ser His
            260                     265                     270
Glu Asp Pro Glu Val Lys Phe Asn Trp Tyr Val Asp Gly Val Glu Val
        275                     280                     285
His Asn Ala Lys Thr Lys Pro Arg Glu Glu Gln Tyr Asn Ser Thr Tyr
    290                     295                     300
Arg Val Val Ser Val Leu Thr Val Leu His Gln Asp Trp Leu Asn Gly
305                     310                     315                 320
Lys Glu Tyr Lys Cys Lys Val Ser Asn Lys Ala Leu Pro Ala Pro Ile
                325                     330                     335
Glu Lys Thr Ile Ser Lys Ala Lys Gly Gln Pro Arg Glu Pro Gln Val
            340                     345                     350
Tyr Thr Leu Pro Pro Ser Arg Asp Glu Leu Thr Lys Asn Gln Val Ser
        355                     360                     365
Leu Thr Cys Leu Val Lys Gly Phe Tyr Pro Ser Asp Ile Ala Val Glu
    370                     375                     380
Trp Glu Ser Asn Gly Gln Pro Glu Asn Asn Tyr Lys Thr Thr Pro Pro
385                     390                     395                 400
Val Leu Asp Ser Asp Gly Ser Phe Phe Leu Tyr Ser Lys Leu Thr Val
                405                     410                     415
Asp Lys Ser Arg Trp Gln Gln Gly Asn Val Phe Ser Cys Ser Val Met
            420                     425                     430
His Glu Ala Leu His Asn His Tyr Thr Gln Lys Ser Leu Ser Leu Ser
        435                     440                     445
Pro Gly Lys Gly Ser Ala Arg Val Asp Gln Thr Pro Arg Ser Val Thr
    450                     455                     460
Lys Glu Thr Gly Glu Ser Leu Thr Ile Asn Cys Val Leu Arg Asp Ala
465                     470                     475                 480
Ser Tyr Ala Leu Gly Ser Thr Cys Trp Tyr Arg Lys Lys Ser Gly Glu
                485                     490                     495
Gly Asn Glu Glu Ser Ile Ser Lys Gly Gly Arg Tyr Val Glu Thr Val
            500                     505                     510
Asn Ser Gly Ser Lys Ser Phe Ser Leu Arg Ile Asn Asp Leu Thr Val
        515                     520                     525
Glu Asp Gly Gly Thr Tyr Arg Cys Gly Leu Gly Val Ala Gly Gly Tyr
    530                     535                     540
Cys Asp Tyr Ala Leu Cys Ser Ser Arg Tyr Ala Glu Cys Gly Asp Gly
545                     550                     555                 560
Thr Ala Val Thr Val Asn
                565
```

<210> 132
<211> 1659
<212> PRT
<213> Artificial Sequence

<220>
<223> Humanised

<400> 132

Cys Ala Gly Gly Thr Gly Ala Cys Cys Cys Thr Gly Ala Gly Gly Gly
1 5 10 15
Ala Gly Ala Gly Cys Gly Gly Cys Cys Cys Cys Gly Cys Cys Cys Thr
20 25 30
Gly Gly Thr Gly Ala Ala Gly Cys Cys Cys Ala Cys Cys Cys Ala Gly
35 40 45
Ala Cys Cys Cys Thr Gly Ala Cys Cys Cys Thr Gly Ala Cys Cys Thr
50 55 60
Gly Cys Ala Cys Cys Thr Thr Cys Ala Gly Cys Gly Gly Cys Thr Thr
65 70 75 80
Thr Ala Gly Cys Cys Thr Cys Ala Gly Cys Ala Cys Cys Thr Cys Cys
85 90 95
Gly Gly Cys Ala Thr Gly Gly Gly Cys Gly Thr Gly Ala Gly Cys Thr
100 105 110
Gly Gly Ala Thr Cys Ala Gly Gly Cys Ala Gly Cys Cys Ala Cys Cys
115 120 125
Cys Gly Gly Cys Ala Ala Ala Gly Gly Cys Cys Thr Gly Gly Ala Gly
130 135 140
Thr Gly Gly Cys Thr Gly Gly Cys Cys Cys Ala Cys Ala Thr Cys Thr
145 150 155 160
Ala Cys Thr Gly Gly Gly Ala Cys Gly Ala Cys Gly Ala Cys Ala Ala
165 170 175
Gly Ala Gly Gly Thr Ala Cys Ala Ala Cys Cys Cys Cys Ala Gly Cys
180 185 190

Cys Thr Gly Ala Ala Gly Ala Gly Cys Cys Gly Gly Cys Thr Gly Ala
195 200 205
Cys Cys Ala Thr Cys Ala Gly Cys Ala Ala Gly Gly Ala Thr Ala Cys
210 215 220
Cys Ala Gly Cys Ala Gly Gly Ala Ala Cys Cys Ala Gly Gly Thr Gly
225 230 235 240
Gly Thr Gly Cys Thr Gly Ala Cys Cys Ala Thr Gly Ala Cys Cys Ala
245 250 255
Ala Cys Ala Thr Gly Gly Ala Cys Cys Cys Cys Gly Thr Gly Gly Ala
260 265 270
Cys Ala Cys Cys Gly Cys Thr Ala Cys Cys Thr Ala Cys Thr Ala Cys
275 280 285
Thr Gly Cys Gly Cys Cys Ala Gly Gly Ala Gly Gly Gly Ala Gly Ala
290 295 300
Cys Cys Gly Thr Cys Thr Thr Cys Thr Ala Cys Thr Gly Gly Thr Ala
305 310 315 320
Cys Thr Thr Cys Gly Ala Cys Gly Thr Gly Thr Gly Gly Gly Gly Ala
325 330 335
Ala Gly Gly Gly Gly Cys Ala Cys Ala Cys Thr Ala Gly Thr Gly Ala
340 345 350
Cys Cys Gly Thr Gly Thr Cys Cys Ala Gly Cys Gly Cys Cys Ala Gly
355 360 365
Cys Ala Cys Cys Ala Ala Gly Gly Gly Cys Cys Cys Cys Ala Gly Cys
370 375 380
Gly Thr Gly Thr Thr Cys Cys Cys Cys Cys Thr Gly Gly Cys Cys Cys
385 390 395 400
Cys Cys Ala Gly Cys Ala Gly Cys Ala Ala Gly Ala Gly Cys Ala Cys
405 410 415
Cys Ala Gly Cys Gly Gly Cys Gly Gly Cys Ala Cys Ala Gly Cys Cys
420 425 430
Gly Cys Cys Cys Thr Gly Gly Gly Cys Thr Gly Cys Cys Thr Gly Gly
435 440 445
Thr Gly Ala Ala Gly Gly Ala Cys Thr Ala Cys Thr Thr Cys Cys Cys
450 455 460
Cys Gly Ala Ala Cys Cys Gly Gly Thr Gly Ala Cys Cys Gly Thr Gly
465 470 475 480
Thr Cys Cys Thr Gly Gly Ala Ala Cys Ala Gly Cys Gly Gly Ala Gly
485 490 495
Cys Cys Cys Thr Gly Ala Cys Cys Ala Gly Cys Gly Gly Cys Gly Thr
500 505 510
Gly Cys Ala Cys Ala Cys Cys Thr Thr Cys Cys Cys Cys Gly Cys Cys
515 520 525
Gly Thr Gly Cys Thr Gly Cys Ala Gly Ala Gly Cys Ala Gly Cys Gly
530 535 540
Gly Cys Cys Thr Gly Thr Ala Cys Ala Gly Cys Cys Thr Gly Ala Gly
545 550 555 560
Cys Ala Gly Cys Gly Thr Gly Gly Thr Gly Ala Cys Cys Gly Thr Gly
565 570 575
Cys Cys Cys Ala Gly Cys Ala Gly Cys Ala Gly Cys Cys Thr Gly Gly
580 585 590
Gly Cys Ala Cys Cys Cys Ala Gly Ala Cys Cys Thr Ala Cys Ala Thr
595 600 605
Cys Thr Gly Thr Ala Ala Cys Gly Thr Gly Ala Ala Cys Cys Ala Cys
610 615 620
Ala Ala Gly Cys Cys Cys Ala Gly Cys Ala Ala Cys Ala Cys Cys Ala
625 630 635 640
Ala Gly Gly Thr Gly Gly Ala Cys Ala Ala Gly Ala Ala Gly Gly Thr
645 650 655
Gly Gly Ala Gly Cys Cys Cys Ala Ala Gly Ala Gly Cys Thr Gly Thr
660 665 670
Gly Ala Cys Ala Ala Gly Ala Cys Cys Cys Ala Cys Ala Cys Cys Thr
675 680 685
Gly Cys Cys Cys Cys Cys Cys Cys Thr Gly Cys Cys Cys Thr Gly Cys
690 695 700
Cys Cys Cys Cys Gly Ala Gly Cys Thr Gly Cys Thr Gly Gly Gly Ala
705 710 715 720
Gly Gly Cys Cys Cys Cys Ala Gly Cys Gly Thr Gly Thr Thr Cys Cys
725 730 735
Thr Gly Thr Thr Cys Cys Cys Cys Cys Cys Cys Ala Ala Gly Cys Cys
740 745 750
Thr Ala Ala Gly Gly Ala Cys Ala Cys Cys Cys Thr Gly Ala Thr Gly
755 760 765
Ala Thr Cys Ala Gly Cys Ala Gly Ala Ala Cys Cys Cys Cys Cys Gly
770 775 780
Ala Gly Gly Thr Gly Ala Cys Cys Thr Gly Thr Gly Thr Gly Gly Thr
785 790 795 800

Gly Gly Thr Gly Gly Ala Thr Gly Thr Gly Ala Gly Cys Cys Ala Cys
805 810 815
Gly Ala Gly Gly Ala Cys Cys Cys Thr Gly Ala Gly Gly Thr Gly Ala
820 825 830
Ala Gly Thr Thr Cys Ala Ala Cys Thr Gly Gly Thr Ala Cys Gly Thr
835 840 845
Gly Gly Ala Cys Gly Gly Cys Gly Thr Gly Gly Ala Gly Gly Thr Gly
850 855 860
Cys Ala Cys Ala Ala Thr Gly Cys Cys Ala Ala Gly Ala Cys Cys Ala
865 870 875 880
Ala Gly Cys Cys Cys Ala Gly Gly Gly Ala Gly Gly Ala Gly Cys Ala
885 890 895
Gly Thr Ala Cys Ala Ala Cys Ala Gly Cys Ala Cys Cys Thr Ala Cys
900 905 910
Cys Gly Gly Gly Thr Gly Gly Thr Gly Thr Cys Cys Gly Thr Gly Cys
915 920 925
Thr Gly Ala Cys Cys Gly Thr Gly Cys Thr Gly Cys Ala Cys Cys Ala
930 935 940
Gly Gly Ala Thr Thr Gly Gly Cys Thr Gly Ala Ala Cys Gly Gly Cys
945 950 955 960
Ala Ala Gly Gly Ala Gly Thr Ala Cys Ala Ala Gly Thr Gly Thr Ala
965 970 975
Ala Gly Gly Thr Gly Thr Cys Cys Ala Ala Cys Ala Ala Gly Gly Cys
980 985 990
Cys Cys Thr Gly Cys Cys Thr Gly Cys Cys Cys Cys Thr Ala Thr Cys
995 1000 1005
Gly Ala Gly Ala Ala Ala Ala Cys Cys Ala Thr Cys Ala Gly Cys Ala
1010 1015 1020
Ala Gly Gly Cys Cys Ala Ala Gly Gly Gly Cys Cys Ala Gly Cys Cys
1025 1030 1035 1040
Cys Ala Gly Ala Gly Ala Gly Cys Cys Cys Cys Ala Gly Gly Thr Gly
1045 1050 1055
Thr Ala Cys Ala Cys Cys Cys Thr Gly Cys Cys Cys Cys Cys Thr Ala
1060 1065 1070
Gly Cys Ala Gly Ala Gly Ala Thr Gly Ala Gly Cys Thr Gly Ala Cys
1075 1080 1085
Cys Ala Ala Gly Ala Ala Cys Cys Ala Gly Gly Thr Gly Thr Cys Cys
1090 1095 1100
Cys Thr Gly Ala Cys Cys Thr Gly Cys Cys Thr Gly Gly Thr Gly Ala
1105 1110 1115 1120
Ala Gly Gly Gly Cys Thr Thr Cys Thr Ala Cys Cys Cys Cys Ala Gly
1125 1130 1135
Cys Gly Ala Cys Ala Thr Cys Gly Cys Cys Gly Thr Gly Gly Ala Gly
1140 1145 1150
Thr Gly Gly Gly Ala Gly Ala Gly Cys Ala Ala Cys Gly Gly Cys Cys
1155 1160 1165
Ala Gly Cys Cys Cys Gly Ala Gly Ala Ala Cys Ala Ala Cys Thr Ala
1170 1175 1180
Cys Ala Ala Gly Ala Cys Cys Ala Cys Cys Cys Cys Cys Cys Cys Thr
1185 1190 1195 1200
Gly Thr Gly Cys Thr Gly Gly Ala Cys Ala Gly Cys Gly Ala Thr Gly
1205 1210 1215
Gly Cys Ala Gly Cys Thr Thr Cys Thr Thr Cys Cys Thr Gly Thr Ala
1220 1225 1230
Cys Ala Gly Cys Ala Ala Gly Cys Thr Gly Ala Cys Cys Gly Thr Gly
1235 1240 1245
Gly Ala Cys Ala Ala Gly Ala Gly Cys Ala Gly Ala Thr Gly Gly Cys
1250 1255 1260
Ala Gly Cys Ala Gly Gly Gly Cys Ala Ala Cys Gly Thr Gly Thr Thr
1265 1270 1275 1280
Cys Ala Gly Cys Thr Gly Cys Thr Cys Cys Gly Thr Gly Ala Thr Gly
1285 1290 1295
Cys Ala Cys Gly Ala Gly Gly Cys Cys Cys Thr Gly Cys Ala Cys Ala
1300 1305 1310
Ala Thr Cys Ala Cys Thr Ala Cys Ala Cys Cys Cys Ala Gly Ala Ala
1315 1320 1325
Gly Ala Gly Cys Cys Thr Gly Ala Gly Cys Cys Thr Gly Thr Cys Cys
1330 1335 1340
Cys Cys Thr Gly Gly Cys Ala Ala Gly Ala Cys Cys Gly Thr Gly Gly
1345 1350 1355 1360
Cys Cys Gly Cys Cys Cys Cys Thr Cys Gly Gly Gly Ala Thr Cys
1365 1370 1375
Cys Gly Thr Gly Ala Gly Cys Gly Ala Cys Gly Thr Gly Cys Cys Ala
1380 1385 1390
Ala Gly Gly Gly Ala Cys Cys Thr Cys Gly Ala Gly Gly Thr Gly Gly
1395 1400 1405

Thr Gly Gly Cys Ala Gly Cys Cys Ala Cys Thr Cys Cys Cys Ala Cys
1410 1415 1420
Cys Thr Cys Thr Cys Thr Gly Cys Thr Gly Ala Thr Cys Ala Gly Cys
1425 1430 1435 1440
Thr Gly Gly Gly Ala Cys Ala Cys Ala Cys Ala Cys Ala Ala Cys Gly
1445 1450 1455
Cys Cys Thr Ala Cys Ala Ala Cys Gly Gly Cys Thr Ala Cys Thr Ala
1460 1465 1470
Cys Ala Gly Gly Ala Thr Cys Ala Cys Cys Thr Ala Cys Gly Gly Ala
1475 1480 1485
Gly Ala Gly Ala Cys Cys Gly Gly Cys Gly Gly Cys Ala Ala Thr Ala
1490 1495 1500
Gly Cys Cys Cys Cys Gly Thr Gly Ala Gly Gly Gly Ala Gly Thr Thr
1505 1510 1515 1520
Cys Ala Cys Cys Gly Thr Gly Cys Cys Cys Cys Ala Cys Cys Cys Cys
1525 1530 1535
Gly Ala Gly Gly Thr Gly Ala Cys Cys Gly Cys Cys Ala Cys Cys Ala
1540 1545 1550
Thr Thr Ala Gly Cys Gly Gly Cys Cys Thr Gly Ala Ala Gly Cys Cys
1555 1560 1565
Cys Gly Gly Cys Gly Thr Gly Gly Ala Cys Gly Ala Thr Ala Cys Cys
1570 1575 1580
Ala Thr Cys Ala Cys Cys Gly Thr Cys Thr Ala Cys Gly Cys Cys Gly
1585 1590 1595 1600
Thr Gly Ala Cys Cys Ala Ala Cys Cys Ala Cys Cys Ala Cys Ala Thr
1605 1610 1615
Gly Cys Cys Cys Cys Thr Gly Ala Gly Gly Ala Thr Cys Thr Thr Cys
1620 1625 1630
Gly Gly Cys Cys Cys Cys Ala Thr Cys Ala Gly Cys Ala Thr Cys Ala
1635 1640 1645
Ala Cys Cys Ala Thr Ala Gly Gly Ala Cys Cys
1650 1655

<210> 133
<211> 547
<212> PRT
<213> Artificial Sequence

<220>
<223> Humanised

<400> 133

Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Gly Leu Glu Trp Met
        35                  40                  45
Gly Asn Ile Asn Pro Asn Asn Gly Gly Thr Asn Tyr Asn Gln Lys Phe
    50                  55                  60
Lys Asp Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Ile Leu Tyr Tyr Gly Arg Ser Lys Trp Tyr Phe Asp Val
            100                 105                 110
Trp Gly Arg Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Gln Thr Tyr Ile Cys Asn Val
        195                 200                 205
Asn His Lys Pro Ser Asn Thr Lys Val Asp Lys Lys Val Glu Pro Lys
    210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val

```
                260                     265                     270
Ser His Glu Asp Pro Glu Val Lys Phe Asn Trp Tyr Val Asp Gly Val
            275                     280                     285
Glu Val His Asn Ala Lys Thr Lys Pro Arg Glu Glu Gln Tyr Asn Ser
    290                     295                     300
Thr Tyr Arg Val Val Ser Val Leu Thr Val Leu His Gln Asp Trp Leu
305                     310                     315                     320
Asn Gly Lys Glu Tyr Lys Cys Lys Val Ser Asn Lys Ala Leu Pro Ala
                325                     330                     335
Pro Ile Glu Lys Thr Ile Ser Lys Ala Lys Gly Gln Pro Arg Glu Pro
            340                     345                     350
Gln Val Tyr Thr Leu Pro Pro Ser Arg Asp Glu Leu Thr Lys Asn Gln
        355                     360                     365
Val Ser Leu Thr Cys Leu Val Lys Gly Phe Tyr Pro Ser Asp Ile Ala
    370                     375                     380
Val Glu Trp Glu Ser Asn Gly Gln Pro Glu Asn Asn Tyr Lys Thr Thr
385                     390                     395                     400
Pro Pro Val Leu Asp Ser Asp Gly Ser Phe Phe Leu Tyr Ser Lys Leu
                405                     410                     415
Thr Val Asp Lys Ser Arg Trp Gln Gln Gly Asn Val Phe Ser Cys Ser
            420                     425                     430
Val Met His Glu Ala Leu His Asn His Tyr Thr Gln Lys Ser Leu Ser
        435                     440                     445
Leu Ser Pro Gly Lys Thr Val Ala Ala Pro Ser Gly Ser Glu Val Val
    450                     455                     460
Ala Ala Thr Pro Thr Ser Leu Leu Ile Ser Trp Arg His Pro His Phe
465                     470                     475                     480
Pro Thr Arg Tyr Tyr Arg Ile Thr Tyr Gly Glu Thr Gly Gly Asn Ser
                485                     490                     495
Pro Val Gln Glu Phe Thr Val Pro Leu Gln Pro Pro Thr Ala Thr Ile
            500                     505                     510
Ser Gly Leu Lys Pro Gly Val Asp Tyr Thr Ile Thr Val Tyr Ala Val
        515                     520                     525
Thr Asp Gly Arg Asn Gly Arg Leu Leu Ser Ile Pro Ile Ser Ile Asn
    530                     535                     540
Tyr Arg Thr
545
```

<210> 134
<211> 551
<212> PRT
<213> Artificial Sequence

<220>
<223> Humanised

<400> 134

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser
180 185 190
Val Val Thr Val Pro Ser Ser Ser Leu Gly Thr Gln Thr Tyr Ile Cys
195 200 205
Asn Val Asn His Lys Pro Ser Asn Thr Lys Val Asp Lys Lys Val Glu
210 215 220

Pro Lys Ser Cys Asp Lys Thr His Thr Cys Pro Pro Cys Pro Ala Pro
225 230 235 240
Glu Leu Leu Gly Gly Pro Ser Val Phe Leu Phe Pro Pro Lys Pro Lys
245 250 255
Asp Thr Leu Met Ile Ser Arg Thr Pro Glu Val Thr Cys Val Val Val
260 265 270
Asp Val Ser His Glu Asp Pro Glu Val Lys Phe Asn Trp Tyr Val Asp
275 280 285
Gly Val Glu Val His Asn Ala Lys Thr Lys Pro Arg Glu Glu Gln Tyr
290 295 300
Asn Ser Thr Tyr Arg Val Val Ser Val Leu Thr Val Leu His Gln Asp
305 310 315 320
Trp Leu Asn Gly Lys Glu Tyr Lys Cys Lys Val Ser Asn Lys Ala Leu
325 330 335
Pro Ala Pro Ile Glu Lys Thr Ile Ser Lys Ala Lys Gly Gln Pro Arg
340 345 350
Glu Pro Gln Val Tyr Thr Leu Pro Pro Ser Arg Asp Glu Leu Thr Lys
355 360 365
Asn Gln Val Ser Leu Thr Cys Leu Val Lys Gly Phe Tyr Pro Ser Asp
370 375 380
Ile Ala Val Glu Trp Glu Ser Asn Gly Gln Pro Glu Asn Asn Tyr Lys
385 390 395 400
Thr Thr Pro Pro Val Leu Asp Ser Asp Gly Ser Phe Phe Leu Tyr Ser
405 410 415
Lys Leu Thr Val Asp Lys Ser Arg Trp Gln Gln Gly Asn Val Phe Ser
420 425 430
Cys Ser Val Met His Glu Ala Leu His Asn His Tyr Thr Gln Lys Ser
435 440 445
Leu Ser Leu Ser Pro Gly Lys Gly Ser Val Ser Asp Val Pro Arg Asp
450 455 460
Leu Glu Val Val Ala Ala Thr Pro Thr Ser Leu Leu Ile Ser Trp Asp
465 470 475 480
Thr His Asn Ala Tyr Asn Gly Tyr Tyr Arg Ile Thr Tyr Gly Glu Thr
485 490 495
Gly Gly Asn Ser Pro Val Arg Glu Phe Thr Val Pro His Pro Glu Val
500 505 510
Thr Ala Thr Ile Ser Gly Leu Lys Pro Gly Val Asp Asp Thr Ile Thr
515 520 525
Val Tyr Ala Val Thr Asn His His Met Pro Leu Arg Ile Phe Gly Pro
530 535 540
Ile Ser Ile Asn His Arg Thr
545 550

<210> 135
<211> 557
<212> PRT
<213> Artificial Sequence

<220>
<223> Humanised

<400> 135

Gln Val Gln Leu Val Gln Ser Gly Ala Glu Val Lys Lys Pro Gly Ser
1 5 10 15
Ser Val Lys Val Ser Cys Lys Ala Ser Gly Phe Tyr Ile Lys Asp Thr
20 25 30
Tyr Met His Trp Val Arg Gln Ala Pro Gly Gln Gly Leu Glu Trp Met
35 40 45
Gly Thr Ile Asp Pro Ala Asn Gly Asn Thr Lys Tyr Val Pro Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Ser Ile Tyr Asp Asp Tyr His Tyr Asp Asp Tyr Tyr Ala Met
100 105 110
Asp Tyr Trp Gly Gln Gly Thr Leu Val Thr Val Ser Ser Ala Ser Thr
115 120 125
Lys Gly Pro Ser Val Phe Pro Leu Ala Pro Ser Ser Lys Ser Thr Ser
130 135 140
Gly Gly Thr Ala Ala Leu Gly Cys Leu Val Lys Asp Tyr Phe Pro Glu
145 150 155 160
Pro Val Thr Val Ser Trp Asn Ser Gly Ala Leu Thr Ser Gly Val His
165 170 175
Thr Phe Pro Ala Val Leu Gln Ser Ser Gly Leu Tyr Ser Leu Ser Ser

```
                    180                           185                     190
Val Val Thr Val Pro Ser Ser Ser Leu Gly Thr Gln Thr Tyr Ile Cys
            195                     200                     205
Asn Val Asn His Lys Pro Ser Asn Thr Lys Val Asp Lys Lys Val Glu
    210                     215                     220
Pro Lys Ser Cys Asp Lys Thr His Thr Cys Pro Pro Cys Pro Ala Pro
225                     230                     235                     240
Glu Leu Leu Gly Gly Pro Ser Val Phe Leu Phe Pro Pro Lys Pro Lys
                245                     250                     255
Asp Thr Leu Met Ile Ser Arg Thr Pro Glu Val Thr Cys Val Val Val
            260                     265                     270
Asp Val Ser His Glu Asp Pro Glu Val Lys Phe Asn Trp Tyr Val Asp
        275                     280                     285
Gly Val Glu Val His Asn Ala Lys Thr Lys Pro Arg Glu Glu Gln Tyr
    290                     295                     300
Asn Ser Thr Tyr Arg Val Val Ser Val Leu Thr Val Leu His Gln Asp
305                     310                     315                     320
Trp Leu Asn Gly Lys Glu Tyr Lys Cys Lys Val Ser Asn Lys Ala Leu
                325                     330                     335
Pro Ala Pro Ile Glu Lys Thr Ile Ser Lys Ala Lys Gly Gln Pro Arg
            340                     345                     350
Glu Pro Gln Val Tyr Thr Leu Pro Pro Ser Arg Asp Glu Leu Thr Lys
        355                     360                     365
Asn Gln Val Ser Leu Thr Cys Leu Val Lys Gly Phe Tyr Pro Ser Asp
    370                     375                     380
Ile Ala Val Glu Trp Glu Ser Asn Gly Gln Pro Glu Asn Asn Tyr Lys
385                     390                     395                     400
Thr Thr Pro Pro Val Leu Asp Ser Asp Gly Ser Phe Phe Leu Tyr Ser
                405                     410                     415
Lys Leu Thr Val Asp Lys Ser Arg Trp Gln Gln Gly Asn Val Phe Ser
            420                     425                     430
Cys Ser Val Met His Glu Ala Leu His Asn His Tyr Thr Gln Lys Ser
        435                     440                     445
Leu Ser Leu Ser Pro Gly Lys Thr Val Ala Ala Pro Ser Gly Ser Val
    450                     455                     460
Ser Asp Val Pro Arg Asp Leu Glu Val Val Ala Ala Thr Pro Thr Ser
465                     470                     475                     480
Leu Leu Ile Ser Trp Asp Thr His Asn Ala Tyr Asn Gly Tyr Tyr Arg
                485                     490                     495
Ile Thr Tyr Gly Glu Thr Gly Gly Asn Ser Pro Val Arg Glu Phe Thr
            500                     505                     510
Val Pro His Pro Glu Val Thr Ala Thr Ile Ser Gly Leu Lys Pro Gly
        515                     520                     525
Val Asp Asp Thr Ile Thr Val Tyr Ala Val Thr Asn His His Met Pro
    530                     535                     540
Leu Arg Ile Phe Gly Pro Ile Ser Ile Asn His Arg Thr
545                     550                     555
```

<210> 136
<211> 537
<212> PRT
<213> Artificial Sequence

<220>
<223> Humanised

<400> 136

```
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Asn Tyr
            20                  25                  30
Gly Val His Trp Val Arg Gln Ser Pro Gly Lys Gly Leu Glu Trp Leu
        35                  40                  45
Gly Val Ile Trp Ser Gly Gly Asn Thr Asp Tyr Asn Thr Pro Phe Thr
    50                  55                  60
Ser Arg Leu Ser Ile Asn Lys Asp Asn Ser Lys Ser Gln Val Phe Phe
65                  70                  75                  80
Lys Met Asn Ser Leu Gln Ser Asn Asp Thr Ala Ile Tyr Tyr Cys Ala
                85                  90                  95
Arg Ala Leu Thr Tyr Tyr Asp Tyr Glu Phe Ala Tyr Trp Gly Gln Gly
            100                 105                 110
Thr Leu Val Thr Val Ser Ala Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
```

Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys Arg Ser Glu Val Val Ala Ala Thr Pro Thr Ser Leu Leu Ile Ser
450 455 460
Trp Arg His Pro His Phe Pro Thr Arg Tyr Tyr Arg Ile Thr Tyr Gly
465 470 475 480
Glu Thr Gly Gly Asn Ser Pro Val Gln Glu Phe Thr Val Pro Leu Gln
485 490 495
Pro Pro Thr Ala Thr Ile Ser Gly Leu Lys Pro Gly Val Asp Tyr Thr
500 505 510
Ile Thr Val Tyr Ala Val Thr Asp Gly Arg Asn Gly Arg Leu Leu Ser
515 520 525
Ile Pro Ile Ser Ile Asn Tyr Arg Thr
530 535

<210> 137
<211> 214
<212> PRT
<213> Artificial Sequence

<220>
<223> Humanised

<400> 137

Asp Ile Leu Leu Thr Gln Ser Pro Val Ile Leu Ser Val Ser Pro Gly
1 5 10 15
Glu Arg Val Ser Phe Ser Cys Arg Ala Ser Gln Ser Ile Gly Thr Asn
20 25 30
Ile His Trp Tyr Gln Gln Arg Thr Asn Gly Ser Pro Arg Leu Leu Ile
35 40 45
Lys Tyr Ala Ser Glu Ser Ile Ser Gly Ile Pro Ser Arg Phe Ser Gly
50 55 60
Ser Gly Ser Gly Thr Asp Phe Thr Leu Ser Ile Asn Ser Val Glu Ser
65 70 75 80
Glu Asp Ile Ala Asp Tyr Tyr Cys Gln Gln Asn Asn Asn Trp Pro Thr
85 90 95
Thr Phe Gly Ala Gly Thr Lys Leu Glu Leu Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 138
<211> 302
<212> PRT
<213> Artificial Sequence

<220>
<223> Humanised

<400> 138

Asp Ile Leu Leu Thr Gln Ser Pro Val Ile Leu Ser Val Ser Pro Gly
1 5 10 15
Glu Arg Val Ser Phe Ser Cys Arg Ala Ser Gln Ser Ile Gly Thr Asn
20 25 30
Ile His Trp Tyr Gln Gln Arg Thr Asn Gly Ser Pro Arg Leu Leu Ile
35 40 45
Lys Tyr Ala Ser Glu Ser Ile Ser Gly Ile Pro Ser Arg Phe Ser Gly
50 55 60
Ser Gly Ser Gly Thr Asp Phe Thr Leu Ser Ile Asn Ser Val Glu Ser
65 70 75 80
Glu Asp Ile Ala Asp Tyr Tyr Cys Gln Gln Asn Asn Asn Trp Pro Thr
85 90 95
Thr Phe Gly Ala Gly Thr Lys Leu Glu Leu Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys Arg Ser Glu Val Val Ala Ala Thr Pro Thr
210 215 220
Ser Leu Leu Ile Ser Trp Arg His Pro His Phe Pro Thr Arg Tyr Tyr
225 230 235 240
Arg Ile Thr Tyr Gly Glu Thr Gly Gly Asn Ser Pro Val Gln Glu Phe
245 250 255
Thr Val Pro Leu Gln Pro Pro Thr Ala Thr Ile Ser Gly Leu Lys Pro
260 265 270
Gly Val Asp Tyr Thr Ile Thr Val Tyr Ala Val Thr Asp Gly Arg Asn
275 280 285
Gly Arg Leu Leu Ser Ile Pro Ile Ser Ile Asn Tyr Arg Thr
290 295 300

<210> 139
<211> 449
<212> PRT
<213> Artificial Sequence

<220>
<223> Humanised

<400> 139

Gln Val Gln Leu Lys Gln Ser Gly Pro Gly Leu Val Gln Pro Ser Gln
1 5 10 15
Ser Leu Ser Ile Thr Cys Thr Val Ser Gly Phe Ser Leu Thr Asn Tyr

```
                20                          25                          30
Gly Val His Trp Val Arg Gln Ser Pro Gly Lys Gly Leu Glu Trp Leu
            35                  40                      45
Gly Val Ile Trp Ser Gly Gly Asn Thr Asp Tyr Asn Thr Pro Phe Thr
    50                  55                      60
Ser Arg Leu Ser Ile Asn Lys Asp Asn Ser Lys Ser Gln Val Phe Phe
65                  70                      75                  80
Lys Met Asn Ser Leu Gln Ser Asn Asp Thr Ala Ile Tyr Tyr Cys Ala
                85                      90                      95
Arg Ala Leu Thr Tyr Tyr Asp Tyr Glu Phe Ala Tyr Trp Gly Gln Gly
            100                     105                     110
Thr Leu Val Thr Val Ser Ala Ala Ser Thr Lys Gly Pro Ser Val Phe
        115                     120                     125
Pro Leu Ala Pro Ser Ser Lys Ser Thr Ser Gly Gly Thr Ala Ala Leu
    130                     135                     140
Gly Cys Leu Val Lys Asp Tyr Phe Pro Glu Pro Val Thr Val Ser Trp
145                     150                     155                 160
Asn Ser Gly Ala Leu Thr Ser Gly Val His Thr Phe Pro Ala Val Leu
                165                     170                     175
Gln Ser Ser Gly Leu Tyr Ser Leu Ser Ser Val Val Thr Val Pro Ser
            180                     185                     190
Ser Ser Leu Gly Thr Gln Thr Tyr Ile Cys Asn Val Asn His Lys Pro
        195                     200                     205
Ser Asn Thr Lys Val Asp Lys Lys Val Glu Pro Lys Ser Cys Asp Lys
    210                     215                     220
Thr His Thr Cys Pro Pro Cys Pro Ala Pro Glu Leu Leu Gly Gly Pro
225                     230                     235                 240
Ser Val Phe Leu Phe Pro Pro Lys Pro Lys Asp Thr Leu Met Ile Ser
                245                     250                     255
Arg Thr Pro Glu Val Thr Cys Val Val Val Asp Val Ser His Glu Asp
            260                     265                     270
Pro Glu Val Lys Phe Asn Trp Tyr Val Asp Gly Val Glu Val His Asn
        275                     280                     285
Ala Lys Thr Lys Pro Arg Glu Glu Gln Tyr Asn Ser Thr Tyr Arg Val
    290                     295                     300
Val Ser Val Leu Thr Val Leu His Gln Asp Trp Leu Asn Gly Lys Glu
305                     310                     315                 320
Tyr Lys Cys Lys Val Ser Asn Lys Ala Leu Pro Ala Pro Ile Glu Lys
                325                     330                     335
Thr Ile Ser Lys Ala Lys Gly Gln Pro Arg Glu Pro Gln Val Tyr Thr
            340                     345                     350
Leu Pro Pro Ser Arg Asp Glu Leu Thr Lys Asn Gln Val Ser Leu Thr
        355                     360                     365
Cys Leu Val Lys Gly Phe Tyr Pro Ser Asp Ile Ala Val Glu Trp Glu
    370                     375                     380
Ser Asn Gly Gln Pro Glu Asn Asn Tyr Lys Thr Thr Pro Pro Val Leu
385                     390                     395                 400
Asp Ser Asp Gly Ser Phe Phe Leu Tyr Ser Lys Leu Thr Val Asp Lys
                405                     410                     415
Ser Arg Trp Gln Gln Gly Asn Val Phe Ser Cys Ser Val Met His Glu
            420                     425                     430
Ala Leu His Asn His Tyr Thr Gln Lys Ser Leu Ser Leu Ser Pro Gly
        435                     440                     445
Lys
```

<210> 140
<211> 539
<212> PRT
<213> Artificial Sequence

<220>
<223> Humanised

<400> 140

Gln Val Gln Leu Gln Glu Ser Gly Pro Gly Leu Val Lys Pro Ser Gln
1 5 10 15
Thr Leu Ser Leu Thr Cys Thr Val Ser Gly Gly Ser Ile Ser Ser Gly
20 25 30
Asp Tyr Tyr Trp Ser Trp Ile Arg Gln Pro Pro Gly Lys Gly Leu Glu
35 40 45
Trp Ile Gly Tyr Ile Tyr Tyr Ser Gly Ser Thr Asp Tyr Asn Pro Ser
50 55 60
Leu Lys Ser Arg Val Thr Met Ser Val Asp Thr Ser Lys Asn Gln Phe
65 70 75 80

Ser Leu Lys Val Asn Ser Val Thr Ala Ala Asp Thr Ala Val Tyr Tyr
85 90 95
Cys Ala Arg Val Ser Ile Phe Gly Val Gly Thr Phe Asp Tyr Trp Gly
100 105 110
Gln Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Ser Glu Val Val Ala Ala Thr Pro Thr Ser Leu Leu
450 455 460
Ile Ser Trp Arg His Pro His Phe Pro Thr Arg Tyr Tyr Arg Ile Thr
465 470 475 480
Tyr Gly Glu Thr Gly Gly Asn Ser Pro Val Gln Glu Phe Thr Val Pro
485 490 495
Leu Gln Pro Pro Thr Ala Thr Ile Ser Gly Leu Lys Pro Gly Val Asp
500 505 510
Tyr Thr Ile Thr Val Tyr Ala Val Thr Asp Gly Arg Asn Gly Arg Leu
515 520 525
Leu Ser Ile Pro Ile Ser Ile Asn Tyr Arg Thr
530 535

<210> 141
<211> 214
<212> PRT
<213> Artificial sequence

<220>

<223> Humanised

<400> 141

Glu Ile Val Met Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys His Gln Tyr Gly Ser Thr Pro Leu
                85                  90                  95
Thr Phe Gly Gly Gly Thr Lys Ala Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 142
<211> 302
<212> PRT
<213> Artificial Sequence

<220>
<223> Humanised

<400> 142

```
Glu Ile Val Met Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys His Gln Tyr Gly Ser Thr Pro Leu
                85                  90                  95
Thr Phe Gly Gly Gly Thr Lys Ala Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Ser Glu Val Val Ala Ala Thr Pro Thr
    210                 215                 220
Ser Leu Leu Ile Ser Trp Arg His Pro His Phe Pro Thr Arg Tyr Tyr
225                 230                 235                 240
Arg Ile Thr Tyr Gly Glu Thr Gly Gly Asn Ser Pro Val Gln Glu Phe
                245                 250                 255
Thr Val Pro Leu Gln Pro Pro Thr Ala Thr Ile Ser Gly Leu Lys Pro
            260                 265                 270
Gly Val Asp Tyr Thr Ile Thr Val Tyr Ala Val Thr Asp Gly Arg Asn
        275                 280                 285
Gly Arg Leu Leu Ser Ile Pro Ile Ser Ile Asn Tyr Arg Thr
    290                 295                 300
```

<210> 143
<211> 451
<212> PRT
<213> Artificial Sequence

<220>
<223> Humanised

<400> 143

Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Gly
            20                  25                  30
Asp Tyr Tyr Trp Ser Trp Ile Arg Gln Pro Pro Gly Lys Gly Leu Glu
        35                  40                  45
Trp Ile Gly Tyr Ile Tyr Tyr Ser Gly Ser Thr Asp Tyr Asn Pro Ser
    50                  55                  60
Leu Lys Ser Arg Val Thr Met Ser Val Asp Thr Ser Lys Asn Gln Phe
65                  70                  75                  80
Ser Leu Lys Val Asn Ser Val Thr Ala Ala Asp Thr Ala Val Tyr Tyr
                85                  90                  95
Cys Ala Arg Val Ser Ile Phe Gly Val Gly Thr Phe Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 144
<211> 539
<212> PRT
<213> Artificial Sequence

<220>

<223> Humanised

<400> 144

Glu Val Val Ala Ala Thr Pro Thr Ser Leu Leu Ile Ser Trp Arg His
1               5                   10                  15

Pro His Phe Pro Thr Arg Tyr Tyr Arg Ile Thr Tyr Gly Glu Thr Gly
20 25 30
Gly Asn Ser Pro Val Gln Glu Phe Thr Val Pro Leu Gln Pro Pro Thr
35 40 45
Ala Thr Ile Ser Gly Leu Lys Pro Gly Val Asp Tyr Thr Ile Thr Val
50 55 60
Tyr Ala Val Thr Asp Gly Arg Asn Gly Arg Leu Leu Ser Ile Pro Ile
65 70 75 80
Ser Ile Asn Tyr Arg Thr Gly Ser Thr Gly Gln Val Gln Leu Lys Gln
85 90 95
Ser Gly Pro Gly Leu Val Gln Pro Ser Gln Ser Leu Ser Ile Thr Cys
100 105 110
Thr Val Ser Gly Phe Ser Leu Thr Asn Tyr Gly Val His Trp Val Arg
115 120 125
Gln Ser Pro Gly Lys Gly Leu Glu Trp Leu Gly Val Ile Trp Ser Gly
130 135 140
Gly Asn Thr Asp Tyr Asn Thr Pro Phe Thr Ser Arg Leu Ser Ile Asn
145 150 155 160
Lys Asp Asn Ser Lys Ser Gln Val Phe Phe Lys Met Asn Ser Leu Gln
165 170 175
Ser Asn Asp Thr Ala Ile Tyr Tyr Cys Ala Arg Ala Leu Thr Tyr Tyr
180 185 190
Asp Tyr Glu Phe Ala Tyr Trp Gly Gln Gly Thr Leu Val Thr Val Ser
195 200 205
Ala Ala Ser Thr Lys Gly Pro Ser Val Phe Pro Leu Ala Pro Ser Ser
210 215 220
Lys Ser Thr Ser Gly Gly Thr Ala Ala Leu Gly Cys Leu Val Lys Asp
225 230 235 240
Tyr Phe Pro Glu Pro Val Thr Val Ser Trp Asn Ser Gly Ala Leu Thr
245 250 255
Ser Gly Val His Thr Phe Pro Ala Val Leu Gln Ser Ser Gly Leu Tyr
260 265 270
Ser Leu Ser Ser Val Val Thr Val Pro Ser Ser Ser Leu Gly Thr Gln
275 280 285
Thr Tyr Ile Cys Asn Val Asn His Lys Pro Ser Asn Thr Lys Val Asp
290 295 300
Lys Lys Val Glu Pro Lys Ser Cys Asp Lys Thr His Thr Cys Pro Pro
305 310 315 320
Cys Pro Ala Pro Glu Leu Leu Gly Gly Pro Ser Val Phe Leu Phe Pro
325 330 335
Pro Lys Pro Lys Asp Thr Leu Met Ile Ser Arg Thr Pro Glu Val Thr
340 345 350
Cys Val Val Val Asp Val Ser His Glu Asp Pro Glu Val Lys Phe Asn
355 360 365
Trp Tyr Val Asp Gly Val Glu Val His Asn Ala Lys Thr Lys Pro Arg
370 375 380
Glu Glu Gln Tyr Asn Ser Thr Tyr Arg Val Val Ser Val Leu Thr Val
385 390 395 400
Leu His Gln Asp Trp Leu Asn Gly Lys Glu Tyr Lys Cys Lys Val Ser
405 410 415
Asn Lys Ala Leu Pro Ala Pro Ile Glu Lys Thr Ile Ser Lys Ala Lys
420 425 430
Gly Gln Pro Arg Glu Pro Gln Val Tyr Thr Leu Pro Pro Ser Arg Asp
435 440 445
Glu Leu Thr Lys Asn Gln Val Ser Leu Thr Cys Leu Val Lys Gly Phe
450 455 460
Tyr Pro Ser Asp Ile Ala Val Glu Trp Glu Ser Asn Gly Gln Pro Glu
465 470 475 480
Asn Asn Tyr Lys Thr Thr Pro Pro Val Leu Asp Ser Asp Gly Ser Phe
485 490 495
Phe Leu Tyr Ser Lys Leu Thr Val Asp Lys Ser Arg Trp Gln Gln Gly
500 505 510
Asn Val Phe Ser Cys Ser Val Met His Glu Ala Leu His Asn His Tyr
515 520 525
Thr Gln Lys Ser Leu Ser Leu Ser Pro Gly Lys
530 535

<210> 145
<211> 303
<212> PRT
<213> Artificial Sequence

<220>
<223> Humanised

<400> 145
Glu Val Val Ala Ala Thr Pro Thr Ser Leu Leu Ile Ser Trp Arg His
1 5 10 15
Pro His Phe Pro Thr Arg Tyr Tyr Arg Ile Thr Tyr Gly Glu Thr Gly
20 25 30
Gly Asn Ser Pro Val Gln Glu Phe Thr Val Pro Leu Gln Pro Pro Thr
35 40 45
Ala Thr Ile Ser Gly Leu Lys Pro Gly Val Asp Tyr Thr Ile Thr Val
50 55 60
Tyr Ala Val Thr Asp Gly Arg Asn Gly Arg Leu Leu Ser Ile Pro Ile
65 70 75 80
Ser Ile Asn Tyr Arg Thr Ser Thr Gly Asp Ile Leu Leu Thr Gln Ser
85 90 95
Pro Val Ile Leu Ser Val Ser Pro Gly Glu Arg Val Ser Phe Ser Cys
100 105 110
Arg Ala Ser Gln Ser Ile Gly Thr Asn Ile His Trp Tyr Gln Gln Arg
115 120 125
Thr Asn Gly Ser Pro Arg Leu Leu Ile Lys Tyr Ala Ser Glu Ser Ile
130 135 140
Ser Gly Ile Pro Ser Arg Phe Ser Gly Ser Gly Ser Gly Thr Asp Phe
145 150 155 160
Thr Leu Ser Ile Asn Ser Val Glu Ser Glu Asp Ile Ala Asp Tyr Tyr
165 170 175
Cys Gln Gln Asn Asn Asn Trp Pro Thr Thr Phe Gly Ala Gly Thr Lys
180 185 190
Leu Glu Leu Lys Arg Thr Val Ala Ala Pro Ser Val Phe Ile Phe Pro
195 200 205
Pro Ser Asp Glu Gln Leu Lys Ser Gly Thr Ala Ser Val Val Cys Leu
210 215 220
Leu Asn Asn Phe Tyr Pro Arg Glu Ala Lys Val Gln Trp Lys Val Asp
225 230 235 240
Asn Ala Leu Gln Ser Gly Asn Ser Gln Glu Ser Val Thr Glu Gln Asp
245 250 255
Ser Lys Asp Ser Thr Tyr Ser Leu Ser Ser Thr Leu Thr Leu Ser Lys
260 265 270
Ala Asp Tyr Glu Lys His Lys Val Tyr Ala Cys Glu Val Thr His Gln
275 280 285
Gly Leu Ser Ser Pro Val Thr Lys Ser Phe Asn Arg Gly Glu Cys
290 295 300

<210> 146
<211> 547
<212> PRT
<213> Artificial Sequence

<220>
<223> Humanised

<400> 146

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205

```
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Ser Val Ser Asp Val Pro Arg Asp Leu Glu Val Val
    450                 455                 460
Ala Ala Thr Pro Thr Ser Leu Leu Ile Ser Trp Asp Thr His Asn Ala
465                 470                 475                 480
Tyr Asn Gly Tyr Tyr Arg Ile Thr Tyr Gly Glu Thr Gly Gly Asn Ser
                485                 490                 495
Pro Val Arg Glu Phe Thr Val Pro His Pro Glu Val Thr Ala Thr Ile
            500                 505                 510
Ser Gly Leu Lys Pro Gly Val Asp Asp Thr Ile Thr Val Tyr Ala Val
        515                 520                 525
Thr Asn His His Met Pro Leu Arg Ile Phe Gly Pro Ile Ser Ile Asn
    530                 535                 540
His Arg Thr
545
```

<210> 147
<211> 553
<212> PRT
<213> Artificial Sequence

<220>
<223> Humanised

<400> 147

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala

```
                        165                                 170                                 175
Val Leu Gln Ser Ser Gly Leu Tyr Ser Leu Ser Ser Val Val Thr Val
                180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Thr Val Ala Ala Pro Ser Gly Ser Val Ser Asp Val Pro
    450                 455                 460
Arg Asp Leu Glu Val Val Ala Ala Thr Pro Thr Ser Leu Leu Ile Ser
465                 470                 475                 480
Trp Asp Thr His Asn Ala Tyr Asn Gly Tyr Tyr Arg Ile Thr Tyr Gly
                485                 490                 495
Glu Thr Gly Gly Asn Ser Pro Val Arg Glu Phe Thr Val Pro His Pro
            500                 505                 510
Glu Val Thr Ala Thr Ile Ser Gly Leu Lys Pro Gly Val Asp Asp Thr
        515                 520                 525
Ile Thr Val Tyr Ala Val Thr Asn His His Met Pro Leu Arg Ile Phe
    530                 535                 540
Gly Pro Ile Ser Ile Asn His Arg Thr
545                 550
```

<210> 148
<211> 118
<212> PRT
<213> Homo Sapiens

<400> 148

Gly Val Gln Leu Leu Glu Ser Gly Gly Gly Leu Val Gln Pro Gly Gly
1 5 10 15
Ser Leu Arg Leu Ser Cys Ala Ala Ser Gly Phe Val Phe Pro Trp Tyr
20 25 30
Asp Met Gly Trp Val Arg Gln Ala Pro Gly Lys Gly Leu Glu Trp Val
35 40 45
Ser Ser Ile Asp Trp His Gly Lys Ile Thr Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Thr Ala Glu Asp Glu Pro Gly Tyr Asp Tyr Trp Gly Gln Gly Thr
100 105 110
Leu Val Thr Val Ser Ser
115

<210> 149
<211> 569
<212> PRT
<213> Artificial Sequence

<220>
<223> Humanised

<400> 149

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Gly Val Gln Leu Leu Glu Ser Gly Gly Gly Leu Val Gln
450 455 460
Pro Gly Gly Ser Leu Arg Leu Ser Cys Ala Ala Ser Gly Phe Val Phe
465 470 475 480
Pro Trp Tyr Asp Met Gly Trp Val Arg Gln Ala Pro Gly Lys Gly Leu
485 490 495
Glu Trp Val Ser Ser Ile Asp Trp His Gly Lys Ile Thr Tyr Tyr Ala
500 505 510
Asp Ser Val Lys Gly Arg Phe Thr Ile Ser Arg Asp Asn Ser Lys Asn
515 520 525
Thr Leu Tyr Leu Gln Met Asn Ser Leu Arg Ala Glu Asp Thr Ala Val
530 535 540

Tyr Tyr Cys Ala Thr Ala Glu Asp Glu Pro Gly Tyr Asp Tyr Trp Gly
545 550 555 560
Gln Gly Thr Leu Val Thr Val Ser Ser
565

<210> 150
<211> 575
<212> PRT
<213> Artificial Sequence

<220>
<223> Humanised

<400> 150

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Thr Val Ala Ala Pro Ser Gly Val Gln Leu Leu Glu Ser
    450                 455                 460
Gly Gly Gly Leu Val Gln Pro Gly Gly Ser Leu Arg Leu Ser Cys Ala
465                 470                 475                 480
Ala Ser Gly Phe Val Phe Pro Trp Tyr Asp Met Gly Trp Val Arg Gln
```

```
                        485                           490                           495
Ala Pro Gly Lys Gly Leu Glu Trp Val Ser Ser Ile Asp Trp His Gly
                    500                           505                           510
Lys Ile Thr Tyr Tyr Ala Asp Ser Val Lys Gly Arg Phe Thr Ile Ser
            515                           520                           525
Arg Asp Asn Ser Lys Asn Thr Leu Tyr Leu Gln Met Asn Ser Leu Arg
    530                           535                           540
Ala Glu Asp Thr Ala Val Tyr Tyr Cys Ala Thr Ala Glu Asp Glu Pro
545                           550                           555                           560
Gly Tyr Asp Tyr Trp Gly Gln Gly Thr Leu Val Thr Val Ser Ser
                        565                           570                           575
```

<210> 151
<211> 579
<212> PRT
<213> Artificial Sequence

<220>
<223> Humanised

<400> 151

Gln Val Gln Leu Val Gln Ser Gly Ala Glu Val Lys Lys Pro Gly Ala
1               5                   10                  15
Ser Val Lys Val Ser Cys Lys Ala Ser Gly Phe Tyr Ile Lys Asp Thr
            20                  25                  30
Tyr Met His Trp Val Arg Gln Ala Pro Gly Gln Gly Leu Glu Trp Met
        35                  40                  45
Gly Thr Ile Asp Pro Ala Asn Gly Asn Thr Lys Tyr Val Pro Lys Phe
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
        355                 360                 365
Asn Gln Val Ser Leu Thr Cys Leu Val Lys Gly Phe Tyr Pro Ser Asp
    370                 375                 380
Ile Ala Val Glu Trp Glu Ser Asn Gly Gln Pro Glu Asn Asn Tyr Lys
385                 390                 395                 400
Thr Thr Pro Pro Val Leu Asp Ser Asp Gly Ser Phe Phe Leu Tyr Ser
                405                 410                 415
Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
435 440 445
Leu Ser Leu Ser Pro Gly Lys Thr Val Ala Ala Pro Ser Gly Ser Glu
450 455 460
Val Gln Leu Leu Glu Ser Gly Gly Gly Leu Val Gln Pro Gly Gly Ser
465 470 475 480
Leu Arg Leu Ser Cys Ala Ala Ser Gly Phe Thr Phe Arg Asn Phe Gly
485 490 495
Met Gly Trp Val Arg Gln Ala Pro Gly Lys Gly Leu Glu Trp Val Ser
500 505 510
Trp Ile Ile Ser Ser Gly Thr Glu Thr Tyr Tyr Ala Asp Ser Val Lys
515 520 525
Gly Arg Phe Thr Ile Ser Arg Asp Asn Ser Lys Asn Thr Leu Tyr Leu
530 535 540
Gln Met Asn Ser Leu Arg Ala Glu Asp Thr Ala Val Tyr Tyr Cys Ala
545 550 555 560
Lys Ser Leu Gly Arg Phe Asp Tyr Trp Gly Gln Gly Thr Leu Val Thr
565 570 575
Val Ser Ser

<210> 152
<211> 579
<212> PRT
<213> Artificial Sequence

<220>
<223> Humanised

<400> 152

```
Glu Val Gln Leu Val Gln Ser Gly Ala Glu Val Lys Lys Pro Gly Glu
1               5                   10                  15
Ser Leu Lys Ile Ser Cys Lys Gly Ser Gly Phe Tyr Ile Lys Asp Thr
            20                  25                  30
Tyr Met His Trp Val Arg Gln Met Pro Gly Lys Gly Leu Glu Trp Met
        35                  40                  45
Gly Thr Ile Asp Pro Ala Asn Gly Asn Thr Lys Tyr Val Pro Lys Phe
    50                  55                  60
Gln Gly Gln Val Thr Ile Ser Ala Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg Ser Ile Tyr Asp Asp Tyr His Tyr Asp Asp Tyr Tyr Ala Met
            100                 105                 110
Asp Tyr Trp Gly Gln Gly Thr Leu Val Thr Val Ser Ser Ala Ser Thr
        115                 120                 125
Lys Gly Pro Ser Val Phe Pro Leu Ala Pro Ser Ser Lys Ser Thr Ser
    130                 135                 140
Gly Gly Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Gln Thr Tyr Ile Cys
        195                 200                 205
Asn Val Asn His Lys Pro Ser Asn Thr Lys Val Asp Lys Lys Val Glu
    210                 215                 220
Pro Lys Ser Cys Asp Lys Thr His Thr Cys Pro Pro Cys Pro Ala Pro
225                 230                 235                 240
Glu Leu Leu Gly Gly Pro Ser Val Phe Leu Phe Pro Pro Lys Pro Lys
                245                 250                 255
Asp Thr Leu Met Ile Ser Arg Thr Pro Glu Val Thr Cys Val Val Val
            260                 265                 270
Asp Val Ser His Glu Asp Pro Glu Val Lys Phe Asn Trp Tyr Val Asp
        275                 280                 285
Gly Val Glu Val His Asn Ala Lys Thr Lys Pro Arg Glu Glu Gln Tyr
    290                 295                 300
Asn Ser Thr Tyr Arg Val Val Ser Val Leu Thr Val Leu His Gln Asp
305                 310                 315                 320
Trp Leu Asn Gly Lys Glu Tyr Lys Cys Lys Val Ser Asn Lys Ala Leu
                325                 330                 335
Pro Ala Pro Ile Glu Lys Thr Ile Ser Lys Ala Lys Gly Gln Pro Arg
            340                 345                 350
Glu Pro Gln Val Tyr Thr Leu Pro Pro Ser Arg Asp Glu Leu Thr Lys
```

```
            355                     360                     365
Asn Gln Val Ser Leu Thr Cys Leu Val Lys Gly Phe Tyr Pro Ser Asp
    370                     375                     380
Ile Ala Val Glu Trp Glu Ser Asn Gly Gln Pro Glu Asn Asn Tyr Lys
385                     390                     395                 400
Thr Thr Pro Pro Val Leu Asp Ser Asp Gly Ser Phe Phe Leu Tyr Ser
                405                     410                     415
Lys Leu Thr Val Asp Lys Ser Arg Trp Gln Gln Gly Asn Val Phe Ser
            420                     425                     430
Cys Ser Val Met His Glu Ala Leu His Asn His Tyr Thr Gln Lys Ser
        435                     440                     445
Leu Ser Leu Ser Pro Gly Lys Thr Val Ala Ala Pro Ser Gly Ser Glu
    450                     455                     460
Val Gln Leu Leu Glu Ser Gly Gly Gly Leu Val Gln Pro Gly Gly Ser
465                     470                     475                 480
Leu Arg Leu Ser Cys Ala Ala Ser Gly Phe Thr Phe Arg Asn Phe Gly
                485                     490                     495
Met Gly Trp Val Arg Gln Ala Pro Gly Lys Gly Leu Glu Trp Val Ser
            500                     505                     510
Trp Ile Ile Ser Ser Gly Thr Glu Thr Tyr Tyr Ala Asp Ser Val Lys
        515                     520                     525
Gly Arg Phe Thr Ile Ser Arg Asp Asn Ser Lys Asn Thr Leu Tyr Leu
    530                     535                     540
Gln Met Asn Ser Leu Arg Ala Glu Asp Thr Ala Val Tyr Tyr Cys Ala
545                     550                     555                 560
Lys Ser Leu Gly Arg Phe Asp Tyr Trp Gly Gln Gly Thr Leu Val Thr
                565                     570                     575
Val Ser Ser
```

<210> 153
<211> 219
<212> PRT
<213> Artificial Sequence

<220>
<223> Humanised

<400> 153

```
Glu Ile Val Leu Thr Gln Ser Pro Ala Thr Leu Ser Leu Ser Pro Gly
1               5                   10                  15
Glu Arg Ala Thr Leu Ser Cys Arg Ser Ser Gln Asn Ile Val His Ile
            20                  25                  30
Asn Gly Asn Thr Tyr Leu Glu Trp Tyr Gln Gln Lys Pro Gly Gln Ala
        35                  40                  45
Pro Arg Leu Leu Ile Tyr Lys Ile Ser Asp Arg Phe Ser Gly Ile Pro
    50                  55                  60
Ala Arg Phe Ser Gly Ser Gly Ser Gly Thr Asp Phe Thr Leu Thr Ile
65                  70                  75                  80
Ser Ser Leu Glu Pro Glu Asp Phe Ala Val Tyr Tyr Cys Phe Gln Gly
                85                  90                  95
Ser His Val Pro Trp Thr Phe Gly Gly Gly Thr Lys Val Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215
```

<210> 154
<211> 219
<212> PRT
<213> Artificial Sequence

<220>
<223> Humanised

<400> 154

Glu Ile Val Leu Thr Gln Ser Pro Ala Thr Leu Ser Leu Ser Pro Gly
1               5                   10                  15
Glu Arg Ala Thr Leu Ser Cys Arg Ser Ser Gln Asn Ile Val His Ile
            20                  25                  30
Asn Gly Asn Thr Tyr Leu Glu Trp Tyr Gln Gln Lys Pro Gly Gln Ala
        35                  40                  45
Pro Arg Leu Leu Ile Tyr Lys Ile Ser Asp Arg Phe Ser Gly Ile Pro
    50                  55                  60
Ala Arg Phe Ser Gly Ser Gly Ser Gly Thr Asp Phe Thr Leu Thr Ile
65                  70                  75                  80
Ser Ser Leu Glu Pro Glu Asp Phe Ala Val Tyr Tyr Cys Phe Gln Gly
                85                  90                  95
Ser His Val Pro Trp Thr Phe Gly Gly Gly Thr Lys Val Glu Ile Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215

<210> 155
<211> 571
<212> PRT
<213> Artificial Sequence

<220>
<223> Humanised

<400> 155

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Ile Asp Tyr
20 25 30
Glu Ile His Trp Val Arg Gln Ala Pro Gly Gln Gly Leu Glu Trp Met
35 40 45
Gly Ala Ile Asp Pro Glu Thr Gly Gly Thr Ala Tyr Asn Gln Lys Phe
50 55 60
Lys Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Thr Arg Ile Leu Leu Tyr Tyr Tyr Pro Met Asp Tyr Trp Gly Gln Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270

```
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Thr Val Ala Ala Pro Ser Glu Val Gln Leu Leu Glu Ser Gly Gly
    450                 455                 460
Gly Leu Val Gln Pro Gly Gly Ser Leu Arg Leu Ser Cys Ala Ala Ser
465                 470                 475                 480
Gly Phe Thr Phe Arg Asn Phe Gly Met Gly Trp Val Arg Gln Ala Pro
                485                 490                 495
Gly Lys Gly Leu Glu Trp Val Ser Trp Ile Ile Ser Ser Gly Thr Glu
            500                 505                 510
Thr Tyr Tyr Ala Asp Ser Val Lys Gly Arg Phe Thr Ile Ser Arg Asp
        515                 520                 525
Asn Ser Lys Asn Thr Leu Tyr Leu Gln Met Asn Ser Leu Arg Ala Glu
    530                 535                 540
Asp Thr Ala Val Tyr Tyr Cys Ala Lys Ser Leu Gly Arg Phe Asp Tyr
545                 550                 555                 560
Trp Gly Gln Gly Thr Leu Val Thr Val Ser Ser
                565                 570
```

<210> 156
<211> 214
<212> PRT
<213> Artificial Sequence

<220>
<223> Humanised

<400> 156

Glu Ile Val Leu Thr Gln Ser Pro Ala Thr Leu Ser Leu Ser Pro Gly
1 5 10 15
Glu Arg Ala Thr Leu Ser Cys Arg Ala Ser Gln Asn Ile Ser Asp Tyr
20 25 30
Leu His Trp Tyr Gln Gln Lys Pro Gly Gln Ala Pro Arg Leu Leu Ile
35 40 45
Tyr Tyr Ala Ser Gln Ser Ile Ser Gly Ile Pro Ala Arg Phe Ser Gly
50 55 60
Ser Gly Ser Gly Thr Asp Phe Thr Leu Thr Ile Ser Ser Leu Glu Pro
65 70 75 80
Glu Asp Phe Ala Val Tyr Tyr Cys Gln Asn Gly His Ser Phe Pro Leu
85 90 95
Thr Phe Gly Gly Gly Thr Lys Val Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys

210

<210> 157
<211> 575
<212> PRT
<213> Artificial Sequence

<220>
<223> Humanised

<400> 157

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Thr Val Ala Ala Pro Ser Gly Val Gln Leu Leu Glu Ser
450 455 460
Gly Gly Gly Leu Val Gln Pro Gly Gly Ser Leu Arg Leu Ser Cys Ala
465 470 475 480
Ala Ser Gly Phe Val Phe Pro Trp Tyr Asp Met Gly Trp Val Arg Gln
485 490 495
Ala Pro Gly Lys Gly Leu Glu Trp Val Ser Ser Ile Asp Trp Lys Gly
500 505 510

```
Gly Lys Thr Tyr Tyr Ala Asp Ser Val Lys Gly Arg Phe Thr Ile Ser
        515                 520                 525
Arg Asp Asn Ser Lys Asn Thr Leu Tyr Leu Gln Met Asn Ser Leu Arg
    530                 535                 540
Ala Glu Asp Thr Ala Val Tyr Tyr Cys Ala Thr Ala Glu Asp Glu Pro
545                 550                 555                 560
Gly Tyr Asp Tyr Trp Gly Gln Gly Thr Leu Val Thr Val Ser Ser
                565                 570                 575
```

<210> 158
<211> 569
<212> PRT
<213> Artificial Sequence

<220>
<223> Humanised

<400> 158

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Gly Val Gln Leu Leu Glu Ser Gly Gly Gly Leu Val Gln
```

```
    450                 455                     460
Pro Gly Gly Ser Leu Arg Leu Ser Cys Ala Ala Ser Gly Phe Val Phe
465                 470                 475                 480
Pro Trp Tyr Asp Met Gly Trp Val Arg Gln Ala Pro Gly Lys Gly Leu
                485                 490                 495
Glu Trp Val Ser Ser Ile Asp Trp Lys Gly Gly Lys Thr Tyr Tyr Ala
            500                 505                 510
Asp Ser Val Lys Gly Arg Phe Thr Ile Ser Arg Asp Asn Ser Lys Asn
        515                 520                 525
Thr Leu Tyr Leu Gln Met Asn Ser Leu Arg Ala Glu Asp Thr Ala Val
    530                 535                 540
Tyr Tyr Cys Ala Thr Ala Glu Asp Glu Pro Gly Tyr Asp Tyr Trp Gly
545                 550                 555                 560
Gln Gly Thr Leu Val Thr Val Ser Ser
                565
```

<210> 159
<211> 575
<212> PRT
<213> Artificial Sequence

<220>
<223> Humanised

<400> 159

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
```

```
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Thr Val Ala Ala Pro Ser Gly Val Gln Leu Leu Glu Ser
    450                 455                 460
Gly Gly Gly Leu Val Gln Pro Gly Gly Ser Leu Arg Leu Ser Cys Ala
465                 470                 475                 480
Ala Ser Gly Phe Val Phe Ala Trp Tyr Asp Met Gly Trp Val Arg Gln
                485                 490                 495
Ala Pro Gly Lys Gly Leu Glu Trp Val Ser Ser Ile Asp Trp His Gly
            500                 505                 510
Glu Val Thr Tyr Tyr Ala Asp Ser Val Lys Gly Arg Phe Thr Ile Ser
        515                 520                 525
Arg Asp Asn Ser Lys Asn Thr Leu Tyr Leu Gln Met Asn Ser Leu Arg
    530                 535                 540
Ala Glu Asp Thr Ala Val Tyr Tyr Cys Ala Thr Ala Glu Asp Glu Pro
545                 550                 555                 560
Gly Tyr Asp Tyr Trp Gly Gln Gly Thr Leu Val Thr Val Ser Ser
                565                 570                 575
```

<210> 160
<211> 569
<212> PRT
<213> Artificial Sequence

<220>
<223> Humanised

<400> 160

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val

```
                340                     345                     350
Tyr Thr Leu Pro Pro Ser Arg Asp Glu Leu Thr Lys Asn Gln Val Ser
            355                     360                     365
Leu Thr Cys Leu Val Lys Gly Phe Tyr Pro Ser Asp Ile Ala Val Glu
        370                     375                     380
Trp Glu Ser Asn Gly Gln Pro Glu Asn Asn Tyr Lys Thr Thr Pro Pro
385                     390                     395                 400
Val Leu Asp Ser Asp Gly Ser Phe Phe Leu Tyr Ser Lys Leu Thr Val
                405                     410                     415
Asp Lys Ser Arg Trp Gln Gln Gly Asn Val Phe Ser Cys Ser Val Met
            420                     425                     430
His Glu Ala Leu His Asn His Tyr Thr Gln Lys Ser Leu Ser Leu Ser
        435                     440                     445
Pro Gly Lys Gly Val Gln Leu Leu Glu Ser Gly Gly Gly Leu Val Gln
    450                     455                     460
Pro Gly Gly Ser Leu Arg Leu Ser Cys Ala Ala Ser Gly Phe Val Phe
465                     470                     475                 480
Ala Trp Tyr Asp Met Gly Trp Val Arg Gln Ala Pro Gly Lys Gly Leu
                485                     490                     495
Glu Trp Val Ser Ser Ile Asp Trp His Gly Glu Val Thr Tyr Tyr Ala
            500                     505                     510
Asp Ser Val Lys Gly Arg Phe Thr Ile Ser Arg Asp Asn Ser Lys Asn
        515                     520                     525
Thr Leu Tyr Leu Gln Met Asn Ser Leu Arg Ala Glu Asp Thr Ala Val
    530                     535                     540
Tyr Tyr Cys Ala Thr Ala Glu Asp Glu Pro Gly Tyr Asp Tyr Trp Gly
545                     550                     555                 560
Gln Gly Thr Leu Val Thr Val Ser Ser
                565
```

<210> 161
<211> 449
<212> PRT
<213> Artificial Sequence

<220>
<223> Humanised

<400> 161

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Ile Asp Tyr
20 25 30
Glu Ile His Trp Val Arg Gln Ala Pro Gly Gln Gly Leu Glu Trp Met
35 40 45
Gly Ala Ile Asp Pro Glu Thr Gly Gly Thr Ala Tyr Asn Gln Lys Phe
50 55 60
Lys Gly Arg Val Thr Met Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Thr Arg Ile Leu Leu Tyr Tyr Tyr Pro Met Asp Tyr Trp Gly Gln Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys

<210> 162
<211> 1707
<212> DNA

<213> Artificial Sequence

<220>
<223> Humanised

<400> 162

```
gaggtgcagc tgttggagtc tgggggaggc ttggtacagc ctggggggtc cctgcgtctc 60
tcctgtgcag cctccggatt cacctttggg gcttatccga tgatgtgggt ccgccaggct 120
ccagggaagg gtctagagtg ggtctcagag atttcgcctt cgggttctta tacatactac 180
gcagactccg tgaagggccg gttcaccatc tcccgcgaca attccaagaa cacgctgtat 240
ctgcaaatga acagcctgcg tgccgaggac accgcggtat attactgtgc gaaagatcct 300
cggaagtttg actactgggg tcagggaacc ctggtcaccg tctcgagcgc tagcaccaag 360
ggccccagcg aggtgcagct gttggagtct gggggaggct tggtacagcc tggggggtcc 420
ctgcgtctct cctgtgcagc ctccggattc acctttagca gctatgccat gagctgggtc 480
cgccaggctc cagggaaggg tctagagtgg gtctcagcta ttagtggtag tggtggtagc 540
acatactacg cagactccgt gaagggccgg ttcaccatct cccgcgacaa ttccaagaac 600
acgctgtatc tgcaaatgaa cagcctgcgt gccgaggaca ccgcggtata ttactgtgcg 660
aaaagttatg gtgcttttga ctactggggc cagggaaccc tggtcaccgt ctcgagcgct 720
agcaccaagg gccccagcgt gttccccctg gcccccagca gcaagagcac cagcggcggc 780
acagccgccc tgggctgcct ggtgaaggac tacttccccg agcctgtgac cgtgtcctgg 840
aatagcggag ccctgacctc cggcgtgcac accttccccg ccgtgctgca gagcagcggc 900
ctgtactccc tgagcagcgt ggtgaccgtg cccagcagca gcctgggcac ccagacctac 960
atctgcaacg tgaaccacaa gcccagcaac accaaagtgg acaagaaagt ggagcccaag 1020
agctgcgata agacccacac ctgccccccc tgccctgccc ccgagctgct gggcggacct 1080
agcgtgttcc tgttcccccc caagcctaag gacaccctga tgatcagcag gacccccgaa 1140
gtgacctgcg tggtggtgga tgtgagccac gaggaccctg aagtgaagtt caactggtac 1200
gtggacggcg tggaagtgca caacgccaag accaagccca gagaggagca gtacaacagc 1260
acctaccgcg tggtgtctgt gctgaccgtg ctgcaccagg attggctgaa cggcaaggag 1320
tacaagtgca aagtgagcaa caaggccctg cctgccccta tcgagaaaac catcagcaag 1380
gccaagggcc agcctagaga gccccaggtc tacaccctgc ctccctccag agatgagctg 1440
accaagaacc aggtgtccct gacctgtctg gtgaagggct tctaccccag cgacatcgcc 1500
gtggagtggg agagcaacgg ccagcccgag aacaactaca agaccacccc ccctgtgctg 1560
gacagcgatg gcagcttctt cctgtactcc aagctgaccg tggacaagag cagatggcag 1620
cagggcaacg tgttcagctg cagcgtgatg cacgaggccc tgcacaatca ctacacccag 1680
aagagtctga gcctgtcccc tggcaag                                     1707
```

<210> 163
<211> 569
<212> PRT
<213> Artificial Sequence

<220>
<223> Humanised

<400> 163

```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Gly Ala Tyr
            20                  25                  30
```

```
Pro Met Met Trp Val Arg Gln Ala Pro Gly Lys Gly Leu Glu Trp Val
        35                  40                  45
Ser Glu Ile Ser Pro Ser Gly Ser Tyr Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Pro Arg Lys Phe Asp Tyr Trp Gly Gln Gly Thr Leu Val
            100                 105                 110
Thr Val Ser Ser Ala Ser Thr Lys Gly Pro Ser Glu Val Gln Leu Leu
        115                 120                 125
Glu Ser Gly Gly Gly Leu Val Gln Pro Gly Gly Ser Leu Arg Leu Ser
    130                 135                 140
Cys Ala Ala Ser Gly Phe Thr Phe Ser Ser Tyr Ala Met Ser Trp Val
145                 150                 155                 160
Arg Gln Ala Pro Gly Lys Gly Leu Glu Trp Val Ser Ala Ile Ser Gly
                165                 170                 175
Ser Gly Gly Ser Thr Tyr Tyr Ala Asp Ser Val Lys Gly Arg Phe Thr
            180                 185                 190
Ile Ser Arg Asp Asn Ser Lys Asn Thr Leu Tyr Leu Gln Met Asn Ser
        195                 200                 205
Leu Arg Ala Glu Asp Thr Ala Val Tyr Tyr Cys Ala Lys Ser Tyr Gly
    210                 215                 220
Ala Phe Asp Tyr Trp Gly Gln Gly Thr Leu Val Thr Val Ser Ser Ala
225                 230                 235                 240
Ser Thr Lys Gly Pro Ser Val Phe Pro Leu Ala Pro Ser Ser Lys Ser
                245                 250                 255
Thr Ser Gly Gly Thr Ala Ala Leu Gly Cys Leu Val Lys Asp Tyr Phe
            260                 265                 270
Pro Glu Pro Val Thr Val Ser Trp Asn Ser Gly Ala Leu Thr Ser Gly
        275                 280                 285
Val His Thr Phe Pro Ala Val Leu Gln Ser Ser Gly Leu Tyr Ser Leu
    290                 295                 300
Ser Ser Val Val Thr Val Pro Ser Ser Ser Leu Gly Thr Gln Thr Tyr
305                 310                 315                 320
Ile Cys Asn Val Asn His Lys Pro Ser Asn Thr Lys Val Asp Lys Lys
                325                 330                 335
Val Glu Pro Lys Ser Cys Asp Lys Thr His Thr Cys Pro Pro Cys Pro
            340                 345                 350
Ala Pro Glu Leu Leu Gly Gly Pro Ser Val Phe Leu Phe Pro Pro Lys
        355                 360                 365
Pro Lys Asp Thr Leu Met Ile Ser Arg Thr Pro Glu Val Thr Cys Val
    370                 375                 380
Val Val Asp Val Ser His Glu Asp Pro Glu Val Lys Phe Asn Trp Tyr
385                 390                 395                 400
Val Asp Gly Val Glu Val His Asn Ala Lys Thr Lys Pro Arg Glu Glu
                405                 410                 415
Gln Tyr Asn Ser Thr Tyr Arg Val Val Ser Val Leu Thr Val Leu His
            420                 425                 430
Gln Asp Trp Leu Asn Gly Lys Glu Tyr Lys Cys Lys Val Ser Asn Lys
        435                 440                 445
Ala Leu Pro Ala Pro Ile Glu Lys Thr Ile Ser Lys Ala Lys Gly Gln
    450                 455                 460
Pro Arg Glu Pro Gln Val Tyr Thr Leu Pro Pro Ser Arg Asp Glu Leu
465                 470                 475                 480
Thr Lys Asn Gln Val Ser Leu Thr Cys Leu Val Lys Gly Phe Tyr Pro
                485                 490                 495
Ser Asp Ile Ala Val Glu Trp Glu Ser Asn Gly Gln Pro Glu Asn Asn
            500                 505                 510
Tyr Lys Thr Thr Pro Pro Val Leu Asp Ser Asp Gly Ser Phe Phe Leu
        515                 520                 525
Tyr Ser Lys Leu Thr Val Asp Lys Ser Arg Trp Gln Gln Gly Asn Val
    530                 535                 540
Phe Ser Cys Ser Val Met His Glu Ala Leu His Asn His Tyr Thr Gln
545                 550                 555                 560
Lys Ser Leu Ser Leu Ser Pro Gly Lys
                565
```

<210> 164
<211> 1704
<212> DNA
<213> Artificial Sequence

<220>
<223> Humanised

<400> 164

```
caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
accccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttcccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgcccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaagtcg accggtgagg tgcagctgtt ggtgtctggg 1380
ggaggcttgg tacagcctgg ggggtccctg cgtctctcct gtgcagcctc cggattcacc 1440
tttaaggctt atccgatgat gtgggtccgc caggctccag ggaagggtct agagtgggtt 1500
tcagagattt cgccttcggg ttcttataca tactacgcag actccgtgaa gggccggttc 1560
accatctccc gcgacaattc caagaacacg ctgtatctgc aaatgaacag cctgcgtgcc 1620
gaggacaccg cggtatatta ctgtgcgaaa gatcctcgga agttagacta ctggggtcag 1680
ggaaccctgg tcaccgtctc gagc 1704
```

<210> 165
<211> 568
<212> PRT
<213> Artificial Sequence

<220>
<223> Humanised

<400> 165

Gln Val Gln Leu Lys Gln Ser Gly Pro Gly Leu Val Gln Pro Ser Gln
1 5 10 15
Ser Leu Ser Ile Thr Cys Thr Val Ser Gly Phe Ser Leu Thr Asn Tyr
20 25 30
Gly Val His Trp Val Arg Gln Ser Pro Gly Lys Gly Leu Glu Trp Leu
35 40 45
Gly Val Ile Trp Ser Gly Gly Asn Thr Asp Tyr Asn Thr Pro Phe Thr
50 55 60
Ser Arg Leu Ser Ile Asn Lys Asp Asn Ser Lys Ser Gln Val Phe Phe
65 70 75 80
Lys Met Asn Ser Leu Gln Ser Asn Asp Thr Ala Ile Tyr Tyr Cys Ala
85 90 95
Arg Ala Leu Thr Tyr Tyr Asp Tyr Glu Phe Ala Tyr Trp Gly Gln Gly
100 105 110
Thr Leu Val Thr Val Ser Ala Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys Ser Thr Gly Glu Val Gln Leu Leu Val Ser Gly Gly Gly Leu Val
450 455 460
Gln Pro Gly Gly Ser Leu Arg Leu Ser Cys Ala Ala Ser Gly Phe Thr
465 470 475 480
Phe Lys Ala Tyr Pro Met Met Trp Val Arg Gln Ala Pro Gly Lys Gly
485 490 495
Leu Glu Trp Val Ser Glu Ile Ser Pro Ser Gly Ser Tyr Thr Tyr Tyr
500 505 510
Ala Asp Ser Val Lys Gly Arg Phe Thr Ile Ser Arg Asp Asn Ser Lys
515 520 525
Asn Thr Leu Tyr Leu Gln Met Asn Ser Leu Arg Ala Glu Asp Thr Ala
530 535 540
Val Tyr Tyr Cys Ala Lys Asp Pro Arg Lys Leu Asp Tyr Trp Gly Gln
545 550 555 560
Gly Thr Leu Val Thr Val Ser Ser
565

<210> 166
<211> 451
<212> PRT
<213> Artificial Sequence

<220>
<223> Humanised

<400> 166

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Pro Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Arg Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Glu Glu Met Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys
450

<210> 167
<211> 1737
<212> DNA
<213> Artificial Sequence

<220>
<223> Humanised

<400> 167

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ccagcgccag caccaagggc cccagcgtgt tccccctggc ccccagcagc 420
aagagcacca gcggcggcac agccgccctg ggctgcctgg tgaaggacta cttccccgaa 480
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaagaccgt ggccgccccc 1380
tcgggatccg aagtgcagct cctggagagc ggcggcggcc tggtgcagcc cggcggcagc 1440
ctgaggctga gctgcgccgc tagcggcttc accttcagga acttcggcat gggctgggtc 1500
aggcaggccc ccggcaaggg cctggagtgg gtcagctgga tcatcagctc cggcaccgag 1560
acctactacg ccgacagcgt gaagggcagg ttcaccatca gccgcgacaa cagcaagaac 1620
accctgtacc tgcagatgaa cagcctgagg gccgaggaca ccgccgtcta ctactgcgcc 1680
aagagcctgg gcaggttcga ctactgggga caggggaccc tggtgactgt gagcagc    1737
```

<210> 168
<211> 1710
<212> DNA
<213> Artificial Sequence

<220>
<223> Humanised

<400> 168

```
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcaccctggt gacagtctcg 360
agcgctagca ccaagggccc cagcgtgttc cccctggccc ccagcagcaa gagcaccagc 420
ggcggcacag ccgccctggg ctgcctggtg aaggactact tccccgagcc tgtgaccgtg 480
tcctggaata gcggagccct gacctccggc gtgcacacct tccccgccgt gctgcagagc 540
agcggcctgt actccctgag cagcgtggtg accgtgccca gcagcagcct gggcacccag 600
acctacatct gcaacgtgaa ccacaagccc agcaacacca aagtggacaa gaaagtggag 660
cccaagagct gcgataagac ccacacctgc ccccctgcc ctgcccccga gctgctgggc 720
ggacctagcg tgttcctgtt cccccccaag cctaaggaca ccctgatgat cagcaggacc 780
cccgaagtga cctgcgtggt ggtggatgtg agccacgagg accctgaagt gaagttcaac 840
tggtacgtgg acggcgtgga agtgcacaac gccaagacca agcccagaga ggagcagtac 900
aacagcacct accgcgtggt gtctgtgctg accgtgctgc accaggattg gctgaacggc 960
aaggagtaca agtgcaaagt gagcaacaag gccctgcctg cccctatcga gaaaaccatc 1020
agcaaggcca agggccagcc tagagagccc caggtctaca ccctgcctcc ctccagagat 1080
gagctgacca agaaccaggt gtccctgacc tgtctggtga agggcttcta ccccagcgac 1140
atcgccgtgg agtgggagag caacggccag cccgagaaca actacaagac cacccccccct 1200
gtgctggaca gcgatggcag cttcttcctg tactccaagc tgaccgtgga caagagcaga 1260
tggcagcagg gcaacgtgtt cagctgcagc gtgatgcacg aggccctgca caatcactac 1320
acccagaaga gtctgagcct gtcccctggc aagtcgaccg gtgaggtgca gctgttggtg 1380
tctgggggag gcttggtaca gcctgggggg tccctgcgtc tctcctgtgc agcctccgga 1440
ttcaccttta aggcttatcc gatgatgtgg gtccgccagg ctccagggaa gggtctagag 1500
tgggtttcag agatttcgcc ttcgggttct tatacatact acgcagactc cgtgaagggc 1560
cggttcacca tctcccgcga caattccaag aacacgctgt atctgcaaat gaacagcctg 1620
cgtgccgagg acaccgcggt atattactgt gcgaaagatc ctcggaagtt agactactgg 1680
ggtcagggaa ccctggtcac cgtctcgagc 1710
```

<210> 169
<211> 642
<212> DNA
<213> Artificial Sequence

<220>
<223> Humanised

<400> 169

```
gatatccaga tgacccagag ccccagcagc ctgagcgcct ctgtgggcga tagagtgacc 60
atcacctgcc gggccagcca gggcatcaga aactacctgg cctggtatca gcagaagcct 120
ggcaaggccc ctaagctgct gatctacgcc gccagcaccc tgcagagcgg cgtgcccagc 180
agattcagcg gcagcggctc cggcaccgac ttcaccctga ccatcagcag cctgcagccc 240
gaggacgtgg ccacctacta ctgccagcgg tacaacagag ccccttacac cttcggccag 300
ggcaccaagg tggagatcaa gcgtacggtg gccgccccca gcgtgttcat cttccccccc 360
agcgatgagc agctcaagag cggcaccgcc agcgtggtgt gtctgctgaa caacttctac 420
ccccgggagg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cggggcgagt gc 642
```

<210> 170
<211> 1686
<212> DNA
<213> Artificial Sequence

<220>
<223> Humanised

<400> 170

```
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcaccctggt gacagtctcg 360
agcgctagca ccaagggccc cagcgtgttc cccctggccc ccagcagcaa gagcaccagc 420
ggcggcacag ccgccctggg ctgcctggtg aaggactact tccccgagcc tgtgaccgtg 480
tcctggaata gcggagccct gacctccggc gtgcacacct tccccgccgt gctgcagagc 540
agcggcctgt actccctgag cagcgtggtg accgtgccca gcagcagcct gggcacccag 600
acctacatct gcaacgtgaa ccacaagccc agcaacacca aagtggacaa gaaagtggag 660

cccaagagct gcgataagac ccacacctgc ccccctgcc ctgcccccga gctgctgggc 720
ggacctagcg tgttcctgtt cccccccaag cctaaggaca ccctgatgat cagcaggacc 780
cccgaagtga cctgcgtggt ggtggatgtg agccacgagg accctgaagt gaagttcaac 840
tggtacgtgg acggcgtgga agtgcacaac gccaagacca agcccagaga ggagcagtac 900
aacagcacct accgcgtggt gtctgtgctg accgtgctgc accaggattg gctgaacggc 960
aaggagtaca agtgcaaagt gagcaacaag gccctgcctg cccctatcga gaaaaccatc 1020
agcaaggcca agggccagcc tagagagccc caggtctaca ccctgcctcc ctccagagat 1080
gagctgacca agaaccaggt gtccctgacc tgtctggtga agggcttcta ccccagcgac 1140
atcgccgtgg agtgggagag caacggccag cccgagaaca actacaagac cacccccct 1200
gtgctggaca gcgatggcag cttcttcctg tactccaagc tgaccgtgga caagagcaga 1260
tggcagcagg gcaacgtgtt cagctgcagc gtgatgcacg aggccctgca caatcactac 1320
acccagaaga gtctgagcct gtcccctggc aagtcgaccg gtgacatcca gatgacccag 1380
agcccttcaa gcctgagcgc cagcgtgggc gacagagtga ccatcacctg ccgggccagc 1440
cagtggatcg gcaacctgct ggactggtat cagcagaagc ccggcaaggc ccccaagctg 1500
ctgatctact acgccagctt cctgcagagc ggcgtgccca gccggtttag cggcagcggc 1560
tacggcaccg acttcaccct gaccatcagc agcctgcagc ccgaggactt cgccacctac 1620
tactgccagc aggccaaccc tgcccccctg accttcggcc agggtaccaa ggtggaaatc 1680
aaacgg 1686
```

<210> 171
<211> 984
<212> DNA
<213> Artificial Sequence

<220>
<223> Humanised

<400> 171

```
gacatccaga tgacccagtc tccatcctcc ctgtctgcat ctgtaggaga ccgtgtcacc 60
atcacttgcc gggcaagtca ggatatttac ctgaatttag actggtatca gcagaaacca 120
gggaaagccc ctaagctcct gatcaatttt ggttccgagt tgcaaagtgg tgtcccatca 180
cgtttcagtg gcagtggata tgggacagat ttcactctca ccatcagcag tctgcaacct 240
gaagatttcg ctacgtacta ctgtcaaccg tctttttact tcccttatac gttcggccaa 300
gggaccaagg tggaaatcaa acgtacggtg gccgccccca gcgacatcca gatgacccag 360
tctccatcct ccctgtctgc atctgtagga gaccgtgtca ccatcacttg ccgggcaagt 420
cagagcatta gcagctattt aaattggtac cagcagaaac cagggaaagc ccctaagctc 480
ctgatctatg ctgcatccag tttgcaaagt ggggtcccat cacgtttcag tggcagtgga 540
tctgggacag atttcactct caccatcagc agtctgcaac ctgaagattt tgctacgtac 600
tactgtcaac agagttacag taccccctaat acgttcggcc aagggaccaa ggtggaaatc 660
aaacgtacgg tggccgcccc cagcgtgttc atcttccccc ccagcgatga gcagctcaag 720
agcggcaccg ccagcgtggt gtgtctgctg aacaacttct acccccggga ggccaaagtg 780
cagtggaaag tggacaacgc cctgcagagc ggcaacagcc aggagagcgt gaccgagcag 840
gacagcaagg actccaccta cagcctgagc agcaccctga ccctgagcaa ggccgactac 900
gagaagcaca aagtgtacgc ctgcgaagtg acccaccagg gcctgtccag ccccgtgacc 960
aagagcttca accggggcga gtgc 984
```

<210> 172
<211> 1707

<212> DNA
<213> Artificial Sequence

<220>
<223> Humanised

<400> 172

```
gaggtgcagc tgttggtgtc tgggggaggc ttggtacagc ctggggggtc cctgcgtctc 60
tcctgtgcag cctccggatt cacctttaag gcttatccga tgatgtgggt ccgccaggct 120
ccagggaagg gtctagagtg ggtttcagag atttcgcctt cgggttctta tacatactac 180
gcagactccg tgaagggccg gttcaccatc tcccgcgaca attccaagaa cacgctgtat 240
ctgcaaatga acagcctgcg tgccgaggac accgcggtat attactgtgc gaaagatcct 300
cggaagttag actactgggg tcagggaacc ctggtcaccg tctcgagcgc tagcaccaag 360
ggccccagcg aggtgcagct gttggagtct gggggaggct tggtacagcc tggggggtcc 420
ctgcgtctct cctgtgcagc ctccggattc acctttagca gctatgccat gagctgggtc 480
cgccaggctc cagggaaggg tctagagtgg gtctcagcta ttagtggtag tggtggtagc 540
acatactacg cagactccgt gaagggccgg ttcaccatct cccgcgacaa ttccaagaac 600
acgctgtatc tgcaaatgaa cagcctgcgt gccgaggaca ccgcggtata ttactgtgcg 660
aaaagttatg gtgcttttga ctactggggc cagggaaccc tggtcaccgt ctcgagcgct 720
agcaccaagg gccccagcgt gttccccctg gcccccagca gcaagagcac cagcggcggc 780
acagccgccc tgggctgcct ggtgaaggac tacttccccg agcctgtgac cgtgtcctgg 840
aatagcggag ccctgacctc cggcgtgcac accttccccg ccgtgctgca gagcagcggc 900
ctgtactccc tgagcagcgt ggtgaccgtg cccagcagca gcctgggcac ccagacctac 960
atctgcaacg tgaaccacaa gcccagcaac accaaagtgg acaagaaagt ggagcccaag 1020
agctgcgata agacccacac ctgccccccc tgccctgccc ccgagctgct gggcggacct 1080
agcgtgttcc tgttcccccc caagcctaag gacaccctga tgatcagcag gacccccgaa 1140
gtgacctgcg tggtggtgga tgtgagccac gaggaccctg aagtgaagtt caactggtac 1200
gtggacggcg tggaagtgca caacgccaag accaagccca gagaggagca gtacaacagc 1260

acctaccgcg tggtgtctgt gctgaccgtg ctgcaccagg attggctgaa cggcaaggag 1320
tacaagtgca aagtgagcaa caaggccctg cctgccccta tcgagaaaac catcagcaag 1380
gccaagggcc agcctagaga gccccaggtc tacaccctgc ctccctccag agatgagctg 1440
accaagaacc aggtgtccct gacctgtctg gtgaagggct tctaccccag cgacatcgcc 1500
gtggagtggg agagcaacgg ccagcccgag aacaactaca agaccacccc ccctgtgctg 1560
gacagcgatg gcagcttctt cctgtactcc aagctgaccg tggacaagag cagatggcag 1620
cagggcaacg tgttcagctg cagcgtgatg cacgaggccc tgcacaatca ctacacccag 1680
aagagtctga gcctgtcccc tggcaag 1707
```

<210> 173
<211> 1695
<212> DNA
<213> Artificial Sequence

<220>
<223> Humanised

<400> 173

```
caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
acccccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttccccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaaggag gtgcagctgt tggtgtctgg gggaggcttg 1380
gtacagcctg gggggtccct gcgtctctcc tgtgcagcct ccggattcac ctttaaggct 1440
tatccgatga tgtgggtccg ccaggctcca gggaagggtc tagagtgggt ttcagagatt 1500
tcgccttcgg gttcttatac atactacgca gactccgtga agggccggtt caccatctcc 1560
cgcgacaatt ccaagaacac gctgtatctg caaatgaaca gcctgcgtgc cgaggacacc 1620
gcggtatatt actgtgcgaa agatcctcgg aagttagact actggggtca gggaaccctg 1680
gtcaccgtct cgagc                                                  1695
```

<210> 174
<211> 565
<212> PRT
<213> Artificial Sequence

<220>
<223> Humanised

<400> 174

```
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Asn Tyr
            20                  25                  30
Gly Val His Trp Val Arg Gln Ser Pro Gly Lys Gly Leu Glu Trp Leu
        35                  40                  45
Gly Val Ile Trp Ser Gly Gly Asn Thr Asp Tyr Asn Thr Pro Phe Thr
    50                  55                  60
Ser Arg Leu Ser Ile Asn Lys Asp Asn Ser Lys Ser Gln Val Phe Phe
65                  70                  75                  80
Lys Met Asn Ser Leu Gln Ser Asn Asp Thr Ala Ile Tyr Tyr Cys Ala
                85                  90                  95
Arg Ala Leu Thr Tyr Tyr Asp Tyr Glu Phe Ala Tyr Trp Gly Gln Gly
            100                 105                 110
Thr Leu Val Thr Val Ser Ala Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
```

```
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Glu Val Gln Leu Leu Val Ser Gly Gly Gly Leu Val Gln Pro Gly
    450                 455                 460
Gly Ser Leu Arg Leu Ser Cys Ala Ala Ser Gly Phe Thr Phe Lys Ala
465                 470                 475                 480
Tyr Pro Met Met Trp Val Arg Gln Ala Pro Gly Lys Gly Leu Glu Trp
                485                 490                 495
Val Ser Glu Ile Ser Pro Ser Gly Ser Tyr Thr Tyr Tyr Ala Asp Ser
            500                 505                 510
Val Lys Gly Arg Phe Thr Ile Ser Arg Asp Asn Ser Lys Asn Thr Leu
        515                 520                 525
Tyr Leu Gln Met Asn Ser Leu Arg Ala Glu Asp Thr Ala Val Tyr Tyr
    530                 535                 540
Cys Ala Lys Asp Pro Arg Lys Leu Asp Tyr Trp Gly Gln Gly Thr Leu
545                 550                 555                 560
Val Thr Val Ser Ser
                565
```

<210> 175
<211> 1719
<212> DNA
<213> Artificial Sequence

<220>
<223> Humanised

<400> 175

caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
accccccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttccccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720

agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaagtcg accggtggtg gaggtggatc agaggtgcag 1380
ctgttggtgt ctgggggagg cttggtacag cctggggggt ccctgcgtct ctcctgtgca 1440
gcctccggat tcacctttaa ggcttatccg atgatgtggg tccgccaggc tccagggaag 1500
ggtctagagt gggtttcaga gatttcgcct tcgggttctt atacatacta cgcagactcc 1560
gtgaagggcc ggttcaccat ctcccgcgac aattccaaga acacgctgta tctgcaaatg 1620
aacagcctgc gtgccgagga caccgcggta tattactgtg cgaaagatcc tcggaagtta 1680
gactactggg gtcagggaac cctggtcacc gtctcgagc 1719

<210> 176
<211> 573
<212> PRT
<213> Artificial Sequence

<220>
<223> Humanised

<400> 176

```
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Asn Tyr
            20                  25                  30
Gly Val His Trp Val Arg Gln Ser Pro Gly Lys Gly Leu Glu Trp Leu
        35                  40                  45
Gly Val Ile Trp Ser Gly Gly Asn Thr Asp Tyr Asn Thr Pro Phe Thr
    50                  55                  60
Ser Arg Leu Ser Ile Asn Lys Asp Asn Ser Lys Ser Gln Val Phe Phe
65                  70                  75                  80
Lys Met Asn Ser Leu Gln Ser Asn Asp Thr Ala Ile Tyr Tyr Cys Ala
                85                  90                  95
Arg Ala Leu Thr Tyr Tyr Asp Tyr Glu Phe Ala Tyr Trp Gly Gln Gly
            100                 105                 110
Thr Leu Val Thr Val Ser Ala Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
```

```
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Ser Thr Gly Gly Gly Gly Gly Ser Glu Val Gln Leu Leu Val Ser
    450                 455                 460
Gly Gly Gly Leu Val Gln Pro Gly Gly Ser Leu Arg Leu Ser Cys Ala
465                 470                 475                 480
Ala Ser Gly Phe Thr Phe Lys Ala Tyr Pro Met Met Trp Val Arg Gln
                485                 490                 495
Ala Pro Gly Lys Gly Leu Glu Trp Val Ser Glu Ile Ser Pro Ser Gly
            500                 505                 510
Ser Tyr Thr Tyr Tyr Ala Asp Ser Val Lys Gly Arg Phe Thr Ile Ser
        515                 520                 525
Arg Asp Asn Ser Lys Asn Thr Leu Tyr Leu Gln Met Asn Ser Leu Arg
    530                 535                 540
Ala Glu Asp Thr Ala Val Tyr Tyr Cys Ala Lys Asp Pro Arg Lys Leu
545                 550                 555                 560
Asp Tyr Trp Gly Gln Gly Thr Leu Val Thr Val Ser Ser
                565                 570
```

<210> 177
<211> 1734
<212> DNA
<213> Artificial Sequence

<220>
<223> Humanised

<400> 177

```
caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
accccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttcccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaagtcg accggtggtg gaggtggatc aggtggaggt 1380
ggatcagagg tgcagctgtt ggtgtctggg ggaggcttgg tacagcctgg ggggtccctg 1440
cgtctctcct gtgcagcctc cggattcacc tttaaggctt atccgatgat gtgggtccgc 1500
caggctccag ggaagggtct agagtgggtt tcagagattt cgccttcggg ttcttataca 1560
tactacgcag actccgtgaa gggccggttc accatctccc gcgacaattc caagaacacg 1620
ctgtatctgc aaatgaacag cctgcgtgcc gaggacaccg cggtatatta ctgtgcgaaa 1680
gatcctcgga agttagacta ctggggtcag ggaaccctgg tcaccgtctc gagc       1734
```

<210> 178
<211> 578
<212> PRT
<213> Artificial Sequence

<220>
<223> Humanised

<400> 178

```
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Asn Tyr
```

```
                20                      25                      30
Gly Val His Trp Val Arg Gln Ser Pro Gly Lys Gly Leu Glu Trp Leu
            35                      40                      45
Gly Val Ile Trp Ser Gly Gly Asn Thr Asp Tyr Asn Thr Pro Phe Thr
    50                      55                      60
Ser Arg Leu Ser Ile Asn Lys Asp Asn Ser Lys Ser Gln Val Phe Phe
65                      70                      75                      80
Lys Met Asn Ser Leu Gln Ser Asn Asp Thr Ala Ile Tyr Tyr Cys Ala
                85                      90                      95
Arg Ala Leu Thr Tyr Tyr Asp Tyr Glu Phe Ala Tyr Trp Gly Gln Gly
                100                     105                     110
Thr Leu Val Thr Val Ser Ala Ala Ser Thr Lys Gly Pro Ser Val Phe
            115                     120                     125
Pro Leu Ala Pro Ser Ser Lys Ser Thr Ser Gly Gly Thr Ala Ala Leu
    130                     135                     140
Gly Cys Leu Val Lys Asp Tyr Phe Pro Glu Pro Val Thr Val Ser Trp
145                     150                     155                     160
Asn Ser Gly Ala Leu Thr Ser Gly Val His Thr Phe Pro Ala Val Leu
                165                     170                     175
Gln Ser Ser Gly Leu Tyr Ser Leu Ser Ser Val Val Thr Val Pro Ser
                180                     185                     190
Ser Ser Leu Gly Thr Gln Thr Tyr Ile Cys Asn Val Asn His Lys Pro
            195                     200                     205
Ser Asn Thr Lys Val Asp Lys Lys Val Glu Pro Lys Ser Cys Asp Lys
    210                     215                     220
Thr His Thr Cys Pro Pro Cys Pro Ala Pro Glu Leu Leu Gly Gly Pro
225                     230                     235                     240
Ser Val Phe Leu Phe Pro Pro Lys Pro Lys Asp Thr Leu Met Ile Ser
                245                     250                     255
Arg Thr Pro Glu Val Thr Cys Val Val Val Asp Val Ser His Glu Asp
                260                     265                     270
Pro Glu Val Lys Phe Asn Trp Tyr Val Asp Gly Val Glu Val His Asn
            275                     280                     285
Ala Lys Thr Lys Pro Arg Glu Glu Gln Tyr Asn Ser Thr Tyr Arg Val
    290                     295                     300
Val Ser Val Leu Thr Val Leu His Gln Asp Trp Leu Asn Gly Lys Glu
305                     310                     315                     320
Tyr Lys Cys Lys Val Ser Asn Lys Ala Leu Pro Ala Pro Ile Glu Lys
                325                     330                     335
Thr Ile Ser Lys Ala Lys Gly Gln Pro Arg Glu Pro Gln Val Tyr Thr
                340                     345                     350
Leu Pro Pro Ser Arg Asp Glu Leu Thr Lys Asn Gln Val Ser Leu Thr
            355                     360                     365
Cys Leu Val Lys Gly Phe Tyr Pro Ser Asp Ile Ala Val Glu Trp Glu
    370                     375                     380
Ser Asn Gly Gln Pro Glu Asn Asn Tyr Lys Thr Thr Pro Pro Val Leu
385                     390                     395                     400
Asp Ser Asp Gly Ser Phe Phe Leu Tyr Ser Lys Leu Thr Val Asp Lys
                405                     410                     415
Ser Arg Trp Gln Gln Gly Asn Val Phe Ser Cys Ser Val Met His Glu
                420                     425                     430
Ala Leu His Asn His Tyr Thr Gln Lys Ser Leu Ser Leu Ser Pro Gly
            435                     440                     445
Lys Ser Thr Gly Gly Gly Gly Gly Ser Gly Gly Gly Gly Ser Glu Val
    450                     455                     460
Gln Leu Leu Val Ser Gly Gly Gly Leu Val Gln Pro Gly Gly Ser Leu
465                     470                     475                     480
Arg Leu Ser Cys Ala Ala Ser Gly Phe Thr Phe Lys Ala Tyr Pro Met
                485                     490                     495
Met Trp Val Arg Gln Ala Pro Gly Lys Gly Leu Glu Trp Val Ser Glu
                500                     505                     510
Ile Ser Pro Ser Gly Ser Tyr Thr Tyr Tyr Ala Asp Ser Val Lys Gly
            515                     520                     525
Arg Phe Thr Ile Ser Arg Asp Asn Ser Lys Asn Thr Leu Tyr Leu Gln
    530                     535                     540
Met Asn Ser Leu Arg Ala Glu Asp Thr Ala Val Tyr Tyr Cys Ala Lys
545                     550                     555                     560
Asp Pro Arg Lys Leu Asp Tyr Trp Gly Gln Gly Thr Leu Val Thr Val
                565                     570                     575
Ser Ser
```

<210> 179
<211> 1692
<212> DNA
<213> Artificial Sequence

<220>
<223> Humanised

<400> 179

```
gaggtgcagc tggtcgagtc tggcggcgga ctggtgcagc ctggcggcag cctgagactg 60
agctgcgccg ccagcggcta caccttcacc aactacggca tgaactgggt gcggcaggcc 120
cctggcaagg gcctggaatg ggtgggctgg atcaacacct acaccggcga gcccacctac 180
gccgccgact tcaagcggcg gttcaccttc agcctggaca ccagcaagag caccgcctac 240
ctgcagatga acagcctgcg ggccgaggac accgccgtgt actactgcgc caagtacccc 300
cactactacg gcagcagcca ctggtacttc gactactggg ggcagggtac cctggtcacc 360
gtctcgagcg ctagcaccaa gggccccagc gtgttccccc tggcccccag cagcaagagc 420
accagcggcg gcacagccgc cctgggctgc ctggtgaagg actacttccc cgagcctgtg 480
accgtgtcct ggaatagcgg agccctgacc tccggcgtgc acaccttccc cgccgtgctg 540
cagagcagcg gcctgtactc cctgagcagc gtggtgaccg tgcccagcag cagcctgggc 600
acccagacct acatctgcaa cgtgaaccac aagcccagca acaccaaagt ggacaagaaa 660
gtggagccca agagctgcga taagacccac acctgccccc cctgccctgc ccccgagctg 720
ctgggcggac ctagcgtgtt cctgttcccc cccaagccta aggacaccct gatgatcagc 780
aggacccccg aagtgacctg cgtggtggtg gatgtgagcc acgaggaccc tgaagtgaag 840
ttcaactggt acgtggacgg cgtggaagtg cacaacgcca agaccaagcc cagagaggag 900
cagtacaaca gcacctaccg cgtggtgtct gtgctgaccg tgctgcacca ggattggctg 960
aacggcaagg agtacaagtg caaagtgagc aacaaggccc tgcctgcccc tatcgagaaa 1020
accatcagca aggccaaggg ccagcctaga gagccccagg tctacaccct gcctccctcc 1080
agagatgagc tgaccaagaa ccaggtgtcc ctgacctgtc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtact ccaagctgac cgtggacaag 1260
agcagatggc agcagggcaa cgtgttcagc tgcagcgtga tgcacgaggc cctgcacaat 1320
cactacaccc agaagagtct gagcctgtcc cctggcaagt cgaccggtga catccagatg 1380
acccagagcc cttcaagcct gagcgccagc gtgggcgaca gagtgaccat cacctgccgg 1440
gccagccagt ggatcggcaa cctgctggac tggtatcagc agaagcccgg caaggccccc 1500
aagctgctga tctactacgc cagcttcctg cagagcggcg tgcccagccg gtttagcggc 1560
agcggctacg gcaccgactt caccctgacc atcagcagcc tgcagcccga ggacttcgcc 1620
acctactact gccagcaggc caaccctgcc cccctgacct tcggccaggg taccaaggtg 1680
gaaatcaaac gg 1692
```

<210> 180
<211> 564
<212> PRT
<213> Artificial Sequence

<220>
<223> Humanised

<400> 180

Glu Val Gln Leu Val Glu Ser Gly Gly Gly Leu Val Gln Pro Gly Gly
1 5 10 15
Ser Leu Arg Leu Ser Cys Ala Ala Ser Gly Tyr Thr Phe Thr Asn Tyr
20 25 30
Gly Met Asn Trp Val Arg Gln Ala Pro Gly Lys Gly Leu Glu Trp Val
35 40 45
Gly Trp Ile Asn Thr Tyr Thr Gly Glu Pro Thr Tyr Ala Ala Asp Phe
50 55 60
Lys Arg Arg Phe Thr Phe Ser Leu Asp Thr Ser Lys Ser Thr Ala Tyr
65 70 75 80
Leu Gln Met Asn Ser Leu Arg Ala Glu Asp Thr Ala Val Tyr Tyr Cys
85 90 95
Ala Lys Tyr Pro His Tyr Tyr Gly Ser Ser His Trp Tyr Phe Asp Tyr
100 105 110
Trp Gly Gln Gly Thr Leu Val Thr Val Ser Ser Ala Ser Thr Lys Gly
115 120 125
Pro Ser Val Phe Pro Leu Ala Pro Ser Ser Lys Ser Thr Ser Gly Gly
130 135 140
Thr Ala Ala Leu Gly Cys Leu Val Lys Asp Tyr Phe Pro Glu Pro Val
145 150 155 160
Thr Val Ser Trp Asn Ser Gly Ala Leu Thr Ser Gly Val His Thr Phe
165 170 175
Pro Ala Val Leu Gln Ser Ser Gly Leu Tyr Ser Leu Ser Ser Val Val
180 185 190
Thr Val Pro Ser Ser Ser Leu Gly Thr Gln Thr Tyr Ile Cys Asn Val
195 200 205
Asn His Lys Pro Ser Asn Thr Lys Val Asp Lys Lys Val Glu Pro Lys
210 215 220
Ser Cys Asp Lys Thr His Thr Cys Pro Pro Cys Pro Ala Pro Glu Leu
225 230 235 240
Leu Gly Gly Pro Ser Val Phe Leu Phe Pro Pro Lys Pro Lys Asp Thr

```
                    245                                     250                                     255
Leu Met Ile Ser Arg Thr Pro Glu Val Thr Cys Val Val Val Asp Val
                260                 265                 270
Ser His Glu Asp Pro Glu Val Lys Phe Asn Trp Tyr Val Asp Gly Val
            275                 280                 285
Glu Val His Asn Ala Lys Thr Lys Pro Arg Glu Glu Gln Tyr Asn Ser
    290                 295                 300
Thr Tyr Arg Val Val Ser Val Leu Thr Val Leu His Gln Asp Trp Leu
305                 310                 315                 320
Asn Gly Lys Glu Tyr Lys Cys Lys Val Ser Asn Lys Ala Leu Pro Ala
                325                 330                 335
Pro Ile Glu Lys Thr Ile Ser Lys Ala Lys Gly Gln Pro Arg Glu Pro
            340                 345                 350
Gln Val Tyr Thr Leu Pro Pro Ser Arg Asp Glu Leu Thr Lys Asn Gln
        355                 360                 365
Val Ser Leu Thr Cys Leu Val Lys Gly Phe Tyr Pro Ser Asp Ile Ala
    370                 375                 380
Val Glu Trp Glu Ser Asn Gly Gln Pro Glu Asn Asn Tyr Lys Thr Thr
385                 390                 395                 400
Pro Pro Val Leu Asp Ser Asp Gly Ser Phe Phe Leu Tyr Ser Lys Leu
                405                 410                 415
Thr Val Asp Lys Ser Arg Trp Gln Gln Gly Asn Val Phe Ser Cys Ser
            420                 425                 430
Val Met His Glu Ala Leu His Asn His Tyr Thr Gln Lys Ser Leu Ser
        435                 440                 445
Leu Ser Pro Gly Lys Ser Thr Gly Asp Ile Gln Met Thr Gln Ser Pro
    450                 455                 460
Ser Ser Leu Ser Ala Ser Val Gly Asp Arg Val Thr Ile Thr Cys Arg
465                 470                 475                 480
Ala Ser Gln Trp Ile Gly Asn Leu Leu Asp Trp Tyr Gln Gln Lys Pro
                485                 490                 495
Gly Lys Ala Pro Lys Leu Leu Ile Tyr Tyr Ala Ser Phe Leu Gln Ser
            500                 505                 510
Gly Val Pro Ser Arg Phe Ser Gly Ser Gly Tyr Gly Thr Asp Phe Thr
        515                 520                 525
Leu Thr Ile Ser Ser Leu Gln Pro Glu Asp Phe Ala Thr Tyr Tyr Cys
    530                 535                 540
Gln Gln Ala Asn Pro Ala Pro Leu Thr Phe Gly Gln Gly Thr Lys Val
545                 550                 555                 560
Glu Ile Lys Arg
```

<210> 181
<211> 642
<212> DNA
<213> Artificial Sequence

<220>
<223> Humanised

<400> 181

```
gacatccaga tgacccagag ccccagcagc ctgagcgcca gcgtgggcga cagagtgacc 60
atcacctgca gcgccagcca ggacatcagc aactacctga actggtatca gcagaagccc 120
ggcaaggccc ccaaggtgct gatctacttc accagctccc tgcacagcgg cgtgcccagc 180
cggtttagcg gcagcggctc cggcaccgac ttcaccctga ccatcagcag cctgcagccc 240
gaggacttcg ccacctacta ctgccagcag tacagcaccg tgccctggac cttcggccag 300
ggtaccaagg tggagatcaa gcgtacggtg gccgctccca gcgtgttcat cttccccccc 360
agcgacgagc agctgaagag cggcaccgcc tccgtggtgt gcctgctgaa caacttctac 420
ccccgggagg ccaaggtgca gtggaaggtg gacaacgccc tgcagtccgg caacagccag 480
gaaagcgtca ccgagcagga ctccaaggac tccacctaca gcctgagcag caccctgacc 540
ctgagcaagg ccgactacga gaagcacaag gtgtacgcct gcgaagtgac ccaccagggc 600
ctgtccagcc ccgtgaccaa gagcttcaac cggggcgagt gc                     642
```

<210> 182
<211> 214
<212> PRT
<213> Artificial Sequence

<220>
<223> Humanised

<400> 182

```
Asp Ile Gln Met Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 183
<211> 1710
<212> DNA
<213> Artificial Sequence

<220>
<223> Humanised

<400> 183

```
caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
acccccttca ccagcagact gagcatcaac aaggacaaca gcaagagcca ggtgttcttc 240
aagatgaaca gcctgcagag caacgacacc gccatctact attgtgccag gggccctgacc 300
tactacgact acgagttcgc ctactggggc cagggcaccc tggtgaccgt gagcgccgct 360
agcaccaagg gccccagcgt gttcccccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaagtcg accggtgggg tgcagctgtt ggagtctggg 1380
ggaggcttgg tacagcctgg ggggtccctg cgtctctcct gtgcagcctc cggattcacc 1440
ttcgcttggt atgatatggg gtgggtccgc caggctccag ggaagggtct agagtgggtc 1500
tcaagtattg attggcatgg tgaggttaca tactacgcag actccgtgaa gggccggttc 1560
accatctccc gcgacaattc caagaacacg ctgtatctgc aaatgaacag cctgcgtgcc 1620
gaggacaccg cggtatatta ctgtgcgaca gcggaggacg agccggggta tgactactgg 1680
ggccagggaa ccctggtcac cgtctcgagc 1710
```

<210> 184
<211> 570
<212> PRT
<213> Artificial Sequence

<220>
<223> Humanised

<400> 184

Gln Val Gln Leu Lys Gln Ser Gly Pro Gly Leu Val Gln Pro Ser Gln

```
1               5                   10                  15
Ser Leu Ser Ile Thr Cys Thr Val Ser Gly Phe Ser Leu Thr Asn Tyr
            20                  25                  30
Gly Val His Trp Val Arg Gln Ser Pro Gly Lys Gly Leu Glu Trp Leu
        35                  40                  45
Gly Val Ile Trp Ser Gly Gly Asn Thr Asp Tyr Asn Thr Pro Phe Thr
    50                  55                  60
Ser Arg Leu Ser Ile Asn Lys Asp Asn Ser Lys Ser Gln Val Phe Phe
65                  70                  75                  80
Lys Met Asn Ser Leu Gln Ser Asn Asp Thr Ala Ile Tyr Tyr Cys Ala
                85                  90                  95
Arg Ala Leu Thr Tyr Tyr Asp Tyr Glu Phe Ala Tyr Trp Gly Gln Gly
            100                 105                 110
Thr Leu Val Thr Val Ser Ala Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Ser Thr Gly Gly Val Gln Leu Leu Glu Ser Gly Gly Gly Leu Val
    450                 455                 460
Gln Pro Gly Gly Ser Leu Arg Leu Ser Cys Ala Ala Ser Gly Phe Thr
465                 470                 475                 480
Phe Ala Trp Tyr Asp Met Gly Trp Val Arg Gln Ala Pro Gly Lys Gly
                485                 490                 495
Leu Glu Trp Val Ser Ser Ile Asp Trp His Gly Glu Val Thr Tyr Tyr
            500                 505                 510
Ala Asp Ser Val Lys Gly Arg Phe Thr Ile Ser Arg Asp Asn Ser Lys
        515                 520                 525
Asn Thr Leu Tyr Leu Gln Met Asn Ser Leu Arg Ala Glu Asp Thr Ala
    530                 535                 540
Val Tyr Tyr Cys Ala Thr Ala Glu Asp Glu Pro Gly Tyr Asp Tyr Trp
545                 550                 555                 560
Gly Gln Gly Thr Leu Val Thr Val Ser Ser
                565                 570
```

<210> 185
<211> 562
<212> PRT
<213> Artificial Sequence

<220>
<223> Humanised

<400> 185

```
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Ser Thr Gly Asp Ile Gln Met Thr Gln Ser Pro Ser Ser
    450                 455                 460
Leu Ser Ala Ser Val Gly Asp Arg Val Thr Ile Thr Cys Arg Ala Ser
465                 470                 475                 480
Gln Trp Ile Gly Pro Glu Leu Arg Trp Tyr Gln Gln Lys Pro Gly Lys
                485                 490                 495
Ala Pro Lys Leu Leu Ile Tyr His Thr Ser Ile Leu Gln Ser Gly Val
            500                 505                 510
Pro Ser Arg Phe Ser Gly Ser Gly Ser Gly Thr Asp Phe Thr Leu Thr
        515                 520                 525
Ile Ser Ser Leu Gln Pro Glu Asp Phe Ala Thr Tyr Tyr Cys Gln Gln
    530                 535                 540
Tyr Met Phe Gln Pro Met Thr Phe Gly Gln Gly Thr Lys Val Glu Ile
545                 550                 555                 560
```

Lys Arg

<210> 186
<211> 560
<212> PRT
<213> Artificial Sequence

<220>
<223> Humanised

<400> 186

Gln Val Gln Leu Lys Gln Ser Gly Pro Gly Leu Val Gln Pro Ser Gln
1 5 10 15
Ser Leu Ser Ile Thr Cys Thr Val Ser Gly Phe Ser Leu Thr Asn Tyr
20 25 30
Gly Val His Trp Val Arg Gln Ser Pro Gly Lys Gly Leu Glu Trp Leu
35 40 45
Gly Val Ile Trp Ser Gly Gly Asn Thr Asp Tyr Asn Thr Pro Phe Thr
50 55 60
Ser Arg Leu Ser Ile Asn Lys Asp Asn Ser Lys Ser Gln Val Phe Phe
65 70 75 80
Lys Met Asn Ser Leu Gln Ser Asn Asp Thr Ala Ile Tyr Tyr Cys Ala
85 90 95
Arg Ala Leu Thr Tyr Tyr Asp Tyr Glu Phe Ala Tyr Trp Gly Gln Gly
100 105 110
Thr Leu Val Thr Val Ser Ala Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys Ser Thr Gly Asp Ile Gln Met Thr Gln Ser Pro Ser Ser Leu Ser
450 455 460
Ala Ser Val Gly Asp Arg Val Thr Ile Thr Cys Arg Ala Ser Gln Trp
465 470 475 480
Ile Gly Pro Glu Leu Arg Trp Tyr Gln Gln Lys Pro Gly Lys Ala Pro
485 490 495
Lys Leu Leu Ile Tyr His Thr Ser Ile Leu Gln Ser Gly Val Pro Ser

```
                500                     505                     510
Arg Phe Ser Gly Ser Gly Ser Gly Thr Asp Phe Thr Leu Thr Ile Ser
            515                     520                     525
Ser Leu Gln Pro Glu Asp Phe Ala Thr Tyr Tyr Cys Gln Gln Tyr Met
        530                     535                     540
Phe Gln Pro Met Thr Phe Gly Gln Gly Thr Lys Val Glu Ile Lys Arg
545                     550                     555                     560
```

<210> 187
<211> 1002
<212> DNA
<213> Artificial Sequence

<220>
<223> Humanised

<400> 187

```
gacatcctgc tgacccagag ccccgtgatc ctgagcgtga gccctggcga gagagtgagc 60
ttcagctgcc gggccagcca gagcatcggc accaacatcc actggtatca gcagcggacc 120
aacggcagcc ccaggctgct gatcaagtac gccagcgagt ccatcagcgg catccccagc 180
cggttcagcg gcagcggctc cggcaccgac ttcaccctga gcatcaacag cgtggagagc 240
gaggatatcg ccgactacta ctgccagcag aacaacaact ggcccaccac cttcggagcc 300
ggcaccaagc tggaactgaa gcgtacggtg gccgccccca gcgtgttcat cttccccccc 360
agcgatgagc agctcaagag cggcaccgcc agcgtggtgt gtctgctgaa caacttctac 420
ccccgggagg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cggggcgagt gcggatccac cggcgaggtg 660
cagctgttgg tgtctggggg aggcttggta cagcctgggg ggtccctgcg tctctcctgt 720
gcagcctccg gattcacctt taaggcttat ccgatgatgt gggtccgcca ggctccaggg 780
aagggtctag agtgggtttc agagatttcg ccttcgggtt cttatacata ctacgcagac 840
tccgtgaagg gccggttcac catctcccgc gacaattcca agaacacgct gtatctgcaa 900
atgaacagcc tgcgtgccga ggacaccgcg gtatattact gtgcgaaaga tcctcggaag 960
ttagactact ggggtcaggg aaccctggtc accgtctcga gc                   1002
```

<210> 188
<211> 334
<212> PRT
<213> Artificial Sequence

<220>
<223> Humanised

<400> 188

Asp Ile Leu Leu Thr Gln Ser Pro Val Ile Leu Ser Val Ser Pro Gly
1 5 10 15
Glu Arg Val Ser Phe Ser Cys Arg Ala Ser Gln Ser Ile Gly Thr Asn
20 25 30
Ile His Trp Tyr Gln Gln Arg Thr Asn Gly Ser Pro Arg Leu Leu Ile
35 40 45
Lys Tyr Ala Ser Glu Ser Ile Ser Gly Ile Pro Ser Arg Phe Ser Gly
50 55 60
Ser Gly Ser Gly Thr Asp Phe Thr Leu Ser Ile Asn Ser Val Glu Ser
65 70 75 80
Glu Asp Ile Ala Asp Tyr Tyr Cys Gln Gln Asn Asn Asn Trp Pro Thr
85 90 95
Thr Phe Gly Ala Gly Thr Lys Leu Glu Leu Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys Gly Ser Thr Gly Glu Val Gln Leu Leu Val
210 215 220
Ser Gly Gly Gly Leu Val Gln Pro Gly Gly Ser Leu Arg Leu Ser Cys
225 230 235 240
Ala Ala Ser Gly Phe Thr Phe Lys Ala Tyr Pro Met Met Trp Val Arg

245 250 255
Gln Ala Pro Gly Lys Gly Leu Glu Trp Val Ser Glu Ile Ser Pro Ser
260 265 270
Gly Ser Tyr Thr Tyr Tyr Ala Asp Ser Val Lys Gly Arg Phe Thr Ile
275 280 285
Ser Arg Asp Asn Ser Lys Asn Thr Leu Tyr Leu Gln Met Asn Ser Leu
290 295 300
Arg Ala Glu Asp Thr Ala Val Tyr Tyr Cys Ala Lys Asp Pro Arg Lys
305 310 315 320
Leu Asp Tyr Trp Gly Gln Gly Thr Leu Val Thr Val Ser Ser
325 330

<210> 189
<211> 1014
<212> DNA
<213> Artificial Sequence

<220>
<223> Humanised

<400> 189

```
gacatcctgc tgacccagag ccccgtgatc ctgagcgtga gccctggcga gagagtgagc 60
ttcagctgcc gggccagcca gagcatcggc accaacatcc actggtatca gcagcggacc 120
aacggcagcc ccaggctgct gatcaagtac gccagcgagt ccatcagcgg catccccagc 180
cggttcagcg gcagcggctc cggcaccgac ttcaccctga gcatcaacag cgtggagagc 240
gaggatatcg ccgactacta ctgccagcag aacaacaact ggcccaccac cttcggagcc 300
ggcaccaagc tggaactgaa gcgtacggtg gccgccccca gcgtgttcat cttccccccc 360
agcgatgagc agctcaagag cggcaccgcc agcgtggtgt gtctgctgaa caacttctac 420
cccgggaggg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cggggcgagt gcggatccac ggtggccgcc 660
cccagcgagg tgcagctgtt ggtgtctggg ggaggcttgg tacagcctgg ggggtccctg 720
cgtctctcct gtgcagcctc cggattcacc tttaaggctt atccgatgat gtgggtccgc 780
caggctccag ggaagggtct agagtgggtt tcagagattt cgccttcggg ttcttataca 840
tactacgcag actccgtgaa gggccggttc accatctccc gcgacaattc caagaacacg 900
ctgtatctgc aaatgaacag cctgcgtgcc gaggacaccg cggtatatta ctgtgcgaaa 960
gatcctcgga agttagacta ctggggtcag ggaaccctgg tcaccgtctc gagc      1014
```

<210> 190
<211> 338
<212> PRT
<213> Artificial Sequence

<220>
<223> Humanised

<400> 190

```
Asp Ile Leu Leu Thr Gln Ser Pro Val Ile Leu Ser Val Ser Pro Gly
1               5                   10                  15
Glu Arg Val Ser Phe Ser Cys Arg Ala Ser Gln Ser Ile Gly Thr Asn
            20                  25                  30
Ile His Trp Tyr Gln Gln Arg Thr Asn Gly Ser Pro Arg Leu Leu Ile
        35                  40                  45
Lys Tyr Ala Ser Glu Ser Ile Ser Gly Ile Pro Ser Arg Phe Ser Gly
    50                  55                  60
Ser Gly Ser Gly Thr Asp Phe Thr Leu Ser Ile Asn Ser Val Glu Ser
65                  70                  75                  80
Glu Asp Ile Ala Asp Tyr Tyr Cys Gln Gln Asn Asn Asn Trp Pro Thr
                85                  90                  95
Thr Phe Gly Ala Gly Thr Lys Leu Glu Leu Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys Gly Ser Thr Val Ala Ala Pro Ser Glu Val
```

```
    210                     215                     220
Gln Leu Leu Val Ser Gly Gly Gly Leu Val Gln Pro Gly Gly Ser Leu
225                 230                     235                 240
Arg Leu Ser Cys Ala Ala Ser Gly Phe Thr Phe Lys Ala Tyr Pro Met
                245                     250                 255
Met Trp Val Arg Gln Ala Pro Gly Lys Gly Leu Glu Trp Val Ser Glu
            260                 265                     270
Ile Ser Pro Ser Gly Ser Tyr Thr Tyr Tyr Ala Asp Ser Val Lys Gly
        275                 280                     285
Arg Phe Thr Ile Ser Arg Asp Asn Ser Lys Asn Thr Leu Tyr Leu Gln
    290                     295                 300
Met Asn Ser Leu Arg Ala Glu Asp Thr Ala Val Tyr Tyr Cys Ala Lys
305                 310                     315                 320
Asp Pro Arg Lys Leu Asp Tyr Trp Gly Gln Gly Thr Leu Val Thr Val
                325                     330                 335
Ser Ser
```

<210> 191
<211> 449
<212> PRT
<213> Artificial Sequence

<220>
<223> Humanised

<400> 191

Gln Val Thr Leu Arg Glu Ser Gly Pro Ala Leu Val Lys Pro Thr Gln
1               5                   10                  15
Thr Leu Thr Leu Thr Cys Thr Val Ser Gly Phe Ser Leu Thr Ser Tyr
            20                  25                  30
Ser Val His Trp Val Arg Gln Pro Pro Gly Lys Gly Leu Glu Trp Leu
        35                  40                  45
Gly Val Ile Trp Ala Ser Gly Gly Thr Asp Tyr Asn Ser Ala Leu Met
    50                  55                  60
Ser Arg Leu Ser Ile Ser Lys Asp Thr Ser Arg Asn Gln Val Val Leu
65                  70                  75                  80
Thr Met Thr Asn Met Asp Pro Val Asp Thr Ala Thr Tyr Tyr Cys Ala
                85                  90                  95
Arg Asp Pro Pro Ser Ser Leu Leu Arg Leu Asp Tyr Trp Gly Arg Gly
            100                 105                 110
Thr Pro Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Arg Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210> 192
<211> 1746
<212> DNA
<213> Artificial Sequence

<220>
<223> Humanised

<400> 192

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ccagcgccag caccaagggc cccagcgtgt tccccctggc ccccagcagc 420
aagagcacca gcggcggcac agccgccctg ggctgcctgg tgaaggacta cttccccgaa 480
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaagggatc agccagcacc 1380
aagggcccca cgggatccga agtgcagctc ctggagagcg gcggcggcct ggtgcagccc 1440
ggcggcagcc tgaggctgag ctgcgccgct agcggcttca ccttcaggaa cttcggcatg 1500
ggctgggtca ggcaggcccc cggcaagggc ctggagtggg tcagctggat catcagctcc 1560
ggcaccgaga cctactacgc cgacagcgtg aagggcaggt tcaccatcag ccgcgacaac 1620
agcaagaaca ccctgtacct gcagatgaac agcctgaggg ccgaggacac cgccgtctac 1680
tactgcgcca agagcctggg caggttcgac tactggggac aggggaccct ggtgactgtg 1740
agcagc                                                            1746
```

<210> 193
<211> 698
<212> PRT
<213> Artificial Sequence

<220>
<223> Humanised

<400> 193

```
Glu Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Lys Ser
            20                  25                  30
Val Met Gly Val Ser Trp Ile Arg Gln Pro Pro Gly Lys Ala Leu Glu
        35                  40                  45
Trp Leu Ala His Ile Tyr Trp Asp Asp Asp Lys Tyr Tyr Asn Pro Ser
    50                  55                  60
Leu Lys Ser Arg Leu Thr Ile Ser Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala Arg Arg Gly Ile Arg Ser Ala Met Asp Tyr Trp Gly Gln Gly
            100                 105                 110
Thr Thr Val Thr Val Ser Ser Ala Ser Thr Lys Gly Pro Glu Val Gln
```

```
            115                     120                     125
Leu Val Gln Ser Gly Thr Glu Val Lys Lys Pro Gly Glu Ser Leu Lys
    130                     135                     140
Ile Ser Cys Lys Gly Ser Gly Tyr Thr Val Thr Ser Tyr Trp Ile Gly
145                     150                     155                 160
Trp Val Arg Gln Met Pro Gly Lys Gly Leu Glu Trp Met Gly Phe Ile
                165                     170                     175
Tyr Pro Gly Asp Ser Glu Thr Arg Tyr Ser Pro Thr Phe Gln Gly Gln
                180                     185                     190
Val Thr Ile Ser Ala Asp Lys Ser Phe Asn Thr Ala Phe Leu Gln Trp
            195                     200                     205
Ser Ser Leu Lys Ala Ser Asp Thr Ala Met Tyr Tyr Cys Ala Arg Val
    210                     215                     220
Gly Ser Gly Trp Tyr Pro Tyr Thr Phe Asp Ile Trp Gly Gln Gly Thr
225                     230                     235                 240
Leu Val Thr Val Ser Ser Ala Ser Thr Lys Gly Pro Ser Val Phe Pro
                245                     250                     255
Leu Ala Pro Ser Ser Lys Ser Thr Ser Gly Gly Thr Ala Ala Leu Gly
                260                     265                     270
Cys Leu Val Lys Asp Tyr Phe Pro Glu Pro Val Thr Val Ser Trp Asn
            275                     280                     285
Ser Gly Ala Leu Thr Ser Gly Val His Thr Phe Pro Ala Val Leu Gln
    290                     295                     300
Ser Ser Gly Leu Tyr Ser Leu Ser Ser Val Val Thr Val Pro Ser Ser
305                     310                     315                 320
Ser Leu Gly Thr Gln Thr Tyr Ile Cys Asn Val Asn His Lys Pro Ser
                325                     330                     335
Asn Thr Lys Val Asp Lys Lys Val Glu Pro Lys Ser Cys Asp Lys Thr
                340                     345                     350
His Thr Cys Pro Pro Cys Pro Ala Pro Glu Leu Leu Gly Gly Pro Ser
            355                     360                     365
Val Phe Leu Phe Pro Pro Lys Pro Lys Asp Thr Leu Met Ile Ser Arg
    370                     375                     380
Thr Pro Glu Val Thr Cys Val Val Val Asp Val Ser His Glu Asp Pro
385                     390                     395                 400
Glu Val Lys Phe Asn Trp Tyr Val Asp Gly Val Glu Val His Asn Ala
                405                     410                     415
Lys Thr Lys Pro Arg Glu Glu Gln Tyr Asn Ser Thr Tyr Arg Val Val
                420                     425                     430
Ser Val Leu Thr Val Leu His Gln Asp Trp Leu Asn Gly Lys Glu Tyr
            435                     440                     445
Lys Cys Lys Val Ser Asn Lys Ala Leu Pro Ala Pro Ile Glu Lys Thr
    450                     455                     460
Ile Ser Lys Ala Lys Gly Gln Pro Arg Glu Pro Gln Val Tyr Thr Leu
465                     470                     475                 480
Pro Pro Ser Arg Asp Glu Leu Thr Lys Asn Gln Val Ser Leu Thr Cys
                485                     490                     495
Leu Val Lys Gly Phe Tyr Pro Ser Asp Ile Ala Val Glu Trp Glu Ser
                500                     505                     510
Asn Gly Gln Pro Glu Asn Asn Tyr Lys Thr Thr Pro Pro Val Leu Asp
            515                     520                     525
Ser Asp Gly Ser Phe Phe Leu Tyr Ser Lys Leu Thr Val Asp Lys Ser
    530                     535                     540
Arg Trp Gln Gln Gly Asn Val Phe Ser Cys Ser Val Met His Glu Ala
545                     550                     555                 560
Leu His Asn His Tyr Thr Gln Lys Ser Leu Ser Leu Ser Pro Gly Lys
                565                     570                     575
Thr Val Ala Ala Pro Ser Glu Val Gln Leu Leu Glu Ser Gly Gly Gly
                580                     585                     590
Leu Val Gln Pro Gly Gly Ser Leu Arg Leu Ser Cys Ala Ala Ser Gly
            595                     600                     605
Phe Thr Phe Arg Asn Phe Gly Met Gly Trp Val Arg Gln Ala Pro Gly
    610                     615                     620
Lys Gly Leu Glu Trp Val Ser Trp Ile Ile Ser Ser Gly Thr Glu Thr
625                     630                     635                 640
Tyr Tyr Ala Asp Ser Val Lys Gly Arg Phe Thr Ile Ser Arg Asp Asn
                645                     650                     655
Ser Lys Asn Thr Leu Tyr Leu Gln Met Asn Ser Leu Arg Ala Glu Asp
                660                     665                     670
Thr Ala Val Tyr Tyr Cys Ala Lys Ser Leu Gly Arg Phe Asp Tyr Trp
            675                     680                     685
Gly Gln Gly Thr Leu Val Thr Val Ser Ser
    690                     695
```

<210> 194
<211> 328
<212> PRT
<213> Artificial Sequence

<220>
<223> Humanised

<400> 194

```
Asp Ile Val Met Thr Gln Ser Pro Asp Ser Leu Ala Val Ser Leu Gly
1               5                   10                  15
Glu Arg Ala Thr Ile Asn Cys Lys Ala Ser Gln Ser Val Ser Asn Asp
            20                  25                  30
Val Ala Trp Tyr Gln Gln Lys Pro Gly Gln Pro Pro Lys Leu Leu Ile
        35                  40                  45
Tyr Tyr Ala Ser Asn Arg Tyr Thr Gly Val Pro Asp Arg Phe Ser Gly
    50                  55                  60
Ser Gly Ser Gly Thr Asp Phe Thr Leu Thr Ile Ser Ser Leu Gln Ala
65                  70                  75                  80
Glu Asp Val Ala Val Tyr Tyr Cys Gln Gln Asp Tyr Asn Ser Pro Trp
                85                  90                  95
Thr Phe Gly Gly Gly Thr Lys Val Glu Ile Lys Arg Thr Val Ala Ala
            100                 105                 110
Pro Glu Ile Val Met Thr Gln Ser Pro Ala Thr Leu Ser Val Ser Pro
        115                 120                 125
Gly Glu Arg Ala Thr Leu Ser Cys Arg Ala Ser Glu Ser Ala Ser Ser
    130                 135                 140
Asn Leu Ala Trp Tyr Gln Gln Lys Pro Gly Gln Ala Pro Arg Leu Phe
145                 150                 155                 160
Ile Tyr Thr Ala Ser Thr Arg Ala Thr Asp Ile Pro Ala Arg Phe Ser
                165                 170                 175
Gly Ser Gly Ser Gly Thr Glu Phe Thr Leu Thr Ile Ser Ser Leu Gln
            180                 185                 190
Ser Glu Asp Phe Ala Val Tyr Tyr Cys Gln Gln Tyr Asn Asn Trp Pro
        195                 200                 205
Ser Ile Thr Phe Gly Gln Gly Thr Arg Leu Glu Ile Lys Arg Thr Val
    210                 215                 220
Ala Ala Pro Ser Val Phe Ile Phe Pro Pro Ser Asp Glu Gln Leu Lys
225                 230                 235                 240
Ser Gly Thr Ala Ser Val Val Cys Leu Leu Asn Asn Phe Tyr Pro Arg
                245                 250                 255
Glu Ala Lys Val Gln Trp Lys Val Asp Asn Ala Leu Gln Ser Gly Asn
            260                 265                 270
Ser Gln Glu Ser Val Thr Glu Gln Asp Ser Lys Asp Ser Thr Tyr Ser
        275                 280                 285
Leu Ser Ser Thr Leu Thr Leu Ser Lys Ala Asp Tyr Glu Lys His Lys
    290                 295                 300
Val Tyr Ala Cys Glu Val Thr His Gln Gly Leu Ser Ser Pro Val Thr
305                 310                 315                 320
Lys Ser Phe Asn Arg Gly Glu Cys
                325
```

<210> 195
<211> 696
<212> PRT
<213> Artificial Sequence

<220>
<223> Humanised

<400> 195

Gln Val Thr Leu Arg Glu Ser Gly Pro Ala Leu Val Lys Pro Thr Gln
1 5 10 15
Thr Leu Thr Leu Thr Cys Thr Val Ser Gly Phe Ser Leu Thr Ser Tyr
20 25 30
Ser Val His Trp Val Arg Gln Pro Pro Gly Lys Gly Leu Glu Trp Leu
35 40 45
Gly Val Ile Trp Ala Ser Gly Gly Thr Asp Tyr Asn Ser Ala Leu Met
50 55 60
Ser Arg Leu Ser Ile Ser Lys Asp Thr Ser Arg Asn Gln Val Val Leu
65 70 75 80
Thr Met Thr Asn Met Asp Pro Val Asp Thr Ala Thr Tyr Tyr Cys Ala
85 90 95
Arg Asp Pro Pro Ser Ser Leu Leu Arg Leu Asp Tyr Trp Gly Arg Gly
100 105 110
Thr Leu Val Thr Val Ser Ser Ala Ser Thr Lys Gly Pro Ser Val Phe

```
                115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys Gly Gly Gly Gly Ser Gly Val Gln Leu Leu Glu Ser Gly Gly Gly
    450                 455                 460
Leu Val Gln Pro Gly Gly Ser Leu Arg Leu Ser Cys Ala Ala Ser Gly
465                 470                 475                 480
Phe Thr Phe Ala Trp Tyr Asp Met Gly Trp Val Arg Gln Ala Pro Gly
                485                 490                 495
Lys Gly Leu Glu Trp Val Ser Ser Ile Asp Trp His Gly Glu Val Thr
            500                 505                 510
Tyr Tyr Ala Asp Ser Val Lys Gly Arg Phe Thr Ile Ser Arg Asp Asn
        515                 520                 525
Ser Lys Asn Thr Leu Tyr Leu Gln Met Asn Ser Leu Arg Ala Glu Asp
    530                 535                 540
Thr Ala Val Tyr Tyr Cys Ala Thr Ala Glu Asp Glu Pro Gly Tyr Asp
545                 550                 555                 560
Tyr Trp Gly Gln Gly Thr Leu Val Thr Val Ser Ser Thr Val Ala Ala
                565                 570                 575
Pro Ser Gly Ser Glu Val Gln Leu Leu Glu Ser Gly Gly Gly Leu Val
            580                 585                 590
Gln Pro Gly Gly Ser Leu Arg Leu Ser Cys Ala Ala Ser Gly Phe Thr
        595                 600                 605
Phe Arg Asn Phe Gly Met Gly Trp Val Arg Gln Ala Pro Gly Lys Gly
    610                 615                 620
Leu Glu Trp Val Ser Trp Ile Ile Ser Ser Gly Thr Glu Thr Tyr Tyr
625                 630                 635                 640
Ala Asp Ser Val Lys Gly Arg Phe Thr Ile Ser Arg Asp Asn Ser Lys
                645                 650                 655
Asn Thr Leu Tyr Leu Gln Met Asn Ser Leu Arg Ala Glu Asp Thr Ala
            660                 665                 670
Val Tyr Tyr Cys Ala Lys Ser Leu Gly Arg Phe Asp Tyr Trp Gly Gln
        675                 680                 685
Gly Thr Leu Val Thr Val Ser Ser
    690                 695
```

<210> 196
<211> 693
<212> PRT
<213> Artificial Sequence

<220>
<223> Humanised

<400> 196

```
Gln Val Gln Leu Gln Gln Pro Gly Ala Glu Leu Val Lys Pro Gly Ala
1               5                   10                  15
Ser Val Lys Met Ser Cys Lys Ala Ser Gly Tyr Thr Phe Thr Ser Tyr
            20                  25                  30
Asn Met His Trp Val Lys Gln Thr Pro Gly Arg Gly Leu Glu Trp Ile
        35                  40                  45
Gly Ala Ile Tyr Pro Gly Asn Gly Asp Thr Ser Tyr Asn Gln Lys Phe
    50                  55                  60
Lys Gly Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Ser Thr Tyr Tyr Gly Gly Asp Trp Tyr Phe Asn Val Trp Gly
            100                 105                 110
Ala Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys Thr Val Ala Ala Pro Ser Gly Ser Asp Ile Gln Met Thr
    450                 455                 460
Gln Ser Pro Ser Ser Leu Ser Ala Ser Val Gly Asp Arg Val Thr Ile
465                 470                 475                 480
Thr Cys Arg Ala Ser Arg Pro Ile Ser Asp Trp Leu His Trp Tyr Gln
                485                 490                 495
Gln Lys Pro Gly Lys Ala Pro Lys Leu Leu Ile Ala Trp Ala Ser Ser
            500                 505                 510
Leu Gln Gly Gly Val Pro Ser Arg Phe Ser Gly Ser Gly Ser Gly Thr
        515                 520                 525
Asp Phe Thr Leu Thr Ile Ser Ser Leu Gln Pro Glu Asp Phe Ala Thr
    530                 535                 540
```

```
Tyr Tyr Cys Leu Gln Glu Gly Trp Gly Pro Pro Thr Phe Gly Gln Gly
545                 550                 555                 560
Thr Lys Val Glu Ile Lys Arg Thr Val Ala Ala Pro Ser Gly Ser Gly
                565                 570                 575
Val Gln Leu Leu Glu Ser Gly Gly Gly Leu Val Gln Pro Gly Gly Ser
            580                 585                 590
Leu Arg Leu Ser Cys Ala Ala Ser Gly Phe Thr Phe Ala Trp Tyr Asp
        595                 600                 605
Met Gly Trp Val Arg Gln Ala Pro Gly Lys Gly Leu Glu Trp Val Ser
    610                 615                 620
Ser Ile Asp Trp His Gly Glu Val Thr Tyr Tyr Ala Asp Ser Val Lys
625                 630                 635                 640
Gly Arg Phe Thr Ile Ser Arg Asp Asn Ser Lys Asn Thr Leu Tyr Leu
                645                 650                 655
Gln Met Asn Ser Leu Arg Ala Glu Asp Thr Ala Val Tyr Tyr Cys Ala
            660                 665                 670
Thr Ala Glu Asp Glu Pro Gly Tyr Asp Tyr Trp Gly Gln Gly Thr Leu
        675                 680                 685
Val Thr Val Ser Ser
    690
```

<210> 197
<211> 701
<212> PRT
<213> Artificial Sequence

<220>
<223> Humanised

<400> 197

Gln Val Gln Leu Gln Gln Pro Gly Ala Glu Leu Val Lys Pro Gly Ala
1 5 10 15
Ser Val Lys Met Ser Cys Lys Ala Ser Gly Tyr Thr Phe Thr Ser Tyr
20 25 30
Asn Met His Trp Val Lys Gln Thr Pro Gly Arg Gly Leu Glu Trp Ile
35 40 45
Gly Ala Ile Tyr Pro Gly Asn Gly Asp Thr Ser Tyr Asn Gln Lys Phe
50 55 60
Lys Gly Lys Ala Thr Leu Thr Ala Asp Lys Ser Ser Ser Thr Ala Tyr
65 70 75 80
Met Gln Leu Ser Ser Leu Thr Ser Glu Asp Ser Ala Val Tyr Tyr Cys
85 90 95
Ala Arg Ser Thr Tyr Tyr Gly Gly Asp Trp Tyr Phe Asn Val Trp Gly
100 105 110
Ala Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser

```
        355                     360                     365
Leu Thr Cys Leu Val Lys Gly Phe Tyr Pro Ser Asp Ile Ala Val Glu
    370                     375                     380
Trp Glu Ser Asn Gly Gln Pro Glu Asn Asn Tyr Lys Thr Thr Pro Pro
385                     390                     395                 400
Val Leu Asp Ser Asp Gly Ser Phe Phe Leu Tyr Ser Lys Leu Thr Val
                405                     410                     415
Asp Lys Ser Arg Trp Gln Gln Gly Asn Val Phe Ser Cys Ser Val Met
            420                     425                     430
His Glu Ala Leu His Asn His Tyr Thr Gln Lys Ser Leu Ser Leu Ser
        435                     440                     445
Pro Gly Lys Thr Val Ala Ala Pro Ser Gly Ser Glu Val Gln Leu Leu
    450                     455                     460
Glu Ser Gly Gly Gly Leu Val Gln Pro Gly Gly Ser Leu Arg Leu Ser
465                     470                     475                 480
Cys Ala Ala Ser Gly Phe Thr Phe Arg Asn Phe Gly Met Gly Trp Val
                485                     490                     495
Arg Gln Ala Pro Gly Lys Gly Leu Glu Trp Val Ser Trp Ile Ile Ser
            500                     505                     510
Ser Gly Thr Glu Thr Tyr Tyr Ala Asp Ser Val Lys Gly Arg Phe Thr
        515                     520                     525
Ile Ser Arg Asp Asn Ser Lys Asn Thr Leu Tyr Leu Gln Met Asn Ser
    530                     535                     540
Leu Arg Ala Glu Asp Thr Ala Val Tyr Tyr Cys Ala Lys Ser Leu Gly
545                     550                     555                 560
Arg Phe Asp Tyr Trp Gly Gln Gly Thr Leu Val Thr Val Ser Ser Thr
                565                     570                     575
Val Ala Ala Pro Ser Gly Ser Gly Val Gln Leu Leu Glu Ser Gly Gly
            580                     585                     590
Gly Leu Val Gln Pro Gly Gly Ser Leu Arg Leu Ser Cys Ala Ala Ser
        595                     600                     605
Gly Phe Thr Phe Ala Trp Tyr Asp Met Gly Trp Val Arg Gln Ala Pro
    610                     615                     620
Gly Lys Gly Leu Glu Trp Val Ser Ser Ile Asp Trp His Gly Glu Val
625                     630                     635                 640
Thr Tyr Tyr Ala Asp Ser Val Lys Gly Arg Phe Thr Ile Ser Arg Asp
                645                     650                     655
Asn Ser Lys Asn Thr Leu Tyr Leu Gln Met Asn Ser Leu Arg Ala Glu
            660                     665                     670
Asp Thr Ala Val Tyr Tyr Cys Ala Thr Ala Glu Asp Glu Pro Gly Tyr
        675                     680                     685
Asp Tyr Trp Gly Gln Gly Thr Leu Val Thr Val Ser Ser
    690                     695                     700
```

<210> 198
<211> 567
<212> PRT
<213> Artificial Sequence

<220>
<223> Humanised

<400> 198

Glu Val Gln Leu Val Glu Ser Gly Gly Gly Leu Val Gln Pro Gly Gly
1 5 10 15
Ser Leu Arg Leu Ser Cys Ala Ala Ser Gly Tyr Thr Phe Thr Ser Tyr
20 25 30
Trp Leu His Trp Val Arg Gln Ala Pro Gly Lys Gly Leu Glu Trp Val
35 40 45
Gly Met Ile Asp Pro Ser Asn Ser Asp Thr Arg Phe Asn Pro Asn Phe
50 55 60
Lys Asp Arg Phe Thr Ile Ser Ala Asp Thr Ser Lys Asn Thr Ala Tyr
65 70 75 80
Leu Gln Met Asn Ser Leu Arg Ala Glu Asp Thr Ala Val Tyr Tyr Cys
85 90 95
Ala Thr Tyr Arg Ser Tyr Val Thr Pro Leu Asp Tyr Trp Gly Gln Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Ser
355 360 365
Cys Ala Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Val Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys Gly Ser Glu Val Gln Leu Leu Val Ser Gly Gly Gly Leu Val Gln
450 455 460
Pro Gly Gly Ser Leu Arg Leu Ser Cys Ala Ala Ser Gly Phe Thr Phe
465 470 475 480
Lys Ala Tyr Pro Met Met Trp Val Arg Gln Ala Pro Gly Lys Gly Leu
485 490 495
Glu Trp Val Ser Glu Ile Ser Pro Ser Gly Ser Tyr Thr Tyr Tyr Ala
500 505 510
Asp Ser Val Lys Gly Arg Phe Thr Ile Ser Arg Asp Asn Ser Lys Asn
515 520 525
Thr Leu Tyr Leu Gln Met Asn Ser Leu Arg Ala Glu Asp Thr Ala Val
530 535 540
Tyr Tyr Cys Ala Lys Asp Pro Arg Lys Leu Asp Tyr Trp Gly Gln Gly
545 550 555 560
Thr Leu Val Thr Val Ser Ser
565

<210> 199
<211> 340
<212> PRT
<213> Artificial Sequence

<220>
<223> Humanised

<400> 199

```
Cys Pro Pro Cys Pro Ala Pro Glu Leu Leu Gly Gly Pro Ser Val Phe
1               5                   10                  15
Leu Phe Pro Pro Lys Pro Lys Asp Thr Leu Met Ile Ser Arg Thr Pro
            20                  25                  30
Glu Val Thr Cys Val Val Val Asp Val Ser His Glu Asp Pro Glu Val
        35                  40                  45
Lys Phe Asn Trp Tyr Val Asp Gly Val Glu Val His Asn Ala Lys Thr
    50                  55                  60
Lys Pro Arg Glu Glu Gln Tyr Asn Ser Thr Tyr Arg Val Val Ser Val
65                  70                  75                  80
Leu Thr Val Leu His Gln Asp Trp Leu Asn Gly Lys Glu Tyr Lys Cys
                85                  90                  95
Lys Val Ser Asn Lys Ala Leu Pro Ala Pro Ile Glu Lys Thr Ile Ser
            100                 105                 110
Lys Ala Lys Gly Gln Pro Arg Glu Pro Gln Val Tyr Thr Leu Pro Pro

        115                 120                 125
Ser Arg Asp Glu Leu Thr Lys Asn Gln Val Ser Leu Trp Cys Leu Val
    130                 135                 140
Lys Gly Phe Tyr Pro Ser Asp Ile Ala Val Glu Trp Glu Ser Asn Gly
145                 150                 155                 160
Gln Pro Glu Asn Asn Tyr Lys Thr Thr Pro Pro Val Leu Asp Ser Asp
                165                 170                 175
Gly Ser Phe Phe Leu Tyr Ser Lys Leu Thr Val Asp Lys Ser Arg Trp
            180                 185                 190
Gln Gln Gly Asn Val Phe Ser Cys Ser Val Met His Glu Ala Leu His
        195                 200                 205
Asn His Tyr Thr Gln Lys Ser Leu Ser Leu Ser Pro Gly Lys Gly Ser
    210                 215                 220
Glu Val Gln Leu Leu Val Ser Gly Gly Gly Leu Val Gln Pro Gly Gly
225                 230                 235                 240
Ser Leu Arg Leu Ser Cys Ala Ala Ser Gly Phe Thr Phe Lys Ala Tyr
                245                 250                 255
Pro Met Met Trp Val Arg Gln Ala Pro Gly Lys Gly Leu Glu Trp Val
            260                 265                 270
Ser Glu Ile Ser Pro Ser Gly Ser Tyr Thr Tyr Tyr Ala Asp Ser Val
        275                 280                 285
Lys Gly Arg Phe Thr Ile Ser Arg Asp Asn Ser Lys Asn Thr Leu Tyr
    290                 295                 300
Leu Gln Met Asn Ser Leu Arg Ala Glu Asp Thr Ala Val Tyr Tyr Cys
305                 310                 315                 320
Ala Lys Asp Pro Arg Lys Leu Asp Tyr Trp Gly Gln Gly Thr Leu Val
                325                 330                 335
Thr Val Ser Ser
            340
```

<210> 200
<211> 220
<212> PRT
<213> Artificial Sequence

<220>
<223> Humanised

<400> 200

Asp Ile Gln Met Thr Gln Ser Pro Ser Ser Leu Ser Ala Ser Val Gly
1 5 10 15
Asp Arg Val Thr Ile Thr Cys Lys Ser Ser Gln Ser Leu Leu Tyr Thr
20 25 30
Ser Ser Gln Lys Asn Tyr Leu Ala Trp Tyr Gln Gln Lys Pro Gly Lys
35 40 45
Ala Pro Lys Leu Leu Ile Tyr Trp Ala Ser Thr Arg Glu Ser Gly Val
50 55 60
Pro Ser Arg Phe Ser Gly Ser Gly Ser Gly Thr Asp Phe Thr Leu Thr
65 70 75 80
Ile Ser Ser Leu Gln Pro Glu Asp Phe Ala Thr Tyr Tyr Cys Gln Gln
85 90 95
Tyr Tyr Ala Tyr Pro Trp Thr Phe Gly Gln Gly Thr Lys Val Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 201
<211> 552
<212> PRT
<213> Artificial Sequence

<220>
<223> Humanised

<400> 201

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Thr Phe Thr Ser Tyr
            20                  25                  30
Trp Leu His Trp Val Arg Gln Ala Pro Gly Lys Gly Leu Glu Trp Val
        35                  40                  45
Gly Met Ile Asp Pro Ser Asn Ser Asp Thr Arg Phe Asn Pro Asn Phe
    50                  55                  60
Lys Asp Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Thr Tyr Arg Ser Tyr Val Thr Pro Leu Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Ala Pro Glu Phe Leu Gly Gly
    210                 215                 220
Pro Ser Val Phe Leu Phe Pro Pro Lys Pro Lys Asp Thr Leu Met Ile
225                 230                 235                 240
Ser Arg Thr Pro Glu Val Thr Cys Val Val Val Asp Val Ser Gln Glu
                245                 250                 255
Asp Pro Glu Val Gln Phe Asn Trp Tyr Val Asp Gly Val Glu Val His
            260                 265                 270
Asn Ala Lys Thr Lys Pro Arg Glu Glu Gln Phe Asn Ser Thr Tyr Arg
        275                 280                 285
Val Val Ser Val Leu Thr Val Leu His Gln Asp Trp Leu Asn Gly Lys
    290                 295                 300
Glu Tyr Lys Cys Lys Val Ser Asn Lys Gly Leu Pro Ser Ser Ile Glu
305                 310                 315                 320
Lys Thr Ile Ser Lys Ala Lys Gly Gln Pro Arg Glu Pro Gln Val Tyr
                325                 330                 335
Thr Leu Pro Pro Ser Gln Glu Glu Met Thr Lys Asn Gln Val Ser Leu
            340                 345                 350
Thr Cys Leu Val Lys Gly Phe Tyr Pro Ser Asp Ile Ala Val Glu Trp
        355                 360                 365
Glu Ser Asn Gly Gln Pro Glu Asn Asn Tyr Lys Thr Thr Pro Pro Val
    370                 375                 380
Leu Asp Ser Asp Gly Ser Phe Phe Leu Tyr Ser Arg Leu Thr Val Asp
385                 390                 395                 400
Lys Ser Arg Trp Gln Glu Gly Asn Val Phe Ser Cys Ser Val Met His
                405                 410                 415
Glu Ala Leu His Asn His Tyr Thr Gln Lys Ser Leu Ser Leu Ser Leu
            420                 425                 430
Gly Lys Gly Ser Glu Val Gln Leu Leu Val Ser Gly Gly Gly Leu Val
        435                 440                 445
Gln Pro Gly Gly Ser Leu Arg Leu Ser Cys Ala Ala Ser Gly Phe Thr
    450                 455                 460
Phe Lys Ala Tyr Pro Met Met Trp Val Arg Gln Ala Pro Gly Lys Gly
465                 470                 475                 480
Leu Glu Trp Val Ser Glu Ile Ser Pro Ser Gly Ser Tyr Thr Tyr Tyr
                485                 490                 495
Ala Asp Ser Val Lys Gly Arg Phe Thr Ile Ser Arg Asp Asn Ser Lys
            500                 505                 510
Asn Thr Leu Tyr Leu Gln Met Asn Ser Leu Arg Ala Glu Asp Thr Ala
        515                 520                 525
Val Tyr Tyr Cys Ala Lys Asp Pro Arg Lys Leu Asp Tyr Trp Gly Gln
    530                 535                 540
Gly Thr Leu Val Thr Val Ser Ser
545                 550
```

<210> 202
<211> 449
<212> PRT
<213> Artificial Sequence

<220>
<223> Humanised

<400> 202

Glu Val Gln Leu Val Glu Ser Gly Gly Gly Leu Val Gln Pro Gly Gly
1 5 10 15
Ser Leu Arg Leu Ser Cys Ala Ala Ser Gly Tyr Thr Phe Thr Ser Tyr
20 25 30
Trp Leu His Trp Val Arg Gln Ala Pro Gly Lys Gly Leu Glu Trp Val
35 40 45
Gly Met Ile Asp Pro Ser Asn Ser Asp Thr Arg Phe Asn Pro Asn Phe
50 55 60
Lys Asp Arg Phe Thr Ile Ser Ala Asp Thr Ser Lys Asn Thr Ala Tyr
65 70 75 80
Leu Gln Met Asn Ser Leu Arg Ala Glu Asp Thr Ala Val Tyr Tyr Cys
85 90 95
Ala Thr Tyr Arg Ser Tyr Val Thr Pro Leu Asp Tyr Trp Gly Gln Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Ser
355 360 365
Cys Ala Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Val Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro Gly
435 440 445
Lys

<210> 203
<211> 222
<212> PRT
<213> Artificial Sequence

<220>
<223> Humanised

<400> 203

```
Cys Pro Pro Cys Pro Ala Pro Glu Leu Leu Gly Gly Pro Ser Val Phe
1               5                   10                  15
Leu Phe Pro Pro Lys Pro Lys Asp Thr Leu Met Ile Ser Arg Thr Pro
            20                  25                  30
Glu Val Thr Cys Val Val Val Asp Val Ser His Glu Asp Pro Glu Val
        35                  40                  45
Lys Phe Asn Trp Tyr Val Asp Gly Val Glu Val His Asn Ala Lys Thr
    50                  55                  60
Lys Pro Arg Glu Glu Gln Tyr Asn Ser Thr Tyr Arg Val Val Ser Val
65                  70                  75                  80
Leu Thr Val Leu His Gln Asp Trp Leu Asn Gly Lys Glu Tyr Lys Cys
                85                  90                  95
Lys Val Ser Asn Lys Ala Leu Pro Ala Pro Ile Glu Lys Thr Ile Ser
            100                 105                 110
Lys Ala Lys Gly Gln Pro Arg Glu Pro Gln Val Tyr Thr Leu Pro Pro
        115                 120                 125
Ser Arg Asp Glu Leu Thr Lys Asn Gln Val Ser Leu Trp Cys Leu Val
    130                 135                 140
Lys Gly Phe Tyr Pro Ser Asp Ile Ala Val Glu Trp Glu Ser Asn Gly
145                 150                 155                 160
Gln Pro Glu Asn Asn Tyr Lys Thr Thr Pro Pro Val Leu Asp Ser Asp
                165                 170                 175
Gly Ser Phe Phe Leu Tyr Ser Lys Leu Thr Val Asp Lys Ser Arg Trp
            180                 185                 190
Gln Gln Gly Asn Val Phe Ser Cys Ser Val Met His Glu Ala Leu His
        195                 200                 205
Asn His Tyr Thr Gln Lys Ser Leu Ser Leu Ser Pro Gly Lys
    210                 215                 220
```

<210> 204
<211> 434
<212> PRT
<213> Artificial Sequence

<220>
<223> Humanised

<400> 204

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Thr Phe Thr Ser Tyr
            20                  25                  30
Trp Leu His Trp Val Arg Gln Ala Pro Gly Lys Gly Leu Glu Trp Val
        35                  40                  45
Gly Met Ile Asp Pro Ser Asn Ser Asp Thr Arg Phe Asn Pro Asn Phe
    50                  55                  60
Lys Asp Arg Phe Thr Ile Ser Ala Asp Thr Ser Lys Asn Thr Ala Tyr
65                  70                  75                  80
Leu Gln Met Asn Ser Leu Arg Ala Glu Asp Thr Ala Val Tyr Tyr Cys
                85                  90                  95
Ala Thr Tyr Arg Ser Tyr Val Thr Pro Leu Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Ala Pro Glu Phe Leu Gly Gly
    210                 215                 220
Pro Ser Val Phe Leu Phe Pro Pro Lys Pro Lys Asp Thr Leu Met Ile
225                 230                 235                 240
Ser Arg Thr Pro Glu Val Thr Cys Val Val Val Asp Val Ser Gln Glu
                245                 250                 255
Asp Pro Glu Val Gln Phe Asn Trp Tyr Val Asp Gly Val Glu Val His
            260                 265                 270
Asn Ala Lys Thr Lys Pro Arg Glu Glu Gln Phe Asn Ser Thr Tyr Arg
        275                 280                 285

Val Val Ser Val Leu Thr Val Leu His Gln Asp Trp Leu Asn Gly Lys
    290                 295                 300
Glu Tyr Lys Cys Lys Val Ser Asn Lys Gly Leu Pro Ser Ser Ile Glu
305                 310                 315                 320
Lys Thr Ile Ser Lys Ala Lys Gly Gln Pro Arg Glu Pro Gln Val Tyr
                325                 330                 335
Thr Leu Pro Pro Ser Gln Glu Glu Met Thr Lys Asn Gln Val Ser Leu
            340                 345                 350
Thr Cys Leu Val Lys Gly Phe Tyr Pro Ser Asp Ile Ala Val Glu Trp
        355                 360                 365
Glu Ser Asn Gly Gln Pro Glu Asn Asn Tyr Lys Thr Thr Pro Pro Val
    370                 375                 380
Leu Asp Ser Asp Gly Ser Phe Phe Leu Tyr Ser Arg Leu Thr Val Asp
385                 390                 395                 400
Lys Ser Arg Trp Gln Glu Gly Asn Val Phe Ser Cys Ser Val Met His
                405                 410                 415
Glu Ala Leu His Asn His Tyr Thr Gln Lys Ser Leu Ser Leu Ser Leu
            420                 425                 430
Gly Lys
```

<210> 205
<211> 1365
<212> DNA
<213> Artificial Sequence

<220>

<223> Humanised

<400> 205

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ccagcgccag caccaagggc cccagcgtgt tccccctggc ccccagcagc 420
aagagcacca gcggcggcac agccgccctg ggctgcctgg tgaaggacta cttccccgaa 480
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaag                 1365
```

<210> 206
<211> 1365
<212> DNA
<213> Artificial Sequence

<220>
<223> Humanised

<400> 206

```
caggtgcagc tggtgcagtc tggggctgag gtgaagaagc ctgggtcctc ggtgaaggtc 60
tcctgcaagg cttctggatt ctacattaaa gacacctata tgcactgggt gcgacaggcc 120
cctggacaag ggcttgagtg gatgggaacg attgatcctg cgaatggtaa tactaaatat 180
gtcccgaagt tccagggcag agtcacgatt accgcggacg aatccacgag cacagcctac 240
atggagctga gcagcctgag atctgaggac acggccgtgt attactgtgc gagaagcatc 300
tatgatgatt accactacga cgattactat gctatggact actggggcca agggacacta 360
gtcacagtct cctcagcctc caccaagggc ccatcggtct tccccctggc accctcctcc 420
aagagcacct ctgggggcac agcggccctg ggctgcctgg tcaaggacta cttccccgaa 480
ccggtgacgg tgtcgtggaa ctcaggcgcc ctgaccagcg gcgtgcacac cttcccggct 540
gtcctacagt cctcaggact ctactccctc agcagcgtgg tgaccgtgcc ctccagcagc 600
ttgggcaccc agacctacat ctgcaacgtg aatcacaagc ccagcaacac caaggtggac 660
aagaaagttg agcccaaatc ttgtgacaaa actcacacat gcccaccgtg cccagcacct 720
```

gaactcctgg ggggaccgtc agtcttcctc ttccccccaa aacccaagga caccctcatg 780
atctcccgga cccctgaggt cacatgcgtg gtggtggacg tgagccacga agaccctgag 840
gtcaagttca actggtacgt ggacggcgtg gaggtgcata atgccaagac aaagccgcgg 900
gaggagcagt acaacagcac gtaccgtgtg gtcagcgtcc tcaccgtcct gcaccaggac 960
tggctgaatg gcaaggagta caagtgcaag gtctccaaca aagccctccc agcccccatc 1020
gagaaaacca tctccaaagc caaagggcag ccccgagaac cacaggtgta caccctgccc 1080
ccatcccggg atgagctgac caagaaccag gtcagcctga cctgcctggt caaaggcttc 1140
tatcccagcg acatcgccgt ggagtgggag agcaatgggc agccggagaa caactacaag 1200
accacgcctc ccgtgctgga ctccgacggc tccttcttcc tctacagcaa gctcaccgtg 1260
gacaagagca ggtggcagca ggggaacgtc ttctcatgct ccgtgatgca tgaggctctg 1320
cacaaccact acacgcagaa gagcctctcc ctgtctccgg gtaaa 1365

<210> 207
<211> 567
<212> PRT
<213> Artificial Sequence

<220>
<223> Humanised

<400> 207

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Asn Phe Gly Thr Gln Thr Tyr Thr Cys Asn Val Asp His
        195                 200                 205
Lys Pro Ser Asn Thr Lys Val Asp Lys Thr Val Glu Arg Lys Cys Cys
    210                 215                 220
Val Glu Cys Pro Pro Cys Pro Ala Pro Pro Val Ala Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser His Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Phe Arg Val Val Ser
    290                 295                 300
Val Leu Thr Val Val His Gln Asp Trp Leu Asn Gly Lys Glu Tyr Lys
305                 310                 315                 320
Cys Lys Val Ser Asn Lys Gly Leu Pro Ala Pro Ile Glu Lys Thr Ile
                325                 330                 335
Ser Lys Thr Lys Gly Gln Pro Arg Glu Pro Gln Val Tyr Thr Leu Pro
            340                 345                 350
Pro Ser Arg Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Met Leu Asp Ser
385                 390                 395                 400
Asp Gly Ser Phe Phe Leu Tyr Ser Lys Leu Thr Val Asp Lys Ser Arg
                405                 410                 415
Trp Gln Gln Gly Asn Val Phe Ser Cys Ser Val Met His Glu Ala Leu
            420                 425                 430
His Asn His Tyr Thr Gln Lys Ser Leu Ser Leu Ser Pro Gly Lys Gly
```

```
                435                     440                     445
Ser Gly Val Gln Leu Leu Glu Ser Gly Gly Gly Leu Val Gln Pro Gly
        450                     455                     460
Gly Ser Leu Arg Leu Ser Cys Ala Ala Ser Gly Phe Thr Phe Ala Trp
465                     470                     475                 480
Tyr Asp Met Gly Trp Val Arg Gln Ala Pro Gly Lys Gly Leu Glu Trp
                    485                     490                 495
Val Ser Ser Ile Asp Trp His Gly Glu Val Thr Tyr Tyr Ala Asp Ser
            500                     505                     510
Val Lys Gly Arg Phe Thr Ile Ser Arg Asp Asn Ser Lys Asn Thr Leu
        515                     520                     525
Tyr Leu Gln Met Asn Ser Leu Arg Ala Glu Asp Thr Ala Val Tyr Tyr
    530                     535                     540
Cys Ala Thr Ala Glu Asp Glu Pro Gly Tyr Asp Tyr Trp Gly Gln Gly
545                     550                     555                 560
Thr Leu Val Thr Val Ser Ser
                565
```

<210> 208
<211> 568
<212> PRT
<213> Artificial Sequence

<220>
<223> Humanised

<400> 208

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Ser Trp Ile Arg Gln Pro Pro Gly Lys Gly Leu Glu
        35                  40                  45
Trp Leu Ala His Ile Tyr Trp Asp Asp Asp Lys Arg Tyr Asn Pro Ser
    50                  55                  60
Leu Lys Ser Arg Leu Thr Ile Ser Lys Asp Thr Ser Arg Asn Gln Val
65                  70                  75                  80
Val Leu Thr Met Thr Asn Met Asp Pro Val Asp Thr Ala Thr Tyr Tyr
                85                  90                  95
Cys Ala Arg Arg Glu Thr Val Phe Tyr Trp Tyr Phe Asp Val Trp Gly
            100                 105                 110
Arg Gly Thr Leu Val Thr Val Ser Ser Ala Ser Thr Lys Gly Pro Ser
        115                 120                 125
Val Phe Pro Leu Ala Pro Cys Ser Arg Ser Thr Ser Glu Ser Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Lys Thr Tyr Thr Cys Asn Val Asp His
        195                 200                 205
Lys Pro Ser Asn Thr Lys Val Asp Lys Arg Val Glu Ser Lys Tyr Gly
    210                 215                 220
Pro Pro Cys Pro Ser Cys Pro Ala Pro Glu Phe Leu Gly Gly Pro Ser
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser Gln Glu Asp Pro
            260                 265                 270
Glu Val Gln Phe Asn Trp Tyr Val Asp Gly Val Glu Val His Asn Ala
        275                 280                 285
Lys Thr Lys Pro Arg Glu Glu Gln Phe Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Gly Leu Pro Ser Ser Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Gln Glu Glu Met Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
```

Asn Gly Gln Pro Glu Asn Asn Tyr Lys Thr Thr Pro Pro Val Leu Asp
385 390 395 400
Ser Asp Gly Ser Phe Phe Leu Tyr Ser Arg Leu Thr Val Asp Lys Ser
405 410 415
Arg Trp Gln Glu Gly Asn Val Phe Ser Cys Ser Val Met His Glu Ala
420 425 430
Leu His Asn His Tyr Thr Gln Lys Ser Leu Ser Leu Ser Leu Gly Lys
435 440 445
Gly Ser Gly Val Gln Leu Leu Glu Ser Gly Gly Gly Leu Val Gln Pro
450 455 460
Gly Gly Ser Leu Arg Leu Ser Cys Ala Ala Ser Gly Phe Thr Phe Ala
465 470 475 480
Trp Tyr Asp Met Gly Trp Val Arg Gln Ala Pro Gly Lys Gly Leu Glu
485 490 495
Trp Val Ser Ser Ile Asp Trp His Gly Glu Val Thr Tyr Tyr Ala Asp
500 505 510
Ser Val Lys Gly Arg Phe Thr Ile Ser Arg Asp Asn Ser Lys Asn Thr
515 520 525
Leu Tyr Leu Gln Met Asn Ser Leu Arg Ala Glu Asp Thr Ala Val Tyr
530 535 540
Tyr Cys Ala Thr Ala Glu Asp Glu Pro Gly Tyr Asp Tyr Trp Gly Gln
545 550 555 560
Gly Thr Leu Val Thr Val Ser Ser
565

<210> 209
<211> 568
<212> PRT
<213> Artificial sequence

<220>
<223> Humanised

<400> 209

Gln Val Thr Leu Arg Glu Ser Gly Pro Ala Leu Val Lys Pro Thr Gln
1 5 10 15
Thr Leu Thr Leu Thr Cys Thr Phe Ser Gly Phe Ser Leu Ser Thr Ser
20 25 30
Gly Met Gly Val Ser Trp Ile Arg Gln Pro Pro Gly Lys Gly Leu Glu
35 40 45
Trp Leu Ala His Ile Tyr Trp Asp Asp Asp Lys Arg Tyr Asn Pro Ser
50 55 60
Leu Lys Ser Arg Leu Thr Ile Ser Lys Asp Thr Ser Arg Asn Gln Val
65 70 75 80
Val Leu Thr Met Thr Asn Met Asp Pro Val Asp Thr Ala Thr Tyr Tyr
85 90 95
Cys Ala Arg Arg Glu Thr Val Phe Tyr Trp Tyr Phe Asp Val Trp Gly
100 105 110
Arg Gly Thr Leu Val Thr Val Ser Ser Ala Ser Thr Lys Gly Pro Ser
115 120 125
Val Phe Pro Leu Ala Pro Cys Ser Arg Ser Thr Ser Glu Ser Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Lys Thr Tyr Thr Cys Asn Val Asp His
195 200 205
Lys Pro Ser Asn Thr Lys Val Asp Lys Arg Val Glu Ser Lys Tyr Gly
210 215 220
Pro Pro Cys Pro Pro Cys Pro Ala Pro Glu Phe Glu Gly Gly Pro Ser
225 230 235 240
Val Phe Leu Phe Pro Pro Lys Pro Lys Asp Thr Leu Met Ile Ser Arg
245 250 255
Thr Pro Glu Val Thr Cys Val Val Val Asp Val Ser Gln Glu Asp Pro
260 265 270
Glu Val Gln Phe Asn Trp Tyr Val Asp Gly Val Glu Val His Asn Ala
275 280 285
Lys Thr Lys Pro Arg Glu Glu Gln Phe Asn Ser Thr Tyr Arg Val Val
290 295 300
Ser Val Leu Thr Val Leu His Gln Asp Trp Leu Asn Gly Lys Glu Tyr
305 310 315 320
Lys Cys Lys Val Ser Asn Lys Gly Leu Pro Ser Ser Ile Glu Lys Thr

```
                        325                         330                     335
Ile Ser Lys Ala Lys Gly Gln Pro Arg Glu Pro Gln Val Tyr Thr Leu
            340                     345                     350
Pro Pro Ser Gln Glu Glu Met Thr Lys Asn Gln Val Ser Leu Thr Cys
        355                     360                     365
Leu Val Lys Gly Phe Tyr Pro Ser Asp Ile Ala Val Glu Trp Glu Ser
    370                     375                     380
Asn Gly Gln Pro Glu Asn Asn Tyr Lys Thr Thr Pro Pro Val Leu Asp
385                     390                     395                     400
Ser Asp Gly Ser Phe Phe Leu Tyr Ser Arg Leu Thr Val Asp Lys Ser
                405                     410                     415
Arg Trp Gln Glu Gly Asn Val Phe Ser Cys Ser Val Met His Glu Ala
            420                     425                     430
Leu His Asn His Tyr Thr Gln Lys Ser Leu Ser Leu Ser Leu Gly Lys
        435                     440                     445
Gly Ser Gly Val Gln Leu Leu Glu Ser Gly Gly Gly Leu Val Gln Pro
    450                     455                     460
Gly Gly Ser Leu Arg Leu Ser Cys Ala Ala Ser Gly Phe Thr Phe Ala
465                     470                     475                     480
Trp Tyr Asp Met Gly Trp Val Arg Gln Ala Pro Gly Lys Gly Leu Glu
                485                     490                     495
Trp Val Ser Ser Ile Asp Trp His Gly Glu Val Thr Tyr Tyr Ala Asp
            500                     505                     510
Ser Val Lys Gly Arg Phe Thr Ile Ser Arg Asp Asn Ser Lys Asn Thr
        515                     520                     525
Leu Tyr Leu Gln Met Asn Ser Leu Arg Ala Glu Asp Thr Ala Val Tyr
    530                     535                     540
Tyr Cys Ala Thr Ala Glu Asp Glu Pro Gly Tyr Asp Tyr Trp Gly Gln
545                     550                     555                     560
Gly Thr Leu Val Thr Val Ser Ser
                565
```

<210> 210
<211> 657
<212> DNA
<213> Artificial Sequence

<220>
<223> Humanised

<400> 210

```
gacatcgtga tgacccagtc tcctctgagc ctccccgtga cccccggcga accagccagc 60
atctcctgca gaagcagcca gaacatcgtg cacatcaacg gcaacaccta cctggagtgg 120
tacctgcaaa agcccggcca gagccccagg ctgctgatct acaagatcag cgacaggttc 180
agcggcgtgc ccgataggtt cagcggcagc ggcagcggca ccgacttcac cctgaagatc 240
agcagggtgg aggccgacga cgtgggcatc tactactgct tccagggcag ccacgtcccc 300
tggactttcg gacagggcac caagctggag attaagcgta cggtggccgc ccccagcgtg 360
ttcatcttcc cccccagcga tgagcagctg aagagcggca ccgccagcgt ggtgtgtctg 420
ctgaacaact tctacccccg ggaggccaag gtgcagtgga aggtggacaa tgccctgcag 480
agcggcaaca gccaggagag cgtgaccgag caggacagca aggactccac ctacagcctg 540
agcagcaccc tgaccctgag caaggccgac tacgagaagc acaaggtgta cgcctgtgag 600
gtgacccacc agggcctgtc cagccccgtg accaagagct tcaaccgggg cgagtgc    657
```

<210> 211
<211> 1353
<212> DNA
<213> Artificial Sequence

<220>
<223> Humanised

<400> 211

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780

cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353
```

<210> 212
<211> 654
<212> DNA
<213> Artificial Sequence

<220>
<223> Humanised

<400> 212

```
gacatcgtgc tgacccagag cccctcttcc ctgagcgcaa gcgtgggcga tagggtgacc 60
atcacctgca aggccagcca gagcgtggac tacgacggcg acagctacat gaactggtac 120
cagcagaagc ccggcaaggc ccccaaactg ctgatctacg ccgccagcaa cctcgagtca 180
ggcattccca gcaggtttag cggcagcggc agcggcaccg acttcacctt cacaatcagc 240
agcctgcagc ccgaggacat cgccacctac tactgccagc agagcaacga ggaccctccc 300
accttcggac agggcaccaa ggtcgagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgc 654
```

<210> 213
<211> 1347
<212> DNA
<213> Artificial Sequence

<220>
<223> Humanised

<400> 213

```
caggtgaccc tgagggagag cggccccgcc ctggtgaagc ccacacagac cctcactctg 60
acctgcaccg tgagcggctt cagcctgacc tcctacagcg tccactgggt gaggcagccc 120
cccggcaagg gcctggagtg gctgggcgtg atctgggcaa gcggcggcac cgactacaac 180
agcgccctga tgagcaggct ctccatcagc aaggacacca gccggaacca ggtggtgctg 240
accatgacca acatggaccc cgtggacacc gccacctatt actgcgccag ggaccctccc 300
tctagcctgc tgaggctgga ctactggggc aggggaacac tagtgaccgt gtccagcgcc 360
agcaccaagg gccccagcgt gttccccctg gccccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gacctgcctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtacagc aagctgaccg tggacaagag cagatggcag 1260
cagggcaacg tgttcagctg ctccgtgatg cacgaggccc tgcacaatca ctacacccag 1320
aagagcctga gcctgtcccc tggcaag 1347
```

<210> 214
<211> 660
<212> DNA
<213> Artificial Sequence

<220>
<223> Humanised

<400> 214

```
gacatcgtga tgacccagtc tcccgattca ctggccgtga gcctgggcga gagggccacc 60
atcaactgca agagcagcca gagcctcctg aacagcggca accagaagaa ctacctggcc 120

tggtaccagc agaaacccgg ccagcccccc aagctgctga tctatggcgc ctccaccagg 180
gagagcggcg tgccagacag gtttagcggc agcggcagcg gcaccgactt caccctgaca 240
atcagcagcc tgcaggccga ggacgtggcc gtgtactact gccagaacgt ccacagcttc 300
cccttcacct tcggcggggg aaccaagctg gagatcaagc gtacggtggc cgcccccagc 360
gtgttcatct tcccccccag cgatgagcag ctgaagagcg gcaccgccag cgtggtgtgt 420
ctgctgaaca acttctaccc ccgggaggcc aaggtgcagt ggaaggtgga caatgccctg 480
cagagcggca acagccagga gagcgtgacc gagcaggaca gcaaggactc cacctacagc 540
ctgagcagca ccctgaccct gagcaaggcc gactacgaga agcacaaggt gtacgcctgt 600
gaggtgaccc accagggcct gtccagcccc gtgaccaaga gcttcaaccg gggcgagtgc 660
```

<210> 215
<211> 1713
<212> DNA
<213> Artificial Sequence

<220>
<223> Humanised

<400> 215

caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt ccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggatccg gcgtgcagct cctggagagc 1380
ggcggaggcc tggtccagcc cggcggcagc ctgaggctga gctgcgccgc cagcggcttc 1440
accttcgcct ggtatgatat gggctgggtg aggcaggccc ccggcaaggg cctggagtgg 1500
gtgtccagca tcgactggca cggggaggtg acctactacg ccgacagcgt gaagggcagg 1560
ttcaccatca gcagggacaa cagcaagaac accctgtacc tgcagatgaa cagcctgagg 1620
gccgaggaca ccgcagtgta ctactgcgcc accgccgagg acgaacccgg ctacgactac 1680
tggggccagg gcaccctggt gactgtgagc agc 1713

<210> 216
<211> 1731
<212> DNA
<213> Artificial Sequence

<220>
<223> Humanised

<400> 216

caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt ccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020

agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgccccctc gggatccggc 1380
gtgcagctcc tggagagcgg cggaggcctg gtccagcccg gcggcagcct gaggctgagc 1440
tgcgccgcca gcggcttcac cttcgcctgg tatgatatgg gctgggtgag gcaggccccc 1500
ggcaagggcc tggagtgggt gtccagcatc gactggcacg gggaggtgac ctactacgcc 1560
gacagcgtga agggcaggtt caccatcagc agggacaaca gcaagaacac cctgtacctg 1620
cagatgaaca gcctgagggc cgaggacacc gcagtgtact actgcgccac cgccgaggac 1680
gaacccggct acgactactg gggccagggc accctggtga ctgtgagcag c 1731

<210> 217
<211> 1014
<212> DNA
<213> Artificial Sequence

<220>
<223> Humanised

<400> 217

gacatcgtgc tgacccagag cccctcttcc ctgagcgcaa gcgtgggcga tagggtgacc 60
atcacctgca aggccagcca gagcgtggac tacgacggcg acagctacat gaactggtac 120
cagcagaagc ccggcaaggc ccccaaactg ctgatctacg ccgccagcaa cctcgagtca 180
ggcattccca gcaggtttag cggcagcggc agcggcaccg acttcacctt cacaatcagc 240
agcctgcagc ccgaggacat cgccacctac tactgccagc agagcaacga ggaccctccc 300
accttcggac agggcaccaa ggtcgagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgtggatcc 660
ggcgtgcagc tcctggagag cggcggaggc ctggtccagc ccggcggcag cctgaggctg 720
agctgcgccg ccagcggctt caccttcgcc tggtatgata tgggctgggt gaggcaggcc 780
cccggcaagg gcctggagtg ggtgtccagc atcgactggc acggggaggt gacctactac 840
gccgacagcg tgaagggcag gttcaccatc agcagggaca acagcaagaa caccctgtac 900
ctgcagatga acagcctgag ggccgaggac accgcagtgt actactgcgc caccgccgag 960
gacgaacccg gctacgacta ctggggccag ggcaccctgg tgactgtgag cagc 1014

<210> 218
<211> 1032
<212> DNA
<213> Artificial Sequence

<220>
<223> Humanised

<400> 218

```
gacatcgtgc tgacccagag cccctcttcc ctgagcgcaa gcgtgggcga tagggtgacc 60
atcacctgca aggccagcca gagcgtggac tacgacggcg acagctacat gaactggtac 120
cagcagaagc ccggcaaggc ccccaaactg ctgatctacg ccgccagcaa cctcgagtca 180
ggcattccca gcaggtttag cggcagcggc agcggcaccg acttcacctt cacaatcagc 240
agcctgcagc ccgaggacat cgccacctac tactgccagc agagcaacga ggaccctccc 300
accttcggac agggcaccaa ggtcgagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgcaccgtg 660
gccgccccct cgggatccgg cgtgcagctc ctggagagcg gcggaggcct ggtccagccc 720
ggcggcagcc tgaggctgag ctgcgccgcc agcggcttca ccttcgcctg gtatgatatg 780
ggctgggtga ggcaggcccc cggcaagggc ctggagtggg tgtccagcat cgactggcac 840
ggggaggtga cctactacgc cgacagcgtg aagggcaggt tcaccatcag cagggacaac 900
agcaagaaca ccctgtacct gcagatgaac agcctgaggg ccgaggacac cgcagtgtac 960
tactgcgcca ccgccgagga cgaacccggc tacgactact ggggccaggg caccctggtg 1020
actgtgagca gc                                                    1032
```

<210> 219

<211> 1710

<212> DNA

<213> Artificial Sequence

<220>

<223> Humanised

<400> 219

```
caggtgaccc tgagggagag cggccccgcc ctggtgaagc ccacacagac cctcactctg 60
acctgcaccg tgagcggctt cagcctgacc tcctacagcg tccactgggt gaggcagccc 120
cccggcaagg gcctggagtg gctgggcgtg atctgggcaa gcggcggcac cgactacaac 180
agcgccctga tgagcaggct ctccatcagc aaggacacca gccggaacca ggtggtgctg 240
accatgacca acatggaccc cgtggacacc gccacctatt actgcgccag ggaccctccc 300
tctagcctgc tgaggctgga ctactggggc aggggaacac tagtgaccgt gtccagcgcc 360
agcaccaagg gccccagcgt gttccccctg gcccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gacctgcctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtacagc aagctgaccg tggacaagag cagatggcag 1260
cagggcaacg tgttcagctg ctccgtgatg cacgaggccc tgcacaatca ctacacccag 1320
aagagcctga gcctgtcccc tggcaagggc ggcggcggat ccgaagtgca gctcctggag 1380
agcggcggcg gcctggtgca gcccggcggc agcctgaggc tgagctgcgc cgctagcggc 1440
ttcaccttca ggaacttcgg catgggctgg gtcaggcagg cccccggcaa gggcctggag 1500
tgggtcagct ggatcatcag ctccggcacc gagacctact acgccgacag cgtgaagggc 1560
aggttcacca tcagccgcga caacagcaag aacaccctgt acctgcagat gaacagcctg 1620
agggccgagg acaccgccgt ctactactgc gccaagagcc tgggcaggtt cgactactgg 1680
ggacagggga ccctggtgac tgtgagcagc                                 1710
```

<210> 220

<211> 1029

<212> DNA

<213> Artificial Sequence

<220>
<223> Humanised

<400> 220

```
gacatcgtga tgacccagtc tcccgattca ctggccgtga gcctgggcga gagggccacc 60
atcaactgca agagcagcca gagcctcctg aacagcggca accagaagaa ctacctggcc 120
tggtaccagc agaaacccgg ccagcccccc aagctgctga tctatggcgc ctccaccagg 180
gagagcggcg tgccagacag gtttagcggc agcggcagcg gcaccgactt caccctgaca 240
atcagcagcc tgcaggccga ggacgtggcc gtgtactact gccagaacgt ccacagcttc 300
cccttcacct tcggcggggg aaccaagctg gagatcaagc gtacggtggc cgcccccagc 360
gtgttcatct tccccccag cgatgagcag ctgaagagcg gcaccgccag cgtggtgtgt 420
ctgctgaaca acttctaccc ccgggaggcc aaggtgcagt ggaaggtgga caatgccctg 480
cagagcggca acagccagga gagcgtgacc gagcaggaca gcaaggactc cacctacagc 540
ctgagcagca ccctgaccct gagcaaggcc gactacgaga agcacaaggt gtacgcctgt 600
gaggtgaccc accagggcct gtccagcccc gtgaccaaga gcttcaaccg gggcgagtgc 660
ggcggcggcg gatccggcgt gcagctcctg gagagcggcg gaggcctggt ccagcccggc 720
ggcagcctga ggctgagctg cgccgccagc ggcttcacct tcgcctggta tgatatgggc 780
tgggtgaggc aggcccccgg caagggcctg gagtgggtgt ccagcatcga ctggcacggg 840
gaggtgacct actacgccga cagcgtgaag ggcaggttca ccatcagcag ggacaacagc 900
aagaacaccc tgtacctgca gatgaacagc ctgagggccg aggacaccgc agtgtactac 960
tgcgccaccg ccgaggacga acccggctac gactactggg gccagggcac cctggtgact 1020
gtgagcagc                                                         1029
```

<210> 221
<211> 1713
<212> DNA
<213> Artificial Sequence

<220>
<223> Humanised

<400> 221

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ccagcgccag caccaagggc cccagcgtgt tcccccctggc ccccagcagc 420
aagagcacca gcggcggcac agccgccctg ggctgcctgg tgaaggacta cttccccgaa 480
```

```
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaaggaagt gcagctcctg 1380
gagagcggcg gcggcctggt gcagcccggc ggcagcctga ggctgagctg cgccgctagc 1440
ggcttcacct tcaggaactt cggcatgggc tgggtcaggc aggcccccgg caagggcctg 1500
gagtgggtca gctggatcat cagctccggc accgagacct actacgccga cagcgtgaag 1560
ggcaggttca ccatcagccg cgacaacagc aagaacaccc tgtacctgca gatgaacagc 1620
ctgagggccg aggacaccgc cgtctactac tgcgccaaga gcctgggcag gttcgactac 1680
tggggacagg ggaccctggt gactgtgagc agc                              1713
```

<210> 222
<211> 1731
<212> DNA
<213> Artificial Sequence

<220>
<223> Humanised

<400> 222

```
caggtgcagc tcgtgcagag cggcgccgaa gtgaaaaagc ccggcagcag cgtgaaggtg 60
agctgcaagg cctccggctt ctacatcaag gacacctaca tgcactgggt caggcaggct 120
cctggccagg gcctggagtg gatgggcact atcgaccccg ccaacggcaa caccaagtac 180
gtgcccaagt tccagggcag ggtgaccatc accgccgatg agagcaccag caccgcctac 240
atggaactga gcagcctgag gtctgaggac accgccgtgt actattgcgc caggagcatc 300
tacgacgact accactacga cgactactac gccatggact actggggaca gggcacacta 360
gtgaccgtgt ctagcgccag caccaagggc cccagcgtgt tccccctggc ccccagcagc 420
aagagcacca gcggcggcac agccgccctg ggctgcctgg tgaaggacta cttccccgaa 480
ccggtgaccg tgtcctggaa cagcggagcc ctgaccagcg gcgtgcacac cttccccgcc 540
gtgctgcaga gcagcggcct gtacagcctg agcagcgtgg tgaccgtgcc cagcagcagc 600
ctgggcaccc agacctacat ctgtaacgtg aaccacaagc ccagcaacac caaggtggac 660
aagaaggtgg agcccaagag ctgtgacaag acccacacct gccccccctg ccctgccccc 720
gagctgctgg gaggccccag cgtgttcctg ttccccccca agcctaagga caccctgatg 780
atcagcagaa cccccgaggt gacctgtgtg gtggtggatg tgagccacga ggaccctgag 840
gtgaagttca actggtacgt ggacggcgtg gaggtgcaca atgccaagac caagcccagg 900
gaggagcagt acaacagcac ctaccgggtg gtgtccgtgc tgaccgtgct gcaccaggat 960
tggctgaacg gcaaggagta caagtgtaag gtgtccaaca aggccctgcc tgcccctatc 1020
gagaaaacca tcagcaaggc caagggccag cccagagagc cccaggtgta caccctgccc 1080
cctagcagag atgagctgac caagaaccag gtgtccctga cctgcctggt gaagggcttc 1140
taccccagcg acatcgccgt ggagtgggag agcaacggcc agcccgagaa caactacaag 1200
accacccccc ctgtgctgga cagcgatggc agcttcttcc tgtacagcaa gctgaccgtg 1260
gacaagagca gatggcagca gggcaacgtg ttcagctgct ccgtgatgca cgaggccctg 1320
cacaatcact acacccagaa gagcctgagc ctgtcccctg gcaagaccgt ggccgccccc 1380
tcggaagtgc agctcctgga gagcggcggc ggcctggtgc agcccggcgg cagcctgagg 1440
ctgagctgcg ccgctagcgg cttcaccttc aggaacttcg gcatgggctg ggtcaggcag 1500
gcccccggca agggcctgga gtgggtcagc tggatcatca gctccggcac cgagacctac 1560
tacgccgaca gcgtgaaggg caggttcacc atcagccgcg acaacagcaa gaacaccctg 1620
tacctgcaga tgaacagcct gagggccgag gacaccgccg tctactactg cgccaagagc 1680
ctgggcaggt tcgactactg gggacagggg accctggtga ctgtgagcag c          1731
```

<210> 223

<211> 1707
<212> DNA
<213> Artificial Sequence

<220>
<223> Humanised

<400> 223

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360

agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt ccccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggcgtgc agctcctgga gagcggcgga 1380
ggcctggtcc agcccggcgg cagcctgagg ctgagctgcg ccgccagcgg cttcaccttc 1440
gcctggtatg atatgggctg ggtgaggcag gcccccggca agggcctgga gtgggtgtcc 1500
agcatcgact ggcacgggga ggtgacctac tacgccgaca gcgtgaaggg caggttcacc 1560
atcagcaggg acaacagcaa gaacaccctg tacctgcaga tgaacagcct gagggccgag 1620
gacaccgcag tgtactactg cgccaccgcc gaggacgaac ccggctacga ctactggggc 1680
cagggcaccc tggtgactgt gagcagc 1707
```

<210> 224
<211> 1725
<212> DNA
<213> Artificial Sequence

<220>
<223> Humanised

<400> 224

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgccccctc gggcgtgcag 1380
ctcctggaga gcggcggagg cctggtccag cccggcggca gcctgaggct gagctgcgcc 1440
gccagcggct tcaccttcgc ctggtatgat atgggctggg tgaggcaggc ccccggcaag 1500
ggcctggagt gggtgtccag catcgactgg cacggggagg tgacctacta cgccgacagc 1560
gtgaagggca ggttcaccat cagcagggac aacagcaaga acaccctgta cctgcagatg 1620
aacagcctga gggccgagga caccgcagtg tactactgcg ccaccgccga ggacgaaccc 1680
ggctacgact actggggcca gggcaccctg gtgactgtga gcagc 1725
```

<210> 225
<211> 1734
<212> DNA
<213> Artificial Sequence

<220>
<223> Humanised

<400> 225

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
```

```
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggatcag ccagcaccaa gggccccacg 1380
ggcgtgcagc tcctggagag cggcggaggc ctggtccagc ccggcggcag cctgaggctg 1440
agctgcgccg ccagcggctt caccttcgcc tggtatgata tgggctgggt gaggcaggcc 1500
cccggcaagg gcctggagtg ggtgtccagc atcgactggc acggggaggt gacctactac 1560
gccgacagcg tgaagggcag gttcaccatc agcagggaca acagcaagaa caccctgtac 1620
ctgcagatga acagcctgag ggccgaggac accgcagtgt actactgcgc caccgccgag 1680
gacgaacccg gctacgacta ctggggccag ggcaccctgg tgactgtgag cagc       1734
```

<210> 226
<211> 1731
<212> DNA
<213> Artificial Sequence

<220>
<223> Humanised

<400> 226

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
cccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaaga ccgtggccgc cccctcggga 1380
tccgaggtgc agctcctggt cagcggcggc ggcctggtcc agcccggagg ctcactgagg 1440
ctgagctgcg ccgctagcgg cttcaccttc aaggcctacc ccatgatgtg ggtcaggcag 1500
gcccccggca aaggcctgga gtgggtgtct gagatcagcc ccagcggcag ctacacctac 1560
tacgccgaca gcgtgaaggg caggttcacc atcagcaggg acaacagcaa gaacaccctg 1620
tacctgcaga tgaactctct gagggccgag gacaccgccg tgtactactg cgccaaggac 1680
cccaggaagc tggactattg ggccagggc actctggtga ccgtgagcag c          1731
```

<210> 227
<211> 1713
<212> DNA
<213> Artificial Sequence

<220>
<223> Humanised

<400> 227

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
```

```
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaagg gatccgaggt gcagctcctg 1380
gtcagcggcg gcggcctggt ccagcccgga ggctcactga ggctgagctg cgccgctagc 1440
ggcttcacct tcaaggccta ccccatgatg tgggtcaggc aggcccccgg caaaggcctg 1500
gagtgggtgt ctgagatcag ccccagcggc agctacacct actacgccga cagcgtgaag 1560
ggcaggttca ccatcagcag ggacaacagc aagaacaccc tgtacctgca gatgaactct 1620
ctgagggccg aggacaccgc cgtgtactac tgcgccaagg accccaggaa gctggactat 1680
tggggccagg gcactctggt gaccgtgagc agc 1713
```

<210> 228
<211> 1359
<212> DNA
<213> Artificial Sequence

<220>
<223> Humanised

<400> 228

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaag 1359
```

<210> 229
<211> 1029
<212> DNA
<213> Artificial Sequence

<220>
<223> Humanised

<400> 229

```
gacatcgtga tgacccagag cccccctgagc ctgcccgtga cccctggcga gcccgccagc 60
atcagctgca gaagcagcca gagcatcgtc cagagcaacg gcgacaccta cctggaatgg 120
tatctgcaga agcccggcca gtcccccccag ctgctgatct acagagtgag caaccggttc 180
agcggcgtgc ccgacagatt cagcggcagc ggctccggca ccgacttcac cctgaagatc 240
agccgggtgg aggccgagga cgtgggcgtg tactactgct ttcaaggcag ccacgtgccc 300
tacaccttcg gccagggcac caagctggaa atcaagcgta cggtggccgc cccccagcgtg 360
```

```
ttcatcttcc cccccagcga tgagcagctg aagagcggca ccgccagcgt ggtgtgtctg 420
ctgaacaact tctacccccg ggaggccaag gtgcagtgga aggtggacaa tgccctgcag 480
agcggcaaca gccaggagag cgtgaccgag caggacagca aggactccac ctacagcctg 540
agcagcaccc tgaccctgag caaggccgac tacgagaagc acaaggtgta cgcctgtgag 600
gtgacccacc agggcctgtc cagccccgtg accaagagct tcaaccgggg cgagtgcacc 660
gtggccgccc cctcgggatc cgaggtgcag ctcctggtca gcggcggcgg cctggtccag 720
cccggaggct cactgaggct gagctgcgcc gctagcggct tcaccttcaa ggcctacccc 780
atgatgtggg tcaggcaggc cccccggcaaa ggcctggagt gggtgtctga gatcagcccc 840
agcggcagct acacctacta cgccgacagc gtgaagggca ggttcaccat cagcagggac 900
aacagcaaga acaccctgta cctgcagatg aactctctga gggccgagga caccgccgtg 960
tactactgcg ccaaggaccc caggaagctg gactattggg gccagggcac tctggtgacc 1020
gtgagcagc 1029
```

<210> 230
<211> 1011
<212> DNA
<213> Artificial Sequence

<220>
<223> Humanised

<400> 230

```
gacatcgtga tgacccagag cccccctgagc ctgcccgtga cccctggcga gcccgccagc 60
atcagctgca gaagcagcca gagcatcgtc cagagcaacg gcgacaccta cctggaatgg 120
tatctgcaga agcccggcca gtcccccccag ctgctgatct acagagtgag caaccggttc 180
agcggcgtgc ccgacagatt cagcggcagc ggctccggca ccgacttcac cctgaagatc 240
agccgggtgg aggccgagga cgtgggcgtg tactactgct ttcaaggcag ccacgtgccc 300
tacaccttcg gccagggcac caagctggaa atcaagcgta cggtggccgc cccccagcgtg 360
ttcatcttcc cccccagcga tgagcagctg aagagcggca ccgccagcgt ggtgtgtctg 420
ctgaacaact tctacccccg ggaggccaag gtgcagtgga aggtggacaa tgccctgcag 480
agcggcaaca gccaggagag cgtgaccgag caggacagca aggactccac ctacagcctg 540
agcagcaccc tgaccctgag caaggccgac tacgagaagc acaaggtgta cgcctgtgag 600
gtgacccacc agggcctgtc cagccccgtg accaagagct tcaaccgggg cgagtgtgga 660
tccgaggtgc agctcctggt cagcggcggc ggcctggtcc agcccggagg ctcactgagg 720
ctgagctgcg ccgctagcgg cttcaccttc aaggcctacc ccatgatgtg ggtcaggcag 780
gcccccggca aaggcctgga gtgggtgtct gagatcagcc ccagcggcag ctacacctac 840
tacgccgaca gcgtgaaggg caggttcacc atcagcaggg acaacagcaa gaacaccctg 900
tacctgcaga tgaactctct gagggccgag gacaccgccg tgtactactg cgccaaggac 960
cccaggaagc tggactattg gggccagggc actctggtga ccgtgagcag c 1011
```

<210> 231
<211> 657
<212> DNA
<213> Artificial Sequence

<220>
<223> Humanised

<400> 231

```
gacatcgtga tgacccagag cccccctgagc ctgcccgtga cccctggcga gcccgccagc 60
atcagctgca gaagcagcca gagcatcgtc cagagcaacg gcgacaccta cctggaatgg 120
tatctgcaga agcccggcca gtcccccag ctgctgatct acagagtgag caaccggttc 180
agcggcgtgc ccgacagatt cagcggcagc ggctccggca ccgacttcac cctgaagatc 240
agccgggtgg aggccgagga cgtgggcgtg tactactgct ttcaaggcag ccacgtgccc 300
tacaccttcg gccagggcac caagctggaa atcaagcgta cggtggccgc cccagcgtg 360
ttcatcttcc cccccagcga tgagcagctg aagagcggca ccgccagcgt ggtgtgtctg 420
ctgaacaact tctacccccg ggaggccaag gtgcagtgga aggtggacaa tgccctgcag 480
agcggcaaca gccaggagag cgtgaccgag caggacagca aggactccac ctacagcctg 540
agcagcaccc tgaccctgag caaggccgac tacgagaagc acaaggtgta cgcctgtgag 600
gtgacccacc agggcctgtc cagccccgtg accaagagct tcaaccgggg cgagtgc 657
```

<210> 232
<211> 1821
<212> DNA
<213> Artificial Sequence

<220>
<223> Humanised

<400> 232

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
```

```
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaagg gatccgacgg cggcggcatt 1380
aggaggagca tgagcggcac ctggtacctg aaggccatga ccgtggatag ggagttcccc 1440
gagatgaacc tggagagcgt gacccccatg acactgaccc tgctcaaggg ccacaacctg 1500
gaggccaagg tcaccatgct gatctcaggc aggtgccagg aggtgaaggc agtgctgggc 1560
aggaccaagg agaggaagaa gtacaccgcc gacgggggca agcacgtggc ctatatcatc 1620
cccagcgccg tgagggacca cgtgatcttc tacagcgagg gccagctcca cggaaagccc 1680
gtgagaggcg tgaagctggt gggcagggac cccaagaaca acctggaggc cctggaggac 1740
ttcgaaaaag ccgcaggcgc caggggcctg tccactgaga gcatcctgat ccctaggcag 1800
agcgagacct gcagccccgg c                                          1821
```

<210> 233
<211> 1623
<212> DNA
<213> Artificial Sequence

<220>
<223> Humanised

<400> 233

caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaagg gatccgaggt ggtggccgcc 1380
acccccacca gcctgctgat ttcctggagg caccccccact tccccacacg ctactacagg 1440
atcacctacg gcgagaccgg cggcaacagc cccgtgcagg agttcaccgt gcccctgcag 1500
cctcccactg ccaccatcag cggcctcaag cccggcgtgg actacaccat caccgtgtac 1560
gccgtcaccg acggaaggaa cggcaggctg ctgagcatcc ccatcagcat caactacagg 1620
acc 1623

<210> 234
<211> 1839
<212> DNA
<213> Artificial Sequence

<220>
<223> Humanised

<400> 234

caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180

aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaaga ccgtggccgc cccctcggga 1380
tccgacggcg gcggcattag gaggagcatg agcggcacct ggtacctgaa ggccatgacc 1440
gtggataggg agttccccga gatgaacctg gagagcgtga cccccatgac actgaccctg 1500
ctcaagggcc acaacctgga ggccaaggtc accatgctga tctcaggcag gtgccaggag 1560
gtgaaggcag tgctgggcag gaccaaggag aggaagaagt acaccgccga cgggggcaag 1620
cacgtggcct atatcatccc cagcgccgtg agggaccacg tgatcttcta cagcgagggc 1680
cagctccacg gaaagcccgt gagaggcgtg aagctggtgg gcagggaccc caagaacaac 1740
ctggaggccc tggaggactt cgaaaaagcc gcaggcgcca ggggcctgtc cactgagagc 1800
atcctgatcc ctaggcagag cgagacctgc agccccggc 1839

<210> 235
<211> 1539
<212> DNA
<213> Artificial Sequence

<220>
<223> Humanised

<400> 235

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaagg gatccgtgga caacaagttc 1380
aacaaggagc tgaggcaggc ctactgggag atccaggccc tgcccaatct gaactggacc 1440
cagagcaggg ccttcatcag gagcctgtac gacgacccca gccagagcgc taacctcctg 1500
gccgaggcca aaaagctgaa cgacgcccag gcccccaag                        1539
```

<210> 236
<211> 1557
<212> DNA
<213> Artificial Sequence

<220>
<223> Humanised

<400> 236

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
```

```
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaaga ccgtggccgc cccctcggga 1380
tccgtggaca acaagttcaa caaggagctg aggcaggcct actgggagat ccaggccctg 1440
cccaatctga actggaccca gagcagggcc ttcatcagga gcctgtacga cgaccccagc 1500
cagagcgcta acctcctggc cgaggccaaa aagctgaacg acgcccaggc ccccaag    1557
```

<210> 237
<211> 1734
<212> DNA
<213> Artificial Sequence

<220>
<223> Humanised

<400> 237

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcagggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
cccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaagg gatccgacct ggggaaaaag 1380
ctgcttgaag ccgctagggc aggacaggat gacgaggtga ggattctgat ggcaaatggc 1440
gccgacgtca atgccaaaga cgagtacggc ctcacccctc tttatctggc cactgcacac 1500
ggacacttgg agatcgtgga ggtgctgctc aagaacggag ctgatgtgaa cgctgtggac 1560
gctattgggt tcacacccct tcacctcgca gcctttattg gccacctgga gatcgccgaa 1620
gttctcctga aacacggcgc agacgtcaac gcacaggata agttcgggaa gaccgccttc 1680
gacatcagca tcggcaatgg gaacgaggat ctggccgaga tcctgcagaa gctg 1734
```

<210> 238

<211> 1752

<212> DNA

<213> Artificial Sequence

<220>

<223> Humanised

<400> 238

```
caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
```

```
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gcccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaaga ccgtggccgc cccctcggga 1380
tccgacctgg ggaaaaagct gcttgaagcc gctagggcag gacaggatga cgaggtgagg 1440
attctgatgg caaatggcgc cgacgtcaat gccaaagacg agtacggcct cacccctctt 1500
tatctggcca ctgcacacgg acacttggag atcgtggagg tgctgctcaa gaacggagct 1560
gatgtgaacg ctgtggacgc tattgggttc acacccctc acctcgcagc ctttattggc 1620
cacctggaga tcgccgaagt tctcctgaaa cacggcgcag acgtcaacgc acaggataag 1680
ttcgggaaga ccgccttcga catcagcatc ggcaatggga acgaggatct ggccgagatc 1740
ctgcagaagc tg                                                     1752
```

<210> 239
<211> 1722
<212> DNA
<213> Artificial Sequence

<220>
<223> Humanised

<400> 239

caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggatccc aggtgcagct ggtggagtct 1380
gggggaggct tggtgcaggc tgggggggtct ctgagactct cctgtgcagc ctctggatac 1440
gcatacactt acatctacat gggctggttc cgccaggctc cagggaaaga gcgtgagggg 1500
gtcgcagcta tggatagtgg tggtggtggc acactctacg ccgactccgt gaagggccga 1560
ttcaccatct cccgcgacaa aggcaagaac acggtgtatc tgcaaatgga cagcctgaaa 1620
cctgaggaca cggccacgta ttactgtgct gcaggtggct acgagctgcg tgaccggaca 1680
tatgggcagt ggggccaggg gacccaggtc accgtctcct ca 1722

<210> 240
<211> 1698
<212> DNA
<213> Artificial Sequence

<220>
<223> Humanised

<400> 240

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggatccg cgcgcgtcga ccagacgccg 1380
cgcagcgtca cgaaggaaac cggcgagtcc ctcaccatca actgcgtgct gcgggatgcc 1440
tcctacgccc tgggcagcac atgttggtac agaaagaaga gcggggaagg caacgaggag 1500
tccatctcca aggggggaag atacgtcgag accgtgaaca gcggaagcaa gagcttcagc 1560
ctgcggatca acgacctcac cgtcgaggac gggggcacct accgttgcgg tctgggcgtg 1620
gccggcggct attgcgatta cgccctgtgc agtagccggt atgctgagtg cggcgacggc 1680
accgctgtga ccgtgaac 1698
```

<210> 241
<211> 1641
<212> DNA
<213> Artificial Sequence

<220>
<223> Humanised

<400> 241

caggtgcagc tggtgcagag cggagccgag gtgaagaagc ctggcgccag cgtcaaggtg 60
tcctgcaagg ccagcggcta caccttcacc gactactaca tgaactgggt gcggcaggcc 120
ccaggccagg gactggaatg gatgggcaac atcaacccca acaacggcgg caccaactac 180
aaccagaagt tcaaggaccg ggtcaccatg accaccgaca ccagcaccag caccgcctac 240
atggaactgc ggagcctgag aagcgacgac accgccgtgt actactgcgc ccggtggatc 300
ctgtactacg gccggtccaa gtggtacttc gacgtgtggg gcaggggcac actagtgacc 360
gtgtccagcg ccagcaccaa gggccccagc gtgttccccc tggcccccag cagcaagagc 420
accagcggcg gcacagccgc cctgggctgc ctggtgaagg actacttccc cgaaccggtg 480
accgtgtcct ggaacagcgg agccctgacc agcggcgtgc acaccttccc cgccgtgctg 540
cagagcagcg gcctgtacag cctgagcagc gtggtgaccg tgcccagcag cagcctgggc 600
acccagacct acatctgtaa cgtgaaccac aagcccagca acaccaaggt ggacaagaag 660
gtggagccca agagctgtga caagacccac acctgccccc cctgccctgc ccccgagctg 720
ctgggaggcc ccagcgtgtt cctgttcccc cccaagccta aggacaccct gatgatcagc 780
agaacccccg aggtgacctg tgtggtggtg gatgtgagcc acgaggaccc tgaggtgaag 840
ttcaactggt acgtggacgg cgtggaggtg cacaatgcca agaccaagcc cagggaggag 900
cagtacaaca gcacctaccg ggtggtgtcc gtgctgaccg tgctgcacca ggattggctg 960
aacggcaagg agtacaagtg taaggtgtcc aacaaggccc tgcctgcccc tatcgagaaa 1020
accatcagca aggccaaggg ccagcccaga gagccccagg tgtacaccct gccccctagc 1080
agagatgagc tgaccaagaa ccaggtgtcc ctgacctgcc tggtgaaggg cttctacccc 1140
agcgacatcg ccgtggagtg ggagagcaac ggccagcccg agaacaacta caagaccacc 1200
ccccctgtgc tggacagcga tggcagcttc ttcctgtaca gcaagctgac cgtggacaag 1260
agcagatggc agcagggcaa cgtgttcagc tgctccgtga tgcacgaggc cctgcacaat 1320
cactacaccc agaagagcct gagcctgtcc cctggcaaga ccgtggccgc cccctcggga 1380
tccgaggtgg tggccgccac ccccaccagc ctgctgattt cctggaggca cccccacttc 1440
cccacacgct actacaggat cacctacggc gagaccggcg gcaacagccc cgtgcaggag 1500
ttcaccgtgc ccctgcagcc tcccactgcc accatcagcg gcctcaagcc cggcgtggac 1560
tacaccatca ccgtgtacgc cgtcaccgac ggaaggaacg gcaggctgct gagcatcccc 1620
atcagcatca actacaggac c 1641

<210> 242
<211> 1611
<212> DNA
<213> Artificial Sequence

<220>
<223> Humanised

<400> 242

```
caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
acccccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttccccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtctcc tggcaagaga tccgaggtgg tggccgccac ccccaccagc 1380
ctgctgattt cctggaggca cccccacttc cccacacgct actacaggat cacctacggc 1440
gagaccggcg gcaacagccc cgtgcaggag ttcaccgtgc ccctgcagcc tcccactgcc 1500
accatcagcg gcctcaagcc cggcgtggac tacaccatca ccgtgtacgc cgtcaccgac 1560
ggaaggaacg gcaggctgct gagcatcccc atcagcatca actacaggac c          1611
```

<210> 243
<211> 642
<212> PRT
<213> Artificial Sequence

<220>
<223> Humanised

<400> 243

Gly Ala Cys Ala Thr Cys Cys Thr Gly Cys Thr Gly Ala Cys Cys Cys
1 5 10 15
Ala Gly Ala Gly Cys Cys Cys Cys Gly Thr Gly Ala Thr Cys Cys Thr
20 25 30
Gly Ala Gly Cys Gly Thr Gly Ala Gly Cys Cys Cys Thr Gly Gly Cys
35 40 45
Gly Ala Gly Ala Gly Ala Gly Thr Gly Ala Gly Cys Thr Thr Cys Ala
50 55 60
Gly Cys Thr Gly Cys Cys Gly Gly Gly Cys Cys Ala Gly Cys Cys Ala
65 70 75 80
Gly Ala Gly Cys Ala Thr Cys Gly Gly Cys Ala Cys Cys Ala Ala Cys
85 90 95
Ala Thr Cys Cys Ala Cys Thr Gly Gly Thr Ala Thr Cys Ala Gly Cys
100 105 110
Ala Gly Cys Gly Gly Ala Cys Cys Ala Ala Cys Gly Gly Cys Ala Gly
115 120 125
Cys Cys Cys Cys Ala Gly Gly Cys Thr Gly Cys Thr Gly Ala Thr Cys
130 135 140
Ala Ala Gly Thr Ala Cys Gly Cys Cys Ala Gly Cys Gly Ala Gly Thr
145 150 155 160
Cys Cys Ala Thr Cys Ala Gly Cys Gly Gly Cys Ala Thr Cys Cys Cys
165 170 175
Cys Ala Gly Cys Cys Gly Gly Thr Thr Cys Ala Gly Cys Gly Gly Cys
180 185 190
Ala Gly Cys Gly Gly Cys Thr Cys Cys Gly Gly Cys Ala Cys Cys Gly
195 200 205
Ala Cys Thr Thr Cys Ala Cys Cys Cys Thr Gly Ala Gly Cys Ala Thr
210 215 220
Cys Ala Ala Cys Ala Gly Cys Gly Thr Gly Gly Ala Gly Ala Gly Cys
225 230 235 240
Gly Ala Gly Gly Ala Thr Ala Thr Cys Gly Cys Cys Gly Ala Cys Thr
245 250 255
Ala Cys Thr Ala Cys Thr Gly Cys Cys Ala Gly Cys Ala Gly Ala Ala
260 265 270
Cys Ala Ala Cys Ala Ala Cys Thr Gly Gly Cys Cys Cys Ala Cys Cys
275 280 285
Ala Cys Cys Thr Thr Cys Gly Gly Ala Gly Cys Cys Gly Gly Cys Ala
290 295 300

Cys Cys Ala Ala Gly Cys Thr Gly Gly Ala Ala Cys Thr Gly Ala Ala
305 310 315 320
Gly Cys Gly Thr Ala Cys Gly Gly Thr Gly Gly Cys Cys Gly Cys Cys
325 330 335
Cys Cys Cys Ala Gly Cys Gly Thr Gly Thr Thr Cys Ala Thr Cys Thr
340 345 350
Thr Cys Cys Cys Cys Cys Cys Cys Ala Gly Cys Gly Ala Thr Gly Ala
355 360 365
Gly Cys Ala Gly Cys Thr Cys Ala Ala Gly Ala Gly Cys Gly Gly Cys
370 375 380
Ala Cys Cys Gly Cys Cys Ala Gly Cys Gly Thr Gly Gly Thr Gly Thr
385 390 395 400
Gly Thr Cys Thr Gly Cys Thr Gly Ala Ala Cys Ala Ala Cys Thr Thr
405 410 415
Cys Thr Ala Cys Cys Cys Cys Cys Gly Gly Gly Ala Gly Gly Cys Cys
420 425 430
Ala Ala Ala Gly Thr Gly Cys Ala Gly Thr Gly Gly Ala Ala Ala Gly
435 440 445
Thr Gly Gly Ala Cys Ala Ala Cys Gly Cys Cys Cys Thr Gly Cys Ala
450 455 460
Gly Ala Gly Cys Gly Gly Cys Ala Ala Cys Ala Gly Cys Cys Ala Gly
465 470 475 480
Gly Ala Gly Ala Gly Cys Gly Thr Gly Ala Cys Cys Gly Ala Gly Cys
485 490 495
Ala Gly Gly Ala Cys Ala Gly Cys Ala Ala Gly Gly Ala Cys Thr Cys
500 505 510
Cys Ala Cys Cys Thr Ala Cys Ala Gly Cys Cys Thr Gly Ala Gly Cys
515 520 525
Ala Gly Cys Ala Cys Cys Cys Thr Gly Ala Cys Cys Cys Thr Gly Ala
530 535 540
Gly Cys Ala Ala Gly Gly Cys Cys Gly Ala Cys Thr Ala Cys Gly Ala
545 550 555 560
Gly Ala Ala Gly Cys Ala Cys Ala Ala Ala Gly Thr Gly Thr Ala Cys
565 570 575
Gly Cys Cys Thr Gly Cys Gly Ala Ala Gly Thr Gly Ala Cys Cys Cys
580 585 590
Ala Cys Cys Ala Gly Gly Gly Cys Cys Thr Gly Thr Cys Cys Ala Gly
595 600 605
Cys Cys Cys Cys Gly Thr Gly Ala Cys Cys Ala Ala Gly Ala Gly Cys
610 615 620
Thr Thr Cys Ala Ala Cys Cys Gly Gly Gly Gly Cys Gly Ala Gly Thr
625 630 635 640
Gly Cys

<210> 244
<211> 906
<212> DNA
<213> Artificial Sequence

<220>
<223> Humanised

<400> 244

gacatcctgc tgacccagag ccccgtgatc ctgagcgtga gccctggcga gagagtgagc 60
ttcagctgcc gggccagcca gagcatcggc accaacatcc actggtatca gcagcggacc 120
aacggcagcc ccaggctgct gatcaagtac gccagcgagt ccatcagcgg catccccagc 180
cggttcagcg gcagcggctc cggcaccgac ttcaccctga gcatcaacag cgtggagagc 240
gaggatatcg ccgactacta ctgccagcag aacaacaact ggcccaccac cttcggagcc 300
ggcaccaagc tggaactgaa gcgtacggtg gccgccccca gcgtgttcat cttccccccc 360
agcgatgagc agctcaagag cggcaccgcc agcgtggtgt gtctgctgaa caacttctac 420
ccccgggagg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cgaggcgagt gcagatccga ggtggtggcc 660
gccaccccca ccagcctgct gatttcctgg aggcaccccc acttccccac acgctactac 720
aggatcacct acggcgagac cggcggcaac agccccgtgc aggagttcac cgtgcccctg 780
cagcctccca ctgccaccat cagcggcctc aagcccggcg tggactacac catcaccgtg 840
tacgccgtca ccgacggaag gaacggcagg ctgctgagca tccccatcag catcaactac 900
aggacc 906

<210> 245
<211> 1347
<212> DNA
<213> Artificial Sequence

<220>
<223> Humanised

<400> 245

caggtgcagc tgaagcagag cggccctggc ctggtgcagc cctctcagag cctgagcatc 60
acctgtaccg tgagcggctt cagcctgacc aattacggcg tgcattgggt gcggcagtct 120
ccaggcaagg gcctggaatg gctgggagtg atctggtccg gcggcaacac cgactacaac 180
acccccttca ccagcagact gagcatcaac aaggacaaca gcaagagcca ggtgttcttc 240
aagatgaaca gcctgcagag caacgacacc gccatctact attgtgccag ggccctgacc 300
tactacgact acgagttcgc ctactggggc cagggcaccc tggtgaccgt gagcgccgct 360
agcaccaagg gccccagcgt gttccccctg gcccccagca gcaagagcac cagcggcggc 420
acagccgccc tgggctgcct ggtgaaggac tacttccccg agcctgtgac cgtgtcctgg 480
aatagcggag ccctgacctc cggcgtgcac accttccccg ccgtgctgca gagcagcggc 540
ctgtactccc tgagcagcgt ggtgaccgtg cccagcagca gcctgggcac ccagacctac 600
atctgcaacg tgaaccacaa gcccagcaac accaaagtgg acaagaaagt ggagcccaag 660
agctgcgata agacccacac ctgccccccc tgccctgccc ccgagctgct gggcggacct 720
agcgtgttcc tgttcccccc caagcctaag gacaccctga tgatcagcag gacccccgaa 780
gtgacctgcg tggtggtgga tgtgagccac gaggaccctg aagtgaagtt caactggtac 840
gtggacggcg tggaagtgca caacgccaag accaagccca gagaggagca gtacaacagc 900
acctaccgcg tggtgtctgt gctgaccgtg ctgcaccagg attggctgaa cggcaaggag 960
tacaagtgca aagtgagcaa caaggccctg cctgccccta tcgagaaaac catcagcaag 1020
gccaagggcc agcctagaga gccccaggtc tacaccctgc ctccctccag agatgagctg 1080
accaagaacc aggtgtccct gacctgtctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtactcc aagctgaccg tggacaagag cagatggcag 1260
cagggcaacg tgttcagctg cagcgtgatg cacgaggccc tgcacaatca ctacacccag 1320
aagagtctga gcctgtcccc tggcaag 1347

<210> 246
<211> 1617
<212> DNA
<213> Artificial Sequence

<220>
<223> Humanised

<400> 246

```
gaggtggtgg ccgccacccc caccagcctg ctgatttcct ggaggcaccc ccacttcccc 60
acacgctact acaggatcac ctacggcgag accggcggca acagccccgt gcaggagttc 120
accgtgcccc tgcagcctcc cactgccacc atcagcggcc tcaagcccgg cgtggactac 180
accatcaccg tgtacgccgt caccgacgga aggaacggca ggctgctgag catccccatc 240
agcatcaact acaggaccgg atccaccggc caggtgcagc tgaagcagag cggccctggc 300
ctggtgcagc cctctcagag cctgagcatc acctgtaccg tgagcggctt cagcctgacc 360
aattacggcg tgcattgggt gcggcagtct ccaggcaagg gcctggaatg gctgggagtg 420
atctggtccg gcggcaacac cgactacaac acccccttca ccagcagact gagcatcaac 480
aaggacaaca gcaagagcca ggtgttcttc aagatgaaca gcctgcagag caacgacacc 540
gccatctact attgtgccag ggccctgacc tactacgact acgagttcgc ctactggggc 600
cagggcaccc tggtgaccgt gagcgccgct agcaccaagg gccccagcgt gttccccctg 660
gcccccagca gcaagagcac cagcggcggc acagccgccc tgggctgcct ggtgaaggac 720
tacttccccg agcctgtgac cgtgtcctgg aatagcggag ccctgacctc cggcgtgcac 780
accttccccg ccgtgctgca gagcagcggc ctgtactccc tgagcagcgt ggtgaccgtg 840
cccagcagca gcctgggcac ccagacctac atctgcaacg tgaaccacaa gcccagcaac 900
accaaagtgg acaagaaagt ggagcccaag agctgcgata agacccacac ctgccccccc 960
tgccctgccc ccgagctgct gggcggacct agcgtgttcc tgttcccccc caagcctaag 1020
gacaccctga tgatcagcag gacccccgaa gtgacctgcg tggtggtgga tgtgagccac 1080
gaggaccctg aagtgaagtt caactggtac gtggacggcg tggaagtgca caacgccaag 1140
accaagccca gagaggagca gtacaacagc acctaccgcg tggtgtctgt gctgaccgtg 1200
ctgcaccagg attggctgaa cggcaaggag tacaagtgca aagtgagcaa caaggccctg 1260
cctgccccta tcgagaaaac catcagcaag gccaagggcc agcctagaga gccccaggtc 1320
tacaccctgc ctccctccag agatgagctg accaagaacc aggtgtccct gacctgtctg 1380
gtgaagggct tctaccccag cgacatcgcc gtggagtggg agagcaacgg ccagcccgag 1440
aacaactaca agaccacccc ccctgtgctg gacagcgatg gcagcttctt cctgtactcc 1500
aagctgaccg tggacaagag cagatggcag cagggcaacg tgttcagctg cagcgtgatg 1560
cacgaggccc tgcacaatca ctacacccag aagagtctga gcctgtcccc tggcaag    1617
```

<210> 247
<211> 909
<212> DNA
<213> Artificial Sequence

<220>
<223> Humanised

<400> 247

```
gaggtggtgg ccgccacccc caccagcctg ctgatttcct ggaggcaccc ccacttcccc 60

acacgctact acaggatcac ctacggcgag accggcggca acagccccgt gcaggagttc 120
accgtgcccc tgcagcctcc cactgccacc atcagcggcc tcaagcccgg cgtggactac 180
accatcaccg tgtacgccgt caccgacgga aggaacggca ggctgctgat catccccatc 240
agcatcaact acaggacgtc gaccggtgac atcctgctga cccagagccc cgtgatcctg 300
agcgtgagcc ctggcgagag agtgagcttc agctgccggg ccagccagag catcggcacc 360
aacatccact ggtatcagca gcggaccaac ggcagcccca ggctgctgat caagtacgcc 420
agcgagtcca tcagcggcat ccccagccgg ttcagcggca gcggctccgg caccgacttc 480
accctgagca tcaacagcgt ggagagcgag gatatcgccg actactactg ccagcagaac 540
aacaactggc ccaccacctt cggagccggc accaagctgg aactgaagcg tacggtggcc 600
gcccccagcg tgttcatctt cccccccagc gatgagcagc tcaagagcgg caccgccagc 660
gtggtgtgtc tgctgaacaa cttctacccc cgggaggcca aagtgcagtg gaaagtggac 720
aacgccctgc agagcggcaa cagccaggag agcgtgaccg agcaggacag caaggactcc 780
acctacagcc tgagcagcac cctgaccctg agcaaggccg actacgagaa gcacaaagtg 840
tacgcctgcg aagtgaccca ccagggcctg tccagccccg tgaccaagag cttcaaccgg 900
ggcgagtgc                                                         909
```

<210> 248
<211> 1707
<212> DNA
<213> Artificial Sequence

<220>
<223> Humanised

<400> 248

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt ccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggcgtgc agctcctgga gagcggcgga 1380
ggcctggtcc agcccggcgg cagcctgagg ctgagctgcg ccgccagcgg cttcgtgttc 1440
ccctggtatg atatgggctg ggtgaggcag gcccccggca agggcctgga gtgggtgtcc 1500
agcatcgact ggcacgggaa gatcacctac tacgccgaca gcgtgaaggg caggttcacc 1560
atcagcaggg acaacagcaa gaacaccctg tacctgcaga tgaacagcct gagggccgag 1620
gacaccgcag tgtactactg cgccaccgcc gaggacgaac ccggctacga ctactggggc 1680
cagggcaccc tggtgactgt gagcagc 1707
```

<210> 249
<211> 1725
<212> DNA
<213> Artificial Sequence

<220>
<223> Humanised

<400> 249

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
```

ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgcccctc gggcgtgcag 1380
ctcctggaga gcggcggagg cctggtccag cccggcggca gcctgaggct gagctgcgcc 1440
gccagcggct tcgtgttccc ctggtatgat atgggctggg tgaggcaggc ccccggcaag 1500
ggcctggagt gggtgtccag catcgactgg cacgggaaga tcacctacta cgccgacagc 1560
gtgaagggca ggttcaccat cagcagggac aacagcaaga acaccctgta cctgcagatg 1620
aacagcctga gggccgagga caccgcagtg tactactgcg ccaccgccga ggacgaaccc 1680
ggctacgact actggggcca gggcaccctg gtgactgtga gcagc 1725

<210> 250
<211> 1713
<212> DNA
<213> Artificial Sequence

<220>
<223> Humanised

<400> 250

caggtgcagc tcgtccagtc tggggccgag gtgaagaagc ccggagcttc tgtgaaggtg 60
tcctgcaagg ccagcggcta taccttcatc gactacgaga tccattgggt gaggcaggct 120
cccgggcagg gcctggagtg gatgggcgcc atcgacccag agaccggagg cacggcgtac 180
aaccagaagt tcaagggacg ggtcaccatg acaaccgata ccagcacctc caccgcttac 240
atggagctgc gcagcctgag aagcgacgac accgcggtgt actactgtac gcgcatcctg 300
ctctactact accccatgga ttactggggc cagggcacac tagtgaccgt gtctagcgcc 360
agcaccaagg gccccagcgt gttccccctg gcccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gacctgcctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtacagc aagctgaccg tggacaagag cagatggcag 1260
cagggcaacg tgttcagctg ctccgtgatg cacgaggccc tgcacaatca ctacacccag 1320
aagagcctga gcctgtcccc tggcaagacc gtggccgccc cctcggaagt gcagctcctg 1380
gagagcggcg gcggcctggt gcagcccggc ggcagcctga ggctgagctg cgccgctagc 1440
ggcttcacct tcaggaactt cggcatgggc tgggtcaggc aggcccccgg caagggcctg 1500
gagtgggtca gctggatcat cagctccggc accgagacct actacgccga cagcgtgaag 1560
ggcaggttca ccatcagccg cgacaacagc aagaacaccc tgtacctgca gatgaacagc 1620
ctgagggccg aggacaccgc cgtctactac tgcgccaaga gcctgggcag gttcgactac 1680
tggggacagg ggaccctggt gactgtgagc agc 1713

<210> 251
<211> 642
<212> DNA
<213> Artificial Sequence

<220>
<223> Humanised

<400> 251

```
gagatcgtgc tgacccagag tccagccacc ctcagcctga gccctgggga acgcgccacc 60
ctgtcctgcc gggcgagtca gaacatctcc gactacctgc attggtacca gcagaagccc 120
ggccaggccc ctcgcctgct gatctactac gcctcccaga gcatcagcgg aatccccgcc 180
cggttctccg gaagtgggtc cggaaccgac tttaccctga ccatcagctc tctcgagcca 240
gaggacttcg cggtgtacta ctgccagaac gggcatagtt tcccactgac cttcggaggg 300
ggcacaaagg tggagatcaa gcgtacggtg gccgccccca gcgtgttcat cttccccccc 360
agcgatgagc agctgaagag cggcaccgcc agcgtggtgt gtctgctgaa caacttctac 420
ccccgggagg ccaaggtgca gtggaaggtg gacaatgccc tgcagagcgg caacagccag 480
gagagcgtga ccgagcagga cagcaaggac tccacctaca gcctgagcag caccctgacc 540
ctgagcaagg ccgactacga gaagcacaag gtgtacgcct gtgaggtgac ccaccagggc 600
ctgtccagcc ccgtgaccaa gagcttcaac cggggcgagt gc 642
```

<210> 252
<211> 1725
<212> DNA
<213> Artificial Sequence

<220>
<223> Humanised

<400> 252

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgccccctc gggcgtgcag 1380
ctcctggaga gcggcggagg cctggtccag cccggcggca gcctgaggct gagctgcgcc 1440
gccagcggct tcgtgttccc ctggtatgat atgggctggg tgaggcaggc ccccggcaag 1500
ggcctggagt gggtgtccag catcgactgg aagggggggca agacctacta cgccgacagc 1560
gtgaagggca ggttcaccat cagcagggac aacagcaaga acaccctgta cctgcagatg 1620
aacagcctga gggccgagga caccgcagtg tactactgcg ccaccgccga ggacgaaccc 1680
ggctacgact actggggcca gggcaccctg gtgactgtga gcagc 1725
```

<210> 253
<211> 1707

<212> DNA
<213> Artificial Sequence

<220>
<223> Humanised

<400> 253

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggcgtgc agctcctgga gagcggcgga 1380
ggcctggtcc agcccggcgg cagcctgagg ctgagctgcg ccgccagcgg cttcgtgttc 1440
ccctggtatg atatgggctg ggtgaggcag gcccccggca agggcctgga gtgggtgtcc 1500
agcatcgact ggaagggggg caagacctac tacgccgaca gcgtgaaggg caggttcacc 1560

atcagcaggg acaacagcaa gaacaccctg tacctgcaga tgaacagcct gagggccgag 1620
gacaccgcag tgtactactg cgccaccgcc gaggacgaac ccggctacga ctactggggc 1680
cagggcaccc tggtgactgt gagcagc                                     1707
```

<210> 254
<211> 1725
<212> DNA
<213> Artificial Sequence

<220>
<223> Humanised

<400> 254

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgccccctc gggcgtgcag 1380
ctcctggaga gcggcggagg cctggtccag cccggcggca gcctgaggct gagctgcgcc 1440
gccagcggct tcgtgttcgc ctggtatgat atgggctggg tgaggcaggc ccccggcaag 1500
ggcctggagt gggtgtccag catcgactgg cacggggagg tgacctacta cgccgacagc 1560
gtgaagggca ggttcaccat cagcagggac aacagcaaga acaccctgta cctgcagatg 1620
aacagcctga gggccgagga caccgcagtg tactactgcg ccaccgccga ggacgaaccc 1680
ggctacgact actggggcca gggcaccctg gtgactgtga gcagc 1725
```

<210> 255
<211> 1707
<212> DNA
<213> Artificial Sequence

<220>
<223> Humanised

<400> 255

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggcgtgc agctcctgga gagcggcgga 1380
ggcctggtcc agcccggcgg cagcctgagg ctgagctgcg ccgccagcgg cttcgtgttc 1440
```

gcctggtatg atatgggctg ggtgaggcag gcccccggca agggcctgga gtgggtgtcc 1500
agcatcgact ggcacgggga ggtgacctac tacgccgaca gcgtgaaggg caggttcacc 1560
atcagcaggg acaacagcaa gaacaccctg tacctgcaga tgaacagcct gagggccgag 1620
gacaccgcag tgtactactg cgccaccgcc gaggacgaac ccggctacga ctactggggc 1680
cagggcaccc tggtgactgt gagcagc 1707

<210> 256
<211> 1347
<212> DNA
<213> Artificial Sequence

<220>
<223> Humanised

<400> 256

caggtgcagc tcgtccagtc tggggccgag gtgaagaagc ccggagcttc tgtgaaggtg 60
tcctgcaagg ccagcggcta taccttcatc gactacgaga tccattgggt gaggcaggct 120
cccgggcagg gcctggagtg gatgggcgcc atcgacccag agaccggagg cacggcgtac 180
aaccagaagt tcaagggacg ggtcaccatg acaaccgata ccagcacctc caccgcttac 240
atggagctgc gcagcctgag aagcgacgac accgcggtgt actactgtac gcgcatcctg 300
ctctactact accccatgga ttactggggc cagggcacac tagtcacagt ctcctcagcc 360
tccaccaagg gcccatcggt cttccccctg gcaccctcct ccaagagcac ctctgggggc 420
acagcggccc tgggctgcct ggtcaaggac tacttccccg aaccggtgac ggtgtcgtgg 480
aactcaggcg ccctgaccag cggcgtgcac accttcccgg ctgtcctaca gtcctcagga 540
ctctactccc tcagcagcgt ggtgaccgtg ccctccagca gcttgggcac ccagacctac 600
atctgcaacg tgaatcacaa gcccagcaac accaaggtgg acaagaaagt tgagcccaaa 660
tcttgtgaca aaactcacac atgcccaccg tgcccagcac ctgaactcct ggggggaccg 720
tcagtcttcc tcttcccccc aaaacccaag gacaccctca tgatctcccg gacccctgag 780
gtcacatgcg tggtggtgga cgtgagccac gaagaccctg aggtcaagtt caactggtac 840
gtggacggcg tggaggtgca taatgccaag acaaagccgc gggaggagca gtacaacagc 900
acgtaccgtg tggtcagcgt cctcaccgtc ctgcaccagg actggctgaa tggcaaggag 960
tacaagtgca aggtctccaa caaagccctc ccagccccca tcgagaaaac catctccaaa 1020
gccaaagggc agccccgaga accacaggtg tacaccctgc ccccatcccg ggatgagctg 1080
accaagaacc aggtcagcct gacctgcctg gtcaaaggct tctatcccag cgacatcgcc 1140
gtggagtggg agagcaatgg gcagccggag aacaactaca agaccacgcc tcccgtgctg 1200
gactccgacg gctccttctt cctctacagc aagctcaccg tggacaagag caggtggcag 1260
caggggaacg tcttctcatg ctccgtgatg catgaggctc tgcacaacca ctacacgcag 1320
aagagcctct ccctgtctcc gggtaaa 1347

<210> 257
<211> 2088
<212> DNA
<213> Artificial Sequence

<220>
<223> Humanised

<400> 257

```
caggtgaccc tgagggagag cggccccgcc ctggtgaagc ccacacagac cctcactctg 60
acctgcaccg tgagcggctt cagcctgacc tcctacagcg tccactgggt gaggcagccc 120
cccggcaagg gcctggagtg gctgggcgtg atctgggcaa gcggcggcac cgactacaac 180
agcgccctga tgagcaggct ctccatcagc aaggacacca gccggaacca ggtggtgctg 240
accatgacca acatggaccc cgtggacacc gccacctatt actgcgccag ggaccctccc 300
tctagcctgc tgaggctgga ctactggggc aggggaacac tagtgaccgt gtccagcgcc 360
agcaccaagg gccccagcgt gttccccctg gcccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gacctgcctg gtgaagggct tctaccccag cgacatcgcc 1140
gtggagtggg agagcaacgg ccagcccgag aacaactaca agaccacccc ccctgtgctg 1200
gacagcgatg gcagcttctt cctgtacagc aagctgaccg tggacaagag cagatggcag 1260
cagggcaacg tgttcagctg ctccgtgatg cacgaggccc tgcacaatca ctacacccag 1320
aagagcctga gcctgtcccc tggcaagggc ggcggcggat ctggcgtgca gctcctggag 1380
agcggcggag gcctggtcca gcccggcggc agcctgaggc tgagctgcgc cgccagcggc 1440
ttcaccttcg cctggtatga tatgggctgg gtgaggcagg cccccggcaa gggcctggag 1500
tgggtgtcca gcatcgactg gcacggggag gtgacctact acgccgacag cgtgaagggc 1560
aggttcacca tcagcaggga caacagcaag aacaccctgt acctgcagat gaacagcctg 1620
agggccgagg acaccgcagt gtactactgc gccaccgccg aggacgaacc cggctacgac 1680

tactggggcc agggcaccct ggtgactgtg agcagcaccg tggccgcccc ctcgggatcc 1740
gaagtgcagc tcctggagag cggcggcggc ctggtgcagc ccggcggcag cctgaggctg 1800
agctgcgccg ctagcggctt caccttcagg aacttcggca tgggctgggt caggcaggcc 1860
cccggcaagg gcctggagtg ggtcagctgg atcatcagct ccggcaccga gacctactac 1920
gccgacagcg tgaagggcag gttcaccatc agccgcgaca acagcaagaa caccctgtac 1980
ctgcagatga acagcctgag ggccgaggac accgccgtct actactgcgc caagagcctg 2040
ggcaggttcg actactgggg acaggggacc ctggtgactg tgagcagc             2088
```

<210> 258

<211> 2079

<212> DNA

<213> Artificial Sequence

<220>

<223> Humanised

<400> 258

```
caggtgcagc tgcagcagcc tggagccgag ctggtgaagc ccggcgccag cgtgaaaatg 60
tcctgcaagg ccagcggcta caccttcacc agctacaaca tgcactgggt gaagcagacc 120
cccggcaggg gcctcgagtg gatcggagct atctaccccg gcaacggcga cactagctac 180
aaccagaagt tcaagggcaa ggccaccctg accgccgaca agagcagcag caccgcctac 240
atgcagctga gcagcctgac cagcgaggac agcgccgtgt attactgcgc caggagcacc 300
tactacggcg gcgactggta cttcaacgtc tggggcgccg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgcccccctc gggatctgac 1380
atccagatga cccagagccc cagcagcctg agcgccagcg tgggcgacag ggtgaccatt 1440
acctgcaggg ccagcaggcc catcagcgac tggctgcact ggtaccaaca gaagcccggc 1500
aaggctccca agctgctgat cgcctgggcc agcagcctgc agggaggcgt gcccagcagg 1560
tttagcggca gcggcagcgg caccgacttc accctcacca tctcttccct gcagcccgag 1620
gacttcgcca cctactactg cctgcaggag ggctgggggc cccctacttt cggccagggc 1680
accaaggtgg agatcaagag gaccgtggcc gccccctcgg gatccggcgt gcagctcctg 1740
gagagcggcg gaggcctggt ccagcccggc ggcagcctga ggctgagctg cgccgccagc 1800
ggcttcacct tcgcctggta tgatatgggc tgggtgaggc aggcccccgg caagggcctg 1860
gagtgggtgt ccagcatcga ctggcacggg gaggtgacct actacgccga cagcgtgaag 1920
ggcaggttca ccatcagcag ggacaacagc aagaacaccc tgtacctgca gatgaacagc 1980
ctgagggccg aggacaccgc agtgtactac tgcgccaccg ccgaggacga acccggctac 2040
gactactggg gccagggcac cctggtgact gtgagcagc                       2079
```

<210> 259
<211> 2103
<212> DNA
<213> Artificial Sequence

<220>
<223> Humanised

<400> 259

```
caggtgcagc tgcagcagcc tggagccgag ctggtgaagc ccggcgccag cgtgaaaatg 60
tcctgcaagg ccagcggcta caccttcacc agctacaaca tgcactgggt gaagcagacc 120
cccggcaggg gcctcgagtg gatcggagct atctaccccg gcaacggcga cactagctac 180
aaccagaagt tcaagggcaa ggccaccctg accgccgaca agagcagcag caccgcctac 240
atgcagctga gcagcctgac cagcgaggac agcgccgtgt attactgcgc caggagcacc 300
tactacggcg gcgactggta cttcaacgtc tggggcgccg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccctgcc ctgcccccga gctgctggga 720
ggccccagcg tgttcctgtt cccccccaag cctaaggaca ccctgatgat cagcagaacc 780
cccgaggtga cctgtgtggt ggtggatgtg agccacgagg accctgaggt gaagttcaac 840
```

```
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagaccgtgg ccgcccccctc gggatctgaa 1380
gtgcagctcc tggagagcgg cggcggcctg gtgcagcccg gcggcagcct gaggctgagc 1440
tgcgccgcta gcggcttcac cttcaggaac ttcggcatgg gctgggtcag gcaggccccc 1500
ggcaagggcc tggagtgggt cagctggatc atcagctccg gcaccgagac ctactacgcc 1560
gacagcgtga agggcaggtt caccatcagc cgcgacaaca gcaagaacac cctgtacctg 1620
cagatgaaca gcctgagggc cgaggacacc gccgtctact actgcgccaa gagcctgggc 1680
aggttcgact actggggaca ggggaccctg gtgactgtga gcagcaccgt ggccgccccc 1740
tcgggatccg gcgtgcagct cctggagagc ggcggaggcc tggtccagcc cggcggcagc 1800
ctgaggctga gctgcgccgc cagcggcttc accttcgcct ggtatgatat gggctgggtg 1860
aggcaggccc ccggcaaggg cctggagtgg gtgtccagca tcgactggca cggggaggtg 1920
acctactacg ccgacagcgt gaagggcagg ttcaccatca gcagggacaa cagcaagaac 1980
accctgtacc tgcagatgaa cagcctgagg gccgaggaca ccgcagtgta ctactgcgcc 2040
accgccgagg acgaacccgg ctacgactac tggggccagg gcaccctggt gactgtgagc 2100
agc                                                               2103
```

<210> 260
<211> 1701
<212> DNA
<213> Artificial Sequence

<220>
<223> Humanised

<400> 260

```
gaggtgcagc tggtggaaag cggcggcggc ctggtgcagc ccggcggctc cctgaggctg 60
agctgcgccg ctagcggcta caccttcacc agctactggc tccactgggt caggcaggcc 120
ccaggcaagg gactggagtg ggtgggcatg atcgacccca gcaacagcga caccaggttc 180
aaccccaact tcaaggacag gttcaccatc agcgccgaca ctagcaagaa caccgcctac 240
ctgcagatga acagcctgag ggccgaggac accgccgtgt attactgcgc cacctacagg 300
agctacgtca cccccctgga ttactggggc cagggcacac tagtgaccgt gtccagcgcc 360
agcaccaagg gccccagcgt gttcccccctg gcccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gagctgcgcc gtgaagggct tctaccccag cgacatcgcc 1140
gtggagtggg agagcaacgg ccagcccgag aacaactaca agaccacccc ccctgtgctg 1200
gacagcgatg gcagcttctt cctggtgagc aagctgaccg tggacaagag cagatggcag 1260
cagggcaacg tgttcagctg ctccgtgatg cacgaggccc tgcacaatca ctacacccag 1320
aagagcctga gcctgtcccc tggcaaggga tccgaggtgc agctcctggt cagcggcggc 1380
ggcctggtcc agcccggagg ctcactgagg ctgagctgcg ccgctagcgg cttcaccttc 1440
aaggcctacc ccatgatgtg ggtcaggcag gcccccggca aaggcctgga gtgggtgtct 1500
gagatcagcc ccagcggcag ctacacctac tacgccgaca gcgtgaaggg caggttcacc 1560
atcagcaggg acaacagcaa gaacaccctg tacctgcaga tgaactctct gagggccgag 1620
gacaccgccg tgtactactg cgccaaggac cccaggaagc tggactattg gggccagggc 1680
actctggtga ccgtgagcag c                                           1701
```

<210> 261
<211> 1020
<212> DNA
<213> Artificial Sequence

<220>
<223> Humanised

<400> 261

```
tgcccccccct gccctgcccc cgagctgctg ggaggcccca gcgtgttcct gttccccccc 60
aagcctaagg acaccctgat gatcagcaga acccccgagg tgacctgtgt ggtggtggat 120
gtgagccacg aggaccctga ggtgaagttc aactggtacg tggacggcgt ggaggtgcac 180
aatgccaaga ccaagcccag ggaggagcag tacaacagca cctaccgggt ggtgtccgtg 240
ctgaccgtgc tgcaccagga ttggctgaac ggcaaggagt acaagtgtaa ggtgtccaac 300
aaggccctgc ctgcccctat cgagaaaacc atcagcaagg ccaagggcca gcccagagag 360
ccccaggtgt acaccctgcc ccctagcaga gatgagctga ccaagaacca ggtgtccctg 420
tggtgcctgg tgaagggctt ctaccccagc gacatcgccg tggagtggga gagcaacggc 480
cagcccgaga acaactacaa gaccaccccc cctgtgctgg acagcgatgg cagcttcttc 540
ctgtacagca agctgaccgt ggacaagagc agatggcagc agggcaacgt gttcagctgc 600
tccgtgatgc acgaggccct gcacaatcac tacacccaga agagcctgag cctgtcccct 660
ggcaagggat ccgaggtgca gctcctggtc agcggcggcg gcctggtcca gcccggaggc 720
tcactgaggc tgagctgcgc cgctagcggc ttcaccttca aggcctaccc catgatgtgg 780
gtcaggcagg cccccggcaa aggcctggag tgggtgtctg agatcagccc cagcggcagc 840
tacacctact acgccgacag cgtgaagggc aggttcacca tcagcaggga caacagcaag 900
aacaccctgt acctgcagat gaactctctg agggccgagg acaccgccgt gtactactgc 960
gccaaggacc ccaggaagct ggactattgg ggccagggca ctctggtgac cgtgagcagc 1020
```

<210> 262
<211> 660
<212> DNA
<213> Artificial Sequence

<220>
<223> Humanised

<400> 262

```
gacatccaga tgacccagag ccccagcagc ctgagcgcct cagtgggaga cagggtgacc 60
atcacctgca agagcagcca gagcctcctg tacaccagca gccagaagaa ctacctggcc 120
tggtaccagc agaaacccgg caaggccccc aagctgctga tctactgggc tagcaccagg 180
gagtcaggcg tgcccagcag gttcagcggc agcggcagcg gcaccgactt cactctgacc 240
atcagcagcc tgcagcccga ggacttcgcc acctactact gccagcagta ctacgcctat 300
ccctggacct tcggccaggg caccaaggtg gagatcaagc gtacggtggc cgcccccagc 360
gtgttcatct tcccccccag cgatgagcag ctgaagagcg gcaccgccag cgtggtgtgt 420
ctgctgaaca acttctaccc ccgggaggcc aaggtgcagt ggaaggtgga caatgccctg 480
cagagcggca acagccagga gagcgtgacc gagcaggaca gcaaggactc cacctacagc 540
ctgagcagca ccctgaccct gagcaaggcc gactacgaga agcacaaggt gtacgcctgt 600
gaggtgaccc accagggcct gtccagcccc gtgaccaaga gcttcaaccg gggcgagtgc 660
```

<210> 263
<211> 1656
<212> DNA
<213> Artificial Sequence

<220>
<223> Humanised

<400> 263

```
gaggtgcagc tggtggaaag cggcggcggc ctggtgcagc ccggcggctc cctgaggctg 60
agctgcgccg ctagcggcta caccttcacc agctactggc tccactgggt caggcaggcc 120
ccaggcaagg gactggagtg ggtgggcatg atcgacccca gcaacagcga caccaggttc 180
aaccccaact tcaaggacag gttcaccatc agcgccgaca ctagcaagaa caccgcctac 240
ctgcagatga acagcctgag ggccgaggac accgccgtgt attactgcgc cacctacagg 300
agctacgtca ccccccctgga ttactggggc cagggcacac tagtcaccgt gagcagcgcc 360
agcaccaagg gccccagcgt gttccccctg gccccctgca gcagaagcac cagcgagagc 420
acagccgccc tgggctgcct ggtgaaggac tacttccccg agcccgtgac cgtgagctgg 480
aacagcggag ccctgaccag cggcgtgcac accttccccg ccgtgctgca gagcagcggc 540
ctgtacagcc tgagcagcgt ggtgaccgtg cccagcagca gcctgggcac caagacctac 600
acctgcaacg tggaccacaa gcccagcaac accaaggtgg acaagcgggt ggcccccgag 660
ttcctgggcg gaccctccgt gttcctgttc cccccccaagc ccaaggacac cctgatgatc 720
agccggaccc ccgaggtgac ctgcgtggtg gtggacgtga gccaggaaga tcccgaggtc 780
cagttcaatt ggtacgtgga cggcgtggag gtgcacaacg ccaagaccaa gccccgggag 840
gaacagttca acagcaccta ccgggtggtg tccgtgctga ccgtgctgca ccaggactgg 900
ctgaacggca aagaatacaa gtgcaaggtg tccaacaagg gcctgcccag ctccatcgag 960
aaaaccatca gcaaggccaa gggccagcct cgggagcccc aggtgtacac cctgccccca 1020
tcccaggaag agatgaccaa gaaccaggtg tccctgacct gtctggtgaa gggcttctac 1080
cccagcgaca tcgccgtgga gtgggagagc aacggccagc ccgagaacaa ctacaagacc 1140
accccccctg tgctggacag cgacggcagc ttcttcctgt acagcaggct gaccgtggac 1200
aagagccggt ggcaggaagg caacgtcttt agctgcagcg tgatgcacga ggccctgcac 1260
aaccactaca cccagaagag cctgagcctg tccctgggca agggatccga ggtgcagctc 1320
ctggtcagcg gcggcggcct ggtccagccc ggaggctcac tgaggctgag ctgcgccgct 1380
agcggcttca ccttcaaggc ctaccccatg atgtgggtca ggcaggcccc cggcaaaggc 1440
ctggagtggg tgtctgagat cagccccagc ggcagctaca cctactacgc cgacagcgtg 1500
aagggcaggt tcaccatcag cagggacaac agcaagaaca ccctgtacct gcagatgaac 1560
tctctgaggg ccgaggacac cgccgtgtac tactgcgcca aggaccccag gaagctggac 1620
tattggggcc agggcactct ggtgaccgtg agcagc 1656
```

<210> 264

<211> 1347

<212> DNA

<213> Artificial Sequence

<220>

<223> Humanised

<400> 264

```
gaggtgcagc tggtggaaag cggcggcggc ctggtgcagc ccggcggctc cctgaggctg 60
agctgcgccg ctagcggcta caccttcacc agctactggc tccactgggt caggcaggcc 120
ccaggcaagg gactggagtg ggtgggcatg atcgacccca gcaacagcga caccaggttc 180
aaccccaact tcaaggacag gttcaccatc agcgccgaca ctagcaagaa caccgcctac 240
ctgcagatga acagcctgag ggccgaggac accgccgtgt attactgcgc cacctacagg 300
agctacgtca ccccccctgga ttactggggc cagggcacac tagtgaccgt gtccagcgcc 360
agcaccaagg gccccagcgt gttcccccctg gcccccagca gcaagagcac cagcggcggc 420
acagccgccc tgggctgcct ggtgaaggac tacttccccg aaccggtgac cgtgtcctgg 480
aacagcggag ccctgaccag cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagaaggt ggagcccaag 660
agctgtgaca agacccacac ctgccccccc tgccctgccc ccgagctgct gggaggcccc 720
agcgtgttcc tgttcccccc caagcctaag gacaccctga tgatcagcag aacccccgag 780
gtgacctgtg tggtggtgga tgtgagccac gaggaccctg aggtgaagtt caactggtac 840
gtggacggcg tggaggtgca caatgccaag accaagccca gggaggagca gtacaacagc 900
acctaccggg tggtgtccgt gctgaccgtg ctgcaccagg attggctgaa cggcaaggag 960
tacaagtgta aggtgtccaa caaggccctg cctgccccta tcgagaaaac catcagcaag 1020
gccaagggcc agcccagaga gccccaggtg tacaccctgc cccctagcag agatgagctg 1080
accaagaacc aggtgtccct gagctgcgcc gtgaagggct tctaccccag cgacatcgcc 1140
gtggagtggg agagcaacgg ccagcccgag aacaactaca agaccacccc ccctgtgctg 1200
gacagcgatg gcagcttctt cctggtgagc aagctgaccg tggacaagag cagatggcag 1260
cagggcaacg tgttcagctg ctccgtgatg cacgaggccc tgcacaatca ctacacccag 1320
aagagcctga gcctgtcccc tggcaag 1347
```

<210> 265
<211> 666
<212> DNA
<213> Artificial Sequence

<220>
<223> Humanised

<400> 265

```
tgcccccccct gccctgcccc cgagctgctg ggaggcccca gcgtgttcct gttcccccccc 60
aagcctaagg acaccctgat gatcagcaga acccccgagg tgacctgtgt ggtggtggat 120
gtgagccacg aggaccctga ggtgaagttc aactggtacg tggacggcgt ggaggtgcac 180
aatgccaaga ccaagcccag ggaggagcag tacaacagca cctaccgggt ggtgtccgtg 240
ctgaccgtgc tgcaccagga ttggctgaac ggcaaggagt acaagtgtaa ggtgtccaac 300
aaggccctgc ctgcccctat cgagaaaacc atcagcaagg ccaagggcca gcccagagag 360
ccccaggtgt acaccctgcc ccctagcaga gatgagctga ccaagaacca ggtgtccctg 420
tggtgcctgg tgaagggctt ctaccccagc gacatcgccg tggagtggga gagcaacggc 480
cagcccgaga acaactacaa gaccaccccc cctgtgctgg acagcgatgg cagcttcttc 540
ctgtacagca agctgaccgt ggacaagagc agatggcagc agggcaacgt gttcagctgc 600
tccgtgatgc acgaggccct gcacaatcac tacacccaga agagcctgag cctgtcccct 660
ggcaag 666
```

<210> 266
<211> 1302
<212> DNA
<213> Artificial Sequence

<220>
<223> Humanised

<400> 266

```
gaggtgcagc tggtggaaag cggcggcggc ctggtgcagc ccggcggctc cctgaggctg 60
agctgcgccg ctagcggcta caccttcacc agctactggc tccactgggt caggcaggcc 120
ccaggcaagg gactggagtg ggtgggcatg atcgacccca gcaacagcga caccaggttc 180
aaccccaact tcaaggacag gttcaccatc agcgccgaca ctagcaagaa caccgcctac 240
ctgcagatga acagcctgag ggccgaggac accgccgtgt attactgcgc cacctacagg 300
agctacgtca cccccctgga ttactggggc cagggcacac tagtcaccgt gagcagcgcc 360
agcaccaagg gccccagcgt gttccccctg gcccccctgca gcagaagcac cagcgagagc 420
acagccgccc tgggctgcct ggtgaaggac tacttccccg agcccgtgac cgtgagctgg 480
aacagcggag ccctgaccag cggcgtgcac accttccccg ccgtgctgca gagcagcggc 540
ctgtacagcc tgagcagcgt ggtgaccgtg cccagcagca gcctgggcac caagacctac 600
acctgcaacg tggaccacaa gcccagcaac accaaggtgg acaagcgggt ggcccccgag 660
ttcctgggcg gaccctccgt gttcctgttc cccccaagc ccaaggacac cctgatgatc 720
agccggaccc ccgaggtgac ctgcgtggtg gtggacgtga gccaggaaga tcccgaggtc 780
cagttcaatt ggtacgtgga cggcgtggag gtgcacaacg ccaagaccaa gccccgggag 840

gaacagttca acagcaccta ccgggtggtg tccgtgctga ccgtgctgca ccaggactgg 900
ctgaacggca aagaatacaa gtgcaaggtg tccaacaagg gcctgcccag ctccatcgag 960
aaaaccatca gcaaggccaa gggccagcct cgggagcccc aggtgtacac cctgccccca 1020
tcccaggaag agatgaccaa gaaccaggtg tccctgacct gtctggtgaa gggcttctac 1080
cccagcgaca tcgccgtgga gtgggagagc aacggccagc ccgagaacaa ctacaagacc 1140
acccccccctg tgctggacag cgacggcagc ttcttcctgt acagcaggct gaccgtggac 1200
aagagccggt ggcaggaagg caacgtcttt agctgcagcg tgatgcacga ggccctgcac 1260
aaccactaca cccagaagag cctgagcctg tccctgggca ag 1302
```

<210> 267
<211> 1701
<212> DNA
<213> Artificial Sequence

<220>
<223> Humanised

<400> 267

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt caccgtgagc 360
agcgccagca ccaagggccc cagcgtgttc cccctggccc cctgcagcag aagcaccagc 420
gagagcacag ccgccctggg ctgcctggtg aaggactact tccccgagcc cgtgaccgtg 480
agctggaaca gcggagccct gaccagcggc gtgcacacct tccccgccgt gctgcagagc 540
agcggcctgt acagcctgag cagcgtggtg accgtgccca gcagcaactt cggcacccag 600
acctacacct gcaacgtgga ccacaagccc agcaacacca aggtggacaa gaccgtggag 660
cggaagtgct gcgtggagtg cccccccctgc cctgcccctc ctgtggccgg accctccgtg 720
ttcctgttcc cccccaagcc caaggacacc ctgatgatca gccggacccc cgaggtgacc 780
tgcgtggtgg tggacgtgag ccacgaggac cccgaggtgc agttcaattg gtacgtggac 840
ggcgtggagg tgcacaacgc caagaccaag ccccgggagg aacagttcaa cagcaccttc 900
cgggtggtgt ccgtgctgac cgtggtgcac caggactggc tgaacggcaa agaatacaag 960
tgcaaggtgt ccaacaaggg cctgcctgcc cccatcgaga aaaccatcag caagaccaag 1020
ggccagccca gggaacccca ggtgtacacc ctgcccccca gccgggagga aatgaccaag 1080
aaccaggtgt ccctgacctg tctggtgaag ggcttctacc ccagcgacat cgccgtggag 1140
tgggagagca acggccagcc cgagaacaac tacaagacca cccccccccat gctggacagc 1200
gacggcagct tcttcctgta cagcaagctg acagtggaca agagccggtg gcagcagggc 1260
aacgtgttca gctgcagcgt gatgcacgag gccctgcaca accactacac ccagaagagc 1320
ctgagcctgt cccccggcaa gggatccggc gtgcagctcc tggagagcgg cggaggcctg 1380
gtccagcccg gcggcagcct gaggctgagc tgcgccgcca gcggcttcac cttcgcctgg 1440
tatgatatgg gctgggtgag gcaggccccc ggcaagggcc tggagtgggt gtccagcatc 1500
gactggcacg gggaggtgac ctactacgcc gacagcgtga agggcaggtt caccatcagc 1560
agggacaaca gcaagaacac cctgtacctg cagatgaaca gcctgagggc cgaggacacc 1620
gcagtgtact actgcgccac cgccgaggac gaacccggct acgactactg gggccagggc 1680
accctggtga ctgtgagcag c 1701
```

<210> 268
<211> 1704
<212> DNA
<213> Artificial sequence

<220>
<223> Humanised

<400> 268

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt caccgtgagc 360
agcgccagca ccaagggccc cagcgtgttc cccctggccc cctgcagcag aagcaccagc 420
gagagcacag ccgccctggg ctgcctggtg aaggactact tccccgagcc cgtgaccgtg 480
agctggaaca gcggagccct gaccagcggc gtgcacacct tccccgccgt gctgcagagc 540
agcggcctgt acagcctgag cagcgtggtg accgtgccca gcagcagcct gggcaccaag 600
acctacacct gcaacgtgga ccacaagccc agcaacacca aggtggacaa gcgggtggag 660
agcaagtacg gccctccctg ccccagctgc cctgcccccg agttcctggg cggaccctcc 720
gtgttcctgt tcccccccaa gcccaaggac accctgatga tcagccggac ccccgaggtg 780
acctgcgtgg tggtggacgt gagccaggaa gatcccgagg tccagttcaa ttggtacgtg 840
gacggcgtgg aggtgcacaa cgccaagacc aagccccggg aggaacagtt caacagcacc 900
taccgggtgg tgtccgtgct gaccgtgctg caccaggact ggctgaacgg caaagaatac 960
aagtgcaagg tgtccaacaa gggcctgccc agctccatcg agaaaaccat cagcaaggcc 1020
aagggccagc ctcgggagcc ccaggtgtac accctgcccc catcccagga agagatgacc 1080
aagaaccagg tgtccctgac ctgtctggtg aagggcttct accccagcga catcgccgtg 1140
```

gagtgggaga gcaacggcca gcccgagaac aactacaaga ccacccccc tgtgctggac 1200
agcgacggca gcttcttcct gtacagcagg ctgaccgtgg acaagagccg gtggcaggaa 1260
ggcaacgtct ttagctgcag cgtgatgcac gaggccctgc acaaccacta cacccagaag 1320
agcctgagcc tgtccctggg caagggatcc ggcgtgcagc tcctggagag cggcggaggc 1380
ctggtccagc ccggcggcag cctgaggctg agctgcgccg ccagcggctt caccttcgcc 1440
tggtatgata tgggctgggt gaggcaggcc cccggcaagg gcctggagtg ggtgtccagc 1500
atcgactggc acggggaggt gacctactac gccgacagcg tgaagggcag gttcaccatc 1560
agcagggaca acagcaagaa caccctgtac ctgcagatga acagcctgag ggccgaggac 1620
accgcagtgt actactgcgc caccgccgag gacgaacccg gctacgacta ctggggccag 1680
ggcaccctgg tgactgtgag cagc 1704

<210> 269
<211> 1704
<212> DNA
<213> Artificial Sequence

<220>
<223> Humanised

<400> 269

caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt caccgtgagc 360
agcgccagca ccaagggccc cagcgtgttc cccctggccc cctgcagcag aagcaccagc 420
gagagcacag ccgccctggg ctgcctggtg aaggactact tccccgagcc cgtgaccgtg 480
agctggaaca gcggagccct gaccagcggc gtgcacacct tccccgccgt gctgcagagc 540
agcggcctgt acagcctgag cagcgtggtg accgtgccca gcagcagcct gggcaccaag 600
acctacacct gcaacgtgga ccacaagccc agcaacacca aggtggacaa gcgggtggag 660
agcaagtacg gccctccctg cccccccctgc cctgcccccg agttcgaggg cggaccctcc 720
gtgttcctgt tccccccaa gcccaaggac accctgatga tcagccggac ccccgaggtg 780
acctgcgtgg tggtggacgt gagccaggaa gatcccgagg tccagttcaa ttggtacgtg 840
gacggcgtgg aggtgcacaa cgccaagacc aagccccggg aggaacagtt caacagcacc 900
taccgggtgg tgtccgtgct gaccgtgctg caccaggact ggctgaacgg caaagaatac 960
aagtgcaagg tgtccaacaa gggcctgccc agctccatcg agaaaaccat cagcaaggcc 1020
aagggccagc ctcgggagcc ccaggtgtac accctgcccc catcccagga agagatgacc 1080
aagaaccagg tgtccctgac ctgtctggtg aagggcttct accccagcga catcgccgtg 1140
gagtgggaga gcaacggcca gcccgagaac aactacaaga ccacccccc tgtgctggac 1200
agcgacggca gcttcttcct gtacagcagg ctgaccgtgg acaagagccg gtggcaggaa 1260
ggcaacgtct ttagctgcag cgtgatgcac gaggccctgc acaaccacta cacccagaag 1320
agcctgagcc tgtccctggg caagggatcc ggcgtgcagc tcctggagag cggcggaggc 1380
ctggtccagc ccggcggcag cctgaggctg agctgcgccg ccagcggctt caccttcgcc 1440
tggtatgata tgggctgggt gaggcaggcc cccggcaagg gcctggagtg ggtgtccagc 1500
atcgactggc acggggaggt gacctactac gccgacagcg tgaagggcag gttcaccatc 1560
agcagggaca acagcaagaa caccctgtac ctgcagatga acagcctgag ggccgaggac 1620
accgcagtgt actactgcgc caccgccgag gacgaacccg gctacgacta ctggggccag 1680
ggcaccctgg tgactgtgag cagc 1704

<210> 270
<211> 1641
<212> DNA
<213> Artificial Sequence

<220>
<223> Humanised

<400> 270

```
caggtgaccc tgagggagag cggccccgcc ctggtgaagc ccacccagac cctgaccctg 60
acctgcacct tcagcggctt tagcctcagc acctccggca tgggcgtgag ctggatcagg 120
cagccacccg gcaaaggcct ggagtggctg gcccacatct actgggacga cgacaagagg 180
tacaacccca gcctgaagag ccggctgacc atcagcaagg ataccagcag gaaccaggtg 240
gtgctgacca tgaccaacat ggaccccgtg gacaccgcta cctactactg cgccaggagg 300
gagaccgtct tctactggta cttcgacgtg tggggaaggg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt ccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020

agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aagggatccg tgagcgacgt gccaagggac 1380
ctcgaggtgg tggcagccac tcccacctct ctgctgatca gctgggacac acacaacgcc 1440
tacaacggct actacaggat cacctacgga gagaccggcg gcaatagccc cgtgagggag 1500
ttcaccgtgc cccaccccga ggtgaccgcc accattagcg gcctgaagcc cggcgtggac 1560
gataccatca ccgtctacgc cgtgaccaac caccacatgc ccctgaggat cttcggcccc 1620
atcagcatca accataggac c                                          1641
```

## Description

### Background

Antibodies are well known for use in therapeutic applications.

Antibodies are heteromultimeric glycoproteins comprising at least two heavy and two light chains. Aside from IgM, intact antibodies are usually heterotetrameric glycoproteins of approximately 150Kda, composed of two identical light (L) chains and two identical heavy (H) chains. Typically, each light chain is linked to a heavy chain by one covalent disulfide bond while the number of disulfide linkages between the heavy chains of different immunoglobulin isotypes varies. Each heavy and light chain also has intrachain disulfide bridges. Each heavy chain has at one end a variable domain (VH) followed by a number of constant regions. Each light chain has a variable domain (VL) and a constant region at its other end; the constant region of the light chain is aligned with the first constant region of the heavy chain and the light chain variable domain is aligned with the variable domain of the heavy chain. The light chains of antibodies from most vertebrate species can be assigned to one of two types called Kappa and Lambda based on the amino acid sequence of the constant region. Depending on the amino acid sequence of the constant region of their heavy chains, human antibodies can be assigned to five different classes, IgA, IgD, IgE, IgG and IgM. IgG and IgA can be further subdivided into subclasses, IgG1, IgG2, IgG3 and IgG4; and IgA1 and IgA2. Species variants exist with mouse and rat having at least IgG2a, IgG2b. The variable domain of the antibody confers binding specificity upon the antibody with certain regions displaying particular variability called complementarity determining regions (CDRs). The more conserved portions of the variable region are called Framework regions (FR). The variable domains of intact heavy and light chains each comprise four FR connected by three CDRs. The CDRs in each chain are held together in close proximity by the FR regions and with the CDRs from the other chain contribute to the formation of the antigen binding site of antibodies. The constant regions are not directly involved in the binding of the antibody to the antigen but exhibit various effector functions such as participation in antibody dependent cell-mediated cytotoxicity (ADCC), phagocytosis via binding to Fcγ receptor, half-life/clearance rate via neonatal Fc receptor (FcRn) and complement dependent cytotoxicity via the C1 q component of the complement cascade.

The nature of the structure of an IgG antibody is such that there are two antigen-binding sites, both of which are specific for the same epitope. They are therefore, monospecific.

A bispecific antibody is an antibody having binding specificities for at least two different epitopes. Methods of making such antibodies are known in the art.

Traditionally, the recombinant production of bispecific antibodies is based on the coexpression of two immunoglobulin H chain-L chain pairs, where the two H chains have different binding specificities see Millstein et al, Nature 305 537-539 (1983), WO93/08829 and Traunecker et al EMBO, 10, 1991, 3655-3659. Because of the random assortment of H and L chains, a potential mixture of ten different antibody structures are produced of which only one has the desired binding specificity. An alternative approach involves fusing the variable domains with the desired binding specificities to heavy chain constant region comprising at least part of the hinge region, CH2 and CH3 regions. It is preferred to have the CH1 region containing the site necessary for light chain binding present in at least one of the fusions. DNA encoding these fusions, and if desired the L chain are inserted into separate expression vectors and are then cotransfected into a suitable host organism. It is possible though to insert the coding sequences for two or all three chains into one expression vector. In one approach, a bispecific antibody is composed of a H chain with a first binding specificity in one arm and a H-L chain pair, providing a second binding specificity in the other arm, see WO94/04690. Also see Suresh et al Methods in Enzymology 121, 210, 1986. Other approaches include antibody molecules which comprise single domain binding sites which is set out in WO2007/095338. Shen Juqun et al (2007) Journal of Immunological Methods, 318, 65-74 describes a single variable domain antibody in the construction of IgG-like bispecific antibodies. WO 2007/066106 describes ligands that have binding specificity for VEGF, for EGFR, or for VEGF and EGFR. US 6,492,123 describes polypeptides comprising a first domain, which comprises a binding region of an immunoglobulin heavy chain variable region, and a second domain, which comprises a binding region of an immunoglobulin light chain variable region, the domains being linked but incapable of associating with each other to form an antigen binding site, associate to form antigen binding multimers, such as dimers, which may be multivalent or have multispecificity. WO 2007/085814 describes recombinant fusion proteins that comprise at least one natural junction.

### Summary of invention

The present invention relates to an antigen-binding construct comprising a protein scaffold which is an antibody immunoglobulin scaffold comprising at least two heavy chains and two light chains, which scaffold is linked to one or more epitope-binding domains wherein the antigen-binding construct has four antigen binding sites, two of which are, from epitope binding domains which are immunoglobulin single variable domains, and two of which are from paired VHNL domains, wherein the antigen binding construct is capable of binding IL-13, wherein at least one of the immunoglobulin single variable domains is directly attached to the scaffold with a linker comprising from 1 to 50 amino acids and wherein the immunoglobulin single variable domains are attached to the immunoglobulin scaffold at the C-terminus of the heavy chain.

The invention relates to IgG based structures which comprise monoclonal antibodies, or fragments linked to one or more domain antibodies, and to methods of making such constructs and uses thereof, particularly uses in therapy.

The invention also provides a polynucleotide sequence encoding a heavy chain of any of the antigen binding constructs described herein, and a polynucleotide encoding a light chain of any of the antigen binding constructs described herein. Such polynucleotides represent the coding sequence which corresponds to the equivalent polypeptide sequences, however it will be understood that such polynucleotide sequences could be cloned into an expression vector along with a start codon, an appropriate signal sequence and a stop codon.

The invention also provides a recombinant transformed or transfected host cell comprising one or more polynucleotides encoding a heavy chain and a light chain of any of the antigen binding constructs described herein.

The invention further provides a method for the production of any of the antigen binding constructs described herein which method comprises the step of culturing a host cell comprising a first and second vector, said first vector comprising a polynucleotide encoding a heavy chain of any of the antigen binding constructs described herein and said second vector comprising a polynucleotide encoding a light chain of any of the antigen binding constructs described herein, in a serum- free culture media.

The invention further provides a pharmaceutical composition comprising an antigen binding construct as described herein a pharmaceutically acceptable carrier.

The invention also provides any of the antigen binding constructs described herein for use in medicine.

The invention further provides any of the antigen binding constructs described herein for the treatment of inflammatory diseases such as asthma, reumathoid arthritis or osteoarthritis.

**Definitions**

The term 'Protein Scaffold' as used herein includes but is not limited to an immunoglobulin (Ig) scaffold, for example an IgG scaffold, which may be a four chain or two chain antibody, or which may comprise only the Fc region of an antibody, or which may comprise one or more constant regions from an antibody, which constant regions may be of human or primate origin, or which may be an artificial chimera of human and primate constant regions. Such protein scaffolds may comprise antigen-binding sites in addition to the one or more constant regions, for example where the protein scaffold comprises a full IgG. Such protein scaffolds will be capable of being linked to other protein domains, for example protein domains which have antigen-binding sites, for example epitope-binding domains or ScFv domains.

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. A "single antibody variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain ($V_H$, $V_{HH}$, $V_L$) that specifically binds an antigen or epitope independently of a different V region or domain. An immunoglobulin single variable domain can be present in a format (*e.g.*, homo- or hetero-multimer) with other, different variable regions or variable domains where the other regions or domains are not required for antigen binding by the single immunoglobulin variable domain (*i.e.*, where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and *Camelid* $V_{HH}$ dAbs. Camelid $V_{HH}$ are immunoglobulin single variable domain polypeptides that are derived from species including camel, llama, alpaca, dromedary, and guanaco, which produce heavy chain antibodies naturally devoid of light chains. Such $V_{HH}$ domains may be humanised according to standard techniques available in the art, and such domains are still considered to be "domain antibodies" according to the invention. As used herein "$V_H$ includes camelid $V_{HH}$ domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a domain antibody (dAb), for example a human, camelid or shark immunoglobulin single variable domain or itmay be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEI and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

CTLA-4 (Cytotoxic T Lymphocyte-associated Antigen 4) is a CD28-family receptor expressed on mainly CD4+ T-cells. Its extracellular domain has a variable domain-like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001)

Lipocalins are a family of extracellular proteins which transport small hydrophobic molecules such as steroids, bilins, retinoids and lipids. They have a rigid β-sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633

An affibody is a scaffold derived from Protein A of *Staphylococcus aureus* which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1

Avimers are multidomain proteins derived from the A-domain scaffold family. The native domains of approximately 35 amino acids adopt a defined disulphide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007)

A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two α-helices and a β-turn. They can be engineered to bind different target antigens by randomising residues in the first α-helix and a β-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the β-sandwich can be engineered to enable an Adnectin to specifically recognize a therapeutic target of interest. For further details see Protein Eng. Des. Sel. 18, 435-444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataB1 and conotoxin and knottins. The microproteins have a loop which can be engineered to include upto 25 amino acids without affecting the overall fold of the microprotein. For further details of engineered knottin domains, see WO2008098796.

Other epitope binding domains include proteins which have been used as a scaffold to engineer different target antigen binding properties include human γ-crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Epitope binding domains of the present invention could be derived from any of these alternative protein domains.

As used herein, the terms "paired VH domain", "paired VL domain", and "paired VH/VL domains" refer to antibody variable domains which specifically bind antigen only when paired with their partner variable domain. There is always one VH and one VL in any pairing, and the term "paired VH domain" refers to the VH partner, the term "paired VL domain" refers to the VL partner, and the term "paired VH/VL domains" refers to the two domains together.

The antigen binding site may bind to antigen with a Kd of at least 1 mM, for example a Kd of 10nM, 1nM, 500pM, 200pM, 100pM, to each antigen as measured by Biacore™, such as the Biacore™ method as described in method 4 or 5.

As used herein, the term "antigen binding site" refers to a site on a construct which is capable of specifically binding to antigen, this may be a single domain, for example an epitope-binding domain, or it may be paired VH/VL domains as can be found on a standard antibody. In some aspects of the invention single-chain Fv (ScFv) domains can provide antigen-binding sites.

The terms "mAb/dAb" and dAb/mAb" are used herein to refer to antigen-binding constructs of the present invention. The two terms can be used interchangeably, and are intended to have the same meaning as used herein.

The term "constant heavy chain 1" is used herein to refer to the CH1 domain of an immunoglobulin heavy chain.

The term "constant light chain" is used herein to refer to the constant domain of an immunoglobulin light chain.

**Detailed description of Invention**

The present invention relates to an antigen-binding construct comprising a protein scaffold which is an antibody immunoglobulin scaffold comprising at least two heavy chains and two light chains, which scaffold is linked to one or more epitope-binding domains wherein the antigen-binding construct has four antigen binding sites, two of which are from epitope binding domains which are immunoglobulin single variable domains, and two of which are from paired VH/VL domains, wherein the antigen binding construct is capable of binding IL-13, wherein at least one of the immunoglobulin single variable domains is directly attached to the scaffold with a linker comprising from 1 to 50 amino acids and wherein the immunoglobulin single variable domains are attached to the immunoglobulin scaffold at the C-terminus of the heavy chain.

The antigen-binding constructs of the present invention are also referred to as mAbdAbs.

In one embodiment the protein scaffold of the antigen-binding construct of the present invention is an Ig scaffold, for example an IgG scaffold or IgA scaffold. The IgG scaffold may comprise all the domains of an antibody (i.e. CH1, CH2, CH3, VH, VL). The antigen-binding construct of the present invention may comprise an IgG scaffold selected from IgG1, IgG2, IgG3, IgG4 or IgG4PE.

The antigen-binding construct of the present invention has at least foour antigen binding sites, for examples it has four binding sites, for example where a first binding site has specificity for a first epitope on an antigen and a second binding site has specificity for a second epitope on the same antigen. In one embodiment the antigen binding construct has specificity for more than one antigen, for example two antigens, or for three antigens, or for four antigens.

In one embodiment of the present invention the epitope binding domain is a dAb.

It will be understood that any of the antigen-binding constructs described herein will be capable of neutralising one or more antigens.

The term "neutralises" and grammatical variations thereof as used throughout the present specification in relation to antigen binding constructs of the invention means that a biological activity of the target is reduced, either totally or partially, in the presence of the antigen binding constructs of the present invention in comparison to the activity of the target in the absence of such antigen binding constructs. Neutralisation may be due to but not limited to one or more of blocking ligand binding, preventing the ligand activating the receptor, down regulating the receptor or affecting effector functionality.

Levels of neutralisation can be measured in several ways, for example by use of any of the assays as set out in the examples and methods below, for example in an assay which measures inhibition of ligand binding to receptor which may be carried out for example as described in any one of Methods 12, 19 or 21 or Example 32. The neutralisation of VEGF, IL-4, IL-13 or HGF in these assays is measured by assessing the decreased binding between the ligand and its receptor in the presence of neutralising antigen binding construct.

Levels of neutralisation can also be measured, for example in a TF1 assay which may be carried out for example as described in Method 8, 9, 10, 20 or 21. The neutralisation of IL-13, IL-4 or both of these cytokines in this assay is measured by assessing the inhibition of TF1 cell proliferation in the presence of neutralising antigen binding construct. Alternatively neutralisation could be measured in an EGFR phosphorylation assay which may be carried out for example as described in Method 13. The neutralisation of EGFR in this assay is measured by assessing the inhibition of tyrosine kinase phosphorylation of the receptor in the presence of neutralising antigen binding construct. Or, neutralisation could be measured in an IL-8 secretion assay in MRC-5 cells which may be carried out for example as described in Method 14 or 15. The neutralisation of TNF$\alpha$ or IL-1R1 in this assay is measured by assessing the inhibition of IL-8 secretion in the presence of neutralising antigen binding construct.

Other methods of assessing neutralisation, for example, by assessing the decreased binding between the ligand and its receptor in the presence of neutralising antigen binding construct are known in the art, and include, for example, Biacore™ assays.

Also described in this application are antigen binding constructs which have at least substantially equivalent neutralising activity to the antibodies exemplified herein, for example antigen binding constructs which retain the neutralising activity of 586H-TVAAPS-210, or PascoH-G4S-474, or PascoH-474, PascoH-474 GS removed, PascoL-G4S-474 or PascoHL-G4S-474 in the TF1 cell proliferation assay, or inhibition of pSTAT6 signalling assay as set out in Examples 4 and 20 respectively, or for example antigen binding constructs which retain the neutralising activity of BPC1603, BPC1604, BPC1605, BPC1606 in the VEGFR binding assay or inhibition of IGF-1R receptor phosphorylation as set out in Examples 14.6 and 14.7.

The antigen binding constructs of the invention include those which have specificity for IL-13, for example which comprise an epitope-binding domain which is capable of binding to IL-13, or which comprise a paired VH/VL which binds to IL-13. The antigen binding construct may comprise an antibody which is capable of binding to IL-13. The antigen binding construct may comprise a dAb which is capable of binding to IL-13.

In one embodiment the antigen-binding construct of the present invention has specificity for more than one antigen, for example where it is capable of binding two or more antigens selected from IL-13, IL-5, and IL-4, for example where it is capable of binding IL-13 and IL-4, or where it is capable of binding IL-13 and IL-5, or where it is capable of binding IL-5 and IL-4.

In one embodiment the antigen-binding construct of the present invention has specificity for more than one antigen, for example where it is capable of binding two or more antigens selected from IL-13, IL-5, and IL-4, for example where it is capable of binding IL-13 and IL-4 simultaneously, or where it is capable of binding IL-13 and IL-5 simultaneously, or where it is capable of binding IL-5 and IL-4 simultaneously.

It will be understood that any of the antigen-binding constructs described herein may be capable of binding two or more antigens simultaneously, for example, as determined by stochiometry analysis by using a suitable assay such as that described in the Examples section, method 7.

Examples of antigen-binding constructs of the invention include IL-13 antibodies which have an epitope binding domain with a specificity for IL-4, for example an anti-IL-4 dAb, attached to the c-terminus or the n-terminus of the heavy chain or the c-terminus or n-terminus of the light chain, for example the mAbdAb having the heavy chain sequence set out in SEQ ID NO:16 to 39, SEQ ID NO:41 to 43, SEQ ID NO:87 to 90, SEQ ID NO:151, SEQ ID NO:152 or SEQ ID NO:155. Antigen binding constructs of the present invention include IL-13 antibodies with an IL-4 epitope binding domain attached to the n-terminus of the heavy chain. Antigen binding constructs of the present invention include IL-13 antibodies with an IL-4 epitope binding domain attached to the n-terminus of the light chain. Antigen binding constructs of the present invention include IL-13 antibodies with an IL-4 epitope binding domain attached to the c-terminus of the heavy chain. Antigen binding constructs of the present invention include IL-13 antibodies with an IL-4 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-4 antibodies which have an epitope binding domain with a specificity for IL-13, for example an anti-IL-13 dAb, attached to the c-terminus or the n-terminus of the heavy chain or the c-terminus or n-terminus of the light chain, for example the mAbdAb having the heavy chain sequence set out in SEQ ID NO:48 to 53, SEQ ID NO:91 SEQ ID NO:92, SEQ ID NO:149, SEQ ID NO:150, or SEQ ID NO:157 to 160, and/or the light chain sequence set out in SEQ ID NO:54 to 59.

Antigen binding constructs of the present invention include IL-4 antibodies with an IL-13 epitope binding domain attached to the n-terminus of the heavy chain. Antigen binding constructs of the present invention include IL-4 antibodies with an IL-13 epitope binding domain attached to the n-terminus of the light chain. Antigen binding constructs of the present invention include IL-4 antibodies with an IL-13 epitope binding domain attached to the c-terminus of the heavy chain. Antigen binding constructs of the present invention include IL-4 antibodies with an IL-13 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-13 antibodies which have an epitope binding domain with a specificity for IL-5, for example an anti-IL-5 dAb, attached to the c-terminus or the n-terminus of the heavy chain or the c-terminus or n-terminus of the light chain.. Antigen binding constructs of the present invention include IL-13 antibodies with an IL-5 epitope binding domain attached to the n-terminus of the heavy chain. Antigen binding constructs of the present invention include IL-13 antibodies with an IL-5 epitope binding domain attached to the n-terminus of the light chain. Antigen binding constructs of the present invention include IL-13 antibodies with an IL-5 epitope binding domain attached to the c-terminus of the heavy chain. Antigen binding constructs of the present invention include IL-13 antibodies with an IL-5 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-5 antibodies which have an epitope binding domain with a specificity for IL-13, for example an anti-IL-13 dAb, attached to the c-terminus or the n-terminus of the heavy chain or the c-terminus or n-terminus of the light chain, for example the mAbdAb having the light chain sequence set out in SEQ ID NO: 72.

Antigen binding constructs of the present invention include IL-5 antibodies with an IL-13 epitope binding domain attached to the n-terminus of the heavy chain. Antigen binding constructs of the present invention include IL-5 antibodies with an IL-13 epitope binding domain attached to the n-terminus of the light chain. Antigen binding constructs of the present invention include IL-5 antibodies with an IL-13 epitope binding domain attached to the c-terminus of the heavy chain. Antigen binding constructs of the present invention include IL-5 antibodies with an IL-13 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-4 antibodies which have an epitope binding domain with a specificity for IL-5, for example an anti-IL-5 dAb, attached to the c-terminus or the n-terminus of the heavy chain or the c-terminus or n-terminus of the light chain.. Antigen binding constructs of the present invention include IL-4 antibodies with an IL-5 epitope binding domain attached to the n-terminus of the heavy chain. Antigen binding constructs of the present invention include IL-4 antibodies with an IL-5 epitope binding domain attached to the n-terminus of the light chain. Antigen binding constructs of the present invention include IL-4 antibodies with an IL-5 epitope binding domain attached to the c-terminus of the heavy chain. Antigen binding constructs of the present invention include IL-4 antibodies with an IL-5 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include IL-5 antibodies which have an epitope binding domain with a specificity for IL-4, for example an anti-IL-4 dAb, attached to the c-terminus or the n-terminus of the heavy chain or the c-terminus or n-terminus of the light chain, for example the mAbdAb having the heavy chain sequence set out in SEQ ID NO: 71.

Antigen binding constructs of the present invention include IL-5 antibodies with an IL-4 epitope binding domain attached to the n-terminus of the heavy chain. Antigen binding constructs of the present invention include IL-5 antibodies with an IL-4 epitope binding domain attached to the n-terminus of the light chain. Antigen binding constructs of the present invention include IL-5 antibodies with an IL-4 epitope binding domain attached to the c-terminus of the heavy chain. Antigen binding constructs of the present invention include IL-5 antibodies with an IL-4 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

The invention also provides a trispecific binding construct which is capable of binding to IL-4, IL-13 and IL-5.

Examples of such antigen-binding constructs include IL-5 antibodies which have an epitope binding domain with a specificity for IL-4, for example an anti-IL-4 dAb, attached to the c-terminus or the n-terminus of the heavy chain or the c-terminus or n-terminus of the light chain and an epitope binding domain with a specificity for IL-13, for example an anti-IL-13 dAb, attached to the c-terminus or the n-terminus of the heavy chain or the c-terminus or n-terminus of the light chain.

Antigen binding constructs of the present invention include IL-5 antibodies with an IL-4 epitope binding domain attached to the n-terminus of the heavy chain and an IL-13 epitope binding domain attached to the c-terminus of the light chain.

Antigen binding constructs of the present invention include IL-5 antibodies with an IL-4 epitope binding domain attached to the n-terminus of the heavy chain and an IL-13 epitope binding domain attached to the c-terminus of the heavy chain.

Antigen binding constructs of the present invention include IL-5 antibodies with an IL-4 epitope binding domain attached to the n-terminus of the light chain and an IL-13 epitope binding domain attached to the c-terminus of the light chain.

Antigen binding constructs of the present invention include IL-5 antibodies with an IL-4 epitope binding domain attached to the n-terminus of the light chain and an IL-13 epitope binding domain attached to the c-terminus of the heavy chain.

Antigen binding constructs of the present invention include IL-5 antibodies with an IL-4 epitope binding domain attached to the c-terminus of the heavy chain and an IL-13 epitope binding domain attached to the c-terminus of the light chain.

Antigen binding constructs of the present invention include IL-5 antibodies with an IL-4 epitope binding domain attached to the c-terminus of the heavy chain and an IL-13 epitope binding domain attached to the n-terminus of the light chain.

Antigen binding constructs of the present invention include IL-5 antibodies with an IL-4 epitope binding domain attached to the c-terminus of the heavy chain and an IL-13 epitope binding domain attached to the n-terminus of the heavy chain.

Antigen binding constructs of the present invention include IL-5 antibodies with an IL-4 epitope binding domain attached to the c-terminus of the light chain and an IL-13 epitope binding domain attached to the c-terminus of the heavy chain.

Antigen binding constructs of the present invention include IL-5 antibodies with an IL-4 epitope binding domain attached to the c-terminus of the light chain and an IL-13 epitope binding domain attached to the n-terminus of the heavy chain.

Antigen binding constructs of the present invention include IL-5 antibodies with an IL-4 epitope binding domain attached to the c-terminus of the light chain and an IL-13 epitope binding domain attached to the n-terminus of the light chain.

Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for IL-18, for example which comprises an epitope-binding domain which is capable of binding to IL-18, or which comprises a paired VHNL which binds to IL-18.

The antigen binding construct may comprise an antibody which is capable of binding to IL-18. The antigen binding construct may comprise a dAb which is capable of binding to IL-18.

The invention also provides a trispecific binding construct which is capable of binding to IL-4, IL-13 and IL-18.

Examples of such antigen-binding constructs include IL-18 antibodies which have an epitope binding domain with a specificity for IL-4, for example an anti-IL-4 dAb, attached to the c-terminus or the n-terminus of the heavy chain or the c-terminus or n-terminus of the light chain and an epitope binding domain with a specificity for IL-13, for example an anti-IL-13 dAb, attached to the c-terminus or the n-terminus of the heavy chain or the c-terminus or n-terminus of the light chain.

Antigen binding constructs of the present invention include IL-18 antibodies with an IL-4 epitope binding domain attached to the n-terminus of the heavy chain and an IL-13 epitope binding domain attached to the c-terminus of the light chain.

Antigen binding constructs of the present invention include IL-18 antibodies with an IL-4 epitope binding domain attached to the n-terminus of the heavy chain and an IL-13 epitope binding domain attached to the c-terminus of the heavy chain.

Antigen binding constructs of the present invention include IL-18 antibodies with an IL-4 epitope binding domain attached to the n-terminus of the light chain and an IL-13 epitope binding domain attached to the c-terminus of the light chain.

Antigen binding constructs of the present invention include IL-18 antibodies with an IL-4 epitope binding domain attached to the n-terminus of the light chain and an IL-13 epitope binding domain attached to the c-terminus of the heavy chain.

Antigen binding constructs of the present invention include IL-18 antibodies with an IL-4 epitope binding domain attached to the n-terminus of the light chain and an IL-13 epitope binding domain attached to the n-terminus of the heavy chain.

Antigen binding constructs of the present invention include IL-18 antibodies with an IL-4 epitope binding domain attached to the c-terminus of the heavy chain and an IL-13 epitope binding domain attached to the c-terminus of the light chain.

Antigen binding constructs of the present invention include IL-18 antibodies with an IL-4 epitope binding domain attached to the c-terminus of the heavy chain and an IL-13 epitope binding domain attached to the n-terminus of the light chain.

Antigen binding constructs of the present invention include IL-18 antibodies with an IL-4 epitope binding domain attached to the c-terminus of the heavy chain and an IL-13 epitope binding domain attached to the n-terminus of the heavy chain.

Antigen binding constructs of the present invention include IL-18 antibodies with an IL-4 epitope binding domain attached to the c-terminus of the light chain and an IL-13 epitope binding domain attached to the c-terminus of the heavy chain.

Antigen binding constructs of the present invention include IL-18 antibodies with an IL-4 epitope binding domain attached to the c-terminus of the light chain and an IL-13 epitope binding domain attached to the n-terminus of the heavy chain.

Antigen binding constructs of the present invention include IL-18 antibodies with an IL-4 epitope binding domain attached to the c-terminus of the light chain and an IL-13 epitope binding domain attached to the n-terminus of the light chain.

Such antigen-binding constructs may also have one or more further epitope binding domains with the same or different antigen-specificity attached to the c-terminus and/or the n-terminus of the heavy chain and/ or the c-terminus and/or n-terminus of the light chain.

Described here are antigen binding constructs which have specificity for TNFα, for example which comprises an epitope-binding domain which is capable of binding to TNFα, or which comprises a paired VH/VL which binds to TNFα.

The antigen binding construct may comprise an antibody which is capable of binding to TNFα The antigen binding construct may comprise a dAb which is capable of binding to TNFα.

The antigen-binding constructs may have specificity for more than one antigen, for example where it is capable of binding two or more antigens selected from TNFα, EGFR and VEGF, for example where it is capable of binding TNFα and EGFR, or where it is capable of binding TNFα and VEGF, or where it is capable of binding EGFR and VEGF. Examples of such antigen-binding constructs include TNFα antibodies which have an epitope binding domain with a specificity for EGFR, for example an anti-EGFR dAb, attached to the c-terminus or the n-terminus of the heavy chain or the c-terminus or n-terminus of the light chain, for example a mAbdAb having the heavy chain sequence set out in SEQ ID NO: 74, and/or the light chain sequence set out in SEQ ID NO: 79.

Antigen binding constructs described here include TNFα antibodies with an EGFR epitope binding domain attached to the n-terminus of the heavy chain. Antigen binding constructs described here include TNFα antibodies with an EGFR epitope binding domain attached to the n-terminus of the light chain. Antigen binding constructs described here include TNFα antibodies with an EGFR epitope binding domain attached to the c-terminus of the heavy chain. Antigen binding constructs described here include TNFα antibodies with an EGFR epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs described here include EGFR antibodies with an TNFα epitope binding domain attached to the n-terminus of the heavy chain. Antigen binding constructs described here include EGFR antibodies with an TNFα epitope binding domain attached to the n-terminus of the light chain. Antigen binding constructs described here include EGFR antibodies with an TNFα epitope binding domain attached to the c-terminus of the heavy chain. Antigen binding constructs described here include EGFR antibodies with an TNFα epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Examples of such antigen-binding constructs include TNFα antibodies which have an epitope binding domain with a specificity for VEGF, for example an anti-VEGF dAb, attached to the c-terminus or the n-terminus of the heavy chain or the c-terminus or n-terminus of the light chain, for example a mAbdAb having the heavy chain sequence set out in SEQ ID NO: 75, 78 or 185.

The antigen-binding construct described here may have specificity for more than one antigen, for example where it is capable of binding TNFα, and one or both antigens selected from IL-4 and IL-13, for example where it is capable of binding TNFα and IL-4, or where it is capable of binding TNFα and IL-13, or where it is capable of binding TNFα and IL-13 and IL-4. Examples of such antigen-binding constructs include IL-13 antibodies which have an epitope binding domain with a specificity for TNFα, for example an anti- TNFα adnectin, attached to the c-terminus or the n-terminus of the heavy chain or the c-terminus or n-terminus of the light chain, for example a mAbdAb having the heavy chain sequence set out in SEQ ID NO: 134 or 135. Other examples of such antigen-binding constructs include IL-4 antibodies which have an epitope binding domain with a specificity for TNFα, for example an anti- TNFα adnectin, attached to the c-terminus or the n-terminus of the heavy chain or the c-terminus or n-terminus of the light chain, for example a mAbdAb having the heavy chain sequence set out in SEQ ID NO: 146 or 147.

Antigen binding constructs described here include TNFα antibodies with an VEGF epitope binding domain attached to the n-terminus of the heavy chain. Antigen binding constructs described here include TNFα antibodies with an VEGF epitope binding domain attached to the n-terminus of the light chain. Antigen binding constructs described here include TNFα antibodies with an VEGF epitope binding domain attached to the c-terminus of the heavy chain. Antigen binding constructs described here include TNFα antibodies with an VEGF epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs described here include VEGF antibodies with an TNFα epitope binding domain attached to the n-terminus of the heavy chain. Antigen binding constructs described here include VEGF antibodies with an TNFα epitope binding domain attached to the n-terminus of the light chain. Antigen binding constructs described here include VEGF antibodies with an TNFα epitope binding domain attached to the c-terminus of the heavy chain. Antigen binding constructs described here include VEGF antibodies with an TNFα epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for CD-20, for example which comprises an epitope-binding domain which is capable of binding to CD-20, or which comprises a paired VH/VL which binds to CD-20.

The antigen binding construct may comprise an antibody which is capable of binding to CD-20, for example it may comprise an antibody having the heavy and light chain sequences of SEQ ID NO: 120 and 117. The antigen binding construct may comprise a dAb which is capable of binding to CD-20. Examples of mAbdAbs with specificity for CD-20 are those having the heavy chain sequence set out in SEQ ID NO: 116, 118 or those having the light chain sequence set out in SEQ ID NO: 119 or 121. Such antigen-binding constructs may also have one or more further epitope binding domains with the same or different antigen-specificity attached to the c-terminus and/or the n-terminus of the heavy chain and/or the c-terminus and/or n-terminus of the light chain.

The antigen binding constructs of the invention include those which have specificity for IL1R1, for example which comprise an epitope-binding domain which is capable of binding to IL1R1, or which comprises a paired VH/VL which binds to IL1R1.

The antigen binding construct may comprise an antibody which is capable of binding to IL1R1. The antigen binding construct may comprise a dAb which is capable of binding to IL1R1.

In one embodiment the antigen-binding construct described here has specificity for more than one antigen, for example where it is capable of binding IL1R1 and a second antigen, for example where it is capable of binding IL1R1 and VEGF.

Examples of such antigen-binding constructs include IL1R1 antibodies which have an epitope binding domain with a specificity for VEGF, for example an anti-VEGF dAb, attached to the c-terminus or the n-terminus of the heavy chain or the c-terminus or n-terminus of the light chain, for example a mAbdAb having the light chain sequence set out in SEQ ID NO: 77.

Antigen binding constructs described here include IL1R1 antibodies with an VEGF epitope binding domain attached to the n-terminus of the heavy chain. Antigen binding constructs described here include IL1R1 antibodies with an VEGF epitope binding domain attached to the n-terminus of the light chain. Antigen binding constructs described here include IL1R1 antibodies with an VEGF epitope binding domain attached to the c-terminus of the heavy chain. Antigen binding constructs described here include IL1R1 antibodies with an VEGF epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

Antigen binding constructs described here include VEGF antibodies with an IL1R1 epitope binding domain attached to the n-terminus of the heavy chain. Antigen binding constructs described here include VEGF antibodies with an IL1R1 epitope binding domain attached to the n-terminus of the light chain. Antigen binding constructs described here include VEGF antibodies with an IL1R1 epitope binding domain attached to the c-terminus of the heavy chain. Antigen binding constructs described here include VEGF antibodies with an IL1R1 epitope binding domain attached to the c-terminus of the light chain. Such antigen-binding constructs may also have one or more further epitope binding domains with the same or different antigen-specificity attached to the c-terminus and/or the n-terminus of the heavy chain and/ or the c-terminus and/or n-terminus of the light chain.

The antigen binding constructs of the invention include those which have specificity for EGFR, for example which comprises an epitope-binding domain which is capable of binding to EGFR, or which comprises a paired VH/VL which binds to EGFR. The antigen binding construct may comprise an antibody which is capable of binding to EGFR. The antigen binding construct may comprise a dAb which is capable of binding to EGFR. Some examples of such antigen binding construct will be capable of binding to an epitope on EGFR comprising SEQ ID NO:103, for example an antigen binding construct comprising one or more of the CDRs set out in SEQ ID NO: 97 to SEQ ID NO: 102 and SEQ ID NO: 104 to SEQ ID NO: 107.

In one embodiment the antigen-binding construct described here has specificity for more than one antigen, for example where it is capable of binding two or more antigens selected from EGFR, IGF-1R, VEGFR2 and VEGF, for example where it is capable of binding EGFR and IGF-1 R, or where it is capable of binding EGFR and VEGF, or where it is capable of binding VEGF and IGF-1 R, or where it is capable of binding EGFR and VEGFR2, or where it is capable of binding IGF-1R and VEGFR2, or where it is capable of binding VEGF and VEGFR2, or where it is capable of binding EGFR, IGF-1 R and VEGFR2, or where it is capable of binding VEGF, IGF-1 R and VEGFR2, or where it is capable of binding EGFR, VEGF and VEGFR2, or where it is capable of binding EGFR, VEGF and IGF1R. Examples of such antigen-binding constructs include EGFR antibodies which have an epitope binding domain with a specificity for VEGFR2, for example an anti-VEGFR2 adnectin, attached to the c-terminus or the n-terminus of the heavy chain or the c-terminus or n-terminus of the light chain, for example the mAbdAb having the heavy chain sequence set out in SEQ ID NO: 136, 140 or 144 and/or the light chain sequence set out in SEQ ID NO: 138, 142 or 145.

Examples of such antigen-binding constructs include EGFR antibodies which have an epitope binding domain with a specificity for VEGF, for example an anti-VEGF dAb, attached to the c-terminus or the n-terminus of the heavy chain or the c-terminus or n-terminus of the light chain, for example the mAbdAb having the heavy chain sequence set out in SEQ ID NO: 165, 174, 176, 178, 184 or 186 and/or the light chain sequence set out in SEQ ID NO: 188 or 190.

Examples of such antigen-binding constructs include VEGF antibodies which have an epitope binding domain with a specificity for EGFR, for example an anti-EGFR dAb, attached to the c-terminus or the n-terminus of the heavy chain or the c-terminus or n-terminus of the light chain, for example the mAbdAb having the heavy chain sequence set out in SEQ ID NO: 180. Such mAbdAbs may also comprise the light chain sequence set out in SEQ ID NO: 182.

Examples of such antigen-binding constructs include IGF-1R antibodies which have an epitope binding domain with a specificity for VEGF, for example an anti-VEGF lipocalin, attached to the c-terminus or the n-terminus of the heavy chain or the c-terminus or n-terminus of the light chain, for example the mAbdAb having the heavy chain sequence set out in SEQ ID NO: 123 or 125. Such mAbdAbs may also comprise the light chain sequence set out in SEQ ID NO:113

Examples of such antigen-binding constructs include IGF-1 R antibodies which have an epitope binding domain with a specificity for VEGFR2, for example an anti-VEGFR2 adnectin, attached to the c-terminus or the n-terminus of the heavy chain or the c-terminus or n-terminus of the light chain, for example the mAbdAb having the heavy chain sequence set out in SEQ ID NO: 124 or 133. Such mAbdAbs may also comprise the light chain sequence set out in SEQ ID NO: 113.

The antigen binding constructs of the invention include those which have specificity for IL-23, for example which comprises an epitope-binding domain which is capable of binding to IL-23, or which comprises a paired VH/VL which binds to IL-23.

The antigen binding construct may comprise an antibody which is capable of binding to IL-23. The antigen binding construct may comprise a dAb which is capable of binding to IL-23.

In one embodiment the antigen-binding construct described here has specificity for more than one antigen, for example where it is capable of binding two or more antigens selected from TH17 type cytokines, for example. IL-17, IL-22, or IL-21, for example where it is capable of binding IL-23 and IL-17, or where it is capable of binding IL-23 and IL-21, or where it is capable of binding IL-23 and IL-22. Examples of such antigen-binding constructs include IL-23 antibodies which have an epitope binding domain with a specificity for IL-17, for example an anti-IL-17 dAb, attached to the c-terminus or the n-terminus of the heavy chain or the c-terminus or n-terminus of the light chain.

In one example of the present application there is provided an antigen binding construct according to the invention described herein and comprising a constant region such that the antibody has reduced ADCC and/or complement activation or effector functionality. In one such embodiment the heavy chain constant region may comprise a naturally disabled constant region of IgG2 or IgG4 isotype or a mutated IgG1 constant region. Examples of suitable modifications are described in EP0307434. One example comprises the substitutions of alanine residues at positions 235 and 237 (EU index numbering).

In one example the antigen-binding constructs of the present invention will retain Fc functionality for example will be capable of one or both of ADCC and CDC activity. Such antigen-binding constructs may comprise an epitope-binding domain located on the light chain, for example on the c-terminus of the light chain.

The application also provides a method of maintaining ADCC and CDC function of antigen-binding constructs by positioning of the epitope binding domain on the light chain of the antibody in particular, by positioning the epitope binding domain on the c-terminus of the light chain. Such ADCC and CDC function can be measured by any suitable assay, for example the ADCC assay set out in Example 15.3 and the CDC assay set out in Example 15.4.

The application also provides a method of reducing CDC function of antigen-binding constructs by positioning of the epitope binding domain on the heavy chain of the antibody, in particular, by positioning the epitope binding domain on the c-terminus of the heavy chain. Such CDC function can be measured by any suitable assay, for example the CDC assay set out in Example 15.4.

The antigen-binding constructs described here may be capable of binding two or more antigens selected from VEGF, IGF-1 R and EGFR, for example it is capable of binding EGFR and VEGF, or EGFR and IGF1 R, or IGF1 R and VEGF, or for example it is capable of binding to TNF and IL1-R. In embodiments of the invention which comprise an IGF-1 R binding site, the IGF-1 R binding site of the antigen-binding construct of the invention may comprise a paired VH/VL domain in the protein scaffold, which paired VH/VL domain may comprise one or more of the CDRs selected from those set out in SEQ ID NO: 80, SEQ ID NO: 81, SEQ ID NO: 82, SEQ ID NO: 83, SEQ ID NO: 84, SEQ ID NO: 85 and SEQ ID NO: 86, for example it may comprise at least CDRH3 as set out in SEQ ID NO:80, for example it may comprise all the CDRs set out in SEQ ID NO: 80, SEQ ID NO: 81, SEQ ID NO: 82, SEQ ID NO: 83, SEQ ID NO: 85, and SEQ ID NO: 86.

In antigen-binding constructs which comprise an EGFR binding site, the antigen-binding construct may bind to an epitope comprising residues 273-501 of the mature or normal or wild type EGFR sequence, for example it may bind to an epitope comprising residues 287-302 of the mature or normal or wildtype EGFR (SEQ ID NO:103).

In one example, the EGFR binding site of the antigen-binding construct of the invention may comprise a paired VH/VL domain in the protein scaffold, which paired VHNL domain may comprise one or more of the CDRs selected from those set out in SEQ ID NO: 104, SEQ ID NO: 105, SEQ ID NO: 106, SEQ ID NO: 100, SEQ ID NO: 101, and SEQ ID NO: 102, for example, it may comprise CDRH3 as set out in SEQ ID NO: 106, or it may comprise all six CDRs set out in SEQ ID NO: 104, SEQ ID NO: 105, SEQ ID NO: 106, SEQ ID NO: 100, SEQ ID NO: 101, and SEQ ID NO: 102. Such paired VH/VL domain may further comprise additional residues, particularly in the heavy chain CDRs, and in one example, CDRH1 may comprise SEQ ID NO: 104 plus up to five additional residues, for example one or more of the five additional residues which are set out in SEQ ID NO: 97, CDRH2 may comprise SEQ ID NO: 105 plus up to two additional residues, for example one or both of the two additional residues which are set out in SEQ ID NO: 98 and SEQ ID NO: 107, and CDRH3 may comprise SEQ ID NO: 106 plus up to two additional residues, for example one or both of the two additional residues which are set out in SEQ ID NO: 99. In one such example, the paired VHNL comprises one or more of the CDRs set out in SEQ ID NO: 97, SEQ ID NO: 98, SEQ ID NO: 99, SEQ ID NO: 100, SEQ ID NO: 101, and SEQ ID NO: 102, for example it may comprise at least CDRH3 as set out in SEQ ID NO:99, for example it may comprise all six CDRs set out in SEQ ID NO: 97, SEQ ID NO: 98, SEQ ID NO: 99, SEQ ID NO: 100, SEQ ID NO: 101 and SEQ ID NO: 102 (more detail of suitable antibodies can be found in WO02/092771 and WO2005/081854).

In one embodiment, the antigen binding constructs comprise an epitope-binding domain which is a domain antibody (dAb), for example the epitope binding domain may be a human VH or human VL, or a camelid $V_{HH}$ or a shark dAb (NARV).

In one embodiment the antigen binding constructs comprise an epitope-binding domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEI and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

The antigen binding constructs of the present invention may comprise a protein scaffold attached to an epitope binding domain which is an adnectin, for example an IgG scaffold with an adnectin attached to the c-terminus of the heavy chain, or it may comprise a protein scaffold attached to an adnectin, for example an IgG scaffold with an adnectin attached to the n-terminus of the heavy chain, or it may comprise a protein scaffold attached to an adnectin, for example an IgG scaffold with an adnectin attached to the c-terminus of the light chain, or it may comprise a protein scaffold attached to an adnectin, for example an IgG scaffold with an adnectin attached to the n-terminus of the light chain.

In other embodiments it may comprise a protein scaffold, for example an IgG scaffold, attached to an epitope binding domain which is CTLA-4, for example an IgG scaffold with CTLA-4 attached to the n-terminus of the heavy chain, or it may comprise for example an IgG scaffold with CTLA-4 attached to the c-terminus of the heavy chain, or it may comprise for example an IgG scaffold with CTLA-4 attached to the n-terminus of the light chain, or it may comprise an IgG scaffold with CTLA-4 attached to the c-terminus of the light chain.

In other embodiments it may comprise a protein scaffold, for example an IgG scaffold, attached to an epitope binding domain which is a lipocalin, for example an IgG scaffold with a lipocalin attached to the n-terminus of the heavy chain, or it may comprise for example an IgG scaffold with a lipocalin attached to the c-terminus of the heavy chain, or it may comprise for example an IgG scaffold with a lipocalin attached to the n-terminus of the light chain, or it may comprise an IgG scaffold with a lipocalin attached to the c-terminus of the light chain.

In other embodiments it may comprise a protein scaffold, for example an IgG scaffold, attached to an epitope binding domain which is an SpA, for example an IgG scaffold with an SpA attached to the n-terminus of the heavy chain, or it may comprise for example an IgG scaffold with an SpA attached to the c-terminus of the heavy chain, or it may comprise for example an IgG scaffold with an SpA attached to the n-terminus of the light chain, or it may comprise an IgG scaffold with an SpA attached to the c-terminus of the light chain.

In other embodiments it may comprise a protein scaffold, for example an IgG scaffold, attached to an epitope binding domain which is an affibody, for example an IgG scaffold with an affibody attached to the n-terminus of the heavy chain, or it may comprise for example an IgG scaffold with an affibody attached to the c-terminus of the heavy chain, or it may comprise for example an IgG scaffold with an affibody attached to the n-terminus of the light chain, or it may comprise an IgG scaffold with an affibody attached to the c-terminus of the light chain.

In other embodiments it may comprise a protein scaffold, for example an IgG scaffold, attached to an epitope binding domain which is an affimer, for example an IgG scaffold with an affimer attached to the n-terminus of the heavy chain, or it may comprise for example an IgG scaffold with an affimer attached to the c-terminus of the heavy chain, or it may comprise for example an IgG scaffold with an affimer attached to the n-terminus of the light chain, or it may comprise an IgG scaffold with an affimer attached to the c-terminus of the light chain.

In other embodiments it may comprise a protein scaffold, for example an IgG scaffold, attached to an epitope binding domain which is a GroEI, for example an IgG scaffold with a GroEI attached to the n-terminus of the heavy chain, or it may comprise for example an IgG scaffold with a GroEI attached to the c-terminus of the heavy chain, or it may comprise for example an IgG scaffold with a GroEI attached to the n-terminus of the light chain, or it may comprise an IgG scaffold with a GroEI attached to the c-terminus of the light chain.

In other embodiments it may comprise a protein scaffold, for example an IgG scaffold, attached to an epitope binding domain which is a transferrin, for example an IgG scaffold with a transferrin attached to the n-terminus of the heavy chain, or it may comprise for example an IgG scaffold with a transferrin attached to the c-terminus of the heavy chain, or it may comprise for example an IgG scaffold with a transferrin attached to the n-terminus of the light chain, or it may comprise an IgG scaffold with a transferrin attached to the c-terminus of the light chain.

In other embodiments it may comprise a protein scaffold, for example an IgG scaffold, attached to an epitope binding domain which is a GroES, for example an IgG scaffold with a GroES attached to the n-terminus of the heavy chain, or it may comprise for example an IgG scaffold with a GroES attached to the c-terminus of the heavy chain, or it may comprise for example an IgG scaffold with a GroES attached to the n-terminus of the light chain, or it may comprise an IgG scaffold with a GroES attached to the c-terminus of the light chain.

In other embodiments it may comprise a protein scaffold, for example an IgG scaffold, attached to an epitope binding domain which is a DARPin, for example an IgG scaffold with a DARPin attached to the n-terminus of the heavy chain, or it may comprise for example an IgG scaffold with a DARPin attached to the c-terminus of the heavy chain, or it may comprise for example an IgG scaffold with a DARPin attached to the n-terminus of the light chain, or it may comprise an IgG scaffold with a DARPin attached to the c-terminus of the light chain.

In other embodiments it may comprise a protein scaffold, for example an IgG scaffold, attached to an epitope binding domain which is a peptide aptamer, for example an IgG scaffold with a peptide aptamer attached to the n-terminus of the heavy chain, or it may comprise for example an IgG scaffold with a peptide aptamer attached to the c-terminus of the heavy chain, or it may comprise for example an IgG scaffold with a peptide aptamer attached to the n-terminus of the light chain, or it may comprise an IgG scaffold with a peptide aptamer attached to the c-terminus of the light chain.

In the present invention there are four epitope binding domains, for example four domain antibodies, two of the epitope binding domains may have specificity for the same antigen, or all of the epitope binding domains present in the antigen-binding construct may have specificity for the same antigen.

Protein scaffolds of the present invention may be linked to epitope-binding domains by the use of linkers. Examples of suitable linkers include amino acid sequences which may be from 1 amino acid to 150 amino acids in length, or from 1 amino acid to 140 amino acids, for example, from 1 amino acid to 130 amino acids, or from 1 to 120 amino acids, or from 1 to 80 amino acids, or from 1 to 50 amino acids, or from 1 to 20 amino acids, or from 1 to 10 amino acids, or from 5 to 18 amino acids. Such sequences may have their own tertiary structure, for example, a linker of the present invention may comprise a single variable domain. The size of a linker in one embodiment is equivalent to a single variable domain. Suitable linkers may be of a size from 1 to 20 angstroms, for example less than 15 angstroms, or less than 10 angstroms, or less than 5 angstroms.

In one embodiment of the present invention at least one of the epitope binding domains is directly attached to the Ig scaffold with a linker comprising from 1 to 50 amino acids, for example 1 to 20 amino acids, for example 1 to 10 amino acids. Such linkers may be selected from any one of those set out in SEQ ID NO: 6 to 11, 'STG' (serine, threonine, glycine), 'GSTG' or 'RS', for example the linker may be 'TVAAPS', or the linker may be 'GGGGS'. Linkers of use in the antigen binding constructs of the present invention may comprise alone or in addition to other linkers, one or more sets of GS residues, for example 'GSTVAAPS' or 'TVAAPSGS' or 'GSTVAAPSGS'. In another embodiment the epitope binding domain, for example a dAb, is linked to the Ig scaffold by the linker 'TVAAPS'. In another embodiment the epitope binding domain, for example a dAb, is linked to the Ig scaffold by the linker 'TVAAPSGS'. In another embodiment the epitope binding domain, for example a dAb, is linked to the Ig scaffold by the linker 'GS'.

The antigen-binding construct of the present invention may comprise at least one epitope binding domain which is capable of binding human serum albumin.

In one embodiment, there are 4 antigen binding sites and the antigen binding construct is capable of binding 3 or 4 antigens simultaneously.

The invention also provides the antigen-binding constructs for use in medicine, for example for use in the manufacture of a medicament for treating cancer or inflammatory diseases such as asthma, rheumatoid arthritis, or osteoarthritis.

The invention provides a method of treating a patient.suffering from cancer or inflammatory diseases such as asthma, rheumatoid arthritis, or osteoarthritis, comprising administering a therapeutic amount of an antigen-binding construct of the invention.

The antigen-binding constructs of the invention may be used for the treatment of cancer or inflammatory diseases such as asthma, rheumatoid arthritis, or osteoarthritis.

The antigen-binding constructs of the invention may have some effector function. For example if the protein scaffold contains an Fc region derived from an antibody with effector function, for example if the protein scaffold comprises CH2 and CH3 from IgG1. Levels of effector function can be varied according to known techniques, for example by mutations in the CH2 domain, for example wherein the IgG1 CH2 domain has one or more mutations at positions selected from 239 and 332 and 330, for example the mutations are selected from S239D and I332E and A330L such that the antibody has enhanced effector function, and/or for example altering the glycosylation profile of the antigen-binding construct of the invention such that there is a reduction in fucosylation of the Fc region.

Protein scaffolds of use in the present invention include full monoclonal antibody scaffolds comprising all the domains of an antibody, or protein scaffolds of the present invention may comprise a non-conventional antibody structure, such as a monovalent antibody. Such monovalent antibodies may comprise a paired heavy and light chain wherein the hinge region of the heavy chain is modified so that the heavy chain does not homodimerise, such as the monovalent antibody described in WO2007059782. Other monovalent antibodies may comprise a paired heavy and light chain which dimerises with a second heavy chain which is lacking a functional variable region and CH1 region, wherein the first and second heavy chains are modified so that they will form heterodimers rather than homodimers, resulting in a monovalent antibody with two heavy chains and one light chain such as the monovalent antibody described in WO2006015371. Such monovalent antibodies can provide the protein scaffold of the present invention to which epitope binding domains can be linked, for example such as the antigen binding constructs describe in Example 32.

Epitope-binding domains of use in the present invention are domains that specifically bind an antigen or epitope independently of a different V region or domain, this may be a domain antibody or may be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human $\gamma$-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand. In one embodiment this may be an domain antibody or other suitable domains such as a domain selected from the group consisting of CTLA-4, lipocallin, SpA, an Affibody, an avimer, GroEI, transferrin, GroES and fibronectin. In one embodiment this may be selected from a dAb, an Affibody, an ankyrin repeat protein (DARPin) and an adnectin. In another embodiment this may be selected from an Affibody, an ankyrin repeat protein (DARPin) and an adnectin. In another embodiment this may be a domain antibody, for example a domain antibody selected from a human, camelid or shark (NARV) domain antibody.

Epitope-binding domains can be linked to the protein scaffold at one or more positions. These positions include the C-terminus and the N-terminus of the protein scaffold, for example at the C-terminus of the heavy chain and/or the C-terminus of the light chain of an IgG, or for example the N-terminus of the heavy chain and/or the N-terminus of the light chain of an IgG.

In one embodiment, a first epitope binding domain is linked to the protein scaffold and a second epitope binding domain is linked to the first epitope binding domain, for example where the protein scaffold is an IgG scaffold, a first epitope binding domain may be linked to the c-terminus of the heavy chain of the IgG scaffold, and that epitope binding domain can be linked at its c-terminus to a second epitope binding domain, or for example a first epitope binding domain may be linked to the c-terminus of the light chain of the IgG scaffold, and that first epitope binding domain may be further linked at its c-terminus to a second epitope binding domain., Examples of such antigen binding constructs are described in Example 31.

When the epitope-binding domain is a domain antibody, some domain antibodies may be suited to particular positions within the scaffold.

Domain antibodies of use in the present invention can be linked at the C-terminal end of the heavy chain and/or the light chain of conventional IgGs. In addition some dAbs can be linked to the C-terminal ends of both the heavy chain and the light chain of conventional antibodies.

In constructs where the N-terminus of dAbs are fused to an antibody constant domain (either $C_H3$ or CL), a peptide linker may help the dAb to bind to antigen. Indeed, the N-terminal end of a dAb is located closely to the complementarity-determining regions (CDRS) involved in antigen-binding activity. Thus a short peptide linker acts as a spacer between the epitope-binding, and the constant domain fo the protein scaffold, which may allow the dAb CDRs to more easily reach the antigen, which may therefore bind with high affinity.

The surroundings in which dAbs are linked to the IgG will differ depending on which antibody chain they are fused to:

When fused at the C-terminal end of the antibody light chain of an IgG scaffold, each dAb is expected to be located in the vicinity of the antibody hinge and the Fc portion. It is likely that such dAbs will be located far apart from each other. In conventional antibodies, the angle between Fab fragments and the angle between each Fab fragment and the Fc portion can vary quite significantly. It is likely that - with mAbdAbs - the angle between the Fab fragments will not be widely different, whilst some angular restrictions may be observed with the angle between each Fab fragment and the Fc portion.

When fused at the C-terminal end of the antibody heavy chain of an IgG scaffold, each dAb is expected to be located in the vicinity of the $C_H3$ domains of the $F_C$ portion. This is not expected to impact on the Fc binding properties to $F_c$ receptors (e.g. FcγRI, II, III an FcRn) as these receptors engage with the $C_H2$ domains (for the FcγRI, II and III class of receptors) or with the hinge between the $C_H2$ and $C_H3$ domains (e.g. FcRn receptor). Another feature of such antigen-binding constructs is that both dAbs are expected to be spatially close to each other and provided that flexibility is provided by provision of appropriate linkers, these dAbs may even form homodimeric species, hence propagating the 'zipped' quaternary structure of the Fc portion, which may enhance stability of the construct.

Such structural considerations can aid in the choice of the most suitable position to link an epitope-binding domain, for example a dAb, on to a protein scaffold, for example an antibody.

The size of the antigen, its localization (in blood or on cell surface), its quaternary structure (monomeric or multimeric) can vary. Conventional antibodies are naturally designed to function as adaptor constructs due to the presence of the hinge region, wherein the orientation of the two antigen-binding sites at the tip of the Fab fragments can vary widely and hence adapt to the molecular feature of the antigen and its surroundings. In contrast dAbs linked to an antibody or other protein scaffold, for example a protein scaffold which comprises an antibody with no hinge region, may have less structural flexibility either directly or indirectly.

Understanding the solution state and mode of binding at the dAb is also helpful. Evidence has accumulated that *in vitro* dAbs can predominantly exist in monomeric, homo-dimeric or multimeric forms in solution (Reiter et al. (1999) J Mol Biol 290 p685-698; Ewert et al (2003) J Mol Biol 325, p531-553, Jespers et al (2004) J Mol Biol 337 p893-903; Jespers et al (2004) Nat Biotechnol 22 p1161-1165; Martin et al (1997) Protein Eng. 10 p607-614; Sepulvada et al (2003) J Mol Biol 333 p355-365).

This is fairly reminiscent to multimerisation events observed *in vivo* with Ig domains such as Bence-Jones proteins (which are dimers of immunoglobulin light chains (Epp et al (1975) Biochemistry 14 p4943-4952; Huan et al (1994) Biochemistry 33 p14848-14857; Huang et al (1997) Mol immunol 34 p1291-1301) and amyloid fibers (James et al. (2007) J Mol Biol. 367:603-8).

For example, it may be desirable to link domain antibodies that tend to dimerise in solution to the C-terminal end of the Fc portion in preference to the C-terminal end of the light chain as linking to the C-terminal end of the Fc will allow those dAbs to dimerise in the context of the antigen-binding construct of the invention.

The antigen-binding constructs of the present invention may comprise antigen-binding sites specific for a single antigen, or may have antigen-binding sites specific for two or more antigens, or for two or more epitopes on a single antigen, or there may be antigen-binding sites each of which is specific for a different epitope on the same or different antigens.

The antigen-binding sites can each have binding specificity for an antigen, such as human or animal proteins, including cytokines, growth factors, cytokine receptors, growth factor receptors, enzymes (*e.g.*, proteases), co-factors for enzymes, DNA binding proteins, lipids and carbohydrates. Suitable targets, including cytokines, growth factors, cytokine receptors, growth factor receptors and other proteins include but are not limited to: ApoE, Apo-SAA, BDNF, Cardiotrophin-1, CEA, CD40, CD40 Ligand, CD56, CD38, CD138, EGF, EGF receptor, ENA-78, Eotaxin, Eotaxin-2, Exodus-2, FAPα, FGF-acidic, FGF-basic, fibroblast growth factor-10, FLT3 ligand, Fractalkine (CX3C), GDNF, G-CSF, GM-CSF, GF-β1, human serum albumin, insulin, IFN-γ, IGF-I, IGF-II, IL-1α, IL-1β, IL-1 receptor, IL-1 receptor type 1, IL-2, IL-3, IL-4, IL-5, IL-6, IL-7, IL-8 (72 a.a.), IL-8 (77 a.a.), IL-9, IL-10, IL-11; IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, c-fms, v-fmsMDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDS1β, SCF, SCGF, stem cell factor (SCF), TARC, TGF-α, TNF-β, TGF-β2, TGF-β3, tumour necrosis factor (TNF), TNF-α, TNF-β, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-β, GRO-γ, HCC1, 1-309, HER 1, HER 2, HER 3, HER 4, serum albumin, vWF, amyloid proteins (*e.g.*, amyloid alpha), MMP12, PDK1, IgE, and other targets disclosed herein. It will be appreciated that this list is by no means exhaustive.

In some embodiments, the protease resistant peptide or polypeptide binds a target in pulmonary tissue, such as a target selected from the group consisting of TNFR1, IL-1, IL-1R, IL-4, IL-4R, IL-5, IL-6, IL-6R, IL-8, IL-8R, IL-9, IL-9R, IL-10, IL-12 IL-12R, IL-13, IL-13Rα1, IL-13Rα2, IL-15, IL-15R, IL-16, IL-17R, IL-17, IL-18, IL-18R, IL-23 IL-23R, IL-25, CD2, CD4, CD11a, CD23, CD25, CD27, CD28, CD30, CD40, CD40L, CD56, CD138, ALK5, EGFR, FcER1, TGFb, CCL2, CCL18, CEA, CR8, CTGF, CXCL12 (SDF-1), chymase, FGF, Furin, Endothelin-1, Eotaxins (*e.g.*, Eotaxin, Eotaxin-2, Eotaxin-3), GM-CSF, ICAM-1, ICOS, IgE, IFNα, I-309, integrins, L-selectin, MIF, MIP4, MDC, MCP-1, MMPs, neutrophil elastase, osteopontin, OX-40, PARC, PD-1, RANTES, SCF, SDF-1, siglec8, TARC, TGFb, Thrombin, Tim-1, TNF, TRANCE, Tryptase, VEGF, VLA-4, VCAM, α4β7, CCR2, CCR3, CCR4, CCR5, CCR7, CCR8, alphavbeta6, alphavbeta8, cMET, CD8, vWF, amyloid proteins (*e.g.*, amyloid alpha), MMP12, PDK1, and IgE.

In particular, the antigen-binding constructs of the present invention may be useful in treating diseases associated with IL-13, IL-5 and IL-4, for example atopic dermatitis, allergic rhinitis, crohn's disease, COPD, fibrotic diseases or disorders such as idiopathic pulmonary fibrosis, progressive systemic sclerosis, hepatic fibrosis, hepatic granulomas, schistosomiasis, leishmaniasis, diseases of cell cycle regulation such as Hodgkins disease, B cell chronic lymphocytic leukaemia, for example the constructs may be useful in treating asthma.

Antigen-binding constructs of the present invention may be useful in treating diseases associated with growth factors such as IGF-1 R, VEGF, and EGFR, for example cancer or rheumatoid arthritis, examples of types of cancer in which such therapies may be useful are breast cancer, prostrate cancer, lung cancer and myeloma.

Antigen-binding constructs of the present invention may be useful in treating diseases associated with TNF, for example arthritis, for example rheumatoid arthritis or osteoarthritis.

Antigen-binding constructs of the present invention may be useful in treating diseases associated with IL1-R, for example arthritis, for example rheumatoid arthritis or osteoarthritis.

Antigen-binding constructs of the present invention may be useful in treating diseases associated with CD-20, for example autoimmune diseases such as psoriasis, inflammatory bowel disease, ulcerative colitis, crohns disease, rheumatoid arthritis, juvenile rheumatoid arthritis, systemic lupus erythematosus, neurodegenerative diseases, for example multiple sclerosis, neutrophil driven diseases, for example COPD, Wegeners vasculitis, cystic fibrosis, Sjogrens syndrome, chronic transplant rejection, type 1 diabetes graft versus host disease, asthma, allergic diseases atoptic dermatitis, eczematous dermatitis, allergic rhinitis, autoimmune diseases other including thyroiditis, spondyloarthropathy, ankylosing spondylitis, uveitis, polychonritis or scleroderma, or cancer e.g. B- cell lymphomas or mature B cell neoplasm such as CLL or SLL.

Antigen-binding constructs of the present invention may be useful in treating diseases associated with IL-17 and IL-23, for example psoriasis, inflammatory bowel disease, ulcerative colitis, crohns disease, rheumatoid arthritis, juvenile rheumatoid arthritis, systemic lupus erythematosus, neurodegenerative diseases, for example multiple sclerosis, neutrophil driven diseases, for example COPD , Wegeners vasculitis, cystic fibrosis, Sjogrens syndrome, chronic transplant rejection, type 1 diabetes graft versus host disease, asthma, allergic diseases atoptic dermatitis, eczematous dermatitis, allergic rhinitis, autoimmune diseases other including thyroiditis, spondyloarthropathy, ankylosing spondylitis, uveitis, polychonritis or scleroderma.

The antigen binding constructs of the present invention may be produced by transfection of a host cell with an expression vector comprising the coding sequence for the antigen binding construct of the invention. An expression vector or recombinant plasmid is produced by placing these coding sequences for the antigen binding construct in operative association with conventional regulatory control sequences capable of controlling the replication and expression in, and/or secretion from, a host cell. Regulatory sequences include promoter sequences, e.g., CMV promoter, and signal sequences which can be derived from other known antibodies. Similarly, a second expression vector can be produced having a DNA sequence which encodes a complementary antigen binding construct light or heavy chain. In certain embodiments this second expression vector is identical to the first except insofar as the coding sequences and selectable markers are concerned, so to ensure as far as possible that each polypeptide chain is functionally expressed. Alternatively, the heavy and light chain coding sequences for the antigen binding construct may reside on a single vector, for example in two expression cassettes in the same vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antigen binding construct of the invention. The antigen binding construct which includes the association of both the recombinant heavy chain and/or light chain is screened from culture by appropriate assay, such as ELISA or RIA. Similar conventional techniques may be employed to construct other antigen binding constructs.

Suitable vectors for the cloning and subcloning steps employed in the methods and construction of the compositions of this invention may be selected by one of skill in the art. For example, the conventional pUC series of cloning vectors may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning. Thus, the selection of the cloning vector is not a limiting factor in this invention.

The expression vectors may also be characterized by genes suitable for amplifying expression of the heterologous DNA sequences, e.g., the mammalian dihydrofolate reductase gene (DHFR). Other preferable vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

The present invention also encompasses a cell line transfected with a recombinant plasmid containing the coding sequences of the antigen binding constructs of the present invention. Host cells useful for the cloning and other manipulations of these cloning vectors are also conventional. However, cells from various strains of E. coli may be used for replication of the cloning vectors and other steps in the construction of antigen binding constructs of this invention.

Suitable host cells or cell lines for the expression of the antigen binding constructs of the invention include mammalian cells such as NS0, Sp2/0, CHO (e.g. DG44), COS, HEK, a fibroblast cell (e.g., 3T3), and myeloma cells, for example it may be expressed in a CHO or a myeloma cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., Sambrook et al., cited above.

Bacterial cells may prove useful as host cells suitable for the expression of the recombinant Fabs or other embodiments of the present invention (see, e.g., Plückthun, A., Immunol. Rev., 130:151-188 (1992)). However, due to the tendency of proteins expressed in bacterial cells to be in an unfolded or improperly folded form or in a non-glycosylated form, any recombinant Fab produced in a bacterial cell would have to be screened for retention of antigen binding ability. If the molecule expressed by the bacterial cell was produced in a properly folded form, that bacterial cell would be a desirable host, or in alternative embodiments the molecule may express in the bacterial host and then be subsequently re-folded. For example, various strains of E. coli used for expression are well-known as host cells in the field of biotechnology. Various strains of B. subtilis, Streptomyces, other bacilli may also be employed in this method.

Where desired, strains of yeast cells known to those skilled in the art are also available as host cells, as well as insect cells, e.g. Drosophila and Lepidoptera and viral expression systems. See, e.g. Miller et al., Genetic Engineering, 8:277-298, Plenum Press (1986) and references cited therein.

The general methods by which the vectors may be constructed, the transfection methods required to produce the host cells of the invention, and culture methods necessary to produce the antigen binding construct of the invention from such host cell may all be conventional techniques. Typically, the culture method of the present invention is a serum-free culture method, usually by culturing cells serum-free in suspension. Likewise, once produced, the antigen binding constructs of the invention may be purified from the cell culture contents according to standard procedures of the art, including ammonium sulfate precipitation, affinity columns, column chromatography and gel electrophoresis. Such techniques are within the skill of the art and do not limit this invention. For example, preparation of altered antibodies are described in WO 99/58679 and WO 96/16990.

Yet another method of expression of the antigen binding constructs may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

In a further aspect of the invention there is provided a method of producing an antibody of the invention which method comprises the step of culturing a host cell transformed or transfected with a vector encoding the light and/or heavy chain of the antibody of the invention and recovering the antibody thereby produced.

In accordance with the present invention there is provided a method of producing an antigen binding construct of the present invention which method comprises the steps of;

(a) providing a first vector encoding a heavy chain of the antigen binding construct;
(b) providing a second vector encoding a light chain of the antigen binding construct;
(c) transforming a mammalian host cell (e.g. CHO) with said first and second vectors;
(d) culturing the host cell of step (c) under conditions conducive to the secretion of the antigen binding construct from said host cell into said culture media;
(e) recovering the secreted antigen binding construct of step (d).

Once expressed by the desired method, the antigen binding construct is then examined for in vitro activity by use of an appropriate assay. Presently conventional ELISA assay formats are employed to assess qualitative and quantitative binding of the antigen binding construct to its target. Additionally, other in vitro assays may also be used to verify neutralizing efficacy prior to subsequent human clinical studies performed to evaluate the persistence of the antigen binding construct in the body despite the usual clearance mechanisms.

The dose and duration of treatment relates to the relative duration of the molecules of the present invention in the human circulation, and can be adjusted by one of skill in the art depending upon the condition being treated and the general health of the patient. It is envisaged that repeated dosing (e.g. once a week or once every two weeks) over an extended time period (e.g. four to six months) maybe required to achieve maximal therapeutic efficacy.

The mode of administration of the therapeutic agent of the invention may be any suitable route which delivers the agent to the host. The antigen binding constructs, and pharmaceutical compositions of the invention are particularly useful for parenteral administration, i.e., subcutaneously (s.c.), intrathecally, intraperitoneally, intramuscularly (i.m.), intravenously (i.v.), or intranasally.

Therapeutic agents of the invention may be prepared as pharmaceutical compositions containing an effective amount of the antigen binding construct of the invention as an active ingredient in a pharmaceutically acceptable carrier. In the prophylactic agent of the invention, an aqueous suspension or solution containing the antigen binding construct, preferably buffered at physiological pH, in a form ready for injection is preferred. The compositions for parenteral administration will commonly comprise a solution of the antigen binding construct of the invention or a cocktail thereof dissolved in a pharmaceutically acceptable carrier, preferably an aqueous carrier. A variety of aqueous carriers may be employed, e.g., 0.9% saline, 0.3% glycine. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen binding construct of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 mL sterile buffered water, and between about 1 ng to about 100 mg, e.g. about 50 ng to about 30 mg or more preferably, about 5 mg to about 25 mg, of an antigen binding construct of the invention. Similarly, a pharmaceutical composition of the invention for intravenous infusion could be made up to contain about 250 ml of sterile Ringer's solution, and about 1 to about 30 and preferably 5 mg to about 25 mg of an antigen binding construct of the invention per ml of Ringer's solution. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antigen binding construct formulations of the invention see Lasmar U and Parkins D "The formulation of Biopharmaceutical products", Pharma. Sci.Tech.today, page 129-137, Vol.3 (3rd April 2000), Wang, W "Instability, stabilisation and formulation of liquid protein pharmaceuticals", Int. J. Pharm 185 (1999) 129-188, Stability of Protein Pharmaceuticals Part A and B ed Ahern T.J., Manning M.C., New York, NY: Plenum Press (1992), Akers,M.J. "Excipient-Drug interactions in Parenteral Formulations", J.Pharm Sci 91 (2002) 2283-2300, Imamura, K et al "Effects of types of sugar on stabilization of Protein in the dried state", J Pharm Sci 92 (2003) 266-274,Izutsu, Kkojima, S. "Excipient crystalinity and its protein-structure-stabilizing effect during freeze-drying", J Pharm. Pharmacol, 54 (2002) 1033-1039, Johnson, R, "Mannitol-sucrose mixtures-versatile formulations for protein lyophilization", J. Pharm. Sci, 91 (2002) 914-922.
Ha,E Wang W, Wang Y.j. "Peroxide formation in polysorbate 80 and protein stability", J. Pharm Sci, 91, 2252-2264,(2002).
It is preferred that the therapeutic agent of the invention, when in a pharmaceutical preparation, be present in unit dose forms. The appropriate therapeutically effective dose will be determined readily by those of skill in the art. Suitable doses may be calculated for patients according to their weight, for example suitable doses may be in the range of 0.01 to 20mg/kg, for example 0.1 to 20mg/kg, for example 1 to 20mg/kg, for example 10 to 20mg/kg or for example 1 to 15mg/kg, for example 10 to 15mg/kg. To effectively treat conditions of use in the present invention in a human, suitable doses may be within the range of 0.01 to 1000 mg, for example 0.1 to 1000mg, for example 0.1 to 500mg, for example 500mg, for example 0.1 to 100mg, or 0.1 to 80mg, or 0.1 to 60mg, or 0.1 to 40mg, or for example 1 to 100mg, or 1 to 50mg, of an antigen binding construct of this invention, which may be administered parenterally, for example subcutaneously, intravenously or intramuscularly. Such dose may, if necessary, be repeated at appropriate time intervals selected as appropriate by a physician.
The antigen binding constructs described herein can be lyophilized for storage and reconstituted in a suitable carrier prior to use. This technique has been shown to be effective with conventional immunoglobulins and art-known lyophilization and reconstitution techniques can be employed.

There are several methods known in the art which can be used to find epitope-binding domains of use in the present invention.

The term "library" refers to a mixture of heterogeneous polypeptides or nucleic acids. The library is composed of members, each of which has a single polypeptide or nucleic acid sequence. To this extent, "library" is synonymous with "repertoire." Sequence differences between library members are responsible for the diversity present in the library. The library may take the form of a simple mixture of polypeptides or nucleic acids, or may be in the form of organisms or cells, for example bacteria, viruses, animal or plant cells, transformed with a library of nucleic acids. In one example, each individual organism or cell contains only one or a limited number of library members. Advantageously, the nucleic acids are incorporated into expression vectors, in order to allow expression of the polypeptides encoded by the nucleic acids. In a one aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of diverse polypeptides.
A "universal framework" is a single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. There may be a single framework, or a set of such frameworks, which has been found to permit the derivation of virtually any binding specificity though variation in the hypervariable regions alone.
Amino acid and nucleotide sequence alignments and homology, similarity or identity, as defined herein are in one embodiment prepared and determined using the algorithm BLAST 2 Sequences, using default parameters (Tatusova, T. A. et a/., FEMS Microbiol Lett, 174:187-188 (1999)).

The epitope binding domain(s) and antigen binding sites can each have binding specificity for a generic ligand or any desired target ligand, such as human or animal proteins, including cytokines, growth factors, cytokine receptors, growth factor receptors, enzymes (e.g., proteases), co-factors for enzymes, DNA binding proteins, lipids and carbohydrates. Suitable targets, including cytokines, growth factors, cytokine receptors, growth factor receptors and other proteins include but are not limited to: ApoE, Apo-SAA, BDNF, Cardiotrophin-1, CEA, CD40, CD40 Ligand, CD56, CD38, CD138, EGF, EGF receptor, ENA-78, Eotaxin, Eotaxin-2, Exodus-2, FAPα, FGF-acidic, FGF-basic, fibroblast growth factor-10, FLT3 ligand, Fractalkine (CX3C), GDNF, G-CSF, GM-CSF, GF-β1, human serum albumin, insulin, IFN-γ, IGF-I, IGF-II, IL-1α, IL-1β, IL-1 receptor, IL-1 receptor type 1, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein, M-CSF, c-fms, v-fmsMDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF A, VEGF B, VEGF C, VEGF D, VEGF receptor 1, VEGF receptor 2, VEGF receptor 3, GCP-2, GRO/MGSA, GRO-β, GRO-γ, HCC1, 1-309, HER 1, HER 2, HER 3, HER 4, serum albumin, vWF, amyloid proteins (*e.g.*, amyloid alpha), MMP12, PDK1, IgE, and other targets disclosed herein. It will be appreciated that this list is by no means exhaustive.

In some embodiments, binding is to a target in pulmonary tissue, such as a target selected from the group consisting of TNFR1, IL-1, IL-1R, IL-4, IL-4R, IL-5, IL-6, IL-6R, IL-8, IL-8R, IL-9, IL-9R, IL-10, IL-12 IL-12R, IL-13, IL-13Rα1, IL-13Ra2, IL-15, IL-15R, IL-16, IL-17R, IL-17, IL-18, IL-18R, IL-23 IL-23R, IL-25, CD2, CD4, CD11a, CD23, CD25, CD27, CD28, CD30, CD40, CD40L, CD56, CD138, ALK5, EGFR, FcER1, TGFb, CCL2, CCL18, CEA, CR8, CTGF, CXCL12 (SDF-1), chymase, FGF, Furin, Endothelin-1, Eotaxins (*e.g.*, Eotaxin, Eotaxin-2, Eotaxin-3), GM-CSF, ICAM-1, ICOS, IgE, IFNa, I-309, integrins, L-selectin, MIF, MIP4, MDC, MCP-1, MMPs, neutrophil elastase, osteopontin, OX-40, PARC, PD-1, RANTES, SCF, SDF-1, siglec8, TARC, TGFb, Thrombin, Tim-1, TNF, TRANCE, Tryptase, VEGF, VLA-4, VCAM, α4β7, CCR2, CCR3, CCR4, CCR5, CCR7, CCR8, alphavbeta6, alphavbeta8, cMET, CD8, vWF, amyloid proteins (*e.g.*, amyloid alpha), MMP12, PDK1, and IgE.

When a display system (*e.g.*, a display system that links coding function of a nucleic acid and functional characteristics of the peptide or polypeptide encoded by the nucleic acid) is used in the methods described herein, eg in the selection of a dAb or other epitope binding domain, it is frequently advantageous to amplify or increase the copy number of the nucleic acids that encode the selected peptides or polypeptides. This provides an efficient way of obtaining sufficient quantities of nucleic acids and/or peptides or polypeptides for additional rounds of selection, using the methods described herein or other suitable methods, or for preparing additional repertoires (*e.g.*, affinity maturation repertoires). Thus, in some embodiments, the methods of selecting epitope binding domains comprises using a display system (*e.g.,* that links coding function of a nucleic acid and functional characteristics of the peptide or polypeptide encoded by the nucleic acid, such as phage display) and further comprises amplifying or increasing the copy number of a nucleic acid that encodes a selected peptide or polypeptide. Nucleic acids can be amplified using any suitable methods, such as by phage amplification, cell growth or polymerase chain reaction.

In one example, the methods employ a display system that links the coding function of a nucleic acid and physical, chemical and/or functional characteristics of the polypeptide encoded by the nucleic acid. Such a display system can comprise a plurality of replicable genetic packages, such as bacteriophage or cells (bacteria). The display system may comprise a library, such as a bacteriophage display library. Bacteriophage display is an example of a display system. A number of suitable bacteriophage display systems (*e.g.,* monovalent display and multivalent display systems) have been described. (See, e.g., Griffiths et al., U.S. Patent No. 6,555,313 B1; Johnson et al., U.S. Patent No. 5,733,743; McCafferty et al., U.S. Patent No. 5,969,108; Mulligan-Kehoe, U.S. Patent No. 5,702,892; Winter, G. et al., Annu. Rev. Immunol. 12:433-455 (1994); Soumillion, P. et al., Appl. Biochem. Biotechnol. 47(2-3):175-189 (1994); Castagnoli, L. et al., Comb. Chem. High Throughput Screen, 4(2):121-133 (2001).) The peptides or polypeptides displayed in a bacteriophage display system can be displayed on any suitable bacteriophage, such as a filamentous phage (e.g., fd, M13, F1), a lytic phage (e.g., T4, T7, lambda), or an RNA phage (e.g., MS2), for example.

Generally, a library of phage that displays a repertoire of peptides or phagepolypeptides, as fusion proteins with a suitable phage coat protein (*e.g.,* fd pIII protein), is produced or provided. The fusion protein can display the peptides or polypeptides at the tip of the phage coat protein, or if desired at an internal position. For example, the displayed peptide or polypeptide can be present at a position that is amino-terminal to domain 1 of pIII. (Domain 1 of pIII is also referred to as N1.) The displayed polypeptide can be directly fused to pIII (*e.g.*, the N-terminus of domain 1 of pIII) or fused to pIII using a linker. If desired, the fusion can further comprise a tag (*e.g.*, myc epitope, His tag). Libraries that comprise a repertoire of peptides or polypeptides that are displayed as fusion proteins with a phage coat protein,can be produced using any suitable methods, such as by introducing a library of phage vectors or phagemid vectors encoding the displayed peptides or polypeptides into suitable host bacteria, and culturing the resulting bacteria to produce phage (*e.g.*, using a suitable helper phage or complementing plasmid if desired). The library of phage can be recovered from the culture using any suitable method, such as precipitation and centrifugation.

The display system can comprise a repertoire of peptides or polypeptides that contains any desired amount of diversity. For example, the repertoire can contain peptides or polypeptides that have amino acid sequences that correspond to naturally occurring polypeptides expressed by an organism, group of organisms, desired tissue or desired cell type, or can contain peptides or polypeptides that have random or randomized amino acid sequences. If desired, the polypeptides can share a common core or scaffold. For example, all polypeptides in the repertoire or library can be based on a scaffold selected from protein A, protein L, protein G, a fibronectin domain, an anticalin, CTLA4, a desired enzyme (*e.g.*, a polymerase, a cellulase), or a polypeptide from the immunoglobulin superfamily, such as an antibody or antibody fragment (*e.g.*, an antibody variable domain). The polypeptides in such a repertoire or library can comprise defined regions of random or randomized amino acid sequence and regions of common amino acid sequence. In certain examples, all or substantially all polypeptides in a repertoire are of a desired type, such as a desired enzyme (*e.g.*, a polymerase) or a desired antigen-binding fragment of an antibody (*e.g.*, human $V_H$ or human $V_L$). In some examples, the polypeptide display system comprises a repertoire of polypeptides wherein each polypeptide comprises an antibody variable domain. For example, each polypeptide in the repertoire can contain a $V_H$, a $V_L$ or an Fv (*e.g.*, a single chain Fv).
Amino acid sequence diversity can be introduced into any desired region of a peptide or polypeptide or scaffold using any suitable method. For example, amino acid sequence diversity can be introduced into a target region, such as a complementarity determining region of an antibody variable domain or a hydrophobic domain, by preparing a library of nucleic acids that encode the diversified polypeptides using any suitable mutagenesis methods (*e.g.*, low fidelity PCR, oligonucleotide-mediated or site directed mutagenesis, diversification using NNK codons) or any other suitable method. If desired, a region of a polypeptide to be diversified can be randomized. The size of the polypeptides that make up the repertoire is largely a matter of choice and uniform polypeptide size is not required. The polypeptides in the repertoire may have at least tertiary structure (form at least one domain).

Selection/Isolation/Recovery

An epitope binding domain or population of domains can be selected, isolated and/or recovered from a repertoire or library (*e.g.*, in a display system) using any suitable method. For example, a domain is selected or isolated based on a selectable characteristic (*e.g.*, physical characteristic, chemical characteristic, functional characteristic). Suitable selectable functional characteristics include biological activities of the peptides or polypeptides in the repertoire, for example, binding to a generic ligand (*e.g.*, a superantigen), binding to a target ligand (*e.g.*, an antigen, an epitope, a substrate), binding to an antibody (*e.g.*, through an epitope expressed on a peptide or polypeptide), and catalytic activity. (See, *e.g.*, Tomlinson et al., WO 99/20749; WO 01/57065; WO 99/58655.)
In some examples; the protease resistant peptide or polypeptide is selected and/or isolated from a library or repertoire of peptides or polypeptides in which substantially all domains share a common selectable feature. For example, the domain can be selected from a library or repertoire in which substantially all domains bind a common generic ligand, bind a common target ligand, bind (or are bound by) a common antibody, or possess a common catalytic activity. This type of selection is particularly useful for preparing a repertoire of domains that are based on a parental peptide or polypeptide that has a desired biological activity, for example, when performing affinity maturation of an immunoglobulin single variable domain.
Selection based on binding to a common generic ligand can yield a collection or population of domains that contain all or substantially all of the domains that were components of the original library or repertoire. For example, domains that bind a target ligand or a generic ligand, such as protein A, protein L or an antibody, can be selected, isolated and/or recovered by panning or using a suitable affinity matrix. Panning can be accomplished by adding a solution of ligand (*e.g.*, generic ligand, target ligand) to a suitable vessel (*e.g.*, tube, petri dish) and allowing the ligand to become deposited or coated onto the walls of the vessel. Excess ligand can be washed away and domains can be added to the vessel and the vessel maintained under conditions suitable for peptides or polypeptides to bind the immobilized ligand. Unbound domains can be washed away and bound domains can be recovered using any suitable method, such as scraping or lowering the pH, for example.
Suitable ligand affinity matrices generally contain a solid support or bead (*e.g.*, agarose) to which a ligand is covalently or noncovalently attached. The affinity matrix can be combined with peptides or polypeptides (*e.g.*, a repertoire that has been incubated with protease) using a batch process, a column process or any other suitable process under conditions suitable for binding of domains to the ligand on the matrix. domains that do not bind the affinity matrix can be washed away and bound domains can be eluted and recovered using any suitable method, such as elution with a lower pH buffer, with a mild denaturing agent (*e.g.,* urea), or with a peptide or domain that competes for binding to the ligand. In one example, a biotinylated target ligand is combined with a repertoire under conditions suitable for domains in the repertoire to bind the target ligand. Bound domains are recovered using immobilized avidin or streptavidin (*e.g.*, on a bead).
In some embodiments, the generic or target ligand is an antibody or antigen binding fragment thereof. Antibodies or antigen binding fragments that bind structural features of peptides or polypeptides that are substantially conserved in the peptides or polypeptides of a library or repertoire are particularly useful as generic ligands. Antibodies and antigen binding fragments suitable for use as ligands for isolating, selecting and/or recovering protease resistant peptides or polypeptides can be monoclonal or polyclonal and can be prepared using any suitable method.

LIBRARIES/REPERTOIRES

Libraries that encode and/or contain protease epitope binding domains can be prepared or obtained using any suitable method. A library can be designed to encode domains based on a domain or scaffold of interest (*e.g.*, a domain selected from a library) or can be selected from another library using the methods described herein. For example, a library enriched in domains can be prepared using a suitable polypeptide display system.
Libraries that encode a repertoire of a desired type of domain can readily be produced using any suitable method. For example, a nucleic acid sequence that encodes a desired type of polypeptide (*e.g.*, an immunoglobulin variable domain) can be obtained and a collection of nucleic acids that each contain one or more mutations can be prepared, for example by amplifying the nucleic acid using an error-prone polymerase chain reaction (PCR) system, by chemical mutagenesis (Deng et al., J. Biol. Chem., 269:9533 (1994)) or using bacterial mutator strains (Low et al., J. Mol. Biol., 260:359 (1996)).

Particular regions of the nucleic acid can be targeted for diversification. Methods for mutating selected positions are also well known in the art and include, for example, the use of mismatched oligonucleotides or degenerate oligonucleotides, with or without the use of PCR. For example, synthetic antibody libraries have been created by targeting mutations to the antigen binding loops. Random or semi-random antibody H3 and L3 regions have been appended to germline immunoblulin V gene segments to produce large libraries with unmutated framework regions (Hoogenboom and Winter (1992) supra; Nissim *et al.* (1994) supra; Griffiths *et al.* (1994) supra; DeKruif *et al.* (1995) supra). Such diversification has been extended to include some or all of the other antigen binding loops (Crameri et al. (1996) Nature Med., 2:100; Riechmann et al. (1995) Bio/Technology, 13:475; Morphosys, WO 97/08320, supra). In other examples, particular regions of the nucleic acid can be targeted for diversification by, for example, a two-step PCR strategy employing the product of the first PCR as a "mega-primer." (See, e.g., Landt, O. et al., Gene 96:125-128 (1990).) Targeted diversification can also be accomplished, for example, by SOE PCR. (See, *e.g.,* Horton, R.M. et al., Gene 77:61-68 (1989).)

Sequence diversity at selected positions can be achieved by altering the coding sequence which specifies the sequence of the polypeptide such that a number of possible amino acids (e.g., all 20 or a subset thereof) can be incorporated at that position. Using the IUPAC nomenclature, the most versatile codon is NNK, which encodes all amino acids as well as the TAG stop codon. The NNK codon may be used in order to introduce the required diversity. Other codons which achieve the same ends are also of use, including the NNN codon, which leads to the production of the additional stop codons TGA and TAA. Such a targeted approach can allow the full sequence space in a target area to be explored.

Some libraries comprise domains that are members of the immunoglobulin superfamily (e.g., antibodies or portions thereof). For example the libraries can comprise domains that have a known main-chain conformation. (See, *e.g.*, Tomlinson et al., WO 99/20749.) Libraries can be prepared in a suitable plasmid or vector. As used herein, vector refers to a discrete element that is used to introduce heterologous DNA into cells for the expression and/or replication thereof. Any suitable vector can be used, including plasmids (*e.g.*, bacterial plasmids), viral or bacteriophage vectors, artificial chromosomes and episomal vectors. Such vectors may be used for simple cloning and mutagenesis, or an expression vector can be used to drive expression of the library. Vectors and plasmids usually contain one or more cloning sites (*e.g.*, a polylinker), an origin of replication and at least one selectable marker gene. Expression vectors can further contain elements to drive transcription and translation of a polypeptide, such as an enhancer element, promoter, transcription termination signal and signal sequences. These elements can be arranged in such a way as to be operably linked to a cloned insert encoding a polypeptide, such that the polypeptide is expressed and produced when such an expression vector is maintained under conditions suitable for expression (e.g., in a suitable host cell).

Cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (*e.g.* SV40, adenovirus) are useful for cloning vectors in mammalian cells. Generally, the origin of replication is not needed for mammalian expression vectors, unless these are used in mammalian cells able to replicate high levels of DNA, such as COS cells.

Cloning or expression vectors can contain a selection gene also referred to as selectable marker. Such marker genes encode a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, *e.g.* ampicillin, neomycin, methotrexate or tetracycline, complement auxotrophic deficiencies, or supply critical nutrients not available in the growth media.

Suitable expression vectors can contain a number of components, for example, an origin of replication, a selectable marker gene, one or more expression control elements, such as a transcription control element (*e.g.*, promoter, enhancer, terminator) and/or one or more translation signals, a signal sequence or leader sequence, and the like. Expression control elements and a signal or leader sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding an antibody, antibody chain or portion thereof, such that it directs transcription of the nucleic acid. A variety of suitable promoters for procaryotic (*e.g.,* the β-lactamase and lactose promoter systems, alkaline phosphatase, the tryptophan (trp) promoter system, lac, tac, T3, T7 promoters for E. *coli*) and eucaryotic (*e.g.*, simian virus 40 early or late promoter, Rous sarcoma virus long terminal repeat promoter, cytomegalovirus promoter, adenovirus late promoter, EG-1a promoter) hosts are available.

In addition, expression vectors typically comprise a selectable marker for selection of host cells carrying the vector, and, in the case of a replicable expression vector, an origin of replication. Genes encoding products which confer antibiotic or drug resistance are common selectable markers and may be used in procaryotic (*e.g.*, β-lactamase gene (ampicillin resistance), *Tet* gene for tetracycline resistance) and eucaryotic cells (*e.g.*, neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host (*e.g.*, *LEU2, URA3, HIS3*) are often used as selectable markers in yeast. Use of viral (*e.g.*, baculovirus) or phage vectors, and vectors which are capable of integrating into the genome of the host cell, such as retroviral vectors, are also contemplated.

Suitable expression vectors for expression in prokaryotic (*e.g.,* bacterial cells such as *E. coli*) or mammalian cells include, for example, a pET vector (*e.g.,* pET-12a, pET-36, pET-37, pET-39, pET-40, Novagen and others), a phage vector (*e.g.*, pCANTAB 5 E, Pharmacia), pRIT2T (Protein A fusion vector, Pharmacia), pCDM8, pCDNA1.1/amp, pcDNA3.1, pRc/RSV, pEF-1 (Invitrogen, Carlsbad, CA), pCMV-SCRIPT, pFB, pSG5, pXT1 (Stratagene, La Jolla, CA), pCDEF3 (Goldman, L.A., et al., Biotechniques, 21:1013-1015 (1996)), pSVSPORT (GibcoBRL, Rockville, MD), pEF-Bos (Mizushima, S., et al., Nucleic Acids Res., 18:5322 (1990)). Expression vectors which are suitable for use in various expression hosts, such as prokaryotic cells (*E. coli*), insect cells (*Drosophila* Schnieder S2 cells, Sf9), yeast (P. *methanolica, P. pastoris, S. cerevisiae*) and mammalian cells (eg, COS cells) are available.

Some examples of vectors are expression vectors that enable the expression of a nucleotide sequence corresponding to a polypeptide library member. Thus, selection with generic and/or target ligands can be performed by separate propagation and expression of a single clone expressing the polypeptide library member. As described above, a particular selection display system is bacteriophage display. Thus, phage or phagemid vectors may be used, for example vectors may be phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector can contain a β-lactamase gene to confer selectivity on the phagemid and a lac promoter upstream of an expression cassette that can contain a suitable leader sequence, a multiple cloning site, one or more peptide tags, one or more TAG stop codons and the phage protein pIII. Thus, using various suppressor and non-suppressor strains of E. *coli* and with the addition of glucose, iso-propyl thio-β-D-galactoside (IPTG) or a helper phage, such as VCS M13, the vector is able to replicate as a plasmid with no expression, produce large quantities of the polypeptide library member only or product phage, some of which contain at least one copy of the polypeptide-pIII fusion on their surface.

Antibody variable domains may comprise a target ligand binding site and/or a generic ligand binding site. In certain examples, the generic ligand binding site is a binding site for a superantigen, such as protein A, protein L or protein G. The variable domains can be based on any desired variable domain, for example a human VH (*e.g.*, $V_H$ 1a, $V_H$ 1b, $V_H$ 2, $V_H$ 3, $V_H$ 4, $V_H$ 5, $V_H$ 6), a human Vλ (*e.g.,* VλI, VλII, VλIII, VλIV, VλV, VλVI or Vκ1) or a human Vκ (*e.g.*, Vκ2, Vκ3, Vκ4, Vκ5, Vκ6, Vκ7, Vκ8, Vκ9 or Vκ10).

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

Characterisation of the epitope binding domains.

The binding of a domain to its specific antigen or epitope can be tested by methods which will be familiar to those skilled in the art and include ELISA. In one example, binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein.

The diversity of the selected phage monoclonal antibodies may also be assessed by gel electrophoresis of PCR products (Marks *et al.* 1991, *supra*; Nissim *et al.* 1994 *supra*), probing (Tomlinson et al., 1992) J. Mol. Biol. 227, 776) or by sequencing of the vector DNA.

E. Structure of dAbs

In the case that the dAbs are selected from V-gene repertoires selected for instance using phage display technology as herein described, then these variable domains comprise a universal framework region, such that is they may be recognised by a specific generic ligand as herein defined. The use of universal frameworks and generic ligands is described in WO99/20749.

Where V-gene repertoires are used variation in polypeptide sequence may be located within the structural loops of the variable domains. The polypeptide sequences of either variable domain may be altered by DNA shuffling or by mutation in order to enhance the interaction of each variable domain with its complimentary pair. DNA shuffling is known in the art and taught, for example, by Stemmer, 1994, Nature 370: 389-391 and U.S. Patent No. 6,297,053. Other methods of mutagenesis are well known to those of skill in the art.

Scaffolds for use in Constructing dAbs

i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

The dAbs are advantageously assembled from libraries of domains, such as libraries of $V_H$ domains and/or libraries of $V_L$ domains. In one aspect, libraries of domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human $V_\kappa$ domain, the L1 loop can adopt one of four canonical structures, the L2 loop has a single canonical structure and that 90% of human $V_\kappa$ domains adopt one of four or five canonical structures for the L3 loop (Tomlinson *et al.* (1995) supra); thus, in the $V_\kappa$ domain alone, different canonical structures can combine to create a range of different main-chain conformations. Given that the $V\lambda$ domain encodes a different range of canonical structures for the L1, L2 and L3 loops and that $V_\kappa$ and $V\lambda$ domains can pair with any $V_H$ domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a one particular aspect, the dAbs possess a single known main-chain conformation.

The single main-chain conformation that is chosen may be commonplace among molecules of the immunoglobulin superfamily type in question. A conformation is commonplace when a significant number of naturally occurring molecules are observed to adopt it. Accordingly, in one aspect, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. The desired combination of main-chain conformations for the different loops may be created by selecting germline gene segments which encode the desired main-chain conformations. In one example, the selected germline gene segments are frequently expressed in nature, and in particular they may be the most frequently expressed of all natural germline gene segments.

In designing libraries the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of $V_{\kappa}$□□(39%), L2 - CS 1 (100%), L3 - CS 1 of $V_{\kappa}$□□(36%) (calculation assumes a κ:λ ratio of 70:30, Hood et al. (1967) Cold Spring Harbor Symp. Quant. Biol, 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the $V_H$ segment 3-23 (DP-47), the $J_H$ segment JH4b, the $V_{\kappa}$ segment O2/012 (DPK9) and the $J_{\kappa}$ segment $J_{\kappa}1$. $V_H$ segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five, or for all six of the antigen binding loops can be determined. Here, the chosen conformation may be commonplace in naturally occurring antibodies and may be observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

## Diversification of the canonical sequence

Having selected several known main-chain conformations or a single known main-chain conformation, dAbs can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or they may be selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J. Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than $10^{15}$ structures for H3 alone and a similarly large number of variants for the other five loops, it is not feasible using current transformation technology or even by using cell free systems to produce a library representing all possible combinations. For example, in one of the largest libraries constructed to date, 6 x $10^{10}$ different antibodies, which is only a fraction of the potential diversity for a library of this design, were generated (Griffiths *et al.* (1994) supra).

In one particular example of this application, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

Libraries of dAbs may be used in which only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library.. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" or "dummy" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

It will be understood that the sequences described herein include sequences which are substantially identical, for example sequences which are at least 90% identical, for example which are at least 91%, or at least 92%, or at least 93%, or at least 94% or at least 95%, or at least 96%, or at least 97% or at least 98%, or at least 99% identical to the sequences described herein.

For nucleic acids, the term "substantial identity" indicates that two nucleic acids, or designated sequences thereof, when optimally aligned and compared, are identical, with appropriate nucleotide insertions or deletions, in at least about 80% of the nucleotides, usually at least about 90% to 95%, and more preferably at least about 98% to 99.5% of the nucleotides. Alternatively, substantial identity exists when the segments will hybridize under selective hybridization conditions, to the complement of the strand.

For nucleotide and amino acid sequences, the term "identical" indicates the degree of identity between two nucleic acid or amino acid sequences when optimally aligned and compared with appropriate insertions or deletions. Alternatively, substantial identity exists when the DNA segments will hybridize under selective hybridization conditions, to the complement of the strand.

The percent identity between two sequences is a function of the number of identical positions shared by the sequences (i.e., % identity = # of identical positions/total # of positions times 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package, using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (Comput. Appl. Biósci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

By way of example, a polynucleotide sequence of the present invention may be identical to the reference sequence of SEQ ID NO: 122, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO: 122 by the numerical percent of the respective percent identity(divided by 100) and subtracting that product from said total number of nucleotides in SEQ ID NO: 122, or:

$$nn \leq xn - (xn \bullet y),$$

wherein nn is the number of nucleotide alterations, xn is the total number of nucleotides in SEQ ID NO: 122, and y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and wherein any non-integer product of xn and y is rounded down to the nearest integer prior to subtracting it from xn. Alterations of the polynucleotide sequence of SEQ ID NO: 122 may create nonsense, missense or frameshift mutations in this coding sequence and thereby alter the polypeptide encoded by the polynucleotide following such alterations.

Similarly, in another example, a polypeptide sequence of the present invention may be identical to the reference sequence encoded by SEQ ID NO: 26, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the polypeptide sequence encoded by SEQ ID NO: 26 by the numerical percent of the respective percent identity (divided by 100) and then subtracting that product from said total number of amino acids in the polypeptide sequence encoded by SEQ ID NO: 26, or:

$$na \leq xa - (xa \bullet y),$$

wherein na is the number of amino acid alterations, xa is the total number of amino acids in the polypeptide sequence encoded by SEQ ID NO: 26, and y is, for instance 0.70 for 70%, 0.80 for 80%, 0.85 for 85% etc., and wherein any non-integer product of xa and y is rounded down to the nearest integer prior to subtracting it from xa.

## Examples

The following methods were used in the examples described herein.

### Method 1

### Binding to E.Coli-expressed recombinant human IL-13 by ELISA

mAbdAb molecules were assessed for binding to recombinant *E.coli*-expressed human IL-13 in a direct binding ELISA. In brief, 5μg/ml recombinant *E.coli*-expressed human IL-13 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01 nM). Binding was detected using an appropriate dilution of anti-human kappa light chain peroxidase conjugated antibody (catalogue number A7164, Sigma-Aldrich) or an appropriate dilution of anti-human IgG γ chain specific peroxidase conjugated detection antibody (catalogue number A6029, Sigma-Aldrich).

**Method 2**

**Binding to E.Coli-expressed recombinant human IL-4 by ELISA**

mAbdAb constructs were assessed for binding to recombinant *E.coli*-expressed human IL-4 in a direct binding ELISA. In brief, 5μg/ml recombinant *E.coli*-expressed human IL-4 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01 nM). Binding was detected using an appropriate dilution of goat anti-human kappa light chain peroxidase conjugated antibody (catalogue number A7164, Sigma-Aldrich) or an appropriate dilution of anti-human IgG γ chain specific peroxidase conjugated detection antibody (catalogue number A6029, Sigma-Aldrich).

**Method 3**

**Binding to *E.Coli*-expressed recombinant human IL-18 by ELISA**

mAbdAb constructs were assessed for binding to recombinant *E.coli*-expressed human IL-18 in a direct binding ELISA. In brief, 5μg/ml recombinant *E.coli*-expressed human IL-18 (made and purified at GSK) was coated to a 96-well ELISA plate. The wells were blocked for 1 hour at room temperature, mAbdAb constructs were then titrated out across the plate (usually from around 100nM in 3-fold dilutions to around 0.01nM). Binding was detected using using a dilution of 1 in 2000 of anti-human kappa light chain peroxidase conjugated antibody (catalogue number A7164, Sigma-Aldrich) or using a dilution of 1 in 2000 of anti-human IgG γ chain specific peroxidase conjugated detection antibody (catalogue number A6029, Sigma-Aldrich).

**Method 4**

**Biacore™ binding affinity assessment for binding to E.Coli-expressed recombinant human IL-13**

The binding affinity of mAbdAb constructs for recombinant *E.Coli*-expressed human IL-13 were assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-13 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-13 binding event. The anti-human IgG surface was regenerated either using similar conditions to the Protein A surface or by using 3M $MgCl_2$. The work was carried out on the Biacore™ 3000 and/or the T100 machine, data were analysed using the evaluation software in the machines and fitted to the 1:1 model of binding. Biacore™ runs were carried out at 25°C or 37°C.

**Method 5**

**Biacore™ binding affinity assessment for binding to E.Coli-expressed recombinant human IL-4**

The binding affinity of mAbdAb constructs for recombinant *E.Coli*-expressed human IL-4 were assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-4 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-4 binding event. The anti-human IgG surface was regenerated either using similar conditions to the Protein A surface or by using 3M $MgCl_{2.}$ The work was carried out on Biacore™ 3000 and / or the T100 and / or the A100 machine, data were analysed using the evaluation software in the machines and fitted to the 1:1 model of binding. Biacore™ runs were carried out at 25°C or 37°C.

**Method 6**

**Biacore™ binding affinity assessment for binding to E.Coli-expressed recombinant human IL-18**

The binding affinity of mAbdAb constructs for recombinant *E.Coli*-expressed human IL-18 was assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb constructs were then captured onto this surface and human IL-18 (made and purified at GSK) passed over at defined concentrations. The surface was regenerated back to the Protein A surface using mild acid elution conditions(such as 100mM phosphoric acid), this did not significantly affect the ability to capture antibody for a subsequent IL-18 binding event. The anti-human IgG surface was regenerated either using similar conditions to the Protein A surface or by using 3M $MgCl_2$. The work was carried out on Biacore™ 3000 and / or the T100 and / or the A100 machine, data were analysed using the evaluation software in the machines and fitted to the 1:1 model of binding. The Biacore™ run was carried out at 25°C.

**Method 7**

**Stoichiometry assessment of mAbdAb bispecific antibodies or trispecific antibody for IL-13, IL-4 or IL-18 (using Biacore™)**

Anti-human IgG was immobilised onto a CM5 biosensor chip by primary amine coupling. mAbdAb constructs were captured onto this surface after which a single concentration of IL-13, IL-4 or IL-18 cytokine was passed over, this concentration was enough to saturate the binding surface and the binding signal observed reached full R-max. Stoichiometries were then calculated using the given formula:

$$\text{Stoich} = \text{Rmax} * \text{Mw (ligand)} / \text{Mw (analyte)} * \text{R (ligand immobilised or captured)}$$

Where the stoichiometries were calculated for more than one analyte binding at the same time, the different cytokines were passed over sequentially at the saturating cytokine concentration and the stoichometries calculated as above. The work was carried out on the Biacore 3000, at 25°C using HBS-EP running buffer.

**Method 8**

**Neutralisation of E.Coli-expressed recombinant human IL-13 in a TF-1 cell proliferation bioassay**

TF-1 cells proliferate in response to a number of different cytokines including human IL-13. The proliferative response of these cells for IL-13 can therefore be used to measure the bioactivity of IL-13 and subsequently an assay has been developed to determine the IL-13 neutralisation potency (inhibition of IL-13 bioactivity) of mAbdAb constructs.

The assay was performed in sterile 96-well tissue culture plates under sterile conditions and all test wells were performed in triplicate. Approximately 14ng/ml recombinant E.Coli-expressed human IL-13 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01 nM), recombinant E.Coli-expressed human IL-13 (at a final concentration of 7ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed human IL-13 bioactivity was expressed as that concentration of the mAbdAb construct required to neutralise the bioactivity of the defined amount of human IL-13 (7ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 9**

**Neutralisation of E.Coli-expressed recombinant human IL-4 in a TF-1 cell proliferation bioassay**

TF-1 cells proliferate in response to a number of different cytokines including human IL-4. The proliferative response of these cells for IL-4 can therefore be used to measure the bioactivity of IL-4 and subsequently an assay has been developed to determine the IL-4 neutralisation potency (inhibition of IL-4 bioactivity) of mAbdAb constructs.

The assay was performed in sterile 96-well tissue culture plates under sterile conditions and all test wells were performed in triplicate. Approximately 2.2ng/ml recombinant E.Coli-expressed human IL-4 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01 nM), recombinant E.Coli-expressed human IL-4 (at a final concentration of 1.1ng/ml) and

TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed human IL-4 bioactivity was expressed as that concentration of the mAbdAb construct required to neutralise the bioactivity of the defined amount of human IL-4 (1.1 ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 10**

**Dual neutralisation of E.Coli-expressed recombinant human IL-13 and E.Coli-expressed recombinant human IL-4 in a TF-1 cell proliferation bioassay**

TF-1 cells proliferate in response to a number of different cytokines including human IL-13 and human IL-4. The proliferative response of these cells for IL-13 and IL-4 can therefore be used to measure the bioactivity of IL-13 and IL-4 simultaneously and subsequently an assay has been developed to determine the dual IL-13 and IL-4 neutralisation potency (dual inhibition of IL-13 and IL-4 bioactivity) of mAbdAb constructs.

The assay was performed in sterile 96-well tissue culture plates under sterile conditions and all test wells were performed in triplicate. Approximately 14ng/ml recombinant E.Coli-expressed human IL-13 and approximately 2.2ng/ml recombinant E.Coli-expressed human IL-4 were pre-incubated with various dilutions of mAbdAbs constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume, contained various dilutions of mAbdAb constructs (at a final concentration of 100nM titrated in 3-fold dilutions to 0.01nM), recombinant E.Coli-expressed human IL-13 (at a final concentration of 7ng/ml), recombinant E.Coli-expressed human IL-4 (at a final concentration of 1.1ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

**Method 11**

**Biacore™ binding affinity assessment for binding to *Sf21*-expressed recombinant human IL-5**

The binding affinity of mAbdAb molecules for recombinant *Sf21*-expressed human IL-5 was assessed by Biacore™ analysis. Analyses were carried but using Protein A or anti-human IgG capture. Briefly, Protein A or anti-human IgG was coupled onto a CM5 chip by primary amine coupling in accordance with the manufactures recommendations. mAbdAb molecules were then captured onto this surface and human IL-5 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-5 binding event. The anti-human IgG surface was regenerated either using similar conditions to the Protein A surface or by using 3M $MgCl_2$. The work was carried out on Biacore™ 3000, T100 and A100 machines, data were analysed using the evaluation software in the machines and fitted to the 1:1 model of binding. The Biacore™ run was carried out at 25°C.

**Method 12**

**VEGF Receptor Binding Assay.**

This assay measures the binding of $VEGF_{165}$ to VEGF R2 (VEGF receptor) and the ability of test molecules to block this interaction. ELISA plates were coated overnight with VEGF receptor (R&D Systems, Cat No: 357-KD-050) (0.5μg/ml final concentration in 0.2M sodium carbonate bicarbonate pH9.4), washed and blocked with 2% BSA in PBS. VEGF (R&D Systems, Cat No: 293-VE-050) and the test molecules (diluted in 0.1%BSA in 0.05% Tween 20™ PBS) were pre-incubated for one hour prior to addition to the plate (3ng/ml VEGF final concentration). Binding of VEGF to VEGF receptor was detected using biotinylated anti-VEGF antibody (0.5μg/ml final concentration) (R&D Systems, Cat No: BAF293) and a peroxidase conjugated anti-biotin secondary antibody (1:5000 dilution) (Stratech, Cat No: 200-032-096) and visualised at OD450 using a colorimetric substrate (Sure Blue TMB peroxidase substrate, KPL) after stopping the reaction with an equal volume of 1 M HCl.

**Method 13**

**EGFR Kinase Assay**

Activation of EGFR expressed on the surface of A431 cells through its interaction with EGF leads to tyrosine kinase phosphorylation of the receptor. Reduction of EGFR tyrosine kinase phosphorylation was measured to determine potency of test molecules. A431 cells were allowed to adhere to 96 well tissue culture plates overnight then the test molecule was added and left for 1 hour and then incubated for 10 min with EGF (at 300ng/ml) (R&D Systems catalogue number 236-EG). The cells were lysed and the lysed preparation transferred to ELISA plates coated with anti-EGFR antibody (at 1ug/ml) (R&D Systems, cat # AF231). Both phosphorylated and non-phosphorylated EGFR present in the lysed cell solution was captured. After washing away unbound material phosphorylated EGFR was specifically detected using a HRP conjugated anti-phosphotyrosine antibody (1:2000 dilution) (Upstate Biotechnology, cat # 16-105). Binding was visualised using a colorimetric substrate.

**Method 14**

**MRC-5/TNF Assay**

The ability of test molecules to prevent human TNF-a binding to human TNFR1 and neutralise IL-8 secretion was determined using human lung fibroblast MRC-5 cells. A dilution series of test samples was incubated with TNF-a (500pg/ml) (Peprotech) for 1 hour. This was then diluted 1 in 2 with a suspension of MRC-5 cells (ATCC, Cat.# CCL-171) ($5x10^3$ cells/well). After an overnight incubation, samples were diluted 1 in 10, and IL-8 release was determined using an IL-8 ABI 8200 cellular detection assay (FMAT) where the IL-8 concentration was determined using anti-IL-8 (R&D systems, Cat# 208-IL) coated polystyrene beads, biotinylated anti-IL-8 (R&D systems, Cat# BAF208) and streptavidin Alexafluor 647 (Molecular Probes, Cat#S32357). The assay readout was localised fluorescence emission at 647nm and unknown IL-8 concentrations were interpolated using an IL-8 standard curve included in the assay.

**Method 15**

**MRC-5/IL-1 Assay.**

The ability of test molecules to prevent human IL-1a binding to human IL1-R and neutralise IL-8 secretion was determined using human lung fibroblast MRC-5 cells. MRC-5 cells (ATCC, Cat.# CCL-171) were trypsinised then incubated with the test samples for one hour as a suspension. IL-1a (200pg/ml final concentration) (R&D Systems cat no: 200-LA) was then added. After an overnight incubation IL-8 release was determined using an IL-8 quantification ELISA kit (R&D Systems) with anti-IL-8 coated ELISA plates, biotinylated anti-IL-8 and streptavidin-HRP. The assay readout is colourimetric absorbance at 450nm and unknown IL-8 concentrations are interpolated using an IL-8 standard curve included in the assay.

**Method 16**

**Neutralisation potency of E.Coli-expressed recombinant human IL-13 or IL-4 in a whole blood phospho-STAT6 bioassay**

Whole blood cells can be stimulated ex-vivo with recombinant E.Coli-expressed human IL-4 (rhIL-14) or IL-13 (rhIL-13) to express phospho STAT6 (pSTAT6). This assay was developed to quantitatively measure pSTAT6 and consequently determine the neutralisation potency (inhibition of IL-4 or IL-13 bioactivity) of mAbdAb constructs.

The assay was performed in sterile 96-well tissue culture plates under sterile conditions and all test wells were performed in triplicate. 12ng/ml of rhIL-13 or rhIL-4 was prepared in serum free cell culture medium and 31.2μL added to wells of a 96-well plate. A 9 point dilution curve of mAbdAb constructs or isotype control was prepared at 6x the final assay concentration and 31.2μL of each dilution added to wells containing either rhIL-4 or rhIL-13. 125μL of heparinized human whole blood was added to all wells and mixed on a shaker for 30 seconds. The final assay volume contained various dilutions of mAbdAb constructs together with rhIL-13 or rhIL-4 at a final concentration of 2ng/mL. The assay plate was incubated at 37°C, 5%$CO_2$ for 60 minutes.

The cells were then lysed by the addition of 62.5μL of 4x lysis buffer. The lysis buffer contained final assay concentrations of 50mM Tris hydrochloride, 300mM sodium chloride, 1% NP40, 0.5% sodium deoxycholate, 50mM sodium fluoride, 1mM sodium orthovanadate, 1mM EDTA, and protease inhibitor cocktail. The plates were placed on ice for 30 minutes then frozen at -80°C until assayed for pSTAT6.

The measurement of pSTAT6 in the whole blood samples was performed using an electro-chemiluminescent immuno-assay (Meso-Scale-Discovery, MSD). In brief, avidin coated 96-well MSD plates were blocked with 150μL per well of 5% MSD blocker A for 1 hour at room temperature on a shaker at 750rpm. The plate was washed 3 times with 150μL per well of MSD Tris wash buffer. 25μL per well of capture antibody (biotinylated mouse anti-human STAT6 monoclonal antibody) was added and the plates incubated overnight at 4°C. The capture antibody had been diluted to 4μg/mL in assay buffer consisting of 50mM Tris, 150mM sodium chloride, 0.2% BSA, 0.5% Tween 20, 1mM EDTA. The plate was washed 3 times with MSD tris wash buffer then blocked with 150μL of 5% MSD blocker A for 1 hour at room temperature on a shaker. Plates were washed 3 times as stated previously, then 25μL of whole blood lysate or pSTAT6 calibrator added per well. Plates were incubated for 3 hours at room temperature on a shaker. Plates were washed 3 times then 25μL of rabbit anti human pSTAT6 antibody (diluted 1 in 800 in assay buffer) was added and then incubated for 1 hour at room temperature. After further washing, 25μL per well of a 1 in 500 dilution of MSD TAG goat anti-rabbit IgG antibody was added and then incubated for 1 hour at room temperature on a shaker. Plates were washed again before addition of 150μL per well of 2x MSD read buffer T. Plates were read immediately on a MSD SECTOR imager.

The ability of the mAbdAb constructs to neutralise rhIL-13 or rhIL-4 bioactivity was expressed as the concentration of the mAbdAb construct required to neutralise 2ng/mL of human IL-4 or human IL-13 by 50% ($IC_{50}$). The lower the concentration of the mAbdAb construct required, the more potent the neutralisation capacity.

**Method 17**

**Binding to E.Coli-expressed recombinant cynomolgus IL-13 by ELISA**

mAbdAb molecules were assessed for binding to recombinant *E.coli*-expressed cynomolgus IL-13 in a direct binding ELISA. In brief, 5μg/ml recombinant *E.coli*-expressed cynomolgus IL-13 (made and purified at GSK) was coated to a 96-well ELISA plate. The wells were blocked for 1 hour at room temperature, mAbdAb molecules were then titrated out across the plate (usually from around 100nM in 3-fold dilutions to around 0.01 nM). Binding was detected using an appropriate dilution of anti-human kappa light chain peroxidase conjugated antibody (catalogue number A7164, Sigma-Aldrich) or an appropriate dilution of anti-human IgG γ chain specific peroxidase conjugated detection antibody (catalogue number A6029, Sigma-Aldrich).

**Method 18**

Not used

**Method 19**

**Inhibition of human IL-4 binding to human IL4 receptor alpha (IL4Rα) by ELISA**

Unless otherwise stated all reagents were diluted to the required concentration in block solution (4% bovine serum albumin in tris-buffered saline and 0.05% Tween20) just prior to use. An ELISA plate was coated over-night at

4°C with 5μg/ml of recombinant human IL4Rα-Fc chimaera (R&D Systems, Cat. No. 604-4R) in phosphate buffered saline. All subsequent steps were carried out at room temperature. The plate was blocked for 2 hours in block solution before addition of 50μl of various concentrations of mAbdAb (or the positive control mAbs or dAbs) which had been pre-mixed with 0.02μg/ml of recombinant human IL-4 (made at GSK). Plates were incubated for 1 hour before washing 4 times in wash buffer (Tris buffered saline and 0.05% Tween20). 50μl of a 0.5μg/ml solution of biotinylated anti-human IL-4 (R&D Systems, Cat. No. BAF 204) was added to each well and incubated for 1 hour. The plate was washed x4 in wash buffer before addition of 50μl/well of a 1/2000 dilution of Extravadin (Sigma, Cat. No. E2886). After one hour the plate was washed 4 times and a colourirmetric signal was detected by incubating with OPD peroxidase substrate (from Sigma), the reaction was stopped with the stop solution (3M $H_2SO_4$ acid) and absorbance data obtained by reading on a plate-reader at 490nm. Mean absorbance and standard error was plotted in GraphPad Prism and $IC_{50}$ values were calculated using Cambridge Soft BioAssay.

**Method 20**

**Neutralisation of E.Coli-expressed recombinant cynomolgus IL-13 in a TF-1 cell proliferation bioassay**

TF-1 cells proliferate in response to a number of different cytokines including cynomolgus IL-13. The proliferative response of these cells for IL-13 can therefore be used to measure the bioactivity of IL-13 and subsequently an assay has been developed to determine the IL-13 neutralisation potency (inhibition of IL-13 bioactivity) of mAbdAb constructs.

The assay was performed in sterile 96-well tissue culture plates under sterile conditions and all test wells were performed in triplicate. Approximately 14ng/ml recombinant E.Coli-expressed cynomolgus IL-13 was pre-incubated with various dilutions of mAbdAb constructs (usually from 1000nM or 200nM titrated in 3-fold dilutions to 1nM or 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 500nM or 100nM titrated in 3-fold dilutions to 0.5nM or 0.01 nM), recombinant E.Coli-expressed cynomolgus IL-13 (at a final concentration of 7ng/ml) and TF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed cynomolgus IL-13 bioactivity was expressed as that concentration of the mAbdAb construct required to neutralise the bioactivity of the defined amount of cynomolgus IL-13 (7ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 21**

**Neutralisation of E.Coli-expressed recombinant cynomolgus IL-4 in a TF-1 cell proliferation bioassay**

TF-1 cells proliferate in response to a number of different cytokines including cynomolgus IL-4. The proliferative response of these cells for IL-4 can therefore be used to measure the bioactivity of IL-4 and subsequently an assay has been developed to determine the IL-4 neutralisation potency (inhibition of IL-4 bioactivity) of mAbdAb constructs.

The assay was performed in sterile 96-well tissue culture plates under sterile conditions and all test wells were performed in triplicate. Approximately 2.2ng/ml recombinant E.Coli-expressed cynomolgus IL-4 was pre-incubated with various dilutions of mAbdAb constructs (usually from 200nM titrated in 3-fold dilutions to 0.02nM) in a total volume of 50μl for 1 hour at 37°C. These samples were then added to 50μl of TF-1 cells (at a concentration of $2x10^5$ cells per ml) in a sterile 96-well tissue culture plate. Thus the final 100μl assay volume contained various dilutions of mAbdAb constructs (at a final concentration of 100nM titrated in 3-fold dilutions to 0.01 nM), recombinant E.Coli-expressed cynomolgus IL-4 (at a final concentration of 1.1ng/ml) and TNF-1 cells (at a final concentration of $1x10^5$ cells per ml). The assay plate was incubated at 37°C for approximately 3 days in a humidified $CO_2$ incubator. The amount of cell proliferation was then determined using the 'CellTitre 96® Non-Radioactive Cell Proliferation Assay' from Promega (catalogue number G4100), as described in the manufacturers instructions. The absorbance of the samples in the 96-well plate was read in a plate reader at 570nm.

The capacity of the mAbdAb constructs to neutralise recombinant E.Coli-expressed cynomolgus IL-4 bioactivity was expressed as that concentration of the mAbdAb construct required to neutralise the bioactivity of the defined amount of cynomolgus IL-4 (1.1ng/ml) by 50% (= $ND_{50}$). The lower the concentration of the mAbdAb construct required, the more potent the neutralisation capacity. The $ND_{50}$ data provided herein were calculated manually or by using the Robosage software package which is inherent within microsoft excel.

**Method 22**

**Inhibition of human IL-13 binding to human IL13 receptor alpha_2 (IL13Rα2) by ELISA**

Unless otherwise stated all reagents were diluted to the required concentration in block solution (1% bovine serum albumin in tris-buffered saline and 0.05% Tween20) just prior to use. An ELISA plate was coated overnight at 4°C with 5μg/ml of recombinant human IL13Rα2/Fc chimera expressed in Sf21 cells (R&D Systems, Cat. No. 614-IR) in a solution of coating buffer (0.05M bicarbonate pH9.6, Sigma C-3041). The plate was blocked for 1 hour at room temperature in block solution (1% BSA in TBST) before addition of various concentrations of mAbdAb (or the positive control mAbs or dAbs) which had been pre-incubated with 30ng/ml of recombinant human IL-13 (made at GSK) for 30 mins at 37°C. Plates were incubated for 1 hour at room temperature before washing 3 times in wash buffer (Tris buffered saline and 0.05% Tween20). 50μl of a 0.5μg/ml solution of biotinylated anti-human IL-13 (R&D Systems, Cat. No. BAF 213) was added to each well and incubated for 1 hour at room temperature. The plate was washed three times in wash buffer before addition of an appropriate dilution of Extravadin (Sigma, Cat. No. E2886). After one hour the plate was washed and a colourimetric signal was detected by incubating with OPD peroxidase substrate (from Sigma), the reaction was stopped with the stop solution (3M acid) and absorbance data obtained by reading on a plate-reader at 490nm. Mean absorbance and standard error was plotted in Excel sheet and $IC_{50}$ values were calculated using the Robosage software from Microsoft Excel.

Method 23

**Biacore™ binding affinity assessment for binding to E.Coli-expressed recombinant cynomolgus IL-13**

The binding affinity of mAbdAb (or mAb) molecules for recombinant *E.Coli*-expressed cynomolgus IL-13 was assessed by Biacore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb (or mAb) molecules were then captured onto this surface and cynomolgus IL-13 (made and purified at GSK) passed over at defined concentrations. The surface was regenerated back to the Protein A surface using mild acid elution conditions, this did not significantly affect the ability to capture antibody for a subsequent IL-13 binding event. The work was carried out on BIAcore™ 3000 and / or the T100 machine, data were analysed using the evaluation software in the machines and fitted to the 1:1 model of binding. BIAcore™ runs were carried out at 25°C or 37°C.

**Method 24**

**BIAcore™ binding affinity assessment for binding to E.Coli-expressed recombinant cynomolgus IL-4**

The binding affinity of mAbdAb (or mAb) molecules for recombinant *E.Coli*-expressed cynomolgus IL-4 were assessed by BIAcore™ analysis. Analyses were carried out using Protein A or anti-human IgG capture. Briefly, Protein A or anti-human IgG was coupled onto a CM5 chip by primary amine coupling in accordance with the manufactures recommendations. mAbdAb (or mAb) molecules were then captured onto this surface and cynomolgus IL-4 (made and purified at GSK) passed over at defined concentrations. The surface was regenerated back to the Protein A surface using mild acid elution conditions (such as 100mM phosphoric acid), this did not significantly affect the ability to capture antibody for a subsequent IL-4 binding event. The anti-human IgG surface was regenerated either using similar conditions to the Protein A surface or by using 3M $MgCl_2$. The work was carried out on BIAcore™ 3000 and / or the T100 and / or the A100 machine, data were analysed using the evaluation software in the machines and fitted to the 1:1 model of binding. BIAcore™ runs were carried out at 25°C or 37°C.

**Method 25**

**IL-13 cell-based neutralisation assay**

The potency of mAbdAbs having specificity for IL13 was assayed in an IL-13 cell assay using the engineered reporter cell line HEK Blue-STAT6.

The transcription factor STAT6 is activated primarily by two cytokines with overlapping biologic functions, IL-4 and IL-13 which bind a receptor complex composed of the IL-4Ralpha and IL-13Ralpha1. Upon ligand binding, the receptor complex activates the receptor-associated Janus kinases (JAK1 and Tyk2) leading to the recruitment of STAT6 and its phosphorylation. Activated STAT6 forms a homodimer that translocates to the nucleus, inducing transcription of genes under the control of the responsive promoter. The HEK Blue-STAT6 line is engineered to express Secreted Embryonic Alkaline Phosphatase (SEAP) under the control of such a promoter.

Cells were plated into 96 well plates and incubated for 20-24 hours with pre-equilibrated human human IL-13 and test molecules. After this incubation period, the amount of SEAP produced by the cells as a result of IL-13 stimulation was then measured using the Quanti-blue system (Invivogen).

## Example 1

### 1. Generation of dual targeting mAbdAbs

The dual targeting mAbdAbs set out in Tables 1-4 were constructed in the following way. Expression constructs were generated by grafting a sequence encoding a domain antibody on to a sequence encoding a heavy chain or a light chain (or both) of a monoclonal antibody such that when expressed the dAb is attached to the C-terminus of the heavy or light chain. For some constructs, linker sequences were used to join the domain antibody to heavy chain CH3 or light chain CK. In other constructs the domain antibody is joined directly to the heavy or light chain with no linker sequence. A general schematic diagram of these mAbdAb constructs is shown in Figure 8 (the mAb heavy chain is drawn in grey; the mAb light chain is drawn in white; the dAb is drawn in black).

An example of mAbdAb type 1 as set out in Figure 8 would be PascoH-G4S-474. An example of mAbdAb type 2 as set out in Figure 8 would be PascoL-G4S-474. An example of mAbdAb type 3 as set out in Figure 8 would be PascoHL-G4S-474: mAbdAb types 1 and 2 are tetravalent constructs, mAbdAb type 3 is a hexavalent construct.

A schematic diagram illustrating the construction of a mAbdAb heavy chain (top illustration) or a mAbdAb light chain (bottom illustration) is shown below. Unless otherwise stated, these restriction sites were used to construct the mAbdAb described in Tables 1-4

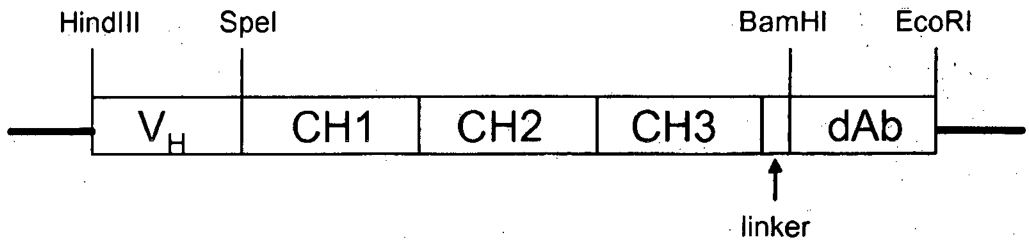


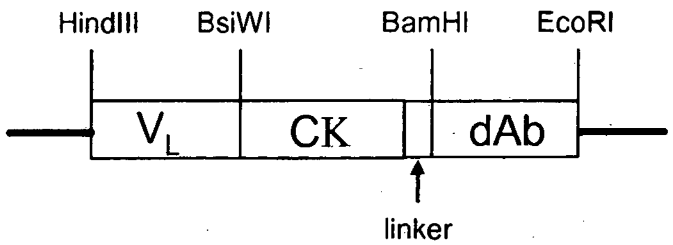


Note that for the heavy chain the term '$V_H$' is the monoclonal antibody variable heavy chain sequence; 'CH1, CH2 and CH3' are human IgG1 heavy chain constant region sequences; 'linker' is the sequence of the specific linker region used; 'dAb' is the domain antibody sequence. For the light chain the term '$V_L$' is the monoclonal antibody variable light chain sequence; 'CK' is the human light chain constant region sequence; 'linker' is the sequence of the specific linker region used; 'dAb' is the domain antibody sequence.

Some DNA expression constructs were made de novo by oligo build. And other DNA expression constructs were derived from exisiting constructs (which were made as described above) by restriction cloning or site-directed mutagenesis.

These constructs (mAbdAb heavy or light chains) were cloned into mammalian expression vectors (Rln, Rid or pTT vector series) using standard molecular biology techniques. A mammalian amino acid signal sequence (as shown in SEQ ID NO: 64) was used in the construction of these constructs.

For expression of mAbdAbs where the dAb is joined to the C-terminal end of the heavy chain of the monoclonal antibody, the appropriate heavy chain mAbdAb expression vector was paired with the appropriate light chain expression vector for that monoclonal antibody. For expression of mAbdAbs where the dAb is joined to the C-terminal end of the light chain of the monoclonal antibody, the appropriate light chain mAbdAb expression vector was paired with the appropriate heavy chain expression vector for that monoclonal antibody.

For expression of mAbdAbs where the dAb is joined to the C-terminal end of the heavy chain of the monoclonal antibody and where the dAb is joined to the C-terminal end of the light chain of the monoclonal antibody, the appropriate heavy chain mAbdAb expression vector was paired with the appropriate light chain mAbdAb expression vector.

### 1.1 Nomenclature and abbreviations used

Monoclonal antibody (mAb)
Monoclonal antibodies (mAbs)
Domain antibody (dAb)
Domain antibodies (dAbs)
Heavy Chain (H chain)
Light chain (L chain)
Heavy chain variable region ($V_H$)
Light chain variable region ($V_L$)
Human IgG1 constant heavy region 1 (CH1)
Human IgG1 constant heavy region 2 (CH2)
Human IgG1 constant heavy region 3 (CH3)
Human kappa light chain constant region (CK)

### 1.2 Anti-IL13mAb-anti-IL4dAbs

Bispecific anti-IL13mAb-anti-IL4dAbs were constructed by as described above. A number of different linkers were used to join the anti-IL4 domain antibodies to the monoclonal antibody. Some constructs had no linker.

Note that a BamHI cloning site (which codes for amino acid residues G and S) was used to clone the linkers and dAbs either to CH3 of the mAb heavy chain or to CK of the mAb light chain. Thus in addition to the given linker sequence, additional G and S amino acid residues are present between the linker sequence and the domain antibody for both heavy chain and light chain expression constructs or between CH3 and the linker sequence in some but not all heavy chain expression constructs. However, when the G4S linker was placed between the mAb and dAb in the mAbdAb format, the BamHI cloning site was already present (due to the G and S amino acid residues inherent within the G4S linker sequence) and thus additional G and S amino acid residues were not present between CH3 or CK and the domain antibody in the constructs using this linker. When no linker sequence was between used n the mAb and dAb in the mAbdAb format, the BamHI cloning site (and hence the G and S amino acid residues) was still present between CH3 or CK and the domain antibody. Full details on the amino acid sequences of mAbdAb heavy and light chains are set out in Table 1.

Several of the following examples use an IL-4 mAb as a control antibody. The control IL-4 mAb used in these examples will either be the antibody having the heavy chain sequence of SEQ ID NO: 14 and the light chain sequence of SEQ ID NO: 15, or will be the antibody having the heavy chain sequence of SEQ ID NO: 166 and the light chain sequence of SEQ ID NO: 15. Both of these IL-4 mAbs share the same CDRs, and are expected to bind in the same way hence both of these antibodies are referred to as 'Pascolizumab' or 'IL-4 mAb' in the following examples.

Several of the following examples use an IL-5 mAb as a control antibody. The control IL-5 mAb used in these examples will either be the antibody having the heavy chain sequence of SEQ ID NO: 65 and the light chain sequence of SEQ ID NO: 66, or the antibody having the heavy chain sequence of SEQ ID NO: 191 and the light chain sequence of SEQ ID NO: 66. Both of these IL-5 antibodies share the same CDRs, and are expected to bind in the same way hence both of these antibodies are referred to as 'Mepolizumab' or 'IL-5 mAb' in the following examples.

The mAbdAbs set out in table 1 were expressed transiently in CHOK1 cell supernatants. Following mAbdAb quantification these mAbdAb containing supernatants were analysed for activity in IL-13 and IL-4 binding ELISAs.

Table 1

| Name | Description | Sequence ID No. |
|---|---|---|
| 586H-25 | H chain = Anti-human IL-13 mAb heavy chain-GS-DOM9-155-25 dAb<br><br>L chain = Anti-human IL-13 mAb light chain | 16 (=H chain)<br>13 (=L chain) |

(continued)

| Name | Description | Sequence ID No. |
|---|---|---|
| 586H-G4S-25 | H chain = Anti-human IL-13 mAb heavy chain-G4S linker-DOM9-155-25 dAb<br>L chain = Anti-human IL-13 mAb light chain | 20 (=H chain)<br>13 (=L chain) |
| 586H-TVAAPS-25 | H chain = Anti-human IL-13 mAb heavy chain-TVAAPS linker-GS-DOM9-155-25 dAb<br>L chain = Anti-human IL-13 mAb light chain | 24 (=H chain)<br>13 (=L chain) |
| 586H-ASTKG-25 | H chain = Anti-human IL-13 mAb heavy chain-GS-ASTKGPT linker-GS-DOM9-155-25 dAb<br>L chain = Anti-human IL-13 mAb light chain | 28 (=H chain)<br>13 (=L chain) |
| 586H-EPKSC-25 | H chain = Anti-human IL-13 mAb heavy chain-GS-EPKSCDKTHTCPPCP linker-GS-DOM9-155-25 dAb<br>L chain = Anti-human IL-13 mAb light chain | 32 (=H chain)<br>13 (=L chain) |
| 586H-ELQLE-25 | H chain = Anti-human IL-13 mAb heavy chain-ELQLEESCAEAQDGELDG linker-GS-DOM9-155-25 dAb<br>L chain = Anti-human IL-13 mAb light chain | 36 (=H chain)<br>13 (=L chain) |
| 586H-147 | H chain = Anti-human IL-13 mAb heavy chain-GS-DOM9-155-147 dAb<br>L chain = Anti-human IL-13 mAb light chain | 17 (=H chain)<br>13 (=L chain) |
| 586H-G4S-147 | H chain = Anti-human IL-13 mAb heavy chain-G4S linker-DOM9-155-147 dAb<br>L chain = Anti-human IL-13 mAb light chain | 21 (=H chain)<br>13 (=L chain) |
| 586H-TVAAPS-147 | H chain = Anti-human IL-13 mAb heavy chain-TVAAPS linker-GS-DOM9-155-147 dAb<br>L chain = Anti-human IL-13 mAb light chain | 25 (=H chain)<br>13 (=L chain) |
| 586H-ASTKG-147 | H chain = Anti-human IL-13 mAb heavy chain-GS-ASTKGPT linker-DOM9-155-147 dAb<br>L chain = Anti-human IL-13 mAb light chain | 29 (=H chain)<br>13 (=L chain) |
| 586H-EPKSC-147 | H chain = Anti-human IL-13 mAb heavy chain-GS-EPKSCDKTHTCPPCP linker-GS-DOM9-155-147 dAb<br>L chain = Anti-human IL-13 mAb light chain | 33 (=H chain)<br>13 (=L chain) |
| 586H-ELQLE-147 | H chain = Anti-human IL-13 mAb heavy chain-GS-ELQLEESCAEAQDGELDG linker-GS-DOM9-155-147 dAb<br>L chain = Anti-human IL-13 mAb light chain | 37 (=H chain)<br>13 (=L chain) |
| 586H-154 | H chain = Anti-human IL-13 mAb heavy chain-GS-DOM9-155-154 dAb<br>L chain = Anti-human IL-13 mAb light chain | 18 (=H chain)<br>13 (=L chain) |
| 586H-G4S-154 | H chain = Anti-human IL-13 mAb heavy chain-G4S linker-DOM9-155-154 dAb<br>L chain = Anti-human IL-13 mAb light chain | 22 (=H chain)<br>13 (=L chain) |
| 586H-TVAAPS-154 | H chain = Anti-human IL-13 mAb heavy chain-TVAAPS linker-GS-DOM9-155-154 dAb<br>L chain = Anti-human IL-13 mAb light chain | 26 (=H chain)<br>13 (=L chain) |
| 586H-ASTKG-154 | H chain = Anti-human IL-13 mAb heavy chain-GS-ASTKGPT linker-GS-DOM9-155-154 dAb<br>L chain = Anti-human IL-13 mAb light chain | 30 (=H chain)<br>13 (=L chain) |

(continued)

| Name | Description | Sequence ID No. |
|---|---|---|
| 586H-EPKSC-154 | H chain = Anti-human IL-13 mAb heavy chain-GS-EPKSCDKTHTCPPCP linker-GS-DOM9-155-154 dAb<br>L chain = Anti-human IL-13 mAb light chain | 34 (=H chain)<br>13 (=L chain) |
| 586H-ELQLE-154 | H chain = Anti-human IL-13 mAb heavy chain-GS-ELQLEESCAEAQDGELDG linker-GS-DOM9-155-154 dAb<br>L chain = Anti-human IL-13 mAb light chain | 38 (=H chain)<br>13 (=L chain) |
| 586H-210 | H chain = Anti-human IL-13 mAb heavy chain-GS-DOM9-112-210 dAb<br>L chain = Anti-human IL-13 mAb light chain | 19 (=H chain)<br>13 (=L chain) |
| 586H-G4S-210 | H chain = Anti-human IL-13 mAb heavy chain-G4S linker-DOM9-112-210 dAb<br>L chain = Anti-human IL-13 mAb light chain | 23 (=H chain)<br>13 (=L chain) |
| 586H-TVAAPS-210 | H chain = Anti-human IL-13 mAb heavy chain-TVAAPS linker-GS-DOM9-112-210 dAb<br>L chain = Anti-human IL-13 mAb light chain | 27 (=H chain)<br>13 (=L chain) |
| 586H-ASTKG-210 | H chain = Anti-human IL-13 mAb heavy chain-GS-ASTKGPT linker-GS-DOM9-112-210 dAb<br>L chain = Anti-human IL-13 mAb light chain | 31 (=H chain)<br>13 (=L chain) |
| 586H-EPKSC-210 | H chain = Anti-human IL-13 mAb heavy chain-GS-EPKSCDKTHTCPPCP linker-GS-DOM9-112-210 dAb<br>L chain = Anti-human IL-13 mAb light chain | 35 (=H chain)<br>13 (=L chain) |
| 586H-ELQLE-210 | H chain = Anti-human IL-13 mAb heavy chain-GS-ELQLEESCAEAQDGELDG linker-GS-DOM9-112-210 dAb<br>L chain = Anti-human IL-13 mAb light chain | 39 (=H chain)<br>13 (=L chain) |
| 586H | H chain = Anti-human IL-13 mAb heavy chain-GS-<br>L chain = Anti-human IL-13 mAb light chain | 40 (=H chain)<br>13 (=L chain) |
| 586H-ASTKG | H chain = Anti-human IL-13 mAb heavy chain-GS-ASTKGPT linker-GS<br>L chain = Anti-human IL-13 mAb light chain | 41 (=H chain)<br>13 (=L chain) |
| 586H-EPKSC | H chain = Anti-human IL-13 mAb heavy chain-GS-EPKSCDKTHTCPPCP liriker-GS<br>L chain = Anti-human IL-13 mAb light chain | 42 (=H chain)<br>13 (=L chain) |
| 586H-ELQLE | H chain = Anti-human IL-13 mAb heavy chain-GS-ELQLEESCAEAQDGELDG linker-GS<br>L chain = Anti-human IL-13 mAb light chain | 43 (=H chain)<br>13 (=L chain) |

The mAbdAbs set out in table 2 were expressed in one or both of CHOK1 or CHOE1a cell supernatants, purified and analysed in a number of IL-13 and IL-4 activity assays.

Table 2

| Name | Description | Sequence ID No. |
|---|---|---|
| 586H-TVAAPS-25 | H chain = Anti-human IL-13 mAb heavy chain-TVAAPS linker-GS-DOM9-155-25 dAb<br>L chain = Anti-human IL-13 mAb light chain | 24 (=H chain) 13 (=L chain) |

(continued)

| Name | Description | Sequence ID No. |
|---|---|---|
| 586H-TVAAPS-154 | H chain = Anti-human IL-13 mAb heavy chain-TVAAPS linker-GS-DOM9-155-154 dAb<br>L chain = Anti-human IL-13 mAb light chain | 26 (=H chain) 13 (=L chain) |
| 586H-TVAAPS-210 | H chain = Anti-human IL-13 mAb heavy chain-TVAAPS linker-GS-DOM9-112-210 dAb<br>L chain = Anti-human IL-13 mAb light chain | 27 (=H chain) 13 (=L chain) |

**1.3 Anti-IL4mAb-anti-IL13dAbs**

Bispecific anti-IL4mAb-anti-IL13dAbs were constructed as described above. A number of different linkers were used to join the anti-IL13 domain antibody to the monoclonal antibody. Some constructs had no linker.

Note that a BamHI cloning site (which codes for amino acid residues G and S) was used to clone the linkers and dAbs either to CH3 of the mAb heavy chain or to CK of the mAb light chain. Thus in addition to the given linker sequence, additional G and S amino acid residues are present between the linker sequence and the domain antibody for both heavy chain and light chain expression constructs or between CH3 and the linker sequence in some but not all heavy chain expression constructs. However, when the G4S linker was placed between the mAb and dAb in the mAbdAb format, the BamHI cloning site was already present (due to the G and S amino acid residues inherent within the G4S linker sequence) and thus additional G and S amino acid residues were not present between CH3 or CK and the domain antibody in the constructs using this linker. When no linker sequence was between used n the mAb and dAb in the mAbdAb format, the BamHI cloning site (and hence the G and S amino acid residues) was still present between CH3 or CK and the domain antibody. Full details on the amino acid sequences of mAbdAb heavy and light chains are set out in table 3.

The mAbdAbs set out in table 3 were expressed transiently in CHOK1 cell supernatants. Following mAbdAb quantification these mAbdAb containing supernatants were analysed for activity in IL-13 and IL-4 binding ELISAs.

Table 3

| Name | Description | Sequence ID No. |
|---|---|---|
| PascoH-474 | H chain = Pascolizumab heavy chain-GS-DOM10-53-474 dAb<br>L chain = Pascolizumab light chain | 48 (=H chain)<br>15 (=L chain) |
| PascoH-G4S-474 | H chain = Pascolizumab heavy chain-G4S linker-DOM10-53-474 dAb<br>L chain = Pascolizumab light chain | 49 (=H chain)<br>15 (=L chain) |
| PascoH-TVAAPS-474 | H chain = Pascolizumab heavy chain-TVAAPS linker-GS-DOM10-53-474 dAb<br>L chain = Pascolizumab light chain | 50 (=H chain)<br>15 (=L chain) |
| PascoH-ASTKG-474 | H chain = Pascolizumab heavy chain-GS-ASTKGPT linker-GS-DOM10-53-474 dAb<br>L chain = Pascolizumab light chain | 51 (=H chain)<br>15 (=L chain) |

(continued)

| Name | Description | Sequence ID No. |
|---|---|---|
| PascoH-EPKSC-474 | H chain = Pascolizumab heavy chain-GS-EPKSCDKTHTCPPCP linker-GS-DOM10-53-474 dAb<br><br>L chain = Pascolizumab light chain | 52 (=H chain)<br>15 (=L chain) |
| PascoH-ELQLE-474 | H chain = Pascolizumab heavy chain-GS-ELQLEESCAEAQDGELDG linker-GS-DOM10-53-474 dAb<br><br>L chain = Pascolizumab light chain | 53 (=H chain)<br>15 (=L chain) |
| PascoL-474 | H chain = Pascolizumab heavy chain<br><br>L chain = Pascolizumab light chain-GS-DOM10-53-474 dAb | 14 (=H chain)<br>54 (=L chain) |
| PascoL-G4S-474 | H chain = Pascolizumab heavy chain<br><br>L chain = Pascolizumab light chain-G4S linker-DOM10-53-474 dAb | 14 (=H chain)<br>55 (=L chain) |
| PascoL-TVAAPS-474 | H chain = Pascolizumab heavy chain<br><br>L chain = Pascolizumab light chain-TVAAPS linker-GS-DOM 10-53-474 dAb | 14 (=H chain)<br>56 (=L chain) |
| PascoL-ASTKG-474 | H chain = Pascolizumab heavy chain<br><br>L chain = Pascolizumab light chain-ASTKGPT linker-GS-DOM10-53-474 dAb | 14 (=H chain)<br>57 (=L chain) |
| PascoL-EPKSC-474 | H chain = Pascolizumab heavy chain<br><br>L chain = Pascolizumab light chain-EPKSCDKTHTCPPCP linker-GS-DOM10-53-474 dAb | 14 (=H chain)<br>58 (=L chain) |
| PascoL-ELQLE-474 | H chain = Pascolizumab heavy chain<br><br>L chain = Pascolizumab light chain-ELQLEESCAEAQDGELDG linker-GS-DOM10-53-474 dAb | 14 (=H chain)<br>59 (=L chain) |

The mAbdAbs set out in Table 4 were expressed in one or more of CHOK1, CHOE1a or HEK293-6E cells.

Table 4

| Name | Description | Sequence ID No. |
|---|---|---|
| PascoH-G4S-474 | H chain = Pascolizumab heavy chain-G4S linker-DOM10-53-474 dAb<br><br>L chain = Pascolizumab light chain | 49 (=H chain)<br>15 (=L chain) |
| PascoH-474 | H chain = Pascolizumab heavy chain-GS-DOM10-53-474 dAb<br><br>L chain = Pascolizumab light chain | 48 (=H chain)<br>15 (=L chain) |
| PascoL-G4S-474 | H chain = Pascolizumab heavy chain | 14 (=H chain) |

(continued)

| Name | Description | Sequence ID No. |
|---|---|---|
| | L chain = Pascolizumab light chain-G4S linker-DOM10-53-474 dAb | 55 (=L chain) |
| PascoHL-G4S-474 | H chain = Pascolizumab heavy chain-G4S linker-DOM10-53-474 dAb<br><br>L chain = Pascolizumab light chain-G4S linker-DOM10-53-474 dAb | 49 (=H chain)<br>55 (=L chain) |

**1.4 Sequence ID numbers for monoclonal antibodies, domain antibodies and linkers**

Sequence IDs numbers for the monoclonal antibodies (mAb), domain antibodies (dAb) and linkers used to generate the mAbdAbs are shown below in table 5.

Table 5

| Name | Specificity | Sequence ID |
|---|---|---|
| Anti-human IL-13 monoclonal antibody (also known as 586) | Human IL-13 | 12 (H chain)<br>13 (L chain) |
| Anti-human IL-4 monoclonal antibody (also known as Pascolizumab) | Human IL-4 | 14 (H chain)<br>15 (L chain) |
| DOM10-53-474 domain antibody | Human IL-13 | 5 |
| Anti-human IL-13 monoclonal antibody (also known as 656) | Human IL-13 | 161 (H chain)<br>156 (L chain) |
| DOM9-112-210 domain antibody | Human IL-4 | 4 |
| DOM10-53-616 domain antibody | Human IL-13 | 148 |
| DOM9-155-25 domain antibody | Human IL-4 | 1 |
| DOM9-155-147 domain antibody | Human IL-4 | 2 |
| DOM9-155-154 domain antibody | Human IL-4 | 3 |
| ASTKGPS linker sequence | Derived from human IgG1 H chain (VH-CH1) | 9 |
| ASTKGPT linker sequence | Derived from human IgG1 H chain (VH-CH1), where the last amino acid residue in the native sequence (S) has been substituted for T | 8 |
| EPKSCDKTHTCPPCP linker sequence | Derived from human IgG1 H chain (CH1-CH2) | 10 |
| TVAAPS linker sequence | Derived from human K L chain (VL-CK) | 7 |
| ELQLEESCAEAQDGELDG linker sequence | Derived from human IgG1 CH3 tether | 11 |
| GGGGS linker sequence | A published linker sequence | 6 |

Mature human IL-13 amino acid sequence (without signal sequence) is given in SEQ ID NO: 63.

Mature human IL-4 amino acid sequence (without signal sequence) is given in SEQ ID NO: 62.

**1.5 Expression and purification of mAbdAbs**

The mAbdAb expression constructs described in Example 1 were transfected into one or more of CHOK1 cells, CHOE1a cells or HEK293-6E cells, expressed at small (approximately 3mls) or medium (approximately 50mls to 100mls) or large (approximately 1 litre) scale and then some of the constructs were purified using immobilised Protein A columns and quantified by reading absorbance at 280nm.

### 1.6 Size exclusion chromatography analyses of purified mAbdAbs

A number of mAbdAbs were analysed by size exclusion chromatography (SEC) and sodium dodecyl sulphate polyacrylamide gel electrophoresis (SDS PAGE). Representative data for some of these molecules (PascoH-G4S-474, PascoL-G4S-474, PascoH-474 and PascoHL-G4S-474.) are shown in Figures 9, 10, 11 and 12 respectively. Representative data showing SEC and SDS Page analysis for these molecules with the 'GS' motif removed are shown in Figures 90-98.

In some cases SEC was used to further purify these molecules to remove aggregates.

## Example 2

## Binding of mAbdAbs to human IL-13 and human IL-4 by ELISA

### 2.1 Binding of anti-IL13mAb-anti-IL4dAbs to IL-13 and IL-4

mAbdAb supernatants, were tested for binding to human IL-13 in a direct binding ELISA (as described in method 1). These data are shown in Figure 13.

Figure 13 shows that all of these anti-IL13mAb-anti-IL4dAbs bound IL-13. The binding activity of these mAbdAbs was also approximately equivalent (within 2-fold to 3-fold) to purified anti-human IL13 mAb alone, which was included in this assay as a positive control for IL-13 binding and in order to directly compare to the mAbdAbs. Purified anti-human IL4 mAb (Pascolizumab) was included as a negative control for IL-13 binding.

These molecules were also tested for binding to human IL-4 in a direct binding ELISA (as described in method 2). These data are shown in Figure 14.

Figure 14 shows that all of these anti-IL13mAb-anti-IL4dAbs bound IL-4, but some variation in IL-4 binding activity was observed. No binding to IL-4 was observed when no anti-IL4 dAb was present in the mAbdAb construct. Purified anti-human IL13 mAb was also included as a negative control for binding to IL-4. Note that the anti-IL-4 dAbs alone were not tested in this assay as the dAbs are not detected by the secondary detection antibody; instead, purified anti-human IL4 mAb (Pascolizumab) was used as a positive control to demonstrate IL-4 binding in this assay.

Purified samples of mAbdAbs, were also tested for binding to human IL-13 in a direct binding ELISA (as described in method 1). These data are shown in Figure 15. These purified anti-IL13mAb-anti-IL4dAbs bound IL-13. The binding activity of these mAbdAbs for IL-13 was equivalent to that of purified anti-human IL13 mAb alone. An isotype-matched mAb (with specificity for an irrelevant antigen) was also included as a negative control for binding to IL-13 in this assay.

These purified mAbdAbs were also tested for binding to human IL-4 in a direct binding ELISA (as described in method 2). These data are shown in Figure 16.

All of these anti-IL13mAb-anti-IL4dAbs bound IL-4. Note that the anti-IL-4 dAbs alone were not tested in this assay as the dAbs are not detected by the secondary detection antibody; instead, purified anti-human IL4 mAb (Pascolizumab) was used as a positive control to demonstrate IL-4 binding in this assay. An isotype-matched mAb (with specificity for an irrelevant antigen) was also included as a negative control for binding to IL-4 in this assay.

### 2.2 Binding of anti-IL4mAb-anti-IL13dAbs to IL-13 and IL-4

mAbdAb supernatants were tested for binding to human IL-4 in a direct binding ELISA (as described in method 2). These data are shown in Figure 17 (some samples were prepared and tested in duplicate and this has been annotated as sample 1 and sample 2).

Figure 17 shows that all of these mAbdAbs bound IL-4. Purified anti-human IL4 mAb alone (Pascolizumab) was included in this assay but did not generate a binding curve as an error was made when diluting this mAb for use in the assay (Pascolizumab has been used successfully in all other subsequent IL-4 binding ELISAs). Purified anti-human IL13 mAb was included as a negative control for IL-4 binding.

The same mAbdAb supernatants were also tested for binding to human IL-13 in a direct binding ELISA (as described in method 1). These data are shown in Figure 18 (some samples were prepared and tested in duplicate and this has been annotated as sample 1 and sample 2).

Figure 18 shows that all of these anti-IL4mAb-anti-IL13dAbs bound IL-13. Purified anti-human IL13 mAb alone was included in this assay but did not generate a binding curve as an error was made when diluting this mAb for use in the assay (purified anti-human IL 13 mAb has been used successfully in all other subsequent IL-13 binding ELISAs). Purified anti-IL4 mAb (Pascolizumab) was included as a negative control for binding to IL-13. Note that the anti-IL-13 dAb alone (DOM10-53-474) was not tested in this assay as this dAb is not detected by the secondary detection antibody.

The purified anti-IL4mAb-anti-IL13dAbs, 'PascoH-G4S-474', 'PascoH-474', 'PascoL-G4S-474' and 'PascoHL-G4S-474', were also tested for binding to human IL-4 in a direct binding ELISA (as described in method 2). These data are shown in Figure 19.

These purified anti-IL4mAb-anti-IL13dAbs bound IL-4. The binding activity of these mAbdAbs was approximately equivalent (within 2-fold) to purified anti-IL4 mAb alone (Pascolizumab). An isotype-matched mAb (with specificity for an irrelevant antigen) was also included as a negative control for binding to IL-4 in this assay.

These same purified anti-IL4mAb-anti-IL13dAbs, PascoH-G4S-474, PascoH-474, PascoL-G4S-474 and PascoHL-G4S-474, were also tested for binding to human IL-13 in a direct binding ELISA (as described in method 1). These data are shown in Figure 20A.

These purified anti-IL4mAb-anti-IL13dAbs bound IL-13. An isotype-matched mAb (with specificity for an irrelevant antigen) was also included as a negative control for binding to IL-13 in this assay. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

Purified PascoH-474, PascoH-TVAAPS-474, PascoH-ASTKG-474 and PascoH-ELQLE-474 were also tested for binding to cynomolgus IL-13 in a direct binding ELISA, as described in method 17 (PascoH-474 GS removed and PascoH-TVAAPS-474 GS removed were also included in this assay, the construction of these molecules is described in Example 18). A graph showing representative data is shown in Figure 20B.

Purified PascoH-474, PascoH-TVAAPS-474, PascoH-ASTKG-474 and PascoH-ELQLE-474 all bound cynomolgus IL-13. Purified anti-human IL4 mAb alone (Pascolizumab) was included in this assay as a negative control for binding to IL-13. Purified anti-human IL13 mAb was included as a positive control for cynomolgus IL-13 binding. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

## Example 3

## Binding of mAbdAbs to human IL-13 and human IL-4 by surface plasmon resonance (BIAcore™)

### 3.1 Binding of anti-IL13mAb-anti-IL4dAbs to IL-13 and IL-4 by BIAcore™

mAbdAbs (in CHO cell supernatants, prepared as described in section 1.5) were tested for binding to human IL-13 using BIAcore™ at 25°C (as described in method 4). For this data set, two IL-13 concentration curves (100nM and 1nM) were assessed and relative response capture levels of between 1000 and 1300 (approximately) were achieved for each mAbdAb construct. Due to the limited number of concentrations of IL-13 used, the data generated are more suitable for ranking of constructs rather than exact kinetic measurements. These data are shown in Table 6.

Table 6

| Antibody | Binding affinity KD (nM) |
|---|---|
| 586H-25 | 0.39 |
| 586H-G4S-25 | 0.41 |
| 586H-TVAAPS-25 | 0.5 |
| 586H-ASTKG-25 | 0.54 |
| 586H-EPKSC-25 | 0.55 |
| 586H-ELQLE-25 | 0.42 |
| 586H-147 | 0.46 |
| 586H-G4S-147 | 0.45 |
| 586H-TVAAPS-147 | 0.56 |
| 586H-ASTKG-147 | 0.44 |
| 586H-EPKSC-147 | 0.46 |
| 586H-ELQLE-147 | 0.51 |

(continued)

| Antibody | Binding affinity KD (nM) |
|---|---|
| 586H-154 | 0.46 |
| 586H-G4S-154 | 0.37 |
| 586H-TVAAPS-154 | 0.56 |
| 586H-ASTKG-154 | 0.44 |
| 586H-EPKSC-154 | 0.42 |
| 586H-ELQLE-154 | 0.44 |
| 586H-210 | 0.44 |
| 586H-G4S-210 | 0.42 |
| 586H-TVAAPS-210 | 0.4 |
| 586H-ASTKG-210 | 0.4 |
| 586H-EPKSC-210 | 0.43 |
| 586H-ELQLE-210 | 0.43 |
| 586H | 0.44 |
| 586H-ASTKG | 0.32 |
| 586H-ELQLE | 0.47 |
| 586H-EPKSC | 0.45 |
| Anti-human IL-13 mAb (purified) | 0.38 |
| Pascolizumab (purified) | no binding |

All of these anti-IL13mAb-anti-IL4dAbs bound IL-13 with similar binding affinities which were approximately equivalent to the binding affinity of purified anti-human IL13 mAb alone. These data suggested that the addition of linkers and/or anti-IL4 dAbs to the heavy chain of the anti-IL13 mAb, did not affect the IL-13 binding affinity of the mAb component within these mAbdAb constructs.

These mAbdAbs were also tested for binding to human IL-4 using BIAcore™ at 25°C (as described in method 5). These data are shown in Table 7. For this data set, four IL-4 concentration curves (512, 128, 32 and 8nM) were assessed and approximate relative response capture levels for each mAbdAb tested are indicated in the table. Note that the anti-IL-4 dAbs alone were not tested in this assay as the dAbs cannot be captured onto the Protein A or anti-human IgG coated CM5 chip; instead, the anti-human IL4 mAb (Pascolizumab) was used as a positive control to demonstrate IL-4 binding in this assay.

Table 7

| Antibody | Capture Level | On rate (ka, $Ms^{-1}$) | Off rate (kd, $s^{-1}$) | Binding affinity KD (nM) |
|---|---|---|---|---|
| 586H-25 | 864 | 6.13e3 | 4.11e-4 | 67 |
| 586H-G4S-25 | 1818 | 6.3e3 | 9.54e-4 | 151 |
| 586H-TVAAPS-25 | 673 | 1.27e5 | 1.2e-4 | 0.95 |
| 586H-ASTKG-25 | 809 | 5.4e5 | 1.20e-3 | 21:8 |
| 586H-EPKSC-25 | 748 | 4.79e4 | 1.42e-3 | 29.6 |
| 586H-ELQLE-25 | 603 | 1.26e6 | 1.63e-6 | 0.001* |
| 586H-147 | 1095 | 3.42e3 | 1.18e-3 | 344.8 |
| 586H-G4S-147 | 1200 | 4.21e3 | 4.57e-4 | 108.5 |
| 586H-TVAAPS-147 | 433 | 6.62e4 | 6.69e-7 | 0.011** |

(continued)

| Antibody | Capture Level | On rate (ka, $Ms^{-1}$) | Off rate (kd, $s^{-1}$) | Binding affinity KD (nM) |
|---|---|---|---|---|
| 586H-ASTKG-147 | 1248 | 3.67e4 | 6.9e-4 | 18.8 |
| 586H-EPKSC-147 | 878 | 2.54e4 | 6.71e-4 | 26.4 |
| 586H-ELQLE-147 | 676 | 7.01e5 | 1.52e-5 | 0.027* |
| 586H-154 | 436 | 6.1e3 | 1.74e-3 | 285 |
| 586H-G4S-154 | 1437 | 5.00e3 | 6.85e-4 | 137.8 |
| 586H-TVAAPS-154 | 1530 | 6.44e4 | 1.15e-7 | 0.002** |
| 586H-ASTKG-154 | 1373 | 3.26e4 | 2.84e-4 | 8.7 |
| 586H-EPKSC-154 | 794 | 3.03e4 | 5.7e-4 | 18.8 |
| 586H-ELQLE-154 | 795 | 1.25e6 | 3.57e-6 | 0.003* |
| 586H-210 | 1520 | not determined | not determined | --- |
| 586H-G4S-210 | 1448 | not determined | not determined | --- |
| 586H-TVAAPS-210 | 1693 | not determined | not determined | --- |
| 586H-ASTKG-210 | 1768 | not determined | not determined | --- |
| 586H-EPKSC-210 | 1729 | not determined | not determined | --- |
| 586H-ELQLE-210 | 1350 | not determined | not determined | --- |
| 586H | 1500 | no binding | no binding | --- |
| 586H-ASTKG | 1615 | no binding | no binding | --- |
| 586H-ELQLE | 343 | no binding | no binding | --- |
| 586H-EPKSC | 1416 | no binding | no binding | --- |
| Pascolizumab (purified) | 1092 | 2.04e6 | 1.23e-4 | 0.060 |

Caveats were observed for some of the above data sets. Poor curve fits were observed for some data sets (*), the actual binding affinity values that have been determined for these data should therefore be treated with caution. Positive dissociation was seen for some curves (**), the actual binding affinity values that have been determined for these data should therefore be treated with caution. In addition, BIAcore™ was unable (ie. not sensitive enough) to determine on and off rates for all mAbdAb constructs containing the DOM9-112-210 dAb, due to the exceptionally tight binding of these mAbdAbs to IL-4. Determination of binding kinetics for these mAbdAbs for IL-4 was further hampered by observed positive dissociation effects. These data are shown in Figure 21.

Similar data was obtained in an additional experiment. These data are shown in Figure 22.

These 2 independent data sets indicated that all of the anti-IL13mAb-anti-IL4dAbs bound IL-4, but the binding affinities varied depending on the linker used to join the anti-IL4 dAb to the anti-IL13 mAb heavy chain. In this experiment, the presence of a linker was found to enhance the binding affinity for IL-4 of the anti-IL4 dAb component (when placed on the heavy chain) in the mAbdAb format. For example the molecules having TVAAPS or ELQLEESCAEAQDGELDG linkers appear to be more potent binders. No binding to IL-4 was observed when no anti-IL4 dAb was present in the mAbdAb construct. It was not possible to measure the binding affinity of the 586-linker-210 mAbdAbs for IL-4, due to the fact that the DOM9-112-210 component of these mAbdAbs binds very tightly and hence the off-rate is too small to determine using BIAcore™.

Purified anti-IL13mAb-anti-IL4dAbs were also tested for binding to human IL-13 and human IL-4 using BIAcore™ at 25°C (as described in methods 4 and 5). These data are shown in Table 8.

Table 8

| Construct | Binding affinity, KD (nM) | |
|---|---|---|
| | Human IL-13 | Human IL-4 |
| 586H-TVAAPS-25 | 0.38 | 1.1 |

(continued)

| Construct | Binding affinity, KD (nM) | |
|---|---|---|
| | Human IL-13 | Human IL-4 |
| 586H-TVAAPS-154 | 0.41 | 0.49 |
| 586H-TVAAPS-210 | 0.38 | very tight binder (unable to determine KD due to positive dissociation effects and sensitivity level of BIAcore™ technique) |
| Anti-human IL-13 mAb (purified) | 0.43 | --- |
| Pascolizumab (purified) | --- | 0.03 |

586H-TVAAPS-25, 586H-TVAAPS-154 and 586H-TVAAPS-210 all bound IL-13 with similar binding affinities and this was approximately equivalent to the binding affinity of purified anti-human IL13 mAb alone. 586H-TVAAPS-25, 586H-TVAAPS-154 and 586H-TVAAPS-210 all bound IL-4. It was not possible to measure the binding affinity of 586-TVAAPS-210 for IL-4, due to the fact that the DOM9-112-210 component of this mAbdAb bound very tightly and hence the off-rate was too small to determine using BIAcore™. Note that the anti-IL-4 dAbs alone (DOM9-155-25, DOM9-155-154 and DOM9-112-210) were not tested in this assay format as the dAbs cannot be captured onto the Protein A or anti-human IgG coated CM5 chip; instead, the anti-human IL4 mAb (Pascolizumab) was used as a positive control to demonstrate IL-4 binding in this assay.

**3.2 Binding of anti-IL4mAb-anti-IL13dAbs to IL-4 and IL-13 by BIAcore™**

mAbdAbs (in CHO cell supernatants prepared as described in section 1.5).were tested for binding to human IL-4 using BIAcore™ at 25°C (as described in method 5). These data are shown in Table 9 (some samples were prepared and tested in duplicate - this has been annotated as sample 1 and sample 2). For this data set, four IL-4 concentrations curves (100nM, 10nM, 1nM and 0.1nM) were assessed and approximate relative response capture levels for each mAbdAb tested are indicated

in the table. An isotype-matched mAb (with specificity for an irrelevant antigen) was also included as a negative control for binding to IL-4 in this assay.

Table 9

| Antibody | Capture Level | On rate (ka, $Ms^{-1}$) | Off rate (kd, $s^{-1}$) | Binding affinity KD (nM) |
|---|---|---|---|---|
| **Experiment 1** | | | | |
| PascoH-G4S-474 | -500 | 5.1e6 | 8.6e-5 | 0.02 |
| PascoH-TVAAPS-474 | -500 | 5.5e6 | 9.7e-5 | 0.02 |
| PascoH-474 | -500 | 4.8e6 | 9.4e-5 | 0.02 |
| PascoH-ASTKG-474 | ~500 | 5.3e6 | 8.6e-5 | 0.02 |
| PascoH-ELQLE-474 | -500 | 5.1e6 | 1.1e-4 | 0.02 |
| PascoH-EPKSC-474 | -500 | 4.9e6 | 9.8e-5 | 0.02 |
| Pascolizumab (purified) | -700 | 5.3e6 | 1.6e-4 | 0.03 |
| **Experiment 2** | | | | |
| PascoL-G4S-474 (sample 1) | 1871 | 2.14e6 | 1.35e-4 | 0.063 |
| PascoL-G4S-474 (sample 2) | 1921 | 2.13e6 | 1.11e-4 | 0.052 |
| PascoL-TVAAPS-474 (sample 1) | 2796 | 2.48e6 | 2.12e-4 | 0.085 |
| PascoL-TVAAPS-474 (sample 2) | 3250 | 3.04e6 | 2.79e-4 | 0.092 |
| PascoL-474 (sample 1) | 3254 | 2.8e6 | 1.84e-4 | 0.065 |

(continued)

| | | | | |
|---|---|---|---|---|
| **Experiment 2** | | | | |
| PascoL-474 (sample 2) | 2756 | 2.53e6 | 1.22e-4 | 0.048 |
| PascoL-ASTKG-474 (sample 1) | 3037 | 2.95e6 | 1.21e-4 | 0.041 |
| PascoL-ASTKG-474 (sample 2) | 3784 | 2.54e6 | 1.52e-4 | 0.060 |
| PascoL-EPKSC-474 (sample 1) | 3238 | 1.86e6 | 2.58e-4 | 0.139 |
| PascoL-EPKSC-474 (sample 2) | 3276 | 2.51e6 | 3.18e-4 | 0.127 |
| Pascolizumab (purified) | 1152 | 2.04e6 | 1.23e-4 | 0.060 |
| Negative control mAb | 2976 | no binding | no binding | --- |

All of the anti-IL4mAb-anti-IL13dAbs tested bound IL-4 with similar binding affinities and this was approximately equivalent to the binding affinity of the anti-human IL4 mAb alone (Pascolizumab). PascoL-EPKSC-474 bound IL-4 approximately 2-fold less potently than Pascolizumab.These data suggested that the addition of linkers and the anti-IL13 dAb to either the heavy chain or the light chain of Pascolizumab, did not overtly affect the IL-4 binding affinity of the mAb component within the mAbdAb construct.

These mAbdAbs were also tested for binding to human IL-13 using BIAcore™ at 25°C (as described in method 4). These data are shown in Table 10 (some samples were prepared and tested in duplicate - this has been annotated as sample 1 and sample 2). For this data set, four IL-13 concentrations curves (128nM, 32nM, 8nM and 2nM) were assessed and approximate relative response capture levels for each mAbdAb tested are indicated in the table.

Table 10

| Antibody | Capture Level | On rate (ka, $Ms^{-1}$) | Off rate (kd, $s^{-1}$) | Binding affinity KD (nM) |
|---|---|---|---|---|
| **Experiment 1** | | | | |
| PascoH-474 | -500 | 3.6e5 | 3.1e-4 | 0.84 |
| PascoH-G4S-474 | ~500 | 3.9e5 | 2.6e-4 | 0.67 |
| PascoH-TVAAPS-474 | -500 | 4.5e5 | 4.2e-4 | 0.94 |
| PascoH-ASTKG-474 | -500 | 3.1e5 | 4.6e-4 | 1.5 |
| PascoH-ELQLE-474 | ~500 | 3.4e5 | 6.2e-4 | 1.8 |
| PascoH-EPKSC-474 | -500 | 3.5e5 | 4.0e-4 | 1.1 |
| Anti-human IL-13 mAb (purified) | ~650 | 8.6e5 | 4.9e-4 | 0.57 |
| **Experiment 2** | | | | |
| PascoL-474 (sample 1) | 3254 | 2.86e5 | 3.82e-4 | 1.34 |
| PascoL-474 (sample 2) | 2756 | 3.12e5 | 3.86e-4 | 1.24 |
| PascoL-G4S-474 (sample 1) | 1871 | 5.63e5 | 4.25e-4 | 0.756 |
| PascoL-G4S-474 (sample 2) | 1921 | 5.59e5 | 3.47e-4 | 0.621 |
| PascoL-TVAAPS-474 (sample 1) | 2796 | 7.42e5 | 2.58e-4 | 0.348 |
| PascoL-TVAAPS-474 (sample 2) | 3250 | 6.22e5 | 1.71e-4 | 0.275 |
| PascoL-ASTKG-474 (sample 1) | 3037 | 5.26e5 | 2.38e-4 | 0.451 |
| PascoL-ASTKG-474 (sample 2) | 3784 | 5.38e5 | 3.20e-4 | 0.595 |
| PascoL-EPKSC-474 (sample 1) | 3238 | 4.17e5 | 3.34e-4 | 0.801 |
| PascoL-EPKSC-474 (sample 2) | 3276 | 3.51e5 | 2.86e-4 | 0.815 |
| Anti-human IL-13 mAb (purified) | 1373 | 9.12e4 | 6.11e-4 | 0.67 |
| Pascolizumab (purified) | 1152 | no binding | no binding | --- |

(continued)

| Experiment 2 | | | | |
|---|---|---|---|---|
| Negative control mAb | 2976 | no binding | no binding | --- |

Binding affinity data for constructs tested in experiment 2 are also shown in figure 23.

All of the anti-IL4mAb-anti-IL13dAbs bound IL-13. in most cases the presence of a linker did not appear to enhance the binding affinity for IL-13 of the anti-IL13 dAb component when placed on the heavy chain of the anti-IL4 mAb. However, the presence of a linker did appear to enhance the binding affinity for IL-13 of the anti-IL13 dAb component when placed on the light chain of the anti-IL4 mAb. PascoL-TVAAPS-474 appeared to have the most potent IL-13 binding affinity in this experiment.

Note that the anti-IL-13 dAb alone (DOM10-53-474) was not tested in this assay as the dAb cannot be captured onto the Protein A or anti-human IgG coated CM5 chip; instead, purified anti-human IL13 mAb was used as a positive control to demonstrate IL-13 binding in this assay. An isotype-matched mAb (with specificity for an irrelevant antigen) was also included as a negative control for binding to IL-13 in this assay.

Purified anti-IL4mAb-anti-IL13dAbs were also tested for binding to human IL-4 and human IL-13 using BIAcore™ at 25°C (as described in methods 4 and 5). These data are shown in Table 11.

Table 11

| Construct | Binding affinity, KD (nM) | |
|---|---|---|
| | Human IL-4 | Human IL-13 |
| PascoH-G4S-474 | 0.036 | 0.58 |
| PascoH-474 | 0.037 | 0.71 |
| PascoL-G4S-474 | 0.028 | 1.2 |
| PascoHL-G4S-474 | 0.035 | 0.87 |
| Anti-human IL-13 mAb (purified) | --- | 0.41 |
| Pascolizumab (purified) | 0.037 | --- |

In this experiment PascoH-G4S-474, PascoH-474, PascoL-G4S-474 and PascoHL-G4S-474 all bound IL-4 with similar binding affinities and this was approximately equivalent to the binding affinity of the anti-human IL4 mAb alone (Pascolizumab). They also all bound IL-13. Note that the anti-IL-13 dAb alone (DOM10-53-474) was not tested in this assay as the dAb cannot be captured onto the Protein A or anti-human IgG coated CM5 chip; instead, the anti-human IL13 mAb was used as a positive control to demonstrate IL-13 binding in this assay.

### 3.3 Stoichiometry of binding of IL-13 and IL-4 to the anti-IL4mAb-anti-IL13dAbs using BIAcore™

Purified anti-IL4mAb-anti-IL13dAbs were evaluated for stoichiometry of binding for IL-13 and IL-4 using BIAcore™ (as described in method 7). These data are shown in Table 12.

Table 12

| Construct | Stoichiometry | |
|---|---|---|
| | Human IL-4 | Human IL-13 |
| PascoL-G4S-474 | 1.8 | 1.8 |
| PascoH-G4S-474 | 1.8 | 1.9 |
| Pasco-474 | 1.8 | 1.9 |
| PascoHL-G4S-474 | 1.7 | 3.5 |
| Anti-human IL-13 mAb (purified) | --- | 1.8 |
| Pascolizumab (purified) | 1.8 | --- |

PascoH-G4S-474, PascoH-474 and PascoL-G4S-474 were able to bind to nearly two molecules of IL-13 and two molecules of IL-4. PascoHL-G4S-474 was able to bind nearly two molecules of IL-4 and nearly four molecules of IL-13. These data indicated that the constructs tested could be fully occupied by the expected number of IL-13 or IL-4 molecules.

## Example 4

## Neutralisation potency of mAbdAbs in IL-13 and IL-4 bioassays

### 4.1 Anti-IL13mAb-anti-IL4dAbs

Purified anti-IL13mAb-anti-IL4dAbs were tested for neutralisation of human IL-13 in a TF-1 cell bioassay (as described in method 8). These data are shown in Figure 24.

Purified anti-IL13mAb-anti-IL4dAbs, 586H-TVAAPS-25, 586H-TVAAPS-154 and 586H-TVAAPS-210, fully neutralised the bioactivity of IL-13 in a TF-1 cell bioassay. The neutralisation potencies of these mAbdAbs were within 2-fold of purified anti-human IL-13 mAb alone. The purified anti-human IL-4 mAb (Pascolizumab) and purified anti-IL4 dAbs (DOM9-155-25, DOM9-155-154 or DOM9-112-210) were included as negative controls for neutralisation of IL-13 in this assay.

The purified anti-IL13mAb-anti-IL4dAbs, 586H-TVAAPS-25, 586H-TVAAPS-154 and 586H-TVAAPS-210, were also tested for neutralisation of human IL-4 in a TF-1 cell bioassay (as described in method 9). These data are shown in Figure 25.

586H-TVAAPS-210 fully neutralised the bioactivity of IL-4 in this TF-1 cell bioassay. The neutralisation potency of this mAbdAb was within 2-fold of purified anti-human IL-4 dAb alone (DOM9-112-210): 586H-TVAAPS-25 and 586H-TVAAPS-154 did not neutralise the bioactivity of IL-4 and this was in contrast to the purified anti-human IL-4 dAbs alone (DOM9-155-25 and DOM9-155-154). As demonstrated by BIAcore™, purified 586H-TVAAPS-25 and 586H-TVAAPS-154 had 1.1 nM and 0.49nM binding affinities (respectively) for IL-4. IL-4 binds the IL-4 receptor very tightly (binding affinities of approximately 50pM have been reported in literature publications) and thus the observation that both 586H-TVAAPS-25 or 586H-TVAAPS-154 were unable to effectively neutralise the bioactivity of IL-4 in the TF-1 cell bioassay maybe a result of the relative lower affinity of these mAbdAbs for IL-4 compared to the potency of IL-4 for the IL-4 receptor.

Purified anti-human IL-4 mAb (Pascolizumab) was included as a positive control for neutralisation of IL-4 in this bioassay. Purified anti-human IL-13 mAb was included as a negative control for neutralisation of IL-4 in this bioassay.

### 4.2 Anti-IL4mAb-anti-IL13dAbs

The purified anti-IL4mAb-anti-IL13dAbs, PascoH-G4S-474, PascoH-474, PascoL-G4S-474 and PascoHL-G4S-474, were tested for neutralisation of human IL-4 in a TF-1 cell bioassay (as described in method 9). These data are shown in Figure 26.

Purified anti-IL4mAb-anti-IL13dAbs, PascoH-G4S-474, PascoH-474, PascoL-G4S-474 and PascoHL-G4S-474, fully neutralised the bioactivity of IL-4 in a TF-1 cell bioassay. The neutralisation potencies of these mAbdAbs were approximately equivalent to that of purified anti-human IL4 mAb alone (Pascolizumab), Purified anti-human IL-13 mAb, purified DOM10-53-474 dAb and a dAb with specificity for an irrelevant antigen (negative control dAb) were also included as negative controls for neutralisation of IL-4 in this bioassay.

The purified anti-IL4mAb-anti-IL13dAbs, PascoH-G4S-474, PascoH-474, PascoL-G4S-474 and PascoHL-G4S-474, were tested for neutralisation of human IL-13 in a TF-1 cell bioassay (as described in method 8). These data are shown in (Figure 27.

Purified anti-IL4mAb-anti-IL13dAbs, PascoH-G4S-474, PascoH-474, PascoL-G4S-474 and PascoHL-G4S-474, fully neutralised the bioactivity of IL-13 in a TF-1 cell bioassay. The neutralisation potencies of these mAbdAbs were within 3-fold of purified anti-IL13 dAb alone (DOM10-53-474). Purified anti-human IL-13 mAb was also included as a positive control for IL-13 neutralisation in this bioassay. A dAb with specificity for an irrelevant antigen (negative control dAb) and purified anti-human IL4 mAb alone (Pascolizumab) were also included as negative controls for neutralisation of IL-4 in this bioassay.

The purified anti-IL4mAb-anti-IL13dAbs, PascoH-G4S-474, PascoH-474, PascoL-G4S-474 and PascoHL-G4S-474, were also tested for simultaneous neutralisation of human IL-4 and human IL-13 in a dual neutralisation TF-1 cell bioassay (as described in method 10). These data are shown in Figure 28.

Purified anti-IL4mAb-anti-IL13dAbs, PascoH-G4S-474, PascoH-474, PascoL-G4S-474 and PascoHL-G4S-474, fully neutralised the bioactivity of both IL-4 and IL-13 in a dual neutralisation TF-1 cell bioassay. The neutralisation potencies of these mAbdAbs were approximately equivalent to that of a combination of purified anti-human IL4 mAb (Pascolizumab) and purified anti-IL13 dAb (DOM10-53-474). Purified anti-human IL-13 mAb alone, purified anti-human IL-4 mAb alone (Pascolizumab) and the anti-human IL-13 dAb (DOM10-53-474) alone (which were included as negative controls) did not fully neutralise the bioactivity of both IL-4 and IL-13 in this dual IL-4 and IL-13 neutralisation bioassay.

## Example 5

## SEC-MALLS analysis of dAbs

Antigen-specific dAbs were characterized for their solution state by SEC-MALLS (size-exclusion chromatography - multi-angle laser light scattering) and the results are shown in Table 13: the DOM10-53-474, dAb exists as a monomer in solution whilst all DOM9 dAbs (DOM9-112-210, DOM9-155-25, DOM9-155-147 and DOM9-155-154) can form stable dimers (and in some instances tetramers).

### 5.1. Preparation of the proteins

Samples were purified and dialysed into appropriate buffer e.g. PBS. Samples were filtered after dialysis and the concentration determined (0.43mg/ml DOM-155-25), (1.35mg/ml DOM9-155-147) and 1.4mg/ml DOM9-155-159). DOM10-53-474 and DOM9-112-210 were adjusted to 1mg/ml.

BSA was purchased from Sigma and used without further purification.

### 5.2. Size-Exclusion chromatography and Detector Set-up

Shimadzu LC-20AD Prominence HPLC system with an autosampler (SIL-20A) and SPD-20A Prominence UV/Vis detector was connected to Wyatt Mini Dawn Treos (MALLS, multi-angle laser light scattering detector) and Wyatt Optilab rEX DRI (differential refractive index) detector. The detectors were connected in the following order - LS-UV-RI. Both RI and LS instruments operated at a wavelength of 488nm. TSK2000 (Tosoh corporation)) column were used (silica-based HPLC column) with mobile phase of 50mM phosphate buffer (without salt), or 1xPBS, both at pH7.4. The flow rate used is 0.5 or 1ml/min, the time of the run was adjusted to reflect different flow rates (45 or 23 min) and was not expected to have significant impact onto separation of the molecules. Proteins were prepared in buffer to a concentration of 1mg/ml and injection volume was 100ul.

### 5.3. Detector Calibration

The light-scattering detector was calibrated with toluene according to manufacturer’s instructions.

### 5.4. Detector Calibration with BSA

The UV detector output and RI detector output were connected to the light scattering instrument so that the signals from all three detectors could be simultaneously collected with the Wyatt ASTRA software. Several injections of BSA in a mobile phase of PBS (1ml/min) are run over a Tosoh TSK2000 column with UV, LS and RI signals collected by the Wyatt software. The traces are then analysed using ASTRA software, and the signals are normalised aligned and corrected for band broadening following manufacturer’s instructions. Calibration constants are then averaged and input into the template which is used for future sample runs.

### 5.5. Absolute molar mass calculations

100ul of each sample were injected onto appropriate pre-equilibrated column. After SEC column the sample passes through 3 on-line detectors - UV, MALLS (multi-angle laser light scattering) and DRI (differential refractive index) allowing absolute molar mass determination. The dilution that takes place on the column is about 10 fold, and the concentration at which in-solution state was determined as appropriate.

The basis of the calculations in ASTRA as well as of the Zimm plot technique, which is often implemented in a batch sample mode is the equation from Zimm [J. Chem. Phys. 16, 1093-1099 (1948)]:

$$\frac{R_\theta}{K^* c} = MP(\theta) - 2A_2 c M^2 P^2(\theta) \quad \text{(Eq. 1)}$$

where

- c is the mass concentration of the solute molecules in the solvent (g/mL)
- *M* is the weight average molar mass (g/mol)
- $A_2$ is the second virial coefficient (mol mL / $g^2$)
- $K^* = 4p^2\ n_0^2\ (dn/dc)^2\ l_0^{-4}\ N_A^{-1}$ is an optical constant where no is the refractive index of the solvent at the incident radiation (vacuum) wavelength, $l_0$ is the incident radiation (vacuum) wavelength, expressed in nanometers, $N_A$ is Avogadro's number, equal to 6.022 x $10^{23}$ $mol^{-1}$, and *dn*/*dc* is the differential refractive index increment of the solvent-solute solution with respect to a change in solute concentration, expressed in mL/g (this factor must be measured independently using a dRI detector).
- P(q) is the theoretically-derived form factor, approximately equal to $1\text{-}2\mu^2(r^2)/31+\ldots$ , where $\mu=(4\pi/\lambda)\sin(\theta/2)$, and $\langle r^2 \rangle$ is the mean square radius. P(q) is a function of the molecules' z-average size, shape, and structure.
- $R_q$ is the excess Rayleigh ratio ($cm^{-1}$)

This equation assumes vertically polarized incident light and is valid to order $c^2$.

To perform calculations with the Zimm fit method, which is a fit to $R_q / K^* c$ vs. $\sin^2(q/2)$, we need to expand the reciprocal of Eq. 1 first order in c:

To perform calculations with the Zimm fit method, which is a fit to Rq /K*c vs. sin2(q/2), we need to expand the reciprocal of Eq. 1 to first order in c:

$$\frac{K^* c}{R_\theta} = \frac{1}{MP(\theta)} + 2A_2 c \quad \text{Eq. 2}$$

The appropriate results in this case are

$$M = \left(\frac{K^* c}{R_0} - 2A_2 c\right)^{-1} \quad \text{Eq. 3}$$

and

$$\langle r^2 \rangle = \frac{3 m_0 \lambda^2 M}{16\pi^2} \quad \text{Eq. 4}$$

where

$$m_0 = d\left[K^* c / R_\theta\right] / d\left[\sin^2(\theta/2)\right]_{\theta \to 0} \quad \text{Eq. 5}$$

The calculations are performed automatically by ASTRA software, resulting in a plot with molar mass determined for each of the slices [Astra manual].

Molar mass obtained from the plot for each of the peaks observed on chromatogram was compared with expected molecular mass of a single unit of the protein. This provides a basis to form conclusions about in-solution state of the protein. Representative data is shown in Table 13.

**Table 13: Summary**

| dAb | SEC-MALLS | MW | Column & mobile phase |
|---|---|---|---|
| DOM10-53-474 | monomer | 14kDa | TSK2000, PBS, pH7.4, 0.5ml/min |
| DOM9-112-210 | dimer | 30kDa | TSK2000, PBS, pH7.4, 0.5ml/min |
| DOM9-155-25 | dimer | 28kDa | TSK2000, 50mM, phosphate buffer pH7.4, 1ml/min |

(continued)

| dAb | SEC-MALLS | MW | Column & mobile phase |
|---|---|---|---|
| DOM9-155-147 | dimer-tetramer equilibrium | 26-51kDa | TSK2000, 50mM phosphate buffer, pH7.4, 1ml/min |
| DOM9-155-159 | dimer | 28kDa | TSK2000, 50mM phosphate Buffer, pH7.4, 1ml/min |

**DOM10-53-474**

Single peak with the average molar mass defined as ~14kDa indicating a monomeric state in solution, shown in Figure 29

**DOM9-112-210**

Single peak with the molar mass defined as 30 kDa indicating a dimeric state in solution, shown in Figure 30

**DOM9-155-25**

Nice symmetrical peak but running at the buffer front. The mid part of the peak has been used for molar mass determination (see figure below with all three signals overlaid). Molar mass is 28 kDa which represents a dimeric dAb, shown in Figure 31. Overlay of all three signals (Figure 32)

**DOM9-155-147**

The main peak is assigned with molar mass of 26kDa over the right part of the peak and increasing steeply over the left part of the peak up to 53kDa. The peak most likely represents a dimer and a smaller fraction of tetramer in a rapid equilibrium. A much smaller peak eluting at 7.6 min, represents tetrameric protein with molar mass of 51kDa (Figure 33).

**DOM9-155-159**

The protein runs as a single symmetric peak, with average molar mass assigned at ~28kDa indicating a dimeric state in solution (Figure 34)

**Control for MW assignment by SEC-MALLS: BSA**

Each BSA run for each of the experiments set out above resulted in the expected MW, e.g. 2 peaks with molar mass of 67and 145kDa (monomer and dimer) (Figure 35).

**Example 6**

**Generation of trispecific mAbdAbs**

Trispecific mAb-dAbs were constructed either by generating VH and VL sequences by assembly PCR which were then cloned into existing mAbdAb expression vectors or by sub-cloning existing VH and VL regions from mAb expression vectors into existing mAbdAb expression vectors, such that when expressed, the trispecific mAbdAb has dAbs attached to both the C-terminus of the heavy and light chains.

A linker sequence was used to join the domain antibody to heavy chain CH3 or light chain CK. A schematic diagram of a trispecific mAbdAb molecule is shown in Figure 36 (the mAb heavy chain is drawn in grey; the mAb light chain is drawn in white; the dAbs are drawn in black).

A schematic diagram illustrating the construction of a trispecific mAbdAb heavy chain (top illustration) and a trispecific mAbdAb light chain (bottom illustration) is shown below

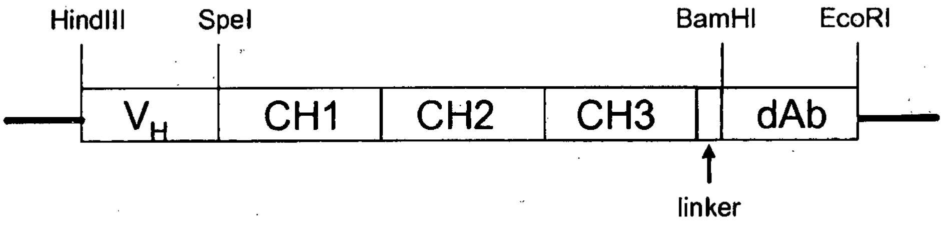


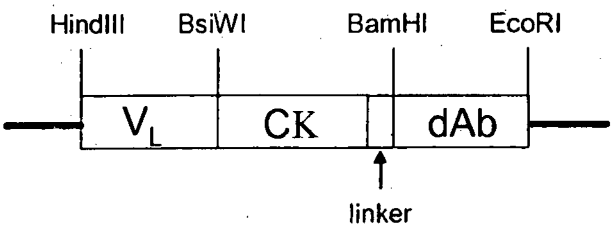


For the heavy chain the term '$V_H$' is the monoclonal antibody variable heavy chain sequence; 'CH1, CH2 and CH3' are human IgG1 heavy chain constant region sequences; 'linker' is the sequence of the specific linker region used; 'dAb' is the domain antibody sequence. For the light chain: '$V_L$' is the monoclonal antibody variable light chain sequence; 'CK' is the human light chain constant region sequence; 'linker' is the sequence of the specific linker region used; 'dAb' is the domain antibody sequence.

A mammalian amino acid signal sequence (as shown in SEQ ID NO: 64) was used in the generation of these constructs.

### 6.1 Trispecific anti-IL18mAb-anti-IL4dAb-anti-IL13dAb

A trispecific anti-IL18mAb-anti-IL4dAb-anti-1L13dAb (also known as IL18mAb-210-474) was constructed by grafting a sequence encoding an anti-human IL-4 domain antibody (DOM9-112-210) onto a sequence encoding the heavy chain and a sequence encoding an anti-iL13 domain antibody (DOM10-53-474) onto a sequence encoding the light chain of an anti-human IL-18 humanised monoclonal antibody. A G4S linker was used to join the anti-IL4 domain antibody onto the heavy chain of the monoclonal antibody. A G4S linker was also used to join the anti-IL13 domain antibody onto the light chain of the monoclonal antibody.

IL18mAb-210-474 was expressed transiently in CHOK1 cell supernatants, and following quantification of IL18mAb-210-474 in the cell supernatant, analysed in a number of IL-18, IL-4 and IL-13 binding assays.

| Name | Description | Sequence ID No. |
|---|---|---|
| IL18mAb-210-474 | H chain = Anti-human IL-18 mAb heavy chain-G4S linker-DOM9-112-210 dAb | 69 (=H chain) |
| | L chain = Anti-human IL-18 mAb light chain-G4S linker-DOM10-53-474 dAb | 70 (=L chain) |

### 6.2 Trispecific anti-IL5mAb-anti-IL4dAb-anti-IL13dAb

A trispecific anti-IL5mAb-anti-IL4dAb-anti-IL13dAb (also known as Mepo-210-474) was constructed by grafting a sequence encoding an anti-human IL-4 domain antibody DOM9-112-210 (SEQ ID NO: 4) onto a sequence encoding the heavy chain of an anti-human IL-5 humanised monoclonal antibody (SEQ ID NO: 65), and grafting a sequence encoding an anti-IL13 domain antibody DOM10-53-474 (SEQ ID NO: 5) onto a sequence encoding the light chain of an anti-human IL-5 humanised monoclonal antibody (SEQ ID NO: 66). A G4S linker was used to join the anti-IL4 domain antibody onto the heavy chain of the monoclonal antibody. A G4S linker was also used to join the anti-IL13 domain antibody onto the light chain of the monoclonal antibody.

This mAbdAb was expressed transiently in CHOK1 and HEK293-6E cell supernatants, and following quantification in the cell supernatant, analysed in a number of IL-4, IL-5 and IL-13 binding assays.

| Name | Description | Sequence ID No. |
|---|---|---|
| Mepo-210-474 | H chain = Anti-human IL-5 mAb heavy chain-G4S linker-DOM9-112-210 dAb<br>L chain = Anti-human IL-5 mAb light chain-G4S linker-DOM10-53-474 dAb | 71 (=H chain)<br>72 (=L chain) |

### 6.3 Sequences of monoclonal antibodies, domain antibodies and linkers

The sequences for the monoclonal antibodies, domain antibodies and linkers used to generate the trispecific mAbdAbs (or used as control reagents in the following exemplifications) are shown below in table 14.

Table 14

| Name | Specificity | Sequence ID |
|---|---|---|
| DOM9-112-210 domain antibody | Human IL-4 | 4 |
| DOM10-53-474 domain antibody | Human IL-13 | 5 |
| GGGGS linker sequence | | 6 |
| Pascolizumab (Anti-human IL-4 monoclonal antibody) | Human IL-4 | 14 (=H chain)<br>15 (=L chain) |
| Mepolizumab (Anti-human IL-5 monoclonal antibody) | Human IL-5 | 65 (=H chain)<br>66 (=L chain) |
| Anti-human IL-13 (humanised) monoclonal antibody | Human IL-13 | 12 (=H chain)<br>13 (=L chain) |
| Anti-human IL-18 (humanised) monoclonal antibody | Human IL-18 | 67 (=H chain)<br>68 (=L chain) |

Mature human IL-4 amino acid sequence (without signal sequence) is given in SEQ ID NO: 62.

Mature human IL-13 amino acid sequence (without signal sequence) is given in SEQ ID NO: 63.

### 6.4 Expression and purification of trispecific mAbdAbs

DNA sequences encoding trispecific mAbdAb molecules were cloned into mammalian expression vectors (Rln, Rld or pTT) using standard molecular biology techniques. The trispecific mAbdAb expression constructs were transiently transfected into one or both of CHOK1 or HEK293-6E cells, expressed at small scale (3mls to 150mls). The expression procedures used to generate the trispecfic mAbdAbs were the same as those routinely used to express and monoclonal antibodies.

Some of the constructs were purified using immobilised Protein A columns and quantified by reading absorbance at 280nm.

## Example 7

## Binding of trispecific mAbdAbs to human IL-4, human IL-13 and human IL-18 by ELISA

### 7.1 Binding of IL-18mAb-210-474 to IL-4, IL-13 and IL-18 by ELISA

Trispecific mAbdAb IL18mAb-210-474 (supernatants) prepared as described in Example 6 (SEQ ID NO: 69 and 70), was tested for binding to human IL-18, human IL-13 and human IL-4 in direct binding ELISAs (as described in methods 1, 2 and 3) and these data are shown in Figures 37, 38 and 39 respectively (IL18mAb-210-474 was prepared and tested a number of times and this has been annotated in the figures as sample 1, sample 2, sample 3, etc).

These figures show that IL18mAb-210-474 bound IL-4, IL-13 and IL-18 by ELISA. Purified anti-human IL18 mAb was included in the IL-18 binding ELISA as a positive control for IL-18 binding. The anti-IL-4 dAb (DOM9-112-210) was not tested in the IL-4 binding ELISA as this dAb is not detected by the secondary detection antibody; instead, purified anti-human IL4 mAb (Pascolizumab) was used as a positive control to demonstrate IL-4 binding in this ELISA. The anti-IL-13 dAb (DOM10-53-474) was not tested in the IL-13 binding ELISA as this dAb is not detected by the secondary detection antibody; instead, purified anti-human IL-13 mAb was included as a positive control to demonstrate IL-13 binding in this ELISA. As shown in the figures, negative control mAbs to an irrelevant antigen were included in each binding ELISA.

In each ELISA the binding curve for IL18mAb-210-474 sample 5 sits apart from the binding curves for the other IL18mAb-210-474 samples. The reason for this is unknown however, it maybe due to a quantification issue in the human IgG quantification ELISA for this particular IL18mAb-210-474 sample 5.

### 7.2 Binding of Mepo-210-474 to IL-4 and IL-13 by ELISA

Trispecific mAbdAbs Mepo-210-474 (supernatant) prepared as described in section 1 (sequence ID numbers 71 and 72), were tested for binding to human IL-13 and human IL-4 in direct binding ELISAs (as described in methods 1 and 2 respectively) and these data are shown in Figures 40 and 41 respectively (Mepo-210-474 was prepared and tested in quadruplicate and this has been annotated as sample 1, sample 2, sample 3 and sample 4).

These figures illustrate that Mepo-210-474 bound IL-4 and IL-13 by ELISA. The anti-IL-4 dAb (DOM9-112-210) was not tested in the IL-4 binding ELISA as this dAb is not detected by the secondary detection antibody; instead, purified anti-human IL4 mAb (Pascolizumab) was used as a positive control to demonstrate IL-4 binding in this ELISA. The anti-IL-13 dAb (DOM10-53-474) was not tested in the IL-13 binding ELISA as this dAb is not detected by the secondary detection antibody; instead, purified anti-human IL-13 mAb was included as a positive control to demonstrate IL-13 binding in this ELISA. As shown in Figures 40 and 41, negative control mAbs to an irrelevant antigen were included in each binding ELISA.

Mepo-210-474 sample 1 and sample 2 were prepared in one transient transfection experiment and Mepo-210-474 sample 3 and sample 4 were prepared in another separate transient transfection experiment. All four samples bound IL-13 and IL-4 in IL-13 and IL-4 binding ELISAs. However, the reason for the different binding profiles of samples 1 and 2 verses samples 3 and 4 is unknown, but may reflect a difference in the quality of the mAbdAb (in the supernatant) generated in each transfection experiment.

## Example 8

## Binding of trispecific mAbdAbs to human IL-4, human IL-5, human IL-13 and human IL-18 by surface plasmon resonance (BIAcore™)

### 8.1 Binding of IL-18mAb-210-474 to IL-4, IL-13 and IL-18 by BIAcore™

Trispecific mAbdAb IL18mAb-210-474 (supernatants) prepared as described in Example 6.1 (SEQ ID NO: 69 and 70), was tested for binding to human IL-4, human IL-13 and human IL-18 using BIAcore™ at 25°C (as described in methods 4, 5 and 6 respectively). Capture levels were within the range of approximately 400 to 850 Response Units. Three concentrations of each analyte were tested (256, 32 and 4nM). The resulting data are shown in Table 15 (samples were prepared and tested in triplicate, this has been annotated as sample 1, sample 2 and sample 3).

Table 15

| Construct | On rate (ka) | Off rate (kd) | Binding affinity, KD (nM) |
|---|---|---|---|
| **Binding to IL-18** | | | |
| IL18mAb-210-474 (sample 1) | 2.1e6 | 2.3e-5 | 0.011 |
| IL18mAb-210-474 (sample 2) | 2.1e6 | 2.8e-5 | 0.014 |
| IL18mAb-210-474 (sample 3) | 2.1e6 | 2.9e-5 | 0.014 |
| Anti-human IL-18 mAb (purified) | 1.9e6 | 6.8e-5 | 0.035 |
| **Binding to IL-13** | | | |
| IL18mAb-210-474 (sample 1) | 5.8e5 | 5.7e-4 | 0.99 |
| IL18mAb-210-474 (sample 2) | 6.2e5 | 6.1e-4 | 0.99 |
| IL18mAb-210-474 (sample 3) | 7.4e5 | 7.4e-4 | 1.0 |
| Anti-human IL-13 mAb (purified) | 1.2e6 | 5.0e-4 | 0.41 |
| **Binding to IL-4** | | | |
| IL18mAb-210-474 (sample 1) | --- | --- | very tight binder * |

(continued)

| | | | |
|---|---|---|---|
| **Binding to IL-4** | | | |
| IL18mAb-210-474 (sample 2) | --- | --- | very tight binder * |
| IL18mAb-210-474 (sample 3) | --- | --- | very tight binder * |
| Pascolizumab (purified) | 4.6e6 | 1.7e-4 | 0.037 |
| *unable to determine KD due to positive dissociation effects and sensitivity level of BIAcore™ technique | | | |

The trispecific mAbdab bound IL-4, IL-13 and IL-18 using BIAcore™. The binding affinity of the mAbdAb for IL-18 was approximately equivalent to that of purified anti-human IL18 mAb alone, which was included in this assay as a positive control for IL-18 binding and in order to directly compare to the binding affinity of the mAbdab. It was not possible to determine the absolute binding affinity of the mAbdab for IL-4, due to the fact that the DOM9-112-210 component of this trispecific mAbdAb bound very tightly to IL-4 and hence the off-rate was too small to determine using BIAcore™. The anti-IL-4 dAb alone (DOM9-112-210) was not tested in this assay as this dAb cannot be captured onto the Protein A or anti-human IgG coated CM5 chip; instead, the anti-human IL4 mAb (Pascolizumab) was included as a positive control to demonstrate IL-4 binding in this assay. The anti-IL-13 dAb alone (DOM10-53-474) was not tested in this assay as this dAb cannot be captured onto the Protein A or anti-human IgG coated CM5 chip; instead, the anti-human IL13 mAb was included as a positive control to demonstrate IL-13 binding in this assay.

**8.2 Binding of Mepo-210-474 to IL-4, IL-5 and IL-13 by BIAcore™**

Trispecific mAbdAb Mepo-210-474 (supernatants) prepared as described in Example 6.2 (SEQ ID NO: 71 and 72), was tested for binding to human IL-4, human IL-5 and human IL-13 using BIAcore™ at 25°C (as described in methods 5, 11 and 4 respectively). Capture levels were within the range of approximately 550 to 900 Response Units. For IL-4 and IL-13 binding five concentrations of each analyte were tested (256, 64, 16, 4 and 1nM). For IL-5 binding four concentrations of each analyte were tested (64, 16, 4 and 1nM). The resulting data are shown in Table 16.

Table 16

| Construct | On rate (ka) | Off rate (kd) | Binding affinity, KD(nM) |
|---|---|---|---|
| **Binding to IL-5** | | | |
| Mepo-210-474 | 3.34e5 | 1.50e-4 | 0.45 |
| Mepolizumab (purified) | 3.78e4 | 1.30e-4 | 0.34 |
| **Binding to IL-13** | | | |
| Mepo-210-474 | 6.38e5 | 1.03e-3 | 1.62 |
| Anti-human IL-13 mAb (purified) | 1.51 e6 | 5.68e-4 | 0.38 |
| **Binding to IL-4** | | | |
| Mepo-210-474 | --- | --- | very tight binder (unable to determine KD due to positive dissociation effects and sensitivity level of BIAcore™ technique) |
| Pascolizumab (purified) | 6.26e6 | 1.43e-4 | 0.02 |

Mepo-210-474 bound IL-4, IL-5 and IL-13 using BIAcore™. The binding affinity of Mepo-210-474 for IL-5 was approximately equivalent to that of purified anti-human IL5 mAb (Mepolizumab) alone, which was included in this assay as a positive control for IL-5 binding and in order to directly compare to the binding affinity of Mepo-210-474. It was not possible to determine the absolute binding affinity of Mepo-210-474 for IL-4, due to the fact that the DOM9-112-210 component of this trispecific mAbdAb bound very tightly to IL-4 and hence the off-rate was too small to determine using BIAcore™. The anti-IL-4 dAb alone (DOM9-112-210) was not tested in this assay as this dAb cannot be captured onto the Protein A or anti-human IgG coated CM5 chip; instead, the anti-human IL4 mAb (Pascolizumab) was included as a positive control to demonstrate IL-4 binding in this assay. The anti-IL-13 dAb alone (DOM10-53-474) was not tested in this assay as this dAb cannot be captured onto the Protein A or anti-human IgG coated CM5 chip; instead, the anti-human IL13 mAb was included as a positive control to demonstrate IL-13 binding in this assay.

## Example 9

## Stoichiometry

### 9.1 Stoichiometry of binding of IL-4, IL-13 and IL-18 to IL-18mAb-210-474 using BIAcor™

IL18mAb-210-474 (in CHO cell supernatants prepared as described in section 1) (SEQ ID NO: 69 and 70), were evaluated for stoichiometry of binding for IL-4, IL-13 and IL-18 using BIAcore™ (as described in method 7). These data are shown in Table 17 (R-max is the saturated binding response and this is required to calculate the stoichiometry, as per the given formulae in method 7). The concentration of each of the cytokines was 500nM. The injection position refers to the order in which each of the cytokines was added.

Table 17

| Cytokine | Injection position | R-max | Stoichiometry |
|---|---|---|---|
| IL-4 | 1st | 59 | 0.9 |
| IL-4 | 2nd | 56 | 0.9 |
| IL-4 | 3rd | 51 | 0.8 |
| IL-13 | 1st | 74 | 1.6 |
| IL-13 | 2nd | 77 | 1.7 |
| IL-13 | 3rd | 80 | 1.8 |
| IL-18 | 1st | 112 | 1.8 |
| IL-18 | 2nd | 113 | 1.8 |
| IL-18 | 3rd | 110 | 1.7 |

The stoichiometry data indicated that IL18mAb-210-474 bound approximately two molecules of IL-18, two molecules of IL-13 and only one molecule of IL-4. The anti-IL4 dAb alone (DOM9-112-210) is known to be a dimer in solution state and is only able to bind one molecule of IL-4. It is therefore not unexpected that IL18mAb-210-474 would bind only one molecule of IL-4. These data indicated that the molecules tested could be fully occupied by the expected number of IL-18, IL-13 and IL-4 molecules. The stoichiometry data also indicated that the order of capture of the cytokines appears to be independent of the order of addition of the cytokines.

## Example 10

### 10.1 Generation of a Dual Targeting anti-TNF/anti-EGFR mAbdAb

This dual targeting mAbdAb was constructed by fusion of a dAb to the C-terminus of the mAb heavy chain. The anti-TNF mAb heavy and light chain expression cassettes had been previously constructed. The restriction sites which were used for cloning are shown below (Figure 42).

To introduce restriction sites for dAb insertion in the heavy chain, site directed mutagenesis was used to create SalI and HindIII cloning sites using the mAb heavy chain expression vector as a template. DNA coding an anti-EGFR dAb (DOM16-39-542) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 170 and 169) for light and heavy chains respectively) were selected and large scale were made using Qiagen Mega Prep Kit following the manufacturer's protocols. mAbdAbs were expressed in mammalian HEK293-6E cells using transient transfection techniques by co-transfection of light and heavy chains (SEQ ID NO: 73 and 74)

### 10.2 Purification and SEC analysis of the Dual Targeting anti-TNF/anti-EGFR mAbdAb

The anti-TNF/anti-EGFR mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 43) of the purified sample shows non-reduced sample running at -170kDa whilst reduced sample shows two bands running at -25 and ~60kDa corresponding light chain and dAb-fused heavy chain respectively.

For size exclusion chromatography (SEC) analysis the anti-TNF/anti-EGFR mAbdAb was applied onto a Superdex-200 10/30 HR column (attached to an Akta Express

FPLC system) pre-equilibrated and running in PBS at 0.5ml/min. The SEC profile shows a single species running as a symmetrical peak (figure 44).

### 10.3 Binding Affinities of the Dual Targeting anti-TNF/anti-EGFR mAbdAb

Binding affinities to EGFR and TNF were determined as described in methods 13 and 14 respectively. Assay data were analysed using Graph Pad Prism. Potency values were determined using a sigmoidal dose response curve and the data fitted using the best fit model. Anti-EGFR potency (Figure 45) of this mAbdAb was calculated to be 39.1 nM whilst the control, an anti-EGFR mAb gave an EC50 value of 3.4nM. In the anti-TNF bioassay (Figure 46) the potency was of the mAbdAb was 3pM (0.0028nM) whilst an anti-TNF control mAb produced an EC50 of 104pM. In conclusion, assay data shows that the construct of example 10, a dual targeting anti-TNF/anti-EGFR mAbdAb is potent against both antigens.

### 10.4 Rat PK of the Dual Targeting anti-TNF/anti-EGFR mAbdAb

This molecule was tested for its *in vivo* pharmacokinetic properties in the rat. The anti-TNF/anti-EGFR mAbdAb was administered i.v. to three rats, and serum samples collected over a period of 7 days (168 hours). The concentration of drug remaining at various time points post-dose was assessed by ELISA against both TNF & EGFR. Results are shown in Figure 125.

The PK parameters confirmed that this molecule had a long half life, in the same region as that previously observed for unmodified adalimumab (125 hours). Further details are shown in Table 17.1

Table 17.1

| Assay Antigen | Half Life (hr) | Cmax (ug/mL) | AUC (0-inf) (hr*ug/mL) | Clearance (mL/hr/kg) | % AUC Extrapolated |
|---|---|---|---|---|---|
| TNF | 157.2 | 149.8 | 10301.3 | 0.5 | 40.6 |
| EGFR | 140.8 | 123.6 | 7986.7 | 0.7 | 35 |

## Example 11

### 11.1 Generation of a Dual Targeting anti-TNF/anti-VEGF mAbdAb

An anti-TNF/anti-VEGF mAbdAb was produced employing a similar strategy described for example 10. For construction of the heavy chain expression cassette, vector DNA encoding the heavy chain of example 10 was taken as a starting point. The anti-EGFR dAb was excised using the restriction enzymes SalI and HindIII. DOM15-26-593, an anti-VEGF dAb was amplified by PCR (Using primers coding SalI and HindIII ends) and ligated into the vector backbone which previously had the anti-EGFR dAb excised using the same restriction sites, resulting in a linker of 'STG' (serine, threonine, glycine) between the mAb and the dAb.

Sequence verified clones (SEQ ID NO: 169 and 168 for light and heavy chains respectively) were selected and large scale DNA preparations were made and the anti-TNF/anti-VEGF mAbdAb was expressed in mammalian HEK293-6E cells using transient transfection techniques by co-transfection of light and heavy chains (SEQ ID NO: 73 and 75).

### 11.2 Purification and SEC analysis of the Dual Targeting anti-TNF/anti-VEGF mAbdAb

The anti-TNF/anti-VEGF mAbdAb (designated DMS4000) was purified from clarified expression supernatant using Protein-A affinity chromatography according to established protocols. Concentrations of purified samples were determined by spectrophotometry from measurements of light absorbance at 280nm. SDS-PAGE analysis (figure 47) of the purified sample shows non-reduced sample running at ~170kDa whilst reduced sample shows two bands running at ~25 and ~60kDa corresponding light chain and dAb-fused heavy chain respectively.

For size exclusion chromatography (SEC) analysis the anti-TNF/anti-VEGF mAbdAb was applied onto a Superdex-200 10/30 HR column (attached to an Akta Express FPLC system) pre-equilibrated and running in PBS at 0.5ml/min. The SEC profile shows a single species running as a symmetrical peak (figure 48).

**11.3 Binding Affinities of the Dual Targeting anti-TNF/anti-VEGF mAbdAb**

Binding affinities to VEGF and TNF were determined as described in methods 12 and 14 respectively. Assay data were analysed using GraphPad Prism. Potency values were determined using a sigmoidal dose response curve and the data fitted using the best fit model. Anti-VEGF potency (Figure 49) of this mAbdAb was calculated to be 57pM whilst the control, an anti-VEGF mAb gave an EC50 value of 366pM. In the anti-TNF bioassay (Figure 50) the potency was 10pM whilst an anti-TNF control mAb produced an EC50 of 22pM. In conclusion, assay data shows that the molecule of Example 11, a dual targeting anti-TNF/anti-VEGF mAbdAb is potent against both antigens.

**11.4 Rat PK of the Dual Targeting anti-TNF/anti-VEGF mAbdAb**

This molecule was tested for its *in vivo* pharmacokinetic properties in the rat. The anti-TNF/anti-VEGF mAbdAb was administered i.v. to three rats, and serum samples collected over a period of 10 days (240 hours). The concentration of drug remaining at various time points post-dose was assessed by ELISA against both TNF & VEGF. The results are shown in Figure 126

The PK parameters confirmed that this molecule had *in vivo* pharmacokinetic properties that compared with those of unmodified adalimumab. The shorter observed $t_{1/2}\beta$ for the VEGF component is not considered to be significant and may be an assay artefact. Further details are shown in Table 17.2

Table 17.2

| Antigen | Half Life (hr) | Cmax (μg/mL) | AUC (0-inf) (hr* μg/mL) | Clearance (mL/hr/kg) | % AUC Extrapolated |
|---|---|---|---|---|---|
| TNF | 180.1 | 89.9 | 7286.3 | 0.7 | 35.8 |
| VEGF | 94.2 | 102.8 | 4747.1 | 1.1 | 14.3 |

**11.5 Generation of an alternative anti-TNF/anti-VEGF mAbdAb**

An alternative anti-TNF/anti-VEGF mAbdAb was constructed in a similar way to that described above in Example 11.1, using the same anti-TNF mAb linked to a VEGF dAb on the C-terminus of the heavy chain using an STG linker. The anti-VEGF dAb used in this case was DOM15-10-11. This molecule was expressed in mammalian HEK293-6E cells using transient transfection techniques by co-transfection of light and heavy chains (SEQ ID NO: 73 and 185). This molecule expressed to give a mAbdAb of similar expression levels to that described in Example 11.2, however when tested for potency in the same VEGF assay as described in Example 11.3 it was found to have undetectable levels of inhibition of VEGF binding to VEGF receptor in this assay.

**Example 12**

**12.1 Generation of a Dual Targeting anti-VEGF/anti-IL1R1 dAb-extended IgG**

Two dual targeting dAb-extended IgG molecules were constructed using standard molecular biology techniques following a strategy of insertion of Dummy V domain coding regions in between dAb and constant regions of both chains.

For the light chain, the anti-IL1R1 dAb DOM4-130-54 was previously cloned into an expression cassette with SalI and BsiWI sites (Figure 51) to produce a dAb-Ck chain. To produce the dAb-extended IgG light chain, Dummy Vk region was amplified by PCR with primers coding BsiWI on both ends. Plasmid containing the dAb-Ck expression cassette was digested with BsiWI. BsiWI digested Dummy Vk domain was ligated into this to produce the dAb-extended IgG light chain, with a linker of 'TVAAPS' between the two variable domains.

An identical strategy was followed to produce the dAb-extended IgG heavy chain where the PCR amplified Dummy VH domain with NheI ends was ligated into an NheI digested dAb heavy chain in between the dAb DOM15-26 and CH1 (Figure 51), with a linker of 'ASTKGPS' between the two variable domains.

This is designated DMS2090 and has the sequence set out in SEQ ID NO: 163 (DNA sequence SEQ ID NO: 162). This was paired with the light chain set out in SEQ ID NO: 77 (DNA sequence SEQ ID NO: 171).

A second heavy chain was constructed in the same way but using the dAb DOM15-26-593. This is designated DMS2091, and has the sequence set out in SEQ ID NO: 76 (DNA sequence SEQ ID NO: 172). This was paired with the light chain set out in SEQ ID NO: 77 (DNA sequence SEQ ID NO: 171).

Sequence verified clones were selected and large scale DNA preparations were made using Qiageri Maxi or Mega Prep Kits following the manufacturer's protocols. The resulting construct was expressed in mammalian cells using transient transfection techniques by co-transfection of light and heavy chains.

**12.2 Purification and SEC analysis of the Dual Targeting anti-VEGF/anti-IL1R1 mAbdAb**

Both anti-IL1R1/anti-VEGF dAb-extended IgG molecules were purified from clarified expression supernatant using Protein-A affinity chromatography according to established protocols. Concentrations of purified samples were determined by spectrophotometry from measurements of light absorbance at 280nm. SDS-PAGE analysis for DMS2090 is shown in Figure 52, and for DMS2091 is shown in Figure 53. Both purified samples show non-reduced samples running at 190kDa whilst the reduced samples show two bands running at 35 and 60kDa corresponding to dAb-extended light chain and heavy chains respectively.

For size exclusion chromatography (SEC) analysis the anti-VEGF/anti-IL1R1 dAb-extended-IgG was applied onto a Superdex-200 10/30 HR column (attached to an Akta Express FPLC system) pre-equilibrated and running in PBS at 0.5ml/min. The SEC profiles for DMS2090 (Figure 54) and DMS2091 (Figure 55) both show a single species running as a symmetrical peak.

**12.3 Binding Affinities of the Dual Targeting anti-VEGF/anti-IL1R1 mAbdAb**

Binding affinities to VEGF and IL1R1 were determined as described in methods 12 and 15 respectively. Assay data were analysed using GraphPad Prism. Potency values were determined using a sigmoidal dose response curve and the data fitted using the best fit model. Anti-VEGF potency (Figure 57) of DMS2090 was calculated to be 158.4pM whilst the control, an anti-VEGF mAb gave an EC50 value of 689.2pM. Anti-VEGF potency (Figure 56) of DMS2091 was calculated to be 55pM whilst the control, an anti-VEGF mAb gave an EC50 value of 766pM.

In the anti-IL1R1 bioassay the potency of DMS2090 (Figure 58) was 32pM whilst an anti-IL1R1 control mAb produced an EC50 of 35pM. The potency of DMS2091 (Figure 59) was 17.47pM whilst an anti-IL1-R1 control mAb produced an EC50 of 35.02pM.

In conclusion, assay data shows that example 12, a dual targeting anti-IL1R1/anti-VEGF dAb-extended IgG is potent against both antigens.

**12.4 Rat PK of the Dual Targeting anti-VEGF/anti-IL1R1 mAbdAb**

This molecule was tested for its *in vivo* pharmacokinetic properties in the rat. The anti-IL1R1/anti-VEGF dAb-extended IgG A was administered i.v. to three rats, and serum samples collected over a period of 7 days (168 hours). The concentration of drug remaining at various time points post-dose was assessed by ELISA against both IL1R1 & VEGF. The results are shown in Figure 127

The PK parameters are shown in Table 17.3

Table 17.3

| Molecule | Assay Antigen | Half Life (hr) | Cmax (ug/mL) | AUC (0-inf) (hr*ug/mL) | Clearance (mL/hr/kg) | % AUC Extrapolated |
|---|---|---|---|---|---|---|
| DMS2090 | VEGF | **72.1** | **100.4** | **4811.6** | **1.1** | **19** |
| DMS2090 | IL-1R1 | **86.3** | **87.7** | **3467.4** | **1.6** | **23.7** |

## Example 13

**13.1 Generation of a Triple Targeting anti-TNF/anti-VEGF/anti-EGFR mAbdAb**

A triple targeting mAb was constructed using standard molecular biology techniques and following a strategy of insertion of mAb V domain coding regions in between dAb and constant regions of both chains.
For the light chain, the anti-EGFR dAb DOM16-39-542 was previously cloned into an expression cassette with Sall and BsiWI sites (Figure 15) to produce a dAb-Ck chain. To produce the mAbdAb light chain, the mAb VL region was amplified by PCR with primers coding BsiWI on both ends. Plasmid containing the dAb-Ck expression cassette was digested with BsiWI. BsiWI digested mAb VL domain was ligated into this to produce the mAbdAb light chain, resulting in a linker of 'TVAAPS' between the two variable domains.

An identical strategy was followed to produce the mAbdAb heavy chain where the PCR amplified mAb VH region with NheI ends was ligated into an NheI digested dAb heavy chain vector in between the dAb (DOM15-26) and CH1 (Figure 60), resulting in a linker of 'ASTKGPS' between the two variable domains.

Sequence verified clones (amino acid SEQ ID NO: 78 and 79 for heavy and light chains respectively) were selected and large scale DNA preparations were made using Qiagen Mega Prep Kits following the manufacturer's protocols. mAbdAbs were expressed in mammalian cells using transient transfection techniques by co-transfection of light and heavy chains.

## 13.2 Purification of the Triple Targeting anti-TNF/anti-VEGF/anti-EGFR mAbdAb

The anti-TNF/anti-VEGF/anti-EGFR mAbdAb was purified from clarified expression supernatant using Protein-A affinity chromatography according to established protocols. Concentrations of purified samples were determined by spectrophotometry from measurements of light absorbance at 280nm. SDS-PAGE analysis (figure 61) of the purified sample shows non-reduced sample running at 190kDa whilst reduced sample shows two bands running at 35 and 60kDa corresponding to dAb-extended light chain and heavy chains respectively.

## 13.3 Binding Affinities of the Triple Targeting anti-TNF/anti-VEGF/anti-EGFR mAbdAb

Binding affinities to VEGF, EGFR and TNF were determined as described in methods 12, 13 and 14 respectively. Assay data were analysed using GraphPad Prism. Potency values were determined using a sigmoidal dose response curve and the data fitted using the best fit model. Anti-VEGF potency (Figure 62) of this mAbdAb was calculated to be 1.885mM whilst the control, an anti-VEGF mAb gave an EC50 value of 0.145nM.

In the anti-TNF bioassay (Figure 63) the potency was 87pM whilst an anti-TNF control mAb produced an EC50 of 104pM. In the anti-EGFR assay (Figure 64) the triple targeting mAbdAb produced -20% inhibition at ~300nM. Whilst EC50 values were calculated for VEGF and TNF binding, for EGFR binding a full curve was not produced to calculate an EC50 value.

## Example 14: IGF-1R/VEGF mAbdAb

## 14.1 Construction of IGF-1R/VEGF mAbdAb

Anti IGF-1 R variable heavy and variable light gene sequences were originally built de *novo* from PCR of overlapping oligonucleotides. These regions were fused to human IgG1 or kappa constant regions in a mammalian expression vector using standard molecular biology techniques. The gene sequence for an anti VEGF domain antibody was likewise constructed by PCR using overlapping oligonucleotides and fused to the 3' end of either the heavy or light chain genes of the anti IGF-1 R components described above. The fusion incorporated either a two amino acid (GS) linker or an 8 amino acid (TVAAPSGS) linker between the antibody and the domain antibody components. Antibody heavy and light chains were also constructed without the domain antibody and linker sequences. Sequence verified clones were selected for large scale DNA preparations using Endofree Qiagen Maxiprep kit following the manufacturer's protocols.

In sequences SEQ ID NO: 108, 109, 111 and 112, the position of the linker sequence between the antibody and domain antibody is underlined. Alternative variants could be constructed by removing the linker entirely or by using different linkers. Examples of other suitable linkers are provided in SEQ ID NO: 6 and 8 to 11. Table 18 provides a list of the antibodies constructed and expressed.

**Table 18** - Antibodies constructed and tested

| Identifier | Alternative names | Antibody | Domain antibody | Linker | SEQ ID NO for heavy chain | SEQ ID NO for light chain | Site of fusion |
|---|---|---|---|---|---|---|---|
| BPC1603 | 381 H TVAAPSGS 593, DMS4019 | Anti-IGF-1R antibody H0L0 | Dom15-26-593 anti-VEGF | TVAAPSGS | 108 | 113 | Heavy chain C terminus |

(continued)

| Identifier | Alternative names | Antibody | Domain antibody | Linker | SEQ ID NO for heavy chain | SEQ ID NO for light chain | Site of fusion |
|---|---|---|---|---|---|---|---|
| BPC1604 | 381 H GS 593, DMS4020 | Anti-IGF-1R antibody H0L0 | Dom15-26-593 anti-VEGF | GS | 109 | 113 | Heavy chain C terminus |
| BPC1605 | 381L TVAAPSGS 593, DMS4021 | Anti-IGF-1R antibody H0L0 | Dom15-26-593 anti-VEGF | TVAAPSGS | 110 | 111 | Light chain C terminus |
| BPC1606 | 381 L GS 593, DMS4022 | Anti-IGF-1R antibody H0L0 | Dom15-26-593 anti-VEGF | GS | 110 | 112 | Light chain C terminus |

**Example 14.2: Expression, purification and SEC profile of IGF-1R/VEGF mAbdAb**

Combinations of the heavy and light chain vectors expressed in transient transfections of HEK293-6E. Briefly, 50 ml of HEK293-6E cells at $1.5x10^6$ cells/ml were transfected with 25μg of heavy and 25μg of light chain plasmid previously incubated with 293fectin reagent (Invitrogen # 51-0031). Cells were placed in a shaking incubator at 37°C, 5% $CO_2$, and 95% releative humidity. After 24 hours, tryptone feeding media was added and the cells grown for a further 72 hours. Supernatant was harvested by centrifugation followed by filtration using a 0.22μm filter. The expressed protein was purified using a Protein A sepharose column and dialysed into PBS. Purified protein was analysed by size exclusion chromatography (SEC) and is shown in Figure 65.

An IGF-1 R antibody (H0L0) was used as a comparator in the following assays. This molecule has the heavy chain sequence set out in SEQ ID NO: 110, and the light chain sequence set out in SEQ ID NO: 113.

Another mAbdAb with irrelevant specificity was used as a comparator in the following assays. This molecule has the heavy chain sequence set out in SEQ ID NO: 87, and the light chain sequence set out in SEQ ID NO: 13 and is designated BPC1601.

**Example 14.3: IGF-1R Binding ELISA**

A binding ELISA was carried out to test the binding of the purified anti-IGF-1R/VEGF mAbdAbs to IGF-1R. Briefly, ELISA plates coated with anti-polyhistidine (AbCam AB9108) at 1μg/ml and blocked with blocking solution (4% BSA in Tris buffered saline) were loaded with 400ng/ml recombinant human IGF-1R-his tag (R&D Systems 305-GR) in PBS. The plate was incubated for 1 hour at room temp before washing in TBS + 0.05% Tween 20 (TBST). Various concentrations of the purified mAbdAbs were added as well as an anti IGF-1R monoclonal antibody (HOLO) and an irrelevant mAbdAb (BPC1601), diluted in blocking solution. The plate was incubated for 1 hour at room temperature before washing in TBST. Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results of the binding ELISA are presented in Figure 66 and confirm that all the IGF1R-VEGF mAbdAb variants tested (BPC1603-1606) show binding to IGF-1 R at levels comparable to the anti-IGF-1R antibody H0L0. EC50 values were calculated using Cambridgesoft Bioassay software and are as follows: H0L0 (0.1797μg/ml)), BPC1603 (0.1602μg/ml), BPC1604 (0.1160μg/ml), BPC1605 (0.1975ng/ml), BPC1606 (0.1403ng/ml). The irrelevant control bispecific antibody BPC1601 showed now detectable binding to IGF-1 R.

**Example 14.4: VEGF Binding ELISA**

A binding ELISA was carried out to test the binding of the purified anti IGF-1 R/VEGF bispecific antibodies to VEGF. ELISA plates were coated with recombinant human VEGF (GSK) at 400ng/ml in PBS and then blocked in blocking solution (4% BSA in TBS). Various concentrations of the purified mAbdAbs diluted in blocking solution were added and mAbdAb BPC1601 was included as a negative control. The plate was incubated for 1 hour at room temperature before washing in TBST. Binding was detected by the addition of a peroxidase labelled anti human kappa light chain antibody (Sigma A7164) at a dilution of 1/1000 in blocking solution. The plate was incubated for 40 minutes at room temp before washing in TBST. The plate was developed by addition of OPD substrate (Sigma P9187): and colour development stopped by addition of 3M $H_2SO_4$. Absorbance was measured at 490nm with a plate reader and the mean absorbance plotted.

The results of the binding ELISA are presented in Figure 67 and confirm that all four anti-IGF-1R/VEGF mAbdAbs (BPC1603-1606) can bind to immobilised VEGF. The apparent lower binding activity of BPC1605 and BPC1606 may be attributable to interference between the domain antibody (located at the C-terminus of the light chain) and the detection antibody. EC50 values were calculated using Cambridgesoft Bioassay software and are as follows: BPC1603 (0.044μg/ml), BPC1604 (0.059μg/ml), BPC1605 (0.571 μg/ml). It was not possible to calculate an accurate EC50 value for BPC1606 due to the lower response values. The anti-IGF-1R antibody H0L0 and the irrelevant control mAbdAb BPC1601 showed now detectable binding to VEGF.

**Examples 14.5: Kinetics of binding to VEGF**

A mouse monoclonal against human IgG (Biacore BR-1008-39) was immobilised by primary amine coupling to a CM5 biosensor chip. The antibody constructs were captured using this surface. After capture VEGF was passed over the surface which was then regenerated using 3M $MgCl_2$. The concentrations of VEGF used to generate kinetics were 256, 64, 16, 4, 1 and 0.25nM, with a buffer only injection over the captured surface used for double referencing. The experiments were carried out on the Biacore T100 machine, using 1x HBS-EP buffer (BR-1006-69) at 25°C. The data were fitted to the 1:1 model inherent to the machine in its analysis software. The data shown in Table 19 is from two independent experiments.

**Table 19 - Kinetics of binding to human VEGF**

| | Experiment 1 | | | Experiment 2 | | |
|---|---|---|---|---|---|---|
| Construct | ka | kd | KD (nM) | ka | kd | KD (nM) |
| BPC1603 | 2.398E+6 | 2.762E-4 | 0.115 | 1.334E+6 | 3.497E-4 | 0.262 |
| BPC1604 | 9.933E+5 | 3.092E-4 | 0.311 | 5.806E+5 | 3.266E-4 | 0.563 |
| BPC1605 | 1.599E+6 | 2.161E-4 | 0.135 | 1.089E+6 | 2.727E-4 | 0.251 |
| BPC1606 | 4.343E+5 | 1.573E-4 | 0.362 | 2.717E+5 | 1.607E-4 | 0.591 |

**Example 14.6: Inhibition of VEGF binding to receptor**

The activity of the mAbdAbs was measured using a VEGF receptor binding assay as described in Method 12. The IC50s obtained in this assay for inhibition of the binding of VEGF to VEGFR2 are:

BPC1603 (0.037nM)
BPC1604 (0.010nM)
BPC1605 (0.167nM)
BPC1606 (0.431 nM)

These results confirm that all four antigen binding constructs inhibit ligand binding to receptor.

**Example 14.7: Inhibition of IGF-1R receptor phosphorylation**

3T3/LISN c4 cells were plated at a density of 10 000 cells/well into 96 well plates and incubated overnight in complete DMEM (DMEM-Hepes modification +10%FCS). Purified mAbdAbs were added to the cells and incubated for 1 hour. rhIGF-1 was added to the treated cells to achieve a final concentration of 50ng/ml and incubated for a further 30 mins to stimulate receptor phosphorylation. The media was aspirated and then the cells lysed by the addition of RIPA lysis buffer (150mM NaCl, 50mM TrisHCl, 6mM Na Deoxycholate, 1% Tween 20) plus protease inhibitor cocktail (Roche 11 697 498 001). The plate was frozen overnight. After thawing, lysate from each well was transferred to a 96 well ELISA plate pre-coated with an anti IGF-1R capture antibody 2B9 (GSK) at 2μg/ml and blocked with 4%BSA/TBS. The plate was washed with TBST (TBS+0.1%Tween 20) and a Europium labelled anti Phosphotyrosine antibody (PerkinElmer DELFIA Eu-N1 PT66) diluted 1/2500 in 4%BSA/TBS was added to each well. After 1 hour incubation the plate was washed and DELFIA Enhancement (PerkinElmer 1244-105) solution added. After 10 min incubation the level of receptor phosphorylation was determined using a plate reader set up to measure Europium time resolved fluorescence (TRF).

The results of the experiment are presented in Figures 68 and 69. The results confirm that the mAbdAbs BPC1603-1606 can inhibit IGF-I mediated receptor phosphorylation at levels comparable to the anti-IGF-1R monoclonal antibody H0L0. an irrelevant antibody (labelled as IgG1, Sigma 15154) showed no activity in this assay.

## Example 15 anti-CD20/IL-13 antigen binding protein

### Example 15.1: Molecular biology

The mammalian expression vectors encoding the heavy and light chain sequences of an anti-CD20 mAb set out in SEQ ID NO: 117 and 120 were constructed de novo using a PCR based approach and standard molecular biology techniques. Bispecific anti-CD20mAb-anti-IL13dAb heavy and light chains were constructed by cloning the sequences encoding anti-CD20 mAb heavy and light variable regions into mammalian expression vectors containing human antibody constant regions fused to an anti-human IL-13 domain antibody (DOM10-53-474).

The mAbdAb expression constructs were transfected into CHOE1a cells. The supernatant was harvested and then the antibody purified using immobilised Protein A and quantified by reading absorbance at 280nm. The mAbdAbs (and the anti-CD20 control mAb) constructed and tested are listed in Table 20.

Table 20

| Identifier | Alternative names | Antibody Target | Domain antibody | Linker | Seq ID: for heavy chain | Seq ID : for light chain | Site of fusion |
|---|---|---|---|---|---|---|---|
| BPC1401 | RituxanH-TVAAPSGS-DOM474 | CD20 | DOM10-53-474 | TVAAPSGS | 116 | 117 | Heavy chain C -term |
| BPC1402 | RituxanH-GS-DOM474 | CD20 | DOM10-53-474 | GS | 118 | 117 | Heavy chain C -term |
| BPC1403 | RituxanL-TVAAPSGS-DOM474 | CD20 | DOM10-53-474 | TVAAPSGS | 120 | 119 | Light chain C -term |
| BPC1404 | RituxanL-GS-DOM474 | CD20 | DOM10-53-474 | GS | 120 | 121 | Light chain C -term |
| anti-CD20 mAb | Rituxan | CD20 | - | - | 120 | 117 | - |

**Example 15.2: Kinetics of binding to human IL-13**

The binding affinity of mAbdAb constructs for human IL-13 were assessed by BIAcore™ analysis. Analyses were carried out anti-human IgG capture. Briefly, Anti-human IgG (Biacore BR-1008-39) was coupled onto a CM5 chip by primary amine coupling. MAbdAb constructs were then captured onto this surface and human IL-13 (made and purified at GSK) passed over at defined concentrations. The surface was regenerated back to the Anti-human IgG surface using 3M $MgCl_2$. This treatment did not significantly affect the ability to capture antibody for a subsequent IL-13 binding event. The runs were carried out at 25°C using HBS-EP buffer, on the BIAcore™ T100 machine. Data were analysed using the evaluation software in the machine and fitted to the 1:1 binding model. The results of the analysis are presented in Table 48, confirming that for all mAbdAb constructs, the kinetics of binding to IL-13 are comparable.

Table 48 - Surface plasmon resonance (BIAcore™) data

| Antibody name | Ka ($M^{-1}.s^{-1}$) | Kd ($s^{-1}$) | KD (nM) |
|---|---|---|---|
| BPC1401 | 5.81 E+5 | 1.82E-4 | 0.313 |
| BPC1402 | 8.52E+5 | 3.05E-4 | 0.358 |
| BPC1403 | 1.07E+6 | 2.95E-4 | 0.277 |
| BPC1404 | 4.99E+5 | 5.08E-4 | 1.02 |
| PascoH-474 GS removed | 6.29E+5 | 2.66E-4 | 0.423 |
| anti-CD20 mAb | No binding detected | | |

Binding of the mAb-dAbs to CD20 was assessed by flow cytometry using a CD20 positive cell line (Wein133). All mAb-dAbs (BPC1401-BPC1404) and the anti-CD20 control antibody showed a dose dependent increase in mean fluorescence intensity (MFI) (data not shown).

**Example 15.3: ADCC assay with anti-CD20/IL-13 bispecific antibody**

The ADCC assay was based on the published method of Boyd et al. (1995) J. Imm. Meth. 184:29-38. Briefly, Raji cells (targets) were labelled with Europium as follows. Cells were harvested, counted and prepared to a final density of $1x10^7$ in a 15ml falcon tube, wash once with Hepes buffer (50mM HEPES, 83mM NaCl, 5mM KCl, 2mM $MgCl_2.H_20$, pH7.4). The cells were pelleted and 1ml of ice cold Europium labelling buffer (HEPES buffer plus 600μM $EuCl_3$, 3mM DTPA and 25mg/ml Dextran sulphate) was added to each tube. The cell suspension was flicked vigorously at the start of the labelling and then every 10 minutes during the 30 minute incubation period on ice. 10ml ice cold repair buffer (Hepes buffer containing 294mg/l $CaCl_2.2H_2O$, 1.8g/l D-Glucose, pH7.4) was added and the cells incubated on ice for a further 10 minutes. The cells were then centrifuged, the supernatant decanted and washed twice with repair buffer and then once with complete medium. The labelled cells were then counted and resuspended in serum free medium at $2x10^5$ cells/ml and stored on ice.

Human purified blood mononuclear cells (PBMCs or effector cells) were prepared as follows. 150mls of whole blood was centrifuged at 2000rpm for 10mins to remove the serum. The cells were the diluted to twice the original volume with PBS (Invitrogen/Gibco, #14190). Accuspin density gradient tubes (Sigma, #A2055-10EA) were prepared by adding 15ml lymphoprep (Axis shield, #NYC1114547) and centrifuged for 1min at 1500rpm. 25ml of blood suspension was added to the density gradient tubes and centrifuged for 20min at 2500rpm with the centrifuge brake off. The top 10ml of supernatant was discarded. The remainder (including the "buffy" layer) was poured into a clean tube, topped up with PBS and centrifuged at 1500rpm for 5mins. The supernatant was discarded, the cell pellets pooled, wash once in RPMI medium, recentrifuged and counted. Effector cells at were prepared at $5x10^6$/ml in serum free RPMI medium.

The assay plates were set up in 96-well round bottom plates (Nunc 96 maxisorb plate, #735-0199) as follows. Antibody dilutions were made in serum free RPMI medium at a starting concentration of approximately 12μg/ml and eleven further 3-fold dilutions. Using the plate layout below, 50μl antibody sample was added to the appropriate wells (rows B-G only), allowing 6 replicates per dilution). 50μl RPMI medium was added to all wells in rows A and H. 50μl of RPMI medium was added to all wells in plates labelled medium. 50μl recombinant human IL-13 diluted in RPMI medium to 4μg/ml (1μg/ml final concentration, GSK in-house material) was added to all wells in plates labelled +IL13. All. plates were incubated at 4°C for minimum 30 minutes. 50μl of Europium labelled target cells were added to all plates. 20μl of a 10X triton was added to all wells in row H on all plates. Plates were incubated 4°C for a minimum of 30 minutes. 50μl RPMI medium was added to all wells in columns labelled targets alone. 50μl PBMCs was added to all wells in columns labelled effector:targets to give a 25:1 ratio. The plates were centrifuged at 1500rpm for 3mins and incubated at 37°C

for 3-4hrs. 200μl of enhancement solution (Wallac/Perkin. Elmer, Catalogue# 1244-105) was added to each well of a nunc immunosorbant ELISA plates (one ELISA plate for each assay plate). 20μl of supernatant was transferred from assay plate to ELISA plate. The ELISA plates were incubated at room temperature on plate shaker for a minimum 30 minutes or stored over night at 4°C. Europium release is measured using time-delayed fluorimetry (Wallac Victor plate reader). Spontaneous lysis = measurement of Europium released from cells and medium alone. Maximum lysis = non-specific lysis of target cells by addition of Triton-X100 (non-ionic detergent).

| | Effector:Targets | | | Targets | | | Effector:Targets | | | Targets | | |
|---|---|---|---|---|---|---|---|---|---|---|---|---|
| | 1 | 2 | 3 | 4 | 5 | 6 | 7 | 8 | 9 | 10 | 11 | 12 |
| A | Spontaneous release | | | | | | Spontaneous release | | | | | |
| B | 3μg/ml | | | | | | 0.003μg/ml | | | | | |
| C | 1μg/ml | | | | | | 0.001μg/ml | | | | | |
| D | 0.3μg/ml | | | | | | 0.0003μg/ml | | | | | |
| E | 0.1μg/ml | | | | | | 0.0001μg/ml | | | | | |
| F | 0.03μg/ml | | | | | | 0.00003μg/ml | | | | | |
| G | 0.01μg/ml | | | | | | 0.00001μg/ml | | | | | |
| H | Maximum release | | | | | | Maximum release | | | | | |

The ADCC assay was performed on two separate occasions using two different donor PBMCs. The results from one representative assay are presented in Figures 70 and 71. In addition, a similar ADCC assay using a shorter dose range was performed on a separate occasion using different donor PBMCs. The results from this assay are presented in Figure 72 and 73.

**Example 15.4: CDC assay with anti-CD20/IL-13 bispecific antibody**

WEIN cells (targets) were labelled with Europium as follows. Briefly, cells were harvested, counted and prepared to a final density of $1x10^7$ in a 15ml falcon tube, wash once with Hepes buffer (50mM HEPES, 83mM NaCl, 5mM KCl, 2mM $MgCl_2.H_20$, pH7.4). The cells were pelleted and 1 ml of ice cold Europium labelling buffer (HEPES buffer plus 600μM $EuCl_3$, 3mM DTPA and 25mg/ml Dextran sulphate) was added to each tube. The cell suspension was flicked vigorously at the start of the labelling and then every 10 minutes during the 30 minute incubation period on ice. 10ml ice cold repair buffer (Hepes buffer containing 294mg/l $CaCl_2.2H_2O$, 1.8g/l D-Glucose, pH7.4) was added and the cells incubated on ice for a further 10 minutes. The cells were then centrifuged; the supernatant decanted and washed twice with repair buffer and then once with complete medium. The labelled cells were then counted and resuspended in serum free medium at $2x10^5$ cells/ml and stored on ice.

Serum was removed from whole blood collected from in house donors by centrifugation. Half of the sample was inactivated by heat treatment at 56°C for 30mins. Antibodies samples were diluted in serum free RPMI medium, starting a 12μg/ml with five further 3-fold dilutions. 50/μl antibody sample was added to appropriate wells in rows B-G only (as per the plate layout below). 50μl RPMI medium was added to all wells in columns 1 to 6. Where indicated, 50μl recombinant human IL-13 (at 4μg/ml in RPMI medium) was added to all wells in columns 7 to 12. The plates were incubated at 4°C for a minimum 30 minutes. 50μl of Europium labelled target cells were added to all the plates and the plates incubated at 4°C for minimum of 30 minutes. 50μl of serum (active or heat-inactivated) was added to the appropriate wells (see plate layout below). The plates were incubated at 37°C incubator for 2-3hrs, after which time the plates were centrifuged at 1500rpm for 3mins. 200μl of enhancement solution (Wallac/Perkin Elmer, Catalogue# 1244-105) was added to each well of a Nunc immunosorbant ELISA plates (one ELISA plate for each assay plate). 20μl of supernatant was transferred from assay plate to ELISA plate. The ELISA plates were incubated at room temperature on plate shaker for a minimum 30 minutes or stored over night at 4°C. Europium release is measured using time-delayed fluorimetry (Wallac Victor plate reader). Spontaneous lysis = measurement of Europium released from cells and medium alone. Maximum lysis = non-specific lysis of target cells by addition of Triton-X100 (non-ionic detergent).

| | MEDIUM | | | | | | IL13 | | | | | |
|---|---|---|---|---|---|---|---|---|---|---|---|---|
| Active complement | | | | Heat treated | | | Active complement | | | Heat treated | | |
| | 1 | 2 | 3 | 4 | 5 | 6 | 7 | 8 | 9 | 10 | 11 | 12 |
| A | Spontaneous release | | | | | | | | | | | |
| B | 3μg/ml | | | | | | | | | | | |
| C | 1μg/ml | | | | | | | | | | | |
| D | 0.3μg/ml | | | | | | | | | | | |
| E | 0.1μg/ml | | | | | | | | | | | |
| F | 0.03μg/ml | | | | | | | | | | | |
| G | 0.01μg/ml | | | | | | | | | | | |
| H | Maximum release | | | | | | | | | | | |

The CDC assay was performed on three separate occasions using three different donor sera. The results from one representative assay are presented in Figures 74 and 75 and show that the CDC activity of the antibody samples is comparable in the absence of IL-13. In the presence of excess IL-13, the CDC activity of antibody samples BPC1401 and BPC1402 (domain antibody fused to the heavy chain) is reduced whilst the CDC activity of BPC1403 and BPC1404 (domain antibody fused to the light chain) is largely unaffected by the presence of IL-13.

## Example 16

### 16.1 Design and construction of antigen binding proteins comprising epitome binding domains composed of alternative scaffolds

Five alternative scaffolds, listed below, were combined with monoclonal antibodies to provide mAb-alternative scaffold bispecific molecules:

- anti VEGF tear lipocalin (TLPC)
- anti HER2 Affibody (AFFI)
- anti HER2 DARPin (DRPN)
- anti hen egg white lysozyme (NARV)
- anti-RNaseA Camelid VHH

The protein sequences of TLPC (for further information see US2007/0224633), AFFI (for further information see WO2005003156A1), DRPN (for further information see Zahnd, C. et al. (2007), J. Mol. Biol., 369, 1015-1028) and NARV (for further information see US20050043519A) were reverse-translated to DNA and codon optimised. A BamHI site at the N-terminus and EcoR1 site at the C-terminus were included on each of these four alternative scaffolds to facilitate cloning.

DNA fragments encoding the four final alternative scaffold DNA sequences were constructed de novo using a PCR-based strategy and overlapping oligonucleotides. The TLPC, AFFI and DRPN PCR products were cloned into mammalian expression vectors containing the heavy chain of H0L0, an anti-hIGF-1R antibody. The resulting DNA sequences encode the alternative scaffolds fused onto the C-terminus of the heavy chain via a TVAAPSGS linker or GS linker. The NAR V PCR product was cloned into mammalian expression vectors containing DNA encoding the heavy chain of Pascolizumab (an anti-IL-4 antibody). The resulting DNA sequence encodes the NAR V fused onto the C-terminus of the heavy chain via a GS linker.

An anti-RNAse A camelid VHH DNA sequence was modified by PCR to include a BamHI site at the 5' end and an EcoR1 site at the 3' end in order to facilitate cloning. The PCR product was cloned into mammalian expression vectors containing the heavy chain of Pascolizumab, an anti-IL4 antibody. The resulting DNA sequence encodes a camelid VHH fused onto the C-terminus of the heavy chain via a GS linker.

Table 21 below is a summary of the antigen binding proteins that have been constructed.

Table 21

| Antibody ID | Description | SEQ ID NO: amino acid sequence |
|---|---|---|
| BPC1803 | antiIGF1R Heavy Chain-GS-TLPC | 123 |
| | antiIGF1R Light Chain | 113 |
| BPC1804 | antiIGF1R Heavy Chain-TVAAPSGS-TLPC | 125 |
| | antiIGF1R Light Chain | 113 |
| BPC1805 | antiIGFIR Heavy Chain-GS-AFFI | 126 |
| | antiIGF1R Light Chain | 113 |
| BPC1806 | antiIGF1R Heavy Chain-TVAAPSGS-AFFI | 127 |
| | antiIGF1R Light Chain | 113 |
| BPC1807 | antiIGF1R Heavy Chain-GS-DRPN | 128 |
| | antiIGF1R Light Chain | 113 |
| BPC1808 | antiIGF1R Heavy Chain-TVAAPSGS-DRPN | 129 |
| | antiIGF1 R Light Chain | 113 |
| BPC1809 | Anti IL-4 heavy Chain-GS-anti RNAse A camelidVHH | 130 |
| | Anti IL-4 Light Chain | 15 |
| BPC1816 | Anti IL-4 heavy Chain-GS-NARV | 131 |
| | Anti IL-4 Light Chain | 15 |

Expression plasmids encoding the heavy and light chains of the antigen binding proteins set out in Table 21 were transiently co-transfected into HEK 293-6E cells using 293fectin (Invitrogen, 12347019). A tryptone feed was added to each of the cell cultures the same day or the following day and the supernatant material was harvested after about 2 to 6 days from initial transfection. The antigen binding protein was purified from the supernatant using a Protein A column before being tested in binding assays.

**16.2: rhIGF-1R Binding ELISA**

96-well high binding plates were coated with 1μg/ml of anti-his-tag antibody (Abcam, ab9108) in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1 hour at room temperature. Another wash step was then performed. 0.4μg/mL of rhIGF-1R (R&D systems) was added to each well at 50μL per well. The plates were incubated for an hour at room temperature and then washed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one, hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figures 76, 78 and 80 show the ELISA results and confirm that antigen binding proteins BPC1803 - BPC1808 bind to recombinant human IGF-1R. The anti-IGF-1R monoclonal antibody H0L0 also showed binding to recombinant human IGF-1R whereas the negative control antibody (sigma 15154) showed no binding to IGF-1R.

**16:3: VEGF Binding ELISA**

96-well high binding plates were coated with 0.4μg/mL of hVEGF165 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 77 shows the results of the VEGF binding ELISA and confirms that bispecific antibodies BPC1803 and BPC1804 bind to human VEGF. An anti VEGF bispecific antibody (BPC1603) was used as a positive control in this assay and showed binding to VEGF. In contrast the anti-IGF-1 R monoclonal antibody H0L0 showed no binding to human VEGF.

**16.4: HER2 Binding ELISA**

96-well high binding plates were coated with 1 μg/mL of HER2 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figures 79 and 81 show the results of the HER2 binding ELISA and confirms that the antigen binding proteins BPC1805, BPC1806, BPC1807 and BPC1808 bind to recombinant human HER2. Herceptin was used as a positive control in this assay and showed binding to HER2. In contrast the anti-IGF-1 R monoclonal antibody H0L0 showed no binding to human HER2.

**16.5: IL-4 Binding ELISA**

96-well high binding plates were coated with 5μg/ml of human IL-4 in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1hour at room temperature. Another wash step was then performed. The purified antigen binding proteins/antibodies were successively diluted across the plates in blocking solution. After 1-hour incubation, the plates were washed. Goat anti-human kappa light chain specific peroxidase conjugated antibody (Sigma, A7164) was diluted in blocking solution to 1 μg/mL and 50μL was added to each well. The plates were incubated for one hour. After another wash step, 50μl of OPD (o-phenylenediamirie dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 82 shows the results of the ELISA and confirms that antigen binding protein BPC1809 binds to human IL-4 at levels comparable to the anti IL-4 monoclonal antibody, Pascolizumab. The negative control antibody (Sigma 15154) showed no binding to IL-4.

Figure 84 shows the results of the ELISA and confirms that antigen binding protein BPC1816 binds to human IL-4 at levels comparable to the anti IL-4 monoclonal antibody, Pascolizumab. The negative control antibody (Sigma 15154) showed no binding to IL-4.

**16.6: RNAse A Binding ELISA**

50uL of 1 ug/mL RNAse A (Qiagen, 19101) that had been diluted in PBS was added to each well of a 96 well Costar plate. The plate was incubated for 2 hours at room temperature then washed with PBST before addition of 200uL of 4% BSA/PBS block to each well. The plate was incubated for an hour and washed before addition of the samples. Purified antibodies and antigen binding protein BPC1809 were added at a concentration of 2ug/mL in wells of column 1 then serially diluted 1 in 2 across the plate in block. The plate was incubated for an hour then washed. 50ul/well of Goat anti-human kappa light chain specific peroxidase conjugated antibody (Sigma, A7164) was added at a 1 in 1000 dilution. The plate was incubated for an hour then washed. 50uL of OPD was added to each well and the reaction was stopped with 3M sulphuric acid after 15-30 minutes. Absorbance was read at 490nm using the VersaMax Tunable Microplate Reader (Molecular Devices) using a basic endpoint protocol.

Figure 83 shows the results of the RNAse A binding ELISA and confirms that purified human monoclonal antibody-camelid VHH bispecific antibody BPC1809 shows binding to RNAse A. In contrast both the IL-4 monoclonal antibody Pascolizumab and the negative control (sigma 15154) showed no binding to RNAse A.

### 16.7: HEL Binding ELISA

A 96-well high binding plate was coated with 5μg/ml of HEL (Hen Egg Lysozyme, Sigma L6876) in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1 hour at room temperature. Another wash step was then performed. The purified antibodies were successively diluted across the plate in blocking solution. After 1 hour incubation, the plate was washed. Goat anti-human kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (molecular Devices) using a basic endpoint protocol.

Figure 85 shows the results of the HEL binding ELISA and confirms that purified human monoclonal antibody-NAR V bispecific antibody BPC1816 binds to HEL. In contrast the IL-4 monoclonal antibody Pascolizumab showed no binding to HEL.

## Example 17

### 17.1 Design and construction of antigen-binding proteins comprising epitope binding domains composed of adnectin

CT01 adnectin is specific for VEGFR2 (for further information see WO2005/056764). The CT01 adnectin protein sequence was reverse-translated to DNA and codon optimised. A BamHI site at the N-terminus and EcoR1 site at the C-terminus were included to facilitate cloning.

DNA fragments encoding the final CT01 DNA sequence were constructed de novo using a PCR-based strategy and overlapping oligonucleotides. The PCR product was cloned into mammalian expression vectors containing the heavy chain of H0L0 (an anti-hIGF-1 R antibody) allowing the adnectin to be fused onto the C-terminus of the heavy chain via either a GS linker or a TVAAPSGS linker. Protein sequences of the heavy and light chains of the IGF-1 R - VEGFR2 bispecific are given in SEQ ID numbers 124, 113 and 133.

Another adnectin protein sequence coding for an anti-TNF-α adnectin (for further information see US20080139791) was reverse translated to DNA, codon optimised and modified to include terminal BamHI and EcoR1 sites before being constructed using the overlapping oligonucleotide PCR method described previously. The PCR product was cloned into mammalian expression vectors containing DNA encoding the heavy chain of Pascolizumab (an anti-IL-4 antibody) allowing DNA encoding the anti-TNF-α adnectin to be fused onto the C-terminus of the heavy chain via either a GS linker or a TVAAPSGS linker. Protein sequences of the heavy and light chains of the IL-4 - TNF-α bispecific are given in SEQ ID NO: 146, 147 and 15.

Antigen binding proteins using the TNF-α specific adnectin fused at the C-terminus of the heavy chain of an IL-13 monoclonal antibody have also been designed. Example protein sequences are given in SEQ ID's 134, 13 and 135. In addition, bispecific molecules based on the fusion of CT01 at either the C-terminus or the N-terminus of the heavy or light chain of anti-EGFR antibodies Erbitux and IMC-11 F8 have been designed. Examples of protein sequences which have been designed are given in SEQ ID NO: 136-145.

Of these example sequences, a number were constructed. DNA sequences encoding SEQ ID NO 136 (CT01 fused onto the C-terminus of the Erbitux heavy chain), SEQ ID NO 144 (CT01 fused onto the N-terminus of Erbitux heavy chain) and SEQ ID NO 138 (CT01 fused onto the C-terminus of the Erbitux light chain) were constructed. All three sequences were constructed using PCR-based cloning methods and cloned into mammalian expression vectors. Table 22 below is a summary of the antigen binding proteins that have been designed and/or constructed:

Table 22

| Antibody ID | Description | SEQ ID NO: amino acid sequence |
|---|---|---|
| BPC1801 (constructed) | antiIGF1R Heavy Chain-GS-CT01 adnectin | 124 |
| | antiIGF1R Light Chain | 113 |

(continued)

| Antibody ID | Description | SEQ ID NO: amino acid sequence |
|---|---|---|
| BPC1802 (constructed) | antiIGF1R Heavy Chain-TVAAPSGS-CT01 adnectin | 133 |
| | antiIGF1R Light Chain | 113 |
| BPC1810 (designed) | antiIL13-Heavy Chaih-GS-antiTNFα adnectin | 134 |
| | antiIL13-Light Chain | 13 |
| BPC1811 (designed) | antiIL13-Heavy Chain-TVAAPSGS-antiTNFα adnectin | 135 |
| | antiIL13-Light Chain | 13 |
| BPC1812 (constructed) | Erbitux Heavy chain-RS-CT01 adnectin | 136 |
| | Erbitux Light Chain | 137 |
| BPC1813 (constructed) | Erbitux Light chain-RS-CT01 adnectin | 138 |
| | Erbitux Heavy Chain | 139 |
| BPC1814 (designed) | 11 F8 Heavy Chain-GS-CT01 adnectin | 140 |
| | 11 F8 Light Chain | 141 |
| BPC1815 | 11 F8 Light Chain-GS-CT01 adnectin | 142 |
| (designed) | 11 F8 Heavy Chain | 143 |
| BPC1818 (constructed) | GS-CT01 adnectin-GSTG- Erbitux Heavy Chain | 144 |
| | Erbitux Light Chain | 137 |
| BPC1819 (designed) | CT01 adnectin-STG-Erbitux Light Chain | 145 |
| | Erbitux Heavy Chain | 139 |
| BPC1823 (constructed) | Anti IL-4 Heavy Chain-GS-anti TNF-α adnectin | 146 |
| | Anti IL-4 Light Chain | 15 |
| BPC1822 (constructed) | Anti IL-4 Heavy Chain-TVAAPSGS- anti TNF-α adnectin | 147 |
| | Anti IL-4 Light Chain | 15 |

Expression plasmids encoding the heavy and light chains of BPC1801, BPC1802, BPC1822, BPC1823, BPC1812, BPC1813 and BPC1818 were transiently co-transfected into HEK 293-6E cells using 293fectin (Invitrogen, 12347019): A tryptone feed was added to the cell culture the same day or the following day and the supernatant material was harvested after about 2 to 6 days from initial transfection. In some instances the supernatant material was used as the test article in binding assays. In other instances, the antigen binding protein was purified using a Protein A column before being tested in binding assays.

**17.2: rhIGF-1R Binding ELISA**

96-well high binding plates were coated with 1μg/ml of anti-his-tag antibody (Abcam, ab9108) in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1 hour at room temperature. Another wash step was then performed. 0.4μg/mL of rhIGF-1R (R&D systems) was added to each well at 50μL per well. The plates were incubated for an hour at room temperature and then washed. The purified antigen binding proteins/antibodies were successively diluted across the plates in blocking solution. After 1 hour incubation, the plates were washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediamine dihydrochloride)

SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 86 shows the results of the IGF-1R binding ELISA and confirms that purified human monoclonal antibody-adnectin bispecific antibodies (BPC1801 and BPC 1802) bind to recombinant human IGF-1R at levels comparable to the anti-IGF-1 R monoclonal antibody H0L0. The negative control antibody (Sigma 15154) showed no binding to IGF-1 R.

## 17.3: VEGFR2 Binding ELISA

96-well high binding plates were coated with 0.4μg/mL of VEGFR2 (R&D Systems) and incubated at +4°C overnight. The plates were washed twice with Tris-Buffered Saline with 0.05% of Tween-20. 200μL of blocking solution (5% BSA in DPBS buffer) was added to each well and the plates were incubated for at least 1 hour at room temperature. Another wash step was then performed. The supernatants or purified antibodies were successively diluted across the plates in blocking solution. After 1 hour incubation, the plate was washed. Goat anti-human kappa light chain specific peroxidase conjugated antibody was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 87 shows the results of the VEGFR2 binding ELISA and confirms that purified human monoclonal antibody-adnectin bispecific antibodies (BPC1801 and BPC1802) bind to recombinant human VEGFR2. In contrast the anti-IGF-1 R monoclonal antibody H0L0 showed no binding to human VEGFR2.

Figure 172 shows the results of the VEGFR2 binding ELISA and confirms that antigen binding proteins BPC1818 and BPC1813 bind to recombinant human VEGFR2. In contrast Erbitux showed no binding to human VEGFR2. For the antigen binding proteins BPC1813 and BPC1818, the amount of antibody in the supernatant was not quantified thus the data presented in Figure 172 is represented as a dilution factor of the neat supernatant material. For Erbitux, purified material was used in the assay aT the starting concentration of 2μg/ml, which is equivalent to dilution factor of 1 in Figure 172.

Figure 175 shows the results of the VEGFR2 binding ELISA and confirms that antigen binding protein BPC1812 binds to recombinant human VEGFR2. In contrast Erbitux and the negative control Sigma IgG 15154 antibody showed no binding to human VEGFR2. For the antigen binding protein BPC1812, the amount of antibody in the supernatant was not quantified thus the data presented in Figure 175 is represented as a dilution factor of the neat supernatant material. For Erbitux and Sigma IgG 15154 purified material was used in the assay at the starting concentration of 2μg/ml, which is equivalent to dilution factor of 1 in Figure 175.

## 17.4: IL-4 Binding ELISA

96-well high binding plates were coated with 5μg/ml of human IL-4 in PBS and stored overnight at 4°C. The plates were washed twice with Tris-Buffered Saline with 0.05% of Tween-20. 200μL of blocking solution (5% BSA in DPBS buffer) was added in each well and the plates were incubated for at least 1 hour at room temperature. Another wash step was then performed. The supernatant or purified antibodies/antigen binding proteins were successively diluted across the plate in blocking solution. After 1 hour incubation, the plates were washed. Goat anti-human kappa light chain specific peroxidase conjugated antibody (Sigma, A7164) was diluted in blocking solution to 1μg/mL and 50μL was added to each well. The plates were incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 88 shows the results of the IL-4 binding ELISA and confirms that human monoclonal antibody-adnectin bispecific antibodies (BPC1823 and BPC 1822) bind to recombinant human IL-4. The positive control anti-IL-4 monoclonal antibody Pascolizumab showed binding to IL-4 and the negative control antibody (Sigma 15154) showed no binding to IL-4.

The HEK transfection for this experiment was repeated to obtain supernatant material with a higher antibody concentration. Figure 88b shows binding of this higher concentration supernatant human monoclonal antibody-adnectin bispecific antibody (BPC1823) to human IL-4 as determined by ELISA

For the antigen binding proteins BPC1823 and BPC1822, the amount of antibody in the supernatant was not quantified thus the data presented in Figure 88 and 88b is represented as a dilution factor of the neat supernatant material. For Pascolizumab and the negative control antibody (Sigma 15154), purified material was used in the assay and the starting concentration of 1μg/ml, which is equivalent to dilution factor of 1 in Figure 88 and 88b.

## 17.5: TNF-A Binding ELISA

A 96-well high binding plate was coated with 0.4μg/ml of recombinant human TNFα (RnD Systems 210-TA-050/CF) in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1 hour at room temperature. Another wash step was then performed. The supernatant or purified antibodies were successively diluted across the plate in blocking solution. After 1 hour incubation, the plate was washed. Goat anti-human kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediamine dihydrochloride) SigmaFast substrate solution was added to each well and the reaction was stopped 15 minutes later by addition of 25μL of 3M sulphuric acid. Absorbance was read at 490nm using the VersaMax Tunable Microplate Reader (Molecular Devices) using a basic endpoint protocol.

Figure 89 shows the results of the TNF-α binding ELISA and confirms that human monoclonal antibody-adnectin bispecific antibodies (BPC1823 and BPC1822) bind to recombinant human TNF-α. In contrast, the anti-IL-4 monoclonal antibody Pascolizumab showed no binding to recombinant human TNF-α.

The HEK transfection for this experiment was repeated to obtain supernatant material with a higher antibody concentration. Figure 89b shows binding of this higher concentration supernatant human monoclonal antibody-adnectin bispecific antibody (BPC1823) to recombinant human TNF-α as determined by ELISA. The IgG control showed no binding to recombinant human TNF-α.

For the antigen binding proteins BPC1822 and BPC823, the amount of antibody in the supernatant was not quantified thus the data presented in Figure 89 and 89b is represented as a dilution factor of the neat supernatant material. For Pascolizumab, purified material was used in the assay at the starting concentration of 1μg/ml, which is equivalent to dilution factor of 1 in Figure 89 and 89b.

## 17.6: EGFR Binding ELISA

A 96-well high binding plate was coated with 0.67μg/ml of recombinant human EGFR protein in PBS and stored overnight at 4°C. The plate was washed twice with Tris-Buffered Saline with 0.05% of Tween-20. 200μL of blocking solution (5% BSA in DPBS buffer) was added in each well and the plate was incubated for at least 1hour at room temperature. Another wash step was then performed. The antigen binding proteins/antibodies were successively diluted across the plate in blocking solution. After 1 hour incubation, the plate was washed. Goat anti-human kappa light chain specific peroxidase conjugated antibody (Sigma, A7164) was diluted in blocking solution to 1μg/mL and 50μL was added to each well. The plate was incubated for one hour. After another wash step, 50μl of OPD (o-phenylenediamine dihydrochloride) SigmaFast substrate solution was added to each well and the reaction was stopped 25 minutes later by addition of 25μL of 3M sulphuric acid. Absorbance was read at 490nm using the VersaMax Tunable Microplate Reader (Molecular Devices) using a basic endpoint protocol.

Figure 171 shows the results of the EGFR binding ELISA and confirms that bispecific antibodies BPC1818 and BPC1813 bind to recombinant human EGFR. The positive control antibody, Erbitux, also showed binding to recombinant human EGFR. In contrast the Sigma IgG 15154 showed no binding to recombinant human EGFR. For the bispecific antibodies BPC1813 and BPC1818, the amount of antibody in the supernatant was not quantified thus the data presented in Figure 171 is represented as a dilution factor of the neat supernatant material. For Erbitux and the negative control antibody (Sigma 15154), purified material was used in the assay and the starting concentration of 2μg/ml and 1μg/ml respectively, which is equivalent to dilution factor of 1 in Figure 171.

Figure 176 shows the results of the EGFR binding ELISA and confirms that bispecific antibodies BPC1812 binds to recombinant human EGFR. The positive control antibody, Erbitux, also showed binding to recombinant human EGFR. In contrast the Sigma IgG 15154 showed no binding to recombinant human EGFR. For the bispecific antibodies BPC1812, the amount of antibody in the supernatant was not quantified thus the data presented in Figure 176 is represented as a dilution factor of the neat supernatant material. For Erbitux and the negative control antibody (Sigma 15154), purified material was used in the assay at the starting concentration of 2μg/ml and 1μg/ml respectively, which is equivalent to dilution factor of 1 in Figure 176.

## Example 18

### Binding activity data of IL-13/IL-4 mAbdAbs where the 'G and S' amino acid residues have been removed.

18.1 Construction of mAbdAbs

mAbdAbs were constructed in which the G and S amino acid residues (next to the linker sequence) were removed. Expression plasmids were constructed using standard molecular biology techniques. These mAbdAbs are described in Table 23. They were cloned, then expressed in one or more of HEK293-6E cells, CHOK1 cells, or CHOE1a cells, they were purified (as described in examples 1, 1.3 and 1.5 respectively) and analysed in a number of IL-13 and IL-4 activity assays.

Table 23

| Name | Description | Sequence ID No. |
|---|---|---|
| PascoH-474 GS removed | H chain = Pascolizuniab heavy chain-DOM10-53-474 dAb<br><br>L chain = Pascolizumab light chain | 91 (=H chain)<br>15 (=L chain) |
| PascoH-TVAAPS-474 GS removed | H chain = Pascolizumab heavy chain-TVAAPS-DOM10-53-474 dAb<br><br>L chain = Pascolizumab light chain | 92 (=H chain)<br>15 (=L chain) |
| PascoH-GS-ASTKGPT-474 2nd GS removed | H chain = Pascolizumab heavy chain-GS-ASTKGPT-DOM10-53-474 dAb<br><br>L chain Pascolizumab light chain | 96 (=H chain)<br>15 (=L chain) |
| 586H-210 GS removed | H chain = Anti-human IL-13 mAb heavy chain-DOM9-112-210 dAb<br><br>L chain = Anti-human IL-13 mAb light chain | 87 (=H chain)<br>13 (=L chain) |
| 586H-TVAAPS-210 GS removed | H chain = Anti-human IL-13 mAb heavy chain-TVAAPS-DOM9-112-210 dAb<br><br>L chain = Anti-human IL-13 mAb light chain | 88 (=H chain)<br>13 (=L chain) |

18.2 Expression and purification

These mAbdAbs were purified and analysed by SEC and SDS PAGE. A number of purified preparations were made and the SEC and SDS PAGE data shown in Figures 90 to 98 are representative of these preparations.

18.3 Binding to human IL-4 in a direct binding ELISA

Purified PascoH-474 GS removed and PascoH-TVAAPS-474 GS removed were tested for binding to human IL-4 in a direct binding ELISA as described in method 2 (PascoH-474, PascoH-TVAAPS-474, PascoH-ASTKG-474 and PascoH-ELQLE-474 were also tested for binding in this assay). A number of ELISA assays have been completed for these molecules, the data shown in Figure 99 is representative of these assays.

PascoH-474 GS removed and PascoH-TVAAPS-474 GS removed, both bound human IL-4. Purified anti-human IL4 mAb alone (Pascolizumab) was included in this assay as a positive control for binding to IL-4. Purified anti-human IL13 mAb was included as a negative control for IL-4 binding. The binding activity of PascoH-474 GS removed and PascoH-TVAAPS-474 GS removed was similar to purified anti-IL4 mAb alone (Pascolizumab), PascoH-474, PascoH-TVAAPS-474, PascoH-ASTKG-474 and PascoH-ELQLE-474.

18.4 Binding to human IL-1.3 in a direct binding ELISA

Purified PascoH-474 GS removed and PascoH-TVAAPS-474 GS removed were also tested for binding to human IL-13 in a direct binding ELISA as described in method 1 (PascoH-616 and PascoH-TVAAPS-616 were also tested for binding in this assay, the generation of these molecules is described in Example 19). A number of ELISA assays have been completed for these molecules, the data shown in Figure 100 is representative of all assays.

PascoH-474 GS removed, PascoH-TVAAPS-474 GS removed, PascoH-616 and PascoH-TVAAPS-616 all bound to human IL-13. Purified anti-human IL4 mAb alone (Pascolizumab) was included in this assay as a negative control for binding to IL-13. Purified anti-human IL13 mAb was included as a positive control for IL-13 binding. Note that the anti-IL-13 dAb alone (DOM10-53-474) was not tested in this assay as the dAb is not detected by the secondary detection antibody; instead, the anti-human IL13 mAb was used as a positive control to demonstrate IL-13 binding in this assay.

18.5 Binding to cynomolgus IL-13 in a direct binding ELISA

Purified PascoH-474 GS removed, PascoH-TVAAPS-474 GS removed, PascoH-616 and PascoH-TVAAPS-616 mAbdAbs were also tested for binding to cynomolgus IL-13 in a direct binding ELISA (as described in method 17). A number of ELISA assays have been completed for these molecules, the data shown in Figure 101 is representative of all assays.

PascoH-474 GS removed, PascoH-TVAAPS-474 GS removed, PascoH-616 and PascoH-TVAAPS-616 all bound cynomolgus IL-13. Purified anti-human IL4 mAb alone (Pascolizumab) was included in this assay as a negative control for binding to IL-13. Purified anti-human IL13 mAb was included as a positive control for cynomolgus IL-13 binding. Note that the anti-IL-13 dAbs alone (DOM10-53-474 and DOM10-53-616) were not tested in this assay as the dAb is not detected by the secondary detection antibody; instead, the anti-human IL13 mAb was used as a positive control to demonstrate IL-13 binding in this assay.

18.6 Biacore analysis for binding to human IL-4 and human IL-13

Purified mAbdAbs were tested for binding to human IL-4 and human IL-13 using the BIAcore™ T100 at 25°C (as described in methods 4 and 5). These data are shown in Table 24.

In experiment 1, a mAbdAb capture level of approximately 600 relative response units was achieved and six IL-13 and IL-4 concentration curves (256nM, 64nM, 16nM, 4nM, 1 nM and 0.25nM) were assessed. Only one IL-13 (256nM) and IL-4 (256nM) concentration curve was assessed for the mAbs in experiment 1.

In experiment 2, a mAbdAb a capture level of approximately 400 relative response units was achieved and six IL-4 (64nM, 16nM, 4nM, 1 nM, 0.25nM and 0.0625nM) and six IL-13 concentration curves (256nM, 64nM, 16nM, 4nM, 1nM and 0.25nM) were assessed. In experiment 2, onty one IL-13 concentration curve (256nM) was assessed for the anti-IL13 mAb and five IL-4 concentration curves (64nM, 16nM, 4nM, 1nM and 0.25nM) were assessed for Pascolizumab.

In experiment 3, a mAbdAb or mAb capture level of approximately 700 relative response units was achieved and six IL-4 concentration curves (256nM, 64nM, 16nM, 4nM, 1nM and 0.25nM) and six IL-13 concentration curves (256nM, 64nM, 16nM, 4nM, 1nM and 0.25nM) were assessed.

Table 24

| **Molecule** (purified material) | **Binding affinity, KD (nM)** | | | | | |
|---|---|---|---|---|---|---|
| | Human IL-4 | | | Human IL-13 | | |
| | Experiment 1 | Experiment 2 | Experiment 3 | Experiment 1 | Experiment 2 | Experiment 3 |
| PascoH-474 GS removed | not done | 0.005 | not done | 0.3307 | 0.351 | not done |
| PascoH-TVAAPS-474 GS removed | not done | 0.011 | not done | 0.2677 | 0.305 | not done |
| 586H-210 GS removed | not done | not done | very tight binder* | not done | not done | 0.438 |

(continued)

| **Molecule** (purified material) | **Binding affinity, KD (nM)** | | | | | |
|---|---|---|---|---|---|---|
| | Human IL-4 | | | Human IL-13 | | |
| | Experiment 1 | Experiment 2 | Experiment 3 | Experiment 1 | Experiment 2 | Experiment 3 |
| 586H-TVAAPS-210 GS removed | not done | not done | very tight binder* | not done | not done | 0.501 |
| Anti-human IL-13 mAb | does not bind | does not bind | does not bind | 0.2799 | 0.31 | 0.547 |
| Pascolizumab | 0.0137 | 0.011 | 0.013 | does not bind | does not bind | does not bind |

In experiments 1 and 2, PascoH-474 GS removed and PascoH-TVAAPS-474 GS removed, both bound IL-4 with similar binding affinities and this was approximately equivalent to the binding affinity of the anti-human IL4 mAb alone (Pascolizumab). PascoH-474 GS removed and PascoH-TVAAPS-474 GS removed, also bound IL-13 with similar binding affinities. Note that the anti-IL-13 dAb alone (DOM10-53-474) was not tested in this assay as the dAb cannot be captured onto the Protein A or anti-human IgG coated CM5 chip; instead, the anti-human IL13 mAb was used as a positive control to demonstrate IL-13 binding in this assay.

In experiment 3, 586H-210 GS removed and 586H-TVAAPS-210 GS removed, both bound IL-13 with similar binding affinities and this was approximately equivalent to the binding affinity of the anti-human IL13 mAb. 586H-210 GS removed and 586H-TVAAPS-210 GS removed, also bound I L-4 very tightly, however this method was unable to determine the binding affinity due to positive dissociation effects and the sensitivity level of the BIAcore™ technique (*). Note that the anti-IL-4 dAb alone (DOM9-112-210) was not tested in this assay as the dAb cannot be captured onto the Protein A or anti-human IgG coated CM5 chip; instead, the anti-human IL4 mAb (Pascolizumab) was used as a positive control to demonstrate IL-4 binding in this assay.

18.7 Biacore analysis for binding to cynomolgus IL-4 and cynomolgus IL-13

Purified mAbdAbs were tested for binding to cynomolgus IL-4 and cynomolgus IL-13 using the BIAcore™ T100 at 25°C (as described in methods 24 and 23). These data are shown in Table 25. A mAbdAb capture level of approximately 600 relative response units was achieved and six IL-13 concentration curves (256, 64, 16, 4, 1, 0.25nM) and five IL-4 concentration curves (64, 16, 4, 1, 0.25nM) were assessed.

Table 25

| **Molecule** (purified material) | **Binding affinity, KD** | | | | | |
|---|---|---|---|---|---|---|
| | Cynomolgus IL-13 | | | Cynomolgus IL-4 | | |
| | on rate (ka, $Ms^{-1}$) | off rate (kd, $s^{-1}$) | KD (nM) | on rate (ka, $Ms^{-1}$) | off rate (kd, $s^{-1}$) | KD (pM) |
| PascoH-474 GS removed | 6.62E+5 | 1.10E-2 | 16.6 | 1.87E+6 | 6.38E-5 | 34.2 |
| PascoH-TVAAPS-474 GS removed | 4.83E+5 | 1.29E-2 | 26.7 | 1.83E+6 | 5.30E-5 | 29.0 |
| PascoH-ASTKGPT-474 2$^{nd}$ GS removed | 4.79E+5 | 1.14E-2 | 23.8 | 1.83E+6 | 5.30E-5 | 29.0 |
| PascoH-474 | 5.86E+5 | 1.09E-2 | 18.6 | 1.85E+6 | 8.14E-5 | 43.9 |
| PascoH-TVAAPS-474 | 4.33E+5 | 1.17E-2 | 27.1 | 1.80E+6 | 8.85E-5 | 49.1 |
| PascoH-ASTKG-474 | 3.64E+5 | 1.07E-2 | 29.5 | 1.78E+6 | 7.90E-5 | 44.5 |

PascoH-474 GS removed, PascoH-TVAAPS-474 GS removed, PascoH-ASTKGPT-474 2$^{nd}$ GS removed, PascoH-474, PascoH-TVAAPS-474 and PascoH-ASTKG-474 all bound cynomolgus IL-4 with similar binding affinities. PascoH-474 GS removed, PascoH-TVAAPS-474 GS removed, PascoH-ASTKGPT-474 2$^{nd}$ GS removed, PascoH-474, PascoH-TVAAPS-474 and PascoH-ASTKG-474, also all bound IL-13 with similar binding affinities.

Purified mAbdAbs were also tested for binding to cynomolgus IL-4 and cynomolgus IL-13 using the BIAcore™ T100 at 25°C (as described in methods 24 and 23). These data are shown in Table 26. A mAbdAb capture level of approximately 600 relative response units was achieved and six IL13 and six IL-4 concentration curves (256, 64, 16, 4, 1 and 0.25nM) were assessed.

Table 26

| **Molecule** (purified material) | **Binding affinity, KD** | | | | | |
|---|---|---|---|---|---|---|
| | Cynomolgus IL-13 | | | Cynomolgus IL-4 | | |
| | on rate (ka, $Ms^{-1}$) | off rate (kd, $s^{-1}$) | KD (pM) | on rate (ka, $Ms^{-1}$) | off rate (kd, $s^{-1}$) | KD (pM) |
| 586H-TVAAPS-210 GS removed | 4.92E+5 | 2.86E-5 | 58 | very tight binder* | very tight binder* | very tight binder* |
| 586H-210 GS removed | 5.07E+5 | 2.24E-5 | 44 | very tight binder* | very tight binder* | very tight binder* |
| Anti-IL13 mAb | 4.74E+5 | 1.05E-4 | 222 | does not bind | does not bind | does not bind |
| Pascolizumab | does not bind | does not bind | does not bind | 2.34E+6 | 1.08E-4 | 46 |

586H-210 GS removed and 586H-TVAAPS-210 GS removed, both bound cynomolgus IL-13 with similar binding affinities; these mAbdAbs appeared to bind IL-13 more potently than the anti-human IL13 mAb, however in the case of the mAb only one concentration curve was completed which is inherently less accurate than a full concentration range assessment. 586H-210 GS removed and 586H-TVAAPS-210 GS removed, also bound IL-4 very tightly, however this method was unable to determine the binding affinity due to positive dissociation effects and the sensitivity level of the BIAcore™ technique (*). Note that the anti-IL-4 dAb alone (DOM9-112-210) was not tested in this assay as the dAb cannot be captured onto the Protein A or anti-human IgG coated CM5 chip; instead, the anti-human IL4 mAb (Pascolizumab) was used as a positive control to demonstrate IL-4 binding in this assay.

18.8 Biacore analysis of effect of IL-4 binding to mAbdAbs on subsequent IL-13 binding kinetics and vice versa; and the effect of IL-13 binding to mAbdAbs on subsequent IL-4 binding kinetics and vice versa.

The IL-13 and IL-4 BIAcore™ binding assays were also used to investigate the effect of IL-4 binding to PascoH-474 GS removed on subsequent IL-13 binding kinetics and vice versa; and the effect of IL-13 binding to 586H-TVAAPS-210 on subsequent IL-4 binding kinetics and vice versa. Analyses were carried out on the BIAcore™ T100 machine at 25°C, using anti-human IgG capture of the mAbdAb (or positive control mAb). Briefly, anti-human IgG was coupled onto a CM5 chip by primary amine coupling in accordance with the manufactures recommendations. mAbdAb constructs (or positive control mAb) were then captured onto this surface (at approximately 250 to 750RUs) and the first analyte (either human IL-13 or human IL-4) was passed over at 256nM for 4 minutes. The second analyte (human IL-4 or human IL-13 respectively) was then passed over at concentrations of 256nM, 64nM, 16nM, 4nM, 1nM and 0.25nM, and for double referencing a buffer injection was passed over the capture antibody or mAbdAb surface. The data was analysed (fitted to the 1:1 model of binding) using the evaluation software in the machine. The surface was then regenerated using 3M magnesium chloride. The data from these experiments are shown in Tables 27 and 28.

Table 27

| Molecule | IL-13 Binding Kinetics | | | | | |
|---|---|---|---|---|---|---|
| | with human IL-4 bound to molecule | | | without human IL-4 bound to molecule | | |
| | on rate (ka, $Ms^{-1}$) | off rate (kd, $s^{-1}$) | KD (pM) | on rate (ka, $Ms^{-1}$) | off rate (kd, $s^{-1}$) | KD (pM) |
| PascoH-474 GS removed | 5.66E+5 | 2.31E-4 | 407.7 | 6.09E+5 | 2.59E-4 | 425.2 |
| 586H-TVAAPS-210 | 9.68E+5 | 3.15E-4 | 325.2 | 9.09E+5 | 3.47E-4 | 381.3 |

(continued)

| Molecule | IL-13 Binding Kinetics | | | | | |
|---|---|---|---|---|---|---|
| | with human IL-4 bound to molecule | | | without human IL-4 bound to molecule | | |
| | on rate (ka, $Ms^{-1}$) | off rate (kd, $s^{-1}$) | KD (pM) | on rate (ka, $Ms^{-1}$) | off rate (kd, $s^{-1}$) | KD (pM) |
| Anti-IL13 mAb | not tested | | | 1.01E+6 | 3.68E-4 | 366.1 |

The binding affinity of PascoH-474 GS removed for human IL-13 was similar, irrespective of whether human IL-4 was bound to this molecule or not. In addition, the binding affinity of 586H-TVAAPS-210 for human IL-13 was similar, irrespective of whether human IL-4 was bound to this molecule or not and it was also similar to the binding affinity of the anti-IL13 mAb for human IL-13.

The off-rates (kd) for IL-4 binding obtained for PascoH-474 GS removed and 586H-TVAAPS-210, are very slow and out of the sensitivity range of the BIAcore™ T100 hence could not be used as an accurate determination of the binding affinity (data not shown). However, the data do indicate that all of the constructs tested bind very tightly to human IL-4. Thus the binding affinity of Pasco-474 GS removed for human IL-4 was very tight, irrespective of whether human IL-13 was bound to this molecule or not. In addition, the binding affinity of 586H-TVAAPS-210 for human IL-4 was very tight, irrespective of whether human IL-13 was bound to this molecule or not.

18.9 Potency of mAbdAbs

mAbdAbs were tested for inhibition of human IL-4 binding to human IL-4Rα by ELISA, as described in Method 19. All of the molecules shown in Table 28 were tested in one experiment, however the data have been plotted on two graphs to distinguish between the curve plots (586H-TVAAPS-210 was run twice, this is labelled as sample 1 and sample 2 in table 25). These data are shown in Figures 102 and 103.

PascoH-474 GS removed, inhibited binding of human IL-4 to human IL4Rα similarly to Pascolizumab. 586H-210 GS removed, 586H-TVAAPS-210 GS removed, 586H-TVAAPS-210, 586H-210, 586H-G4S-210 and 586H-ASTKG-210 all inhibited binding of human IL-4 to human IL4Rα similarly to DOM9-112-210. Pascolizumab and DOM9-112-210 were included as positive controls for the inhibition of IL-4 binding to IL4Rα. DOM10-53-474 and an isotype-matched mAb (with specificity for an irrelevant antigen) were included as negative controls for the inhibition of IL-4 binding to IL4Rα.

These data were also used to determine $IC_{50}$ values for each molecule. The $IC_{50}$ value is the concentration of mAbdAb or mAb or dAb, which is able to inhibit binding of human IL-4 to human IL4Rα by 50%. The $IC_{50}$ values are shown in Table 28.

Table 28

| **Molecule** | **$IC_{50}$ (nM)** |
|---|---|
| PascoH-474 GS removed | 5.14 |
| Pascolizumab | 3.45 |
| DOM9-112-210 | 36.77 |
| 586H-210 | 26.79 |
| 586H-210 GS removed | 36.18 |
| 586H-TVAAPS-210 (sample 1) | 23.77 |
| 586H-TVAAPS-210 (sample 2) | 21.03 |
| 586H-TVAAPS-210 GS removed | 19.21 |
| 586H-ASTKG-210 | 27.32 |
| 586H-G4S-210 | 29.85 |
| DOM10-53-474 | No inhibition at concentration range tested |
| Negative control mAb | No inhibition at concentration range tested |

These data confirm that PascoH-474 GS removed behaves similarly to Pascolizumab and that all 586H-210 mAbdAb 'family members' behave similarly to the DOM9-112-210 dAb.

18.10 Neutralisation of human and cynomolgus IL-13 in TF-1 cell bioassays by mAbdAbs

A number of purified mAbdAbs were tested for neutralisation of human and cynomolgus IL-13 in TF-1 cell bioassays (as described in method 8 and method 20 respectively). Each molecule was tested between one and nine times in these assays, not all graphs are shown, but Figures 104 and 105 are representative graphs showing the neutralisation data for human IL-13 and cynomolgus IL-13 respectively. DOM10-53-474 was included as a positive control for neutralisation of human or cynomolgus IL-13 in the bioassays. A dAb with specificity for an irrelevant antigen (negative control dAb) was also included as a negative control for neutralisation of human or cynomolgus IL-13 in the bioassays.

PascoH-474 GS removed, PascoH-TVAAPS-474 GS removed and PascoH-ASTKG-474 2nd GS removed, as well as PascoH-616, PascoH-TVAAPS-616 and DOM10-53-616 (these are described in Example 19), fully neutralised the bioactivity of both human and cynomolgus IL-13 in TF-1 cell bioassays.

18.11 Neutralisation of human and cynomolgus IL-4 in TF-1 cell bioassays by mAbdAbs

A number of purified mAbdAbs were also tested for neutralisation of human and cynomolgus IL-4 in TF-1 cell bioassays (as described in method 9 and method 21 respectively). Each molecule was tested twice in these assays, not all graphs are shown, but Figures 106 and 107 are representative graphs (from the dataset) showing neutralisation data for human IL-4 and cynomolgus IL-4 respectively. The anti-IL13 mAb was included as a negative control and Pascolizumab was included as a positive control for neutralisation of human or cynomolgus IL-4 in the bioassays. In addition, PascoH-474, PascoH-TVAAPS-474, PascoH-ASTKG-474 and PascoH-G4S-474 were also tested for neutralisation of human and cynomolgus IL-4 in these bioassays.

PascoH-474 GS removed and PascoH-TVAAPS-474 GS removed, fully neutralised the bioactivity of both human and cynomolgus IL-4 in TF-1 cell bioassays. In addition PascoH-474, PascoH-TVAAPS-474, PascoH-ASTKG-474 and PascoH-G4S-474 also fully neutralised the bioactivity of human IL-4 in the TF-1 cell bioassay.

$ND_{50}$ values were derived based on data obtained from a number of different experiments. The $ND_{50}$ value is the concentration of mAbdAb or mAb or dAb, which is able to neutralise the bioactivity of IL-13 or IL-4 by 50%. The mean $ND_{50}$ value, the standard deviation (SD) and the number of times tested (n) are shown in table 29.

Table 29

| Molecule | Mean $ND_{50}$ value & standard deviation (nM) | | | | | | | |
|---|---|---|---|---|---|---|---|---|
| | human IL-13 | | cyno IL-13 | | human IL-4 | | cyno IL-4 | |
| | mean (SD) | n | mean (SD) | n | mean (SD) | n | mean (SD) | n |
| **PascoH-474 GS removed** | 2.269 (0.763) | 9 | 39.834 (14.675) | 8 | 2.855 (1.464) | 2 | 1.89 (0.325) | 2 |
| **PascoH-TVAAPS-474 GS removed** | 2.114 (0.766) | 9 | 47.882 (13.181) | 9 | 3.015 (2.015) | 2 | 1.36 (0.41) | 2 |
| **PascoH-ASTKGPT-474 2nd GS removed** | 1.37 | 1 | 21.49 | 1 | not done | | not done | |
| **DOM10-53-474** | 1.035 (0.741) | 4 | 10.495 (6.958) | 4 | did not neutralise | | did not neutralise | |
| **Negative control dAb** | did not neutralise | | did not neutralise | | not done | | not done | |
| **Pascolizumab** | did not neutralise | | did not neutralise | | 1.36 (0.198) | 2 | 2.615 (2.242) | 2 |

PascoH-474 GS removed, PascoH-TVAAPS-474 GS removed, and PascoH-ASTKGPT-474 2nd GS removed, all fully neutralised the bioactivity of human and cynomolgus IL-13 in TF-1 cell bioassays. In addition, the neutralisation potencies ($ND_{50}$ values) of PascoH-474 GS removed, PascoH-TVAAPS-474 GS removed, and PascoH-ASTKGPT-474 2nd GS removed, for human IL-13 were similar and withinfold of the $ND_{50}$ value for purified anti-IL13 dAb alone (DOM10-53-474).

PascoH-474 GS removed and PascoH-TVAAPS-474 GS removed, both fully neutralised the bioactivity of human and cynomolgus IL-4 in TF-1 cell bioassays. In addition, the neutralisation potencies ($ND_{50}$ values) of PascoH-474 GS removed and PascoH-TVAAPS-474 GS removed, for human IL-13 and cyno IL-13 were similar and withinfold of of the $ND_{50}$ value for Pascolizumab.

18.12 Ability of mAbdAbs to inhibit binding of human IL-13 binding to human IL-13Rα2

The molecules listed in Table 30 were tested for inhibition of human IL-13 binding to human IL-13Rα2 by ELISA, as described in Method 22. All molecules were tested in one experiment. The data are shown in Figure 108.

All mAbdAbs tested inhibited binding of human IL-13 to human IL13Rα2. The level of inhibition was similar to that of DOM10-53-474, DOM10-53-616 and the anti-IL13 mAb. Pascolizumab and a negative control dAb (with specificity for an irrelevant antigen), were included as negative controls for the inhibition of IL-13 binding to IL13Rα2.

These data were also used to determine $IC_{50}$ values for each molecule. The $IC_{50}$ value is the concentration of mAbdAb or mAb or dAb, which is able to inhibit binding of human IL-13 to human IL13Rα2 by 50%. The $IC_{50}$ values are shown in Table 30.

Table 30

| Molecule | $IC_{50}$ (nM) |
|---|---|
| PascoH-474 GS removed | 9.58 |
| PascoH-TVAAPS-474 GS removed | 7.41 |
| DOM10-53-474 | 7.61 |
| PascoH-616 | 6.41 |
| PascoH-TVAAPS-616 | 6.17 |
| DOM10-53-616 | 5.76 |
| Anti-IL13 mAb | 6.43 |
| Pascolizumab | No inhibition at concentration range tested |
| Negative control dAb | No inhibition at concentration range tested |

These data confirm that PascoH-474 GS removed, PascoH-TVAAPS-474 GS removed, PascoH-616 and PascoH-TVAAPS-616 behaved similarly to DOM10-53-474, DOM10-53-616 and the anti-IL13 mAb.

**Example 19**

**mAbdAbs containing the anti-IL13 DOM10-53-616 dAb**

19.1 Construction of mAbdAbs containing the anti-IL13 DOM10-53-616 dAb

Two anti-IL4mAb-anti-IL13dAbs as set out in Table 31 were cloned from exisiting vectors by site-directed mutagenesis as described in examples 1.

Table 31

| Name | Description | Sequence ID No. |
|---|---|---|
| PascoH-616 | H chain = Pascolizumab heavy chain-DOM10-53-616 dAb<br><br>L chain = Pascolizumab light chain | 149 (=H chain)<br>15 (=L chain) |
| PascoH-TVAAPS-616 | H chain = Pascolizumab heavy chain-TVAAPS-DOM10-53-616 dAb<br><br>L chain = Pascolizumab light chain | 150 (=H chain)<br>15 (=L chain) |

19.2 Expression and purification of mAbdAbs containing the anti-IL13 DOM10-53-616 dAb

These mAbdAbs were expressed in HEK293-6E cells and CHOE1a cells as described in example 1.3.

The mAbdAbs were purified and analysed by SEC and SDS PAGE. A number of purified preparations of PascoH-616 and PascoH-TVAAPS-616 mAbdAbs were made, the SEC and SDS PAGE data shown in Figures 109 (SEC profile for PascoH-616), 110 (SEC profile for PascoH-TVAAPS_616), 111(SDS PAGE for PascoH-616) and 112 (SDS PAGE for PascoH-TVAAPS-616), are representative of these preparations.

19.3 Binding of mAbdAbs to human IL-13 in a direct binding ELISA

PascoH-616 and PascoH-TVAAPS-616 purified mAb dAbs were tested for binding to human IL-13 in a direct binding ELISA (as described in method 1). These data are shown in Figure 113.

Purified PascoH-616 and PascoH-TVAAPS-616 both bound human IL-13. Purified anti-human IL4 mAb alone (Pascolizumab) was included in this assay as a negative control for binding to IL-13. Purified anti-human IL13 mAb was included as a positive control for IL-13 binding. Note that the anti-IL-13 dAb alone (DOM10-53-616) was not tested in this assay as the dAb is not detected by the secondary detection antibody; instead, the anti-human IL13 mAb was used as a positive control to demonstrate IL-13 binding in this assay.

19.4 Biacore analysis for binding to human IL-4 and human IL-13

Purified mAbdAbs were tested for binding to human IL-4 and human IL-13 using the BIAcore™ T100 at 25°C (as described in methods 4 and 5). These data are shown in Table 32.

In experiment 1, a mAbdAb capture level of approximately 600 relative response units was achieved and six IL-13 and IL-4 concentration curves (256nM, 64nM, 16nM, 4nM, 1 nM and 0.25nM) were assessed. Only one IL-13 (256nM) and IL-4 (256nM) concentration curve was assessed for the mAbs in experiment 1.

In experiment 2, a mAbdAb a capture level of approximately 400 relative response units was achieved and six IL-4 (64nM, 16nM, 4nM, 1 nM, 0.25nM and 0.0625nM) and six IL-13 concentration curves (256nM, 64nM, 16nM, 4nM, 1 nM and 0.25nM) were assessed. In experiment 2, only one IL-13 concentration curve (256nM) was assessed for the anti-IL13 mAb and five IL-4 concentration curves (64nM, 16nM, 4nM, 1nM and 0.25nM) were assessed for Pascolizumab.

Table 32

| **Molecule** (purified material) | **Bindiny affinity, KD (nM)** | | | |
|---|---|---|---|---|
| | Human IL-4 | | Human IL-13 | |
| | Experiment 1 | Experiment 2 | Experiment 1 | Experiment 2 |
| PascoH-616 | 0.00172 | Very tight binder | 0.1137 | 0.15 |
| PascoH-TVAAPS-616 | 0.003 | 0.005 | 0.0497 | 0.056 |
| Anti-human IL-13 mAb | does not bind | does not bind | 0.2799 | 0.31 |
| Pascolizumab | 0.0137 | 0.011 | does not bind | does not bind |

PascoH-616 and PascoH-TVAAPS-616 both bound IL-4 with similar binding affinities and this was similar to the binding affinity of the anti-human IL4 mAb alone (Pascolizumab). PascoH-616 and PascoH-TVAAPS-616 both bound IL-13. Note that the anti-IL-13 dAb alone (DOM10-53-616) was not tested in this assay as the dAb cannot be captured onto the Protein A or anti-human IgG coated CM5 chip; instead, the anti-human IL13 mAb was used as a positive control to demonstrate IL-13 binding in this assay.

19.5 Biacore analysis for binding to cynomolgus IL-13

Purified mAbdAbs were also tested for binding to cynomolgus IL-13 using the BIAcore™ T100 at 25°C (as described in method and 30). These data are shown in Table 33. A mAbdAb capture level of approximately 400 relative response units was achieved and six IL-13 concentration curves (256, 64, 16, 4, 1 and 0.25nM) were assessed. There was only one IL-13 concentration curve (256nM) for the anti-IL13 mAb.

Table 33

| **Molecule** (purified material) | **Binding affinity for cynomolgus IL-13 (KD)** | | |
|---|---|---|---|
| | on rate (ka,$Ms^{-1}$) | off rate (kd, $s^{-1}$) | KD (nM) |
| PascoH-616 | 3.411E+5 | 1.842E-3 | 5.4 |
| PascoH-TVAAPS-616 | 4.597E+5 | 4.514E-3 | 9.8 |
| Anti-IL13 mAb | 5.498E+5 | 6.549E-5 | 0.119 |
| Pascolizumab | does not bind | does not bind | does not bind |

PascoH-616 and PascoH-TVAAPS-616 both bound cynomolgus IL-13 with similar binding affinities. Note that the anti-IL-13 dAb alone (DOM10-53-616) was not tested in this assay as the dAb cannot be captured onto the Protein A or anti-human IgG coated CM5 chip; instead, the anti-human IL13 mAb was used as a positive control to demonstrate IL-13 binding in this assay.

<u>19.6 Neutralisation of human and cynomolgus IL-13 in TF-1 cell bioassays by mAbdAbs</u>

Purified mAbdAbs were tested for neutralisation of human IL-13 and cynomolgus IL-13 in TF-1 cell bioassays (as described in method 8 and method 20 respectively). These molecules were tested 3 times in each assay, and Figure 114 is a representative graph showing the neutralisation data for human IL-13. Figure 114a is a representative graph showing the neutralisation data for cyno IL-13. DOM10-53-616 was included as a positive control for neutralisation of IL-13 in this bioassay. A dAb with specificity for an irrelevant antigen (negative control dAb) was also included as a negative control for neutralisation of IL-13 in this bioassay.

The mean $ND_{50}$ value, the standard deviation (SD) and the number of times tested (n) are shown in table 34.

Table 34

| **Molecule** | **Mean $ND_{50}$ value & standard deviation (nM)** | | | |
|---|---|---|---|---|
| | human IL-13 | | cyno IL-13 | |
| | mean (SD) | n | mean (SD) | n |
| **PascoH-616** | 0.7956 (0.129) | 3 | 9.23 (1.422) | 3 |
| **PascoH-TVAAPS-616** | 0.722 (0.245) | 3 | 14.477 (2.847) | 3 |
| **DOM10-53-616** | 0.416 (0.144) | 3 | 4.81 (3.266) | 3 |
| **Negative control dAb** | did not neutralise | | did not neutralise | |

Both PascoH-616 and PascoH-TVAAPS-616, as well as In addition mAbdAbs PascoH-TVAAPS-474 GS removed and PascoH-474 GS removed fully neutralised the bioactivity of human and cynomolgus IL-13 in TF-1 cell bioassays.
In addition, the neutralisation potencies ($ND_{50}$ values) of PascoH-616 and PascoH-TVAAPS-616 for human IL-13 were similar and within 2-fold of the $ND_{50}$ value for the purified anti-IL13 dAb alone (DOM10-53-616).
PascoH-616 and PascoH-TVAAPS-616 were also tested for inhibition of human IL-13 binding to human IL-13R$\alpha$2 by ELISA, as described in Method 22. These data are presented in Example 18.12

## <u>Example 20</u>

### <u>Ability of mAbdAbs to neutralise human IL-13 or IL-4 in a human whole blood phospho STAT6 bioassay</u>

The ability of mAbdAbs to neutralise human IL-13 or IL-4 in a human whole blood phospho STAT6 bioassay was carried out as described in Method 16.
The IL-4 or IL-13 neutralisation potencies (ie. inhibition of IL-4 or IL-13 bioactivity) of 2 mAbdAb constructs (the purified anti-IL13mAb-anti-IL4dAb, 586H-TVAAPS-210; and the purified anti-IL4mAb-anti-IL13dAb, PascoH-474 GS removed) were determined. Purified anti-human IL-4 mAb (Pascolizumab) and purified anti-IL4 dAb (DOM9-112-210) were included as positive controls for neutralisation of rhIL-4 in this assay. Purified anti-human IL-13 mAb and purified anti-IL13 dAb (DOM10-53-474) were included as positive controls for neutralisation of rhIL-13. An isotype matched mAb mixed with a dAb (both with specificities for irrelevant antigens), were included as a negative control for neutralisation of rhIL-4 or rhIL-13. Each molecule was tested at least twice, using blood from different donors. Figures 115 to 124 are graphs showing representative data.

The purified mAbdAbs fully neutralised the bioactivity of rhIL-13 and rhIL-4.

As described in method 16, the ability of the test molecules to neutralise rhIL-13 or rhIL-4 bioactivity was expressed as the concentration of the molecules (e.g. mAbdAbs) required to neutralise 2ng/mL of human IL-4 or human IL-13 by 50% ($IC_{50}$). These data are shown in Table 35. The combined mean $IC_{50}$ from all donors for each molecule is presented, along with the standard deviation.

Table 35

| Molecule | Target in assay | Mean $IC_{50}$ (standard deviation) nM | Number of donors |
|---|---|---|---|
| 586-TVAAPS-210 | IL-4 | 1.23 (0.6) | 3 |
| | IL-13 | 2.68 (1.2) | 3 |
| PascoH-474 GS removed | IL-4 | 7.95(7.8) | 3 |
| | IL-13 | 2.78 (0.7) | 3 |
| Anti-IL13 mAb | IL-13 | 1.47 (0.4) | 3 |
| Pascolizumab | IL-4 | 2.44(1.2) | 3 |
| DOM10-53-474 | IL-13 | 6.83 (2.2) | 2 |
| DOM9-112-210 | IL-4 | 3.51 (0.8) | 2 |
| Negative control mAb | --- | No inhibition shown | 4 |
| Negative control dAb | --- | No inhibition shown | 4 |

Comparison of $IC_{50}$ values indicated that 586-TVAAPS-210 inhibited IL-13 and IL-4 induced pSTAT-6 similarly to the anti-IL13 mAb and DOM9-112-210 (in the IL-13 and IL-4 whole blood assays respectively). Comparison of $IC_{50}$ data also indicated that PascoH-474 GS removed, inhibited IL-13 and IL-4 induced pSTAT-6 similarly to DOM10-53-474 and Pascolizumab (in the IL-13 and IL-4 whole blood assays respectively). The control mAb showed no inhibition up to the maximum concentration tested of 661nM in all donors, and the control dAb showed no neutralisation up to the maximum concentration tested of 2291nM in all donors.

**Example 21**

**Rat PK studies of the Dual Targeting anti-IL4/anti-IL13 mAbdAb**

PascoH-G4S-474, PascoL-G4S-474, 586H-TVAAPS-210 and 586H-TVAAPS-154 were assessed in rat PK studies (as summarised in Table 35.1). In brief, male Sprague-Dawley rats (approximately 200 grams to 220 grams in weight) were given a single intravenous (i/v) administration of mAbdAb at a target dose level of 2mg/kg. At allotted time points (0 hours through to 312 hours) 100μl blood samples were withdrawn and processed for plasma. The rat plasma samples were evaluated for the presence of the test molecule in a human IgG detection assay, and/or an IL-13 ligand binding assay, and/or an IL-4 ligand binding assay. In addition, the PK profile in plasma for Pascolizumab (in rat) was also evaluated: in this case the rat plasma samples were evaluated for the presence of Pascolizumab in a human IgG detection assay and an IL-4 ligand binding assay.

In a first study, pascolizumab was given to 4 rats. In a second study, there were four treatment groups (2mg/kg PascoH-G4S-474, 2mg/kg PascoL-G4S-474, 2mg/kg 586H-TVAAPS-210 and 2mg/kg 586H-TVAAPS-154), with 4 rats in each group.

The PK parameters (shown in Table 35.1) were derived from plasma concentration-time profile data (which are not shown). Note that, some plasma samples were analysed more than once in these assays and the PK parameters in Table 35.1 were derived from only one of these datasets. Also note that the PK parameters have not been normalised for individual animal doses, instead nominal doses of 2mg/kg have been assumed. Note that some technical difficulties were encountered with the IgG PK assay for PascoH-G4S-474 hence the concentrations may be overestimated. In addition, analysis of the IgG plasma concentration-time profiles was performed twice in some cases (at analysis 1 and analysis 2, as annotated in Table 35.1). Analysis of the plasma concentration-time profile data generated from the IL-13 and IL-4 ligand binding PK assays was only performed at analysis 2. The plasma concentration-time profile data generated from the IL-13 and IL-4 ligand binding assays for PascoL-G4S-474 and 586H-TVAAPS-154 have not been used to derive PK parameters for these molecules.

Table 35.1

| **Molecule** | **Assay** | **Analysis** | **T1/2** (hr) | **Cmax** (ng/mL) | **AUC (last)** (ng.h/mL) | **AUC (extrap)** (%) | **Clearance** (mL/hr/kg) | **Mean residence time** (hr) |
|---|---|---|---|---|---|---|---|---|
| PascoH-G4S-474 | IgG | 1 | 129 | 60950 | 3430345 | 18.7 | 0.49 | 83 |
| | IL-4 | 2 | 117 | 21975 | 1231243 | 17.2 | 1.42 | 83 |
| | IL-13 | 2 | 87 | 22050 | 1328651 | 13.2 | 1.36 | 85 |
| 586H-TVAAPS-210 | IgG | 1 | 134 | 42400 | 1932615 | 21.9 | 0.81 | 88 |
| | IgG | 2 | 92 | 42400 | 2215579 | 16.1 | 0.76 | 82 |
| | IL-4 | 2 | 60 | 41350 | 1674740 | 20.9 | 1.08 | 77 |
| | IL-13 | 2 | 101 | 31125 | 1515155 | 16.0 | 1.13 | 83 |
| Pascolizumab | IgG | 1 | 188 | 45150 | 3528174 | 31.9 | 0.39 | 110 |
| | IgG | 2 | 193 | 45150 | 3496503 | 34.9 | 0.38 | 109 |
| | IL-4 | 2 | 136 | 42825 | 2590114 | 30.6 | 0.54 | 110 |
| PascoL-G4S-474 | IgG | 1 | 69 | 42550 | 2539978 | 6.8 | 0.75 | 87 |
| 586H-TVAAPS-154 | IgG | 1 | 166 | 43900 | 3315164 | 41.7 | 0.36 | 93 |

Plasma concentration-time profile data, generated in the IL-13 and IL-4 assays for PascoH-G4S-474 (and subsequent derived PK parameters), tended to be comparable; this was also the case for the plasma concentration-time profile data generated in the IL-13, IL-4 and IgG PK assays for 586H-TVAAPS-210 (and subsequent derived PK parameters). This suggests that these mAbdAbs were 'intact' in the rat plasma throughout the course of the study. The derived PK parameters for 586H-TVAAPS-154 appeared to be more similar to the derived PK parameters for Pascolizumab than any of the other mAbdAbs.

**Example 22**

**Cyno PK studies**

PK studies were carried out in cynomolgus monkeys. The animals were given a single intravenous administration at a target dose level of 1mg/kg. At allotted time points, 500μl blood samples were withdrawn and processed for plasma. The cynomolgus plasma samples were then evaluated for the presence of the test molecule in an IL-13 ligand binding assay, an IL-4 ligand binding assay and an IL-13/IL-4 bridging assay.
Preliminary data from these studies with the mAbdAbs 'PascoH-474 GS removed' and '586H-TVAAPS-210' are consistent with the rat PK data shown above and indicated that these molecules are cleared from the systemic circulation more rapidly than a mAb but less rapidly than a dAb.

**Example 23**

**23.1 Generation of a Dual targeting anti-EGFR/anti-VEGF mAbdAb**

This dual targeting mAbdAb was constructed by fusion of a dAb to the C-terminus of the mAb heavy chain. The anti-EGFR mAb heavy and light chain expression cassettes had been previously constructed. The restriction sites which were used for cloning are the same as those set out in Example 10 (SalI and HindIII).
DNA coding an anti-VEGF dAb (DOM15-26-593) was then amplified by PCR (using primers coding SalI and HindIII ends) and inserted into the modified 3' coding region, resulting in a linker of 'STG' (serine, threonine, glycine) between the mAb and the dAb.
Sequence verified clones (SEQ ID NO: 164 and 243) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 165 and 137).

**23.2 Purification and SEC analysis of the Dual targeting anti-EGFR/anti-VEGF mAbdAb**

This dual targeting mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 128) of the purified sample (designated DMS4010) shows non-reduced sample running at ~170kDa whilst reduced sample shows two bands running at -25 and ~60kDa corresponding light chain and dAb-fused heavy chain respectively.
For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile shows a single species running as a symmetrical peak (figure 129).

**23.3 Potency of the Dual targeting anti-EGFR/anti-VEGF mAbdAb**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 130) of this mAbdAb (designated DMS4010) was calculated to be 4.784nM whilst the control, an anti-EGFR mAb gave an EC50 value of 4.214nM. In the anti-VEGF receptor binding assay (Figure 131) the EC50 of the mAbdAb (designated DMS4010) was 58pM (0.058nM) whilst an anti-VEGF control mAb produced an EC50 of 214.1pM (0.2141nM). In conclusion, assay data shows that the construct of example 23, a dual targeting anti-EGFR/anti-VEGF mAbdAb is potent against both antigens.

**23.4 PK of the dual targeting anti-EGFR/anti-VEGF mAbdAb**

The pharmacokinetic profile of the dual targeting anti-EGFR/anti-VEGF mAbdAb (designated DMS4010) was determined after administration to cynomolgus monkeys. The compound was administered at a dose of 5mg/kg i.v. and the serum levels of drug at multiple time points post-administration was determined by binding to both EGFR and VEGF in separate ELISA assays. Figure 132 shows the results for this assay in which the data was compared with historical data that had been generated for the mAbs cetuximab (anti-EGFR) and bevacizumab (anti-VEGF). Further details are shown in table 36.

Table 36

| | Antigen | Half Life | Cmax | AUC (0-inf) | Clearance | % AUC Extrapolated |
|---|---|---|---|---|---|---|
| | | (hr) | (ug/mL) | (hr*ug/mL) | (mL/hr/kg) | |
| cetuximab | EGFR | 43.6 | 151.4 | 5684.3 | 0.9 | 18.4 |
| bevacizumab | VEGF | 238.7 | 167.4 | 24201.9 | 0.2 | 13.4 |
| DMS4010 | EGFR | 7.5 | 89.7 | 623.2 | 8.1 | 5.2 |
| DMS4010 | VEGF | 6.7 | 125.5 | 733.8 | 7 | 7.2 |

**23.5 Generation of an alternative anti-EGFR/anti-VEGF mAbdAb**

An alternative anti-EGFR/anti-VEGF mAbdAb was constructed in a similar way to that described above in Example 11.1, using the same anti-EGFR mAb linked to a VEGF dAb on the C-terminus of the heavy chain using an STG linker. The anti-VEGF dAb used in this case was DOM15-10-11. This molecule was expressed in mammalian HEK293-6E cells using transient transfection techniques by co-transfection of light and heavy chains (SEQ ID NO: 165 and 186), however significantly reduced levels of expression were achieved in comparison to the expression of the molecule described in Example 23.2. When tested for potency in the same VEGF assay as described in Example 23.3 it was found to have undetectable levels of inhibition of VEGF binding to VEGF receptor in this assay.

**Example 24**

**24.1 Generation of a dual targeting anti-EGFR/anti-VEGF mAbdAb with no linker**

A derivative of the mAbdAb described above in Example 23 was made where the 'STG' linker between the dAb and the CH3 domain of the mAb was removed. SDM was used to delete the residues encoding the STG linker from the plasmid encoding the heavy chain. Sequence verified clones for light and heavy chains (SEQ ID NO: 243 and SEQ ID NO: 174) respectively were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 175 and 137).

**24.2 Purification and SEC analysis of the dual targeting anti-EGFR/anti-VEGF mAbdAb with no linker**

This dual targeting mAbdAb was purified from clarified expression supernatant using Protein-A affinity chromatography according to established protocols. Concentrations of purified samples were determined by spectrophotometry from measurements of light absorbance at 280nm. SDS-PAGE analysis (Figure 133) of the purified sample (designated DMS4011) shows non-reduced sample running at ~170kDa whilst reduced sample shows two bands running at -25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile shows a single species running as a symmetrical peak (figure 134).

**24.3 Potency of the dual targeting anti-EGFR/anti-VEGF mAbdAb with no linker**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 135) of this mAbdAb (designated DMS4011) was calculated to be 3.529nM whilst the control, an anti-EGFR mAb gave an EC50 value of 3.647nM. In the anti-VEGF receptor binding assay (Figure 136) the EC50 of the mAbdAb (designated DMS4011) was 342.9pM (0.3429nM) whilst an anti-VEGF control mAb produced an EC50 of 214.1pM (0.2141nM). In conclusion, assay data shows that the construct of example 24, a dual targeting anti-EGFR/anti-VEGF mAbdAb with no linker is potent against both antigens.

## Example 25

### 25.1 Generation of a dual-targeting anti-EGFR/anti-VEGF mAbdAb with longer linkers

Derivatives of the mAbdAb described above in Example 23 were made where the linker between the dAb and the CH3 domain of the mAb was lengthened by the insertion of one or two repeats of a flexible "GGGGS" motif into the plasmid encoding the heavy chain.

The first molecule with a heavy chain sequence as set out in SEQ ID NO: 175 has one repeat of this motif, hence having a linker of 'STGGGGGS'.

The second molecule with a heavy chain sequence as set out in SEQ ID NO: 177 has two repeats of this motif, hence having a linker of 'STGGGGGSGGGGS'. These were both indepently paired with the same light chain as used in Example 23 (SEQ ID NO: 243)

Sequence verified clones for light and heavy chains were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 176 and 137 which is designated DMS4023; and SEQ ID NO: 178 and 137 which is designated DMS4024).

### 25.2 Purification and SEC analysis of the dual targeting anti-EGFR/anti-VEGF mAbdAb with longer linkers

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified samples DMS4023 and DMS4024 (Figure 137) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1 ml/min. The SEC profile for both DMS4023

(Figure 138) and DMS4024 (Figure 139) show a single species with a slightly trailing peak.

### 25.3 Potency of the dual targeting anti-EGFR/anti-VEGF mAbdAb with longer linkers

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 140) of the mAbdAb DMS4023 was calculated to be 7.066nM and the potency of mAbdAb DMS4024 was calculated to be 6.420nM whilst the control, an anti-EGFR mAb gave an EC50 value of 7.291nM. In the anti-VEGF receptor binding assay (Figure 141) the EC50 of the mAbdAb DMS4023 was 91.79pM (0.091 nM) and the EC50 of the mAbdAb DMS4024 was 90pM (0.0906nM) whilst an anti-VEGF control mAb produced an EC50 of 463.2pM (0.4632nM). In conclusion, assay data shows that the constructs of example 25, dual targeting anti-EGFR/anti-VEGF mAbdAbs with longer linkers are potent against both antigens.

## Example 26

### 26.1 Generation of a dual targeting anti-VEGF/anti-EGFR mAbdAb

This dual targeting mAbdAb was constructed by fusion of a dAb to the C-terminus of the mAb heavy chain. The anti-VEGF mAb heavy and light chain expression cassettes had been previously constructed. The restriction sites which were used for cloning are the same as those set out in Example 10 (SalI and HindIII).

DNA coding an anti-EGFR dAb (DOM16-39-542) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID NO: 179 and 181) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 180 and 182).

**26.2 Purification and SEC analysis of the dual targeting anti-VEGF/anti-EGFR mAbdAb**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified sample (designated DMS4009) (Figure 142) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile for this molecule (Figure 143) shows a single species with a symmetrical peak.

**26.3 Potency of the dual targeting anti-VEGF/anti-EGFR mAbdAb**

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 144) of the mAbdAb DMS4009 was calculated to be 132.4nM whilst the control, an anti-EGFR mAb gave an EC50 value of 6.585nM. In the anti-VEGF receptor binding assay (Figure 145) the EC50 of the mAbdAb was 539.7pM (0.5397nM) whilst an anti-VEGF control mAb produced an EC50 of 380.5pM (0.3805nM). In conclusion, assay data shows that the construct of example 26, a dual targeting anti-VEGF/anti-EGFR mAbdAb is potent against both antigens.

**Example 27**

**27.1 Generation of a dual targeting anti-EGFR/anti-IL-13 mAbdAb**

This dual targeting mAbdAb was constructed by fusion of a dAb to the C-terminus of the mAb heavy chain. The anti-EGFR mAb heavy and light chain expression cassettes had been previously constructed. The restriction sites which were used for cloning are the same as those set out in Example 10 (SalI and HindIII).

DNA coding an anti-IL-13 dAb (DOM10-53-474) was then amplified by PCR (using primers coding SalI and HindIII ends) and inserted into the modified 3' coding region, resulting in a linker of 'STG' (serine, threonine, glycine) between the mAb and the dAb.

Sequence verified clones (SEQ ID. NO: 243 and 183) for light and heavy chains respectively) were selected and large scale DNA preparations were made using Qiagen Mega Prep Kit following the manufacturer's protocols. mAbdAbs were expressed in mammalian HEK293-6E cells using transient transfection techniques by co transfection of light and heavy chains (SEQ-ID NO: 137 and 184).

**27.2 Purification and SEC analysis of the dual targeting anti-EGFR/anti-IL-13 mAbdAb**

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified sample (designated DMS4029) (Figure 146) shows non-reduced samples running at ~170kDa whilst reduced samples show two bands running at ~25 and ~60kDa corresponding light chain and dAb-fused heavy chain respectively.

For size exclusion chromatography (SEC) analysis the anti-EGFR/anti-IL-13 mAbdAb was applied onto a S-200 10/300 GL column (attached to an HPLC system) pre-equilibrated and running in PBS at 0.5ml/min. The SEC profile for this molecule (Figure 147) shows a single species with a symmetrical peak.

**27.3 Potency of the dual targeting anti-EGFR/anti-IL-13 mAbdAb**

The ability of the molecule to neutralise EGFR and IL-13 were determined as described in methods 13 and 25 respectively. Assay data were analysed using GraphPad Prism. Potency values were determined using a sigmoidal dose response curve and the data fitted using the best fit model. Anti-EGFR potency (Figure 148) of the mAbdAb DMS4029 was calculated to be 9.033nM whilst the control, an anti-EGFR mAb gave an EC50 value of 8.874nM. In the IL-13 cell-based neutralisation assay (Figure 149) the EC50 of the mAbdAb was 1.654nM whilst an anti-IL-13 control dAb produced an EC50 of 0.996nM. In conclusion, assay data shows that the construct of example 27, a dual targeting anti-EGFR/anti-IL-13 mAbdAb is potent against both antigens.

## Example 28

### 28.1 Generation of a dual targeting anti-EGFR/anti-VEGF mAbdAbs where the dAb is located on the light chain

Dual targeting anti-EGFR/anti-VEGF mAbdAbs were constructed by fusion of a dAb to the C-terminus of the mAb light chain. The anti-EGFR mAb heavy and light chain expression cassettes had been previously constructed.

To introduce restriction sites for dAb insertion in the light chain, site directed mutagenesis was used to create BamHI and HindIII cloning sites using the mAb light chain expression vector as a template. DNA coding an anti-VEGF dAb (DOM15-26-593) was then amplified by PCR (using primers coding BamHI and HindIII ends) and inserted into the modified 3' coding region, resulting in a linker of either 'GSTG' or 'GSTVAAPS' between the mAb and the dAb.
The first molecule with a light chain sequence as set out in SEQ ID NO: 187 has a linker of 'GSTG'.
The second molecule with a light chain sequence as set out in SEQ ID NO: 189 has a linker of 'GSTVAAPS'.
These were both independently paired with the heavy chain of SEQ ID NO: 245.

Sequence verified clones for light and heavy chains were selected and large scale DNA preparations were made using Qiagen Mega Prep Kit following the manufacturer's protocols. mAbdAbs were expressed in mammalian HEK293-6E cells using transient transfection techniques by co-transfection of light and heavy chains (SEQ ID NO: 188 and 139 which is designated DMS4013; and SEQ ID NO: 190 and 139 which is designated DMS4027).

### 28.2 Purification and SEC analysis of the dual targeting anti-EGFR/anti-VEGF mAbdAbs where the dAb is located on the light chain

These dual targeting mAbdAbs were purified from clarified expression supernatant using Protein-A affinity chromatography according to established protocols. Concentrations of purified samples were determined by spectrophotometry from measurements of light absorbance at 280nm. SDS-PAGE analysis of the purified samples DMS4013 and. DMS4027 (Figure 150) shows non-reduced samples running at ~170kDa whilst reduced samples show two bands running at ~38 and ~50kDa corresponding to dAb-fused light chain and heavy chain respectively.

For size exclusion chromatography (SEC) analysis the anti-EGFR/anti-VEGF mAbdAb was applied onto a S-200 10/300 GL column (attached to an HPLC system) pre-equilibrated and running in PBS at 1ml/min. The SEC profile for both DMS4013 (Figure 151) and DMS4027 (Figure 152) show a single species with a symmetrical peak.

### 28.3 Potency of the dual targeting anti-EGFR/anti-VEGF mAbdAbs where the dAb is located on the light chain

The ability of the molecule to neutralise VEGF and EGFR were determined as described in methods 12 and 13 respectively. Assay data were analysed using GraphPad Prism. Potency values were determined using a sigmoidal dose response curve and the data fitted using the best fit model. Anti-EGFR potency (Figure 153) of the mAbdAb DMS4013 was calculated to be 7.384nM and the potency of mAbdAb DMS4027 was calculated to be 7.554nM whilst the control, an anti-EGFR mAb gave an EC50 value of 7.093nM. In the anti-VEGF receptor binding assay (Figure 154) the EC50 of the mAbdAb DMS4013 was 1.179nM and the EC50 of the mAbdAb DMS4027 was 0.1731nM whilst an anti-VEGF control mAb produced an EC50 of 0.130nM. In conclusion, assay data shows that the constructs of example 28, dual targeting anti-EGFR/anti-VEGF mAbdAbs where the dAb is located on the light chain are potent against both antigens.

## Example 29

### Biacore analysis of dual targeting anti-EGFR/anti-VEGF and anti-TNF/anti-VEGF mAbdAbs

The mAbdAbs described in example 11 (anti-TNF/anti-VEGF mAbdAb) and examples 23, 24, 25 and 28 (anti-EGFR/anti-VEGF mAbdAbs) were subjected to BIAcore analysis to determine kinetic association and dissociation constants for binding to their corresponding antigens. Analysis was performed on BIAcore™ 3000 instrument. The temperature of the instrument was set to 25°C. HBS-EP buffer was used as running buffer. Experimental data were collected at the highest possible rate for the instrument. One flow cell on a research grade CM5 chip was coated with protein A using standard amine coupling chemistry according to manufacturers instructions, and a second flow cells was treated equally but buffer was used instead of protein A to generate a reference surface. The flow cell coated with protein A was then used to capture mAbdAbs. Antigen was injected as a series 2x serial dilutions as detailed in table 37. Several dilutions were run in duplicate. Injections of buffer alone instead of ligand were used for background subtraction. Samples were injected in random order using the kinetics Wizard inherent to the instrument software. The surface was regenerated at the end of each cycle by injecting 10mM Glycine, pH 1.5. Both data processing and kinetic fitting were performed using BIAevaluation software 4.1. Data showing averages of duplicate results (from the same run) is shown in Table 37. The multiple values shown for DMS4010 represent two experiments run on separate occasions. The value of 787nM probably overestimates the affinity due to the concentrations of ligand analysed

Table 37

| **mAbdAb Example** | **Molecule number** | Antigen | Ka [1/Ms] | Kd[1/s] | KD [pM] | Top concentration (nM) | # dilutions |
|---|---|---|---|---|---|---|---|
| **11** | **4000** | TNF | 3.65E+05 | 4.16E-05 | 112 | 10 | 6 |
| **23** | **4010** | EGFR | 1.47E+06 | 1.16E-03 | 787 | 1.25 | 5 |
| **23** | **4010** | EGFR | 3.14E+05 | 1.16E-03 | 3700 | 10 | 6 |
| **24** | **4011** | EGFR | 1.81E+05 | 1.11E-03 | 6120 | 5 | 6 |
| **28** | **4013** | EGFR | 2.20E+05 | 1.17E-03 | 5310 | 20 | 5 |
| **28** | **4013** | EGFR | 3.01E+05 | 1.40E-03 | 4650 | 10 | 7 |
| **25** | **4023** | EGFR | 2.38E+05 | 1.10E-03 | 4630 | 5 | 7 |
| **25** | **4024** | EGFR | 2.34E+05 | 1.10E-03 | 4700 | 10 | 6 |
| **28** | **4027** | EGFR | 2.80E+05 | 1.14E-03 | 4060 | 20 | 7 |
| **11** | **4000** | VEGF | 9.19E+05 | 4.78E-04 | 520 | 2.5 | 5 |
| **23** | **4010** | VEGF | 9.85E+05 | 1.90E-04 | 193 | 10 | 8 |
| **24** | **4011** | VEGF | 6.17E+05 | 1.26E-04 | 204 | 10 | 8 |
| **28** | **4013** | VEGF | 7.62E+05 | 3.64E-04 | 478 | 2 | 5 |
| **25** | **4023** | VEGF | 1.60E+06 | 2.40E-04 | 150 | 2 | 6 |
| **25** | **4024** | VEGF | 1.01E+06 | 2.30E-04 | 224 | 2 | 4 |
| **28** | **4027** | VEGF | 7.47E+05 | 2.23E-04 | 229 | 2 | 5 |

**Example 30**

**Trispecific antibodies which comprise single domain antibodies fused onto a bispecific antibody scaffold**

30.1 Construction

Genes encoding variable heavy and light domains of a bispecific antibody molecule which has specificity for IL-18 and IL-12 antigens (for further information see WO 2007/024715) were constructed de-novo with appropriate restriction enzyme sites and signal sequence added. Using standard molecular biology techniques, the variable heavy domains were cloned into an expression vector containing the IgG1 heavy chain constant region fused to an anti-IL4 domain antibody DOM9-112-210 (SEQ ID NO: 4) via a TVAAPS linker at the c-terminus of the constant region. The light chain variable domain was similarly cloned into an expression vector containing the Ck constant region sequence. The antibodies constructed and expressed are listed in Table 38.

Table 38

| Antibody ID | Description | SED ID NO: of amino acid sequence |
|---|---|---|
| BPC1616 | IL-12/18 DVDH TVAAPS-210 heavy chain | 193 |
| | IL-12/18 DVD Kappa liqht chain | 194 |

30.2 Expression and purification

Briefly, 25 ml of HEK293 cells at $1.5x10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 24 hours Tryptone feeding media was added and the cells grown for a further 48 hours. Supernatant was harvested by centrifugation and IgG levels quantified by ELISA. The resulting mAbdAb was designated BPC1616 (SEQ ID NO: 193 and 194)

30.3 IL-12 binding ELISA

The cell supernatant from the transfections were assessed for binding to recombinant human IL-12. Briefly, ELISA plates coated with anti-human IL-12 (R&D Systems AF219NA) at 2μg/ml and blocked with blocking solution (4% BSA in Tris buffered saline). The plates were then loaded with 25ng/ml recombinant human IL-12 (PeproTech #200-12) in blocking solution. The plate was incubated for 1 hour at room temp before washing in TBS + 0.05% Tween 20 (TBST). Various dilutions of the cell supernatant were added as well as irrelevant control antibodies (Pascolizumab and an isotyped matched control hIgG) diluted in blocking solution. The plate was incubated for 1 hour at room temperature before washing in TBST. Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results are presented in Figure 155 and show that BPC1616 binds to recombinant human IL-12 whereas the two control antibodies showed no binding.

30.4 IL-18 binding ELISA

The cell supernatants from the transfections were assessed for binding to recombinant human IL-18. Briefly, ELISA plates were coated with human IL-18 (made at GSK) at 1μg/ml and blocked with blocking solution (4% BSA in Tris buffered saline). Various dilutions of the cell supernatant were added as well as irrelevant antibodies (Pascolizumab and an isotype matched control human IgG), diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted. The results are presented in Figure 156 and show that BPC1616 binds to recombinant human IL-18 whereas the two control antibodies show no binding.

30.5 IL-4 Binding ELISA

The cell supernatants from the transfections were assessed for binding to recombinant human IL-4. Briefly, ELISA plates were coated with human IL-4 (made at GSK) at 1 μg/ml and blocked with blocking solution (4% BSA in Tris buffered saline). Various dilutions of the cell supernatant were added as well as an anti IL-4 monoclonal antibody (Pascolizumab) and irrelevant antibody (Isotype matched control hIgG,), diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results are presented in Figure 157 show that BPC1616 and Pascolizumab bind to recombinant human IL-4 whereas the control antibody shows no binding.

**Example 31**

**Trispecific mAbdAbs comprising two single domains antibodies fused in-line at the C-terminus of a monoclonal antibody**

31.1 Construction

Three trispecific antibodies (mAbdAb-dAb) were constructed where two single domain antibodies are fused in-line at the C-terminus of the heavy chain of a monoclonal antibody.

Briefly, a BglII restriction site at the N-terminus and BamHI restriction site at the C-terminus were introduced by PCR to flank the DNA sequences encoding the domain antibodies into the DOM 10-53-474 (SEQ ID NO: 5), Dorm9-155-154 (SEQ ID NO: 3) and DOM9-112-210 (SEQ ID NO: 4).

The DNA fragment encoding the DOM10-53-474 domain antibody was then cloned into a BamHI site of mammalian expression vector encoding the heavy chain of an anti IL-5 monoclonal antibody fused with an anti IL-4 domain antibody DOM9-112-210 (SEQ ID NO: 71). The DNA fragments encoding the DOM9-155-154 and DOM9-112-210 domain antibodies were both independently cloned into the BamHI site of a mammalian expression vector encoding the heavy chain of an anti-CD20 monoclonal antibody fused with an anti IL-13 domain antibody DOM10-53-474 (SEQ ID NO: 116). The resulting expression vectors encode a heavy chain with two single domain antibodies fused onto the C-terminus. The protein sequences of the heavy chains are given in SEQ ID NO: 195, 196 and 197 as set out in Table 39.

Table 39 is a summary of the mAbdAbs that have been constructed.

Table 39

| Antibody ID | Description | SEQ ID NO: of amino acid sequence |
|---|---|---|
| BPC1008 | Anti IL-5 Heavy Chain-G4S-dAb474-TVAAPSGS=dAb210 | 195 |
| | Anti IL-5 Light Chain | 66 |
| BPC1009 | Anti CD-20 Heavy Chain-TVAAPSGS-dAb154-TVAAPSGS-dAb474 | 196 |
| | Anti CD-20 Light Chain | 117 |
| BPC1010 | Anti CD-20 Heavy Chain-TVAAPSGS-dAb210-TVAAPSGS-dAb474 | 197 |
| | Anti CD-20 Light Chain | 117 |

31.2 Expression and purification

Expression plasmids encoding the heavy and light chains of BPC1008, BPC1009 and BPC1010 were co-transfected into HEK 2936E, cells using 293fectin (Invitrogen, 12347019). A tryptone feed was added to the cell culture the following day and the supernatant material was harvested after about 2 to 6 days from initial transfection. The antibodies were purified using a Protein A column before being tested in binding assays.

31.3: IL-4 Binding ELISA

96-well high binding plates were coated with 5μg/ml human IL-4 (GSK) in Coating buffer (0.05M bicarbonate pH9.6, Sigma C-3041)$_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. The plates were washed three times, then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

The results of the ELISA are shown in the Figure 158 and confirm that antibodies BPC1008, 1009 and BPC1010 bind to recombinant human IL-4. The positive control Pascolizumab also showed binding to recombinant IL-4 whereas the negative control anti IL-13 mAb and Mepolizumab showed no binding to IL-4. Antibodies BPC1009 and BPC1010 were also tested in a separate experiment which gave similar result to those shown in Figure 158.

31.4: IL-5 Binding ELISA

96-well high binding plates were coated with 5.9μg/ml human IL-5 (GSK) in coating buffer (0.05M bicarbonate pH9.6) and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. This was washed three times, then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

Figure 159 shows the results of the ELISA which confirms that antibodies BPC1008 bind to recombinant human IL-5 whereas BPC1009 and BPC1010 showed no binding to IL-5. The positive control Mepolizumab also showed binding to recombinant IL-5 whereas the negative control anti IL-13 mAb and Pascolizumab showed no binding to IL-5.

IL-13 Binding ELISA

96-well high binding plates were coated with 5μg/ml human IL-13 (GSK) in Coating buffer (0.05M bicarbonate pH9.6, Sigma C-3041)$_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least one hour at room temperature. The purified antibodies were successively diluted across the plates in blocking solution. After one hour incubation, the plates were washed three times. Goat anti-human kappa light chain specific peroxidase conjugated antibody (Sigma A7164) was diluted 1 in 2000 in blocking solution and 50μL was added to each well. The plates were incubated for one hour. This was washed three times, then OPD (o-phenylenediamine dihydrochloride) SigmaFast substrate solution was added to each well and the reaction was stopped 5 minutes later by addition of 25μL of 3M sulphuric acid. Absorbance was read at 490nm using the VersaMax Tunable Microplate Reader (Molecular Devices) using a basic endpoint protocol.

The results of the ELISA are shown in the Figure 160 and confirm that antibodies BPC1008, 1009 and BPC1010 bind to recombinant human IL-13. The positive control anti IL-13 mAb also showed binding to recombinant IL-13 whereas the negative control Pascolizumab and Mepolizumab showed no binding to IL-13. Antibodies BPC1009 and BPC1010 were also tested in a separate experiment which gave similar result to those shown in Figure 160.

Example 32

**mAbdAbs with single domain antibodies fused onto monovalent scaffold**

32.1 Construction of mAbdAbs

Bispecific antibodies comprising a fusion of a monovalent antibody (for further information see WO2006015371 and WO2007059782) and a domain antibody DOM-15-26-293 were constructed as follows. DNA sequences encoding the anti-c-Met Knob-into-hole heavy chain (SEQ ID NO: 202 and 203) was constructed using a PCR-based strategy followed by site directed mutagenesis. The DNA sequence encoding the anti-c-Met Unibody heavy chain (SEQ ID NO: 204) was constructed using a PCR-based strategy followed by removal of the hinge region by a PCR-based approach. Additionally, for fusion constructs, BamHI and EcoRI restriction sites were included at the C-terminus of the heavy chain expression cassette to facilitate the subsequent cloning of the anti VEGF-A domain antibody (DOM-15-26-593) DNA sequence (encoding amino acids 455-570 of SEQ ID NO: 75) as a BamHI-EcoRI fragment from an existing vector. The resulting expression vectors encode an anti-VEGFA domain antibody fused onto the C-terminus of the heavy chain via a GS linker (SEQ ID NO: 198,199 and 201). The DNA sequence encoding the anti-c-Met light chain (SEQ ID NO: 200) was constructed by a PCR-based strategy.

The construction of BPC1604 is described in Example 14. Table 40 below is a summary of the monovalent scaffold mAbdAbs and antibodies that have been generated and expressed.

Table 40

| Antibody ID | Description | SED ID NO: of amino acid sequence |
|---|---|---|
| BPC1017 | anti cMET 5D5v2 Heavy Chain (hole)-GS-dAb593 | 198 |
| | anti cMET 5D5v2 Heavy Chain (knob)-GS-dAb593 | 199 |
| | anti cMET 5D5v2 Light Chain | 200 |
| BPC1018 | anti cMET 5D5v2 IgG4 Heavy Chain (UNIBODY)-GS-dAb593 | 201 |
| | anti cMET 5D5v2 Light Chain | 200 |

(continued)

| Antibody ID | Description | SED ID NO: of amino acid sequence |
|---|---|---|
| BPC1019 | anti cMET 5D5v2 Heavy Chain (hole) | 202 |
| | anti cMET 5D5v2 Heavy Chain (knob) | 203 |
| | anti cMET 5D5v2 Light Chain | 200 |
| BPC1020 | anti cMET 5D5v2 IgG4 Heavy Chain (UNIBODY) | 204 |
| | anti cMET 5D5v2 Light Chain | 200 |

32.2 Expression and purification

Expression plasmids encoding the heavy chain of BPC1017, BPC1018, BPC1019 and BPC1020 were co-transfected into HEK 2936E cells using 293fectin (Invitrogen, 12347019). A tryptone feed was added to the cell culture the following day and the supernatant material was harvested after about 2 to 6 days from initial transfection. The antibodies were purified using a Protein A column before being tested in binding assays.

32.3 HGF Receptor Binding ELISA

96-well high binding plates were coated with 5μg/ml Recombinant Human HGF R (c-MET)/Fc Chimera (R&D system, Catalog Number: 358-MT/CF) in Coating buffer (0.05M bicarbonate pH9.6; Sigma C-3041)$_3$ and stored overnight at 4°C. The plates were washed twice with Tris-Buffered Saline with 0.05% of Tween-20 (TBST). 100μL of blocking solution (1% BSA in TBST buffer) was added in each well and the plates were incubated for at least 30 minutes at room temperature. The plates were washed three times. Then the purified antibodies were successively diluted across the plates in blocking solution. After one hour incubation at room temp, the plates were washed three times. Goat anti-human kappa light chain specific peroxidase conjugated antibody (Sigma A7164) was diluted in blocking solution to 1 in 2000 and was added to each well. The plates were incubated for one hour. This was washed three times and then OPD (o=phenylenediamine dihydrochloride) SigmaFast substrate solution was added to each well and the reaction was stopped 5 minutes later by the addition of 25μL of 3M sulphuric acid. Absorbance was read at 490nm using the VersaMax Tunable Microplate Reader (Molecular Devices) using a basic endpoint protocol.

The results of the ELISA are shown in the Figure 161 and confirm that mAbdAbs BPC1017 and BPC1018 bind to recombinant human c-MET with comparable activity to the antibodies BPC1019 and BPC1020. The negative control Pascolizumab and BPC1604 (an IGF-1 R/VEGF mAbdAb) showed no binding to c-MET.

32.4 VEGF Binding ELISA

96-well high binding plates were coated with 0.4μg/mL of human VEGF (GSK) in PBS and incubated at 4°C overnight. The plates were washed twice with Tris-Buffered Saline with 0.05% of Tween-20 (TBST). 100μL of blocking solution (4% BSA in TBST buffer) was added to each well and the plates were incubated for at least one hour at room temperature. Another wash step was then performed. The purified antibodies were successively diluted across the plates in blocking solution. After one hour incubation at room temp, the plates were washed. Goat anti-human kappa light chain specific peroxidase conjugated antibody was diluted in blocking solution to 1 in 2000 and was added to each well. The plates were incubated for one hour at room temp. After another wash step, OPD (o-phenylenediamine dihydrochloride) SigmaFast substrate solution was added to each well and the reaction was stopped 5 minutes later by the addition of 25μL of 3M sulphuric acid. Absorbance was read at 490nm using the VersaMax Tunable Microplate Reader (Molecular Devices) using a basic endpoint protocol.

Figure 162 shows the results of the ELISA which confirms that mAbdAbs BPC1017 and BPC1018 bind to recombinant human VEGF. The positive control BPC1604 also showed binding to recombinant human VEGF whereas Pascolizumab, BPC1019 and BPC1020 showed no binding to VEGF.

**Example 33**

**mAbdAbs containing the anti-IL13 dAbs DOM10-53-546 dAb and DOM10-53-567** 33.1 Construction, expression and purification

The anti-IL4mAb-anti-IL13dAbs shown in Table 41 were cloned and expressed transiently in HEK2936E cells, purified (as described in examples 1, 1.3 and 1.5 respectively).

Table 41

| Name | Description | Sequence ID No. |
|---|---|---|
| PascoH-TVAAPS-546 | H chain = Pascolizumab heavy chain-TVAAPS-DOM10-53-546 dAb<br>L chain = Pascolizumab light chain | 157 (=H chain)<br>15 (=L chain) |
| PascoH-TVAAPS-567 | H chain = Pascolizumab heavy chain-TVAAPS-DOM10-53-567 dAb<br>L chain = Pascolizumab liqht chain | 159 (=H chain)<br>15 (=L chain) |

Purified PascoH-TVAAPS-546 and PascoH-TVAAPS-567 mAbdAbs were analysed by size exclusion chromatography (SEC) and sodium dodecyl sulphate poly acrylamide gel electrophoresis (SDS PAGE), under reducing conditions. THE SEC and SDS PAGE data are shown in Figures 163, 164, 165 and 166.

**33.2 Biacore analysis of binding to IL-13 and IL-4**

Purified PascoH-TVAAPS-546 and PascoH-TVAAPS-567 were tested for binding to human IL-13 and human IL-4 using the BIAcore™ T100 at 25°C (as described in methods 4 and 5). These data-are shown in Table 42.

Table 42

| **Molecule** (purified material) | **Binding affinity, KD (nM)** | | | | | |
|---|---|---|---|---|---|---|
| | Human IL-4 | | | Human IL-13 | | |
| | on rate (ka, $Ms^{-1}$) | off rate (kd, $s^{-1}$) | KD (nM) | on rate (ka, $Ms^{-1}$) | off rate (kd, $s^{-1}$) | KD (nM) |
| PascoH-TVAAPS-546 | 4.92E+6 | 2.37E-5 | 0.00482 | 2.26E+5 | 1.69E-4 | 0.747 |
| PascoH-TVAAPS-567 | - | - | Tight binding observed | 4.46E+5 | 1.70E-5 | 0.038 |
| Anti-human IL-13 mAb | - | - | Does not bind | 1.00E+6 | 3.78E-4 | 0.377 |
| Pascolizumab | 4.25E+6 | 2.43E-5 | 0.00572 | - | - | Does not bind |

The mAbdAbs tested in this assay both bound IL-4 with very high affinity (NB, for PascoH-TVAAPS-567 this was beyond the sensitivity of the machine) and with similar binding affinity to that of the anti-human IL4 mAb alone (Pascolizumab). PascoH-TVAAPS-546 and PascoH-TVAAPS-567 both bound IL-13. Note that the anti-IL-13 dAbs alone (DOM10-53-546 and DOM10-53-567) were not tested in this assay as the dAb cannot be captured onto the Protein A or anti-human IgG coated CM5 chip; instead, the anti-human IL13 mAb was used as a positive control to demonstrate IL-13 binding in this assay.

These mAbdAbs were also tested for binding to cynomolgus IL-13 using the BIAcore™ T100 at 25°C (as described in method 23). These data are shown in Table 43. A mAbdAb capture level between 500 and 750 relative response units was achieved, six IL-13 concentration curves (256, 64, 16, 4, 1 and 0.25nM) were assessed for both the mAbdAbs and the anti-IL13 mAb.

Table 43

| **Molecule** (purified material) | **Binding affinity for cynomolgus IL=13 (KD)** | | |
|---|---|---|---|
| | on rate (ka, $Ms^{-1}$) | off rate (kd, $S^{-1}$) | KD (nM) |
| PascoH-TVAAPS-546 | 1.67E+6 | 2.09E-2 | 12.5 |
| PascoH-TVAAPS-567 | 5.92E+5 | 5.22E-3 | 8.8 |
| Anti-IL13 mAb | 4.79E+5 | 8.22E-5 | 0.171 |

PascoH-TVAAPS-546 and PascoH-TVAAPS-567 both bound cynomolgus IL-13 with similar binding affinities. Note that the anti-IL-13 dAbs alone (DOM10-53-546 and DOM10-53-567) were not tested in this assay as the dAb cannot be captured onto the Protein A or anti-human IgG coated CM5 chip; instead, the anti-human IL13 mAb was used as a positive control to demonstrate IL-13 binding in this assay.

33.3 Neutralisation of human IL-13 and cynomolgus IL-13 in TF-1 cell bioassays

Purified PascoH-TVAAPS-546 and PascoH-TVAAPS-567 were tested for neutralisation of human IL-13 and cynomolgus IL-13 in TF-1 cell bioassays (as described in method 8 and method 20 respectively). Figures 167 and 168 show the neutralisation data for human IL-13 and cynomolgus IL-13 (in the TF-1 cell bioassays) respectively. DOM10-53-616 was included as a positive control for neutralisation of IL-13 in these bioassays. A dAb with specificity for an irrelevant antigen (negative control dAb) was also included as a negative control for neutralisation of IL-13. In addition, PascoH-616 and PascoH-TVAAPS-616 were also tested in these assays.
Both PascoH-TVAAPS-546 and PascoH-TVAAPS-567 fully neutralised the bioactivity of human and cynomolgus IL-13 in the TF-1 cell bioassays.
$ND_{50}$ values were calculated from the dataset. The $ND_{50}$ value is the concentration of mAbdAb or mAb or dAb, which is able to neutralise the bioactivity of IL-13 by 50%. The mean $ND_{50}$ value, the standard deviation (SD) and the number of times tested (n) are shown in table 44.

Table 44

| **Molecule** | **Mean $ND_{50}$ value & standard deviation (nM)** | | | |
|---|---|---|---|---|
| | human IL-13 | | cyno IL-13 | |
| | mean (SD) | n | mean (SD) | n |
| **PascoH-TVAAPS-546** | 1.522 | 1 | 33.88 | 1 |
| **PascoH-TVAAPS-567** | 2.06 | 1 | 38.25 | 1 |
| **DOM10-53-616** | 0.536 | 1 | 3.57 | 1 |
| **Negative control dAb** | did not neutralise | | did not neutralise | |

Example 34

mAbdAbs with IgG2, IgG4 and IgG4PE heavy chain constant regions

34.1 Construction of mAbdAbs

The heavy chain constant regions of human antibody isotypes IgG2, IgG4 and a variant IgG4 (IgG4PE) genes were amplified from existing constructs by PCR and cloned using standard molecular biology techniques into an expression vector encoding the PascoH-GS-474 heavy chain (SEQ ID NO: 48). The mAbdAbs antibodies designed and tested are listed in Table 45.

Table 45

| Antibody ID | Description | SED ID NO: of amino acid sequence |
|---|---|---|
| BPC1617 | PascoH IgG2-GS-474 | 207 |
| | Pasco Kappa | 15 |
| BPC1618 | PascoH IgG4-GS-474 | 208 |
| | Pasco Kappa | 15 |
| BPC1619 | PascoH IgG4PE-GS-474 | 209 |
| | Pasco Kappa | 15 |

34.2 Expression

The mAbdAbs set out in table 45 were expressed, along with PascoH-GS-474 (SEQ ID NO: 48 and 15) which is designated BPC1000. Briefly, 750μl of HEK293 cells at $1.5x10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 1 hour, Tryptone feeding media was added and the cells grown for a further 72 hours. Supernatant was barvested by centrifugation.

34.3 IL-4 Binding ELISA

The supernatants containing these mAbdAbs were assessed for binding to recombinant human IL-4. Briefly, ELISA plates were coated with human IL-4 (made at GSK) at 1μg/ml and blocked with blocking solution (4% BSA in Tris buffered saline). Various dilutions of the cell supernatant, an anti IL-4 monoclonal antibody (Pascolizumab) and an antibody of irrelevant specificity (586 anti IL-13) were added. All samples were diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results presented in Figure 169 show that the mAbdAbs containing the alternative isotypes all bind to human IL-4. For the mAbdAbs BPC1000, BPC1617, BPC1618 and BPC1619, the amount of antibody in the supernatant was not quantified thus the data presented in Figure 169 is represented as a dilution factor of the neat supernatant material. For the anti-IL4 and IL-13 control antibodies, purified material was used in the assay and the starting concentration of 1μg/ml and 1μg/ml was used respectively (which is equivalent to dilution factor of 1 in Figure 169).

34.4 IL-13 Binding ELISA

The supernatants containing these mAbdAbs were assessed for binding to recombinant human IL-13. Briefly, ELISA plates were coated with human IL-13 (made at GSK) at 5μg/ml and blocked with blocking solution (4% BSA in Tris buffered saline). Various dilutions of the cell supernatant, an anti IL-13 monoclonal antibody (586) and an antibody of irrelevant specificity (Pascolizumab anti IL-4) were added. All samples were diluted in blocking solution. The plate was incubated for 1 hour at room temp before washing in TBS + 0.05% Tween 20 (TBST). Binding was detected by the addition of a peroxidase labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 3M $H_2SO_4$. Absorbance was measured at 490nm with a plate reader and the mean absorbance plotted.

The results presented in Figure 170 show that the bispecific antibodies containing the alternative isotypes all bind to human IL-13. For the bispecific antibodies BPC1000, BPC1617, BPC1618 and BPC1619, the amount of antibody in the supernatant was not quantified thus the data presented in Figure 170 is represented as a dilution factor of the neat supernatant material. For the anti-IL4 and IL-13 control antibodies, purified material was used in the assay at a starting concentration of 1 μg/ml and 1μg/ml respectively (which is equivalent to dilution factor of 1 in Figure 170).

**Example 35**

Alternative anti-IL-13/IL-4 mAbdAbs

35.1 Construction of anti-IL-13/IL-4 mAbdAbs with alternative variable region

sequences

Using standard molecular biology techniques, DNA sequences encoding alternative heavy chain variable region anti-IL-13 mAb designated 'C1' and 'D1' were transferred from existing constructs to an expression vector containing DNA encoding the hIgG1 constant region fused to an anti IL-4 domain antibody (DOM9-112-210) via a TVAAPSGS linker at the c-terminus of the constant region. DNA sequences encoding alternative light chain variable region of IL-13mAbs designated 'M0' and 'NO' were assembled *de novo* and cloned into expression vectors containing the human Ck constant region. These alternative heavy and light chain antibody variable regions comprise the same CDR regions as the anti-IL-13 antibody described in SEQ ID NO: 12 and 13 but with an alternative humanised variable framework region.

35.2 Construction of mAbdAbs using the variable regions of the anti IL-13 mAb '656'

Using standard molecular biology techniques, a DNA sequence encoding the heavy chain variable region of the humanised anti IL-13 mAb '656', were transferred from an existing construct and cloned into an expression vector containing DNA encoding the hIgG1 constant region fused to an anti IL-4 domain antibody (DOM9-112-210) via a TVAAPS linker at the c-terminus of the constant region. A DNA sequence encoding the variable light region was transferred from an existing construct and cloned into an expression vector containing the human Ck constant region.

35.3 Expression of mAbdAbs

Briefly, 25 ml of HEK293 cells at $1.5x10^6$ cells/ml were co-transfected with heavy and light chain expression plasmids previously incubated with 293fectin reagent (Invitrogen # 51-0031). These were placed in a shaking incubator at 37°C, 5% $CO_2$, and 95%RH. After 24 hours Tryptone feeding media was added and the cells grown for a further 72 hours. Supernatant was harvested by centrifugation and IgG levels quantified by ELISA. The antibodies constructed and expressed are listed in Table 46.

Table 46

| Antibody ID / Name | Description | SED ID NO: of amino acid sequence |
|---|---|---|
| BPC1607 | H chain = G1-TVAAPSGS-210 | 151 |
| | L chain = M0 Kappa | 154 |
| BPC1608 | H chain = C1-TVAAPSGS-210 | 151 |
| | L chain = N0 Kappa | 153 |
| BPC1609 | H chain = C1-TVAAPSGS-210 | 151 |
| | L chain = 586 Kappa (Anti-human IL-13 mAb light chain) | |
| BPC1610 | H chain = D1-TVAAPSGS-210 | 152 |
| | L chain = M0 Kappa | 154 |
| BPC1611 | H chain = D1-TVAAPSGS-210 | 152 |
| | L chain = N0 Kappa | 153 |
| BPC1612 | H chain = D1-TVAAPSGS-210 | 152 |
| | L chain = 586 Kappa (Anti-human IL-13 mAb light chain) | 13 |
| BPC1613 | H chain = 586H-TVAAPS-210 (Anti-human IL-13 mAb heavy chain-TVAAPS-DOM9-112-210 dAb) | 88 |
| | L chain = M0 Kappa | 154 |
| BPC1614 | H chain = 586H-TVAAPS-210 (Anti-human IL-13 mAb heavy chain-TVAAPS-DOM9-112-210 dAb) | 88 |
| | L chain = N0 Kappa | 153 |
| BPC1615 | H chain = 656H-TVAAPS-210 (Anti-human IL-13 mAb 2 heavy chain-TVAAPS-DOM9-112-210 dAb) | 155 |
| | L chain = 656 Kappa (Anti-human IL-13 mAb 2 light chain) | 156 |
| BPC1602 (586H-TVAAPS-210 GS removed) | H chain = 586H-TVAAPS-210 (Anti-human IL-13 mAb heavy chain-TVAAPS-DOM9-112-210 dAb) | 88 |
| | L chain = 586 Kappa (Anti-human IL-13 mAb light chain) | 13 |

35.4 Binding of the mAbdAbs to IL-13

The binding activity of the mAbdAbs to IL-13 was assessed by ELISA. In brief, 5μg/ml recombinant *E.coli*-expressed human IL-13 (made and purified at GSK) was coated to a 96-well ELISA plate. The wells were blocked for 2 hours at room temperature, mAbdAb constructs were then titrated out down the plate. Binding was detected using a 1 in 1000 dilution of anti-human kappa light chain peroxidase conjugated antibody (catalogue number A7164, Sigma-Aldrich).

Figure 177 shows that all of the tested molecules were able to bind to human IL-13. Although BPC1615 showed binding in this ELISA it was not possible to accurately quantify the concentration of this molecule and therefore the IL-13 binding ELISA data for this molecule is not plotted in Figure 177. BPC1615 has also been shown to have high affinity binding to IL-13 in an independent Biacore assay (Table 47).

35.5 Binding of anti-IL 13mAb-anti-IL4dAbs to IL-13 by BIAcore™

Cell supernatants from the HEK cell transfections were also tested for binding to recombinant E.Coli-expressed human IL-13 using BIAcore™ at 25°C (as described in method 4). BPC1601 was tested as a purified protein. Binding affinities, presented in Table 47, confirm that all antibodies show high affinity binding to human IL-13.

Table 47

| | ka | kd | KD (nM) |
|---|---|---|---|
| **C1-TVAAPSGS-210 & M0Kappa** BPC1607 | 5.15E+5 | 8.89E-4 | 1.73 |
| **C1-TVAAPSGS-210 &** N0Kappa BPC1608 | 4.90E+5 | 8.83E-4 | 1.80 |
| **C1-TVAAPSGS-210 & 586kappa** BPC1609 | 7.55E+5 | 7.61E-4 | 1.01 |
| **D1-TVAAPSGS-210 & M0kappa** BPC1610 | 3.31E+5 | 4.66E-4 | 1.41 |
| **D1-TVAAPSGS-210 & N0kappa** BFC1611 | 2.59E+5 | 3.31E-4 | 1.28 |
| **D1-TVAAPSGS-210 & 586kappa** BPC1612 | 4.85E+5 | 2.74E-4 | 0.565 |
| **586H TVAAPS-210 & M0kappa** BPC1613 | 5.54E+5 | 4.45E-4 | 0.804 |
| **586H TVAAPS-210 & N0kappa** BPC1614 | 5.49E+5 | 4.42E-4 | 0.805 |
| **656H TVAAPS-210 & 656kappa** BPC1615 | 4.89E+6 | 4.17E-4 | 0.085 |
| **586H-TVAAPS-210noGS** BPC1602 | 8.21E+5 | 4.62E-4 | 0.562 |
| **586H-nolinker-210noGS** BPC1601 purified | 8.93E+5 | 3.93E-4 | 0.440 |

## Example 36

### Generation of mAbdAb with specificity for human IL-5 and human IL-13

36.1 Construction and expression of mAbdAb

A mAbdAb molecule having the heavy chain set out in SEQ ID NO: 65 and the light chain set out in SEQ ID NO: 72 was expressed in HEK2936E cells. This was designated MepolizumabL-G4S-474 or BPC1021.

36.2 Binding of anti-IL5mAb-anti-IL13dAb to IL-5 and IL-13

This mAbdAb (in cell supernatants) was tested for binding to human IL-13 in a direct binding ELISA (as described in method 1). These data are shown in Figure 173. The sample was transfected and tested in duplicate and this has been annotated as sample A and sample B.

This mAbdAb bound IL-13. Purified anti-human IL13 mAb alone was included in this assay as a positive control for IL-13 binding. Purified anti-human IL-4 mAb (Pascolizumab) and anti-human IL-5 mAb (Mepolizumab) were included as negative controls for IL-13 binding.

This mAbdAb was also tested for binding to human IL-5 in a direct binding ELISA (as described in Example 31.4) These data are illustrated in Figure 174.

MepolizumabL-G4S-474 bound IL-5. Purified anti-human IL4 mAb (Pascolizumab) and purified anti human 13 mAb were included as negative controls for binding to IL-5. Purified anti-human IL5 mAb (Mepolizumab) was used as a positive control to demonstrate IL-5 binding in this assay.

**Sequences**

Table 49:

| Protein or polynucleotide description | DNA Sequence (SEQ ID NO:) | Protein Sequence (SEQ ID NO:) |
|---|---|---|
| DO M9-155-25 | - | 1 |
| DOM9-155-147 | 60 | 2 |
| DOM9-155-154 | 61 | 3 |
| DOM9-112-210 | - | 4 |
| DO M10-53-474 | - | 5 |
| G4S Linker | - | 6 |
| Linker | - | 7 |
| Linker | - | 8 |
| Linker | - | 9 |
| Linker | - | 10 |
| Linker | - | 11 |
| Anti-human IL13 mAb Heavy chain & alternative polynucleotide sequence | 205<br>206 | 12 |
| Anti-human IL13 mAb Light chain | 210 | 13 |
| Pascolizumab Heavy chain | 211 | 14 |
| Pascolizumab Light chain | 212 | 15 |
| Mepolizumab Heavy chain | 213 | 65 |
| Mepolizumab Light chain | 214 | 66 |
| Anti-human IL18 mAb Heavy chain | - | 67 |
| Anti-human IL18 mAb Light chain | - | 68 |
| 586H-25 Heavy chain | - | 16 |
| 586H3147 Heavy chain | - | 17 |
| 586H-154 Heavy chain | - | 18 |
| 586H-210 Heavy chain | - | 19 |
| 586H-G4S-25 Heavy chain | - | 20 |
| 586H-G4S-147 Heavy chain | - | 21 |
| 586H-G4S-154 Heavy chain | - | 22 |
| 586H-G4S-210 Heavy chain | - | 23 |
| 586H-TVAAPS-25 Heavy chain | - | 24 |
| 586H-TVAAPS-147 Heavy chain | - | 25 |
| 586H-TVAAPS-154 Heavy chain | 122 | 26 |
| 586H-TVAAPS-210 Heavy chain | 167 | 27 |
| 586H-ASTKG-25 Heavy chain | - | 28 |
| 586H-ASTKG-147 Heavy chain | - | 29 |
| 586H-ASTKG-154 Heavy chain | - | 30 |

(continued)

| Protein or polynucleotide description | DNA Sequence (SEQ ID NO:) | Protein Sequence (SEQ ID NO:) |
|---|---|---|
| 586H-ASTKG-210 Heavy chain | 192 | 31 |
| 586H-EPKSC-25 Heavy chain | - | 32 |
| 586H-EPKSC-147 Heavy chain | - | 33 |
| 586H-EPKSC-154 Heavy chain | - | 34 |
| 586H-EPKSC-210 Heavy chain | - | 35 |
| 586H-ELQLE-25 Heavy chain | - | 36 |
| 586H-ELQLE-147 Heavy chain | - | 37 |
| 586H-ELQLE-154 Heavy chain | - | 38 |
| 586H-ELQLE-210 Heavy chain | - | 39 |
| 586H Heavy chain-GS | - | 40 |
| 586H-ASTKG Heavy chain | - | 41 |
| 586H-EPKSC Heavy chain | - | 42 |
| 586H-ELQLE Heavy chain | - | 43 |
| 586L-G4S-25 Light chain | - | 44 |
| 586L-G4S-147 Light chain | - | 45 |
| 586L-G4S-154 Light chain | - | 46 |
| 586L-G4S-210 Light chain | - | 47 |
| PascoH-474 Heavy chain | 215 | 48 |
| PascoH-G4S-474 Heavy chain | - | 49 |
| PascoH-TVAAPS-474 Heavy chain | 216 | 50 |
| PascoH-ASTKG-474 Heavy chain | - | 51 |
| PascoH-EPKSC-474 Heavy chain | - | 52 |
| PascoH-ELQLE-474 Heavy chain | - | 53 |
| PascoL-474 Light chain | 217 | 54 |
| PascoL-G4S-474 Light chain | - | 55 |
| PascoL-TVAAPS-474 Light chain | 218 | 56 |
| PascoL-ASTKG-474 Light chain | - | 57 |
| PascoL-EPKSC-474 Light chain | - | 58 |
| PascoL-ELQLE-474 Light chain | - | 59 |
| Interleukin-4 | - | 62 |
| Interleukin-13 | - | 63 |
| Mammalian signal sequence | - | 64 |
| IGF1 R binding VH CDR3 | - | 80 |
| IGF1 R binding VH CDR2 | - | 81 |
| IGF1R binding VH CDR1 | - | 82 |
| IGF1R binding VL GDR1 | - | 83 |
| IGF1 R alternative VL CDR2 | - | 84 |

(continued)

| Protein or polynucleotide description | DNA Sequence (SEQ ID NO:) | Protein Sequence (SEQ ID NO:) |
|---|---|---|
| IGF1R binding VL CDR3 | - | 85 |
| IGF1R binding VL CDR2 | - | 86 |
| IL-18mAb-G4S-DOM9-112-210 heavy chain | - | 69 |
| IL-18mAb-G4S-DOM10-53-474 Light Chain | - | 70 |
| IL-5 mAb-G4S-DOM9-112-210 heavy chain | 219 | 71 |
| IL-5 mAb-G4S-DOM10-53-474 light chain | 220 | 72 |
| Anti-TNF mAb Light Chain | 169 | 73 |
| Anti-TNF mAb-DOM16-39-542 Heavy Chain | 170 | 74 |
| Anti-TNF mAb-DOM15-26-593 Heavy Chain | 168 | 75 |
| DOM 15-26-593-VHdUMMY Heavy Chain | 172 | 76 |
| DOM 4-130-54-VκdUMMY Light Chain | 171 | 77 |
| DOM 15-26-anti-TNFmAb Heavy Chain | - | 78 |
| DOM 16-39-542-anti-TNFmAb Light Chain | - | 79 |
| 586H-210 Heavy chain (GS removed) | 221 | 87 |
| 586H-TVAAPS-210 Heavy chain (GS removed) | 222 | 88 |
| 586H-ASTKGPT-210 Heavy chain (both GS removed) | - | 89 |
| 586H-ASTKGPS-210 Heavy chain (both GS removed, linker ASTKGPS) | - | 90 |
| PascoH-474 Heavy Chain (GS removed) | 223 | 91 |
| PascoH-TVAAPS-474 Heavy Chain (GS removed) | 224 | 92 |
| PascoH-ASTKGPT-474 Heavy Chain (both GS removed) | - | 93 |
| PascoH-ASTKGPS-474 Heavy Chain (both GS removed, linker ASTKGPS) | | 94 |
| PascoH-ASTKGPS-474 Heavy Chain (second GS removed, linker ASTKGPS) | - | 95 |
| PascoH-ASTKGPT-474 Heavy Chain (second GS removed) | 225 | 96 |
| EGFR binding VH CDR1 | - | 97 |
| EGFR binding VH CDR2 | - | 98 |
| EGFR binding VH CDR3 | - | 99 |
| EGFR binding VL CDR1 | - | 100 |
| EGFR binding VL CDR2 | - | 101 |
| EGFR binding VL CDR3 | - | 102 |
| EGFR epitope | - | 103 |
| EGFR binding VH alternative CDR1 | - | 104 |
| EGFR binding VH alternative CDR2 | - | 105 |
| EGFR binding VH alternative CDR3 | - | 106 |
| EGFR binding VH alternative CDR2 | - | 107 |

(continued)

| Protein or polynucleotide description | DNA Sequence (SEQ ID NO:) | Protein Sequence (SEQ ID NO:) |
|---|---|---|
| Heavy chain of anti-IGF-1 R antibody H0L0 with DOM15-26-593 fused at C-terminus with TVAAPSGS linker | 226 | 108 |
| IGF1RmAb-GS-DOM15-26-593 Heavy chain | 227 | 109 |
| anti-IGF-1 R antibody Heavy chain | 228 | 110 |
| Light chain of anti-IGF-1R antibody H0L0 with DOM15-26-593 fused at C-terminus with TVAAPSGS linker | 229 | 111 |
| Light chain of anti-IGF-1 R antibody H0L0 with DOM15-26-593 fused at C-terminus with GS linker | 230 | 112 |
| anti-IGF-1 R antibody Light chain | 231 | 113 |
| Variable heavy domain of antibody 2B9 | - | 114 |
| Variable light domain of antibody 2B9 | - | 115 |
| Anti-CD20 mAb heavy chain with TVAAPSGS linker and DOM10-53-474 domain antibody fused at C-terminus | - | 116 |
| Anti-CD20 mAb Light Chain | - | 117 |
| Anti-CD20 mAb heavy chain with GS linker and DOM10-53-474 domain antibody fused at C-terminus | - | 118 |
| Anti-CD20 light chain with TVAAPSGS linker and DOM10-53-474 domain antibody fused at C-terminus | - | 119 |
| Anti-CD20 mAb heavy chain | - | 120 |
| Anti-CD20 mAb light chain with GS linker and DOM10-53-474 domain antibody fused at C-terminus | - | 121 |
| antiIGF1R Heavy Chain-GS-TLPC | 232 | 123 |
| antiIGF1R Heavy Chain-GS-CT01 adnectin | 233 | 124 |
| antiIGF1R Heavy Chain-TVAAPSGS-TLPC | 234 | 125 |
| antiIGF1R Heavy Chain-GS-AFFI | 235 | 126 |
| antiIGF1R Heavy Chain-TVAAPSGS-AFFI | 236 | 127 |
| antiIGF1R Heavy Chain-GS-DRPN | 237 | 128 |
| antiIGF1R Heavy Chain-TVAAPSGS-DRPN | 238 | 129 |
| Anti IL-4 heavy Chain-GS-anti RNAse A camelidVHH | 239 | 130 |
| Anti IL-4 heavy Chain-GS-NARV | 240 | 131 |
| antiIGF1R Heavy Chain-TVAAPSGS-CT01 adnectin | 241 | 133 |
| antiIL13-Heavy Chain-GS-antiTNFα adnectin | - | 134 |
| antiIL13-Heavy Chain-TVAAPSGS-antiTNFα adnectin | - | 135 |
| Erbitux Heavy chain-RS-CT01 adnectin | 242 | 136 |
| Anti-EGFR mAb Light chain (RS12) | 243 | 137 |
| Erbitux Light chain-RS-CT01 adnectin | 244 | 138 |
| Anti-EGFR mAb Heavy chain | 245 | 139 |
| 11 F8 Heavy Chain-GS-CT01 adnectin | - | 140 |
| 11 F8 Light Chain | - | 141 |

(continued)

| Protein or polynucleotide description | DNA Sequence (SEQ ID NO:) | Protein Sequence (SEQ ID NO:) |
|---|---|---|
| 11 F8 Light Chain-GS-CT01 adnectin | - | 142 |
| 11F8 Heavy Chain | - | 143 |
| CT01 adnectin-GSTG- Erbitux Heavy Chain | 246 | 144 |
| CT01 adnectin-STG-Erbitux Light Chain | 247 | 145 |
| Anti IL-4 Heavy Chain-GS-anti TNF-$\alpha$ adnectin | 270 | 146 |
| Anti IL-4 Heavy Chain-TVAAPSGS- anti TNF-$\alpha$ adnectin | 132 | 147 |
| DOM10-53-616 | - | 148 |
| PascoH-616 Heavy chain | 248 | 149 |
| PascoH-TVAAPS-616 Heavy chain | 249 | 150 |
| C1-TVAAPSGS-210 Heavy chain | - | 151 |
| D1-TVAAPSGS-210 Heavy chain | - | 152 |
| N0 Light chain | - | 153 |
| M0 Light chain | - 154 | 154 |
| 656H-TVAAPS-210 Heavy chain | 250 | 155 |
| 656 Light chain | 251 | 156 |
| PascoH-TVAAPS-546 Heavy chain | 252 | 157 |
| PascoH-546 Heavy chain | 253 | 158 |
| PascoH-TVAAPS-567 Heavy chain | 254 | 159 |
| PascoH-567 Heavy chain | 255 | 160 |
| 656 Heavy chain | 256 | 161 |
| DOM15-26-VHdUMMY Heavy Chain | 162 | 163 |
| Cetuximab-DOM15-26-593 Heavy Chain | 164 | 165 |
| Pascolizumab alternative Heavy chain | | 166 |
| Cetuximab-DOM15-26-593 Heavy Chain no linker | 173 | 174 |
| Cetuximab-DOM15-26-593 Heavy Chain STGGGGGS linker | 175 | 176 |
| Cetuximab-DOM15-26-593 Heavy Chain STGGGGGSGGGGS linker | 177 | 178 |
| Avastin-DOM16-39-542 Heavy Chain | 179 | 180 |
| Avastin Light chain | 181 | 182 |
| Cetuximab-DOM10-53-474 Heavy chain | 183 | 184 |
| Anti-TNF mAb-DOM15-10-11 Heavy Chain | - | 185 |
| Anti-EGFR mAb-DOM15-10-11 Heavy Chain | - | 186 |
| Anti-EGFR mAb-DOM15-26-593 Light Chain GSTG | 187 | 188 |
| Anti-EGFR mAb-DOM15-26-593 Light Chain GSTVAAPS | 189 | 190 |
| Mepolizumab alternative Heavy chain | | 191 |
| IL-12/18 DVDH TVAAPS-210 heavy chain | - | 193 |
| IL-12/18 DVD Kappa light chain | - | 194 |
| Anti IL-5 Heavy Chain-G4S-dAb474-TVAAPSGS-dAb210 | 257 | 195 |

(continued)

| Protein or polynucleotide description | DNA Sequence (SEQ ID NO:) | Protein Sequence (SEQ ID NO:) |
|---|---|---|
| Anti CD-20 Heavy Chain-TVAAPSGS-dAb154-TVAAPSGS-dAb474 | 258 | 196 |
| Anti CD-20 Heavy Chain-TVAAPSGS-dAb210-TVAAPSGS-dAb474 | 259 | 197 |
| anti cMET 5D5v2 Heavy Chain (hole)-GS-dAb593 | 260 | 198 |
| anti cMET 5D5v2 Heavy Chain (knob)-GS-dAb593 | 261 | 199 |
| anti cMET 5D5v2 Light Chain | 262 | 200 |
| anti cMET 5D5v2 IgG4 Heavy Chain (UNIBODY)-GS-dAb593 | 263 | 201 |
| anti cMET 5D5v2 Heavy Chain (hole) | 264 | 202 |
| anti cMET 5D5v2 Heavy Chain (knob) | 265 | 203 |
| anti cMET 5D5v2 IgG4 Heavy Chain (UNIBODY) | 266 | 204 |
| PascoH IgG2-GS-474 heavy chain | 267 | 207 |
| PascoH IgG4-GS-474 heavy chain | 268 | 208 |
| PascoH IgG4PE-GS-474 heavy chain | 269 | 209 |

**Brief Description of Figures**

Figures 1 to 7: Examples of antigen-binding constructs
Figure 8: Schematic diagram of mAbdAb constructs.
Figures 9: SEC and SDS Page analysis of PascoH-G4S-474
Figure 10: SEC and SDS Page analysis of PascoL-G4S-474
Figure 11: SEC and SDS Page analysis of PascoH-474
Figure 12: SEC and SDS Page analysis of PascoHL-G4S-474
Figure 13: mAbdAb supernatants binding to human IL-13 in a direct binding ELISA
Figure 14: mAbdAb supernatants binding to human IL-4 in a direct binding ELISA
Figure 15: Purified mAbdAbs binding to human IL-13 in a direct binding ELISA
Figure 16: purified mAbdAbs binding to human IL-4 in a direct binding ELISA
Figure 17: mAbdAb supernatants binding to human IL-4 in a direct binding ELISA
Figure 18: mAbdAb supernatants binding to human IL-13 in a direct binding ELISA
Figure 19: purified mAbdAb binding to human IL-4 in a direct binding ELISA
Figure 20A: purified mAbdAb binding to human IL-13 in a direct binding ELISA
Figure 20B: purified mAbdAb binding to cynomolgus IL-13 in a direct binding ELISA
Figure 21: mAbdAb binding kinetics for IL-4 using BIAcore™
Figure 22: mAbdAb binding kinetics for IL-4 using BIAcore™
Figure 23: mAbdAbs binding kinetics for IL-13 using BIAcore™
Figure 24: Purified anti-IL 13mAb-anti-IL4dAbs ability to neutralise human IL-13 in a TF-1 cell bioassay
Figure 25: Purified anti-IL 13mAb=anti-IL4dAbs ability to neutralise human IL-4 in a TF-1 cell bioassay
Figure 26: purified anti-IL4mAb-anti-IL13dAbs PascoH-G4S-474, PascoH-474, PascoL-G4S-474 and PascoHL-G4S-474 ability to neutralise human IL-4 in a TF-1 cell bioassay
Figure 27: purified anti-IL4mAb-anti-IL13dAbs, PascoH-G4S-474, PascoH-474, PascoL-G4S-474 and PascoHL-G4S-474 ability to neutralise human IL-13 in a TF-1 cell bioassay
Figure 28: purified anti-IL4mAb-anti-IL 13dAbs, PascoH-G4S-474, PascoH-474, PascoL-G4S-474 and PascoHL-G4S-474 ability to simultaneously neutralise human IL-4 and human IL-13 in a dual neutralisation TF-1 cell bioassay
Figure 29: DOM10-53-474 SEC-MALLS
Figure 30: DOM9-112-210 SEC-MALLS
Figure 31: DOM9-155-25 SEX-MALLS
Figure 32: DOM9-155-25 SEC-MALLS Overlay of all three signals
Figure 33: DOM9-155-147 SEC-MALLS

Figure 34: DOM9-155-159 SEC-MALLS
Figure 35: Control for MW assignment by SEC-MALLS: BSA
Figure 36: schematic diagram of a trispecific mAbdAb molecule
Figure 37: Trispecific mAbdAb IL18mAb-210-474 (supernatants) binding to human IL-18 in direct binding ELISA
Figure 38: Trispecific mAbdAb IL18mAb-210-474 (supernatants) binding to human IL-13 in direct binding ELISA
Figure 39: Trispecific mAbdAb IL18mAb-210-474 (supernatants) binding to human IL-4 in direct binding ELISA
Figure 40: Trispecific mAbdAb Mepo-210-474 (supernatant) binding to human IL-13 in direct binding ELISA
Figure 41: Trispecific mAbdAb Mepo-210-474 (supernatant) binding to human IL-4 in direct binding ELISA
Figure 42: Cloning of the anti-TNF/anti-EGFR mAb-dAb
Figure 43. SDS-PAGE analysis of the anti-TNF/anti-EGFR mAb-dAb
Figure 44. SEC profile of the anti-TNF/anti-EGFR mAb-dAb (Example 10)
Figure 45: Anti-EGFR activity of Example 10
Figure 46. Anti-TNF activity of Example 10
Figure 47. SDS-PAGE analysis of the anti-TNF/anti-VEGF mAb-dAb (Example 11)
Figure 48. SEC profile of the anti-TNF/anti-VEGF mAb-dAb (Example 11)
Figure 49. Anti-VEGF activity of Example 11
Figure 50. Anti-TNF activity of example 11
Figure 51. Cloning of the anti-VEGF/anti-IL1R1 dAb-extended-IgG (Example 12)
Figure 52. SDS-PAGE analysis of the anti-TNF/anti-VEGF dAb-extended IgG A (Example 12)
Figure 53: SDS-PAGE analysis of the anti-TNF/anti-VEGF dAb-extended IgG B (Example 12)
Figure 54. SEC profile of the anti-TNF/anti-VEGF dAb-extended IgG A (Example 12)
Figure 55: SEC profile of the anti-TNF/anti-VEGF dAb-extended IgG B (Example 12)
Figure 56. Anti-VEGF activity of Example 12 (DMS2091)
Figure 57 Anti-VEGF activity of Example 12 (DMS2090)
Figure 58. Anti-IL1R1 activity of Example 12 (DMS2090)
Figure 59: Anti-IL1R1 activity of Example 12 (DMS2091)
Figure 60: Cloning of the anti-TNF/anti-VEGF/anti-EGFR mAb-dAb (Example 13)
Figure 61. SDS-PAGE analysis of the anti-TNF/anti-VEGF/anti-EGFR mAb-dAb (Example 13)
Figure 62: Anti-VEGF activity of Example 13
Figure 63: Anti-TNF activity of Example 13
Figure 64: Anti-EGFR activity of Example 13
Figure 65: SEC analysis of purified Bispecific antibodies, BPC1603 (A), BPC1604 (B), BPC1605 (C), BPC1606 (D)
Figure 66. Binding of bispecific antibodies to immobilised IGF-1 R
Figure 67. Binding of Bispecific antibodies to immobilised VEGF
Figure 68. Inhibition of ligand mediated receptor phosphorylation by various bispecific antibodies
Figure 69: Inhibition of ligand mediated receptor phosphorylation by various bispecific antibodies
Figure 70 ADCC assay with anti-CD20/IL-13 bispecifc antibody
Figure 71: ADCC assay with anti-CD20/IL-13 bispecific antibody
Figure 72: ADCC assay with anti-CD20/IL-13 bispecific antibody using a shorter dose range
Figure 73: ADCC assay with anti-CD20/IL-13 bispecific antibody using a shorter dose range
Figure 74: CDC assay with anti-CD20/IL-13 bispecific antibody
Figure 75: CDC assay with anti-CD20/IL-13 bispecific antibody
Figure 76: BPC1803 and BPC1804 binding in recombinant human IGF-1R ELISA
Figure 77: BPC1803 and BPC1804 binding in recombinant VEGF binding ELISA
Figure 78: BPC1805 and BPC1806 binding in recombinant human IGF-1R ELISA
Figure 79: BPC1805 and BPC1806 binding in recombinant human HER2 ELISA
Figure 80: BPC1807 and BPC1808 binding in recombinant human IGF-1R ELISA
Figure 81: BPC1807 and BPC1808 binding in recombinant human HER2 ELISA
Figure 82: BPC1809 binding in recombinant human IL-4 ELISA
Figure 83: BPC1809 binding in RNAse A ELISA.
Figure 84: BPC1816 binding in recombinant human IL-4 ELISA
Figure 85: BPC1816 binding in HEL ELISA
Figure 86: BPC1801 and BPC 1802 binding in recombinant human IGF-1R ELISA
Figure 87: BPC1801 and BPC1802 binding in recombinant human VEGFR2 ELISA
Figure 88 BPC1823 and BPC 1822 binding in recombinant human IL-4 ELISA
Figure 88b BPC1823 (higher concentration supernatant) binding in recombinant human IL-4 ELISA
Figure 89: BPC1823 and BPC1822 binding in recombinant human TNF-$\alpha$ ELISA
Figure 89b: BPC1823 (higher concentration supernatant) binding in recombinant human TNF-$\alpha$ ELISA

Figure 90: SEC profile for PascoH-474 GS removed
Figure 91:SEC profile for PascoH-TVAAPS-474 GS removed
Figure 92: SEC profile for PascoH-GS-ASTKGPT-474 2nd GS removed
Figure 93: SEC profile for 586H-210 GS removed
Figure 94: SEC profile for 586H-TVAAPS-210 GS removed
Figure 95: SDS PAGE for PascoH-474 GS removed (lane B) and PascoH-TVAAPS-474 GS removed (lane A)
Figure 96: SDS PAGE for PascoH-GS-ASTKGPT-474 2nd GS removed [A = non-reducing conditions, B = reducing conditions]
Figure 97: SDS PAGE for 586H-210 GS removed (lane A)
Figure 98: SDS PAGE for 586H-TVAAPS-210 GS removed (lane A)
Figure 99: Purified PascoH-474 GS removed and PascoH-TVAAPS-474 GS removed binding in human IL-4 ELISA
Figure 100: Purified PascoH-474 GS removed and PascoH-TVAAPS-474 GS removed binding in human IL-13 ELISA
Figure 101: Purified PascoH-474 GS removed, PascoH-TVAAPS-474 GS removed, PascoH-616 and PascoH-TVAAPS-616 binding in cynomolgus IL-13 ELISA Figure 102: mAbdAbs inhibition of human IL-4 binding to human IL-4Rα by ELISA
Figure 103: mAbdAbs inhibition of human IL-4 binding to human IL-4Rα by ELISA
Figure 104 Neutralisation of human IL-13 in TF-1 cell bioassays by mAbdAbs
Figure 105: Neutralisation of cynomolgus IL-13 in TF-1 cell bioassays by mAbdAbs
Figure 106: Neutralisation of human IL-4 in TF-1 cell bioassays by mAbdAbs
Figure 107: Neutralisation of cynomolgus IL-4 in TF-1 cell bioassays by mAbdAbs
Figure 108: Ability of mAbdAbs to inhibit binding of human IL-13 binding to human IL-13Rα2
Figure 109: SEC profile for PascoH-616
Figure 110: SEC profile for PascoH-TVAAPS_616
Figure 111:SDS PAGE for PascoH-616 [E1 = non-reducing conditions, E2 = reducing conditions]
Figure 112: SDS PAGE for PascoH-TVAAPS-616 [A = non-reducing conditions, B = reducing conditions]
Figure 113: purified PascoH-616 and PascoH-TVAAPS-616 binding in human IL-13 ELISA
Figure 114: Neutralisation of human IL-13 in TF-1 cell bioassays by mAbdAbs
Figure 114a: Neutralisation of cynomolgus IL-13 in TF-1 cell bioassays by mAbdAbs
Figure 115: Inhibition of IL-4 activity by PascoH-474 GS removed
Figure 116: Inhibition of IL-13 activity by PascoH-474 GS removed
Figure 117:Inhibition of IL-4 activity by 586-TVAAPS-210
Figure 118: Inhibition of IL-13 activity by 586-TVAAPS-210
Figure 119: Inhibition of IL-4 activity by Pascolizumab
Figure 120: Inhibition of IL-4 activity by DOM9-112-210
Figure 121: Inhibition of IL-13 activity by anti-IL13 mAb
Figure 122: Inhibition of IL-13 activity by DOM10-53-474
Figure 123: Activity of control mAb and dAb in IL-4 whole blood assay
Figure 124: Activity of control mAb and dAb in IL-13 whole blood assay
Figure 125: The concentration of drug remaining at various time points post-dose assessed by ELISA against both TNF & EGFR.
Figure 126: The concentration of drug remaining at various time points post-dose assessed by ELISA against both TNF & VEGF.
Figure 127: The concentration of drug remaining at various time points post-dose assessed by ELISA against both IL1R1 & VEGF.
Figure 128: SDS-PAGE of the purified DMS4010
Figure 129: SEC profile of the purified DMS4010
Figure 130: Anti-EGFR potency of DMS4010
Figure 131: anti-VEGF receptor binding assay
Figure 132: pharmacokinetic profile of the dual targeting anti-EGFR/anti-VEGF mAbdAb
Figure 133: SDS-PAGE analysis purified DMS4011
Figure 134: SEC profile of the purified DMS4011
Figure 135: Anti-EGFR potency of DMS4011
Figure 136: DMS4011 in anti-VEGF receptor binding assay
Figure 137: SDS-PAGE analysis of the purified samples DMS4023 and DMS4024
Figure 138: The SEC profile for DMS4023
Figure 139: The SEC profile for DMS4024
Figure 140: Anti-EGFR potency of the mAbdAb DMS4023
Figure 141: DMS4023 and DMS4024 in anti-VEGF receptor binding assay

Figure 142: SDS-PAGE analysis of the purified DMS4009
Figure 143: The SEC profile for DMS4009
Figure 144: Anti-EGFR potency of the mAbdAb DMS4009
Figure 145: DMS4009 in anti-VEGF receptor binding assay
Figure 146: SDS-PAGE analysis of the purified DMS4029
Figure 147: The SEC profile for DMS4029
Figure 148: Anti-EGFR potency of the mAbdAb DMS4029
Figure 149: DMS4029 in the IL-13 cell-based neutralisation assay
Figure 150: SDS-PAGE analysis of the purified samples DMS4013 and DMS4027
Figure 151: The SEC profile for DMS4013
Figure 152: The SEC profile for DMS4027
Figure 153: Anti-EGFR potency of the mAbdAb DMS4013
Figure 154: DMS4013 in anti-VEGF receptor binding assay
Figure 155: ;BPC1616 binding in recombinant human IL-12 ELISA
Figure 156: BPC1616 binding in recombinant human IL-18 ELISA
Figure 157: BPC1616 binding in recombinant human IL-4 ELISA
Figure 158: BPC1008, 1009 and BPC1010 binding in recombinant human IL-4 ELISA
Figure 159: BPC1008 binding in recombinant human IL-5 ELISA
Figure 160: BPC1008, 1009 and BPC1010 binding in recombinant human IL-1.3 ELISA
Figure 161: BPC1017 and BPC1018 binding in recombinant human c-MET ELISA
Figure 162: BPC1017 and BPC1018 binding in recombinant human VEGF ELISA
Figure 163: SEC profile for PascoH-TVAAPS-546
Figure 164: SEC profile for PascoH-TVAAPS-567
Figure 165: SDS PAGE for PascoH-TVAAPS-546 [A = non-reducing conditions, B = reducing conditions]
Figure 166: SDS PAGE for PascoH-TVAAPS-567 [A = non-reducing conditions, B = reducing conditions]
Figure 167: neutralisation data for human IL-13 in the TF-1 cell bioassay
Figure 168: neutralisation data for cynomolgus IL-13 in the TF-1 cell bioassay
Figure 169: mAbdAbs containing alternative isotypes binding in human IL-4 ELISA
Figure 170: mAbdAbs containing alternative isotypes binding in human IL-13 ELISA
Figure 171: BPC1818 and BPC1813 binding in recombinant human EGFR ELISA
Figure 172: BPC1818 and BPC1813 binding in recombinant human VEGFR2 ELISA
Figure 173: anti-IL5mAb-anti-IL13dAb binding in IL-13 ELISA
Figure 174: anti-IL5mAb-anti-IL13dAb binding in IL-5 ELISA
Figure 175: BPC1812 binding in recombinant human VEGFR2 ELISA
Figure 176: BPC1812 binding in recombinant human EGFR ELISA
Figure 177: mAbdAb binding in human IL-13 ELISA

**1. Domain antibodies**

**SEQ ID NO: 1 = DOM9-155-25**

DIQMTQSPSSLSASVGDRVTITCRASRPISDWLHWYQQKPGKAPKLLIAWASTLDSGVPSRF
SGSGSGTDFTLTISSLQPEDFATYYCLQEGWGPPTFGQGTKVEIKR

**SEQ ID NO: 2 = DOM9-155-147**

DIQMTQSPSSLSASVGDRVTITCRASRPISDWLHWYQQKPGKAPKLLIAWASSLYEGVPSRF
SGSGSGTDFTLTISSLQPEDFATYYCLQEGWGPPTFGQGTKVEIKR

**SEQ ID NO: 3 = DOM9-155-154.**

DIQMTQSPSSLSASVGDRVTITCRASRPISDWLHWYQQKPGKAPKLLIAWASSLQGGVPSRF
SGSGSGTDFTLTISSLQPEDFATYYCLQEGWGPPTFGQGTKVEIKR

**SEQ ID NO: 4 = DOM9-112-210**

EVQLLESGGGLVQPGGSLRLSCAASGFTFRNFGMGWVRQAPGKGLEWVSWIISSGTETYYAD
SVKGRFTISRDNSKNTLYLQMNSLRAEDTAVYYCAKSLGRFDYWGQGTLVTVSS

**SEQ ID NO: 5 = DOM10-53-474**

GVQLLESGGGLVQPGGSLRLSCAASGFTFAWYDMGWVRQAPGKGLEWVSSIDWHGEVTYYAD
SVKGRFTISRDNSKNTLYLQMNSLRAEDTAVYYCATAEDEPGYDYWGQGTLVTVSS

**SEQ ID NO: 60 = DNA sequence of DOM9-155-147 (protein = SEQ ID NO:2)**

GACATCCAGATGACCCAATCACCATCCTCCCTGTCTGCATCTGTAGGAGACCGTGTCACCAT
CACTtGCCGGGCAAGTCGCCCCATtAGCGACTGGTTACATtGGTATCAGCAGAAACCAGGGA
AAGCCCCCAAGCTCCTGATCGCCTGGGCGtCCTCGTTGTACGAGGGGGtCCCATCACGtTTC
AGTGGCAGTGGGTCGGGGACAGATTTCACTCTCACCATCAGCAGTCTGCAACCCGAAGATTT
CGCTACGTACTACTGTTTGCAGGAGGGGTGGGGTCCTCCGACGTTCGGCCAAGGGACCAAGG
TGGAAATCAAACGG

**SEQ ID NO: 61 = DNA sequence of DOM9-155-154 (protein = SEQ ID NO:3)**

GACATCCAGATGACCCAATCACCATCCTCCCTGTCTGCATCTGTAGGAGACCGTGTCACCAT
CACTTGCCGGGCAAGTCGCCCCATTAGCGACTGGTTACATTGGTATCAGCAGAAACCAGGGA
AAGCCCCCAAGCTCCTGATCGCCTGGGCGTCCAGCTTGCAGGGGGGGGTCCCATCACGTTTC
AGTGGCAGTGGGTCGGGGACAGATTTCACTCTCACCATCAGCAGTCTGCAACCCGAAGATTT
CGCTACGTACTACTGTTTGCAGGAGGGGTGGGGTCCTCCGACGTTCGGCCAAGGGACCAAGG
TGGAAATCAAACGG

**2. Linkers**

**SEQ ID NO: 6 = G4S linker**
GGGGS

**SEQ ID NO: 7 = linker**
TVAAPS

**SEQ ID NO: 8 = linker**
ASTKGPT

**SEQ ID NO: 9 = linker**
ASTKGPS

**SEQ ID NO: 10 = linker**
EPKSCDKTHTCPPCP

**SEQ ID NO: 11 = linker**
ELQLEESCAEAQDGELDG

### 3. Monoclonal antibodies

**SEQ ID NO: 12 = Anti-human IL13 mAb (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK

**SEQ ID NO: 13 = Anti-human IL13 mAb (L chain)**

DIVMTQSPLSLPVTPGEPASISCRSSQNIVHINGNTYLEWYLQKPGQSPRLLIYKISDRFSG
VPDRFSGSGSGTDFTLKISRVEADDVGIYYCFQGSHVPWTFGQGTKLEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**SEQ ID NO: 14 = Pascolizumab (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL

VKGFYPSDIAVEWESNGQPENNYKTTPPVLDSDGSFFLYSKLTVDKSRWQQGNVFSCSVMHE
ALHNHYTQKSLSLSPGK

**SEQ ID NO: 15 = Pascolizumab (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC

**SEQ ID NO: 65 = Mepolizumab (H chain)**

QVTLRESGPALVKPTQTLTLTCTVSGFSLTSYSVHWVRQPPGKGLEWLGVIWASGGTDYNSA
LMSRLSISKDTSRNQVVLTMTNMDPVDTATYYCARDPPSSLLRLDYWGRGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK

**SEQ ID NO: 66 = Mepolizumab (L chain)**

DIVMTQSPDSLAVSLGERATINCKSSQSLLNSGNQKNYLAWYQQKPGQPPKLLIYGASTRES
GVPDRFSGSGSGTDFTLTISSLQAEDVAVYYCQNVHSFPFTFGGGTKLEIKRTVAAPSVFIF
PPSDEQLKSGTASVVCLLNNFYPREAKVQWKVDNALQSGNSQESVTEQDSKDSTYSLSSTLT
LSKADYEKHKVYACEVTHQGLSSPVTKSFNRGEC

**SEQ ID NO: 67 = Anti-human IL-18 mAb (H chain)**

QVQLVQSGAEVKKPGASVKVSCKVSGEISTGYYFHWVRQAPGKGLEWMGRIDPEDDSTKYAE
RFKDRVTMTEDTSTDTAYMELSSLRSEDTAVYYCTTWRIYRDSSGRPFYVMDAWGQGTLVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSPGK

**SEQ ID NO: 68 = Anti-human IL-18 mAb (L chain)**

DIQMTQSPSSVSASVGDRVTITCLASEDIYTYLTWYQQKPGKAPKLLIYGANKLQDGVPSRF
SGSGSGTDYTLTISSLQPEDFATYYCLQGSKFPLTFGQGTKLEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

**4. Bispecific mAbdAbs**

NB, the underlined portion of the sequence in "bold" text corresponds to the linker. 'GS' amino acid residues) the DNA coding sequence of which was used as the BamHI cloning site) are not in 'bold' text, but are underlined.

**SEQ ID NO: 16 = 586H-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGSDIQMTQSPSSLSASVGDRVTITCRASRPISDWLHWYQQK
PGKAPKLLIAWASTLDSGVPSRFSGSGSGTDFTLTISSLQPEDFATYYCLQEGWGPPTFGQG
TKVEIKR

**SEQ ID NO: 17 = 586H-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGSDIQMTQSPSSLSASVGDRVTITCRASRPISDWLHWYQQK
PGKAPKLLIAWASSLYEGVPSRFSGSGSGTDFTLTISSLQPEDFATYYCLQEGWGPPTFGQG
TKVEIKR

**SEQ ID NO: 18 = 586H-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGSDIQMTQSPSSLSASVGDRVTITCRASRPISDWLHWYQQK
PGKAPKLLIAWASSLQGGVPSRFSGSGSGTDFTLTISSLQPEDFATYYCLQEGWGPPTFGQG
TKVEIKR

**SEQ ID NO: 19 = 586H-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK<u>GS</u>EVQLLESGGGLVQPGGSLRLSCAASGFTFRNFGMGWVRQ
APGKGLEWVSWIISSGTETYYADSVKGRFTISRDNSKNTLYLQMNSLRAEDTAVYYCAKSLG
RFDYWGQGTLVTVSS

**SEQ ID NO: 20 = 586H-G4S-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK<u>**GGGGS**</u>DIQMTQSPSSLSASVGDRVTITCRASRPISDWLHWY
QQKPGKAPKLLIAWASTLDSGVPSRFSGSGSGTDFTLTISSLQPEDFATYYCLQEGWGPPTF
GQGTKVEIKR

**SEQ ID NO: 21 = 586H-G4S-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK<u>**GGGGS**</u>DIQMTQSPSSLSASVGDRVTITCRASRPISDWLHWY
QQKPGKAPKLLIAWASSLYEGVPSRFSGSGSGTDFTLTISSLQPEDFATYYCLQEGWGPPTF
GQGTKVEIKR

**SEQ ID NO: 22 = 586H-G4S-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS

VMHEALHNHYTQKSLSLSPGK**GGGGS**DIQMTQSPSSLSASVGDRVTITCRASRPISDWLHWY
QQKPGKAPKLLIAWASSLQGGVPSRFSGSGSGTDFTLTISSLQPEDFATYYCLQEGWGPPTF
GQGTKVEIKR

**SEQ ID NO: 23 = 586H-G4S-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**GGGGS**EVQLLESGGGLVQPGGSLRLSCAASGFTFRNFGMGW
VRQAPGKGLEWVSWIISSGTETYYADSVKGRFTISRDNSKNTLYLQMNSLRAEDTAVYYCAK
SLGRFDYWGQGTLVTVSS

**SEQ ID NO: 24 = 586H-TVAAPS-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSDIQMTQSPSSLSASVGDRVTITCRASRPISDWL
HWYQQKPGKAPKLLIAWASTLDSGVPSRFSGSGSGTDFTLTISSLQPEDFATYYCLQEGWGP
PTFGQGTKVEIKR

**SEQ ID NO: 25 = 586H-TVAAPS-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSDIQMTQSPSSLSASVGDRVTITCRASRPISDWL
HWYQQKPGKAPKLLIAWASSLYEGVPSRFSGSGSGTDFTLTISSLQPEDFATYYCLQEGWGP
PTFGQGTKVEIKR

**SEQ ID NO: 26 = 586H-TVAAPS-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS

SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSDIQMTQSPSSLSASVGDRVTITCRASRPISDWL
HWYQQKPGKAPKLLIAWASSLQGGVPSRFSGSGSGTDFTLTISSLQPEDFATYYCLQEGWGP
PTFGQGTKVEIKR

**SEQ ID NO: 27 = 586H-TVAAPS-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSEVQLLESGGGLVQPGGSLRLSCAASGFTFRNFG
MGWVRQAPGKGLEWVSWIISSGTETYYADSVKGRFTISRDNSKNTLYLQMNSLRAEDTAVYY
CAKSLGRFDYWGQGTLVTVSS

**SEQ ID NO: 28 = 586H-ASTKG-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ASTKGPT**GSDIQMTQSPSSLSASVGDRVTITCRASRPIS
DWLHWYQQKPGKAPKLLIAWASTLDSGVPSRFSGSGSGTDFTLTISSLQPEDFATYYCLQEG
WGPPTFGQGTKVEIKR

**SEQ ID NO: 29 = 586H-ASTKG-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ASTKGPT**GSDIQMTQSPSSLSASVGDRVTITCRASRPIS

DWLHWYQQKPGKAPKLLIAWASSLYEGVPSRFSGSGSGTDFTLTISSLQPEDFATYYCLQEG
WGPPTFGQGTKVEIKR

**SEQ ID NO: 30 = 586H-ASTKG-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ASTKGPT**GSDIQMTQSPSSLSASVGDRVTITCRASRPIS
DWLHWYQQKPGKAPKLLIAWASSLQGGVPSRFSGSGSGTDFTLTISSLQPEDFATYYCLQEG
WGPPTFGQGTKVEIKR

**SEQ ID NO: 31 = 586H-ASTKG-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ASTKGPT**GSEVQLLESGGGLVQPGGSLRLSCAASGFTFR
NFGMGWVRQAPGKGLEWVSWIISSGTETYYADSVKGRFTISRDNSKNTLYLQMNSLRAEDTA
VYYCAKSLGRFDYWGQGTLVTVSS

**SEQ ID NO: 32 = 586H-EPKSC-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**EPKSCDKTHTCPPCP**GSDIQMTQSPSSLSASVGDRVTIT
CRASRPISDWLHWYQQKPGKAPKLLIAWASTLDSGVPSRFSGSGSGTDFTLTISSLQPEDFA
TYYCLQEGWGPPTFGQGTKVEIKR

**SEQ ID NO: 33 = 586H-EPKSC-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG

LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**EPKSCDKTHTCPPCP**GSDIQMTQSPSSLSASVGDRVTIT
CRASRPISDWLHWYQQKPGKAPKLLIAWASSLYEGVPSRFSGSGSGTDFTLTISSLQPEDFA
TYYCLQEGWGPPTFGQGTKVEIKR

**SEQ ID NO: 34 = 586H-EPKSC-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**EPKSCDKTHTCPPCP**GSDIQMTQSPSSLSASVGDRVTIT
CRASRPISDWLHWYQQKPGKAPKLLIAWASSLQGGVPSRFSGSGSGTDFTLTISSLQPEDFA
TYYCLQEGWGPPTFGQGTKVEIKR

**SEQ ID NO: 35 = 586H-EPKSC-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**EPKSCDKTHTCPPCP**GSEVQLLESGGGLVQPGGSLRLSC
AASGFTFRNFGMGWVRQAPGKGLEWVSWIISSGTETYYADSVKGRFTISRDNSKNTLYLQMN
SLRAEDTAVYYCAKSLGRFDYWGQGTLVTVSS

**SEQ ID NO: 36 = 586H-ELQLE-25 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ELQLEESCAEAQDGELDG**GSDIQMTQSPSSLSASVGDRV
TITCRASRPISDWLHWYQQKPGKAPKLLIAWASTLDSGVPSRFSGSGSGTDFTLTISSLQPE
DFATYYCLQEGWGPPTFGQGTKVEIKR

**SEQ ID NO: 37 = 586H-ELQLE-147 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ELQLEESCAEAQDGELDG**GSDIQMTQSPSSLSASVGDRV
TITCRASRPISDWLHWYQQKPGKAPKLLIAWASSLYEGVPSRFSGSGSGTDFTLTISSLQPE
DFATYYCLQEGWGPPTFGQGTKVEIKR

**SEQ ID NO: 38 = 586H-ELQLE-154 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ELQLEESCAEAQDGELDG**GSDIQMTQSPSSLSASVGDRV
TITCRASRPISDWLHWYQQKPGKAPKLLIAWASSLQGGVPSRFSGSGSGTDFTLTISSLQPE
DFATYYCLQEGWGPPTFGQGTKVEIKR

**SEQ ID NO: 39 = 586H-ELQLE-210 (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ELQLEESCAEAQDGELDG**GSEVQLLESGGGLVQPGGSLR
LSCAASGFTFRNFGMGWVRQAPGKGLEWVSWIISSGTETYYADSVKGRFTISRDNSKNTLYL
QMNSLRAEDTAVYYCAKSLGRFDYWGQGTLVTVSS

**SEQ ID NO: 40 = 586H-GS (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV

VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS

**SEQ ID NO: 41 = 586H-ASTKG (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ASTKGPT**GS

**SEQ ID NO: 42 = 586H-EPKSC (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**EPKSCDKTHTCPPCP**GS

**SEQ ID NO: 43 = 586H-ELQLE (H chain)**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGS**ELQLEESCAEAQDGELDG**GS

**SEQ ID NO: 44 = 586L-G4S-25 (L chain)**

DIVMTQSPLSLPVTPGEPASISCRSSQNIVHINGNTYLEWYLQKPGQSPRLLIYKISDRFSG VPDRFSGSGSGTDFTLKISRVEADDVGIYYCFQGSHVPWTFGQGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGEC**GGGGS**DIQMTQSPSSLSASVGDRVTITCR ASRPISDWLHWYQQKPGKAPKLLIAWASTLDSGVPSRFSGSGSGTDFTLTISSLQPEDFATY YCLQEGWGPPTFGQGTKVEIKR

**SEQ ID NO: 45 = 586L-G4S-147 (L chain)**

DIVMTQSPLSLPVTPGEPASISCRSSQNIVHINGNTYLEWYLQKPGQSPRLLIYKISDRFSG VPDRFSGSGSGTDFTLKISRVEADDVGIYYCFQGSHVPWTFGQGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGEC**GGGGS**DIQMTQSPSSLSASVGDRVTITCR ASRPISDWLHWYQQKPGKAPKLLIAWASSLYEGVPSRFSGSGSGTDFTLTISSLQPEDFATY YCLQEGWGPPTFGQGTKVEIKR

**SEQ ID NO: 46 = 586L-G4S-154 (L chain)**

DIVMTQSPLSLPVTPGEPASISCRSSQNIVHINGNTYLEWYLQKPGQSPRLLIYKISDRFSG VPDRFSGSGSGTDFTLKISRVEADDVGIYYCFQGSHVPWTFGQGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGEC**GGGGS**DIQMTQSPSSLSASVGDRVTITCR ASRPISDWLHWYQQKPGKAPKLLIAWASSLQGGVPSRFSGSGSGTDFTLTISSLQPEDFATY YCLQEGWGPPTFGQGTKVEIKR

**SEQ ID NO: 47 = 586L-G4S-210 (L chain)**

DIVMTQSPLSLPVTPGEPASISCRSSQNIVHINGNTYLEWYLQKPGQSPRLLIYKISDRFSG VPDRFSGSGSGTDFTLKISRVEADDVGIYYCFQGSHVPWTFGQGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGEC**GGGGS**EVQLLESGGGLVQPGGSLRLSCAA SGFTFRNFGMGWVRQAPGKGLEWVSWIISSGTETYYADSVKGRFTISRDNSKNTLYLQMNSL RAEDTAVYYCAKSLGRFDYWGQGTLVTVSS

**SEQ ID NO: 48 = PascoH-474 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSGVQLLESGGGLVQPGGSLRLSCAASGFTFAWYDMGWVRQAPGK
GLEWVSSIDWHGEVTYYADSVKGRFTISRDNSKNTLYLQMNSLRAEDTAVYYCATAEDEPGY
DYWGQGTLVTVSS

**SEQ ID NO: 49 = PascoH-G4S-474 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE

ALHNHYTQKSLSLSPGK**GGGGS**GVQLLESGGGLVQPGGSLRLSCAASGFTFAWYDMGWVRQA
PGKGLEWVSSIDWHGEVTYYADSVKGRFTISRDNSKNTLYLQMNSLRAEDTAVYYCATAEDE
PGYDYWGQGTLVTVSS

**SEQ ID NO: 50 = PascoH-TVAAPS-474** (H chain)

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**TVAAPS**GSGVQLLESGGGLVQPGGSLRLSCAASGFTFAWYDMGWV
RQAPGKGLEWVSSIDWHGEVTYYADSVKGRFTISRDNSKNTLYLQMNSLRAEDTAVYYCATA
EDEPGYDYWGQGTLVTVSS

**SEQ ID NO: 51 = PascoH-ASTKG-474 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGS**ASTKGPT**GSGVQLLESGGGLVQPGGSLRLSCAASGFTFAWYDM
GWVRQAPGKGLEWVSSIDWHGEVTYYADSVKGRFTISRDNSKNTLYLQMNSLRAEDTAVYYC
ATAEDEPGYDYWGQGTLVTVSS

**SEQ ID NO: 52 = PascoH-EPKSC-474 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGS**EPKSCDKTHTCPPCP**GSGVQLLESGGGLVQPGGSLRLSCAASG
FTFAWYDMGWVRQAPGKGLEWVSSIDWHGEVTYYADSVKGRFTISRDNSKNTLYLQMNSLRA
EDTAVYYCATAEDEPGYDYWGQGTLVTVSS

**SEQ ID NO: 53 = PascoH-ELQLE-474 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST

KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGS**ELQLEESCAEAQDGELDG**GSGVQLLESGGGLVQPGGSLRLSCA
ASGFTFAWYDMGWVRQAPGKGLEWVSSIDWHGEVTYYADSVKGRFTISRDNSKNTLYLQMNS
LRAEDTAVYYCATAEDEPGYDYWGQGTLVTVSS

**SEQ ID NO: 54 = PascoL-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGECGSGVQLLESGGGLVQPGGSLRLSCAASGFT
FAWYDMGWVRQAPGKGLEWVSSIDWHGEVTYYADSVKGRFTISRDNSKNTLYLQMNSLRAED
TAVYYCATAEDEPGYDYWGQGTLVTVSS

**SEQ ID NO: 55 = PascoL-G4S-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC**GGGGS**GVQLLESGGGLVQPGGSLRLSCAAS
GFTFAWYDMGWVRQAPGKGLEWVSSIDWHGEVTYYADSVKGRFTISRDNSKNTLYLQMNSLR
AEDTAVYYCATAEDEPGYDYWGQGTLVTVSS

**SEQ ID NO: 56 = PascoL-TVAAPS-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC**TVAAPS**GSGVQLLESGGGLVQPGGSLRLSC
AASGFTFAWYDMGWVRQAPGKGLEWVSSIDWHGEVTYYADSVKGRFTISRDNSKNTLYLQMN
SLRAEDTAVYYCATAEDEPGYDYWGQGTLVTVSS

**SEQ ID NO: 57 = PascoL-ASTKG-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC**ASTKGPT**GSGVQLLESGGGLVQPGGSLRLS
CAASGFTFAWYDMGWVRQAPGKGLEWVSSIDWHGEVTYYADSVKGRFTISRDNSKNTLYLQM
NSLRAEDTAVYYCATAEDEPGYDYWGQGTLVTVSS

**SEQ ID NO: 58 = PascoL-EPKSC-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC**EPKSCDKTHTCPPCP**GSGVQLLESGGGLVQ
PGGSLRLSCAASGFTFAWYDMGWVRQAPGKGLEWVSSIDWHGEVTYYADSVKGRFTISRDNS
KNTLYLQMNSLRAEDTAVYYCATAEDEPGYDYWGQGTLVTVSS

**SEQ ID NO: 59 = PascoL-ELQLE-474 (L chain)**

DIVLTQSPSSLSASVGDRVTITCKASQSVDYDGDSYMNWYQQKPGKAPKLLIYAASNLESGI
PSRFSGSGSGTDFTFTISSLQPEDIATYYCQQSNEDPPTFGQGTKVEIKRTVAAPSVFIFPP
SDEQLKSGTASVVCLLNNFYPREAKVQWKVDNALQSGNSQESVTEQDSKDSTYSLSSTLTLS
KADYEKHKVYACEVTHQGLSSPVTKSFNRGEC**ELQLEESCAEAQDGELDG**GSGVQLLESGGG
LVQPGGSLRLSCAASGFTFAWYDMGWVRQAPGKGLEWVSSIDWHGEVTYYADSVKGRFTISR
DNSKNTLYLQMNSLRAEDTAVYYCATAEDEPGYDYWGQGTLVTVSS

5. **Cytokines**

**SEQ ID NO: 62 = IL-4 (Interleukin-4)**

HKCDITLQEIIKTLNSLTEQKTLCTELTVTDIFAASKNTTEKETFCRAATVLRQFYSHHEKD
TRCLGATAQQFHRHKQLIRFLKRLDRNLWGLAGLNSCPVKEANQSTLENFLERLKTIMREKY
SKCSS

**SEQ ID NO: 63 = IL-13 (Interleukin-13)**

GPVPPSTALRELIEELVNITQNQKAPLCNGSMVWSINLTAGMYCAALESLINVSGCSAIEKT
QRMLSGFCPHKVSAGQFSSLHVRDTKIEVAQFVKDLLLHLKKLFREGRFN

**6. Signal sequence**

**SEQ ID NO: 64 = Mammalian amino acid signal sequence**
MGWSCIILFLVATATGVHS

**7. IGF-1R binding CDRs**

**SEQ ID 80 = VH CDR3**
WILYYGRSKWYFDV

**SEQ ID 81 = VH CDR2**
NINPNNGGTNYNQKFKD

**SEQ ID 82 = VH CDR1**
DYYMN

**SEQ ID 83 = VL COR1**
RSSQSIVQSNGDTYLE

**SEQ ID 84 = alternative VL CDR2**
RISNRFS

**SEQ ID 85 = VL CDR3**
FQGSHVPYT

**SEQ ID 86 = VL CDR2**
RVSNRFS

**8. Trispecific mAbdAbs**

**SEQ ID NO: 69 = IL18mAb-G4S-DOM9-112-210 (H chain)**

QVQLVQSGAEVKKPGASVKVSCKVSGEISTGYYFHWVRQAPGKGLEWMGRIDPEDDSTKYAE
RFKDRVTMTEDTSTDTAYMELSSLRSEDTAVYYCTTWRIYRDSSGRPFYVMDAWGQGTLVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSC
SVMHEALHNHYTQKSLSLSPGK**GGGGS**EVQLLESGGGLVQPGGSLRLSCAASGFTFRNFGMG
WVRQAPGKGLEWVSWIISSGTETYYADSVKGRFTISRDNSKNTLYLQMNSLRAEDTAVYYCA
KSLGRFDYWGQGTLVTVSS

**SEQ ID NO: 70 = IL18mAb-G4S-DOM10-53-474 (L chain)**

DIQMTQSPSSVSASVGDRVTITCLASEDIYTYLTWYQQKPGKAPKLLIYGANKLQDGVPSRF
SGSGSGTDYTLTISSLQPEDFATYYCLQGSKFPLTFGQGTKLEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC**GGGGS**GVQLLESGGGLVQPGGSLRLSCAASGFTF
AWYDMGWVRQAPGKGLEWVSSIDWHGEVTYYADSVKGRFTISRDNSKNTLYLQMNSLRAEDT
AVYYCATAEDEPGYDYWGQGTLVTVSS

**SEQ ID NO: 71 = IL-5 mAb-G4S-DOM9-112-210 heavy chain**

QVTLRESGPALVKPTQTLTLTCTVSGFSLTSYSVHWVRQPPGKGLEWLGVIWASGGTDYNSA
LMSRLSISKDTSRNQVVLTMTNMDPVDTATYYCARDPPSSLLRLDYWGRGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK**GGGGS**EVQLLESGGGLVQPGGSLRLSCAASGFTFRNFGMGWVRQAPG
KGLEWVSWIISSGTETYYADSVKGRFTISRDNSKNTLYLQMNSLRAEDTAVYYCAKSLGRFD
YWGQGTLVTVSS

**SEQ ID NO: 72 = IL-5 mAb-G4S-DOM10-53-474 light chain**

DIVMTQSPDSLAVSLGERATINCKSSQSLLNSGNQKNYLAWYQQKPGQPPKLLIYGASTRES
GVPDRFSGSGSGTDFTLTISSLQAEDVAVYYCQNVHSFPFTFGGGTKLEIKRTVAAPSVFIF
PPSDEQLKSGTASVVCLLNNFYPREAKVQWKVDNALQSGNSQESVTEQDSKDSTYSLSSTLT
LSKADYEKHKVYACEVTHQGLSSPVTKSFNRGEC**GGGGS**GVQLLESGGGLVQPGGSLRLSCA
ASGFTFAWYDMGWVRQAPGKGLEWVSSIDWHGEVTYYADSVKGRFTISRDNSKNTLYLQMNS
LRAEDTAVYYCATAEDEPGYDYWGQGTLVTVSS

**9. Dual Targeting anti-TNF/anti-EGFR mAbdAb**

**SEQ ID NO: 73 = anti-TNFmAb (L chain)**

DIQMTQSPSSLSASVGDRVTITCRASQGIRNYLAWYQQKPGKAPKLLIYAASTLQSGVPSRF
SGSGSGTDFTLTISSLQPEDVATYYCQRYNRAPYTFGQGTKVEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

**SEQ ID NO: 74 = anti-TNFmAb-DOM16-39-542 (H chain)**

EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKSTGDIQMTQSPSSLSASVGDRVTITCRASQWIGNLLDWYQQKPGK
APKLLIYYASFLQSGVPSRFSGSGYGTDFTLTISSLQPEDFATYYCQQANPAPLTFGQGTKV
EIKR

**10. Dual Targeting anti-TNF/anti-VEGF mAbdAb**

**SEQ ID NO: 75 = anti-TNFmAb-DOM15-26-593 (H chain)**

EVQLVESGGGLVQPGRSLRLSCAASGFTFDDYAMHWVRQAPGKGLEWVSAITWNSGHIDYAD
SVEGRFTISRDNAKNSLYLQMNSLRAEDTAVYYCAKVSYLSTASSLDYWGQGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKSTGEVQLLVSGGGLVQPGGSLRLSCAASGFTFKAYPMMWVRQAPG
KGLEWVSEISPSGSYTYYADSVKGRFTISRDNSKNTLYLQMNSLRAEDTAVYYCAKDPRKLD
YWGQGTLVTVSS

**11. Dual Targeting anti-IL1R1/anti-VEGF dAb-extended IgG**

**SEQ ID NO: 76 = DOM 15-26-593-VHdUMMY (H chain)**

EVQLLVSGGGLVQPGGSLRLSCAASGFTFKAYPMMWVRQAPGKGLEWVSEISPSGSYTYYAD
SVKGRFTISRDNSKNTLYLQMNSLRAEDTAVYYCAKDPRKLDYWGQGTLVTVSSASTKGPSE
VQLLESGGGLVQPGGSLRLSCAASGFTFSSYAMSWVRQAPGKGLEWVSAISGSGGSTYYADS
VKGRFTISRDNSKNTLYLQMNSLRAEDTAVYYCAKSYGAFDYWGQGTLVTVSSASTKGPSVF

PLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHY
TQKSLSLSPGK

**SEQ ID NO: 77 = DOM4-130-54-VkdUMMY (L chain)**

DIQMTQSPSSLSASVGDRVTITCRASQDIYLNLDWYQQKPGKAPKLLINFGSELQSGVPSRF
SGSGYGTDFTLTISSLQPEDFATYYCQPSFYFPYTFGQGTKVEIKRTVAAPSDIQMTQSPSS
LSASVGDRVTITCRASQSISSYLNWYQQKPGKAPKLLIYAASSLQSGVPSRFSGSGSGTDFT
LTISSLQPEDFATYYCQQSYSTPNTFGQGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEV
THQGLSSPVTKSFNRGEC

**12. Triple Targeting anti-TNF/anti-EGFR/anti-VEGF mAbdAb**

**SEQ ID NO: 78 = DOM 15-26-anti-TNFmAb (H chain)**

EVQLLESGGGLVQPGGSLRLSCAASGFTFGAYPMMWVRQAPGKGLEWVSEISPSGSYTYYAD
SVKGRFTISRDNSKNTLYLQMNSLRAEDTAVYYCAKDPRKFDYWGQGTLVTVSSASTKGPSE
VQLVESGGGLVQPGRSLRLSCAASGFTFDDYAMHWVRQAPGKGLEWVSAITWNSGHIDYADS
VEGRFTISRDNAKNSLYLQMNSLRAEDTAVYYCAKVSYLSTASSLDYWGQGTLVTVSSASTK
GPSVFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVFSCSVMHEA
LHNHYTQKSLSLSPGK

**SEQ ID NO: 79 = DOM 16-39-542-anti-TNFmAb (L chain)**

DIQMTQSPSSLSASVGDRVTITCRASQWIGNLLDWYQQKPGKAPKLLIYYASFLQSGVPSRF
SGSGYGTDFTLTISSLQPEDFATYYCQQANPAPLTFGQGTKVEIKRTVAAPSDIQMTQSPSS
LSASVGDRVTITCRASQGIRNYLAWYQQKPGKAPKLLIYAASTLQSGVPSRFSGSGSGTDFT
LTISSLQPEDVATYYCQRYNRAPYTFGQGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEV
THQGLSSPVTKSFNRGEC

**SEQ ID NO: 87 = 586H-210 (H chain) GS removed**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV

VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKEVQLLESGGGLVQPGGSLRLSCAASGFTFRNFGMGWVRQAP
GKGLEWVSWIISSGTETYYADSVKGRFTISRDNSKNTLYLQMNSLRAEDTAVYYCAKSLGRF
DYWGQGTLVTVSS

**SEQ ID NO: 88 = 586H-TVAAPS-210 (H chain) GS removed**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**EVQLLESGGGLVQPGGSLRLSCAASGFTFRNFGMG
WVRQAPGKGLEWVSWIISSGTETYYADSVKGRFTISRDNSKNTLYLQMNSLRAEDTAVYYCA
KSLGRFDYWGQGTLVTVSS

**SEQ ID NO: 89 = 586H-ASTKGPT-210 (H chain) both GS removed**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**ASTKGPT**EVQLLESGGGLVQPGGSLRLSCAASGFTFRNFGM
GWVRQAPGKGLEWVSWIISSGTETYYADSVKGRFTISRDNSKNTLYLQMNSLRAEDTAVYYC
AKSLGRFDYWGQGTLVTVSS

**SEQ ID NO: 90 = 586H-ASTKGPS-210 (H chain) both GS removed**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**ASTKGPS**EVQLLESGGGLVQPGGSLRLSCAASGFTFRNFGM
GWVRQAPGKGLEWVSWIISSGTETYYADSVKGRFTISRDNSKNTLYLQMNSLRAEDTAVYYC
AKSLGRFDYWGQGTLVTVSS

**SEQ ID NO: 91 = PascoH-474 (H chain) GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGVQLLESGGGLVQPGGSLRLSCAASGFTFAWYDMGWVRQAPGKGL
EWVSSIDWHGEVTYYADSVKGRFTISRDNSKNTLYLQMNSLRAEDTAVYYCATAEDEPGYDY
WGQGTLVTVSS

**SEQ ID NO: 92 = PascoH-TVAAPS-474 (H chain) GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**TVAAPS**GVQLLESGGGLVQPGGSLRLSCAASGFTFAWYDMGWVRQ
APGKGLEWVSSIDWHGEVTYYADSVKGRFTISRDNSKNTLYLQMNSLRAEDTAVYYCATAED
EPGYDYWGQGTLVTVSS

**SEQ ID NO: 93 = PascoH-ASTKGPT-474 (H chain) Both GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**ASTKGPT**GVQLLESGGGLVQPGGSLRLSCAASGFTFAWYDMGWVR
QAPGKGLEWVSSIDWHGEVTYYADSVKGRFTISRDNSKNTLYLQMNSLRAEDTAVYYCATAE
DEPGYDYWGQGTLVTVSS

**SEQ ID NO: 94 = PascoH-ASTKGPS-474 (H chain) Both GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL

VKGFYPSDIAVEWESNGQPENNYKTTPPVLDSDGSFFLYSKLTVDKSRWQQGNVFSCSVMHE
ALHNHYTQKSLSLSPGK**ASTKGPS**GVQLLESGGGLVQPGGSLRLSCAASGFTFAWYDMGWVR
QAPGKGLEWVSSIDWHGEVTYYADSVKGRFTISRDNSKNTLYLQMNSLRAEDTAVYYCATAE
DEPGYDYWGQGTLVTVSS

**SEQ ID NO: 95 = PascoH-ASTKGPS-474 (H chain) Second GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGS**ASTKGPS**GVQLLESGGGLVQPGGSLRLSCAASGFTFAWYDMGW
VRQAPGKGLEWVSSIDWHGEVTYYADSVKGRFTISRDNSKNTLYLQMNSLRAEDTAVYYCAT
AEDEPGYDYWGQGTLVTVSS

**SEQ ID NO: 96 = PascoH-ASTKGPT-474 (H chain) Second GS removed**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGS**ASTKGPT**GVQLLESGGGLVQPGGSLRLSCAASGFTFAWYDMGW
VRQAPGKGLEWVSSIDWHGEVTYYADSVKGRFTISRDNSKNTLYLQMNSLRAEDTAVYYCAT
AEDEPGYDYWGQGTLVTVSS

**SEQ ID NO: 97 = CDRH1**
GYSITSDFAWN

**SEQ ID NO: 98 = CDRH2**
GYISYSGNYNPSLK

**SEQ ID NO: 99 = CDRH3**

VTAGRGFPY

**SEQ ID NO: 100 = CDRL1**
HSSQDINSNIG

**SEQ ID NO: 101 = CDRL2**
HGINLDD

**SEQ ID NO: 102 = CDRL3**
VQYAQFPWT

**SEQ ID NO: 103 = EGFR epitope**
CGADSYEMEEDGVRKC

**SEQ ID NO: 104 = CDRH1**
SDFAWN

**SEQ ID NO: 105 = CDRH2**
YISYSGNYNPSLK

**SEQ ID NO: 106 = CDRH3**
AGRGFPY

**SEQ ID NO: 107 = CDRH2**
YISYSGNYNPSLKS

**SEQ ID NO: 108 = Heavy chain of anti-IGF-1R antibody H0L0 with DOM15-26-593 fused at C-terminus with TVAAPSGS linker**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKTVAAPSGSEVQLLVSGGGLVQPGGSLRLSCAASGFTFKAYPMM
WVRQAPGKGLEWVSEISPSGSYTYYADSVKGRFTISRDNSKNTLYLQMNSLRAEDTAVYYCA
KDPRKLDYWGQGTLVTVSS

**SEQ ID NO: 109 = Heavy chain of anti-IGF-1R antibody H0L0 with DOM15-26-593 fused at C-terminus with GS linker**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKGSEVQLLVSGGGLVQPGGSLRLSCAASGFTFKAYPMMWVRQAP
GKGLEWVSEISPSGSYTYYADSVKGRFTISRDNSKNTLYLQMNSLRAEDTAVYYCAKDPRKL
DYWGQGTLVTVSS

**SEQ ID NO: 110 = Heavy chain of anti-IGF-1R antibody H0L0**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA

STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK

**SEQ ID NO: 111 = Light chain of anti-IGF-1R antibody H0L0 with DOM15-26-593 fused at C-terminus with TVAAPSGS linker**

DIVMTQSPLSLPVTPGEPASISCRSSQSIVQSNGDTYLEWYLQKPGQSPQLLIYRVSNRFSG
VPDRFSGSGSGTDFTLKISRVEAEDVGVYYCFQGSHVPYTFGQGTKLEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGECTVAAPSGSEVQLLVSGGGLVQPGGSLRLS
CAASGFTFKAYPMMWVRQAPGKGLEWVSEISPSGSYTYYADSVKGRFTISRDNSKNTLYLQM
NSLRAEDTAVYYCAKDPRKLDYWGQGTLVTVSS

**SEQ ID NO: 112 = Light chain of anti-IGF-1R antibody H0L0 with DOM15-26-593 fused at C-terminus with GS linker**

DIVMTQSPLSLPVTPGEPASISCRSSQSIVQSNGDTYLEWYLQKPGQSPQLLIYRVSNRFSG
VPDRFSGSGSGTDFTLKISRVEAEDVGVYYCFQGSHVPYTFGQGTKLEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGECGSEVQLLVSGGGLVQPGGSLRLSCAASGF
TFKAYPMMWVRQAPGKGLEWVSEISPSGSYTYYADSVKGRFTISRDNSKNTLYLQMNSLRAE
DTAVYYCAKDPRKLDYWGQGTLVTVSS

**SEQ ID NO: 113 = Light chain of anti-IGF-1R antibody H0L0**

DIVMTQSPLSLPVTPGEPASISCRSSQSIVQSNGDTYLEWYLQKPGQSPQLLIYRVSNRFSG
VPDRFSGSGSGTDFTLKISRVEAEDVGVYYCFQGSHVPYTFGQGTKLEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**SEQ ID NO: 114 = Variable heavy domain of antibody 2B9**

QVQLKQSGPGLVQSSQSLSITCTISGFSLTSHGIYWLRQSPGKGLEWLGVIWSGGSADYNAA
FISRLSISKDNSKSQVFFKMNSLQADDTAIYYCARSPYYYRSSLYAMDYWGQGTSVTVSS

**SEQ ID NO: 115 = Variable light domain of antibody 2B9**

NIVLTQSPKSMSMSIGERVTLSCKASENVGTYVSWYQQKAEQSPKLLIYGASNRHTGVPDRF
TGSGSSTDFTLTISSVQAEDLADYHCGQSYSDPLTFGAGTKLELKRA

**SEQ ID NO: 116 = Protein sequence of anti-CD20 mAb heavy chain with TVAAPSGS linker and DOM10-53-474 domain antibody fused at C-terminus**

QVQLQQPGAELVKPGASVKMSCKASGYTFTSYNMHWVKQTPGRGLEWIGAIYPGNGDTSYNQ
KFKGKATLTADKSSSTAYMQLSSLTSEDSAVYYCARSTYYGGDWYFNVWGAGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP

PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKTVAAPSGSGVQLLESGGGLVQPGGSLRLSCAASGFTFAWYDMGWV
RQAPGKGLEWVSSIDWHGEVTYYADSVKGRFTISRDNSKNTLYLQMNSLRAEDTAVYYCATA
EDEPGYDYWGQGTLVTVSS

**SEQ ID NO: 117 = Protein sequence anti-CD20 mAb VL-human CK light chain**

QIVLSQSPAILSASPGEKVTMTCRASSSVSYIHWFQQKPGSSPKPWIYATSNLASGVPVRFS
GSGSGTSYSLTISRVEAEDAATYYCQQWTSNPPTFGGGTKLEIKRTVAAPSVFIFPPSDEQL
KSGTASVVCLLNNFYPREAKVQWKVDNALQSGNSQESVTEQDSKDSTYSLSSTLTLSKADYE
KHKVYACEVTHQGLSSPVTKSFNRGEC

**SEQ ID NO: 118 = Protein sequence of anti-CD20 mAb heavy chain with GS linker and DOM10-53-474 domain antibody fused at C-terminus**

QVQLQQPGAELVKPGASVKMSCKASGYTFTSYNMHWVKQTPGRGLEWIGAIYPGNGDTSYNQ
KFKGKATLTADKSSSTAYMQLSSLTSEDSAVYYCARSTYYGGDWYFNVWGAGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSGVQLLESGGGLVQPGGSLRLSCAASGFTFAWYDMGWVRQAPGK
GLEWVSSIDWHGEVTYYADSVKGRFTISRDNSKNTLYLQMNSLRAEDTAVYYCATAEDEPGY
DYWGQGTLVTVSS

**SEQ ID NO: 119 = Protein sequence of anti-CD20 mAb light chain with TVAAPSGS linker and DOM10-53-474 domain antibody fused at C-terminus**

QIVLSQSPAILSASPGEKVTMTCRASSSVSYIHWFQQKPGSSPKPWIYATSNLASGVPVRFS
GSGSGTSYSLTISRVEAEDAATYYCQQWTSNPPTFGGGTKLEIKRTVAAPSVFIFPPSDEQL
KSGTASVVCLLNNFYPREAKVQWKVDNALQSGNSQESVTEQDSKDSTYSLSSTLTLSKADYE
KHKVYACEVTHQGLSSPVTKSFNRGECTVAAPSGSGVQLLESGGGLVQPGGSLRLSCAASGF
TFAWYDMGWVRQAPGKGLEWVSSIDWHGEVTYYADSVKGRFTISRDNSKNTLYLQMNSLRAE
DTAVYYCATAEDEPGYDYWGQGTLVTVSS

**SEQ ID NO: 120 = Protein sequence of anti-CD20 mAb VH-human IgG1 heavy chain**

QVQLQQPGAELVKPGASVKMSCKASGYTFTSYNMHWVKQTPGRGLEWIGAIYPGNGDTSYNQ
KFKGKATLTADKSSSTAYMQLSSLTSEDSAVYYCARSTYYGGDWYFNVWGAGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP

PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK

**SEQ ID NO: 121 = Protein sequence of anti-CD20 mAb light chain with GS linker and DOM10-53-474 domain antibody fused at C-terminus**

QIVLSQSPAILSASPGEKVTMTCRASSSVSYIHWFQQKPGSSPKPWIYATSNLASGVPVRFS
GSGSGTSYSLTISRVEAEDAATYYCQQWTSNPPTFGGGTKLEIKRTVAAPSVFIFPPSDEQL
KSGTASVVCLLNNFYPREAKVQWKVDNALQSGNSQESVTEQDSKDSTYSLSSTLTLSKADYE
KHKVYACEVTHQGLSSPVTKSFNRGECGSGVQLLESGGGLVQPGGSLRLSCAASGFTFAWYD
MGWVRQAPGKGLEWVSSIDWHGEVTYYADSVKGRFTISRDNSKNTLYLQMNSLRAEDTAVYY
CATAEDEPGYDYWGQGTLVTVSS

**SEQ ID NO: 122: 586H-TVAAPS-154 Heavy chain**

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCC
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC
GTGATGCACGAGGCCCTGCACAATCACTACACCCAGAAGAGCCTGAGCCTGTCCCCTGGCAA
GACCGTGGCCGCCCCCTCGGGATCCGACATCCAGATGACCCAGAGCCCCAGCAGCCTGAGCG
CCAGCGTGGGCGACAGGGTGACCATTACCTGCAGGGCCAGCAGGCCCATCAGCGACTGGCTG
CACTGGTACCAACAGAAGCCCGGCAAGGCTCCCAAGCTGCTGATCGCCTGGGCCAGCAGCCT
GCAGGGAGGCGTGCCCAGCAGGTTTAGCGGCAGCGGCAGCGGCACCGACTTCACCCTCACCA
TCTCTTCCCTGCAGCCCGAGGACTTCGCCACCTACTACTGCCTGCAGGAGGGCTGGGGGCCC
CCTACTTTCGGCCAGGGCACCAAGGTGGAGATCAAGAGG

**SEQ ID NO: 123**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKGSDGGIRRSMSGTWYLKAMTVDREFPEMNLESVTPMTLTLLK

GHNLEAKVTMLISGRCQEVKAVLGRTKERKKYTADGGKHVAYIIPSAVRDHVIFYSEGQLHG
KPVRGVKLVGRDPKNNLEALEDFEKAAGARGLSTESILIPRQSETCSPG

**SEQ ID NO: 124**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKGSEVVAATPTSLLISWRHPHFPTRYYRITYGETGGNSPVQEFT
VPLQPPTATISGLKPGVDYTITVYAVTDGRNGRLLSIPISINYRT

**SEQ ID NO:125**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKTVAAPSGSDGGGIRRSMSGTWYLKAMTVDREFPEMNLESVTPM
TLTLLKGHNLEAKVTMLISGRCQEVKAVLGRTKERKKYTADGGKHVAYIIPSAVRDHVIFYS
EGQLHGKPVRGVKLVGRDPKNNLEALEDFEKAAGARGLSTESILIPRQSETCSPG

**SEQ ID NO: 126**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKGSVDNKFNKELRQAYWEIQALPNLNWTQSRAFIRSLYDDPSQS
ANLLAEAKKLNDAQAPK

**SEQ ID NO: 127**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKTVAAPSGSVDNKFNKELRQAYWEIQALPNLNWTQSRAFIRSLY
DDPSQSANLLAEAKKLNDAQAPK

**SEQ ID NO: 128**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT

CLVKGFYPSDIAVEWESNGQPENNYKTTPPVLDSDGSFFLYSKLTVDKSRWQQGNVFSCSVM
HEALHNHYTQKSLSLSPGKGSDLGKKLLEAARAGQDDEVRILMANGADVNAKDEYGLTPLYL
ATAHGHLEIVEVLLKNGADVNAVDAIGFTPLHLAAFIGHLEIAEVLLKHGADVNAQDKFGKT
AFDISIGNGNEDLAEILQKL

**SEQ ID NO: 129**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKTVAAPSGSDLGKKLLEAARAGQDDEVRILMANGADVNAKDEYG
LTPLYLATAHGHLEIVEVLLKNGADVNAVDAIGFTPLHLAAFIGHLEIAEVLLKHGADVNAQ
DKFGKTAFDISIGNGNEDLAEILQKL

**SEQ ID NO: 130**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSQVQLVESGGGLVQAGGSLRLSCAASGYAYTYIYMGWFRQAPGK
EREGVAAMDSGGGGTLYADSVKGRFTISRDKGKNTVYLQMDSLKPEDTATYYCAAGGYELRD
RTYGQWGQGTQVTVSS

**SEQ ID NO: 131**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSARVDQTPRSVTKETGESLTINCVLRDASYALGSTCWYRKKSGE
GNEESISKGGRYVETVNSGSKSFSLRINDLTVEDGGTYRCGLGVAGGYCDYALCSSRYAECG
DGTAVTVN

**SEQ ID NO: 132: Anti IL-4 Heavy Chain-TVAAPSGS- anti TNF-a adnectin**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA

TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGACCGTGGCCGC
CCCCTCGGGATCCGTGAGCGACGTGCCAAGGGACCTCGAGGTGGTGGCAGCCACTCCCACCT
CTCTGCTGATCAGCTGGGACACACACAACGCCTACAACGGCTACTACAGGATCACCTACGGA
GAGACCGGCGGCAATAGCCCCGTGAGGGAGTTCACCGTGCCCCACCCCGAGGTGACCGCCAC
CATTAGCGGCCTGAAGCCCGGCGTGGACGATACCATCACCGTCTACGCCGTGACCAACCACC
ACATGCCCCTGAGGATCTTCGGCCCCATCAGCATCAACCATAGGACC

**SEQ ID NO: 133**

QVQLVQSGAEVKKPGASVKVSCKASGYTFTDYYMNWVRQAPGQGLEWMGNINPNNGGTNYNQ
KFKDRVTMTTDTSTSTAYMELRSLRSDDTAVYYCARWILYYGRSKWYFDVWGRGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKTVAAPSGSEVVAATPTSLLISWRHPHFPTRYYRITYGETGGNS
PVQEFTVPLQPPTATISGLKPGVDYTITVYAVTDGRNGRLLSIPISINYRT

**SEQ ID NO: 134**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKGSVSDVPRDLEVVAATPTSLLISWDTHNAYNGYYRITYGET
GGNSPVREFTVPHPEVTATISGLKPGVDDTITVYAVTNHHMPLRIFGPISINHRT

**SEQ ID NO: 135**

QVQLVQSGAEVKKPGSSVKVSCKASGFYIKDTYMHWVRQAPGQGLEWMGTIDPANGNTKYVP
KFQGRVTITADESTSTAYMELSSLRSEDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGKTVAAPSGSVSDVPRDLEVVAATPTSLLISWDTHNAYNGYYR
ITYGETGGNSPVREFTVPHPEVTATISGLKPGVDDTITVYAVTNHHMPLRIFGPISINHRT

**SEQ ID NO: 136**

QVQLKQSGPGLVQPSQSLSITCTVSGFSLTNYGVHWVRQSPGKGLEWLGVIWSGGNTDYNTP
FTSRLSINKDNSKSQVFFKMNSLQSNDTAIYYCARALTYYDYEFAYWGQGTLVTVSAASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV

LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGKRSEVVAATPTSLLISWRHPHFPTRYYRITYGETGGNSPVQEFTVPLQ
PPTATISGLKPGVDYTITVYAVTDGRNGRLLSIPISINYRT

**SEQ ID NO: 137**

DILLTQSPVILSVSPGERVSFSCRASQSIGTNIHWYQQRTNGSPRLLIKYASESISGIPSRF
SGSGSGTDFTLSINSVESEDIADYYCQQNNNWPTTFGAGTKLELKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

**SEQ ID NO: 138**

DILLTQSPVILSVSPGERVSFSCRASQSIGTNIHWYQQRTNGSPRLLIKYASESISGIPSRF
SGSGSGTDFTLSINSVESEDIADYYCQQNNNWPTTFGAGTKLELKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGECRSEVVAATPTSLLISWRHPHFPTRYYRITYGETG
GNSPVQEFTVPLQPPTATISGLKPGVDYTITVYAVTDGRNGRLLSIPISINYRT

**SEQ ID NO: 139**

QVQLKQSGPGLVQPSQSLSITCTVSGFSLTNYGVHWVRQSPGKGLEWLGVIWSGGNTDYNTP
FTSRLSINKDNSKSQVFFKMNSLQSNDTAIYYCARALTYYDYEFAYWGQGTLVTVSAASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK

**SEQ ID NO: 140**

QVQLQESGPGLVKPSQTLSLTCTVSGGSISSGDYYWSWIRQPPGKGLEWIGYIYYSGSTDYN
PSLKSRVTMSVDTSKNQFSLKVNSVTAADTAVYYCARVSIFGVGTFDYWGQGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSEVVAATPTSLLISWRHPHFPTRYYRITYGETGGNSPVQEFTVP
LQPPTATISGLKPGVDYTITVYAVTDGRNGRLLSIPISINYRT

**SEQ ID NO: 141**

EIVMTQSPATLSLSPGERATLSCRASQSVSSYLAWYQQKPGQAPRLLIYDASNRATGIPARF
SGSGSGTDFTLTISSLEPEDFAVYYCHQYGSTPLTFGGGTKAEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

**SEQ ID NO: 142**

EIVMTQSPATLSLSPGERATLSCRASQSVSSYLAWYQQKPGQAPRLLIYDASNRATGIPARF
SGSGSGTDFTLTISSLEPEDFAVYYCHQYGSTPLTFGGGTKAEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGECGSEVVAATPTSLLISWRHPHFPTRYYRITYGETG
GNSPVQEFTVPLQPPTATISGLKPGVDYTITVYAVTDGRNGRLLSIPISINYRT

**SEQ ID NO: 143**

QVQLQESGPGLVKPSQTLSLTCTVSGGSISSGDYYWSWIRQPPGKGLEWIGYIYYSGSTDYN
PSLKSRVTMSVDTSKNQFSLKVNSVTAADTAVYYCARVSIFGVGTFDYWGQGTLVTVSSAST
KGPSVFPLAPSSKSTSGGTAALGCLVKDYFPEPVTVSWNSGALTSGVHTFPAVLQSSGLYSL

SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK

**SEQ ID NO: 144**

EVVAATPTSLLISWRHPHFPTRYYRITYGETGGNSPVQEFTVPLQPPTATISGLKPGVDYTI
TVYAVTDGRNGRLLSIPISINYRTGSTGQVQLKQSGPGLVQPSQSLSITCTVSGFSLTNYGV
HWVRQSPGKGLEWLGVIWSGGNTDYNTPFTSRLSINKDNSKSQVFFKMNSLQSNDTAIYYCA
RALTYYDYEFAYWGQGTLVTVSAASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK

**SEQ ID NO: 145**

EVVAATPTSLLISWRHPHFPTRYYRITYGETGGNSPVQEFTVPLQPPTATISGLKPGVDYTI
TVYAVTDGRNGRLLSIPISINYRTSTGDILLTQSPVILSVSPGERVSFSCRASQSIGTNIHW
YQQRTNGSPRLLIKYASESISGIPSRFSGSGSGTDFTLSINSVESEDIADYYCQQNNNWPTT
FGAGTKLELKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGEC

**SEQ ID NO: 146**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGSVSDVPRDLEVVAATPTSLLISWDTHNAYNGYYRITYGETGGNS
PVREFTVPHPEVTATISGLKPGVDDTITVYAVTNHHMPLRIFGPISINHRT

**SEQ ID NO: 147**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKTVAAPSGSVSDVPRDLEVVAATPTSLLISWDTHNAYNGYYRITYG
ETGGNSPVREFTVPHPEVTATISGLKPGVDDTITVYAVTNHHMPLRIFGPISINHRT

**SEQ ID NO: 148 = DOM10-53-616 (dAb)**

GVQLLESGGGLVQPGGSLRLSCAASGFVFPWYDMGWVRQAPGKGLEWVSSIDWHGKITYYAD
SVKGRFTISRDNSKNTLYLQMNSLRAEDTAVYYCATAEDEPGYDYWGQGTLVTVSS

**SEQ ID NO: 149 = PascoH-616 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL

SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGVQLLESGGGLVQPGGSLRLSCAASGFVFPWYDMGWVRQAPGKGL
EWVSSIDWHGKITYYADSVKGRFTISRDNSKNTLYLQMNSLRAEDTAVYYCATAEDEPGYDY
WGQGTLVTVSS

**SEQ ID NO: 150 = PascoH-TVAAPS-616 (H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**TVAAPS**GVQLLESGGGLVQPGGSLRLSCAASGFVFPWYDMGWVRQ
APGKGLEWVSSIDWHGKITYYADSVKGRFTISRDNSKNTLYLQMNSLRAEDTAVYYCATAED
EPGYDYWGQGTLVTVSS

**SEQ ID NO: 151 = C1-TVAAPSGS-210 (H chain)**

QVQLVQSGAEVKKPGASVKVSCKASGFYIKDTYMHWVRQAPGQGLEWMGTIDPANGNTKYVP
KFQGRVTMTTDTSTSTAYMELRSLRSDDTAVYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSEVQLLESGGGLVQPGGSLRLSCAASGFTFRNFG
MGWVRQAPGKGLEWVSWIISSGTETYYADSVKGRFTISRDNSKNTLYLQMNSLRAEDTAVYY
CAKSLGRFDYWGQGTLVTVSS

**SEQ ID NO: 152 = D1-TVAAPSGS-210 (H chain)**

EVQLVQSGAEVKKPGESLKISCKGSGFYIKDTYMHWVRQMPGKGLEWMGTIDPANGNTKYVP
KFQGQVTISADKSISTAYLQWSSLKASDTAMYYCARSIYDDYHYDDYYAMDYWGQGTLVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCS
VMHEALHNHYTQKSLSLSPGK**TVAAPS**GSEVQLLESGGGLVQPGGSLRLSCAASGFTFRNFG

MGWVRQAPGKGLEWVSWIISSGTETYYADSVKGRFTISRDNSKNTLYLQMNSLRAEDTAVYY
CAKSLGRFDYWGQGTLVTVSS

**SEQ ID NO: 153 = N0 (L chain)**

EIVLTQSPATLSLSPGERATLSCRSSQNIVHINGNTYLEWYQQKPGQAPRLLIYKISDRFSG
IPARFSGSGSGTDFTLTISSLEPEDFAVYYCFQGSHVPWTFGGGTKVEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**SEQ ID NO: 154 = M0 (L chain)**

EIVLTQSPATLSLSPGERATLSCRSSQNIVHINGNTYLEWYQQKPGQAPRLLIYKISDRFSG
IPARFSGSGSGTDFTLTISSLEPEDFAVYYCFQGSHVPWTFGGGTKVEIKRTVAAPSVFIFP
PSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

**SEQ ID NO: 155 = 656H-TVAAPS-210_(H chain)**

QVQLVQSGAEVKKPGASVKVSCKASGYTFIDYEIHWVRQAPGQGLEWMGAIDPETGGTAYNQ
KFKGRVTMTTDTSTSTAYMELRSLRSDDTAVYYCTRILLYYYPMDYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK**TVAAPS**EVQLLESGGGLVQPGGSLRLSCAASGFTFRNFGMGWVRQAP
GKGLEWVSWIISSGTETYYADSVKGRFTISRDNSKNTLYLQMNSLRAEDTAVYYCAKSLGRF
DYWGQGTLVTVSS

**SEQ ID NO: 156 = 656_(L chain)**

EIVLTQSPATLSLSPGERATLSCRASQNISDYLHWYQQKPGQAPRLLIYYASQSISGIPARF
SGSGSGTDFTLTISSLEPEDFAVYYCQNGHSFPLTFGGGTKVEIKRTVAAPSVFIFPPSDEQ
LKSGTASVVCLLNNFYPREAKVQWKVDNALQSGNSQESVTEQDSKDSTYSLSSTLTLSKADY
EKHKVYACEVTHQGLSSPVTKSFNRGEC

**SEQ ID NO: 157 = PascoH-TVAAPS-546_(H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL

TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**TVAAPS**GVQLLESGGGLVQPGGSLRLSCAASGFVFPWYDMGWVRQ
APGKGLEWVSSIDWKGGKTYYADSVKGRFTISRDNSKNTLYLQMNSLRAEDTAVYYCATAED
EPGYDYWGQGTLVTVSS

**SEQ ID NO: 158 = PascoH-546_(H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGVQLLESGGGLVQPGGSLRLSCAASGFVFPWYDMGWVRQAPGKGL
EWVSSIDWKGGKTYYADSVKGRFTISRDNSKNTLYLQMNSLRAEDTAVYYCATAEDEPGYDY
WGQGTLVTVSS

**SEQ ID NO: 159 = PascoH-TVAAPS-567_(H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGK**TVAAPS**GVQLLESGGGLVQPGGSLRLSCAASGFVFAWYDMGWVRQ
APGKGLEWVSSIDWHGEVTYYADSVKGRFTISRDNSKNTLYLQMNSLRAEDTAVYYCATAED
EPGYDYWGQGTLVTVSS

**SEQ ID NO: 160 = PascoH-567_(H chain)**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKGVQLLESGGGLVQPGGSLRLSCAASGFVFAWYDMGWVRQAPGKGL
EWVSSIDWHGEVTYYADSVKGRFTISRDNSKNTLYLQMNSLRAEDTAVYYCATAEDEPGYDY
WGQGTLVTVSS

**SEQ ID NO: 161 = 656_(H chain)**

QVQLVQSGAEVKKPGASVKVSCKASGYTFIDYEIHWVRQAPGQGLEWMGAIDPETGGTAYNQ
KFKGRVTMTTDTSTSTAYMELRSLRSDDTAVYYCTRILLYYYPMDYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGK

**SEQ ID NO: 162**

GAGGTGCAGCTGTTGGAGTCTGGGGGAGGCTTGGTACAGCCTGGGGGGTCCCTGCGTCTCTC
CTGTGCAGCCTCCGGATTCACCTTTGGGGCTTATCCGATGATGTGGGTCCGCCAGGCTCCAG
GGAAGGGTCTAGAGTGGGTCTCAGAGATTTCGCCTTCGGGTTCTTATACATACTACGCAGAC
TCCGTGAAGGGCCGGTTCACCATCTCCCGCGACAATTCCAAGAACACGCTGTATCTGCAAAT
GAACAGCCTGCGTGCCGAGGACACCGCGGTATATTACTGTGCGAAAGATCCTCGGAAGTTTG
ACTACTGGGGTCAGGGAACCCTGGTCACCGTCTCGAGCGCTAGCACCAAGGGCCCCAGCGAG
GTGCAGCTGTTGGAGTCTGGGGGAGGCTTGGTACAGCCTGGGGGGTCCCTGCGTCTCTCCTG
TGCAGCCTCCGGATTCACCTTTAGCAGCTATGCCATGAGCTGGGTCCGCCAGGCTCCAGGGA
AGGGTCTAGAGTGGGTCTCAGCTATTAGTGGTAGTGGTGGTAGCACATACTACGCAGACTCC
GTGAAGGGCCGGTTCACCATCTCCCGCGACAATTCCAAGAACACGCTGTATCTGCAAATGAA
CAGCCTGCGTGCCGAGGACACCGCGGTATATTACTGTGCGAAAAGTTATGGTGCTTTTGACT
ACTGGGGCCAGGGAACCCTGGTCACCGTCTCGAGCGCTAGCACCAAGGGCCCCAGCGTGTTC
CCCCTGGCCCCCAGCAGCAAGAGCACCAGCGGCGGCACAGCCGCCCTGGGCTGCCTGGTGAA
GGACTACTTCCCCGAGCCTGTGACCGTGTCCTGGAATAGCGGAGCCCTGACCTCCGGCGTGC
ACACCTTCCCCGCCGTGCTGCAGAGCAGCGGCCTGTACTCCCTGAGCAGCGTGGTGACCGTG
CCCAGCAGCAGCCTGGGCACCCAGACCTACATCTGCAACGTGAACCACAAGCCCAGCAACAC
CAAAGTGGACAAGAAAGTGGAGCCCAAGAGCTGCGATAAGACCCACACCTGCCCCCCCTGCC
CTGCCCCCGAGCTGCTGGGCGGACCTAGCGTGTTCCTGTTCCCCCCCAAGCCTAAGGACACC
CTGATGATCAGCAGGACCCCCGAAGTGACCTGCGTGGTGGTGGATGTGAGCCACGAGGACCC
TGAAGTGAAGTTCAACTGGTACGTGGACGGCGTGGAAGTGCACAACGCCAAGACCAAGCCCA
GAGAGGAGCAGTACAACAGCACCTACCGCGTGGTGTCTGTGCTGACCGTGCTGCACCAGGAT
TGGCTGAACGGCAAGGAGTACAAGTGCAAAGTGAGCAACAAGGCCCTGCCTGCCCCTATCGA
GAAAACCATCAGCAAGGCCAAGGGCCAGCCTAGAGAGCCCCAGGTCTACACCCTGCCTCCCT
CCAGAGATGAGCTGACCAAGAACCAGGTGTCCCTGACCTGTCTGGTGAAGGGCTTCTACCCC
AGCGACATCGCCGTGGAGTGGGAGAGCAACGGCCAGCCCGAGAACAACTACAAGACCACCCC
CCCTGTGCTGGACAGCGATGGCAGCTTCTTCCTGTACTCCAAGCTGACCGTGGACAAGAGCA
GATGGCAGCAGGGCAACGTGTTCAGCTGCAGCGTGATGCACGAGGCCCTGCACAATCACTAC
ACCCAGAAGAGTCTGAGCCTGTCCCCTGGCAAG

**SEQ ID NO: 163**

EVQLLESGGGLVQPGGSLRLSCAASGFTFGAYPMMWVRQAPGKGLEWVSEISPSGSYTYYAD
SVKGRFTISRDNSKNTLYLQMNSLRAEDTAVYYCAKDPRKFDYWGQGTLVTVSSASTKGPSE
VQLLESGGGLVQPGGSLRLSCAASGFTFSSYAMSWVRQAPGKGLEWVSAISGSGGSTYYADS

VKGRFTISRDNSKNTLYLQMNSLRAEDTAVYYCAKSYGAFDYWGQGTLVTVSSASTKGPSVF
PLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHY
TQKSLSLSPGK

**SEQ ID NO: 164**

CAGGTGCAGCTGAAGCAGAGCGGCCCTGGCCTGGTGCAGCCCTCTCAGAGCCTGAGCATCACCTGTACCGTGAGCG
GCTTCAGCCTGACCAATTACGGCGTGCATTGGGTGCGGCAGTCTCCAGGCAAGGGCCTGGAATGGCTGGGAGTGAT
CTGGTCCGGCGGCAACACCGACTACAACACCCCCTTCACCAGCAGACTGAGCATCAACAAGGACAACAGCAAGAGC
CAGGTGTTCTTCAAGATGAACAGCCTGCAGAGCAACGACACCGCCATCTACTATTGTGCCAGGGCCCTGACCTACT
ACGACTACGAGTTCGCCTACTGGGGCCAGGGCACCCTGGTGACCGTGAGCGCCGCTAGCACCAAGGGCCCCAGCGT
GTTCCCCCTGGCCCCCAGCAGCAAGAGCACCAGCGGCGGCACAGCCGCCCTGGGCTGCCTGGTGAAGGACTACTTC
CCCGAGCCTGTGACCGTGTCCTGGAATAGCGGAGCCCTGACCTCCGGCGTGCACACCTTCCCCGCCGTGCTGCAGA
GCAGCGGCCTGTACTCCCTGAGCAGCGTGGTGACCGTGCCCAGCAGCAGCCTGGGCACCCAGACCTACATCTGCAA
CGTGAACCACAAGCCCAGCAACACCAAAGTGGACAAGAAAGTGGAGCCCAAGAGCTGCGATAAGACCCACACCTGC
CCCCCCTGCCCTGCCCCCGAGCTGCTGGGCGGACCTAGCGTGTTCCTGTTCCCCCCCAAGCCTAAGGACACCCTGA
TGATCAGCAGGACCCCCGAAGTGACCTGCGTGGTGGTGGATGTGAGCCACGAGGACCCTGAAGTGAAGTTCAACTG
GTACGTGGACGGCGTGGAAGTGCACAACGCCAAGACCAAGCCCAGAGAGGAGCAGTACAACAGCACCTACCGCGTG
GTGTCTGTGCTGACCGTGCTGCACCAGGATTGGCTGAACGGCAAGGAGTACAAGTGCAAAGTGAGCAACAAGGCCC
TGCCTGCCCCTATCGAGAAAACCATCAGCAAGGCCAAGGGCCAGCCTAGAGAGCCCCAGGTCTACACCCTGCCTCC
CTCCAGAGATGAGCTGACCAAGAACCAGGTGTCCCTGACCTGTCTGGTGAAGGGCTTCTACCCCAGCGACATCGCC
GTGGAGTGGGAGAGCAACGGCCAGCCCGAGAACAACTACAAGACCACCCCCCCTGTGCTGGACAGCGATGGCAGCT
TCTTCCTGTACTCCAAGCTGACCGTGGACAAGAGCAGATGGCAGCAGGGCAACGTGTTCAGCTGCAGCGTGATGCA
CGAGGCCCTGCACAATCACTACACCCAGAAGAGTCTGAGCCTGTCCCCTGGCAAGTCGACCGGTGAGGTGCAGCTG
TTGGTGTCTGGGGGAGGCTTGGTACAGCCTGGGGGGTCCCTGCGTCTCTCCTGTGCAGCCTCCGGATTCACCTTTA
AGGCTTATCCGATGATGTGGGTCCGCCAGGCTCCAGGGAAGGGTCTAGAGTGGGTTTCAGAGATTTCGCCTTCGGG
TTCTTATACATACTACGCAGACTCCGTGAAGGGCCGGTTCACCATCTCCCGCGACAATTCCAAGAACACGCTGTAT
CTGCAAATGAACAGCCTGCGTGCCGAGGACACCGCGGTATATTACTGTGCGAAAGATCCTCGGAAGTTAGACTACT
GGGGTCAGGGAACCCTGGTCACCGTCTCGAGC

**SEQ ID NO: 165:**

QVQLKQSGPGLVQPSQSLSITCTVSGFSLTNYGVHWVRQSPGKGLEWLGVIWSGGNTDYNTPFTSRLSINKD
NSKSQVFFKMNSLQSNDTAIYYCARALTYYDYEFAYWGQGTLVTVSAASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHE
ALHNHYTQKSLSLSPGKSTGEVQLLVSGGGLVQPGGSLRLSCAASGFTFKAYPMMWVRQAPGKGLEWVSEIS
PSGSYTYYADSVKGRFTISRDNSKNTLYLQMNSLRAEDTAVYYCAKDPRKLDYWGQGTLVTVSS

**SEQ ID NO: 166**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTPVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVSLTCL

VKGFYPSDIAVEWESNGQPENNYKTTPPVLDSDGSFFLYSKLTVDKSRWQQGNVFSCSVMHE
ALHNHYTQKSLSLSPGK

**SEQ ID NO: 167** 586H-TVAAPS-210 Heavy chain

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCC
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC
GTGATGCACGAGGCCCTGCACAATCACTACACCCAGAAGAGCCTGAGCCTGTCCCCTGGCAA
GACCGTGGCCGCCCCCTCGGGATCCGAAGTGCAGCTCCTGGAGAGCGGCGGCGGCCTGGTGC
AGCCCGGCGGCAGCCTGAGGCTGAGCTGCGCCGCTAGCGGCTTCACCTTCAGGAACTTCGGC
ATGGGCTGGGTCAGGCAGGCCCCCGGCAAGGGCCTGGAGTGGGTCAGCTGGATCATCAGCTC
CGGCACCGAGACCTACTACGCCGACAGCGTGAAGGGCAGGTTCACCATCAGCCGCGACAACA
GCAAGAACACCCTGTACCTGCAGATGAACAGCCTGAGGGCCGAGGACACCGCCGTCTACTAC
TGCGCCAAGAGCCTGGGCAGGTTCGACTACTGGGGACAGGGGACCCTGGTGACTGTGAGCAG
C

**SEQ ID NO: 168**

GAGGTGCAGCTGGTGGAGTCTGGCGGCGGACTGGTGCAGCCCGGCAGAAGCCTGAGACTGAG
CTGTGCCGCCAGCGGCTTCACCTTCGACGACTACGCCATGCACTGGGTGAGGCAGGCCCCTG
GCAAGGGCCTGGAGTGGGTGTCCGCCATCACCTGGAATAGCGGCCACATCGACTACGCCGAC
AGCGTGGAGGGCAGATTCACCATCAGCCGGGACAACGCCAAGAACAGCCTGTACCTGCAGAT
GAACAGCCTGAGAGCCGAGGACACCGCCGTGTACTACTGTGCCAAGGTGTCCTACCTGAGCA
CCGCCAGCAGCCTGGACTACTGGGGCCAGGGCACCCTGGTGACAGTCTCGAGCGCTAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAG
CCCTGACCTCCGGCGTGCACACCTTCCCCGCCGTGCTGCAGAGCAGCGGCCTGTACTCCCTG
AGCAGCGTGGTGACCGTGCCCAGCAGCAGCCTGGGCACCCAGACCTACATCTGCAACGTGAA
CCACAAGCCCAGCAACACCAAAGTGGACAAGAAAGTGGAGCCCAAGAGCTGCGATAAGACCC
ACACCTGCCCCCCCTGCCCTGCCCCCGAGCTGCTGGGCGGACCTAGCGTGTTCCTGTTCCCC
CCCAAGCCTAAGGACACCCTGATGATCAGCAGGACCCCCGAAGTGACCTGCGTGGTGGTGGA
TGTGAGCCACGAGGACCCTGAAGTGAAGTTCAACTGGTACGTGGACGGCGTGGAAGTGCACA
ACGCCAAGACCAAGCCCAGAGAGGAGCAGTACAACAGCACCTACCGCGTGGTGTCTGTGCTG
ACCGTGCTGCACCAGGATTGGCTGAACGGCAAGGAGTACAAGTGCAAAGTGAGCAACAAGGC

CCTGCCTGCCCCTATCGAGAAAACCATCAGCAAGGCCAAGGGCCAGCCTAGAGAGCCCCAGG
TCTACACCCTGCCTCCCTCCAGAGATGAGCTGACCAAGAACCAGGTGTCCCTGACCTGTCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACTCCAAGC
TGACCGTGGACAAGAGCAGATGGCAGCAGGGCAACGTGTTCAGCTGCAGCGTGATGCACGAG
GCCCTGCACAATCACTACACCCAGAAGAGTCTGAGCCTGTCCCCTGGCAAGTCGACCGGTGA
GGTGCAGCTGTTGGTGTCTGGGGGAGGCTTGGTACAGCCTGGGGGGTCCCTGCGTCTCTCCT
GTGCAGCCTCCGGATTCACCTTTAAGGCTTATCCGATGATGTGGGTCCGCCAGGCTCCAGGG
AAGGGTCTAGAGTGGGTTTCAGAGATTTCGCCTTCGGGTTCTTATACATACTACGCAGACTC
CGTGAAGGGCCGGTTCACCATCTCCCGCGACAATTCCAAGAACACGCTGTATCTGCAAATGA
ACAGCCTGCGTGCCGAGGACACCGCGGTATATTACTGTGCGAAAGATCCTCGGAAGTTAGAC
TACTGGGGTCAGGGAACCCTGGTCACCGTCTCGAGC

**SEQ ID NO: 169**

GATATCCAGATGACCCAGAGCCCCAGCAGCCTGAGCGCCTCTGTGGGCGATAGAGTGACCATCACCTGCCGGGCCA
GCCAGGGCATCAGAAACTACCTGGCCTGGTATCAGCAGAAGCCTGGCAAGGCCCCTAAGCTGCTGATCTACGCCGC
CAGCACCCTGCAGAGCGGCGTGCCCAGCAGATTCAGCGGCAGCGGCTCCGGCACCGACTTCACCCTGACCATCAGC
AGCCTGCAGCCCGAGGACGTGGCCACCTACTACTGCCAGCGGTACAACAGAGCCCCTTACACCTTCGGCCAGGGCA
CCAAGGTGGAGATCAAGCGTACGGTGGCCGCCCCCAGCGTGTTCATCTTCCCCCCCAGCGATGAGCAGCTCAAGAG
CGGCACCGCCAGCGTGGTGTGTCTGCTGAACAACTTCTACCCCCGGGAGGCCAAAGTGCAGTGGAAAGTGGACAAC
GCCCTGCAGAGCGGCAACAGCCAGGAGAGCGTGACCGAGCAGGACAGCAAGGACTCCACCTACAGCCTGAGCAGCA
CCCTGACCCTGAGCAAGGCCGACTACGAGAAGCACAAAGTGTACGCCTGCGAAGTGACCCACCAGGGCCTGTCCAG
CCCCGTGACCAAGAGCTTCAACCGGGGCGAGTGC

**SEQ ID NO: 170**

GAGGTGCAGCTGGTGGAGTCTGGCGGCGGACTGGTGCAGCCCGGCAGAAGCCTGAGACTGAG
CTGTGCCGCCAGCGGCTTCACCTTCGACGACTACGCCATGCACTGGGTGAGGCAGGCCCCTG
GCAAGGGCCTGGAGTGGGTGTCCGCCATCACCTGGAATAGCGGCCACATCGACTACGCCGAC
AGCGTGGAGGGCAGATTCACCATCAGCCGGGACAACGCCAAGAACAGCCTGTACCTGCAGAT
GAACAGCCTGAGAGCCGAGGACACCGCCGTGTACTACTGTGCCAAGGTGTCCTACCTGAGCA
CCGCCAGCAGCCTGGACTACTGGGGCCAGGGCACCCTGGTGACAGTCTCGAGCGCTAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAGCCTGTGACCGTGTCCTGGAATAGCGGAG
CCCTGACCTCCGGCGTGCACACCTTCCCCGCCGTGCTGCAGAGCAGCGGCCTGTACTCCCTG
AGCAGCGTGGTGACCGTGCCCAGCAGCAGCCTGGGCACCCAGACCTACATCTGCAACGTGAA
CCACAAGCCCAGCAACACCAAAGTGGACAAGAAAGTGGAGCCCAAGAGCTGCGATAAGACCC
ACACCTGCCCCCCCTGCCCTGCCCCCGAGCTGCTGGGCGGACCTAGCGTGTTCCTGTTCCCC
CCCAAGCCTAAGGACACCCTGATGATCAGCAGGACCCCCGAAGTGACCTGCGTGGTGGTGGA
TGTGAGCCACGAGGACCCTGAAGTGAAGTTCAACTGGTACGTGGACGGCGTGGAAGTGCACA
ACGCCAAGACCAAGCCCAGAGAGGAGCAGTACAACAGCACCTACCGCGTGGTGTCTGTGCTG
ACCGTGCTGCACCAGGATTGGCTGAACGGCAAGGAGTACAAGTGCAAAGTGAGCAACAAGGC
CCTGCCTGCCCCTATCGAGAAAACCATCAGCAAGGCCAAGGGCCAGCCTAGAGAGCCCCAGG
TCTACACCCTGCCTCCCTCCAGAGATGAGCTGACCAAGAACCAGGTGTCCCTGACCTGTCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACTCCAAGC
TGACCGTGGACAAGAGCAGATGGCAGCAGGGCAACGTGTTCAGCTGCAGCGTGATGCACGAG
GCCCTGCACAATCACTACACCCAGAAGAGTCTGAGCCTGTCCCCTGGCAAGTCGACCGGTGA
CATCCAGATGACCCAGAGCCCTTCAAGCCTGAGCGCCAGCGTGGGCGACAGAGTGACCATCA
CCTGCCGGGCCAGCCAGTGGATCGGCAACCTGCTGGACTGGTATCAGCAGAAGCCCGGCAAG
GCCCCCAAGCTGCTGATCTACTACGCCAGCTTCCTGCAGAGCGGCGTGCCCAGCCGGTTTAG
CGGCAGCGGCTACGGCACCGACTTCACCCTGACCATCAGCAGCCTGCAGCCCGAGGACTTCG

CCACCTACTACTGCCAGCAGGCCAACCCTGCCCCCCTGACCTTCGGCCAGGGTACCAAGGTG
GAAATCAAACGG

**SEQ ID NO: 171**

GACATCCAGATGACCCAGTCTCCATCCTCCCTGTCTGCATCTGTAGGAGACCGTGTCACCAT
CACTTGCCGGGCAAGTCAGGATATTTACCTGAATTTAGACTGGTATCAGCAGAAACCAGGGA
AAGCCCCTAAGCTCCTGATCAATTTTGGTTCCGAGTTGCAAAGTGGTGTCCCATCACGTTTC
AGTGGCAGTGGATATGGGACAGATTTCACTCTCACCATCAGCAGTCTGCAACCTGAAGATTT
CGCTACGTACTACTGTCAACCGTCTTTTTACTTCCCTTATACGTTCGGCCAAGGGACCAAGG
TGGAAATCAAACGTACGGTGGCCGCCCCCAGCGACATCCAGATGACCCAGTCTCCATCCTCC
CTGTCTGCATCTGTAGGAGACCGTGTCACCATCACTTGCCGGGCAAGTCAGAGCATTAGCAG
CTATTTAAATTGGTACCAGCAGAAACCAGGGAAAGCCCCTAAGCTCCTGATCTATGCTGCAT
CCAGTTTGCAAAGTGGGGTCCCATCACGTTTCAGTGGCAGTGGATCTGGGACAGATTTCACT
CTCACCATCAGCAGTCTGCAACCTGAAGATTTTGCTACGTACTACTGTCAACAGAGTTACAG
TACCCCTAATACGTTCGGCCAAGGGACCAAGGTGGAAATCAAACGTACGGTGGCCGCCCCCA
GCGTGTTCATCTTCCCCCCCAGCGATGAGCAGCTCAAGAGCGGCACCGCCAGCGTGGTGTGT
CTGCTGAACAACTTCTACCCCCGGGAGGCCAAAGTGCAGTGGAAAGTGGACAACGCCCTGCA
GAGCGGCAACAGCCAGGAGAGCGTGACCGAGCAGGACAGCAAGGACTCCACCTACAGCCTGA
GCAGCACCCTGACCCTGAGCAAGGCCGACTACGAGAAGCACAAAGTGTACGCCTGCGAAGTG
ACCCACCAGGGCCTGTCCAGCCCCGTGACCAAGAGCTTCAACCGGGGCGAGTGC

**SEQ ID NO: 172**

GAGGTGCAGCTGTTGGTGTCTGGGGGAGGCTTGGTACAGCCTGGGGGGTCCCTGCGTCTCTC
CTGTGCAGCCTCCGGATTCACCTTTAAGGCTTATCCGATGATGTGGGTCCGCCAGGCTCCAG
GGAAGGGTCTAGAGTGGGTTTCAGAGATTTCGCCTTCGGGTTCTTATACATACTACGCAGAC
TCCGTGAAGGGCCGGTTCACCATCTCCCGCGACAATTCCAAGAACACGCTGTATCTGCAAAT
GAACAGCCTGCGTGCCGAGGACACCGCGGTATATTACTGTGCGAAAGATCCTCGGAAGTTAG
ACTACTGGGGTCAGGGAACCCTGGTCACCGTCTCGAGCGCTAGCACCAAGGGCCCCAGCGAG
GTGCAGCTGTTGGAGTCTGGGGGAGGCTTGGTACAGCCTGGGGGGTCCCTGCGTCTCTCCTG
TGCAGCCTCCGGATTCACCTTTAGCAGCTATGCCATGAGCTGGGTCCGCCAGGCTCCAGGGA
AGGGTCTAGAGTGGGTCTCAGCTATTAGTGGTAGTGGTGGTAGCACATACTACGCAGACTCC
GTGAAGGGCCGGTTCACCATCTCCCGCGACAATTCCAAGAACACGCTGTATCTGCAAATGAA
CAGCCTGCGTGCCGAGGACACCGCGGTATATTACTGTGCGAAAAGTTATGGTGCTTTTGACT
ACTGGGGCCAGGGAACCCTGGTCACCGTCTCGAGCGCTAGCACCAAGGGCCCCAGCGTGTTC
CCCCTGGCCCCCAGCAGCAAGAGCACCAGCGGCGGCACAGCCGCCCTGGGCTGCCTGGTGAA
GGACTACTTCCCCGAGCCTGTGACCGTGTCCTGGAATAGCGGAGCCCTGACCTCCGGCGTGC
ACACCTTCCCCGCCGTGCTGCAGAGCAGCGGCCTGTACTCCCTGAGCAGCGTGGTGACCGTG
CCCAGCAGCAGCCTGGGCACCCAGACCTACATCTGCAACGTGAACCACAAGCCCAGCAACAC
CAAAGTGGACAAGAAAGTGGAGCCCAAGAGCTGCGATAAGACCCACACCTGCCCCCCCTGCC
CTGCCCCCGAGCTGCTGGGCGGACCTAGCGTGTTCCTGTTCCCCCCCAAGCCTAAGGACACC
CTGATGATCAGCAGGACCCCCGAAGTGACCTGCGTGGTGGTGGATGTGAGCCACGAGGACCC
TGAAGTGAAGTTCAACTGGTACGTGGACGGCGTGGAAGTGCACAACGCCAAGACCAAGCCCA
GAGAGGAGCAGTACAACAGCACCTACCGCGTGGTGTCTGTGCTGACCGTGCTGCACCAGGAT
TGGCTGAACGGCAAGGAGTACAAGTGCAAAGTGAGCAACAAGGCCCTGCCTGCCCCTATCGA
GAAAACCATCAGCAAGGCCAAGGGCCAGCCTAGAGAGCCCCAGGTCTACACCCTGCCTCCCT
CCAGAGATGAGCTGACCAAGAACCAGGTGTCCCTGACCTGTCTGGTGAAGGGCTTCTACCCC
AGCGACATCGCCGTGGAGTGGGAGAGCAACGGCCAGCCCGAGAACAACTACAAGACCACCCC
CCCTGTGCTGGACAGCGATGGCAGCTTCTTCCTGTACTCCAAGCTGACCGTGGACAAGAGCA
GATGGCAGCAGGGCAACGTGTTCAGCTGCAGCGTGATGCACGAGGCCCTGCACAATCACTAC
ACCCAGAAGAGTCTGAGCCTGTCCCCTGGCAAG

**SEQ ID NO: 173**

CAGGTGCAGCTGAAGCAGAGCGGCCCTGGCCTGGTGCAGCCCTCTCAGAGCCTGAGCATCAC
CTGTACCGTGAGCGGCTTCAGCCTGACCAATTACGGCGTGCATTGGGTGCGGCAGTCTCCAG
GCAAGGGCCTGGAATGGCTGGGAGTGATCTGGTCCGGCGGCAACACCGACTACAACACCCCC

TTCACCAGCAGACTGAGCATCAACAAGGACAACAGCAAGAGCCAGGTGTTCTTCAAGATGAA
CAGCCTGCAGAGCAACGACACCGCCATCTACTATTGTGCCAGGGCCCTGACCTACTACGACT
ACGAGTTCGCCTACTGGGGCCAGGGCACCCTGGTGACCGTGAGCGCCGCTAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAGCCTGTGACCGTGTCCTGGAATAGCGGAGCCCTGA
CCTCCGGCGTGCACACCTTCCCCGCCGTGCTGCAGAGCAGCGGCCTGTACTCCCTGAGCAGC
GTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCT
GCCCCCCCTGCCCTGCCCCCGAGCTGCTGGGCGGACCTAGCGTGTTCCTGTTCCCCCCCAAG
CCTAAGGACACCCTGATGATCAGCAGGACCCCCGAAGTGACCTGCGTGGTGGTGGATGTGAG
CCACGAGGACCCTGAAGTGAAGTTCAACTGGTACGTGGACGGCGTGGAAGTGCACAACGCCA
AGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCC
TGCCCCTATCGAGAAAACCATCAGCAAGGCCAAGGGCCAGCCTAGAGAGCCCCAGGTCTACA
CCCTGCCTCCCTCCAGAGATGAGCTGACCAAGAACCAGGTGTCCCTGACCTGTCTGGTGAAG
GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTG
CACAATCACTACACCCAGAAGAGTCTGAGCCTGTCCCCTGGCAAGGAGGTGCAGCTGTTGGT
GTCTGGGGGAGGCTTGGTACAGCCTGGGGGGTCCCTGCGTCTCTCCTGTGCAGCCTCCGGAT
TCACCTTTAAGGCTTATCCGATGATGTGGGTCCGCCAGGCTCCAGGGAAGGGTCTAGAGTGG
GTTTCAGAGATTTCGCCTTCGGGTTCTTATACATACTACGCAGACTCCGTGAAGGGCCGGTT
CACCATCTCCCGCGACAATTCCAAGAACACGCTGTATCTGCAAATGAACAGCCTGCGTGCCG
AGGACACCGCGGTATATTACTGTGCGAAAGATCCTCGGAAGTTAGACTACTGGGGTCAGGGA
ACCCTGGTCACCGTCTCGAGC

**SEQ ID NO: 174**

QVQLKQSGPGLVQPSQSLSITCTVSGFSLTNYGVHWVRQSPGKGLEWLGVIWSGGNTDYNTPFTSRLSINKD
NSKSQVFFKMNSLQSNDTAIYYCARALTYYDYEFAYWGQGTLVTVSAASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHE
ALHNHYTQKSLSLSPGKEVQLLVSGGGLVQPGGSLRLSCAASGFTFKAYPMMWVRQAPGKGLEWVSEISPSG
SYTYYADSVKGRFTISRDNSKNTLYLQMNSLRAEDTAVYYCAKDPRKLDYWGQGTLVTVSS

**SEQ ID NO: 175**

CAGGTGCAGCTGAAGCAGAGCGGCCCTGGCCTGGTGCAGCCCTCTCAGAGCCTGAGCATCACCTGTACCGTGAGCG
GCTTCAGCCTGACCAATTACGGCGTGCATTGGGTGCGGCAGTCTCCAGGCAAGGGCCTGGAATGGCTGGGAGTGAT
CTGGTCCGGCGGCAACACCGACTACAACACCCCCTTCACCAGCAGACTGAGCATCAACAAGGACAACAGCAAGAGC
CAGGTGTTCTTCAAGATGAACAGCCTGCAGAGCAACGACACCGCCATCTACTATTGTGCCAGGGCCCTGACCTACT
ACGACTACGAGTTCGCCTACTGGGGCCAGGGCACCCTGGTGACCGTGAGCGCCGCTAGCACCAAGGGCCCCAGCGT
GTTCCCCCTGGCCCCCAGCAGCAAGAGCACCAGCGGCGGCACAGCCGCCCTGGGCTGCCTGGTGAAGGACTACTTC
CCCGAGCCTGTGACCGTGTCCTGGAATAGCGGAGCCCTGACCTCCGGCGTGCACACCTTCCCCGCCGTGCTGCAGA
GCAGCGGCCTGTACTCCCTGAGCAGCGTGGTGACCGTGCCCAGCAGCAGCCTGGGCACCCAGACCTACATCTGCAA
CGTGAACCACAAGCCCAGCAACACCAAAGTGGACAAGAAAGTGGAGCCCAAGAGCTGCGATAAGACCCACACCTGC
CCCCCCTGCCCTGCCCCCGAGCTGCTGGGCGGACCTAGCGTGTTCCTGTTCCCCCCCAAGCCTAAGGACACCCTGA
TGATCAGCAGGACCCCCGAAGTGACCTGCGTGGTGGTGGATGTGAGCCACGAGGACCCTGAAGTGAAGTTCAACTG
GTACGTGGACGGCGTGGAAGTGCACAACGCCAAGACCAAGCCCAGAGAGGAGCAGTACAACAGCACCTACCGCGTG
GTGTCTGTGCTGACCGTGCTGCACCAGGATTGGCTGAACGGCAAGGAGTACAAGTGCAAAGTGAGCAACAAGGCCC
TGCCTGCCCCTATCGAGAAAACCATCAGCAAGGCCAAGGGCCAGCCTAGAGAGCCCCAGGTCTACACCCTGCCTCC
CTCCAGAGATGAGCTGACCAAGAACCAGGTGTCCCTGACCTGTCTGGTGAAGGGCTTCTACCCCAGCGACATCGCC
GTGGAGTGGGAGAGCAACGGCCAGCCCGAGAACAACTACAAGACCACCCCCCCTGTGCTGGACAGCGATGGCAGCT
TCTTCCTGTACTCCAAGCTGACCGTGGACAAGAGCAGATGGCAGCAGGGCAACGTGTTCAGCTGCAGCGTGATGCA

CGAGGCCCTGCACAATCACTACACCCAGAAGAGTCTGAGCCTGTCCCCTGGCAAGTCGACCGGTGGTGGAGGTGGA
TCAGAGGTGCAGCTGTTGGTGTCTGGGGGAGGCTTGGTACAGCCTGGGGGGTCCCTGCGTCTCTCCTGTGCAGCCT
CCGGATTCACCTTTAAGGCTTATCCGATGATGTGGGTCCGCCAGGCTCCAGGGAAGGGTCTAGAGTGGGTTTCAGA
GATTTCGCCTTCGGGTTCTTATACATACTACGCAGACTCCGTGAAGGGCCGGTTCACCATCTCCCGCGACAATTCC
AAGAACACGCTGTATCTGCAAATGAACAGCCTGCGTGCCGAGGACACCGCGGTATATTACTGTGCGAAAGATCCTC
GGAAGTTAGACTACTGGGGTCAGGGAACCCTGGTCACCGTCTCGAGC

**SEQ ID NO: 176**

QVQLKQSGPGLVQPSQSLSITCTVSGFSLTNYGVHWVRQSPGKGLEWLGVIWSGGNTDYNTPFTSRLSINKD
NSKSQVFFKMNSLQSNDTAIYYCARALTYYDYEFAYWGQGTLVTVSAASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHE
ALHNHYTQKSLSLSPGKSTGGGGGSEVQLLVSGGGLVQPGGSLRLSCAASGFTFKAYPMMWVRQAPGKGLEW
VSEISPSGSYTYYADSVKGRFTISRDNSKNTLYLQMNSLRAEDTAVYYCAKDPRKLDYWGQGTLVTVSS

**SEQ ID NO: 177**

CAGGTGCAGCTGAAGCAGAGCGGCCCTGGCCTGGTGCAGCCCTCTCAGAGCCTGAGCATCACCTGTACCGTGAGCG
GCTTCAGCCTGACCAATTACGGCGTGCATTGGGTGCGGCAGTCTCCAGGCAAGGGCCTGGAATGGCTGGGAGTGAT
CTGGTCCGGCGGCAACACCGACTACAACACCCCCTTCACCAGCAGACTGAGCATCAACAAGGACAACAGCAAGAGC
CAGGTGTTCTTCAAGATGAACAGCCTGCAGAGCAACGACACCGCCATCTACTATTGTGCCAGGGCCCTGACCTACT
ACGACTACGAGTTCGCCTACTGGGGCCAGGGCACCCTGGTGACCGTGAGCGCCGCTAGCACCAAGGGCCCCAGCGT
GTTCCCCCTGGCCCCCAGCAGCAAGAGCACCAGCGGCGGCACAGCCGCCCTGGGCTGCCTGGTGAAGGACTACTTC
CCCGAGCCTGTGACCGTGTCCTGGAATAGCGGAGCCCTGACCTCCGGCGTGCACACCTTCCCCGCCGTGCTGCAGA
GCAGCGGCCTGTACTCCCTGAGCAGCGTGGTGACCGTGCCCAGCAGCAGCCTGGGCACCCAGACCTACATCTGCAA
CGTGAACCACAAGCCCAGCAACACCAAAGTGGACAAGAAAGTGGAGCCCAAGAGCTGCGATAAGACCCACACCTGC
CCCCCCTGCCCTGCCCCCGAGCTGCTGGGCGGACCTAGCGTGTTCCTGTTCCCCCCCAAGCCTAAGGACACCCTGA
TGATCAGCAGGACCCCCGAAGTGACCTGCGTGGTGGTGGATGTGAGCCACGAGGACCCTGAAGTGAAGTTCAACTG
GTACGTGGACGGCGTGGAAGTGCACAACGCCAAGACCAAGCCCAGAGAGGAGCAGTACAACAGCACCTACCGCGTG
GTGTCTGTGCTGACCGTGCTGCACCAGGATTGGCTGAACGGCAAGGAGTACAAGTGCAAAGTGAGCAACAAGGCCC
TGCCTGCCCCTATCGAGAAAACCATCAGCAAGGCCAAGGGCCAGCCTAGAGAGCCCCAGGTCTACACCCTGCCTCC
CTCCAGAGATGAGCTGACCAAGAACCAGGTGTCCCTGACCTGTCTGGTGAAGGGCTTCTACCCCAGCGACATCGCC
GTGGAGTGGGAGAGCAACGGCCAGCCCGAGAACAACTACAAGACCACCCCCCCTGTGCTGGACAGCGATGGCAGCT
TCTTCCTGTACTCCAAGCTGACCGTGGACAAGAGCAGATGGCAGCAGGGCAACGTGTTCAGCTGCAGCGTGATGCA
CGAGGCCCTGCACAATCACTACACCCAGAAGAGTCTGAGCCTGTCCCCTGGCAAGTCGACCGGTGGTGGAGGTGGA
TCAGGTGGAGGTGGATCAGAGGTGCAGCTGTTGGTGTCTGGGGGAGGCTTGGTACAGCCTGGGGGGTCCCTGCGTC
TCTCCTGTGCAGCCTCCGGATTCACCTTTAAGGCTTATCCGATGATGTGGGTCCGCCAGGCTCCAGGGAAGGGTCT
AGAGTGGGTTTCAGAGATTTCGCCTTCGGGTTCTTATACATACTACGCAGACTCCGTGAAGGGCCGGTTCACCATC
TCCCGCGACAATTCCAAGAACACGCTGTATCTGCAAATGAACAGCCTGCGTGCCGAGGACACCGCGGTATATTACT
GTGCGAAAGATCCTCGGAAGTTAGACTACTGGGGTCAGGGAACCCTGGTCACCGTCTCGAGC

**SEQ ID NO: 178**

QVQLKQSGPGLVQPSQSLSITCTVSGFSLTNYGVHWVRQSPGKGLEWLGVIWSGGNTDYNTPFTSRLSINKD
NSKSQVFFKMNSLQSNDTAIYYCARALTYYDYEFAYWGQGTLVTVSAASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHN

AKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHE
ALHNHYTQKSLSLSPGKSTGGGGSGGGGSEVQLLVSGGGLVQPGGSLRLSCAASGFTFKAYPMMWVRQAPG
KGLEWVSEISPSGSYTYYADSVKGRFTISRDNSKNTLYLQMNSLRAEDTAVYYCAKDPRKLDYWGQGTLVTV
SS

**SEO ID NO: 179**

GAGGTGCAGCTGGTCGAGTCTGGCGGCGGACTGGTGCAGCCTGGCGGCAGCCTGAGACTGAG
CTGCGCCGCCAGCGGCTACACCTTCACCAACTACGGCATGAACTGGGTGCGGCAGGCCCCTG
GCAAGGGCCTGGAATGGGTGGGCTGGATCAACACCTACACCGGCGAGCCCACCTACGCCGCC
GACTTCAAGCGGCGGTTCACCTTCAGCCTGGACACCAGCAAGAGCACCGCCTACCTGCAGAT
GAACAGCCTGCGGGCCGAGGACACCGCCGTGTACTACTGCGCCAAGTACCCCCACTACTACG
GCAGCAGCCACTGGTACTTCGACTACTGGGGGCAGGGTACCCTGGTCACCGTCTCGAGCGCT
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAGCCTGTGACCGTGTCCTGGAATA
GCGGAGCCCTGACCTCCGGCGTGCACACCTTCCCCGCCGTGCTGCAGAGCAGCGGCCTGTAC
TCCCTGAGCAGCGTGGTGACCGTGCCCAGCAGCAGCCTGGGCACCCAGACCTACATCTGCAA
CGTGAACCACAAGCCCAGCAACACCAAAGTGGACAAGAAAGTGGAGCCCAAGAGCTGCGATA
AGACCCACACCTGCCCCCCCTGCCCTGCCCCCGAGCTGCTGGGCGGACCTAGCGTGTTCCTG
TTCCCCCCCAAGCCTAAGGACACCCTGATGATCAGCAGGACCCCCGAAGTGACCTGCGTGGT
GGTGGATGTGAGCCACGAGGACCCTGAAGTGAAGTTCAACTGGTACGTGGACGGCGTGGAAG
TGCACAACGCCAAGACCAAGCCCAGAGAGGAGCAGTACAACAGCACCTACCGCGTGGTGTCT
GTGCTGACCGTGCTGCACCAGGATTGGCTGAACGGCAAGGAGTACAAGTGCAAAGTGAGCAA
CAAGGCCCTGCCTGCCCCTATCGAGAAAACCATCAGCAAGGCCAAGGGCCAGCCTAGAGAGC
CCCAGGTCTACACCCTGCCTCCCTCCAGAGATGAGCTGACCAAGAACCAGGTGTCCCTGACC
TGTCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACT
CCAAGCTGACCGTGGACAAGAGCAGATGGCAGCAGGGCAACGTGTTCAGCTGCAGCGTGATG
CACGAGGCCCTGCACAATCACTACACCCAGAAGAGTCTGAGCCTGTCCCCTGGCAAGTCGAC
CGGTGACATCCAGATGACCCAGAGCCCTTCAAGCCTGAGCGCCAGCGTGGGCGACAGAGTGA
CCATCACCTGCCGGGCCAGCCAGTGGATCGGCAACCTGCTGGACTGGTATCAGCAGAAGCCC
GGCAAGGCCCCCAAGCTGCTGATCTACTACGCCAGCTTCCTGCAGAGCGGCGTGCCCAGCCG
GTTTAGCGGCAGCGGCTACGGCACCGACTTCACCCTGACCATCAGCAGCCTGCAGCCCGAGG
ACTTCGCCACCTACTACTGCCAGCAGGCCAACCCTGCCCCCCTGACCTTCGGCCAGGGTACC
AAGGTGGAAATCAAACGG

**SEQ ID NO: 180**

EVQLVESGGGLVQPGGSLRLSCAASGYTFTNYGMNWVRQAPGKGLEWVGWINTYTGEPTYAA
DFKRRFTFSLDTSKSTAYLQMNSLRAEDTAVYYCAKYPHYYGSSHWYFDYWGQGTLVTVSSA
STKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGKSTGDIQMTQSPSSLSASVGDRVTITCRASQWIGNLLDWYQQKP

GKAPKLLIYYASFLQSGVPSRFSGSGYGTDFTLTISSLQPEDFATYYCQQANPAPLTFGQGT
KVEIKR

**SEQ ID NO: 181**

GACATCCAGATGACCCAGAGCCCCAGCAGCCTGAGCGCCAGCGTGGGCGACAGAGTGACCAT
CACCTGCAGCGCCAGCCAGGACATCAGCAACTACCTGAACTGGTATCAGCAGAAGCCCGGCA
AGGCCCCCAAGGTGCTGATCTACTTCACCAGCTCCCTGCACAGCGGCGTGCCCAGCCGGTTT
AGCGGCAGCGGCTCCGGCACCGACTTCACCCTGACCATCAGCAGCCTGCAGCCCGAGGACTT
CGCCACCTACTACTGCCAGCAGTACAGCACCGTGCCCTGGACCTTCGGCCAGGGTACCAAGG
TGGAGATCAAGCGTACGGTGGCCGCTCCCAGCGTGTTCATCTTCCCCCCCAGCGACGAGCAG
CTGAAGAGCGGCACCGCCTCCGTGGTGTGCCTGCTGAACAACTTCTACCCCCGGGAGGCCAA
GGTGCAGTGGAAGGTGGACAACGCCCTGCAGTCCGGCAACAGCCAGGAAAGCGTCACCGAGC
AGGACTCCAAGGACTCCACCTACAGCCTGAGCAGCACCCTGACCCTGAGCAAGGCCGACTAC
GAGAAGCACAAGGTGTACGCCTGCGAAGTGACCCACCAGGGCCTGTCCAGCCCCGTGACCAA
GAGCTTCAACCGGGGCGAGTGC

**SEQ ID NO: 182**

DIQMTQSPSSLSASVGDRVTITCSASQDISNYLNWYQQKPGKAPKVLIYFTSSLHSGVPSRFSGSGSGTDFT
LTISSLQPEDFATYYCQQYSTVPWTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC

**SEQ ID NO: 183**

CAGGTGCAGCTGAAGCAGAGCGGCCCTGGCCTGGTGCAGCCCTCTCAGAGCCTGAGCATCAC
CTGTACCGTGAGCGGCTTCAGCCTGACCAATTACGGCGTGCATTGGGTGCGGCAGTCTCCAG
GCAAGGGCCTGGAATGGCTGGGAGTGATCTGGTCCGGCGGCAACACCGACTACAACACCCCC
TTCACCAGCAGACTGAGCATCAACAAGGACAACAGCAAGAGCCAGGTGTTCTTCAAGATGAA
CAGCCTGCAGAGCAACGACACCGCCATCTACTATTGTGCCAGGGCCCTGACCTACTACGACT
ACGAGTTCGCCTACTGGGGCCAGGGCACCCTGGTGACCGTGAGCGCCGCTAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAGCCTGTGACCGTGTCCTGGAATAGCGGAGCCCTGA
CCTCCGGCGTGCACACCTTCCCCGCCGTGCTGCAGAGCAGCGGCCTGTACTCCCTGAGCAGC
GTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCT
GCCCCCCCTGCCCTGCCCCCGAGCTGCTGGGCGGACCTAGCGTGTTCCTGTTCCCCCCCAAG
CCTAAGGACACCCTGATGATCAGCAGGACCCCCGAAGTGACCTGCGTGGTGGTGGATGTGAG
CCACGAGGACCCTGAAGTGAAGTTCAACTGGTACGTGGACGGCGTGGAAGTGCACAACGCCA
AGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCC
TGCCCCTATCGAGAAAACCATCAGCAAGGCCAAGGGCCAGCCTAGAGAGCCCCAGGTCTACA
CCCTGCCTCCCTCCAGAGATGAGCTGACCAAGAACCAGGTGTCCCTGACCTGTCTGGTGAAG
GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTG
CACAATCACTACACCCAGAAGAGTCTGAGCCTGTCCCCTGGCAAGTCGACCGGTGGGGTGCA
GCTGTTGGAGTCTGGGGGAGGCTTGGTACAGCCTGGGGGGTCCCTGCGTCTCTCCTGTGCAG
CCTCCGGATTCACCTTCGCTTGGTATGATATGGGGTGGGTCCGCCAGGCTCCAGGGAAGGGT

CTAGAGTGGGTCTCAAGTATTGATTGGCATGGTGAGGTTACATACTACGCAGACTCCGTGAA
GGGCCGGTTCACCATCTCCCGCGACAATTCCAAGAACACGCTGTATCTGCAAATGAACAGCC
TGCGTGCCGAGGACACCGCGGTATATTACTGTGCGACAGCGGAGGACGAGCCGGGGTATGAC
TACTGGGGCCAGGGAACCCTGGTCACCGTCTCGAGC

**SEQ ID NO:184**

QVQLKQSGPGLVQPSQSLSITCTVSGFSLTNYGVHWVRQSPGKGLEWLGVIWSGGNTDYNTPFTSRLSINKDNSKS
QVFFKMNSLQSNDTAIYYCARALTYYDYEFAYWGQGTLVTVSAASTKGPSVFPLAPSSKSTSGGTAALGCLVKDYF
PEPVTVSWNSGALTSGVHTFPAVLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEALHNHYTQKSLSLSPGKSTGGVQL
LESGGGLVQPGGSLRLSCAASGFTFAWYDMGWVRQAPGKGLEWVSSIDWHGEVTYYADSVKGRFTISRDNSKNTLY
LQMNSLRAEDTAVYYCATAEDEPGYDYWGQGTLVTVSS

**SEQ ID NO: 185**

EVQLVESGGGLVQPGRSLRLSCAASGFTFDDYAMHWVRQAPGKGLEWVSAITWNSGHIDYADSVEGRFTISR
DNAKNSLYLQMNSLRAEDTAVYYCAKVSYLSTASSLDYWGQGTLVTVSSASTKGPSVFPLAPSSKSTSGGTA
ALGCLVKDYFPEPVTVSWNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQQGNVFSCSVM
HEALHNHYTQKSLSLSPGKSTGDIQMTQSPSSLSASVGDRVTITCRASQWIGPELRWYQQKPGKAPKLLIYH
TSILQSGVPSRFSGSGSGTDFTLTISSLQPEDFATYYCQQYMFQPMTFGQGTKVEIKR

**SEQ ID NO: 186**

QVQLKQSGPGLVQPSQSLSITCTVSGFSLTNYGVHWVRQSPGKGLEWLGVIWSGGNTDYNTPFTSRLSINKD
NSKSQVFFKMNSLQSNDTAIYYCARALTYYDYEFAYWGQGTLVTVSAASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQQGNVFSCSVMHE
ALHNHYTQKSLSLSPGKSTGDIQMTQSPSSLSASVGDRVTITCRASQWIGPELRWYQQKPGKAPKLLIYHTS
ILQSGVPSRFSGSGSGTDFTLTISSLQPEDFATYYCQQYMFQPMTFGQGTKVEIKR

**SEQ ID NO: 187**

GACATCCTGCTGACCCAGAGCCCCGTGATCCTGAGCGTGAGCCCTGGCGAGAGAGTGAGCTTCAGCTGCCGGGCCA
GCCAGAGCATCGGCACCAACATCCACTGGTATCAGCAGCGGACCAACGGCAGCCCCAGGCTGCTGATCAAGTACGC
CAGCGAGTCCATCAGCGGCATCCCCAGCCGGTTCAGCGGCAGCGGCTCCGGCACCGACTTCACCCTGAGCATCAAC
AGCGTGGAGAGCGAGGATATCGCCGACTACTACTGCCAGCAGAACAACAACTGGCCCACCACCTTCGGAGCCGGCA
CCAAGCTGGAACTGAAGCGTACGGTGGCCGCCCCCAGCGTGTTCATCTTCCCCCCCAGCGATGAGCAGCTCAAGAG
CGGCACCGCCAGCGTGGTGTGTCTGCTGAACAACTTCTACCCCCGGGAGGCCAAAGTGCAGTGGAAAGTGGACAAC
GCCCTGCAGAGCGGCAACAGCCAGGAGAGCGTGACCGAGCAGGACAGCAAGGACTCCACCTACAGCCTGAGCAGCA
CCCTGACCCTGAGCAAGGCCGACTACGAGAAGCACAAAGTGTACGCCTGCGAAGTGACCCACCAGGGCCTGTCCAG
CCCCGTGACCAAGAGCTTCAACCGGGGCGAGTGCGGATCCACCGGCGAGGTGCAGCTGTTGGTGTCTGGGGGAGGC
TTGGTACAGCCTGGGGGGTCCCTGCGTCTCTCCTGTGCAGCCTCCGGATTCACCTTTAAGGCTTATCCGATGATGT

GGGTCCGCCAGGCTCCAGGGAAGGGTCTAGAGTGGGTTTCAGAGATTTCGCCTTCGGGTTCTTATACATACTACGC
AGACTCCGTGAAGGGCCGGTTCACCATCTCCCGCGACAATTCCAAGAACACGCTGTATCTGCAAATGAACAGCCTG
CGTGCCGAGGACACCGCGGTATATTACTGTGCGAAAGATCCTCGGAAGTTAGACTACTGGGGTCAGGGAACCCTGG
TCACCGTCTCGAGC

**SEQ ID NO: 188**

DILLTQSPVILSVSPGERVSFSCRASQSIGTNIHWYQQRTNGSPRLLIKYASESISGIPSRFSGSGSGTDFT
LSINSVESEDIADYYCQQNNNWPTTFGAGTKLELKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGECGS
TGEVQLLVSGGGLVQPGGSLRLSCAASGFTFKAYPMMWVRQAPGKGLEWVSEISPSGSYTYYADSVKGRFTI
SRDNSKNTLYLQMNSLRAEDTAVYYCAKDPRKLDYWGQGTLVTVSS

**SEQ ID NO: 189**

GACATCCTGCTGACCCAGAGCCCCGTGATCCTGAGCGTGAGCCCTGGCGAGAGAGTGAGCTTCAGCTGCCGGGCCA
GCCAGAGCATCGGCACCAACATCCACTGGTATCAGCAGCGGACCAACGGCAGCCCCAGGCTGCTGATCAAGTACGC
CAGCGAGTCCATCAGCGGCATCCCCAGCCGGTTCAGCGGCAGCGGCTCCGGCACCGACTTCACCCTGAGCATCAAC
AGCGTGGAGAGCGAGGATATCGCCGACTACTACTGCCAGCAGAACAACAACTGGCCCACCACCTTCGGAGCCGGCA
CCAAGCTGGAACTGAAGCGTACGGTGGCCGCCCCCAGCGTGTTCATCTTCCCCCCCAGCGATGAGCAGCTCAAGAG
CGGCACCGCCAGCGTGGTGTGTCTGCTGAACAACTTCTACCCCCGGGAGGCCAAAGTGCAGTGGAAAGTGGACAAC
GCCCTGCAGAGCGGCAACAGCCAGGAGAGCGTGACCGAGCAGGACAGCAAGGACTCCACCTACAGCCTGAGCAGCA
CCCTGACCCTGAGCAAGGCCGACTACGAGAAGCACAAAGTGTACGCCTGCGAAGTGACCCACCAGGGCCTGTCCAG
CCCCGTGACCAAGAGCTTCAACCGGGGCGAGTGCGGATCCACGGTGGCCGCCCCCAGCGAGGTGCAGCTGTTGGTG
TCTGGGGGAGGCTTGGTACAGCCTGGGGGGTCCCTGCGTCTCTCCTGTGCAGCCTCCGGATTCACCTTTAAGGCTT
ATCCGATGATGTGGGTCCGCCAGGCTCCAGGGAAGGGTCTAGAGTGGGTTTCAGAGATTTCGCCTTCGGGTTCTTA
TACATACTACGCAGACTCCGTGAAGGGCCGGTTCACCATCTCCCGCGACAATTCCAAGAACACGCTGTATCTGCAA
ATGAACAGCCTGCGTGCCGAGGACACCGCGGTATATTACTGTGCGAAAGATCCTCGGAAGTTAGACTACTGGGGTC
AGGGAACCCTGGTCACCGTCTCGAGC

**SEQ ID NO: 190**

DILLTQSPVILSVSPGERVSFSCRASQSIGTNIHWYQQRTNGSPRLLIKYASESISGIPSRFSGSGSGTDFT
LSINSVESEDIADYYCQQNNNWPTTFGAGTKLELKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGECGS
TVAAPSEVQLLVSGGGLVQPGGSLRLSCAASGFTFKAYPMMWVRQAPGKGLEWVSEISPSGSYTYYADSVKG
RFTISRDNSKNTLYLQMNSLRAEDTAVYYCAKDPRKLDYWGQGTLVTVSS

**SEQ ID NO: 191**

QVTLRESGPALVKPTQTLTLTCTVSGFSLTSYSVHWVRQPPGKGLEWLGVIWASGGTDYNSALMSRLSI
SKDTSRNQVVLTMTNMDPVDTATYYCARDPPSSLLRLDYWGRGTPVTVSSASTKGPSVFPLAPSSKSTS
GGTAALGCLVKDYFPEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK

**SEQ ID** NO: 192: 586H-ASTKG-210 Heavy chain

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCC
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC
GTGATGCACGAGGCCCTGCACAATCACTACACCCAGAAGAGCCTGAGCCTGTCCCCTGGCAA
GGGATCAGCCAGCACCAAGGGCCCCACGGGATCCGAAGTGCAGCTCCTGGAGAGCGGCGGCG
GCCTGGTGCAGCCCGGCGGCAGCCTGAGGCTGAGCTGCGCCGCTAGCGGCTTCACCTTCAGG
AACTTCGGCATGGGCTGGGTCAGGCAGGCCCCCGGCAAGGGCCTGGAGTGGGTCAGCTGGAT
CATCAGCTCCGGCACCGAGACCTACTACGCCGACAGCGTGAAGGGCAGGTTCACCATCAGCC
GCGACAACAGCAAGAACACCCTGTACCTGCAGATGAACAGCCTGAGGGCCGAGGACACCGCC
GTCTACTACTGCGCCAAGAGCCTGGGCAGGTTCGACTACTGGGGACAGGGGACCCTGGTGAC
TGTGAGCAGC

**SEQ ID NO: 193**

EVTLRESGPALVKPTQTLTLTCTFSGFSLSKSVMGVSWIRQPPGKALEWLAHIYWDDDKYYN
PSLKSRLTISKDTSKNQVVLTMTNMDPVDTATYYCARRGIRSAMDYWGQGTTVTVSSASTKG
PEVQLVQSGTEVKKPGESLKISCKGSGYTVTSYWIGWVRQMPGKGLEWMGFIYPGDSETRYS
PTFQGQVTISADKSFNTAFLQWSSLKASDTAMYYCARVGSGWYPYTFDIWGQGTLVTVSSAS
TKGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMH
EALHNHYTQKSLSLSPGKTVAAPSEVQLLESGGGLVQPGGSLRLSCAASGFTFRNFGMGWVR
QAPGKGLEWVSWIISSGTETYYADSVKGRFTISRDNSKNTLYLQMNSLRAEDTAVYYCAKSL
GRFDYWGQGTLVTVSS

**SEQ ID NO: 194**

DIVMTQSPDSLAVSLGERATINCKASQSVSNDVAWYQQKPGQPPKLLIYYASNRYTGVPDRF
SGSGSGTDFTLTISSLQAEDVAVYYCQQDYNSPWTFGGGTKVEIKRTVAAPEIVMTQSPATL
SVSPGERATLSCRASESASSNLAWYQQKPGQAPRLFIYTASTRATDIPARFSGSGSGTEFTL
TISSLQSEDFAVYYCQQYNNWPSITFGQGTRLEIKRTVAAPSVFIFPPSDEQLKSGTASVVC
LLNNFYPREAKVQWKVDNALQSGNSQESVTEQDSKDSTYSLSSTLTLSKADYEKHKVYACEV
THQGLSSPVTKSFNRGEC

**SEQ ID NO: 195: Anti IL-5 Heavy Chain-G4S-dAb474-TVAAPSGS-dAb210**

QVTLRESGPALVKPTQTLTLTCTVSGFSLTSYSVHWVRQPPGKGLEWLGVIWASGGTDYNSA
LMSRLSISKDTSRNQVVLTMTNMDPVDTATYYCARDPPSSLLRLDYWGRGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFSCSVMHEAL
HNHYTQKSLSLSPGKGGGGSGVQLLESGGGLVQPGGSLRLSCAASGFTFAWYDMGWVRQAPG
KGLEWVSSIDWHGEVTYYADSVKGRFTISRDNSKNTLYLQMNSLRAEDTAVYYCATAEDEPG
YDYWGQGTLVTVSSTVAAPSGSEVQLLESGGGLVQPGGSLRLSCAASGFTFRNFGMGWVRQA
PGKGLEWVSWIISSGTETYYADSVKGRFTISRDNSKNTLYLQMNSLRAEDTAVYYCAKSLGR
FDYWGQGTLVTVSS

**SEQ ID NO: 196: Anti CD-20 Heavy Chain-TVAAPSGS-dAb154-TVAAPSGS-dAb474**

QVQLQQPGAELVKPGASVKMSCKASGYTFTSYNMHWVKQTPGRGLEWIGAIYPGNGDTSYNQ
KFKGKATLTADKSSSTAYMQLSSLTSEDSAVYYCARSTYYGGDWYFNVWGAGTLVTVSSAST
KGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKTVAAPSGSDIQMTQSPSSLSASVGDRVTITCRASRPISDWLHWYQ
QKPGKAPKLLIAWASSLQGGVPSRFSGSGSGTDFTLTISSLQPEDFATYYCLQEGWGPPTFG
QGTKVEIKRTVAAPSGSGVQLLESGGGLVQPGGSLRLSCAASGFTFAWYDMGWVRQAPGKGL
EWVSSIDWHGEVTYYADSVKGRFTISRDNSKNTLYLQMNSLRAEDTAVYYCATAEDEPGYDY
WGQGTLVTVSS

**SEQ ID NO: 197: Anti CD-20 Heavy Chain-TVAAPSGS-dAb210-TVAAPSGS-dAb474**

QVQLQQPGAELVKPGASVKMSCKASGYTFTSYNMHWVKQTPGRGLEWIGAIYPGNGDTSYNQ
KFKGKATLTADKSSSTAYMQLSSLTSEDSAVYYCARSTYYGGDWYFNVWGAGTLVTVSSAST
KGPSVFPLAPSSKSTSGGTAALGCLVKDYFPEPVTVSWNSGALTSGVHTFPAVLQSSGLYSL

SSVVTVPSSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHE
ALHNHYTQKSLSLSPGKTVAAPSGSEVQLLESGGGLVQPGGSLRLSCAASGFTFRNFGMGWV
RQAPGKGLEWVSWIISSGTETYYADSVKGRFTISRDNSKNTLYLQMNSLRAEDTAVYYCAKS
LGRFDYWGQGTLVTVSSTVAAPSGSGVQLLESGGGLVQPGGSLRLSCAASGFTFAWYDMGWV
RQAPGKGLEWVSSIDWHGEVTYYADSVKGRFTISRDNSKNTLYLQMNSLRAEDTAVYYCATA
EDEPGYDYWGQGTLVTVSS

**SEQ ID NO: 198: anti cMET 5D5v2 Heavy Chain (hole)-GS-dAb593**

EVQLVESGGGLVQPGGSLRLSCAASGYTFTSYWLHWVRQAPGKGLEWVGMIDPSNSDTRFNP
NFKDRFTISADTSKNTAYLQMNSLRAEDTAVYYCATYRSYVTPLDYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLSCAVK
GFYPSDIAVEWESNGQPENNYKTTPPVLDSDGSFFLVSKLTVDKSRWQQGNVFSCSVMHEAL
HNHYTQKSLSLSPGKGSEVQLLVSGGGLVQPGGSLRLSCAASGFTFKAYPMMWVRQAPGKGL
EWVSEISPSGSYTYYADSVKGRFTISRDNSKNTLYLQMNSLRAEDTAVYYCAKDPRKLDYWG
QGTLVTVSS

**SEQ ID NO: 199: anti cMET 5D5v2 Heavy Chain (knob)-GS-dAb593**

CPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVY
TLPPSRDELTKNQVSLWCLVKGFYPSDIAVEWESNGQPENNYKTTPPVLDSDGSFFLYSKLT
VDKSRWQQGNVFSCSVMHEALHNHYTQKSLSLSPGKGSEVQLLVSGGGLVQPGGSLRLSCAA
SGFTFKAYPMMWVRQAPGKGLEWVSEISPSGSYTYYADSVKGRFTISRDNSKNTLYLQMNSL
RAEDTAVYYCAKDPRKLDYWGQGTLVTVSS

**SEQ ID NO: 200: anti cMET 5D5v2 Light Chain**

DIQMTQSPSSLSASVGDRVTITCKSSQSLLYTSSQKNYLAWYQQKPGKAPKLLIYWASTRES
GVPSRFSGSGSGTDFTLTISSLQPEDFATYYCQQYYAYPWTFGQGTKVEIKRTVAAPSVFIF
PPSDEQLKSGTASVVCLLNNFYPREAKVQWKVDNALQSGNSQESVTEQDSKDSTYSLSSTLT
LSKADYEKHKVYACEVTHQGLSSPVTKSFNRGEC

**SEQ ID NO: 201: anti cMET 5D5v2 IgG4 Heavy Chain (UNIBODY)-GS-dAb593**

EVQLVESGGGLVQPGGSLRLSCAASGYTFTSYWLHWVRQAPGKGLEWVGMIDPSNSDTRFNP
NFKDRFTISADTSKNTAYLQMNSLRAEDTAVYYCATYRSYVTPLDYWGQGTLVTVSSASTKG
PSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVAPEFLGGPSVFLFPPKPKDTLMISRTPEVTC
VVVDVSQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSLSLSLGK
GSEVQLLVSGGGLVQPGGSLRLSCAASGFTFKAYPMMWVRQAPGKGLEWVSEISPSGSYTYY
ADSVKGRFTISRDNSKNTLYLQMNSLRAEDTAVYYCAKDPRKLDYWGQGTLVTVSS

**SEQ ID NO: 202: anti cMET 5D5v2 Heavy Chain (hole)**

EVQLVESGGGLVQPGGSLRLSCAASGYTFTSYWLHWVRQAPGKGLEWVGMIDPSNSDTRFNP
NFKDRFTISADTSKNTAYLQMNSLRAEDTAVYYCATYRSYVTPLDYWGQGTLVTVSSASTKG
PSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLSCAVK
GFYPSDIAVEWESNGQPENNYKTTPPVLDSDGSFFLVSKLTVDKSRWQQGNVFSCSVMHEAL
HNHYTQKSLSLSPGK

**SEQ ID NO: 203: anti cMET 5D5v2 Heavy Chain (knob)**

CPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVY
TLPPSRDELTKNQVSLWCLVKGFYPSDIAVEWESNGQPENNYKTTPPVLDSDGSFFLYSKLT
VDKSRWQQGNVFSCSVMHEALHNHYTQKSLSLSPGK

**SEQ ID NO: 204: anti cMET 5D5v2 IgG4 Heavy Chain (UNIBODY)**

EVQLVESGGGLVQPGGSLRLSCAASGYTFTSYWLHWVRQAPGKGLEWVGMIDPSNSDTRFNP
NFKDRFTISADTSKNTAYLQMNSLRAEDTAVYYCATYRSYVTPLDYWGQGTLVTVSSASTKG
PSVFPLAPCSRSTSESTAALGCLVKDYFPEPVTVSWNSGALTSGVHTFPAVLQSSGLYSLSS
VVTVPSSSLGTKTYTCNVDHKPSNTKVDKRVAPEFLGGPSVFLFPPKPKDTLMISRTPEVTC
VVVDVSQEDPEVQFNWYVDGVEVHNAKTKPREEQFNSTYRVVSVLTVLHQDWLNGKEYKCKV

SNKGLPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSLSLSLGK

**SEQ ID NO: 205**: Anti-human IL13 mAb Heavy chain

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCC
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC
GTGATGCACGAGGCCCTGCACAATCACTACACCCAGAAGAGCCTGAGCCTGTCCCCTGGCAA
G

**SEQ ID NO: 206:** Alternative Anti-human IL13 mAb Heavy chain

CAGGTGCAGCTGGTGCAGTCTGGGGCTGAGGTGAAGAAGCCTGGGTCCTCGGTGAAGGTCTC
CTGCAAGGCTTCTGGATTCTACATTAAAGACACCTATATGCACTGGGTGCGACAGGCCCCTG
GACAAGGGCTTGAGTGGATGGGAACGATTGATCCTGCGAATGGTAATACTAAATATGTCCCG
AAGTTCCAGGGCAGAGTCACGATTACCGCGGACGAATCCACGAGCACAGCCTACATGGAGCT
GAGCAGCCTGAGATCTGAGGACACGGCCGTGTATTACTGTGCGAGAAGCATCTATGATGATT
ACCACTACGACGATTACTATGCTATGGACTACTGGGGCCAAGGGACACTAGTCACAGTCTCC
TCAGCCTCCACCAAGGGCCCATCGGTCTTCCCCCTGGCACCCTCCTCCAAGAGCACCTCTGG
GGGCACAGCGGCCCTGGGCTGCCTGGTCAAGGACTACTTCCCCGAACCGGTGACGGTGTCGT
GGAACTCAGGCGCCCTGACCAGCGGCGTGCACACCTTCCCGGCTGTCCTACAGTCCTCAGGA
CTCTACTCCCTCAGCAGCGTGGTGACCGTGCCCTCCAGCAGCTTGGGCACCCAGACCTACAT
CTGCAACGTGAATCACAAGCCCAGCAACACCAAGGTGGACAAGAAAGTTGAGCCCAAATCTT
GTGACAAAACTCACACATGCCCACCGTGCCCAGCACCTGAACTCCTGGGGGGACCGTCAGTC
TTCCTCTTCCCCCCAAAACCCAAGGACACCCTCATGATCTCCCGGACCCCTGAGGTCACATG
CGTGGTGGTGGACGTGAGCCACGAAGACCCTGAGGTCAAGTTCAACTGGTACGTGGACGGCG
TGGAGGTGCATAATGCCAAGACAAAGCCGCGGGAGGAGCAGTACAACAGCACGTACCGTGTG
GTCAGCGTCCTCACCGTCCTGCACCAGGACTGGCTGAATGGCAAGGAGTACAAGTGCAAGGT
CTCCAACAAAGCCCTCCCAGCCCCCATCGAGAAAACCATCTCCAAAGCCAAAGGGCAGCCCC
GAGAACCACAGGTGTACACCCTGCCCCCATCCCGGGATGAGCTGACCAAGAACCAGGTCAGC
CTGACCTGCCTGGTCAAAGGCTTCTATCCCAGCGACATCGCCGTGGAGTGGGAGAGCAATGG
GCAGCCGGAGAACAACTACAAGACCACGCCTCCCGTGCTGGACTCCGACGGCTCCTTCTTCC
TCTACAGCAAGCTCACCGTGGACAAGAGCAGGTGGCAGCAGGGGAACGTCTTCTCATGCTCC
GTGATGCATGAGGCTCTGCACAACCACTACACGCAGAAGAGCCTCTCCCTGTCTCCGGGTAA
A

**SEQ ID NO 207: PascoH IgG2-GS-474 heavy**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPCSRSTSESTAALGCLVKDYFPEPVTVSWNSGALTSGVHTFPAVLQSSGLYSL
SSVVTVPSSNFGTQTYTCNVDHKPSNTKVDKTVERKCCVECPPCPAPPVAGPSVFLFPPKPK
DTLMISRTPEVTCVVVDVSHEDPEVQFNWYVDGVEVHNAKTKPREEQFNSTFRVVSVLTVVH
QDWLNGKEYKCKVSNKGLPAPIEKTISKTKGQPREPQVYTLPPSREEMTKNQVSLTCLVKGF
YPSDIAVEWESNGQPENNYKTTPPMLDSDGSFFLYSKLTVDKSRWQQGNVFSCSVMHEALHN
HYTQKSLSLSPGKGSGVQLLESGGGLVQPGGSLRLSCAASGFTFAWYDMGWVRQAPGKGLEW
VSSIDWHGEVTYYADSVKGRFTISRDNSKNTLYLQMNSLRAEDTAVYYCATAEDEPGYDYWG
QGTLVTVSS

**SEQ ID NO 208: PascoH IgG4-GS-474 heavy chain**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPCSRSTSESTAALGCLVKDYFPEPVTVSWNSGALTSGVHTFPAVLQSSGLYSL
SSVVTVPSSSLGTKTYTCNVDHKPSNTKVDKRVESKYGPPCPSCPAPEFLGGPSVFLFPPKP
KDTLMISRTPEVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVMHEALH
NHYTQKSLSLSLGKGSGVQLLESGGGLVQPGGSLRLSCAASGFTFAWYDMGWVRQAPGKGLE
WVSSIDWHGEVTYYADSVKGRFTISRDNSKNTLYLQMNSLRAEDTAVYYCATAEDEPGYDYW
GQGTLVTVSS

**SEQ ID NO 209: PascoH IgG4PE-GS-474 heavy chain**

QVTLRESGPALVKPTQTLTLTCTFSGFSLSTSGMGVSWIRQPPGKGLEWLAHIYWDDDKRYN
PSLKSRLTISKDTSRNQVVLTMTNMDPVDTATYYCARRETVFYWYFDVWGRGTLVTVSSAST
KGPSVFPLAPCSRSTSESTAALGCLVKDYFPEPVTVSWNSGALTSGVHTFPAVLQSSGLYSL
SSVVTVPSSSLGTKTYTCNVDHKPSNTKVDKRVESKYGPPCPPCPAPEFEGGPSVFLFPPKP
KDTLMISRTPEVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVMHEALH
NHYTQKSLSLSLGKGSGVQLLESGGGLVQPGGSLRLSCAASGFTFAWYDMGWVRQAPGKGLE
WVSSIDWHGEVTYYADSVKGRFTISRDNSKNTLYLQMNSLRAEDTAVYYCATAEDEPGYDYW
GQGTLVTVSS

**SEQ ID NO: 210:** Anti-human IL13 mAb Light chain

GACATCGTGATGACCCAGTCTCCTCTGAGCCTCCCCGTGACCCCCGGCGAACCAGCCAGCAT
CTCCTGCAGAAGCAGCCAGAACATCGTGCACATCAACGGCAACACCTACCTGGAGTGGTACC
TGCAAAAGCCCGGCCAGAGCCCCAGGCTGCTGATCTACAAGATCAGCGACAGGTTCAGCGGC
GTGCCCGATAGGTTCAGCGGCAGCGGCAGCGGCACCGACTTCACCCTGAAGATCAGCAGGGT
GGAGGCCGACGACGTGGGCATCTACTACTGCTTCCAGGGCAGCCACGTCCCCTGGACTTTCG
GACAGGGCACCAAGCTGGAGATTAAGCGTACGGTGGCCGCCCCCAGCGTGTTCATCTTCCCC
CCCAGCGATGAGCAGCTGAAGAGCGGCACCGCCAGCGTGGTGTGTCTGCTGAACAACTTCTA
CCCCCGGGAGGCCAAGGTGCAGTGGAAGGTGGACAATGCCCTGCAGAGCGGCAACAGCCAGG
AGAGCGTGACCGAGCAGGACAGCAAGGACTCCACCTACAGCCTGAGCAGCACCCTGACCCTG
AGCAAGGCCGACTACGAGAAGCACAAGGTGTACGCCTGTGAGGTGACCCACCAGGGCCTGTC
CAGCCCCGTGACCAAGAGCTTCAACCGGGGCGAGTGC

**SEQ ID NO: 211:** Pascolizumab Heavy chain

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC

CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAG

**SEQ ID NO: 212:** Pascolizumab Light chain

GACATCGTGCTGACCCAGAGCCCCTCTTCCCTGAGCGCAAGCGTGGGCGATAGGGTGACCAT
CACCTGCAAGGCCAGCCAGAGCGTGGACTACGACGGCGACAGCTACATGAACTGGTACCAGC
AGAAGCCCGGCAAGGCCCCCAAACTGCTGATCTACGCCGCCAGCAACCTCGAGTCAGGCATT
CCCAGCAGGTTTAGCGGCAGCGGCAGCGGCACCGACTTCACCTTCACAATCAGCAGCCTGCA
GCCCGAGGACATCGCCACCTACTACTGCCAGCAGAGCAACGAGGACCCTCCCACCTTCGGAC
AGGGCACCAAGGTCGAGATCAAGCGTACGGTGGCCGCCCCCAGCGTGTTCATCTTCCCCCCC
AGCGATGAGCAGCTGAAGAGCGGCACCGCCAGCGTGGTGTGTCTGCTGAACAACTTCTACCC
CCGGGAGGCCAAGGTGCAGTGGAAGGTGGACAATGCCCTGCAGAGCGGCAACAGCCAGGAGA
GCGTGACCGAGCAGGACAGCAAGGACTCCACCTACAGCCTGAGCAGCACCCTGACCCTGAGC
AAGGCCGACTACGAGAAGCACAAGGTGTACGCCTGTGAGGTGACCCACCAGGGCCTGTCCAG
CCCCGTGACCAAGAGCTTCAACCGGGGCGAGTGC

**SEQ ID NO: 213:** Mepolizumab Heavy chain

CAGGTGACCCTGAGGGAGAGCGGCCCCGCCCTGGTGAAGCCCACACAGACCCTCACTCTGAC
CTGCACCGTGAGCGGCTTCAGCCTGACCTCCTACAGCGTCCACTGGGTGAGGCAGCCCCCCG
GCAAGGGCCTGGAGTGGCTGGGCGTGATCTGGGCAAGCGGCGGCACCGACTACAACAGCGCC
CTGATGAGCAGGCTCTCCATCAGCAAGGACACCAGCCGGAACCAGGTGGTGCTGACCATGAC
CAACATGGACCCCGTGGACACCGCCACCTATTACTGCGCCAGGGACCCTCCCTCTAGCCTGC
TGAGGCTGGACTACTGGGGCAGGGGAACACTAGTGACCGTGTCCAGCGCCAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAACCGGTGACCGTGTCCTGGAACAGCGGAGCCCTGA
CCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAA
GCCCAGCAACACCAAGGTGGACAAGAAGGTGGAGCCCAAGAGCTGTGACAAGACCCACACCT
GCCCCCCCTGCCCTGCCCCCGAGCTGCTGGGAGGCCCCAGCGTGTTCCTGTTCCCCCCCAAG
CCTAAGGACACCCTGATGATCAGCAGAACCCCCGAGGTGACCTGTGTGGTGGTGGATGTGAG
CCACGAGGACCCTGAGGTGAAGTTCAACTGGTACGTGGACGGCGTGGAGGTGCACAATGCCA
AGACCAAGCCCAGGGAGGAGCAGTACAACAGCACCTACCGGGTGGTGTCCGTGCTGACCGTG
CTGCACCAGGATTGGCTGAACGGCAAGGAGTACAAGTGTAAGGTGTCCAACAAGGCCCTGCC
TGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAG
GGCTTCTACCCCAGCGACATCGCCGTGGAGTGGGAGAGCAACGGCCAGCCCGAGAACAACTA

CAAGACCACCCCCCCTGTGCTGGACAGCGATGGCAGCTTCTTCCTGTACAGCAAGCTGACCG
TGGACAAGAGCAGATGGCAGCAGGGCAACGTGTTCAGCTGCTCCGTGATGCACGAGGCCCTG
CACAATCACTACACCCAGAAGAGCCTGAGCCTGTCCCCTGGCAAG

**SEQ ID NO: 214:** Mepolizumab Light chain

GACATCGTGATGACCCAGTCTCCCGATTCACTGGCCGTGAGCCTGGGCGAGAGGGCCACCAT
CAACTGCAAGAGCAGCCAGAGCCTCCTGAACAGCGGCAACCAGAAGAACTACCTGGCCTGGT
ACCAGCAGAAACCCGGCCAGCCCCCCAAGCTGCTGATCTATGGCGCCTCCACCAGGGAGAGC
GGCGTGCCAGACAGGTTTAGCGGCAGCGGCAGCGGCACCGACTTCACCCTGACAATCAGCAG
CCTGCAGGCCGAGGACGTGGCCGTGTACTACTGCCAGAACGTCCACAGCTTCCCCTTCACCT
TCGGCGGGGGAACCAAGCTGGAGATCAAGCGTACGGTGGCCGCCCCCAGCGTGTTCATCTTC
CCCCCCAGCGATGAGCAGCTGAAGAGCGGCACCGCCAGCGTGGTGTGTCTGCTGAACAACTT
CTACCCCCGGGAGGCCAAGGTGCAGTGGAAGGTGGACAATGCCCTGCAGAGCGGCAACAGCC
AGGAGAGCGTGACCGAGCAGGACAGCAAGGACTCCACCTACAGCCTGAGCAGCACCCTGACC
CTGAGCAAGGCCGACTACGAGAAGCACAAGGTGTACGCCTGTGAGGTGACCCACCAGGGCCT
GTCCAGCCCCGTGACCAAGAGCTTCAACCGGGGCGAGTGC

**SEQ ID NO: 215:** PascoH-474 Heavy chain

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGGGATCCGGCGT
GCAGCTCCTGGAGAGCGGCGGAGGCCTGGTCCAGCCCGGCGGCAGCCTGAGGCTGAGCTGCG
CCGCCAGCGGCTTCACCTTCGCCTGGTATGATATGGGCTGGGTGAGGCAGGCCCCCGGCAAG
GGCCTGGAGTGGGTGTCCAGCATCGACTGGCACGGGGAGGTGACCTACTACGCCGACAGCGT
GAAGGGCAGGTTCACCATCAGCAGGGACAACAGCAAGAACACCCTGTACCTGCAGATGAACA
GCCTGAGGGCCGAGGACACCGCAGTGTACTACTGCGCCACCGCCGAGGACGAACCCGGCTAC
GACTACTGGGGCCAGGGCACCCTGGTGACTGTGAGCAGC

**SEQ ID NO: 216:** PascoH-TVAAPS-474 Heavy chain

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC

AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGACCGTGGCCGC
CCCCTCGGGATCCGGCGTGCAGCTCCTGGAGAGCGGCGGAGGCCTGGTCCAGCCCGGCGGCA
GCCTGAGGCTGAGCTGCGCCGCCAGCGGCTTCACCTTCGCCTGGTATGATATGGGCTGGGTG
AGGCAGGCCCCCGGCAAGGGCCTGGAGTGGGTGTCCAGCATCGACTGGCACGGGGAGGTGAC
CTACTACGCCGACAGCGTGAAGGGCAGGTTCACCATCAGCAGGGACAACAGCAAGAACACCC
TGTACCTGCAGATGAACAGCCTGAGGGCCGAGGACACCGCAGTGTACTACTGCGCCACCGCC
GAGGACGAACCCGGCTACGACTACTGGGGCCAGGGCACCCTGGTGACTGTGAGCAGC

**SEQ ID NO: 217:** Pasco-474 Light chain

GACATCGTGCTGACCCAGAGCCCCTCTTCCCTGAGCGCAAGCGTGGGCGATAGGGTGACC
ATCACCTGCAAGGCCAGCCAGAGCGTGGACTACGACGGCGACAGCTACATGAACTGGTAC
CAGCAGAAGCCCGGCAAGGCCCCCAAACTGCTGATCTACGCCGCCAGCAACCTCGAGTCA
GGCATTCCCAGCAGGTTTAGCGGCAGCGGCAGCGGCACCGACTTCACCTTCACAATCAGC
AGCCTGCAGCCCGAGGACATCGCCACCTACTACTGCCAGCAGAGCAACGAGGACCCTCCC
ACCTTCGGACAGGGCACCAAGGTCGAGATCAAGCGTACGGTGGCCGCCCCCAGCGTGTTC
ATCTTCCCCCCCAGCGATGAGCAGCTGAAGAGCGGCACCGCCAGCGTGGTGTGTCTGCTG
AACAACTTCTACCCCCGGGAGGCCAAGGTGCAGTGGAAGGTGGACAATGCCCTGCAGAGC
GGCAACAGCCAGGAGAGCGTGACCGAGCAGGACAGCAAGGACTCCACCTACAGCCTGAGC
AGCACCCTGACCCTGAGCAAGGCCGACTACGAGAAGCACAAGGTGTACGCCTGTGAGGTG
ACCCACCAGGGCCTGTCCAGCCCCGTGACCAAGAGCTTCAACCGGGGCGAGTGTGGATCC
GGCGTGCAGCTCCTGGAGAGCGGCGGAGGCCTGGTCCAGCCCGGCGGCAGCCTGAGGCTG
AGCTGCGCCGCCAGCGGCTTCACCTTCGCCTGGTATGATATGGGCTGGGTGAGGCAGGCC
CCCGGCAAGGGCCTGGAGTGGGTGTCCAGCATCGACTGGCACGGGGAGGTGACCTACTAC
GCCGACAGCGTGAAGGGCAGGTTCACCATCAGCAGGGACAACAGCAAGAACACCCTGTAC
CTGCAGATGAACAGCCTGAGGGCCGAGGACACCGCAGTGTACTACTGCGCCACCGCCGAG
GACGAACCCGGCTACGACTACTGGGGCCAGGGCACCCTGGTGACTGTGAGCAGC

**SEQ ID NO: 218:** PascoL-TVAAPS-474 Light chain

GACATCGTGCTGACCCAGAGCCCCTCTTCCCTGAGCGCAAGCGTGGGCGATAGGGTGACC
ATCACCTGCAAGGCCAGCCAGAGCGTGGACTACGACGGCGACAGCTACATGAACTGGTAC
CAGCAGAAGCCCGGCAAGGCCCCCAAACTGCTGATCTACGCCGCCAGCAACCTCGAGTCA
GGCATTCCCAGCAGGTTTAGCGGCAGCGGCAGCGGCACCGACTTCACCTTCACAATCAGC
AGCCTGCAGCCCGAGGACATCGCCACCTACTACTGCCAGCAGAGCAACGAGGACCCTCCC
ACCTTCGGACAGGGCACCAAGGTCGAGATCAAGCGTACGGTGGCCGCCCCCAGCGTGTTC
ATCTTCCCCCCCAGCGATGAGCAGCTGAAGAGCGGCACCGCCAGCGTGGTGTGTCTGCTG
AACAACTTCTACCCCCGGGAGGCCAAGGTGCAGTGGAAGGTGGACAATGCCCTGCAGAGC
GGCAACAGCCAGGAGAGCGTGACCGAGCAGGACAGCAAGGACTCCACCTACAGCCTGAGC
AGCACCCTGACCCTGAGCAAGGCCGACTACGAGAAGCACAAGGTGTACGCCTGTGAGGTG
ACCCACCAGGGCCTGTCCAGCCCCGTGACCAAGAGCTTCAACCGGGGCGAGTGCACCGTG
GCCGCCCCCTCGGGATCCGGCGTGCAGCTCCTGGAGAGCGGCGGAGGCCTGGTCCAGCCC

GGCGGCAGCCTGAGGCTGAGCTGCGCCGCCAGCGGCTTCACCTTCGCCTGGTATGATATG
GGCTGGGTGAGGCAGGCCCCCGGCAAGGGCCTGGAGTGGGTGTCCAGCATCGACTGGCAC
GGGGAGGTGACCTACTACGCCGACAGCGTGAAGGGCAGGTTCACCATCAGCAGGGACAAC
AGCAAGAACACCCTGTACCTGCAGATGAACAGCCTGAGGGCCGAGGACACCGCAGTGTAC
TACTGCGCCACCGCCGAGGACGAACCCGGCTACGACTACTGGGGCCAGGGCACCCTGGTG
ACTGTGAGCAGC

**SEQ ID NO: 219:** IL-5 mAb-G4S-DOM9-112-210 heavy chain

CAGGTGACCCTGAGGGAGAGCGGCCCCGCCCTGGTGAAGCCCACACAGACCCTCACTCTGAC
CTGCACCGTGAGCGGCTTCAGCCTGACCTCCTACAGCGTCCACTGGGTGAGGCAGCCCCCCG
GCAAGGGCCTGGAGTGGCTGGGCGTGATCTGGGCAAGCGGCGGCACCGACTACAACAGCGCC
CTGATGAGCAGGCTCTCCATCAGCAAGGACACCAGCCGGAACCAGGTGGTGCTGACCATGAC
CAACATGGACCCCGTGGACACCGCCACCTATTACTGCGCCAGGGACCCTCCCTCTAGCCTGC
TGAGGCTGGACTACTGGGGCAGGGGAACACTAGTGACCGTGTCCAGCGCCAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAACCGGTGACCGTGTCCTGGAACAGCGGAGCCCTGA
CCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAA
GCCCAGCAACACCAAGGTGGACAAGAAGGTGGAGCCCAAGAGCTGTGACAAGACCCACACCT
GCCCCCCCTGCCCTGCCCCCGAGCTGCTGGGAGGCCCCAGCGTGTTCCTGTTCCCCCCCAAG
CCTAAGGACACCCTGATGATCAGCAGAACCCCCGAGGTGACCTGTGTGGTGGTGGATGTGAG
CCACGAGGACCCTGAGGTGAAGTTCAACTGGTACGTGGACGGCGTGGAGGTGCACAATGCCA
AGACCAAGCCCAGGGAGGAGCAGTACAACAGCACCTACCGGGTGGTGTCCGTGCTGACCGTG
CTGCACCAGGATTGGCTGAACGGCAAGGAGTACAAGTGTAAGGTGTCCAACAAGGCCCTGCC
TGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAG
GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACAGCAAGCTGACCG
TGGACAAGAGCAGATGGCAGCAGGGCAACGTGTTCAGCTGCTCCGTGATGCACGAGGCCCTG
CACAATCACTACACCCAGAAGAGCCTGAGCCTGTCCCCTGGCAAGGGCGGCGGCGGATCCGA
AGTGCAGCTCCTGGAGAGCGGCGGCGGCCTGGTGCAGCCCGGCGGCAGCCTGAGGCTGAGCT
GCGCCGCTAGCGGCTTCACCTTCAGGAACTTCGGCATGGGCTGGGTCAGGCAGGCCCCCGGC
AAGGGCCTGGAGTGGGTCAGCTGGATCATCAGCTCCGGCACCGAGACCTACTACGCCGACAG
CGTGAAGGGCAGGTTCACCATCAGCCGCGACAACAGCAAGAACACCCTGTACCTGCAGATGA
ACAGCCTGAGGGCCGAGGACACCGCCGTCTACTACTGCGCCAAGAGCCTGGGCAGGTTCGAC
TACTGGGGACAGGGGACCCTGGTGACTGTGAGCAGC

**SEQ ID NO: 220:** IL-5 mAb-G4S-DOM10-53-474 light chain

GACATCGTGATGACCCAGTCTCCCGATTCACTGGCCGTGAGCCTGGGCGAGAGGGCCACC
ATCAACTGCAAGAGCAGCCAGAGCCTCCTGAACAGCGGCAACCAGAAGAACTACCTGGCC
TGGTACCAGCAGAAACCCGGCCAGCCCCCCAAGCTGCTGATCTATGGCGCCTCCACCAGG
GAGAGCGGCGTGCCAGACAGGTTTAGCGGCAGCGGCAGCGGCACCGACTTCACCCTGACA
ATCAGCAGCCTGCAGGCCGAGGACGTGGCCGTGTACTACTGCCAGAACGTCCACAGCTTC
CCCTTCACCTTCGGCGGGGGAACCAAGCTGGAGATCAAGCGTACGGTGGCCGCCCCCAGC
GTGTTCATCTTCCCCCCCAGCGATGAGCAGCTGAAGAGCGGCACCGCCAGCGTGGTGTGT
CTGCTGAACAACTTCTACCCCCGGGAGGCCAAGGTGCAGTGGAAGGTGGACAATGCCCTG
CAGAGCGGCAACAGCCAGGAGAGCGTGACCGAGCAGGACAGCAAGGACTCCACCTACAGC
CTGAGCAGCACCCTGACCCTGAGCAAGGCCGACTACGAGAAGCACAAGGTGTACGCCTGT
GAGGTGACCCACCAGGGCCTGTCCAGCCCCGTGACCAAGAGCTTCAACCGGGGCGAGTGC
GGCGGCGGCGGATCCGGCGTGCAGCTCCTGGAGAGCGGCGGAGGCCTGGTCCAGCCCGGC
GGCAGCCTGAGGCTGAGCTGCGCCGCCAGCGGCTTCACCTTCGCCTGGTATGATATGGGC
TGGGTGAGGCAGGCCCCCGGCAAGGGCCTGGAGTGGGTGTCCAGCATCGACTGGCACGGG
GAGGTGACCTACTACGCCGACAGCGTGAAGGGCAGGTTCACCATCAGCAGGGACAACAGC
AAGAACACCCTGTACCTGCAGATGAACAGCCTGAGGGCCGAGGACACCGCAGTGTACTAC
TGCGCCACCGCCGAGGACGAACCCGGCTACGACTACTGGGGCCAGGGCACCCTGGTGACT

GTGAGCAGC

**SEQ ID NO: 221: 586H-210 Heavy chain (GS removed)**

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCC
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC
GTGATGCACGAGGCCCTGCACAATCACTACACCCAGAAGAGCCTGAGCCTGTCCCCTGGCAA
GGAAGTGCAGCTCCTGGAGAGCGGCGGCGGCCTGGTGCAGCCCGGCGGCAGCCTGAGGCTGA
GCTGCGCCGCTAGCGGCTTCACCTTCAGGAACTTCGGCATGGGCTGGGTCAGGCAGGCCCCC
GGCAAGGGCCTGGAGTGGGTCAGCTGGATCATCAGCTCCGGCACCGAGACCTACTACGCCGA
CAGCGTGAAGGGCAGGTTCACCATCAGCCGCGACAACAGCAAGAACACCCTGTACCTGCAGA
TGAACAGCCTGAGGGCCGAGGACACCGCCGTCTACTACTGCGCCAAGAGCCTGGGCAGGTTC
GACTACTGGGGACAGGGGACCCTGGTGACTGTGAGCAGC

**SEQ ID NO: 222: 586H-TVAAPS-210 Heavy chain (GS removed)**

CAGGTGCAGCTCGTGCAGAGCGGCGCCGAAGTGAAAAAGCCCGGCAGCAGCGTGAAGGTGAG
CTGCAAGGCCTCCGGCTTCTACATCAAGGACACCTACATGCACTGGGTCAGGCAGGCTCCTG
GCCAGGGCCTGGAGTGGATGGGCACTATCGACCCCGCCAACGGCAACACCAAGTACGTGCCC
AAGTTCCAGGGCAGGGTGACCATCACCGCCGATGAGAGCACCAGCACCGCCTACATGGAACT
GAGCAGCCTGAGGTCTGAGGACACCGCCGTGTACTATTGCGCCAGGAGCATCTACGACGACT
ACCACTACGACGACTACTACGCCATGGACTACTGGGGACAGGGCACACTAGTGACCGTGTCT
AGCGCCAGCACCAAGGGCCCCAGCGTGTTCCCCCTGGCCCCCAGCAGCAAGAGCACCAGCGG
CGGCACAGCCGCCCTGGGCTGCCTGGTGAAGGACTACTTCCCCGAACCGGTGACCGTGTCCT
GGAACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTACAT
CTGTAACGTGAACCACAAGCCCAGCAACACCAAGGTGGACAAGAAGGTGGAGCCCAAGAGCT
GTGACAAGACCCACACCTGCCCCCCCTGCCCTGCCCCCGAGCTGCTGGGAGGCCCCAGCGTG
TTCCTGTTCCCCCCCAAGCCTAAGGACACCCTGATGATCAGCAGAACCCCCGAGGTGACCTG
TGTGGTGGTGGATGTGAGCCACGAGGACCCTGAGGTGAAGTTCAACTGGTACGTGGACGGCG
TGGAGGTGCACAATGCCAAGACCAAGCCCAGGGAGGAGCAGTACAACAGCACCTACCGGGTG
GTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCA
GAGAGCCCCAGGTGTACACCCTGCCCCCTAGCAGAGATGAGCTGACCAAGAACCAGGTGTCC
CTGACCTGCCTGGTGAAGGGCTTCTACCCCAGCGACATCGCCGTGGAGTGGGAGAGCAACGG
CCAGCCCGAGAACAACTACAAGACCACCCCCCCTGTGCTGGACAGCGATGGCAGCTTCTTCC
TGTACAGCAAGCTGACCGTGGACAAGAGCAGATGGCAGCAGGGCAACGTGTTCAGCTGCTCC

GTGATGCACGAGGCCCTGCACAATCACTACACCCAGAAGAGCCTGAGCCTGTCCCCTGGCAA
GACCGTGGCCGCCCCCTCGGAAGTGCAGCTCCTGGAGAGCGGCGGCGGCCTGGTGCAGCCCG
GCGGCAGCCTGAGGCTGAGCTGCGCCGCTAGCGGCTTCACCTTCAGGAACTTCGGCATGGGC
TGGGTCAGGCAGGCCCCCGGCAAGGGCCTGGAGTGGGTCAGCTGGATCATCAGCTCCGGCAC
CGAGACCTACTACGCCGACAGCGTGAAGGGCAGGTTCACCATCAGCCGCGACAACAGCAAGA
ACACCCTGTACCTGCAGATGAACAGCCTGAGGGCCGAGGACACCGCCGTCTACTACTGCGCC
AAGAGCCTGGGCAGGTTCGACTACTGGGGACAGGGGACCCTGGTGACTGTGAGCAGC

**SEQ ID NO: 223:** PascoH-474 Heavy Chain (GS removed)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGGGCGTGCAGCTCCTGGAGAGCGGCGGA
GGCCTGGTCCAGCCCGGCGGCAGCCTGAGGCTGAGCTGCGCCGCCAGCGGCTTCACCTTC
GCCTGGTATGATATGGGCTGGGTGAGGCAGGCCCCCGGCAAGGGCCTGGAGTGGGTGTCC
AGCATCGACTGGCACGGGGAGGTGACCTACTACGCCGACAGCGTGAAGGGCAGGTTCACC
ATCAGCAGGGACAACAGCAAGAACACCCTGTACCTGCAGATGAACAGCCTGAGGGCCGAG
GACACCGCAGTGTACTACTGCGCCACCGCCGAGGACGAACCCGGCTACGACTACTGGGGC
CAGGGCACCCTGGTGACTGTGAGCAGC

**SEQ ID NO: 224:** PascoH-TVAAPS-474 Heavy Chain (GS removed)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC

AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGCGTGCAG
CTCCTGGAGAGCGGCGGAGGCCTGGTCCAGCCCGGCGGCAGCCTGAGGCTGAGCTGCGCC
GCCAGCGGCTTCACCTTCGCCTGGTATGATATGGGCTGGGTGAGGCAGGCCCCCGGCAAG
GGCCTGGAGTGGGTGTCCAGCATCGACTGGCACGGGGAGGTGACCTACTACGCCGACAGC
GTGAAGGGCAGGTTCACCATCAGCAGGGACAACAGCAAGAACACCCTGTACCTGCAGATG
AACAGCCTGAGGGCCGAGGACACCGCAGTGTACTACTGCGCCACCGCCGAGGACGAACCC
GGCTACGACTACTGGGGCCAGGGCACCCTGGTGACTGTGAGCAGC

**SEQ ID NO: 225:** PascoH-ASTKGPT-474 Heavy Chain (second GS removed)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGGGATCAGCCAGCACCAAGGGCCCCACG
GGCGTGCAGCTCCTGGAGAGCGGCGGAGGCCTGGTCCAGCCCGGCGGCAGCCTGAGGCTG
AGCTGCGCCGCCAGCGGCTTCACCTTCGCCTGGTATGATATGGGCTGGGTGAGGCAGGCC
CCCGGCAAGGGCCTGGAGTGGGTGTCCAGCATCGACTGGCACGGGGAGGTGACCTACTAC
GCCGACAGCGTGAAGGGCAGGTTCACCATCAGCAGGGACAACAGCAAGAACACCCTGTAC
CTGCAGATGAACAGCCTGAGGGCCGAGGACACCGCAGTGTACTACTGCGCCACCGCCGAG
GACGAACCCGGCTACGACTACTGGGGCCAGGGCACCCTGGTGACTGTGAGCAGC

**SEQ ID NO: 226: Heavy chain of anti-IGF-1R-antibody H0L0 with DOM15-26-593 fused at C-terminus with TVAAPSGS linker**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC

AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTgTAa
CgTgAACcACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGACCGT
GGCCGCCCCCTCGGGATCCGAGGTGCAGCTCCTGGTCAGCGGCGGCGGCCTGGTCCAGCCCG
GAGGCTCACTGAGGCTGAGCTGCGCCGCTAGCGGCTTCACCTTCAAGGCCTACCCCATGATG
TGGGTCAGGCAGGCCCCCGGCAAAGGCCTGGAGTGGGTGTCTGAGATCAGCCCCAGCGGCAG
CTACACCTACTACGCCGACAGCGTGAAGGGCAGGTTCACCATCAGCAGGGACAACAGCAAGA
ACACCCTGTACCTGCAGATGAACTCTCTGAGGGCCGAGGACACCGCCGTGTACTACTGCGCC
AAGGACCCCAGGAAGCTGGACTATTGGGGCCAGGGCACTCTGGTGACCGTGAGCAGC

**SEQ ID NO: 227: IGF1RmAb-GS-DOM15-26-593 Heavy chain**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTgTAa
CgTgAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGGGATC
CGAGGTGCAGCTCCTGGTCAGCGGCGGCGGCCTGGTCCAGCCCGGAGGCTCACTGAGGCTGA
GCTGCGCCGCTAGCGGCTTCACCTTCAAGGCCTACCCCATGATGTGGGTCAGGCAGGCCCCC
GGCAAAGGCCTGGAGTGGGTGTCTGAGATCAGCCCCAGCGGCAGCTACACCTACTACGCCGA
CAGCGTGAAGGGCAGGTTCACCATCAGCAGGGACAACAGCAAGAACACCCTGTACCTGCAGA
TGAACTCTCTGAGGGCCGAGGACACCGCCGTGTACTACTGCGCCAAGGACCCCAGGAAGCTG
GACTATTGGGGCCAGGGCACTCTGGTGACCGTGAGCAGC

**SEQ ID NO: 228: anti-IGF-1R antibody Heavy chain**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG

GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAG

**SEQ ID NO: 229: Light chain of anti-IGF-1R antibody H0L0 with DOM15-26-593 fused at C-terminus with TVAAPSGS linker**

GACATCGTGATGACCCAGAGCCCCCTGAGCCTGCCCGTGACCCCTGGCGAGCCCGCCAGCAT
CAGCTGCAGAAGCAGCCAGAGCATCGTCCAGAGCAACGGCGACACCTACCTGGAATGGTATC
TGCAGAAGCCCGGCCAGTCCCCCCAGCTGCTGATCTACAGAGTGAGCAACCGGTTCAGCGGC
GTGCCCGACAGATTCAGCGGCAGCGGCTCCGGCACCGACTTCACCCTGAAGATCAGCCGGGT
GGAGGCCGAGGACGTGGGCGTGTACTACTGCTTTCAAGGCAGCCACGTGCCCTACACCTTCG
GCCAGGGCACCAAGCTGGAAATCAAGCGTACGGTGGCCGCCCCCAGCGTGTTCATCTTCCCC
CCCAGCGATGAGCAGCTGAAGAGCGGCACCGCCAGCGTGGTGTGTCTGCTGAACAACTTCTA
CCCCCGGGAGGCCAAGGTGCAGTGGAAGGTGGACAATGCCCTGCAGAGCGGCAACAGCCAGG
AGAGCGTGACCGAGCAGGACAGCAAGGACTCCACCTACAGCCTGAGCAGCACCCTGACCCTG
AGCAAGGCCGACTACGAGAAGCACAAGGTGTACGCCTGTGAGGTGACCCACCAGGGCCTGTC
CAGCCCCGTGACCAAGAGCTTCAACCGGGGCGAGTGCACCGTGGCCGCCCCCTCGGGATCCG
AGGTGCAGCTCCTGGTCAGCGGCGGCGGCCTGGTCCAGCCCGGAGGCTCACTGAGGCTGAGC
TGCGCCGCTAGCGGCTTCACCTTCAAGGCCTACCCCATGATGTGGGTCAGGCAGGCCCCCGG
CAAAGGCCTGGAGTGGGTGTCTGAGATCAGCCCCAGCGGCAGCTACACCTACTACGCCGACA
GCGTGAAGGGCAGGTTCACCATCAGCAGGGACAACAGCAAGAACACCCTGTACCTGCAGATG
AACTCTCTGAGGGCCGAGGACACCGCCGTGTACTACTGCGCCAAGGACCCCAGGAAGCTGGA
CTATTGGGGCCAGGGCACTCTGGTGACCGTGAGCAGC

**SEQ ID NO: 230: Light chain of anti-IGF-1R antibody H0L0 with DOM15-26-593 fused at C-terminus with GS linker**

GACATCGTGATGACCCAGAGCCCCCTGAGCCTGCCCGTGACCCCTGGCGAGCCCGCCAGCAT
CAGCTGCAGAAGCAGCCAGAGCATCGTCCAGAGCAACGGCGACACCTACCTGGAATGGTATC
TGCAGAAGCCCGGCCAGTCCCCCCAGCTGCTGATCTACAGAGTGAGCAACCGGTTCAGCGGC
GTGCCCGACAGATTCAGCGGCAGCGGCTCCGGCACCGACTTCACCCTGAAGATCAGCCGGGT
GGAGGCCGAGGACGTGGGCGTGTACTACTGCTTTCAAGGCAGCCACGTGCCCTACACCTTCG
GCCAGGGCACCAAGCTGGAAATCAAGCGTACGGTGGCCGCCCCCAGCGTGTTCATCTTCCCC
CCCAGCGATGAGCAGCTGAAGAGCGGCACCGCCAGCGTGGTGTGTCTGCTGAACAACTTCTA
CCCCCGGGAGGCCAAGGTGCAGTGGAAGGTGGACAATGCCCTGCAGAGCGGCAACAGCCAGG
AGAGCGTGACCGAGCAGGACAGCAAGGACTCCACCTACAGCCTGAGCAGCACCCTGACCCTG
AGCAAGGCCGACTACGAGAAGCACAAGGTGTACGCCTGTGAGGTGACCCACCAGGGCCTGTC
CAGCCCCGTGACCAAGAGCTTCAACCGGGGCGAGTGTGGATCCGAGGTGCAGCTCCTGGTCA

GCGGCGGCGGCCTGGTCCAGCCCGGAGGCTCACTGAGGCTGAGCTGCGCCGCTAGCGGCTTC
ACCTTCAAGGCCTACCCCATGATGTGGGTCAGGCAGGCCCCCGGCAAAGGCCTGGAGTGGGT
GTCTGAGATCAGCCCCAGCGGCAGCTACACCTACTACGCCGACAGCGTGAAGGGCAGGTTCA
CCATCAGCAGGGACAACAGCAAGAACACCCTGTACCTGCAGATGAACTCTCTGAGGGCCGAG
GACACCGCCGTGTACTACTGCGCCAAGGACCCCAGGAAGCTGGACTATTGGGGCCAGGGCAC
TCTGGTGACCGTGAGCAGC

**SEQ ID NO: 231: anti-IGF-1R antibody Light chain**

GACATCGTGATGACCCAGAGCCCCCTGAGCCTGCCCGTGACCCCTGGCGAGCCCGCCAGCAT
CAGCTGCAGAAGCAGCCAGAGCATCGTCCAGAGCAACGGCGACACCTACCTGGAATGGTATC
TGCAGAAGCCCGGCCAGTCCCCCCAGCTGCTGATCTACAGAGTGAGCAACCGGTTCAGCGGC
GTGCCCGACAGATTCAGCGGCAGCGGCTCCGGCACCGACTTCACCCTGAAGATCAGCCGGGT
GGAGGCCGAGGACGTGGGCGTGTACTACTGCTTTCAAGGCAGCCACGTGCCCTACACCTTCG
GCCAGGGCACCAAGCTGGAAATCAAGCGTACGGTGGCCGCCCCCAGCGTGTTCATCTTCCCC
CCCAGCGATGAGCAGCTGAAGAGCGGCACCGCCAGCGTGGTGTGTCTGCTGAACAACTTCTA
CCCCCGGGAGGCCAAGGTGCAGTGGAAGGTGGACAATGCCCTGCAGAGCGGCAACAGCCAGG
AGAGCGTGACCGAGCAGGACAGCAAGGACTCCACCTACAGCCTGAGCAGCACCCTGACCCTG
AGCAAGGCCGACTACGAGAAGCACAAGGTGTACGCCTGTGAGGTGACCCACCAGGGCCTGTC
CAGCCCCGTGACCAAGAGCTTCAACCGGGGCGAGTGC

**SEQ ID NO: 232: anti-IGF1R Heavy Chain-GS-TLPC**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGGGATC
CGACGGCGGCGGCATTAGGAGGAGCATGAGCGGCACCTGGTACCTGAAGGCCATGACCGTGG
ATAGGGAGTTCCCCGAGATGAACCTGGAGAGCGTGACCCCCATGACACTGACCCTGCTCAAG
GGCCACAACCTGGAGGCCAAGGTCACCATGCTGATCTCAGGCAGGTGCCAGGAGGTGAAGGC
AGTGCTGGGCAGGACCAAGGAGAGGAAGAAGTACACCGCCGACGGGGGCAAGCACGTGGCCT
ATATCATCCCCAGCGCCGTGAGGGACCACGTGATCTTCTACAGCGAGGGCCAGCTCCACGGA
AAGCCCGTGAGAGGCGTGAAGCTGGTGGGCAGGGACCCCAAGAACAACCTGGAGGCCCTGGA
GGACTTCGAAAAAGCCGCAGGCGCCAGGGGCCTGTCCACTGAGAGCATCCTGATCCCTAGGC
AGAGCGAGACCTGCAGCCCCGGC

**SEQ ID NO: 233: anti-IGF1R Heavy Chain-GS-CT01 adnectin**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG

GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGGGATC
CGAGGTGGTGGCCGCCACCCCCACCAGCCTGCTGATTTCCTGGAGGCACCCCCACTTCCCCA
CACGCTACTACAGGATCACCTACGGCGAGACCGGCGGCAACAGCCCCGTGCAGGAGTTCACC
GTGCCCCTGCAGCCTCCCACTGCCACCATCAGCGGCCTCAAGCCCGGCGTGGACTACACCAT
CACCGTGTACGCCGTCACCGACGGAAGGAACGGCAGGCTGCTGAGCATCCCCATCAGCATCA
ACTACAGGACC

**SEQ ID NO: 234: anti-IGF1R Heavy Chain-TVAAPSGS-TLPC**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGACCGT
GGCCGCCCCCTCGGGATCCGACGGCGGCGGCATTAGGAGGAGCATGAGCGGCACCTGGTACC
TGAAGGCCATGACCGTGGATAGGGAGTTCCCCGAGATGAACCTGGAGAGCGTGACCCCCATG
ACACTGACCCTGCTCAAGGGCCACAACCTGGAGGCCAAGGTCACCATGCTGATCTCAGGCAG
GTGCCAGGAGGTGAAGGCAGTGCTGGGCAGGACCAAGGAGAGGAAGAAGTACACCGCCGACG
GGGGCAAGCACGTGGCCTATATCATCCCCAGCGCCGTGAGGGACCACGTGATCTTCTACAGC
GAGGGCCAGCTCCACGGAAAGCCCGTGAGAGGCGTGAAGCTGGTGGGCAGGGACCCCAAGAA

CAACCTGGAGGCCCTGGAGGACTTCGAAAAAGCCGCAGGCGCCAGGGGCCTGTCCACTGAGA
GCATCCTGATCCCTAGGCAGAGCGAGACCTGCAGCCCCGGC

**SEQ ID NO: 235: anti-IGF1R Heavy Chain-GS-AFFI**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGGGATC
CGTGGACAACAAGTTCAACAAGGAGCTGAGGCAGGCCTACTGGGAGATCCAGGCCCTGCCCA
ATCTGAACTGGACCCAGAGCAGGGCCTTCATCAGGAGCCTGTACGACGACCCCAGCCAGAGC
GCTAACCTCCTGGCCGAGGCCAAAAAGCTGAACGACGCCCAGGCCCCCAAG

**SEQ ID NO: 236 anti-IGF1R Heavy Chain-TVAAPSGS-AFFI**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGACCGT
GGCCGCCCCCTCGGGATCCGTGGACAACAAGTTCAACAAGGAGCTGAGGCAGGCCTACTGGG
AGATCCAGGCCCTGCCCAATCTGAACTGGACCCAGAGCAGGGCCTTCATCAGGAGCCTGTAC

GACGACCCCAGCCAGAGCGCTAACCTCCTGGCCGAGGCCAAAAAGCTGAACGACGCCCAGGC
CCCCAAG

**SEQ ID NO: 237: anti-IGF1 R Heavy Chain-GS-DRPN**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGGGATC
CGACCTGGGGAAAAAGCTGCTTGAAGCCGCTAGGGCAGGACAGGATGACGAGGTGAGGATTC
TGATGGCAAATGGCGCCGACGTCAATGCCAAAGACGAGTACGGCCTCACCCCTCTTTATCTG
GCCACTGCACACGGACACTTGGAGATCGTGGAGGTGCTGCTCAAGAACGGAGCTGATGTGAA
CGCTGTGGACGCTATTGGGTTCACACCCCTTCACCTCGCAGCCTTTATTGGCCACCTGGAGA
TCGCCGAAGTTCTCCTGAAACACGGCGCAGACGTCAACGCACAGGATAAGTTCGGGAAGACC
GCCTTCGACATCAGCATCGGCAATGGGAACGAGGATCTGGCCGAGATCCTGCAGAAGCTG

**SEQ ID NO: 238: anti-IGF1R Heavy Chain-TVAAPSGS-DRPN**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG

CACGAGGCCCTGCACAATCACTACACCCAGAAGAGCCTGAGCCTGTCCCCTGGCAAGACCGT
GGCCGCCCCCTCGGGATCCGACCTGGGGAAAAAGCTGCTTGAAGCCGCTAGGGCAGGACAGG
ATGACGAGGTGAGGATTCTGATGGCAAATGGCGCCGACGTCAATGCCAAAGACGAGTACGGC
CTCACCCCTCTTTATCTGGCCACTGCACACGGACACTTGGAGATCGTGGAGGTGCTGCTCAA
GAACGGAGCTGATGTGAACGCTGTGGACGCTATTGGGTTCACACCCCTTCACCTCGCAGCCT
TTATTGGCCACCTGGAGATCGCCGAAGTTCTCCTGAAACACGGCGCAGACGTCAACGCACAG
GATAAGTTCGGGAAGACCGCCTTCGACATCAGCATCGGCAATGGGAACGAGGATCTGGCCGA
GATCCTGCAGAAGCTG

**SEQ ID NO: 239: Anti IL-4 heavy Chain-GS-anti RNAse A camelid$V_{HH}$**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGGGATCCCAGGT
GCAGCTGGTGGAGTCTGGGGGAGGCTTGGTGCAGGCTGGGGGGTCTCTGAGACTCTCCTGTG
CAGCCTCTGGATACGCATACACTTACATCTACATGGGCTGGTTCCGCCAGGCTCCAGGGAAA
GAGCGTGAGGGGGTCGCAGCTATGGATAGTGGTGGTGGTGGCACACTCTACGCCGACTCCGT
GAAGGGCCGATTCACCATCTCCCGCGACAAAGGCAAGAACACGGTGTATCTGCAAATGGACA
GCCTGAAACCTGAGGACACGGCCACGTATTACTGTGCTGCAGGTGGCTACGAGCTGCGTGAC
CGGACATATGGGCAGTGGGGCCAGGGGACCCAGGTCACCGTCTCCTCA

**SEQ ID NO: 240: Anti IL-4 heavy Chain-GS-NARV**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA

TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGGGATCCGCGCG
CGTCGACCAGACGCCGCGCAGCGTCACGAAGGAAACCGGCGAGTCCCTCACCATCAACTGCG
TGCTGCGGGATGCCTCCTACGCCCTGGGCAGCACATGTTGGTACAGAAAGAAGAGCGGGGAA
GGCAACGAGGAGTCCATCTCCAAGGGGGGAAGATACGTCGAGACCGTGAACAGCGGAAGCAA
GAGCTTCAGCCTGCGGATCAACGACCTCACCGTCGAGGACGGGGGCACCTACCGTTGCGGTC
TGGGCGTGGCCGGCGGCTATTGCGATTACGCCCTGTGCAGTAGCCGGTATGCTGAGTGCGGC
GACGGCACCGCTGTGACCGTGAAC

**SEQ ID NO: 241 anti-IGF1R Heavy Chain-TVAAPSGS-CT01 adnectin**

CAGGTGCAGCTGGTGCAGAGCGGAGCCGAGGTGAAGAAGCCTGGCGCCAGCGTCAAGGTGTC
CTGCAAGGCCAGCGGCTACACCTTCACCGACTACTACATGAACTGGGTGCGGCAGGCCCCAG
GCCAGGGACTGGAATGGATGGGCAACATCAACCCCAACAACGGCGGCACCAACTACAACCAG
AAGTTCAAGGACCGGGTCACCATGACCACCGACACCAGCACCAGCACCGCCTACATGGAACT
GCGGAGCCTGAGAAGCGACGACACCGCCGTGTACTACTGCGCCCGGTGGATCCTGTACTACG
GCCGGTCCAAGTGGTACTTCGACGTGTGGGGCAGGGGCACACTAGTGACCGTGTCCAGCGCC
AGCACCAAGGGCCCCAGCGTGTTCCCCCTGGCCCCCAGCAGCAAGAGCACCAGCGGCGGCAC
AGCCGCCCTGGGCTGCCTGGTGAAGGACTACTTCCCCGAACCGGTGACCGTGTCCTGGAACA
GCGGAGCCCTGACCAGCGGCGTGCACACCTTCCCCGCCGTGCTGCAGAGCAGCGGCCTGTAC
AGCCTGAGCAGCGTGGTGACCGTGCCCAGCAGCAGCCTGGGCACCCAGACCTACATCTGTAA
CGTGAACCACAAGCCCAGCAACACCAAGGTGGACAAGAAGGTGGAGCCCAAGAGCTGTGACA
AGACCCACACCTGCCCCCCCTGCCCTGCCCCCGAGCTGCTGGGAGGCCCCAGCGTGTTCCTG
TTCCCCCCCAAGCCTAAGGACACCCTGATGATCAGCAGAACCCCCGAGGTGACCTGTGTGGT
GGTGGATGTGAGCCACGAGGACCCTGAGGTGAAGTTCAACTGGTACGTGGACGGCGTGGAGG
TGCACAATGCCAAGACCAAGCCCAGGGAGGAGCAGTACAACAGCACCTACCGGGTGGTGTCC
GTGCTGACCGTGCTGCACCAGGATTGGCTGAACGGCAAGGAGTACAAGTGTAAGGTGTCCAA
CAAGGCCCTGCCTGCCCCTATCGAGAAAACCATCAGCAAGGCCAAGGGCCAGCCCAGAGAGC
CCCAGGTGTACACCCTGCCCCCTAGCAGAGATGAGCTGACCAAGAACCAGGTGTCCCTGACC
TGCCTGGTGAAGGGCTTCTACCCCAGCGACATCGCCGTGGAGTGGGAGAGCAACGGCCAGCC
CGAGAACAACTACAAGACCACCCCCCCTGTGCTGGACAGCGATGGCAGCTTCTTCCTGTACA
GCAAGCTGACCGTGGACAAGAGCAGATGGCAGCAGGGCAACGTGTTCAGCTGCTCCGTGATG
CACGAGGCCCTGCACAATCACTACACCCAGAAGAGCCTGAGCCTGTCCCCTGGCAAGACCGT
GGCCGCCCCCTCGGGATCCGAGGTGGTGGCCGCCACCCCCACCAGCCTGCTGATTTCCTGGA
GGCACCCCCACTTCCCCACACGCTACTACAGGATCACCTACGGCGAGACCGGCGGCAACAGC
CCCGTGCAGGAGTTCACCGTGCCCCTGCAGCCTCCCACTGCCACCATCAGCGGCCTCAAGCC
CGGCGTGGACTACACCATCACCGTGTACGCCGTCACCGACGGAAGGAACGGCAGGCTGCTGA
GCATCCCCATCAGCATCAACTACAGGACC

**SEQ ID NO: 242: Erbitux Heavy chain-RS-CT01 adnectin**

CAGGTGCAGCTGAAGCAGAGCGGCCCTGGCCTGGTGCAGCCCTCTCAGAGCCTGAGCATCAC
CTGTACCGTGAGCGGCTTCAGCCTGACCAATTACGGCGTGCATTGGGTGCGGCAGTCTCCAG
GCAAGGGCCTGGAATGGCTGGGAGTGATCTGGTCCGGCGGCAACACCGACTACAACACCCCC
TTCACCAGCAGACTGAGCATCAACAAGGACAACAGCAAGAGCCAGGTGTTCTTCAAGATGAA
CAGCCTGCAGAGCAACGACACCGCCATCTACTATTGTGCCAGGGCCCTGACCTACTACGACT
ACGAGTTCGCCTACTGGGGCCAGGGCACCCTGGTGACCGTGAGCGCCGCTAGCACCAAGGGC
CCCAGCGTGTTCCCCCTGGCCCCCAGCAGCAAGAGCACCAGCGGCGGCACAGCCGCCCTGGG

CTGCCTGGTGAAGGACTACTTCCCCGAGCCTGTGACCGTGTCCTGGAATAGCGGAGCCCTGA
CCTCCGGCGTGCACACCTTCCCCGCCGTGCTGCAGAGCAGCGGCCTGTACTCCCTGAGCAGC
GTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCT
GCCCCCCCTGCCCTGCCCCCGAGCTGCTGGGCGGACCTAGCGTGTTCCTGTTCCCCCCCAAG
CCTAAGGACACCCTGATGATCAGCAGGACCCCCGAAGTGACCTGCGTGGTGGTGGATGTGAG
CCACGAGGACCCTGAAGTGAAGTTCAACTGGTACGTGGACGGCGTGGAAGTGCACAACGCCA
AGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCC
TGCCCCTATCGAGAAAACCATCAGCAAGGCCAAGGGCCAGCCTAGAGAGCCCCAGGTCTACA
CCCTGCCTCCCTCCAGAGATGAGCTGACCAAGAACCAGGTGTCCCTGACCTGTCTGGTGAAG
GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTG
CACAATCACTACACCCAGAAGAGTCTGAGCCTGTCTCCTGGCAAGAGATCCGAGGTGGTGGC
CGCCACCCCCACCAGCCTGCTGATTTCCTGGAGGCACCCCCACTTCCCCACACGCTACTACA
GGATCACCTACGGCGAGACCGGCGGCAACAGCCCCGTGCAGGAGTTCACCGTGCCCCTGCAG
CCTCCCACTGCCACCATCAGCGGCCTCAAGCCCGGCGTGGACTACACCATCACCGTGTACGC
CGTCACCGACGGAAGGAACGGCAGGCTGCTGAGCATCCCCATCAGCATCAACTACAGGACC

**SEQ ID NO: 243: Erbitux Light Chain**

GACATCCTGCTGACCCAGAGCCCCGTGATCCTGAGCGTGAGCCCTGGCGAGAGAGTGAGCTT
CAGCTGCCGGGCCAGCCAGAGCATCGGCACCAACATCCACTGGTATCAGCAGCGGACCAACG
GCAGCCCCAGGCTGCTGATCAAGTACGCCAGCGAGTCCATCAGCGGCATCCCCAGCCGGTTC
AGCGGCAGCGGCTCCGGCACCGACTTCACCCTGAGCATCAACAGCGTGGAGAGCGAGGATAT
CGCCGACTACTACTGCCAGCAGAACAACAACTGGCCCACCACCTTCGGAGCCGGCACCAAGC
TGGAACTGAAGCGTACGGTGGCCGCCCCCAGCGTGTTCATCTTCCCCCCCAGCGATGAGCAG
CTCAAGAGCGGCACCGCCAGCGTGGTGTGTCTGCTGAACAACTTCTACCCCCGGGAGGCCAA
AGTGCAGTGGAAAGTGGACAACGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTAC
GAGAAGCACAAAGTGTACGCCTGCGAAGTGACCCACCAGGGCCTGTCCAGCCCCGTGACCAA
GAGCTTCAACCGGGGCGAGTGC

**SEQ ID NO: 244: Erbitux Light chain-RS-CT01 adnectin**

GACATCCTGCTGACCCAGAGCCCCGTGATCCTGAGCGTGAGCCCTGGCGAGAGAGTGAGCTT
CAGCTGCCGGGCCAGCCAGAGCATCGGCACCAACATCCACTGGTATCAGCAGCGGACCAACG
GCAGCCCCAGGCTGCTGATCAAGTACGCCAGCGAGTCCATCAGCGGCATCCCCAGCCGGTTC
AGCGGCAGCGGCTCCGGCACCGACTTCACCCTGAGCATCAACAGCGTGGAGAGCGAGGATAT
CGCCGACTACTACTGCCAGCAGAACAACAACTGGCCCACCACCTTCGGAGCCGGCACCAAGC
TGGAACTGAAGCGTACGGTGGCCGCCCCCAGCGTGTTCATCTTCCCCCCCAGCGATGAGCAG
CTCAAGAGCGGCACCGCCAGCGTGGTGTGTCTGCTGAACAACTTCTACCCCCGGGAGGCCAA
AGTGCAGTGGAAAGTGGACAACGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTAC
GAGAAGCACAAAGTGTACGCCTGCGAAGTGACCCACCAGGGCCTGTCCAGCCCCGTGACCAA
GAGCTTCAACCGAGGCGAGTGCAGATCCGAGGTGGTGGCCGCCACCCCCACCAGCCTGCTGA
TTTCCTGGAGGCACCCCCACTTCCCCACACGCTACTACAGGATCACCTACGGCGAGACCGGC
GGCAACAGCCCCGTGCAGGAGTTCACCGTGCCCCTGCAGCCTCCCACTGCCACCATCAGCGG
CCTCAAGCCCGGCGTGGACTACACCATCACCGTGTACGCCGTCACCGACGGAAGGAACGGCA
GGCTGCTGAGCATCCCCATCAGCATCAACTACAGGACC

**SEQ ID** NO: 245: Erbitux **Heavy** Chain

CAGGTGCAGCTGAAGCAGAGCGGCCCTGGCCTGGTGCAGCCCTCTCAGAGCCTGAGCATCAC
CTGTACCGTGAGCGGCTTCAGCCTGACCAATTACGGCGTGCATTGGGTGCGGCAGTCTCCAG

GCAAGGGCCTGGAATGGCTGGGAGTGATCTGGTCCGGCGGCAACACCGACTACAACACCCCC
TTCACCAGCAGACTGAGCATCAACAAGGACAACAGCAAGAGCCAGGTGTTCTTCAAGATGAA
CAGCCTGCAGAGCAACGACACCGCCATCTACTATTGTGCCAGGGCCCTGACCTACTACGACT
ACGAGTTCGCCTACTGGGGCCAGGGCACCCTGGTGACCGTGAGCGCCGCTAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAGCCTGTGACCGTGTCCTGGAATAGCGGAGCCCTGA
CCTCCGGCGTGCACACCTTCCCCGCCGTGCTGCAGAGCAGCGGCCTGTACTCCCTGAGCAGC
GTGGTGACCGTGCCCAGCAGCAGCCTGGGCACCCAGACCTACATCTGCAACGTGAACCACAA
GCCCAGCAACACCAAAGTGGACAAGAAAGTGGAGCCCAAGAGCTGCGATAAGACCCACACCT
GCCCCCCCTGCCCTGCCCCCGAGCTGCTGGGCGGACCTAGCGTGTTCCTGTTCCCCCCCAAG
CCTAAGGACACCCTGATGATCAGCAGGACCCCCGAAGTGACCTGCGTGGTGGTGGATGTGAG
CCACGAGGACCCTGAAGTGAAGTTCAACTGGTACGTGGACGGCGTGGAAGTGCACAACGCCA
AGACCAAGCCCAGAGAGGAGCAGTACAACAGCACCTACCGCGTGGTGTCTGTGCTGACCGTG
CTGCACCAGGATTGGCTGAACGGCAAGGAGTACAAGTGCAAAGTGAGCAACAAGGCCCTGCC
TGCCCCTATCGAGAAAACCATCAGCAAGGCCAAGGGCCAGCCTAGAGAGCCCCAGGTCTACA
CCCTGCCTCCCTCCAGAGATGAGCTGACCAAGAACCAGGTGTCCCTGACCTGTCTGGTGAAG
GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACTCCAAGCTGACCG
TGGACAAGAGCAGATGGCAGCAGGGCAACGTGTTCAGCTGCAGCGTGATGCACGAGGCCCTG
CACAATCACTACACCCAGAAGAGTCTGAGCCTGTCCCCTGGCAAG

**SEQ ID NO: 246: CT01 adnectin-GSTG- Erbitux Heavy Chain**

GAGGTGGTGGCCGCCACCCCCACCAGCCTGCTGATTTCCTGGAGGCACCCCCACTTCCCCAC
ACGCTACTACAGGATCACCTACGGCGAGACCGGCGGCAACAGCCCCGTGCAGGAGTTCACCG
TGCCCCTGCAGCCTCCCACTGCCACCATCAGCGGCCTCAAGCCCGGCGTGGACTACACCATC
ACCGTGTACGCCGTCACCGACGGAAGGAACGGCAGGCTGCTGAGCATCCCCATCAGCATCAA
CTACAGGACCGGATCCACCGGCCAGGTGCAGCTGAAGCAGAGCGGCCCTGGCCTGGTGCAGC
CCTCTCAGAGCCTGAGCATCACCTGTACCGTGAGCGGCTTCAGCCTGACCAATTACGGCGTG
CATTGGGTGCGGCAGTCTCCAGGCAAGGGCCTGGAATGGCTGGGAGTGATCTGGTCCGGCGG
CAACACCGACTACAACACCCCCTTCACCAGCAGACTGAGCATCAACAAGGACAACAGCAAGA
GCCAGGTGTTCTTCAAGATGAACAGCCTGCAGAGCAACGACACCGCCATCTACTATTGTGCC
AGGGCCCTGACCTACTACGACTACGAGTTCGCCTACTGGGGCCAGGGCACCCTGGTGACCGT
GAGCGCCGCTAGCACCAAGGGCCCCAGCGTGTTCCCCCTGGCCCCCAGCAGCAAGAGCACCA
GCGGCGGCACAGCCGCCCTGGGCTGCCTGGTGAAGGACTACTTCCCCGAGCCTGTGACCGTG
TCCTGGAATAGCGGAGCCCTGACCTCCGGCGTGCACACCTTCCCCGCCGTGCTGCAGAGCAG
CGGCCTGTACTCCCTGAGCAGCGTGGTGACCGTGCCCAGCAGCAGCCTGGGCACCCAGACCT
ACATCTGCAACGTGAACCACAAGCCCAGCAACACCAAAGTGGACAAGAAAGTGGAGCCCAAG
AGCTGCGATAAGACCCACACCTGCCCCCCCTGCCCTGCCCCCGAGCTGCTGGGCGGACCTAG
CGTGTTCCTGTTCCCCCCCAAGCCTAAGGACACCCTGATGATCAGCAGGACCCCCGAAGTGA
CCTGCGTGGTGGTGGATGTGAGCCACGAGGACCCTGAAGTGAAGTTCAACTGGTACGTGGAC
GGCGTGGAAGTGCACAACGCCAAGACCAAGCCCAGAGAGGAGCAGTACAACAGCACCTACCG
CGTGGTGTCTGTGCTGACCGTGCTGCACCAGGATTGGCTGAACGGCAAGGAGTACAAGTGCA
AAGTGAGCAACAAGGCCCTGCCTGCCCCTATCGAGAAAACCATCAGCAAGGCCAAGGGCCAG
CCTAGAGAGCCCCAGGTCTACACCCTGCCTCCCTCCAGAGATGAGCTGACCAAGAACCAGGT
GTCCCTGACCTGTCTGGTGAAGGGCTTCTACCCCAGCGACATCGCCGTGGAGTGGGAGAGCA
ACGGCCAGCCCGAGAACAACTACAAGACCACCCCCCCTGTGCTGGACAGCGATGGCAGCTTC
TTCCTGTACTCCAAGCTGACCGTGGACAAGAGCAGATGGCAGCAGGGCAACGTGTTCAGCTG
CAGCGTGATGCACGAGGCCCTGCACAATCACTACACCCAGAAGAGTCTGAGCCTGTCCCCTG
GCAAG

**SEQ ID NO: 247: CT01 adnectin-STG-Erbitux Light Chain**

GAGGTGGTGGCCGCCACCCCCACCAGCCTGCTGATTTCCTGGAGGCACCCCCACTTCCCCAC
ACGCTACTACAGGATCACCTACGGCGAGACCGGCGGCAACAGCCCCGTGCAGGAGTTCACCG
TGCCCCTGCAGCCTCCCACTGCCACCATCAGCGGCCTCAAGCCCGGCGTGGACTACACCATC
ACCGTGTACGCCGTCACCGACGGAAGGAACGGCAGGCTGCTGAGCATCCCCATCAGCATCAA

CTACAGGACGTCGACCGGTGACATCCTGCTGACCCAGAGCCCCGTGATCCTGAGCGTGAGCC
CTGGCGAGAGAGTGAGCTTCAGCTGCCGGGCCAGCCAGAGCATCGGCACCAACATCCACTGG
TATCAGCAGCGGACCAACGGCAGCCCCAGGCTGCTGATCAAGTACGCCAGCGAGTCCATCAG
CGGCATCCCCAGCCGGTTCAGCGGCAGCGGCTCCGGCACCGACTTCACCCTGAGCATCAACA
GCGTGGAGAGCGAGGATATCGCCGACTACTACTGCCAGCAGAACAACAACTGGCCCACCACC
TTCGGAGCCGGCACCAAGCTGGAACTGAAGCGTACGGTGGCCGCCCCCAGCGTGTTCATCTT
CCCCCCCAGCGATGAGCAGCTCAAGAGCGGCACCGCCAGCGTGGTGTGTCTGCTGAACAACT
TCTACCCCCGGGAGGCCAAAGTGCAGTGGAAAGTGGACAACGCCCTGCAGAGCGGCAACAGC
CAGGAGAGCGTGACCGAGCAGGACAGCAAGGACTCCACCTACAGCCTGAGCAGCACCCTGAC
CCTGAGCAAGGCCGACTACGAGAAGCACAAAGTGTACGCCTGCGAAGTGACCCACCAGGGCC
TGTCCAGCCCCGTGACCAAGAGCTTCAACCGGGGCGAGTGC

**SEQ ID NO: 248** (PascoH-616 Heavy chain)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGGGCGTGCAGCTCCTGGAGAGCGGCGGA
GGCCTGGTCCAGCCCGGCGGCAGCCTGAGGCTGAGCTGCGCCGCCAGCGGCTTCGTGTTC
CCCTGGTATGATATGGGCTGGGTGAGGCAGGCCCCCGGCAAGGGCCTGGAGTGGGTGTCC
AGCATCGACTGGCACGGGAAGATCACCTACTACGCCGACAGCGTGAAGGGCAGGTTCACC
ATCAGCAGGGACAACAGCAAGAACACCCTGTACCTGCAGATGAACAGCCTGAGGGCCGAG
GACACCGCAGTGTACTACTGCGCCACCGCCGAGGACGAACCCGGCTACGACTACTGGGGC
CAGGGCACCCTGGTGACTGTGAGCAGC

**SEQ ID NO: 249** (PascoH-TVAAPS-616 Heavy chain)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG

CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGCGTGCAG
CTCCTGGAGAGCGGCGGAGGCCTGGTCCAGCCCGGCGGCAGCCTGAGGCTGAGCTGCGCC
GCCAGCGGCTTCGTGTTCCCCTGGTATGATATGGGCTGGGTGAGGCAGGCCCCCGGCAAG
GGCCTGGAGTGGGTGTCCAGCATCGACTGGCACGGGAAGATCACCTACTACGCCGACAGC
GTGAAGGGCAGGTTCACCATCAGCAGGGACAACAGCAAGAACACCCTGTACCTGCAGATG
AACAGCCTGAGGGCCGAGGACACCGCAGTGTACTACTGCGCCACCGCCGAGGACGAACCC
GGCTACGACTACTGGGGCCAGGGCACCCTGGTGACTGTGAGCAGC

**SEQ ID NO: 250: 656H-TVAAPS-210 Heavy chain**

CAGGTGCAGCTCGTCCAGTCTGGGGCCGAGGTGAAGAAGCCCGGAGCTTCTGTGAAGGTGTC
CTGCAAGGCCAGCGGCTATACCTTCATCGACTACGAGATCCATTGGGTGAGGCAGGCTCCCG
GGCAGGGCCTGGAGTGGATGGGCGCCATCGACCCAGAGACCGGAGGCACGGCGTACAACCAG
AAGTTCAAGGGACGGGTCACCATGACAACCGATACCAGCACCTCCACCGCTTACATGGAGCT
GCGCAGCCTGAGAAGCGACGACACCGCGGTGTACTACTGTACGCGCATCCTGCTCTACTACT
ACCCCATGGATTACTGGGGCCAGGGCACACTAGTGACCGTGTCTAGCGCCAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAACCGGTGACCGTGTCCTGGAACAGCGGAGCCCTGA
CCAGCGGCGTGCACACCTTCCCCGCCGTGCTGCAGAGCAGCGGCCTGTACAGCCTGAGCAGC
GTGGTGACCGTGCCCAGCAGCAGCCTGGGCAcCCAGACCTACATCTgTAACgTgAACCACAa
GCCCAGCAACACCAAGGTGGACAAGAAGGTGGAGCCCAAGAGCTGTGACAAGACCCACACCT
GCCCCCCCTGCCCTGCCCCCGAGCTGCTGGGAGGCCCCAGCGTGTTCCTGTTCCCCCCCAAG
CCTAAGGACACCCTGATGATCAGCAGAACCCCCGAGGTGACCTGTGTGGTGGTGGATGTGAG
CCACGAGGACCCTGAGGTGAAGTTCAACTGGTACGTGGACGGCGTGGAGGTGCACAATGCCA
AGACCAAGCCCAGGGAGGAGCAGTACAACAGCACCTACCGGGTGGTGTCCGTGCTGACCGTG
CTGCACCAGGATTGGCTGAACGGCAAGGAGTACAAGTGTAAGGTGTCCAACAAGGCCCTGCC
TGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAG

GGCTTCTACCCCAGCGACATCGCCGTGGAGTGGGAGAGCAACGGCCAGCCCGAGAACAACTA
CAAGACCACCCCCCCTGTGCTGGACAGCGATGGCAGCTTCTTCCTGTACAGCAAGCTGACCG
TGGACAAGAGCAGATGGCAGCAGGGCAACGTGTTCAGCTGCTCCGTGATGCACGAGGCCCTG
CACAATCACTACACCCAGAAGAGCCTGAGCCTGTCCCCTGGCAAGACCGTGGCCGCCCCCTC
GGAAGTGCAGCTCCTGGAGAGCGGCGGCGGCCTGGTGCAGCCCGGCGGCAGCCTGAGGCTGA
GCTGCGCCGCTAGCGGCTTCACCTTCAGGAACTTCGGCATGGGCTGGGTCAGGCAGGCCCCC
GGCAAGGGCCTGGAGTGGGTCAGCTGGATCATCAGCTCCGGCACCGAGACCTACTACGCCGA
CAGCGTGAAGGGCAGGTTCACCATCAGCCGCGACAACAGCAAGAACACCCTGTACCTGCAGA
TGAACAGCCTGAGGGCCGAGGACACCGCCGTCTACTACTGCGCCAAGAGCCTGGGCAGGTTC
GACTACTGGGGACAGGGGACCCTGGTGACTGTGAGCAGC

**SEQ ID NO: 251: 656 Light chain**

GAGATCGTGCTGACCCAGAGTCCAGCCACCCTCAGCCTGAGCCCTGGGGAACGCGCCACCCT
GTCCTGCCGGGCGAGTCAGAACATCTCCGACTACCTGCATTGGTACCAGCAGAAGCCCGGCC
AGGCCCCTCGCCTGCTGATCTACTACGCCTCCCAGAGCATCAGCGGAATCCCCGCCCGGTTC
TCCGGAAGTGGGTCCGGAACCGACTTTACCCTGACCATCAGCTCTCTCGAGCCAGAGGACTT
CGCGGTGTACTACTGCCAGAACGGGCATAGTTTCCCACTGACCTTCGGAGGGGGCACAAAGG
TGGAGATCAAGCGTACGGTGGCCGCCCCCAGCGTGTTCATCTTCCCCCCCAGCGATGAGCAG
CTGAAGAGCGGCACCGCCAGCGTGGTGTGTCTGCTGAACAACTTCTACCCCCGGGAGGCCAA
GGTGCAGTGGAAGGTGGACAATGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTAC
GAGAAGCACAAGGTGTACGCCTGTGAGGTGACCCACCAGGGCCTGTCCAGCCCCGTGACCAA
GAGCTTCAACCGGGGCGAGTGC

**SEQ ID NO: 252:** PascoH-TVAAPS-546_Heavy chain

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGCGTGCAG

CTCCTGGAGAGCGGCGGAGGCCTGGTCCAGCCCGGCGGCAGCCTGAGGCTGAGCTGCGCC
GCCAGCGGCTTCGTGTTCCCCTGGTATGATATGGGCTGGGTGAGGCAGGCCCCCGGCAAG
GGCCTGGAGTGGGTGTCCAGCATCGACTGGAAGGGGGGCAAGACCTACTACGCCGACAGC
GTGAAGGGCAGGTTCACCATCAGCAGGGACAACAGCAAGAACACCCTGTACCTGCAGATG
AACAGCCTGAGGGCCGAGGACACCGCAGTGTACTACTGCGCCACCGCCGAGGACGAACCC
GGCTACGACTACTGGGGCCAGGGCACCCTGGTGACTGTGAGCAGC

**SEQ ID NO: 253:** PascoH-546_Heavy chain

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGGGCGTGCAGCT
CCTGGAGAGCGGCGGAGGCCTGGTCCAGCCCGGCGGCAGCCTGAGGCTGAGCTGCGCCGCCA
GCGGCTTCGTGTTCCCCTGGTATGATATGGGCTGGGTGAGGCAGGCCCCCGGCAAGGGCCTG
GAGTGGGTGTCCAGCATCGACTGGAAGGGGGGCAAGACCTACTACGCCGACAGCGTGAAGGG
CAGGTTCACCATCAGCAGGGACAACAGCAAGAACACCCTGTACCTGCAGATGAACAGCCTGA
GGGCCGAGGACACCGCAGTGTACTACTGCGCCACCGCCGAGGACGAACCCGGCTACGACTAC
TGGGGCCAGGGCACCCTGGTGACTGTGAGCAGC

**SEQ ID NO: 254:** PascoH-TVAAPS-567_Heavy chain

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC

AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGCGTGCAG
CTCCTGGAGAGCGGCGGAGGCCTGGTCCAGCCCGGCGGCAGCCTGAGGCTGAGCTGCGCC
GCCAGCGGCTTCGTGTTCGCCTGGTATGATATGGGCTGGGTGAGGCAGGCCCCCGGCAAG
GGCCTGGAGTGGGTGTCCAGCATCGACTGGCACGGGGAGGTGACCTACTACGCCGACAGC
GTGAAGGGCAGGTTCACCATCAGCAGGGACAACAGCAAGAACACCCTGTACCTGCAGATG
AACAGCCTGAGGGCCGAGGACACCGCAGTGTACTACTGCGCCACCGCCGAGGACGAACCC
GGCTACGACTACTGGGGCCAGGGCACCCTGGTGACTGTGAGCAGC

**SEQ ID NO: 255:** (PascoH-567_Heavy chain)

CAGGTGACCCTGAGGGAGAGCGGCCCCGCCCTGGTGAAGCCCACCCAGACCCTGACCCTG
ACCTGCACCTTCAGCGGCTTTAGCCTCAGCACCTCCGGCATGGGCGTGAGCTGGATCAGG
CAGCCACCCGGCAAAGGCCTGGAGTGGCTGGCCCACATCTACTGGGACGACGACAAGAGG
TACAACCCCAGCCTGAAGAGCCGGCTGACCATCAGCAAGGATACCAGCAGGAACCAGGTG
GTGCTGACCATGACCAACATGGACCCCGTGGACACCGCTACCTACTACTGCGCCAGGAGG
GAGACCGTCTTCTACTGGTACTTCGACGTGTGGGGAAGGGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGGGCGTGCAGCTCCTGGAGAGCGGCGGA
GGCCTGGTCCAGCCCGGCGGCAGCCTGAGGCTGAGCTGCGCCGCCAGCGGCTTCGTGTTC

GCCTGGTATGATATGGGCTGGGTGAGGCAGGCCCCCGGCAAGGGCCTGGAGTGGGTGTCC
AGCATCGACTGGCACGGGGAGGTGACCTACTACGCCGACAGCGTGAAGGGCAGGTTCACC
ATCAGCAGGGACAACAGCAAGAACACCCTGTACCTGCAGATGAACAGCCTGAGGGCCGAG
GACACCGCAGTGTACTACTGCGCCACCGCCGAGGACGAACCCGGCTACGACTACTGGGGC
CAGGGCACCCTGGTGACTGTGAGCAGC

**SEQ ID NO: 256: 656 Heavy chain**

CAGGTGCAGCTCGTCCAGTCTGGGGCCGAGGTGAAGAAGCCCGGAGCTTCTGTGAAGGTGTC
CTGCAAGGCCAGCGGCTATACCTTCATCGACTACGAGATCCATTGGGTGAGGCAGGCTCCCG
GGCAGGGCCTGGAGTGGATGGGCGCCATCGACCCAGAGACCGGAGGCACGGCGTACAACCAG
AAGTTCAAGGGACGGGTCACCATGACAACCGATACCAGCACCTCCACCGCTTACATGGAGCT
GCGCAGCCTGAGAAGCGACGACACCGCGGTGTACTACTGTACGCGCATCCTGCTCTACTACT
ACCCCATGGATTACTGGGGCCAGGGCACACTAGTCACAGTCTCCTCAGCCTCCACCAAGGGC
CCATCGGTCTTCCCCCTGGCACCCTCCTCCAAGAGCACCTCTGGGGGCACAGCGGCCCTGGG
CTGCCTGGTCAAGGACTACTTCCCCGAACCGGTGACGGTGTCGTGGAACTCAGGCGCCCTGA
CCAGCGGCGTGCACACCTTCCCGGCTGTCCTACAGTCCTCAGGACTCTACTCCCTCAGCAGC
GTGGTGACCGTGCCCTCCAGCAGCTTGGGCACCCAGACCTACATCTGCAACGTGAATCACAA
GCCCAGCAACACCAAGGTGGACAAGAAAGTTGAGCCCAAATCTTGTGACAAAACTCACACAT
GCCCACCGTGCCCAGCACCTGAACTCCTGGGGGGACCGTCAGTCTTCCTCTTCCCCCCAAAA
CCCAAGGACACCCTCATGATCTCCCGGACCCCTGAGGTCACATGCGTGGTGGTGGACGTGAG
CCACGAAGACCCTGAGGTCAAGTTCAACTGGTACGTGGACGGCGTGGAGGTGCATAATGCCA
AGACAAAGCCGCGGGAGGAGCAGTACAACAGCACGTACCGTGTGGTCAGCGTCCTCACCGTC
CTGCACCAGGACTGGCTGAATGGCAAGGAGTACAAGTGCAAGGTCTCCAACAAAGCCCTCCC
AGCCCCCATCGAGAAAACCATCTCCAAAGCCAAAGGGCAGCCCCGAGAACCACAGGTGTACA
CCCTGCCCCCATCCCGGGATGAGCTGACCAAGAACCAGGTCAGCCTGACCTGCCTGGTCAAA
GGCTTCTATCCCAGCGACATCGCCGTGGAGTGGGAGAGCAATGGGCAGCCGGAGAACAACTA
CAAGACCACGCCTCCCGTGCTGGACTCCGACGGCTCCTTCTTCCTCTACAGCAAGCTCACCG
TGGACAAGAGCAGGTGGCAGCAGGGGAACGTCTTCTCATGCTCCGTGATGCATGAGGCTCTG
CACAACCACTACACGCAGAAGAGCCTCTCCCTGTCTCCGGGTAAA

**SEQ ID NO: 257:** Anti IL-5 Heavy Chain-G4S-dAb474-TVAAPSGS-dAb210

CAGGTGACCCTGAGGGAGAGCGGCCCCGCCCTGGTGAAGCCCACACAGACCCTCACTCTG
ACCTGCACCGTGAGCGGCTTCAGCCTGACCTCCTACAGCGTCCACTGGGTGAGGCAGCCC
CCCGGCAAGGGCCTGGAGTGGCTGGGCGTGATCTGGGCAAGCGGCGGCACCGACTACAAC
AGCGCCCTGATGAGCAGGCTCTCCATCAGCAAGGACACCAGCCGGAACCAGGTGGTGCTG
ACCATGACCAACATGGACCCCGTGGACACCGCCACCTATTACTGCGCCAGGGACCCTCCC
TCTAGCCTGCTGAGGCTGGACTACTGGGGCAGGGGAACACTAGTGACCGTGTCCAGCGCC
AGCACCAAGGGCCCCAGCGTGTTCCCCCTGGCCCCCAGCAGCAAGAGCACCAGCGGCGGC
ACAGCCGCCCTGGGCTGCCTGGTGAAGGACTACTTCCCCGAACCGGTGACCGTGTCCTGG
AACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAG
AGCTGTGACAAGACCCACACCTGCCCCCCCTGCCCTGCCCCCGAGCTGCTGGGAGGCCCC
AGCGTGTTCCTGTTCCCCCCCAAGCCTAAGGACACCCTGATGATCAGCAGAACCCCCGAG
GTGACCTGTGTGGTGGTGGATGTGAGCCACGAGGACCCTGAGGTGAAGTTCAACTGGTAC
GTGGACGGCGTGGAGGTGCACAATGCCAAGACCAAGCCCAGGGAGGAGCAGTACAACAGC
ACCTACCGGGTGGTGTCCGTGCTGACCGTGCTGCACCAGGATTGGCTGAACGGCAAGGAG
TACAAGTGTAAGGTGTCCAACAAGGCCCTGCCTGCCCCTATCGAGAAAACCATCAGCAAG
GCCAAGGGCCAGCCCAGAGAGCCCCAGGTGTACACCCTGCCCCCTAGCAGAGATGAGCTG
ACCAAGAACCAGGTGTCCCTGACCTGCCTGGTGAAGGGCTTCTACCCCAGCGACATCGCC
GTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAG
CAGGGCAACGTGTTCAGCTGCTCCGTGATGCACGAGGCCCTGCACAATCACTACACCCAG
AAGAGCCTGAGCCTGTCCCCTGGCAAGGGCGGCGGCGGATCTGGCGTGCAGCTCCTGGAG

AGCGGCGGAGGCCTGGTCCAGCCCGGCGGCAGCCTGAGGCTGAGCTGCGCCGCCAGCGGC
TTCACCTTCGCCTGGTATGATATGGGCTGGGTGAGGCAGGCCCCCGGCAAGGGCCTGGAG
TGGGTGTCCAGCATCGACTGGCACGGGGAGGTGACCTACTACGCCGACAGCGTGAAGGGC
AGGTTCACCATCAGCAGGGACAACAGCAAGAACACCCTGTACCTGCAGATGAACAGCCTG
AGGGCCGAGGACACCGCAGTGTACTACTGCGCCACCGCCGAGGACGAACCCGGCTACGAC
TACTGGGGCCAGGGCACCCTGGTGACTGTGAGCAGCACCGTGGCCGCCCCCTCGGGATCC
GAAGTGCAGCTCCTGGAGAGCGGCGGCGGCCTGGTGCAGCCCGGCGGCAGCCTGAGGCTG
AGCTGCGCCGCTAGCGGCTTCACCTTCAGGAACTTCGGCATGGGCTGGGTCAGGCAGGCC
CCCGGCAAGGGCCTGGAGTGGGTCAGCTGGATCATCAGCTCCGGCACCGAGACCTACTAC
GCCGACAGCGTGAAGGGCAGGTTCACCATCAGCCGCGACAACAGCAAGAACACCCTGTAC
CTGCAGATGAACAGCCTGAGGGCCGAGGACACCGCCGTCTACTACTGCGCCAAGAGCCTG
GGCAGGTTCGACTACTGGGGACAGGGGACCCTGGTGACTGTGAGCAGC

**SEQ ID NO: 258:** Anti CD-20 Heavy Chain-TVAAPSGS-dAb154-TVAAPSGS-dAb474

CAGGTGCAGCTGCAGCAGCCTGGAGCCGAGCTGGTGAAGCCCGGCGCCAGCGTGAAAATG
TCCTGCAAGGCCAGCGGCTACACCTTCACCAGCTACAACATGCACTGGGTGAAGCAGACC
CCCGGCAGGGGCCTCGAGTGGATCGGAGCTATCTACCCCGGCAACGGCGACACTAGCTAC
AACCAGAAGTTCAAGGGCAAGGCCACCCTGACCGCCGACAAGAGCAGCAGCACCGCCTAC
ATGCAGCTGAGCAGCCTGACCAGCGAGGACAGCGCCGTGTATTACTGCGCCAGGAGCACC
TACTACGGCGGCGACTGGTACTTCAACGTCTGGGGCGCCGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGATCTGAC
ATCCAGATGACCCAGAGCCCCAGCAGCCTGAGCGCCAGCGTGGGCGACAGGGTGACCATT
ACCTGCAGGGCCAGCAGGCCCATCAGCGACTGGCTGCACTGGTACCAACAGAAGCCCGGC
AAGGCTCCCAAGCTGCTGATCGCCTGGGCCAGCAGCCTGCAGGGAGGCGTGCCCAGCAGG
TTTAGCGGCAGCGGCAGCGGCACCGACTTCACCCTCACCATCTCTTCCCTGCAGCCCGAG
GACTTCGCCACCTACTACTGCCTGCAGGAGGGCTGGGGGCCCCCTACTTTCGGCCAGGGC
ACCAAGGTGGAGATCAAGAGGACCGTGGCCGCCCCCTCGGGATCCGGCGTGCAGCTCCTG
GAGAGCGGCGGAGGCCTGGTCCAGCCCGGCGGCAGCCTGAGGCTGAGCTGCGCCGCCAGC
GGCTTCACCTTCGCCTGGTATGATATGGGCTGGGTGAGGCAGGCCCCCGGCAAGGGCCTG
GAGTGGGTGTCCAGCATCGACTGGCACGGGGAGGTGACCTACTACGCCGACAGCGTGAAG
GGCAGGTTCACCATCAGCAGGGACAACAGCAAGAACACCCTGTACCTGCAGATGAACAGC
CTGAGGGCCGAGGACACCGCAGTGTACTACTGCGCCACCGCCGAGGACGAACCCGGCTAC
GACTACTGGGGCCAGGGCACCCTGGTGACTGTGAGCAGC

**SEQ ID NO: 259: Anti CD-20 Heavy Chain-TVAAPSGS-dAb210-TVAAPSGS-dAb474**

CAGGTGCAGCTGCAGCAGCCTGGAGCCGAGCTGGTGAAGCCCGGCGCCAGCGTGAAAATG
TCCTGCAAGGCCAGCGGCTACACCTTCACCAGCTACAACATGCACTGGGTGAAGCAGACC

CCCGGCAGGGGCCTCGAGTGGATCGGAGCTATCTACCCCGGCAACGGCGACACTAGCTAC
AACCAGAAGTTCAAGGGCAAGGCCACCCTGACCGCCGACAAGAGCAGCAGCACCGCCTAC
ATGCAGCTGAGCAGCCTGACCAGCGAGGACAGCGCCGTGTATTACTGCGCCAGGAGCACC
TACTACGGCGGCGACTGGTACTTCAACGTCTGGGGCGCCGGCACACTAGTGACCGTGTCC
AGCGCCAGCACCAAGGGCCCCAGCGTGTTCCCCCTGGCCCCCAGCAGCAAGAGCACCAGC
GGCGGCACAGCCGCCCTGGGCTGCCTGGTGAAGGACTACTTCCCCGAACCGGTGACCGTG
TCCTGGAACAGCGGAGCCCTGACCAGCGGCGTGCACACCTTCCCCGCCGTGCTGCAGAGC
AGCGGCCTGTACAGCCTGAGCAGCGTGGTGACCGTGCCCAGCAGCAGCCTGGGCACCCAG
ACCTACATCTGTAACGTGAACCACAAGCCCAGCAACACCAAGGTGGACAAGAAGGTGGAG
CCCAAGAGCTGTGACAAGACCCACACCTGCCCCCCCTGCCCTGCCCCCGAGCTGCTGGGA
GGCCCCAGCGTGTTCCTGTTCCCCCCCAAGCCTAAGGACACCCTGATGATCAGCAGAACC
CCCGAGGTGACCTGTGTGGTGGTGGATGTGAGCCACGAGGACCCTGAGGTGAAGTTCAAC
TGGTACGTGGACGGCGTGGAGGTGCACAATGCCAAGACCAAGCCCAGGGAGGAGCAGTAC
AACAGCACCTACCGGGTGGTGTCCGTGCTGACCGTGCTGCACCAGGATTGGCTGAACGGC
AAGGAGTACAAGTGTAAGGTGTCCAACAAGGCCCTGCCTGCCCCTATCGAGAAAACCATC
AGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGAC
ATCGCCGTGGAGTGGGAGAGCAACGGCCAGCCCGAGAACAACTACAAGACCACCCCCCCT
GTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTAC
ACCCAGAAGAGCCTGAGCCTGTCCCCTGGCAAGACCGTGGCCGCCCCCTCGGGATCTGAA
GTGCAGCTCCTGGAGAGCGGCGGCGGCCTGGTGCAGCCCGGCGGCAGCCTGAGGCTGAGC
TGCGCCGCTAGCGGCTTCACCTTCAGGAACTTCGGCATGGGCTGGGTCAGGCAGGCCCCC
GGCAAGGGCCTGGAGTGGGTCAGCTGGATCATCAGCTCCGGCACCGAGACCTACTACGCC
GACAGCGTGAAGGGCAGGTTCACCATCAGCCGCGACAACAGCAAGAACACCCTGTACCTG
CAGATGAACAGCCTGAGGGCCGAGGACACCGCCGTCTACTACTGCGCCAAGAGCCTGGGC
AGGTTCGACTACTGGGGACAGGGGACCCTGGTGACTGTGAGCAGCACCGTGGCCGCCCCC
TCGGGATCCGGCGTGCAGCTCCTGGAGAGCGGCGGAGGCCTGGTCCAGCCCGGCGGCAGC
CTGAGGCTGAGCTGCGCCGCCAGCGGCTTCACCTTCGCCTGGTATGATATGGGCTGGGTG
AGGCAGGCCCCCGGCAAGGGCCTGGAGTGGGTGTCCAGCATCGACTGGCACGGGGAGGTG
ACCTACTACGCCGACAGCGTGAAGGGCAGGTTCACCATCAGCAGGGACAACAGCAAGAAC
ACCCTGTACCTGCAGATGAACAGCCTGAGGGCCGAGGACACCGCAGTGTACTACTGCGCC
ACCGCCGAGGACGAACCCGGCTACGACTACTGGGGCCAGGGCACCCTGGTGACTGTGAGC
AGC

**SEQ ID NO: 260:** anti cMET 5D5v2 Heavy Chain (hole)-GS-dAb593

GAGGTGCAGCTGGTGGAAAGCGGCGGCGGCCTGGTGCAGCCCGGCGGCTCCCTGAGGCTGAG
CTGCGCCGCTAGCGGCTACACCTTCACCAGCTACTGGCTCCACTGGGTCAGGCAGGCCCCAG
GCAAGGGACTGGAGTGGGTGGGCATGATCGACCCCAGCAACAGCGACACCAGGTTCAACCCC
AACTTCAAGGACAGGTTCACCATCAGCGCCGACACTAGCAAGAACACCGCCTACCTGCAGAT
GAACAGCCTGAGGGCCGAGGACACCGCCGTGTATTACTGCGCCACCTACAGGAGCTACGTCA
CCCCCCTGGATTACTGGGGCCAGGGCACACTAGTGACCGTGTCCAGCGCCAGCACCAAGGGC
CCCAGCGTGTTCCCCCTGGCCCCCAGCAGCAAGAGCACCAGCGGCGGCACAGCCGCCCTGGG
CTGCCTGGTGAAGGACTACTTCCCCGAACCGGTGACCGTGTCCTGGAACAGCGGAGCCCTGA
CCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAA
GCCCAGCAACACCAAGGTGGACAAGAAGGTGGAGCCCAAGAGCTGTGACAAGACCCACACCT
GCCCCCCCTGCCCTGCCCCCGAGCTGCTGGGAGGCCCCAGCGTGTTCCTGTTCCCCCCCAAG
CCTAAGGACACCCTGATGATCAGCAGAACCCCCGAGGTGACCTGTGTGGTGGTGGATGTGAG
CCACGAGGACCCTGAGGTGAAGTTCAACTGGTACGTGGACGGCGTGGAGGTGCACAATGCCA
AGACCAAGCCCAGGGAGGAGCAGTACAACAGCACCTACCGGGTGGTGTCCGTGCTGACCGTG
CTGCACCAGGATTGGCTGAACGGCAAGGAGTACAAGTGTAAGGTGTCCAACAAGGCCCTGCC
TGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGAGCTGCGCCGTGAAG
GGCTTCTACCCCAGCGACATCGCCGTGGAGTGGGAGAGCAACGGCCAGCCCGAGAACAACTA

CAAGACCACCCCCCCTGTGCTGGACAGCGATGGCAGCTTCTTCCTGGTGAGCAAGCTGACCG
TGGACAAGAGCAGATGGCAGCAGGGCAACGTGTTCAGCTGCTCCGTGATGCACGAGGCCCTG
CACAATCACTACACCCAGAAGAGCCTGAGCCTGTCCCCTGGCAAGGGATCCGAGGTGCAGCT
CCTGGTCAGCGGCGGCGGCCTGGTCCAGCCCGGAGGCTCACTGAGGCTGAGCTGCGCCGCTA
GCGGCTTCACCTTCAAGGCCTACCCCATGATGTGGGTCAGGCAGGCCCCCGGCAAAGGCCTG
GAGTGGGTGTCTGAGATCAGCCCCAGCGGCAGCTACACCTACTACGCCGACAGCGTGAAGGG
CAGGTTCACCATCAGCAGGGACAACAGCAAGAACACCCTGTACCTGCAGATGAACTCTCTGA
GGGCCGAGGACACCGCCGTGTACTACTGCGCCAAGGACCCCAGGAAGCTGGACTATTGGGGC
CAGGGCACTCTGGTGACCGTGAGCAGC

**SEQ ID NO: 261:** anti cMET 5D5v2 Heavy Chain (knob)-GS-dAb593

TGCCCCCCCTGCCCTGCCCCCGAGCTGCTGGGAGGCCCCAGCGTGTTCCTGTTCCCCCCC
AAGCCTAAGGACACCCTGATGATCAGCAGAACCCCCGAGGTGACCTGTGTGGTGGTGGAT
GTGAGCCACGAGGACCCTGAGGTGAAGTTCAACTGGTACGTGGACGGCGTGGAGGTGCAC
AATGCCAAGACCAAGCCCAGGGAGGAGCAGTACAACAGCACCTACCGGGTGGTGTCCGTG
CTGACCGTGCTGCACCAGGATTGGCTGAACGGCAAGGAGTACAAGTGTAAGGTGTCCAAC
AAGGCCCTGCCTGCCCCTATCGAGAAAACCATCAGCAAGGCCAAGGGCCAGCCCAGAGAG
CCCCAGGTGTACACCCTGCCCCCTAGCAGAGATGAGCTGACCAAGAACCAGGTGTCCCTG
TGGTGCCTGGTGAAGGGCTTCTACCCCAGCGACATCGCCGTGGAGTGGGAGAGCAACGGC
CAGCCCGAGAACAACTACAAGACCACCCCCCCTGTGCTGGACAGCGATGGCAGCTTCTTC
CTGTACAGCAAGCTGACCGTGGACAAGAGCAGATGGCAGCAGGGCAACGTGTTCAGCTGC
TCCGTGATGCACGAGGCCCTGCACAATCACTACACCCAGAAGAGCCTGAGCCTGTCCCCT
GGCAAGGGATCCGAGGTGCAGCTCCTGGTCAGCGGCGGCGGCCTGGTCCAGCCCGGAGGC
TCACTGAGGCTGAGCTGCGCCGCTAGCGGCTTCACCTTCAAGGCCTACCCCATGATGTGG
GTCAGGCAGGCCCCCGGCAAAGGCCTGGAGTGGGTGTCTGAGATCAGCCCCAGCGGCAGC
TACACCTACTACGCCGACAGCGTGAAGGGCAGGTTCACCATCAGCAGGGACAACAGCAAG
AACACCCTGTACCTGCAGATGAACTCTCTGAGGGCCGAGGACACCGCCGTGTACTACTGC
GCCAAGGACCCCAGGAAGCTGGACTATTGGGGCCAGGGCACTCTGGTGACCGTGAGCAGC

**SEQ ID NO: 262:** anti cMET 5D5v2 Light Chain

GACATCCAGATGACCCAGAGCCCCAGCAGCCTGAGCGCCTCAGTGGGAGACAGGGTGACC
ATCACCTGCAAGAGCAGCCAGAGCCTCCTGTACACCAGCAGCCAGAAGAACTACCTGGCC
TGGTACCAGCAGAAACCCGGCAAGGCCCCCAAGCTGCTGATCTACTGGGCTAGCACCAGG
GAGTCAGGCGTGCCCAGCAGGTTCAGCGGCAGCGGCAGCGGCACCGACTTCACTCTGACC
ATCAGCAGCCTGCAGCCCGAGGACTTCGCCACCTACTACTGCCAGCAGTACTACGCCTAT
CCCTGGACCTTCGGCCAGGGCACCAAGGTGGAGATCAAGCGTACGGTGGCCGCCCCCAGC
GTGTTCATCTTCCCCCCCAGCGATGAGCAGCTGAAGAGCGGCACCGCCAGCGTGGTGTGT
CTGCTGAACAACTTCTACCCCCGGGAGGCCAAGGTGCAGTGGAAGGTGGACAATGCCCTG
CAGAGCGGCAACAGCCAGGAGAGCGTGACCGAGCAGGACAGCAAGGACTCCACCTACAGC
CTGAGCAGCACCCTGACCCTGAGCAAGGCCGACTACGAGAAGCACAAGGTGTACGCCTGT
GAGGTGACCCACCAGGGCCTGTCCAGCCCCGTGACCAAGAGCTTCAACCGGGGCGAGTGC

**SEQ ID NO: 263:** anti cMET 5D5v2 IgG4 Heavy Chain (UNIBODY)-GS-dAb593

GAGGTGCAGCTGGTGGAAAGCGGCGGCGGCCTGGTGCAGCCCGGCGGCTCCCTGAGGCTG
AGCTGCGCCGCTAGCGGCTACACCTTCACCAGCTACTGGCTCCACTGGGTCAGGCAGGCC
CCAGGCAAGGGACTGGAGTGGGTGGGCATGATCGACCCCAGCAACAGCGACACCAGGTTC
AACCCCAACTTCAAGGACAGGTTCACCATCAGCGCCGACACTAGCAAGAACACCGCCTAC
CTGCAGATGAACAGCCTGAGGGCCGAGGACACCGCCGTGTATTACTGCGCCACCTACAGG
AGCTACGTCACCCCCCTGGATTACTGGGGCCAGGGCACACTAGTCACCGTGAGCAGCGCC

AGCACCAAGGGCCCCAGCGTGTTCCCCCTGGCCCCCTGCAGCAGAAGCACCAGCGAGAGC
ACAGCCGCCCTGGGCTGCCTGGTGAAGGACTACTTCCCCGAGCCCGTGACCGTGAGCTGG
AACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCAAGACCTAC
ACCTGCAACGTGGACCACAAGCCCAGCAACACCAAGGTGGACAAGCGGGTGGCCCCCGAG
TTCCTGGGCGGACCCTCCGTGTTCCTGTTCCCCCCCAAGCCCAAGGACACCCTGATGATC
AGCCGGACCCCCGAGGTGACCTGCGTGGTGGTGGACGTGAGCCAGGAAGATCCCGAGGTC
CAGTTCAATTGGTACGTGGACGGCGTGGAGGTGCACAACGCCAAGACCAAGCCCCGGGAG
GAACAGTTCAACAGCACCTACCGGGTGGTGTCCGTGCTGACCGTGCTGCACCAGGACTGG
CTGAACGGCAAAGAATACAAGTGCAAGGTGTCCAACAAGGGCCTGCCCAGCTCCATCGAG
AAAACCATCAGCAAGGCCAAGGGCCAGCCTCGGGAGCCCCAGGTGTACACCCTGCCCCCA
TCCCAGGAAGAGATGACCAAGAACCAGGTGTCCCTGACCTGTCTGGTGAAGGGCTTCTAC
CCCAGCGACATCGCCGTGGAGTGGGAGAGCAACGGCCAGCCCGAGAACAACTACAAGACC
ACCCCCCCTGTGCTGGACAGCGACGGCAGCTTCTTCCTGTACAGCAGGCTGACCGTGGAC
AAGAGCCGGTGGCAGGAAGGCAACGTCTTTAGCTGCAGCGTGATGCACGAGGCCCTGCAC
AACCACTACACCCAGAAGAGCCTGAGCCTGTCCCTGGGCAAGGGATCCGAGGTGCAGCTC
CTGGTCAGCGGCGGCGGCCTGGTCCAGCCCGGAGGCTCACTGAGGCTGAGCTGCGCCGCT
AGCGGCTTCACCTTCAAGGCCTACCCCATGATGTGGGTCAGGCAGGCCCCCGGCAAAGGC
CTGGAGTGGGTGTCTGAGATCAGCCCCAGCGGCAGCTACACCTACTACGCCGACAGCGTG
AAGGGCAGGTTCACCATCAGCAGGGACAACAGCAAGAACACCCTGTACCTGCAGATGAAC
TCTCTGAGGGCCGAGGACACCGCCGTGTACTACTGCGCCAAGGACCCCAGGAAGCTGGAC
TATTGGGGCCAGGGCACTCTGGTGACCGTGAGCAGC

**SEQ ID NO: 264:** anti cMET 5D5v2 Heavy Chain (hole)

GAGGTGCAGCTGGTGGAAAGCGGCGGCGGCCTGGTGCAGCCCGGCGGCTCCCTGAGGCTG
AGCTGCGCCGCTAGCGGCTACACCTTCACCAGCTACTGGCTCCACTGGGTCAGGCAGGCC
CCAGGCAAGGGACTGGAGTGGGTGGGCATGATCGACCCCAGCAACAGCGACACCAGGTTC
AACCCCAACTTCAAGGACAGGTTCACCATCAGCGCCGACACTAGCAAGAACACCGCCTAC
CTGCAGATGAACAGCCTGAGGGCCGAGGACACCGCCGTGTATTACTGCGCCACCTACAGG
AGCTACGTCACCCCCCTGGATTACTGGGGCCAGGGCACACTAGTGACCGTGTCCAGCGCC
AGCACCAAGGGCCCCAGCGTGTTCCCCCTGGCCCCCAGCAGCAAGAGCACCAGCGGCGGC
ACAGCCGCCCTGGGCTGCCTGGTGAAGGACTACTTCCCCGAACCGGTGACCGTGTCCTGG
AACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAG
AGCTGTGACAAGACCCACACCTGCCCCCCCTGCCCTGCCCCCGAGCTGCTGGGAGGCCCC
AGCGTGTTCCTGTTCCCCCCCAAGCCTAAGGACACCCTGATGATCAGCAGAACCCCCGAG
GTGACCTGTGTGGTGGTGGATGTGAGCCACGAGGACCCTGAGGTGAAGTTCAACTGGTAC
GTGGACGGCGTGGAGGTGCACAATGCCAAGACCAAGCCCAGGGAGGAGCAGTACAACAGC
ACCTACCGGGTGGTGTCCGTGCTGACCGTGCTGCACCAGGATTGGCTGAACGGCAAGGAG
TACAAGTGTAAGGTGTCCAACAAGGCCCTGCCTGCCCCTATCGAGAAAACCATCAGCAAG
GCCAAGGGCCAGCCCAGAGAGCCCCAGGTGTACACCCTGCCCCCTAGCAGAGATGAGCTG
ACCAAGAACCAGGTGTCCCTGAGCTGCGCCGTGAAGGGCTTCTACCCCAGCGACATCGCC
GTGGAGTGGGAGAGCAACGGCCAGCCCGAGAACAACTACAAGACCACCCCCCCTGTGCTG
GACAGCGATGGCAGCTTCTTCCTGGTGAGCAAGCTGACCGTGGACAAGAGCAGATGGCAG
CAGGGCAACGTGTTCAGCTGCTCCGTGATGCACGAGGCCCTGCACAATCACTACACCCAG
AAGAGCCTGAGCCTGTCCCCTGGCAAG

**SEQ ID NO: 265:** anti cMET 5D5v2 Heavy Chain (knob)

TGCCCCCCCTGCCCTGCCCCCGAGCTGCTGGGAGGCCCCAGCGTGTTCCTGTTCCCCCCC
AAGCCTAAGGACACCCTGATGATCAGCAGAACCCCCGAGGTGACCTGTGTGGTGGTGGAT
GTGAGCCACGAGGACCCTGAGGTGAAGTTCAACTGGTACGTGGACGGCGTGGAGGTGCAC
AATGCCAAGACCAAGCCCAGGGAGGAGCAGTACAACAGCACCTACCGGGTGGTGTCCGTG
CTGACCGTGCTGCACCAGGATTGGCTGAACGGCAAGGAGTACAAGTGTAAGGTGTCCAAC
AAGGCCCTGCCTGCCCCTATCGAGAAAACCATCAGCAAGGCCAAGGGCCAGCCCAGAGAG
CCCCAGGTGTACACCCTGCCCCCTAGCAGAGATGAGCTGACCAAGAACCAGGTGTCCCTG
TGGTGCCTGGTGAAGGGCTTCTACCCCAGCGACATCGCCGTGGAGTGGGAGAGCAACGGC
CAGCCCGAGAACAACTACAAGACCACCCCCCCTGTGCTGGACAGCGATGGCAGCTTCTTC
CTGTACAGCAAGCTGACCGTGGACAAGAGCAGATGGCAGCAGGGCAACGTGTTCAGCTGC
TCCGTGATGCACGAGGCCCTGCACAATCACTACACCCAGAAGAGCCTGAGCCTGTCCCCT
GGCAAG

**SEQ ID NO: 266:** anti cMET 5D5v2 IgG4 Heavy Chain (UNIBODY)

GAGGTGCAGCTGGTGGAAAGCGGCGGCGGCCTGGTGCAGCCCGGCGGCTCCCTGAGGCTG
AGCTGCGCCGCTAGCGGCTACACCTTCACCAGCTACTGGCTCCACTGGGTCAGGCAGGCC
CCAGGCAAGGGACTGGAGTGGGTGGGCATGATCGACCCCAGCAACAGCGACACCAGGTTC
AACCCCAACTTCAAGGACAGGTTCACCATCAGCGCCGACACTAGCAAGAACACCGCCTAC
CTGCAGATGAACAGCCTGAGGGCCGAGGACACCGCCGTGTATTACTGCGCCACCTACAGG
AGCTACGTCACCCCCCTGGATTACTGGGGCCAGGGCACACTAGTCACCGTGAGCAGCGCC
AGCACCAAGGGCCCCAGCGTGTTCCCCCTGGCCCCCTGCAGCAGAAGCACCAGCGAGAGC
ACAGCCGCCCTGGGCTGCCTGGTGAAGGACTACTTCCCCGAGCCCGTGACCGTGAGCTGG
AACAGCGGAGCCCTGACCAGCGGCGTGCACACCTTCCCCGCCGTGCTGCAGAGCAGCGGC
CTGTACAGCCTGAGCAGCGTGGTGACCGTGCCCAGCAGCAGCCTGGGCACCAAGACCTAC
ACCTGCAACGTGGACCACAAGCCCAGCAACACCAAGGTGGACAAGCGGGTGGCCCCCGAG
TTCCTGGGCGGACCCTCCGTGTTCCTGTTCCCCCCCAAGCCCAAGGACACCCTGATGATC
AGCCGGACCCCCGAGGTGACCTGCGTGGTGGTGGACGTGAGCCAGGAAGATCCCGAGGTC
CAGTTCAATTGGTACGTGGACGGCGTGGAGGTGCACAACGCCAAGACCAAGCCCCGGGAG
GAACAGTTCAACAGCACCTACCGGGTGGTGTCCGTGCTGACCGTGCTGCACCAGGACTGG
CTGAACGGCAAAGAATACAAGTGCAAGGTGTCCAACAAGGGCCTGCCCAGCTCCATCGAG
AAAACCATCAGCAAGGCCAAGGGCCAGCCTCGGGAGCCCCAGGTGTACACCCTGCCCCCA
TCCCAGGAAGAGATGACCAAGAACCAGGTGTCCCTGACCTGTCTGGTGAAGGGCTTCTAC
CCCAGCGACATCGCCGTGGAGTGGGAGAGCAACGGCCAGCCCGAGAACAACTACAAGACC
ACCCCCCCTGTGCTGGACAGCGACGGCAGCTTCTTCCTGTACAGCAGGCTGACCGTGGAC
AAGAGCCGGTGGCAGGAAGGCAACGTCTTTAGCTGCAGCGTGATGCACGAGGCCCTGCAC
AACCACTACACCCAGAAGAGCCTGAGCCTGTCCCTGGGCAAG

**SEQ ID NO: 267: PascoH IgG2-GS-474 heavy chain**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTCACCGTGAGCAGCGCCAGCACC
AAGGGCCCCAGCGTGTTCCCCCTGGCCCCCTGCAGCAGAAGCACCAGCGAGAGCACAGCCGC
CCTGGGCTGCCTGGTGAAGGACTACTTCCCCGAGCCCGTGACCGTGAGCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAACTTCGGCACCCAGACCTACACCTGCAACGTGGA
CCACAAGCCCAGCAACACCAAGGTGGACAAGACCGTGGAGCGGAAGTGCTGCGTGGAGTGCC

CCCCCTGCCCTGCCCCTCCTGTGGCCGGACCCTCCGTGTTCCTGTTCCCCCCCAAGCCCAAG
GACACCCTGATGATCAGCCGGACCCCCGAGGTGACCTGCGTGGTGGTGGACGTGAGCCACGA
GGACCCCGAGGTGCAGTTCAATTGGTACGTGGACGGCGTGGAGGTGCACAACGCCAAGACCA
AGCCCCGGGAGGAACAGTTCAACAGCACCTTCCGGGTGGTGTCCGTGCTGACCGTGGTGCAC
CAGGACTGGCTGAACGGCAAAGAATACAAGTGCAAGGTGTCCAACAAGGGCCTGCCTGCCCC
CATCGAGAAAACCATCAGCAAGACCAAGGGCCAGCCCAGGGAACCCCAGGTGTACACCCTGC
CCCCCAGCCGGGAGGAAATGACCAAGAACCAGGTGTCCCTGACCTGTCTGGTGAAGGGCTTC
TACCCCAGCGACATCGCCGTGGAGTGGGAGAGCAACGGCCAGCCCGAGAACAACTACAAGAC
CACCCCCCCCATGCTGGACAGCGACGGCAGCTTCTTCCTGTACAGCAAGCTGACAGTGGACA
AGAGCCGGTGGCAGCAGGGCAACGTGTTCAGCTGCAGCGTGATGCACGAGGCCCTGCACAAC
CACTACACCCAGAAGAGCCTGAGCCTGTCCCCCGGCAAGGGATCCGGCGTGCAGCTCCTGGA
GAGCGGCGGAGGCCTGGTCCAGCCCGGCGGCAGCCTGAGGCTGAGCTGCGCCGCCAGCGGCT
TCACCTTCGCCTGGTATGATATGGGCTGGGTGAGGCAGGCCCCCGGCAAGGGCCTGGAGTGG
GTGTCCAGCATCGACTGGCACGGGGAGGTGACCTACTACGCCGACAGCGTGAAGGGCAGGTT
CACCATCAGCAGGGACAACAGCAAGAACACCCTGTACCTGCAGATGAACAGCCTGAGGGCCG
AGGACACCGCAGTGTACTACTGCGCCACCGCCGAGGACGAACCCGGCTACGACTACTGGGGC
CAGGGCACCCTGGTGACTGTGAGCAGC

**SEQ ID NO: 268: PascoH IgG4-GS-474 heavy chain**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTCACCGTGAGCAGCGCCAGCACC
AAGGGCCCCAGCGTGTTCCCCCTGGCCCCCTGCAGCAGAAGCACCAGCGAGAGCACAGCCGC
CCTGGGCTGCCTGGTGAAGGACTACTTCCCCGAGCCCGTGACCGTGAGCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCAAGACCTACACCTGCAACGTGGA
CCACAAGCCCAGCAACACCAAGGTGGACAAGCGGGTGGAGAGCAAGTACGGCCCTCCCTGCC
CCAGCTGCCCTGCCCCCGAGTTCCTGGGCGGACCCTCCGTGTTCCTGTTCCCCCCCAAGCCC
AAGGACACCCTGATGATCAGCCGGACCCCCGAGGTGACCTGCGTGGTGGTGGACGTGAGCCA
GGAAGATCCCGAGGTCCAGTTCAATTGGTACGTGGACGGCGTGGAGGTGCACAACGCCAAGA
CCAAGCCCCGGGAGGAACAGTTCAACAGCACCTACCGGGTGGTGTCCGTGCTGACCGTGCTG
CACCAGGACTGGCTGAACGGCAAAGAATACAAGTGCAAGGTGTCCAACAAGGGCCTGCCCAG
CTCCATCGAGAAAACCATCAGCAAGGCCAAGGGCCAGCCTCGGGAGCCCCAGGTGTACACCC
TGCCCCCATCCCAGGAAGAGATGACCAAGAACCAGGTGTCCCTGACCTGTCTGGTGAAGGGC
TTCTACCCCAGCGACATCGCCGTGGAGTGGGAGAGCAACGGCCAGCCCGAGAACAACTACAA
GACCACCCCCCCTGTGCTGGACAGCGACGGCAGCTTCTTCCTGTACAGCAGGCTGACCGTGG
ACAAGAGCCGGTGGCAGGAAGGCAACGTCTTTAGCTGCAGCGTGATGCACGAGGCCCTGCAC
AACCACTACACCCAGAAGAGCCTGAGCCTGTCCCTGGGCAAGGGATCCGGCGTGCAGCTCCT
GGAGAGCGGCGGAGGCCTGGTCCAGCCCGGCGGCAGCCTGAGGCTGAGCTGCGCCGCCAGCG
GCTTCACCTTCGCCTGGTATGATATGGGCTGGGTGAGGCAGGCCCCCGGCAAGGGCCTGGAG
TGGGTGTCCAGCATCGACTGGCACGGGGAGGTGACCTACTACGCCGACAGCGTGAAGGGCAG
GTTCACCATCAGCAGGGACAACAGCAAGAACACCCTGTACCTGCAGATGAACAGCCTGAGGG
CCGAGGACACCGCAGTGTACTACTGCGCCACCGCCGAGGACGAACCCGGCTACGACTACTGG
GGCCAGGGCACCCTGGTGACTGTGAGCAGC

**SEQ ID NO: 269: PascoH IgG4PE-GS-474 heavy chain**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT

TCTACTGGTACTTCGACGTGTGGGGAAGGGGCACACTAGTCACCGTGAGCAGCGCCAGCACC
AAGGGCCCCAGCGTGTTCCCCCTGGCCCCCTGCAGCAGAAGCACCAGCGAGAGCACAGCCGC
CCTGGGCTGCCTGGTGAAGGACTACTTCCCCGAGCCCGTGACCGTGAGCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCAAGACCTACACCTGCAACGTGGA
CCACAAGCCCAGCAACACCAAGGTGGACAAGCGGGTGGAGAGCAAGTACGGCCCTCCCTGCC
CCCCCTGCCCTGCCCCCGAGTTCGAGGGCGGACCCTCCGTGTTCCTGTTCCCCCCCAAGCCC
AAGGACACCCTGATGATCAGCCGGACCCCCGAGGTGACCTGCGTGGTGGTGGACGTGAGCCA
GGAAGATCCCGAGGTCCAGTTCAATTGGTACGTGGACGGCGTGGAGGTGCACAACGCCAAGA
CCAAGCCCCGGGAGGAACAGTTCAACAGCACCTACCGGGTGGTGTCCGTGCTGACCGTGCTG
CACCAGGACTGGCTGAACGGCAAAGAATACAAGTGCAAGGTGTCCAACAAGGGCCTGCCCAG
CTCCATCGAGAAAACCATCAGCAAGGCCAAGGGCCAGCCTCGGGAGCCCCAGGTGTACACCC
TGCCCCCATCCCAGGAAGAGATGACCAAGAACCAGGTGTCCCTGACCTGTCTGGTGAAGGGC
TTCTACCCCAGCGACATCGCCGTGGAGTGGGAGAGCAACGGCCAGCCCGAGAACAACTACAA
GACCACCCCCCCTGTGCTGGACAGCGACGGCAGCTTCTTCCTGTACAGCAGGCTGACCGTGG
ACAAGAGCCGGTGGCAGGAAGGCAACGTCTTTAGCTGCAGCGTGATGCACGAGGCCCTGCAC
AACCACTACACCCAGAAGAGCCTGAGCCTGTCCCTGGGCAAGGGATCCGGCGTGCAGCTCCT
GGAGAGCGGCGGAGGCCTGGTCCAGCCCGGCGGCAGCCTGAGGCTGAGCTGCGCCGCCAGCG
GCTTCACCTTCGCCTGGTATGATATGGGCTGGGTGAGGCAGGCCCCCGGCAAGGGCCTGGAG
TGGGTGTCCAGCATCGACTGGCACGGGGAGGTGACCTACTACGCCGACAGCGTGAAGGGCAG
GTTCACCATCAGCAGGGACAACAGCAAGAACACCCTGTACCTGCAGATGAACAGCCTGAGGG
CCGAGGACACCGCAGTGTACTACTGCGCCACCGCCGAGGACGAACCCGGCTACGACTACTGG
GGCCAGGGCACCCTGGTGACTGTGAGCAGC

**SEQ ID NO: 270: Anti IL-4 Heavy Chain-GS-anti TNF-a adnectin**

CAGGTGACCCTGAGGGAGAGCGGCCCCGCCCTGGTGAAGCCCACCCAGACCCTGACCCTGAC
CTGCACCTTCAGCGGCTTTAGCCTCAGCACCTCCGGCATGGGCGTGAGCTGGATCAGGCAGC
CACCCGGCAAAGGCCTGGAGTGGCTGGCCCACATCTACTGGGACGACGACAAGAGGTACAAC
CCCAGCCTGAAGAGCCGGCTGACCATCAGCAAGGATACCAGCAGGAACCAGGTGGTGCTGAC
CATGACCAACATGGACCCCGTGGACACCGCTACCTACTACTGCGCCAGGAGGGAGACCGTCT
TCTACTGGTACTTCGACGTGTGGGGAAGGGGCACACTAGTGACCGTGTCCAGCGCCAGCACC
AAGGGCCCCAGCGTGTTCCCCCTGGCCCCCAGCAGCAAGAGCACCAGCGGCGGCACAGCCGC
CCTGGGCTGCCTGGTGAAGGACTACTTCCCCGAACCGGTGACCGTGTCCTGGAACAGCGGAG
CCCTGACCAGCGGCGTGCACACCTTCCCCGCCGTGCTGCAGAGCAGCGGCCTGTACAGCCTG
AGCAGCGTGGTGACCGTGCCCAGCAGCAGCCTGGGCACCCAGACCTACATCTGTAACGTGAA
CCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCC
CCCAAGCCTAAGGACACCCTGATGATCAGCAGAACCCCCGAGGTGACCTGTGTGGTGGTGGA
TGTGAGCCACGAGGACCCTGAGGTGAAGTTCAACTGGTACGTGGACGGCGTGGAGGTGCACA
ATGCCAAGACCAAGCCCAGGGAGGAGCAGTACAACAGCACCTACCGGGTGGTGTCCGTGCTG
ACCGTGCTGCACCAGGATTGGCTGAACGGCAAGGAGTACAAGTGTAAGGTGTCCAACAAGGC
CCTGCCTGCCCCTATCGAGAAAACCATCAGCAAGGCCAAGGGCCAGCCCAGAGAGCCCCAGG
TGTACACCCTGCCCCCTAGCAGAGATGAGCTGACCAAGAACCAGGTGTCCCTGACCTGCCTG
GTGAAGGGCTTCTACCCCAGCGACATCGCCGTGGAGTGGGAGAGCAACGGCCAGCCCGAGAA
CAACTACAAGACCACCCCCCCTGTGCTGGACAGCGATGGCAGCTTCTTCCTGTACAGCAAGC
TGACCGTGGACAAGAGCAGATGGCAGCAGGGCAACGTGTTCAGCTGCTCCGTGATGCACGAG
GCCCTGCACAATCACTACACCCAGAAGAGCCTGAGCCTGTCCCCTGGCAAGGGATCCGTGAG
CGACGTGCCAAGGGACCTCGAGGTGGTGGCAGCCACTCCCACCTCTCTGCTGATCAGCTGGG
ACACACACAACGCCTACAACGGCTACTACAGGATCACCTACGGAGAGACCGGCGGCAATAGC
CCCGTGAGGGAGTTCACCGTGCCCCACCCCGAGGTGACCGCCACCATTAGCGGCCTGAAGCC
CGGCGTGGACGATACCATCACCGTCTACGCCGTGACCAACCACCACATGCCCCTGAGGATCT
TCGGCCCCATCAGCATCAACCATAGGACC

## Claims

1. An antigen-binding construct comprising a protein scaffold which is an antibody immunoglobulin scaffold comprising at least two heavy chains and two light chains, which scaffold is linked to one or more epitope-binding domains wherein the antigen-binding construct has four antigen binding sites, two of which are from epitope binding domains which are immunoglobulin single variable domains, and two of which are from paired VHNL domains, wherein the antigen binding construct is capable of binding IL-13, wherein at least one of the immunoglobulin single variable domains is directly attached to the scaffold with a linker comprising from 1 to 50 amino acids and wherein the immunoglobulin single variable domains are attached to the immunoglobulin scaffold at the C-terminus of the heavy chain.

2. An antigen-binding construct according to claim 1, wherein the binding construct has specificity for more than one antigen.

3. An antigen-binding construct according to any preceding claim wherein the antigen-binding construct is also capable of binding one or more antigens selected from I L-4 and I L-5.

4. An antigen-binding construct according to any preceding claim wherein the Immunoglobulin scaffold is an IgG scaffold.

5. An antigen-binding construct according to claim 4 wherein the IgG scaffold comprises all the domains of an antibody.

6. An antigen-binding construct according to claim 1 wherein at least one of the immunoglobulin single variable domain is directly attached to the Immunoglobulin scaffold with a linker selected from any one of those set out in SEQ ID NO: 6 to 11 or ’GS’, or any combination thereof.

7. An antigen-binding construct according to claim 6 wherein the linker comprises the sequence of SEQ ID NO: 7

8. An antigen binding construct according to any one of claims 1 to 7 which comprises an IL-13 antibody and which further comprises an immunoglobulin single variable domain with specificity for IL-4.

**9.** An antigen binding construct according to claim 8 wherein the antigen binding construct comprises the light chain sequence of SEQ ID NO: 13.

**10.** An antigen binding construct according to claim 8 comprising a heavy chain and a light chain, wherein the heavy chain comprises the antibody sequence of SEQ ID NO:12, the linker sequence of SEQ ID NO:7 and the immunoglobulin single variable domain of SEQ ID NO:3.

**11.** An antigen binding construct according to any one of claims 1 to 7 which comprises an IL-5 antibody and which further comprises an immunoglobulin single variable domain with specificity for IL-13.

**12.** An antigen binding construct according to claim 11 comprising a heavy chain and a light chain, wherein the heavy chain sequence comprises an antibody sequence which has at least 90% sequence identity to SEQ ID NO: 65 and wherein the light chain comprises an antibody sequence which has at least 90% sequence identity to SEQ ID NO: 66.

**13.** An antigen binding construct according to claim 12 comprising a heavy chain and a light chain, wherein the light chain sequence has at least 90% sequence identity to SEQ ID NO: 72.

**14.** An antigen binding construct according to claim 1 comprising a heavy chain and a light chain, wherein the heavy chain sequence has at least 90% sequence identity to SEQ ID NO: 26 and wherein the light chain sequence has at least 90% sequence identity to SEQ ID NO: 13.

**15.** A polynucleotide encoding a heavy chain of an antigen binding construct according to any one of claims 1 to 14.

**16.** A recombinant transformed or transfected host cell comprising one or more polynucleotide sequences encoding a heavy chain and a light chain of an antigen binding construct of any one of claims 1 to 14.

**17.** A method for the production of an antigen binding construct according to claims 1 to 14 which method comprises the step of culturing a host cell of claim 16 and isolating the antigen binding construct.

**18.** A pharmaceutical composition comprising an antigen binding construct of any one of claims 1 to 14 and a pharmaceutically acceptable carrier.

**19.** An antigen-binding construct according to any one of claims 1 to 14 for use in medicine.

**20.** An antigen-binding construct according to any one of claims 1 to 14 for the treatment of fibrotic diseases or disorders, and inflammatory diseases, such as asthma, rheumatoid arthritis or osteoarthritis.

## Patentansprüche

**1.** Ein Antigen-bindendes Konstrukt umfassend ein Proteingerüst, welches ein Antikörper-Immunglobulingerüst ist, das mindestens zwei schwere Ketten und zwei leichte Ketten umfasst, wobei das Gerüst mit einer oder mehreren Epitop-bindenden Domänen verbunden ist, und wobei das Antigen-bindende Konstrukt vier Antigenbindungsstellen aufweist, von denen zwei von Epitopebindenden Domänen stammen, bei denen es sich um einzelne variable Immunglobulindomänen handelt, und von denen zwei von gepaarten VH/VL-Domänen stammen, und wobei das Antigen-bindende Konstrukt in der Lage ist, IL-13 zu binden, wobei ferner mindestens eine der einzelnen variablen Immunglobulindomänen direkt mit dem Gerüst über einen Linker verbunden ist, der 1-50 Aminosäuren umfasst, und wobei die einzelnen variablen Immunglobulindomänen an dem C-Terminus der schweren Kette an das Immunglobulingerüst angehängt sind.

**2.** Antigen-bindendes Konstrukt nach Anspruch 1, wobei das bindende Konstrukt eine Spezifität für mehr als ein Antigen aufweist.

**3.** Antigen-bindendes Konstrukt nach einem der vorhergehenden Ansprüche, wobei das Antigen-bindende Konstrukt auch in der Lage ist, ein oder mehrere Antigene ausgewählt aus IL-4 und IL-5 zu binden.

4. Antigen-bindendes Konstrukt nach einem der vorhergehenden Ansprüche, wobei das Immunglobulingerüst ein IgG-Gerüst ist.

5. Antigen-bindendes Konstrukt nach Anspruch 4, wobei das IgG-Gerüst alle Domänen eines Antikörpers umfasst.

6. Antigen-bindendes Konstrukt nach Anspruch 1, wobei mindestens eine der einzelnen variablen Immunglobulindomänen direkt mit dem Immunglobulingerüst verbunden ist über einen Linker ausgewählt aus denen, die in SEQ ID NO: 6 bis 11 gezeigt sind, "GS", oder eine beliebige Kombination aus diesen.

7. Antigen-bindendes Konstrukt nach Anspruch 6, wobei der Linker die in SEQ ID NO: 7 gezeigte Sequenz aufweist.

8. Antigen-bindendes Konstrukt nach einem der Ansprüche 1 bis 7, welches einen IL-13-Antikörper umfasst und darüber hinaus eine einzelne variable Immunglobulindomäne mit einer Spezifität für IL-4.

9. Antigen-bindendes Konstrukt nach Anspruch 8, wobei das Antigen-bindende Konstrukt die Sequenz der leichten Kette wie in SEQ ID NO: 13 gezeigt, umfasst.

10. Antigen-bindendes Konstrukt nach Anspruch 8, welches eine schwere Kette und eine leichte Kette umfasst, wobei die schwere Kette die in SEQ ID NO: 12 gezeigte Antikörpersequenz, die in SEQ ID NO: 7 gezeigte Linkersequenz und die in SEQ ID NO: 3 gezeigte einzelne variable Immunglobulindomäne umfasst.

11. Antigen-bindendes Konstrukt nach einem der Ansprüche 1 bis 7, welches einen IL-15-Antikörper umfasst und darüber hinaus eine einzelne variable Immunglobulindomäne mit einer Spezifität für IL-13.

12. Antigen-bindendes Konstrukt nach Anspruch 11, welches eine schwere und eine leichte Kette umfasst, wobei die Sequenz der schweren Kette eine Antikörpersequenz umfasst, die mindestens 90% Sequenzidentität zu SEQ ID NO: 65 aufweist, und wobei die leichte Kette eine Antikörpersequenz umfasst, die mindestens 90% Sequenzidentität zu SEQ ID NO: 66 aufweist.

13. Antigen-bindendes Konstrukt nach Anspruch 12, welches eine schwere Kette und eine leichte Kette umfasst, wobei die Sequenz der leichten Kette mindestens 90% Sequenzidentität zu SEQ ID NO: 72 aufweist.

14. Antigen-bindendes Konstrukt nach Anspruch 1 umfassend eine schwere Kette und eine leichte Kette, wobei die Sequenz der schweren Kette mindestens 90% Sequenzidentität zu SEQ ID NO: 26 aufweist und wobei die Sequenz der leichten Kette mindestens 90% Sequenzidentität zu SEQ ID NO: 13 aufweist.

15. Polynucleotid, welches eine schwere Kette eines Antigen-bindenden Konstrukts nach einem der Ansprüche 1 bis 14 codiert.

16. Rekombinante transformierte oder transfizierte Wirtszelle, umfassend eine oder mehrere Polynucleotidsequenzen, welche eine schwere Kette und eine leichte Kette eines Antigen-bindenden Konstrukts nach einem der Ansprüche 1 bis 14 codieren.

17. Verfahren zur Herstellung eines Antigen-bindenden Konstruktes nach den Ansprüchen 1 bis 14, wobei das Verfahren den Schritt des Kultivierens einer Wirtszelle nach Anspruch 16 umfasst und die Isolierung des Antigen-bindenden Konstruktes.

18. Pharmazeutische Zusammensetzung enthaltend ein Antigen-bindendes Konstrukt nach einem der Ansprüche 1 bis 14 und einen pharmazeutisch verträglichen Träger.

19. Antigen-bindendes Konstrukt nach einem der Ansprüche 1 bis 14 zur Verwendung in der Medizin.

20. Antigen-bindendes Konstrukt nach einem der Ansprüche 1 bis 14 zur Behandlung von fibrotischen Erkrankungen oder Störungen und von entzündlichen Erkrankungen, wie Asthma, rheumatoide Arthritis oder Osteoarthritis.

## Revendications

1. Construction de liaison à un antigène comprenant un échafaudage protéique qui est un échafaudage d'anticorps immunoglobuline comprenant au moins deux chaînes lourdes et deux chaînes légères, ledit échafaudage étant relié à un ou plusieurs domaines de liaison à un épitope, la construction de liaison à un antigène ayant quatre sites de liaison à un antigène dont deux proviennent de domaines de liaison à un épitope qui sont des domaines variables simples d'immunoglobulines et deux proviennent de domaines VH/VL appariés, la construction de liaison à un antigène pouvant se lier à IL-13, où au moins l'un des domaines variables simples d'immunoglobuline est fixé directement à l'échafaudage avec un segment de liaison comprenant 1 à 50 acides aminés et où les domaines variables simples d'immunoglobuline sont fixés à l'échafaudage d'immunoglobuline à la terminaison C de la chaîne lourde.

2. Construction de liaison à un antigène selon la revendication 1, où la construction de liaison a une spécificité pour plus d'un antigène.

3. Construction de liaison à un antigène selon l'une quelconque des revendications précédentes, où la construction de liaison à un antigène peut également se lier à un ou plusieurs antigènes choisis parmi IL-4 et IL-5.

4. Construction de liaison à un antigène selon l'une quelconque des revendications précédentes, où l'échafaudage d'immunoglobuline est un échafaudage d'IgG.

5. Construction de liaison à un antigène selon la revendication 4, où l'échafaudage d'IgG comprend tous les domaines d'un anticorps.

6. Construction de liaison à un antigène selon la revendication 1, où au moins l'un des domaines variables simples de l'immunoglobuline est directement fixé à l'échafaudage d'immunoglobuline avec un segment de liaison choisi parmi l'un de ceux définis dans SEQ ID N° : 6 à 11 ou « GS » ou une combinaison de ceux-ci.

7. Construction de liaison à un antigène selon la revendication 6, où le segment de liaison comprend la séquence de SEQ ID N° : 7.

8. Construction de liaison à un antigène selon l'une quelconque des revendications 1 à 7, qui comprend un anticorps IL-13 et qui comprend en outre un domaine variable simple d'immunoglobuline présentant une spécificité pour IL-4.

9. Construction de liaison à un antigène selon la revendication 8, où la construction de liaison à un antigène comprend la séquence de chaîne légère de SEQ ID N° : 13.

10. Construction de liaison à un antigène selon la revendication 8, comprenant une chaîne lourde et une chaîne légère, où la chaîne lourde comprend la séquence d'anticorps de SEQ ID N° : 12, la séquence de segment de liaison de SEQ ID N° : 7 et le domaine variable simple d'immunoglobuline de SEQ ID N° : 3.

11. Construction de liaison à un antigène selon l'une quelconque des revendications 1 à 7, qui comprend un anticorps IL-5 et qui comprend en outre un domaine variable simple d'immunoglobuline présentant une spécificité pour IL-13.

12. Construction de liaison à un antigène selon la revendication 11 comprenant une chaîne lourde et une chaîne légère, où la séquence de chaîne lourde comprend une séquence d'anticorps qui a une identité de séquence d'au moins 90 % avec SEQ ID N° : 65 et où la chaîne légère comprend une séquence d'anticorps qui a une identité de séquence d'au moins 90 % avec SEQ ID N° : 66.

13. Construction de liaison à un antigène selon la revendication 12, comprenant une chaîne lourde et une chaîne légère, où la séquence de chaîne légère a une identité de séquence d'au moins 90 % avec SEQ ID N° : 72.

14. Construction de liaison à un antigène selon la revendication 1, comprenant une chaîne lourde et une chaîne légère, où la séquence de chaîne lourde a une identité de séquence d'au moins 90 % avec SEQ ID N° : 26 et où la séquence de chaîne légère a une identité de séquence d'au moins 90 % avec SEQ ID N° : 13.

15. Polynucléotide codant pour une chaîne lourde d'une construction de liaison à un antigène selon l'une quelconque des revendications 1 à 14.

**16.** Cellule hôte recombinante transformée ou transfectée comprenant une ou plusieurs séquences polynucléotidiques codant pour une chaîne lourde et une chaîne légère d'une construction de liaison à un antigène selon l'une quelconque des revendications 1 à 14.

**17.** Procédé de production d'une construction de liaison à un antigène selon les revendications 1 à 14, ledit procédé comprenant l'étape de culture d'une cellule hôte selon la revendication 16 et d'isolement de la construction de liaison à un antigène.

**18.** Composition pharmaceutique comprenant une construction de liaison à un antigène selon l'une quelconque des revendications 1 à 14 et un véhicule pharmaceutiquement acceptable.

**19.** Construction de liaison à un antigène selon l'une quelconque des revendications 1 à 14 pour son utilisation en médecine.

**20.** Construction de liaison à un antigène selon l'une quelconque des revendications 1 à 14 pour le traitement de maladies ou de troubles fibreux et de maladies inflammatoires telles que l'asthme, la polyarthrite rhumatoïde ou l'arthrose.

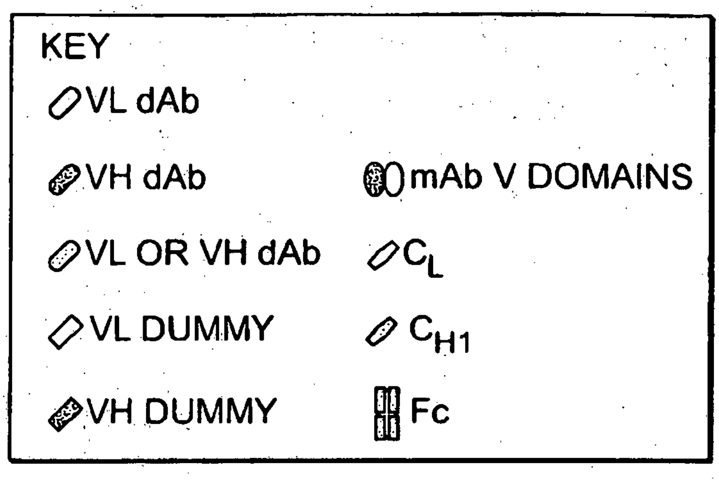


*FIG. 1*

CATEGORY 1:
mAb + dAbs

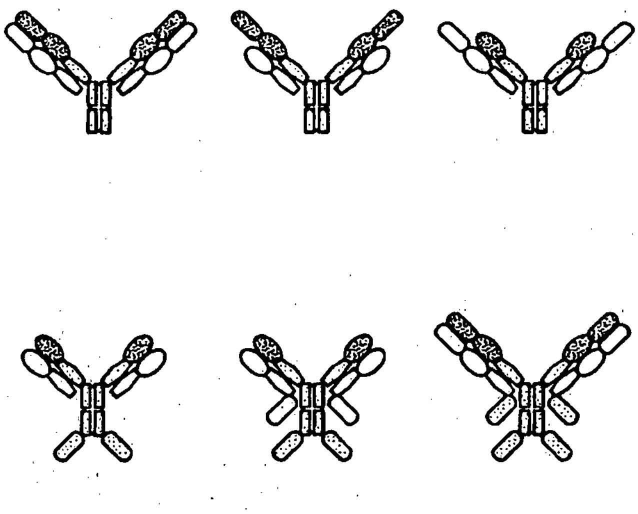

*FIG. 2*

CATEGORY 2:
DUMMY IgG + VARIABLE DOMAINS (Fv AND/OR dAbs)

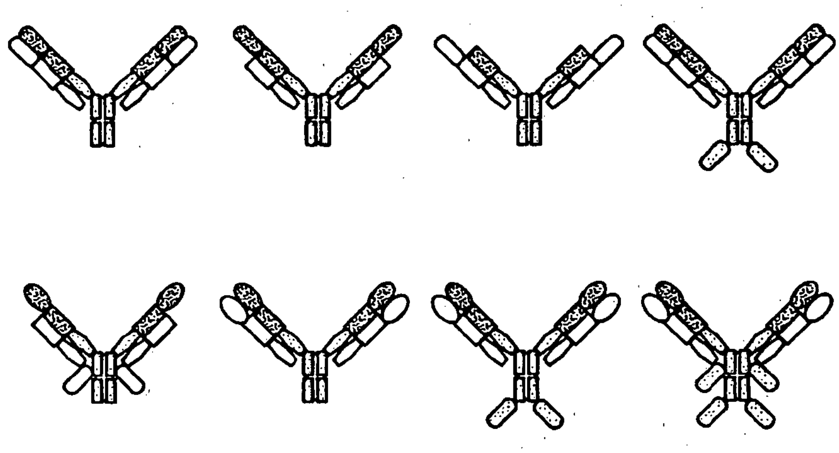

*FIG. 3*

CATEGORY 3:
PORTION OF DUMMY IgG + VARIABLE DOMAINS (Fv AND/OR dAbs)

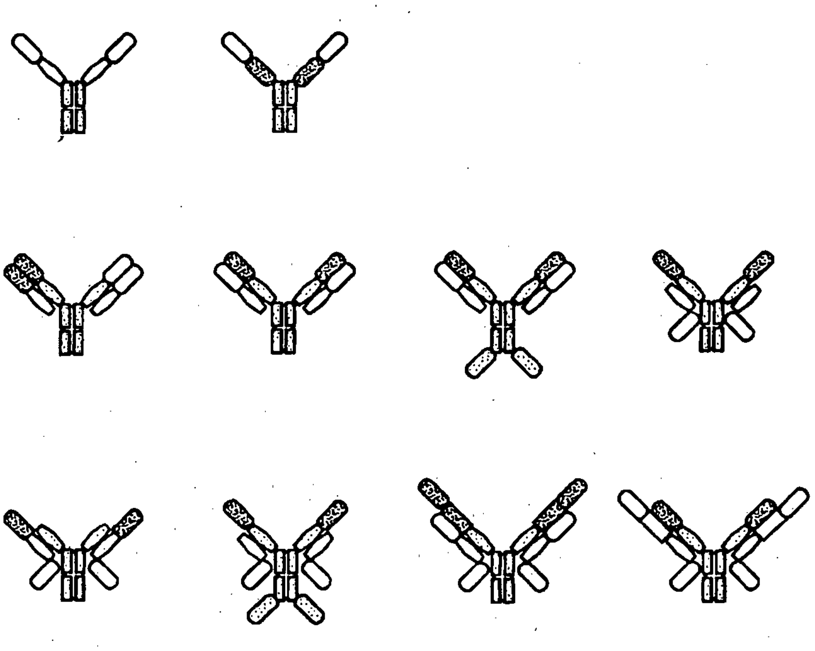

*FIG. 4*

CATEGORY 4:
VARIABLE AND/OR CONSTANT DOMAINS SWAPPED

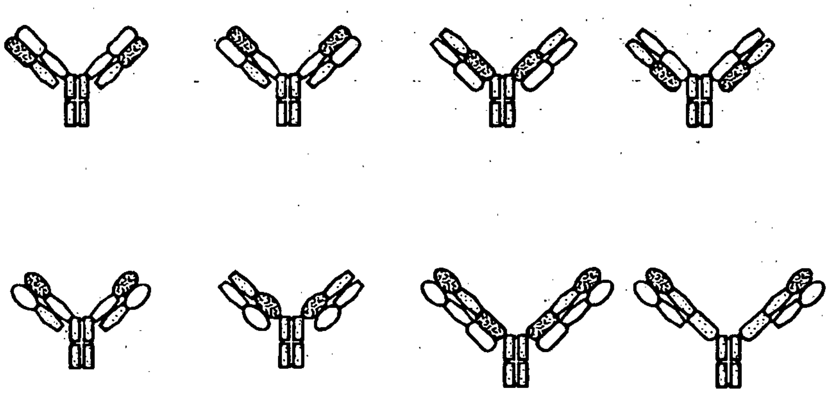

FIG. 5

CATEGORY 5:
CONSTANT DOMAINS DUPLICATED

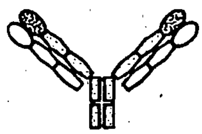

FIG. 6

CATEGORY 6:
AlbudAbs

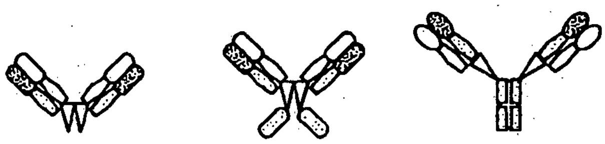

FIG. 7

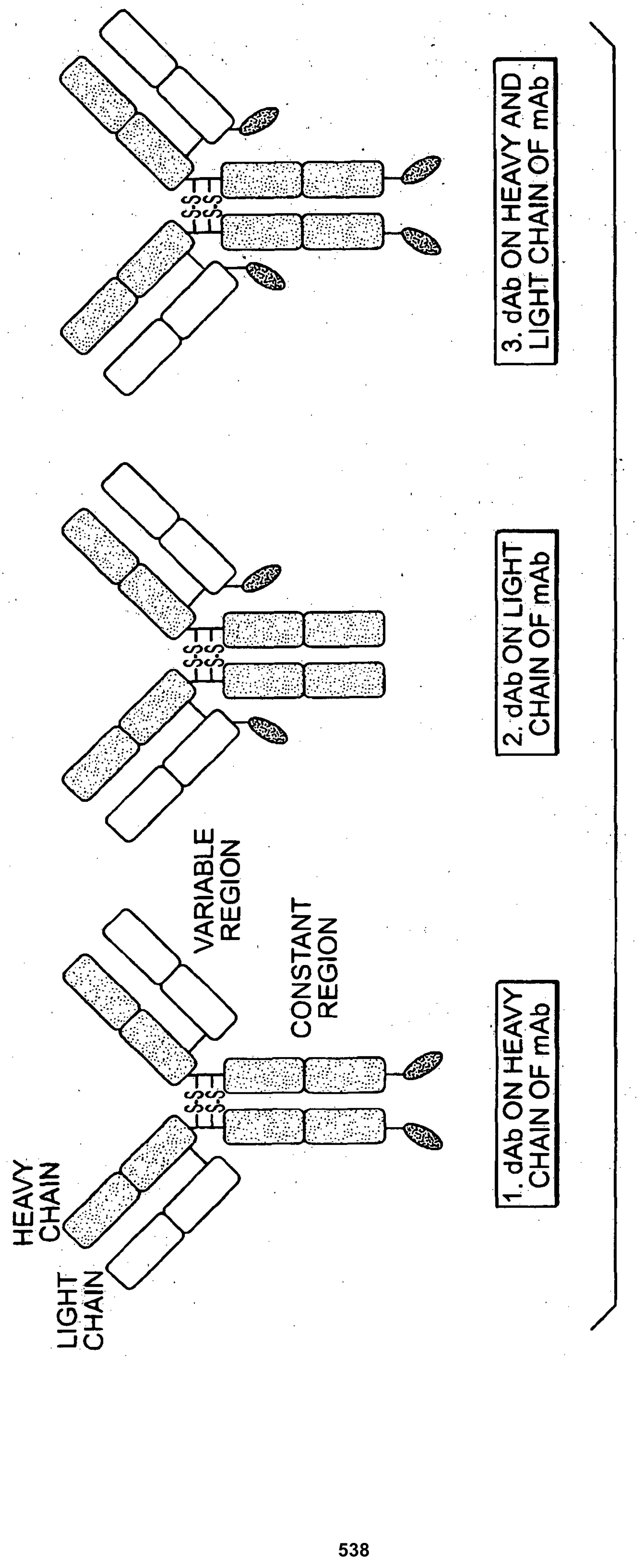
HEAVY CHAIN
LIGHT CHAIN
VARIABLE REGION
CONSTANT REGION
S-S
S-S
1. dAb ON HEAVY CHAIN OF mAb
2. dAb ON LIGHT CHAIN OF mAb
3. dAb ON HEAVY AND LIGHT CHAIN OF mAb

*FIG. 8*

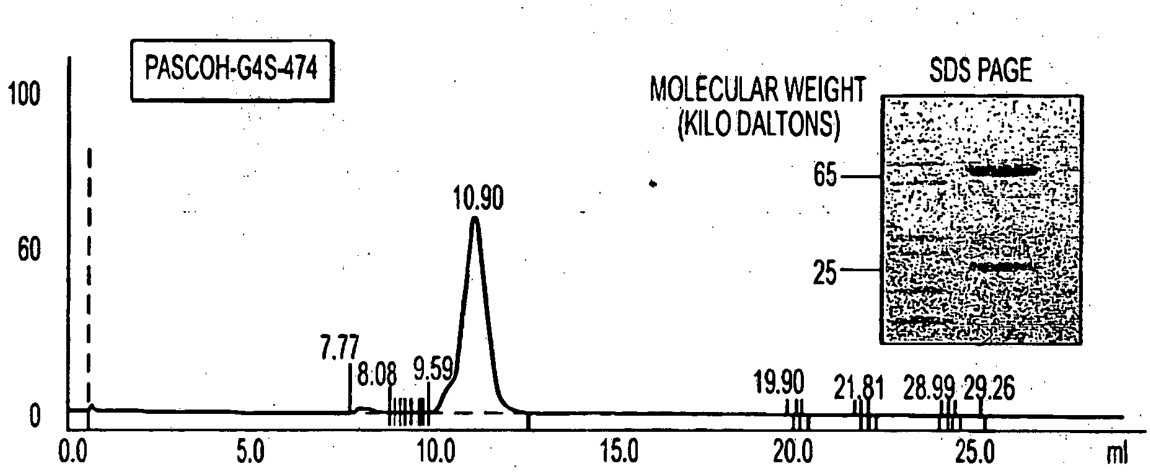


FIG. 9

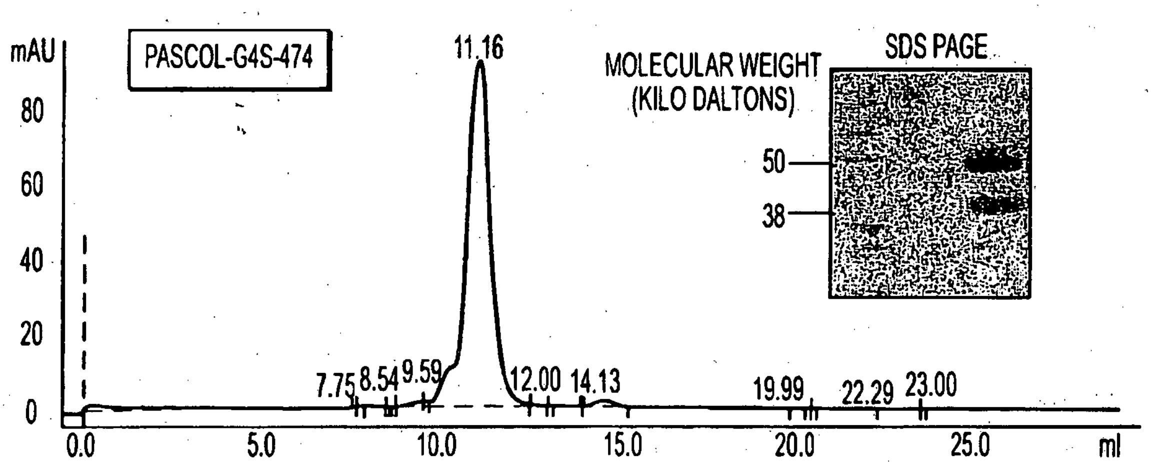


FIG. 10

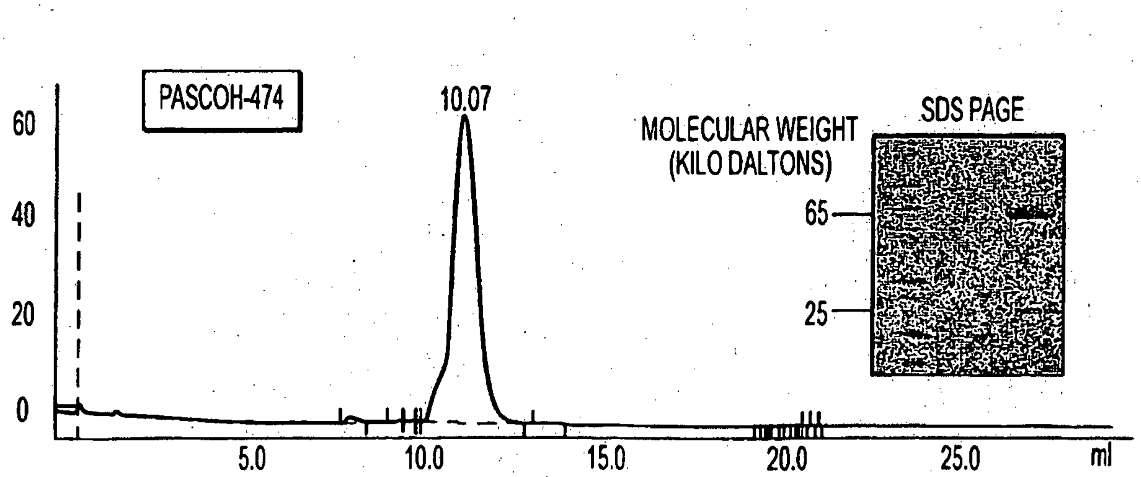


FIG. 11

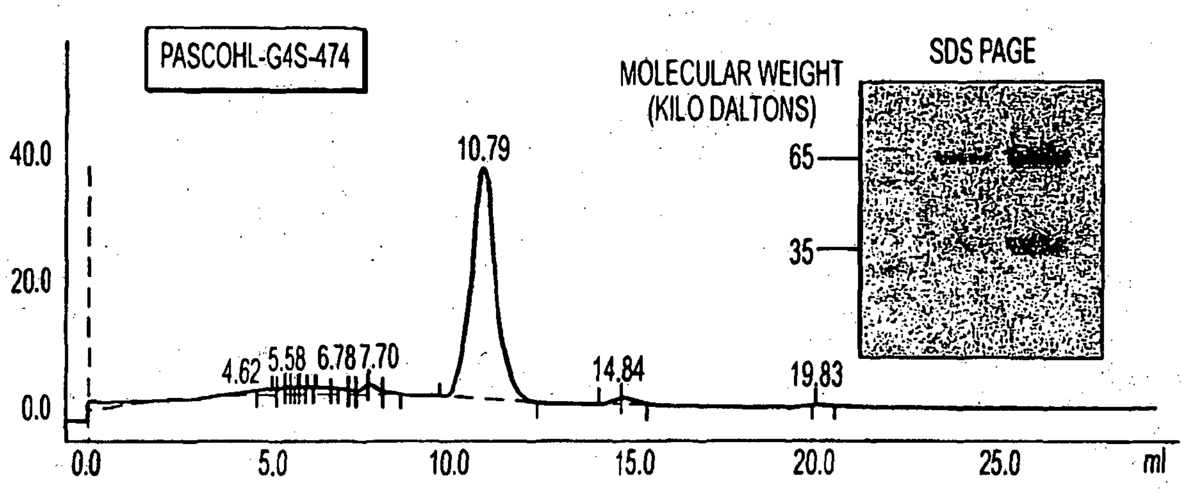
PASCOHL-G4S-474
MOLECULAR WEIGHT
(KILO DALTONS)
SDS PAGE
65
35
40.0
20.0
0.0
10.79
4.62
5.58
6.78
7.70
14.84
19.83
0.0
5.0
10.0
15.0
20.0
25.0
ml

*FIG. 12*

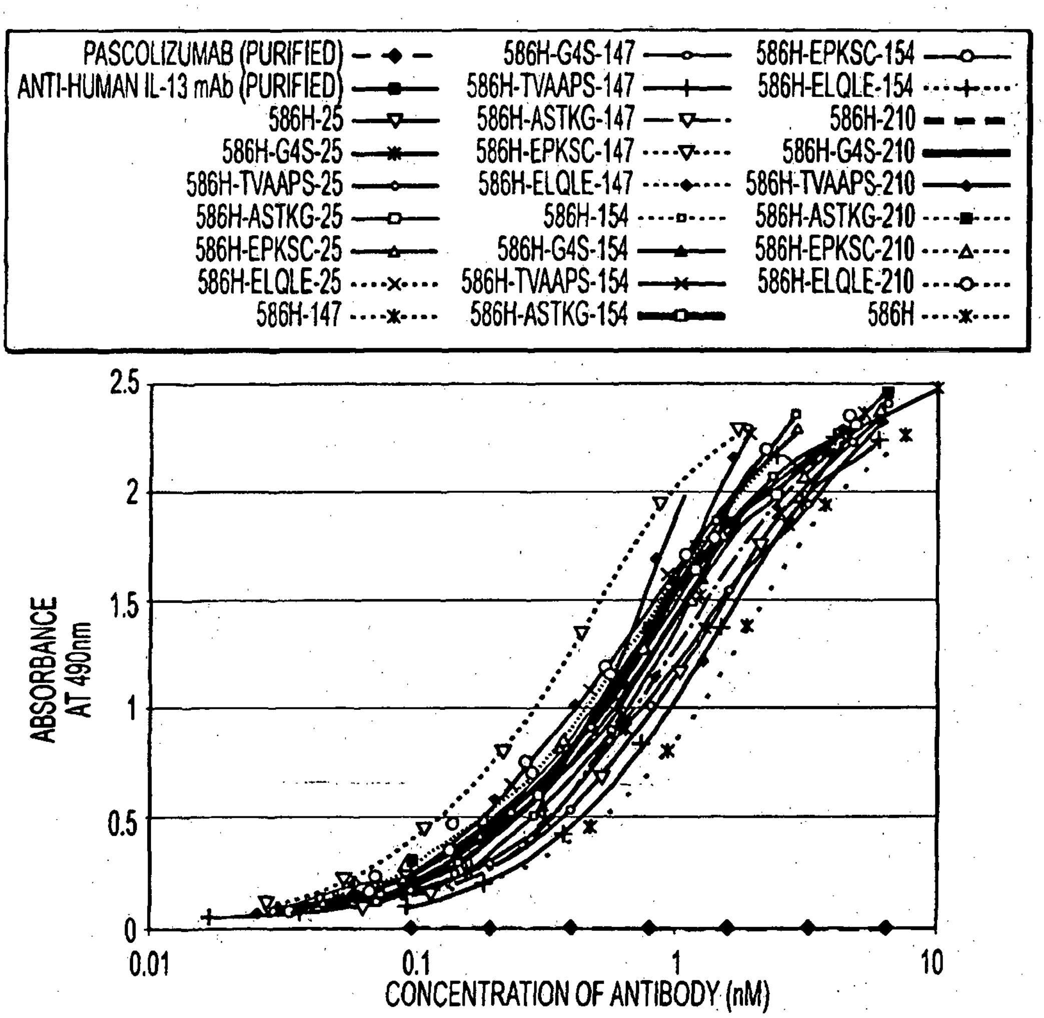
PASCOLIZUMAB (PURIFIED)
ANTI-HUMAN IL-13 mAb (PURIFIED)
586H-25
586H-G4S-25
586H-TVAAPS-25
586H-ASTKG-25
586H-EPKSC-25
586H-ELQLE-25
586H-147
586H-G4S-147
586H-TVAAPS-147
586H-ASTKG-147
586H-EPKSC-147
586H-ELQLE-147
586H-154
586H-G4S-154
586H-TVAAPS-154
586H-ASTKG-154
586H-EPKSC-154
586H-ELQLE-154
586H-210
586H-G4S-210
586H-TVAAPS-210
586H-ASTKG-210
586H-EPKSC-210
586H-ELQLE-210
586H
ABSORBANCE AT 490nm
2.5
2
1.5
1
0.5
0
0.01
0.1
1
10
CONCENTRATION OF ANTIBODY (nM)

*FIG. 13*

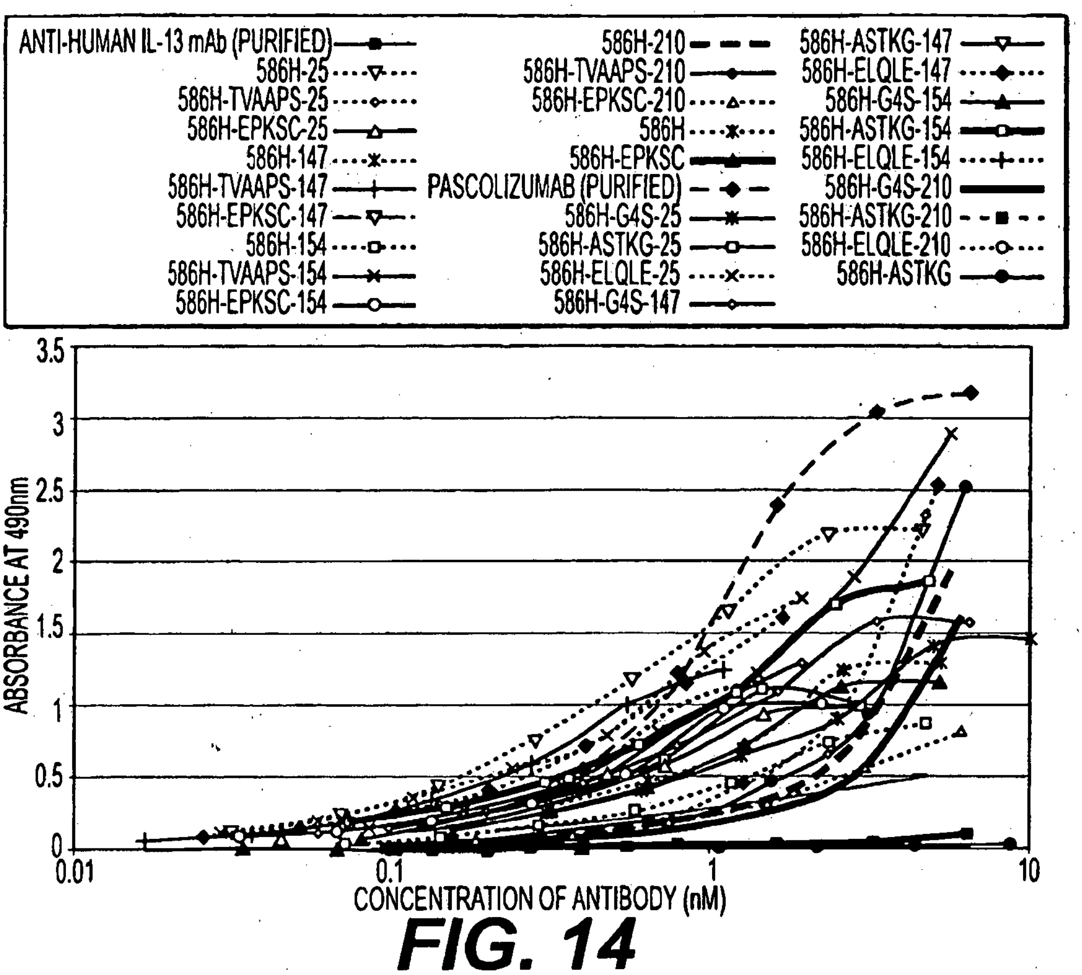
ANTI-HUMAN IL-13 mAb (PURIFIED)
586H-25
586H-TVAAPS-25
586H-EPKSC-25
586H-147
586H-TVAAPS-147
586H-EPKSC-147
586H-154
586H-TVAAPS-154
586H-EPKSC-154
586H-210
586H-TVAAPS-210
586H-EPKSC-210
586H
586H-EPKSC
PASCOLIZUMAB (PURIFIED)
586H-G4S-25
586H-ASTKG-25
586H-ELQLE-25
586H-G4S-147
586H-ASTKG-147
586H-ELQLE-147
586H-G4S-154
586H-ASTKG-154
586H-ELQLE-154
586H-G4S-210
586H-ASTKG-210
586H-ELQLE-210
586H-ASTKG
ABSORBANCE AT 490nm
CONCENTRATION OF ANTIBODY (nM)

FIG. 14

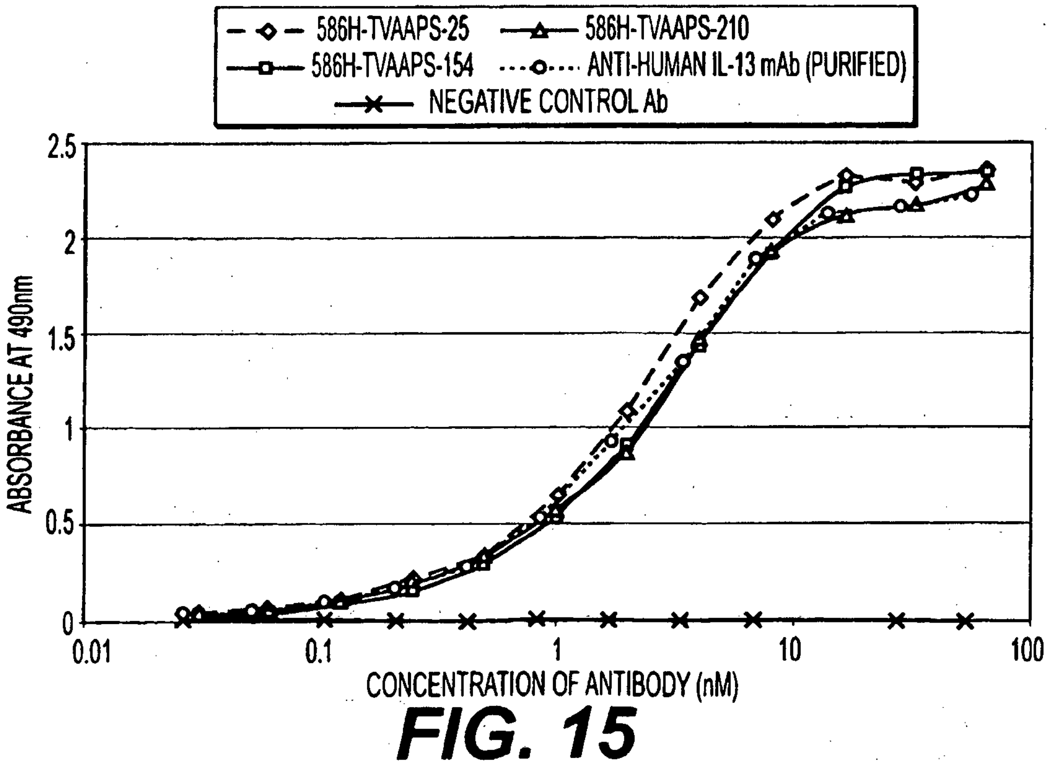
586H-TVAAPS-25
586H-TVAAPS-210
586H-TVAAPS-154
ANTI-HUMAN IL-13 mAb (PURIFIED)
NEGATIVE CONTROL Ab
ABSORBANCE AT 490nm
CONCENTRATION OF ANTIBODY (nM)

FIG. 15

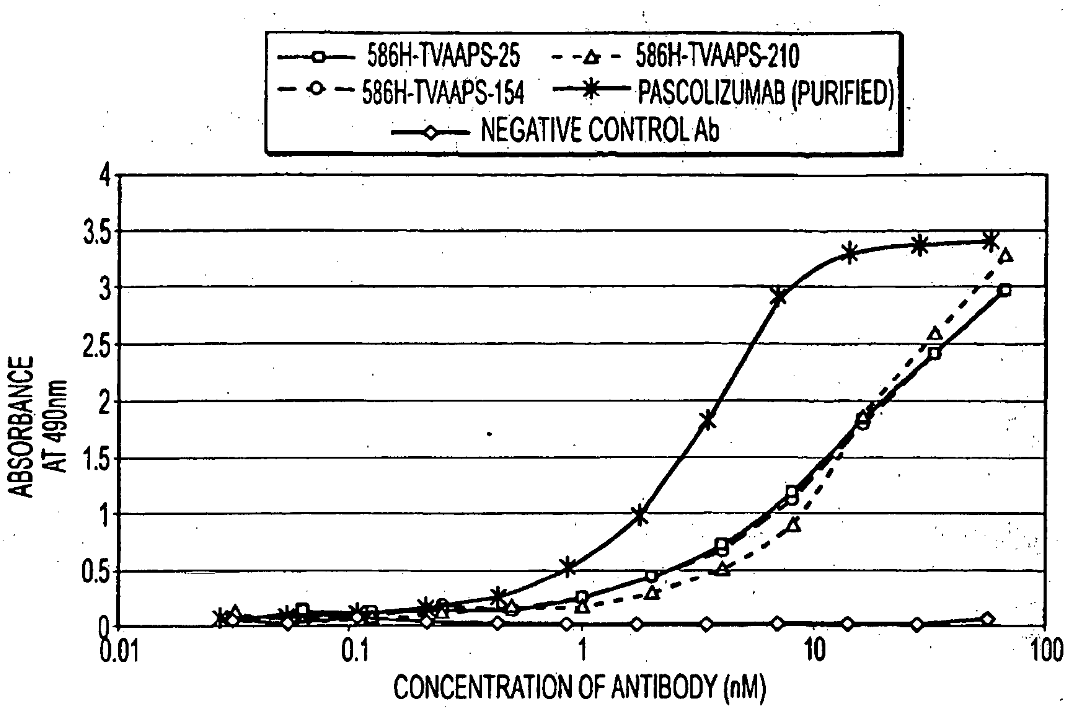
586H-TVAAPS-25
586H-TVAAPS-210
586H-TVAAPS-154
PASCOLIZUMAB (PURIFIED)
NEGATIVE CONTROL Ab
ABSORBANCE AT 490nm
CONCENTRATION OF ANTIBODY (nM)
4
3.5
3
2.5
2
1.5
1
0.5
0
0.01
0.1
1
10
100

FIG. 16

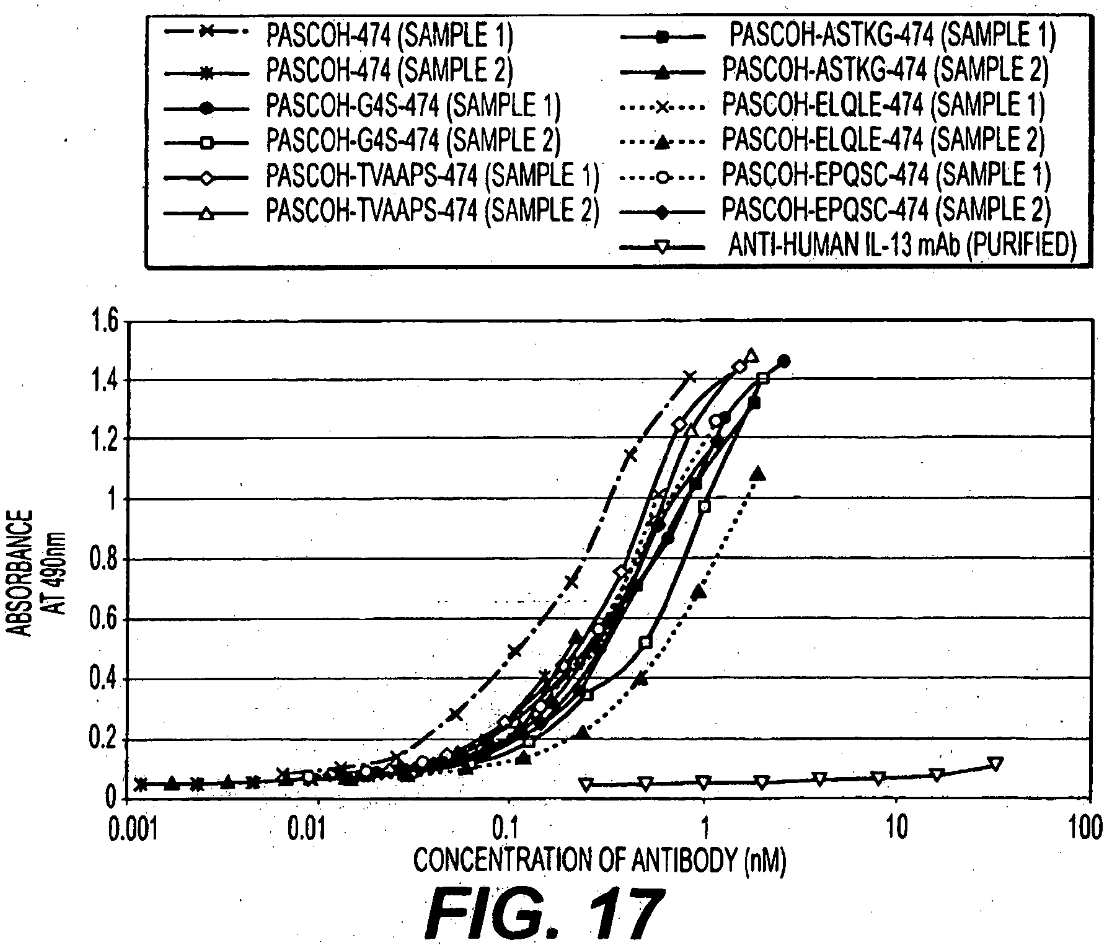
PASCOH-474 (SAMPLE 1)
PASCOH-474 (SAMPLE 2)
PASCOH-G4S-474 (SAMPLE 1)
PASCOH-G4S-474 (SAMPLE 2)
PASCOH-TVAAPS-474 (SAMPLE 1)
PASCOH-TVAAPS-474 (SAMPLE 2)
PASCOH-ASTKG-474 (SAMPLE 1)
PASCOH-ASTKG-474 (SAMPLE 2)
PASCOH-ELQLE-474 (SAMPLE 1)
PASCOH-ELQLE-474 (SAMPLE 2)
PASCOH-EPQSC-474 (SAMPLE 1)
PASCOH-EPQSC-474 (SAMPLE 2)
ANTI-HUMAN IL-13 mAb (PURIFIED)
ABSORBANCE AT 490nm
CONCENTRATION OF ANTIBODY (nM)
1.6
1.4
1.2
1
0.8
0.6
0.4
0.2
0
0.001
0.01
0.1
1
10
100

FIG. 17

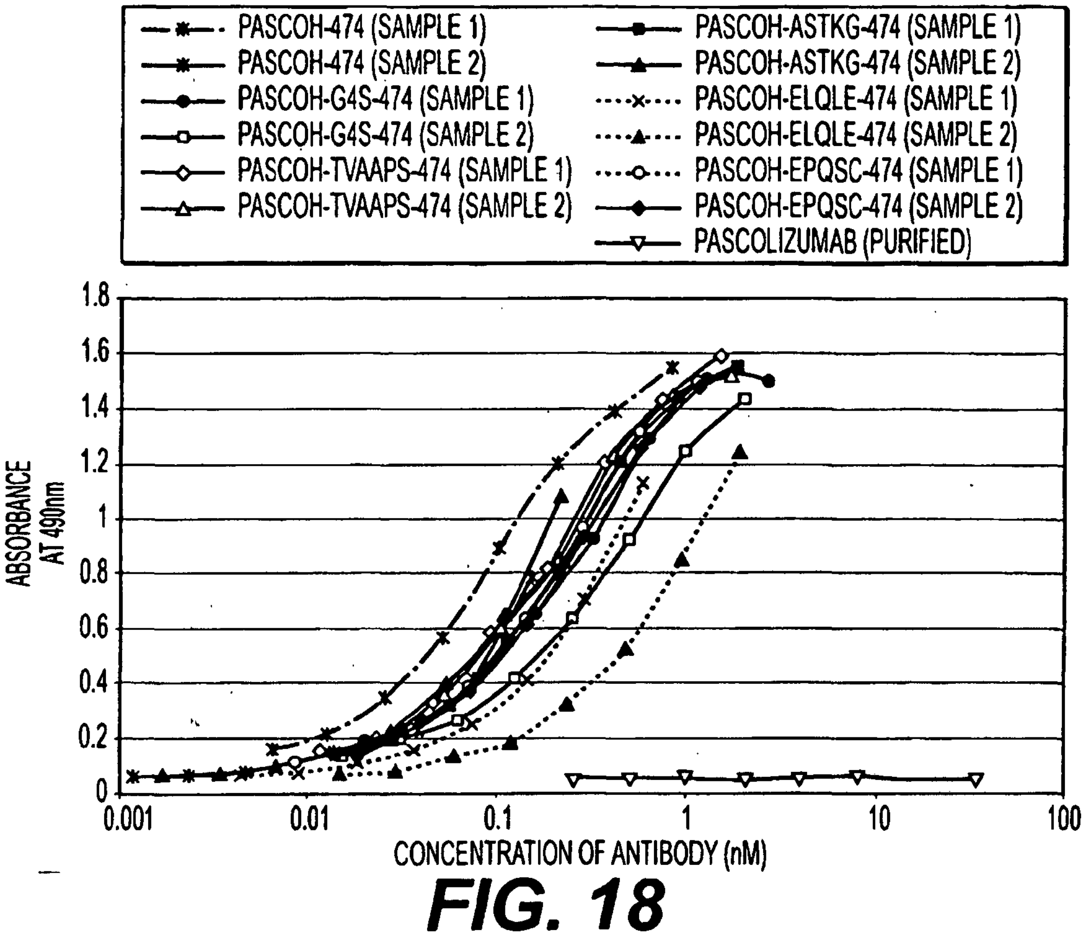
PASCOH-474 (SAMPLE 1)
PASCOH-474 (SAMPLE 2)
PASCOH-G4S-474 (SAMPLE 1)
PASCOH-G4S-474 (SAMPLE 2)
PASCOH-TVAAPS-474 (SAMPLE 1)
PASCOH-TVAAPS-474 (SAMPLE 2)
PASCOH-ASTKG-474 (SAMPLE 1)
PASCOH-ASTKG-474 (SAMPLE 2)
PASCOH-ELQLE-474 (SAMPLE 1)
PASCOH-ELQLE-474 (SAMPLE 2)
PASCOH-EPQSC-474 (SAMPLE 1)
PASCOH-EPQSC-474 (SAMPLE 2)
PASCOLIZUMAB (PURIFIED)
ABSORBANCE AT 490nm
1.8
1.6
1.4
1.2
1
0.8
0.6
0.4
0.2
0
0.001
0.01
0.1
1
10
100
CONCENTRATION OF ANTIBODY (nM)

*FIG. 18*

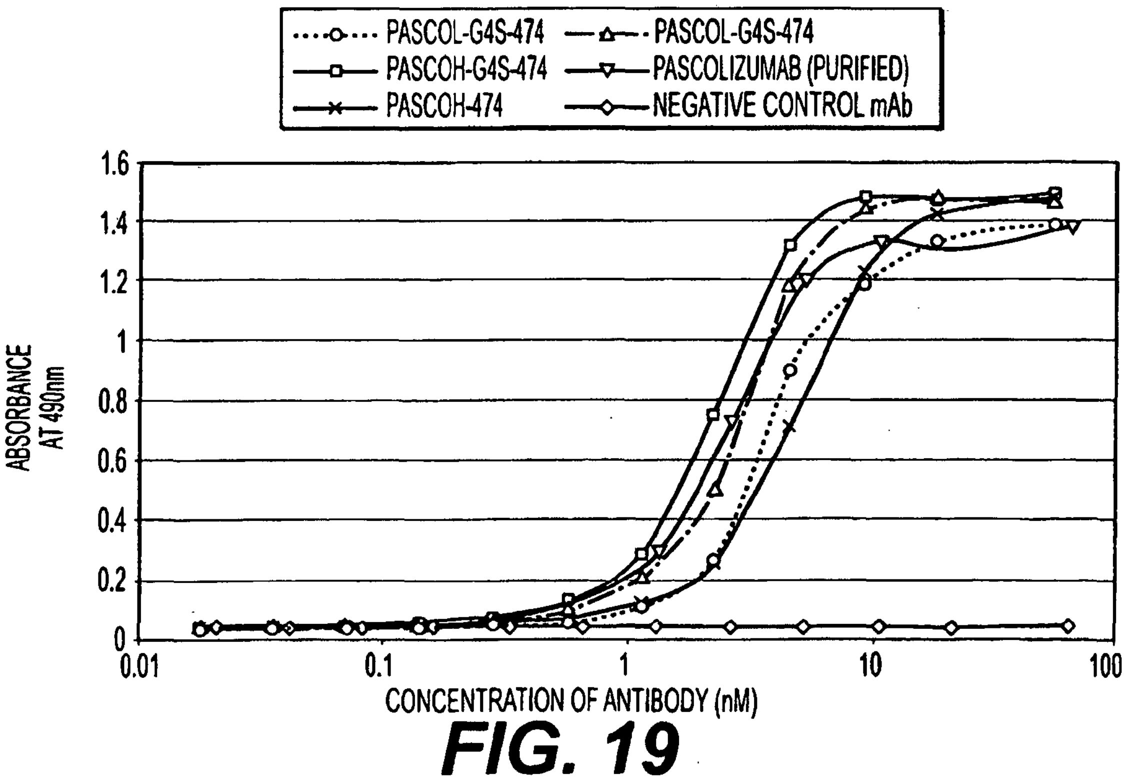
PASCOL-G4S-474
PASCOL-G4S-474
PASCOH-G4S-474
PASCOLIZUMAB (PURIFIED)
PASCOH-474
NEGATIVE CONTROL mAb
ABSORBANCE AT 490nm
1.6
1.4
1.2
1
0.8
0.6
0.4
0.2
0
0.01
0.1
1
10
100
CONCENTRATION OF ANTIBODY (nM)

*FIG. 19*

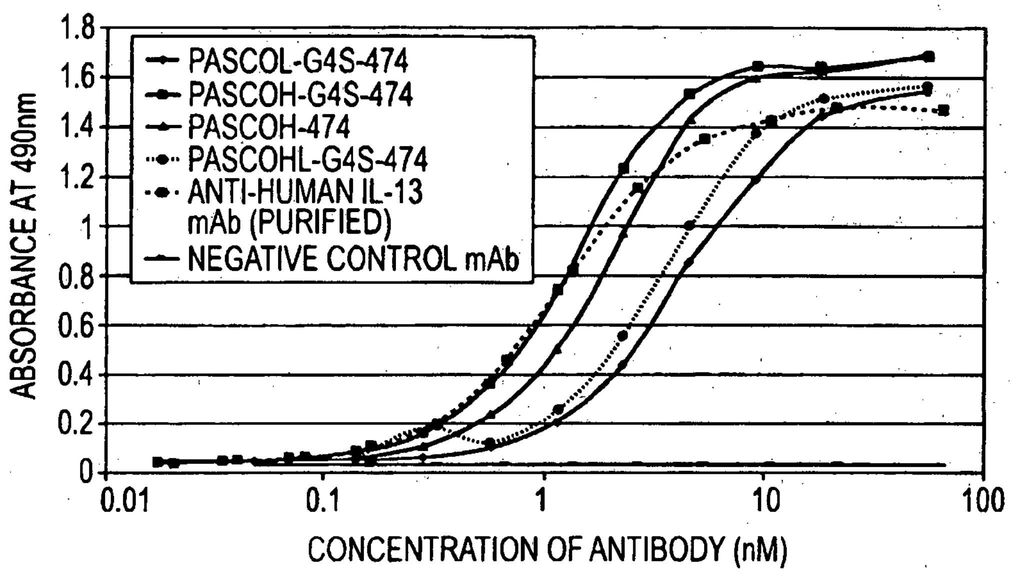


*FIG. 20A*

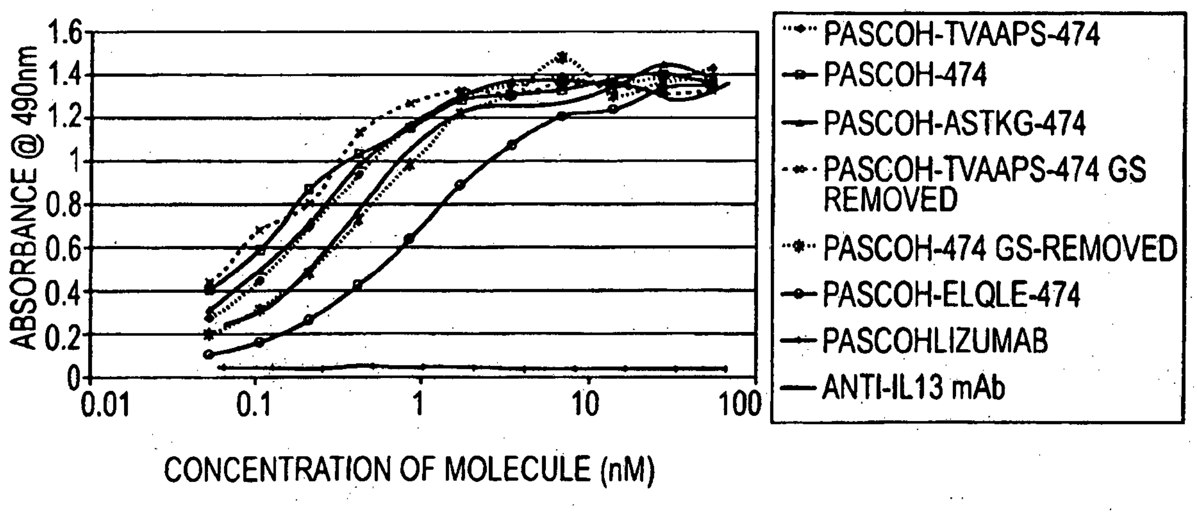


*FIG. 20B*

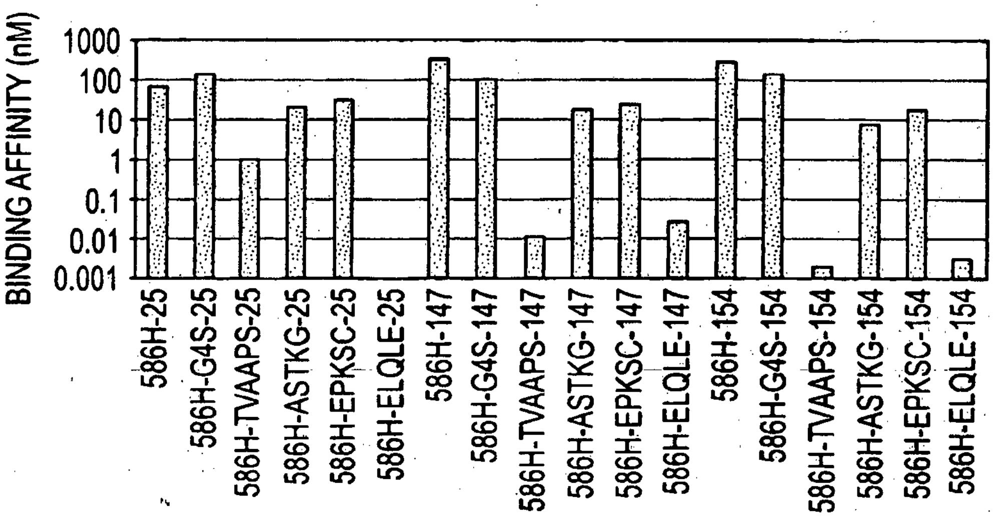
BINDING AFFINITY (nM)
1000
100
10
1
0.1
0.01
0.001
586H-25
586H-G4S-25
586H-TVAAPS-25
586H-ASTKG-25
586H-EPKSC-25
586H-ELQLE-25
586H-147
586H-G4S-147
586H-TVAAPS-147
586H-ASTKG-147
586H-EPKSC-147
586H-ELQLE-147
586H-154
586H-G4S-154
586H-TVAAPS-154
586H-ASTKG-154
586H-EPKSC-154
586H-ELQLE-154

*FIG. 21*

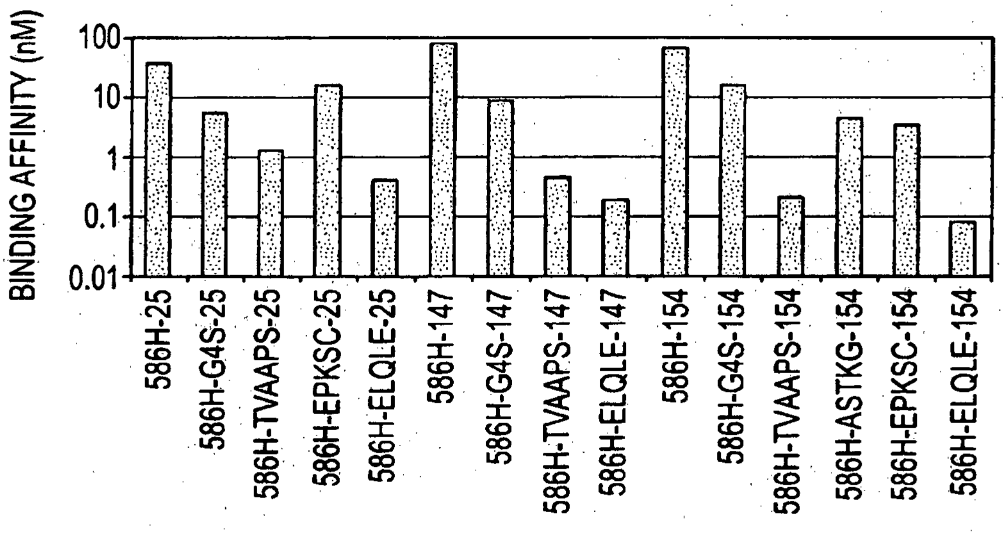
BINDING AFFINITY (nM)
100
10
1
0.1
0.01
586H-25
586H-G4S-25
586H-TVAAPS-25
586H-EPKSC-25
586H-ELQLE-25
586H-147
586H-G4S-147
586H-TVAAPS-147
586H-ELQLE-147
586H-154
586H-G4S-154
586H-TVAAPS-154
586H-ASTKG-154
586H-EPKSC-154
586H-ELQLE-154

*FIG. 22*

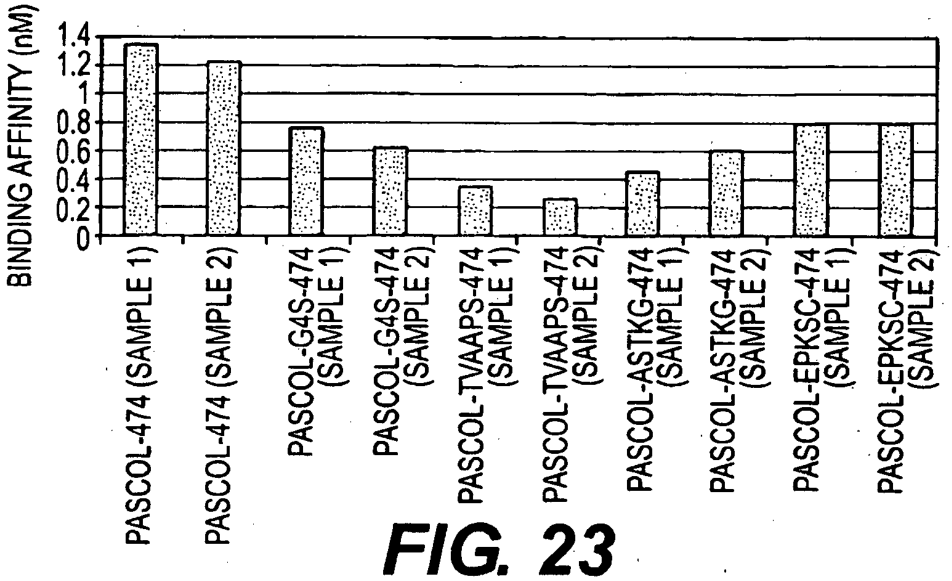


*FIG. 23*

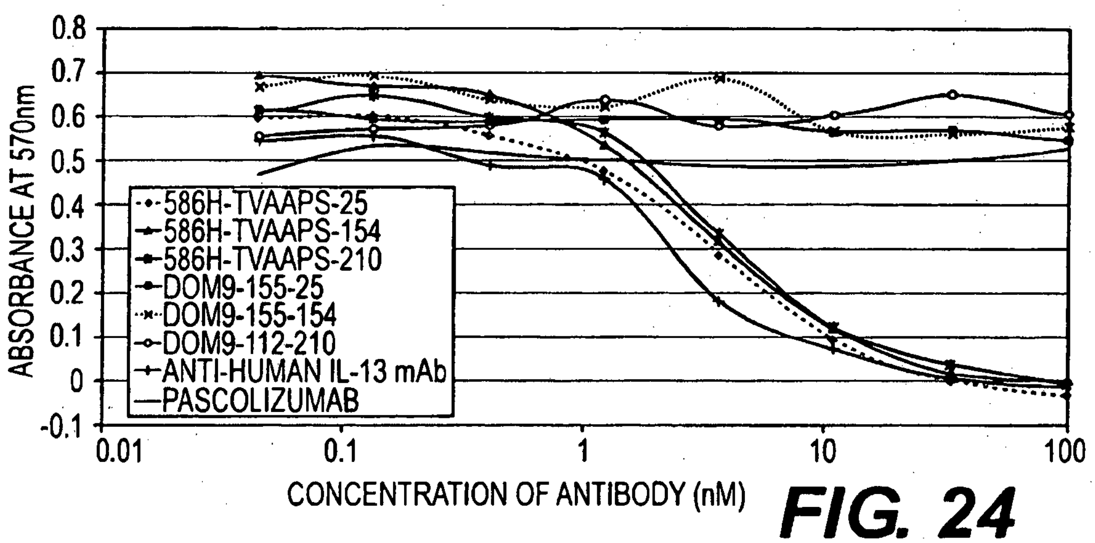


*FIG. 24*

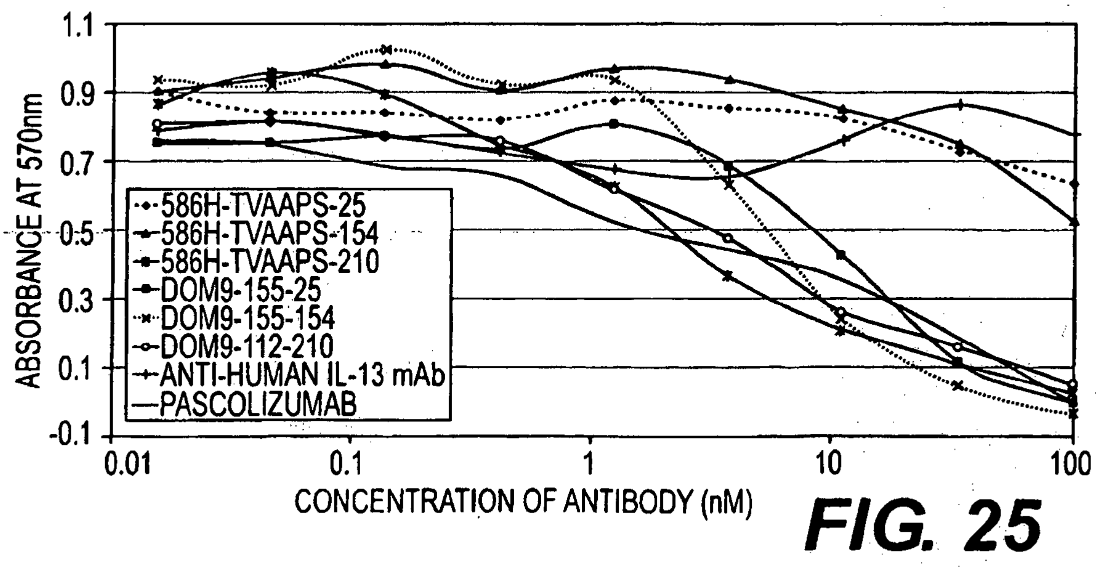


*FIG. 25*

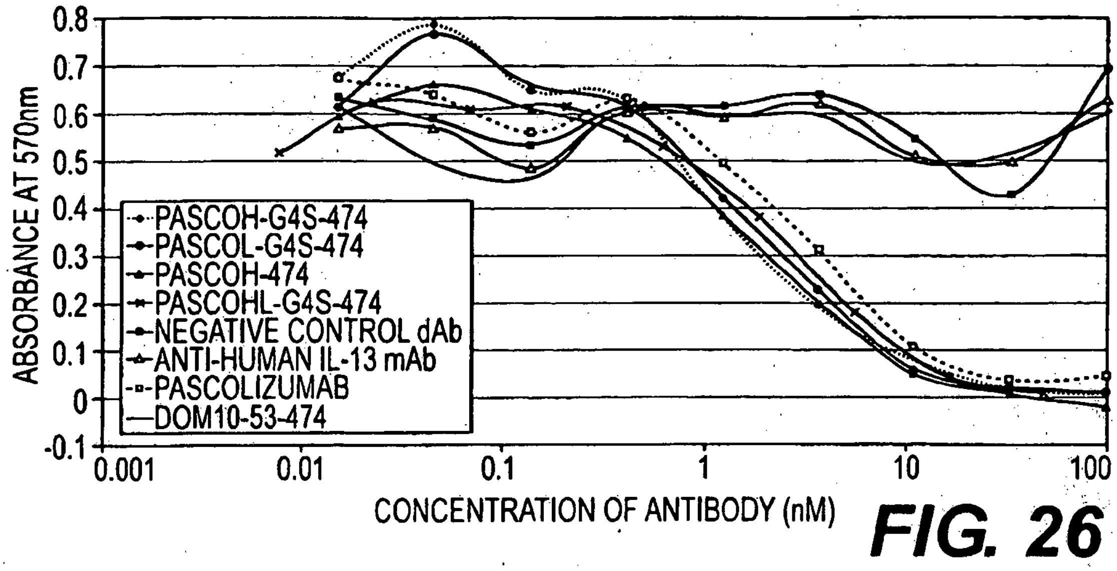


FIG. 26

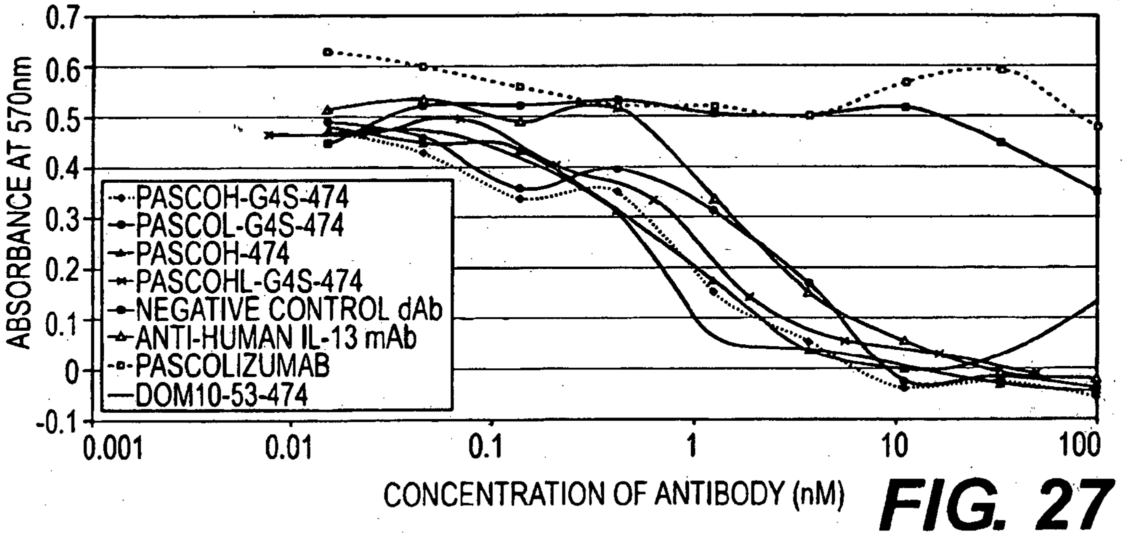


FIG. 27

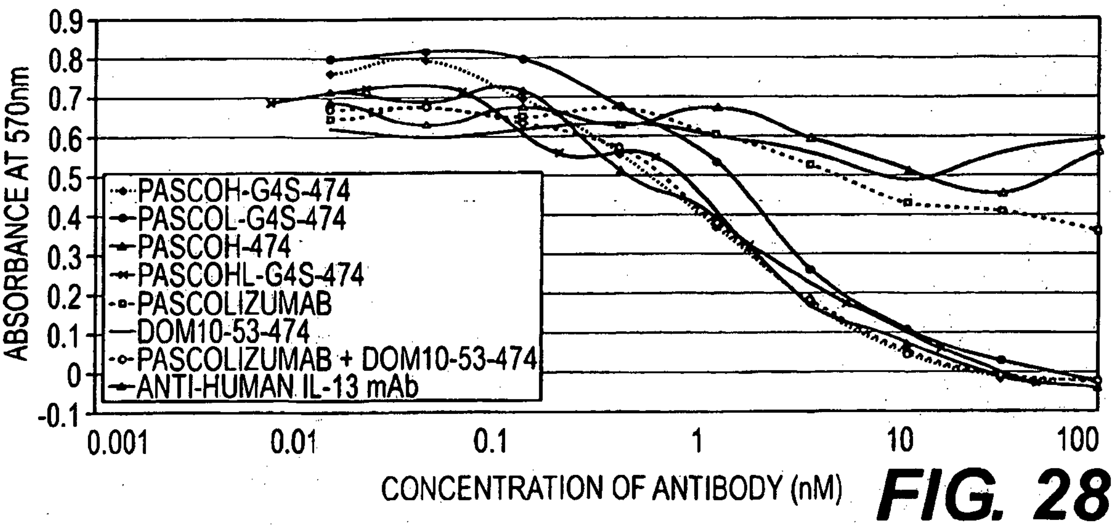


FIG. 28

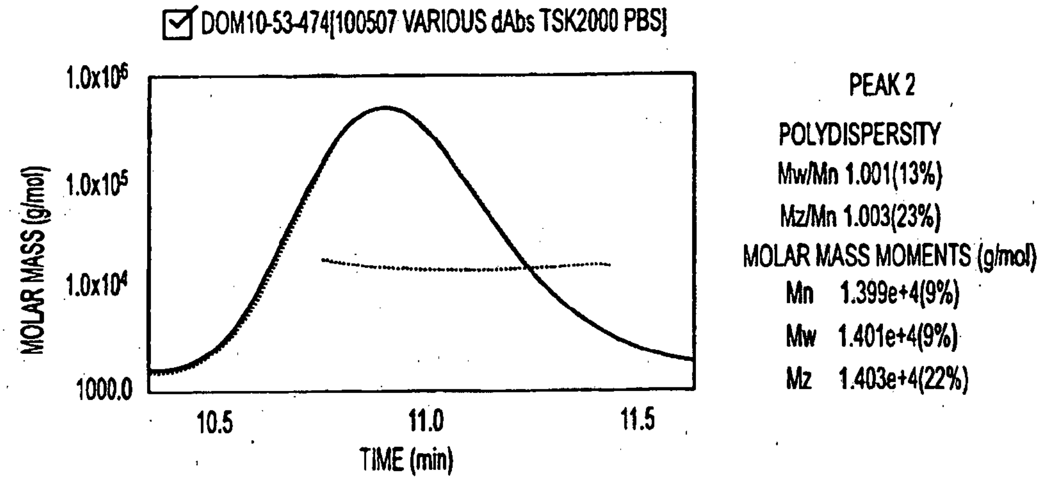


*FIG. 29*

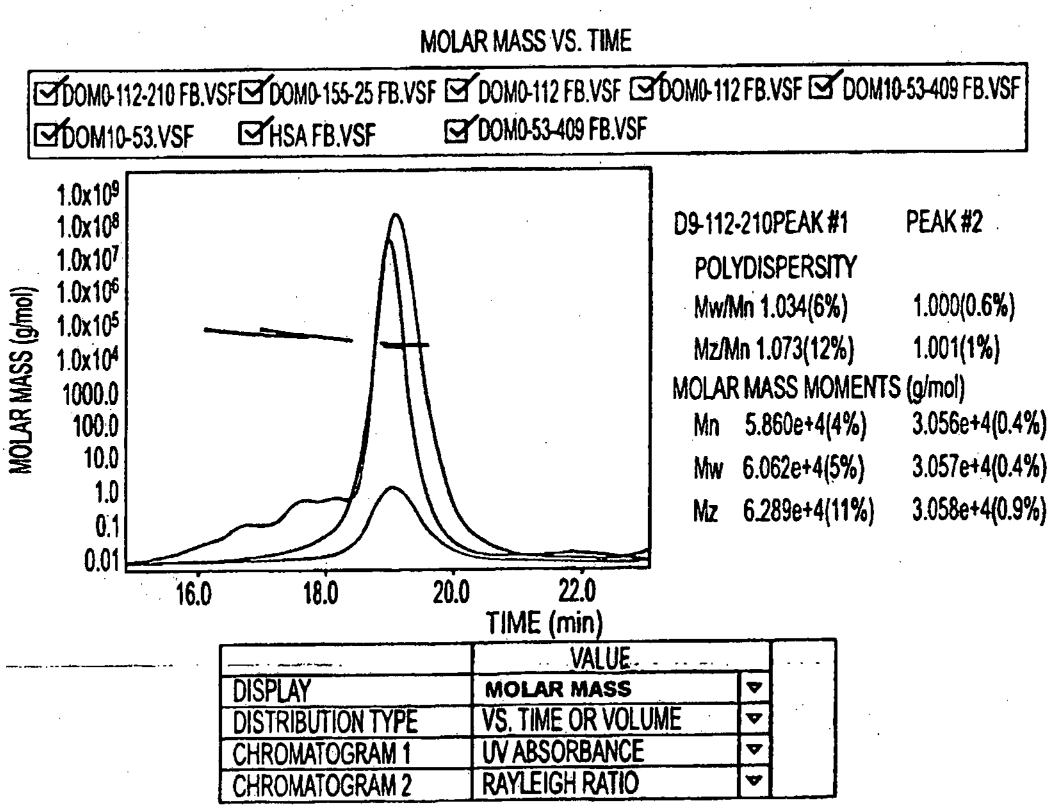


| | VALUE |
|---|---|
| DISPLAY | MOLAR MASS |
| DISTRIBUTION TYPE | VS. TIME OR VOLUME |
| CHROMATOGRAM 1 | UV ABSORBANCE |
| CHROMATOGRAM 2 | RAYLEIGH RATIO |

*FIG. 30*

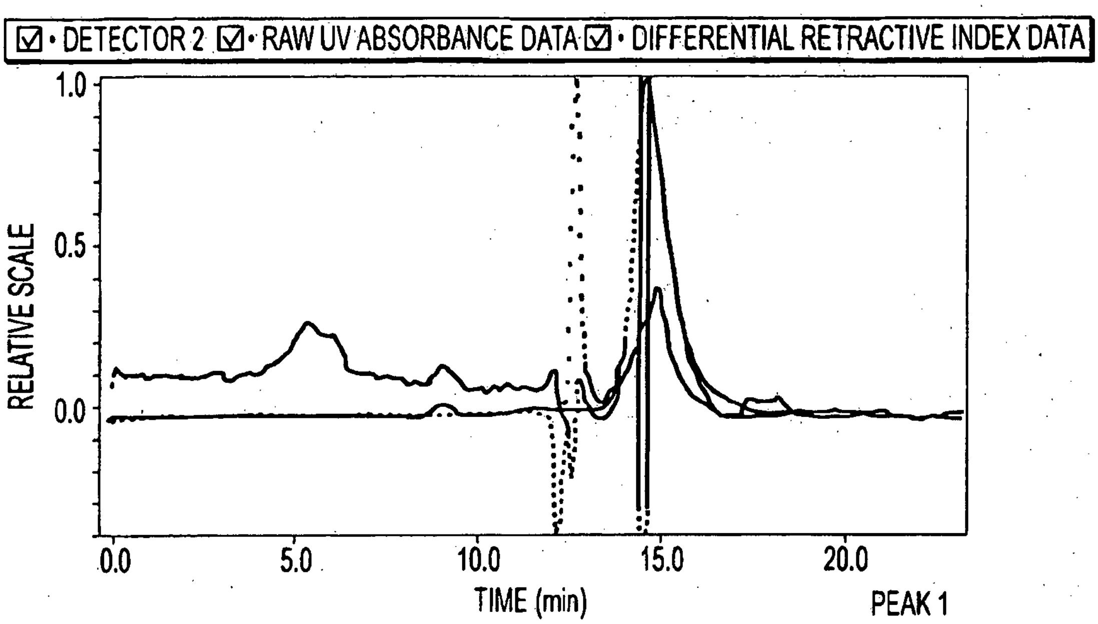


POLY DISPERSITY
Mw/Mn 1.000 (20%)
Mz/Mn 1.000 (34%)
MOLAR MASS MOMENTS (g/mol)
Mn 2.800e+4 (14%)
Mw 2.801e+4 (14%)
Mz 2.802e+4 (31%)

*FIG. 31*

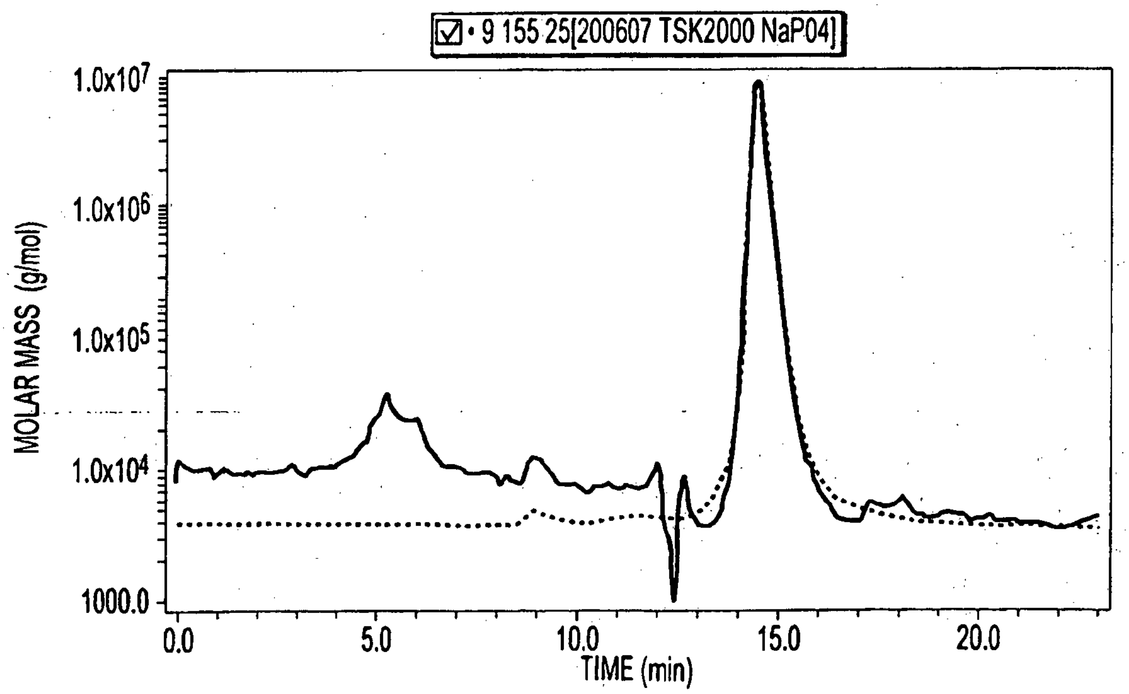


*FIG. 32*

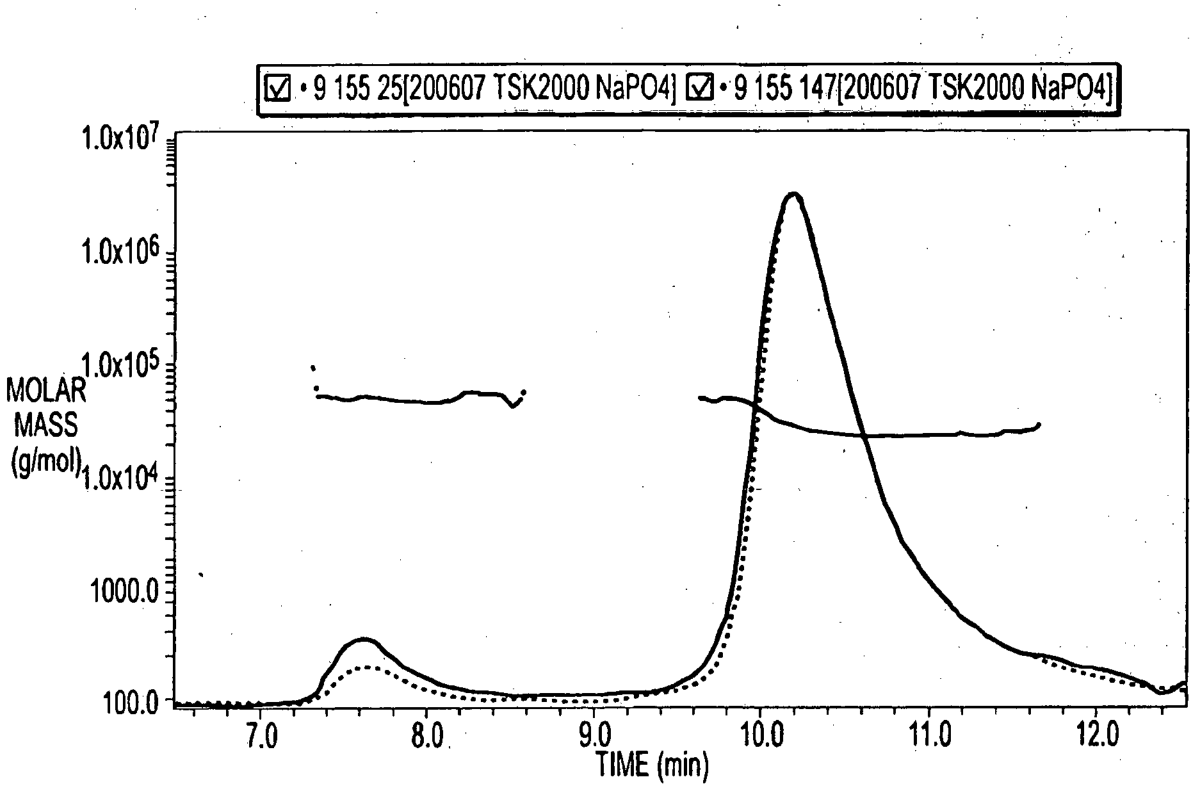


| | PEAK 1 | PEAK 2 (ALL) |
|---|---|---|
| POLYDISPERSITY | | |
| Mw/Mn | 1.002 (6%) | 1.039 (9%) |
| Mz/Mn | 1.003 (10%) | 1.092 (19%) |
| MOLAR MASS MOMENTS (g/mol) | | |
| Mn | 5.041e+4 (4%) | 2.942e+4 (5%) |
| Mw | 5.049e+4 (4%) | 3.058e+4 (7%) |
| Mz | 5.057e+4 (9%) | 3.214e+4 (18%) |

PEAK 2 (RIGHT PART)

| POLYDISPERSITY | |
|---|---|
| Mw/Mn | 1.001 (4%) |
| Mz/Mn | 1.002 (7%) |
| MOLAR MASS MOMENTS (g/mol) | |
| Mn | 2.589e+4 (3%) |
| Mw | 2.591e+4 (3%) |
| Mz | 2.593e+4 (6%) |

*FIG. 33*

PEAK 3

POLYDISPERSITY

Mw/Mn 1.011 (10%)
Mz/Mn 1.022 (19%)

MOLAR MASS MOMENTS (g/mol)

| | |
|---|---|
| Mn | 2.740e+4 (7%) |
| Mw | 2.769e+4 (7%) |
| Mz | 2.801e+4 (17%) |

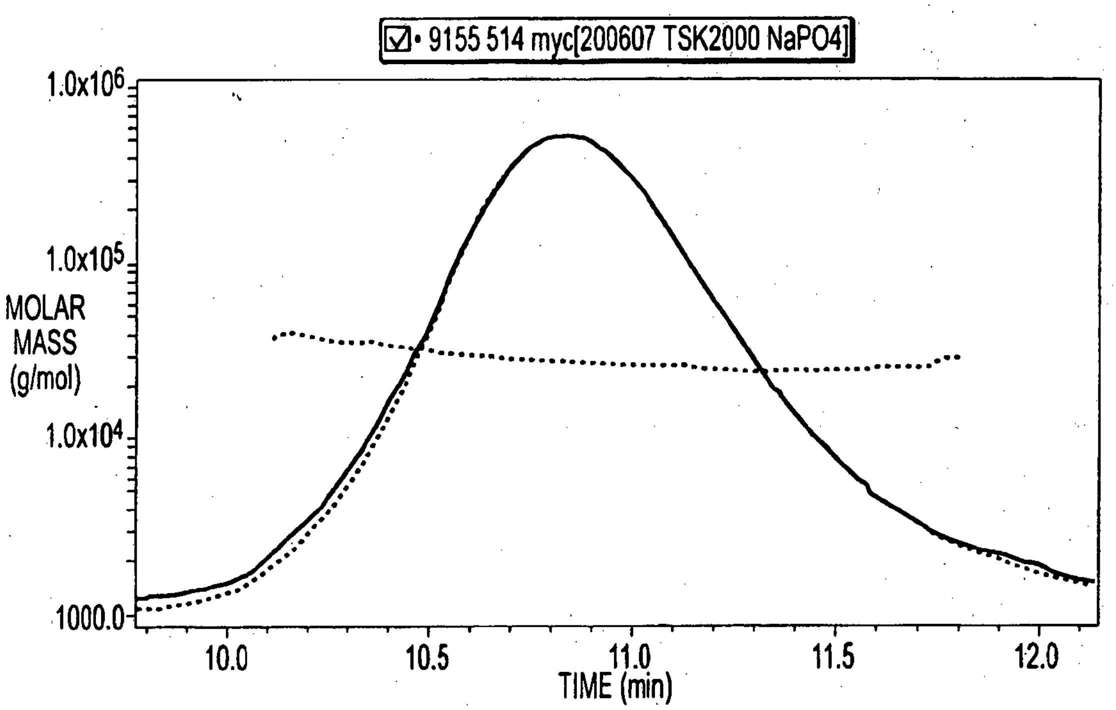


*FIG. 34*

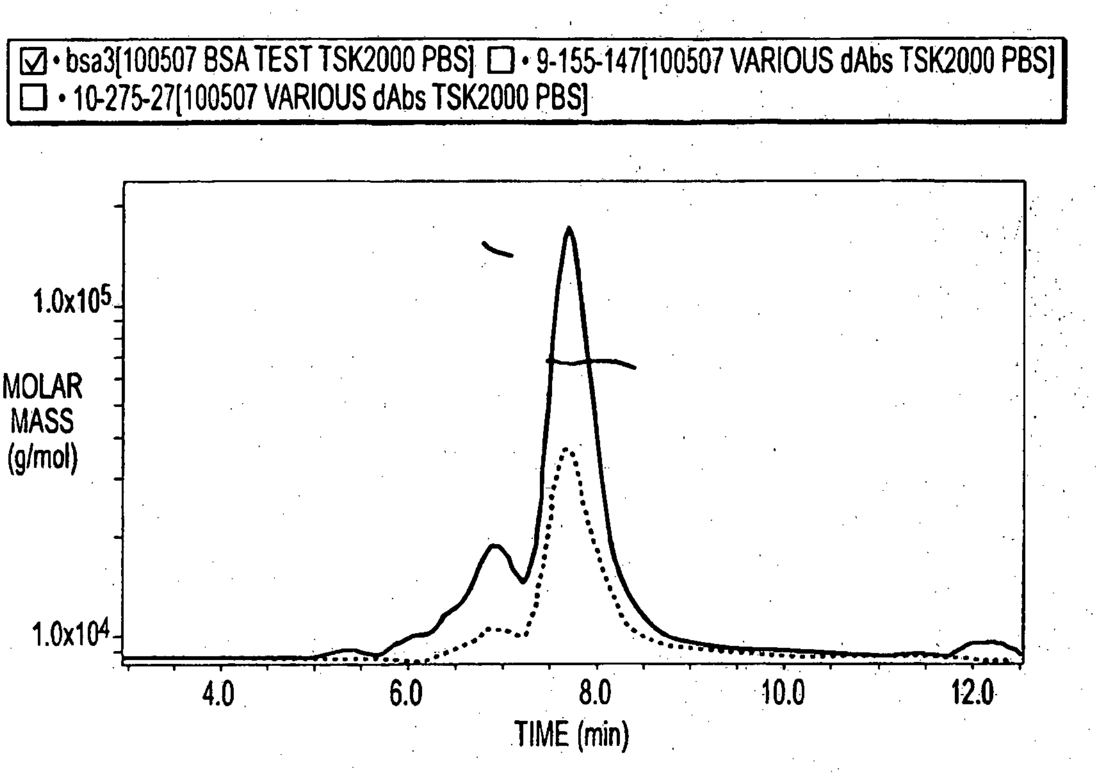


| | PEAK 2 | PEAK 3 |
|---|---|---|
| POLYDISPERSITY | | |
| Mw/Mn | 1.000 (2%) | 1.001 (3%) |
| Mz/Mn | 1.000 (3%) | 1.001 (6%) |
| MOLAR MASS MOMENTS (g/mol) | | |
| Mn | 6.735e+4 (1%) | 1.449e+5 (2%) |
| Mw | 6.736e+4 (1%) | 1.450e+5 (2%) |
| Mz | 6.737e+4 (3%) | 1.451e+5 (5%) |

*FIG. 35*

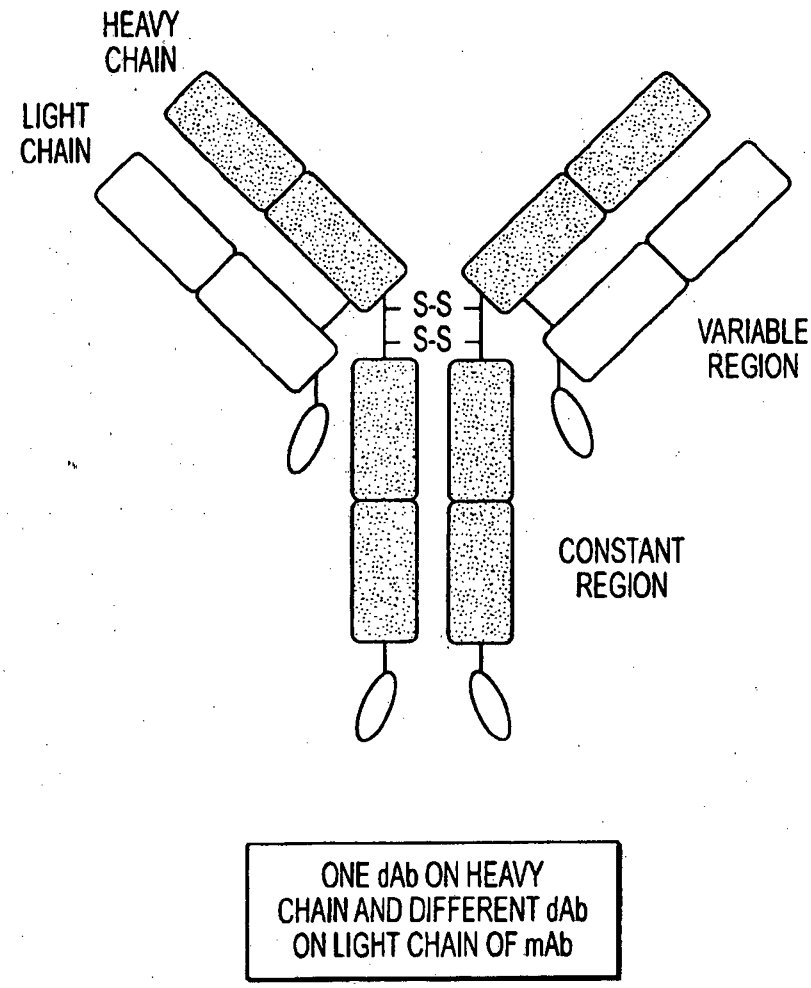
HEAVY
CHAIN
LIGHT
CHAIN
S-S
S-S
VARIABLE
REGION
CONSTANT
REGION
ONE dAb ON HEAVY
CHAIN AND DIFFERENT dAb
ON LIGHT CHAIN OF mAb

FIG. 36

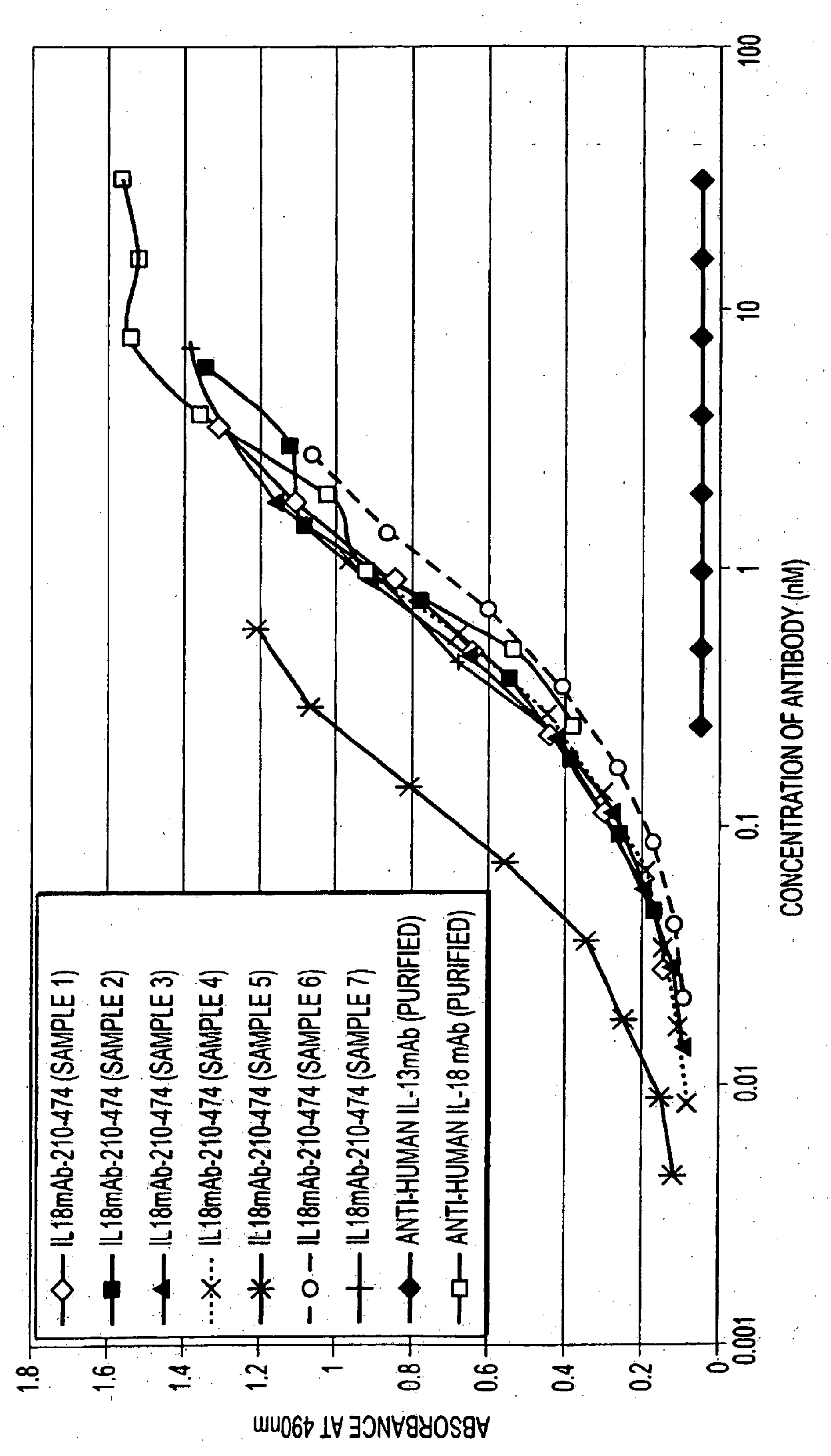
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
ANTI-HUMAN IL-13mAb (PURIFIED)
ANTI-HUMAN IL-18 mAb (PURIFIED)
ABSORBANCE AT 490nm
1.8
1.6
1.4
1.2
1
0.8
0.6
0.4
0.2
0
0.001
0.01
0.1
1
10
100
CONCENTRATION OF ANTIBODY (nM)

*FIG. 37*

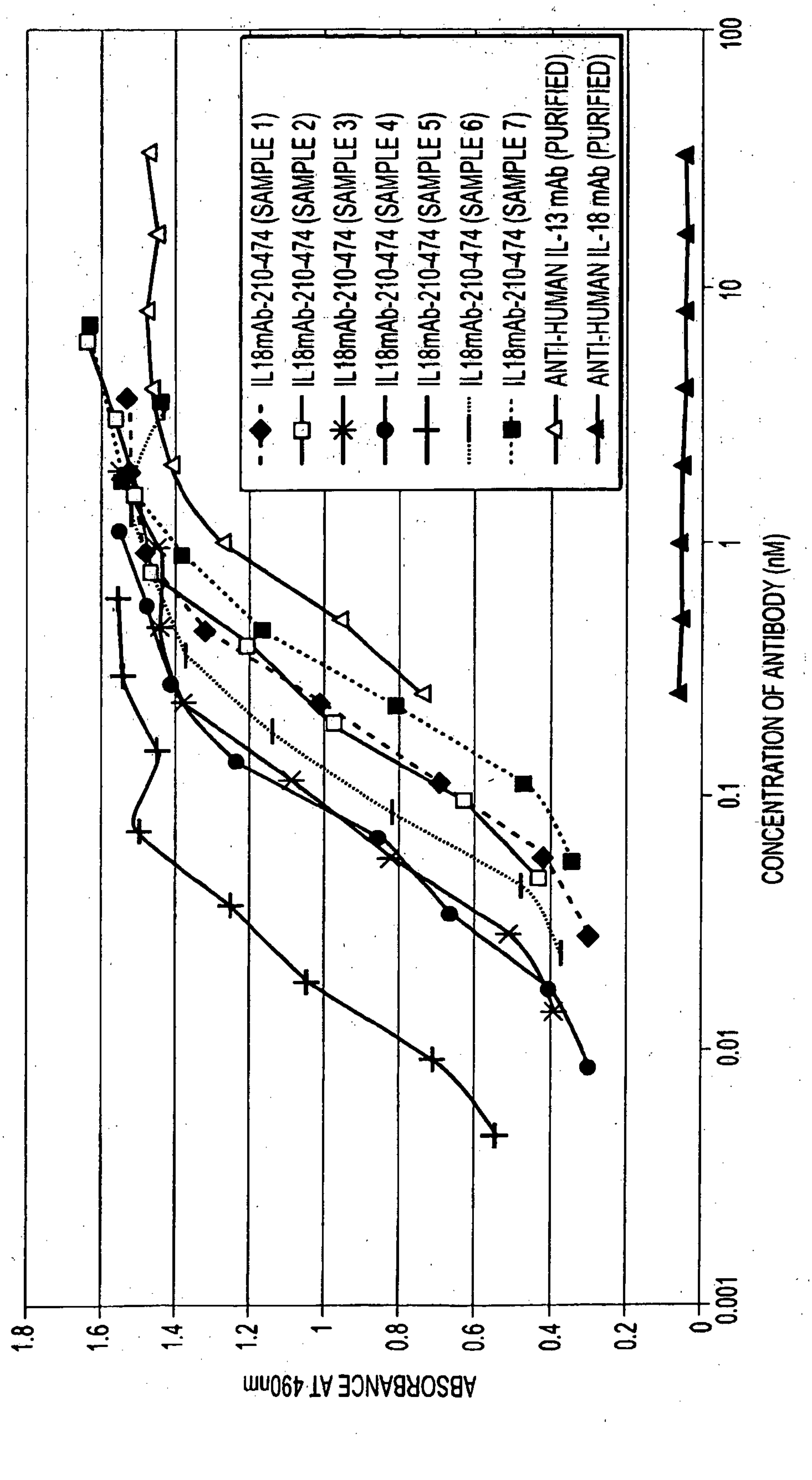
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
ANTI-HUMAN IL-13 mAb (PURIFIED)
ANTI-HUMAN IL-18 mAb (PURIFIED)
ABSORBANCE AT 490nm
1.8
1.6
1.4
1.2
1
0.8
0.6
0.4
0.2
0
0.001
0.01
0.1
1
10
100
CONCENTRATION OF ANTIBODY (nM)

FIG. 38

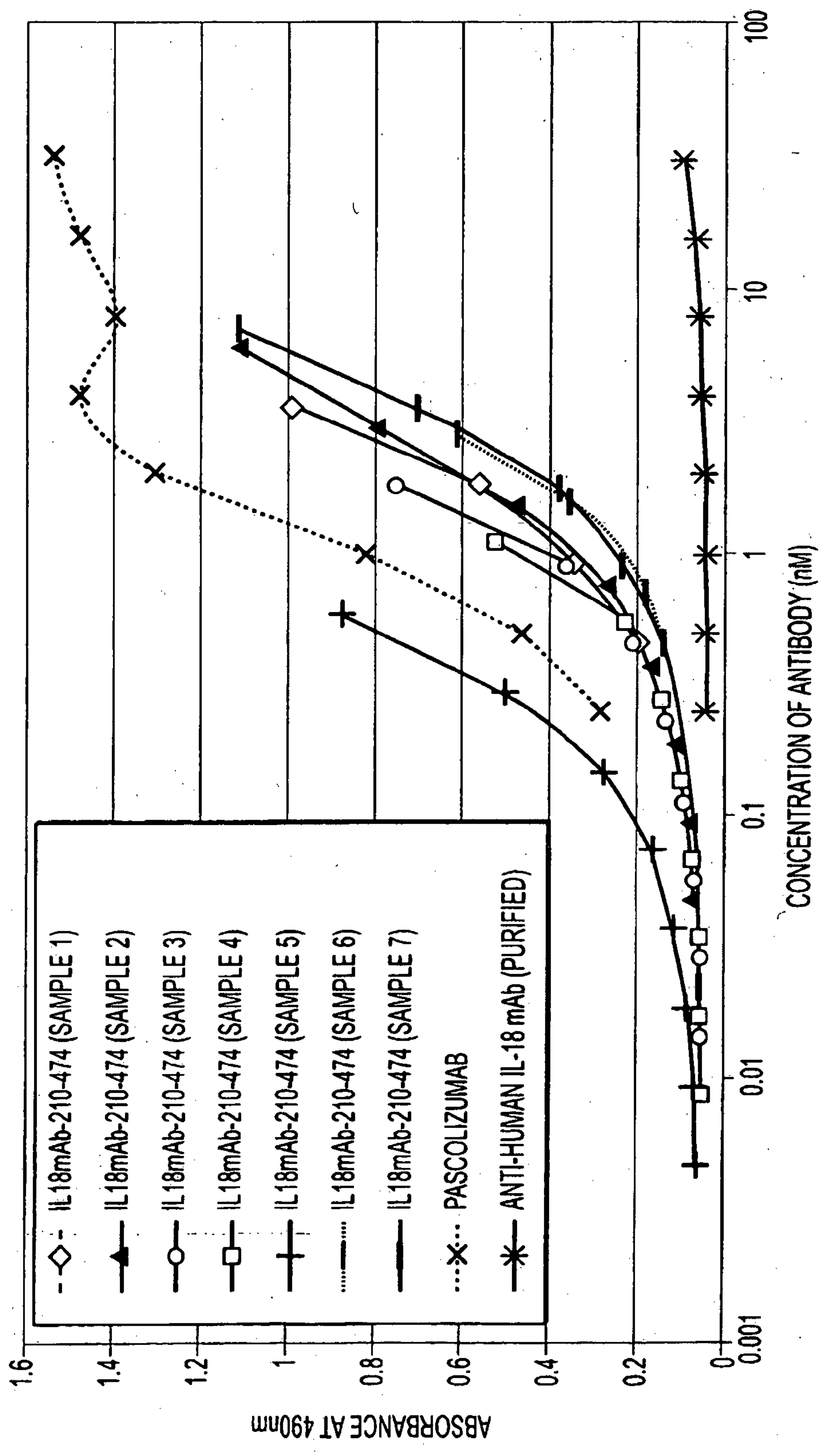
ABSORBANCE AT 490nm
1.6
1.4
1.2
1
0.8
0.6
0.4
0.2
0
0.001
0.01
0.1
1
10
100
CONCENTRATION OF ANTIBODY (nM)
IL18mAb-210-474 (SAMPLE 1)
IL18mAb-210-474 (SAMPLE 2)
IL18mAb-210-474 (SAMPLE 3)
IL18mAb-210-474 (SAMPLE 4)
IL18mAb-210-474 (SAMPLE 5)
IL18mAb-210-474 (SAMPLE 6)
IL18mAb-210-474 (SAMPLE 7)
PASCOLIZUMAB
ANTI-HUMAN IL-18 mAb (PURIFIED)

FIG. 39

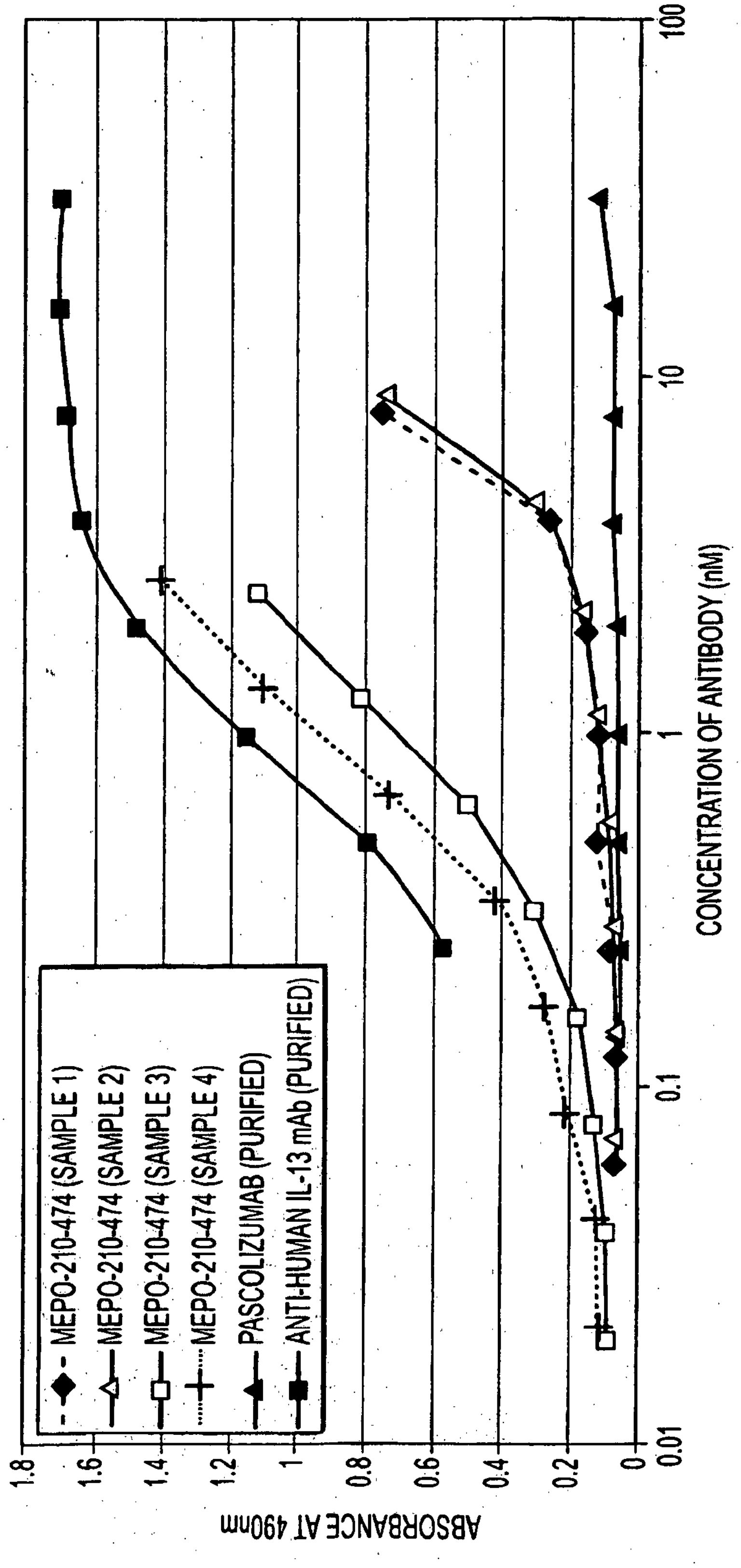
MEPO-210-474 (SAMPLE 1)
MEPO-210-474 (SAMPLE 2)
MEPO-210-474 (SAMPLE 3)
MEPO-210-474 (SAMPLE 4)
PASCOLIZUMAB (PURIFIED)
ANTI-HUMAN IL-13 mAb (PURIFIED)
ABSORBANCE AT 490nm
1.8
1.6
1.4
1.2
1
0.8
0.6
0.4
0.2
0
0.01
0.1
1
10
100
CONCENTRATION OF ANTIBODY (nM)

*FIG. 40*

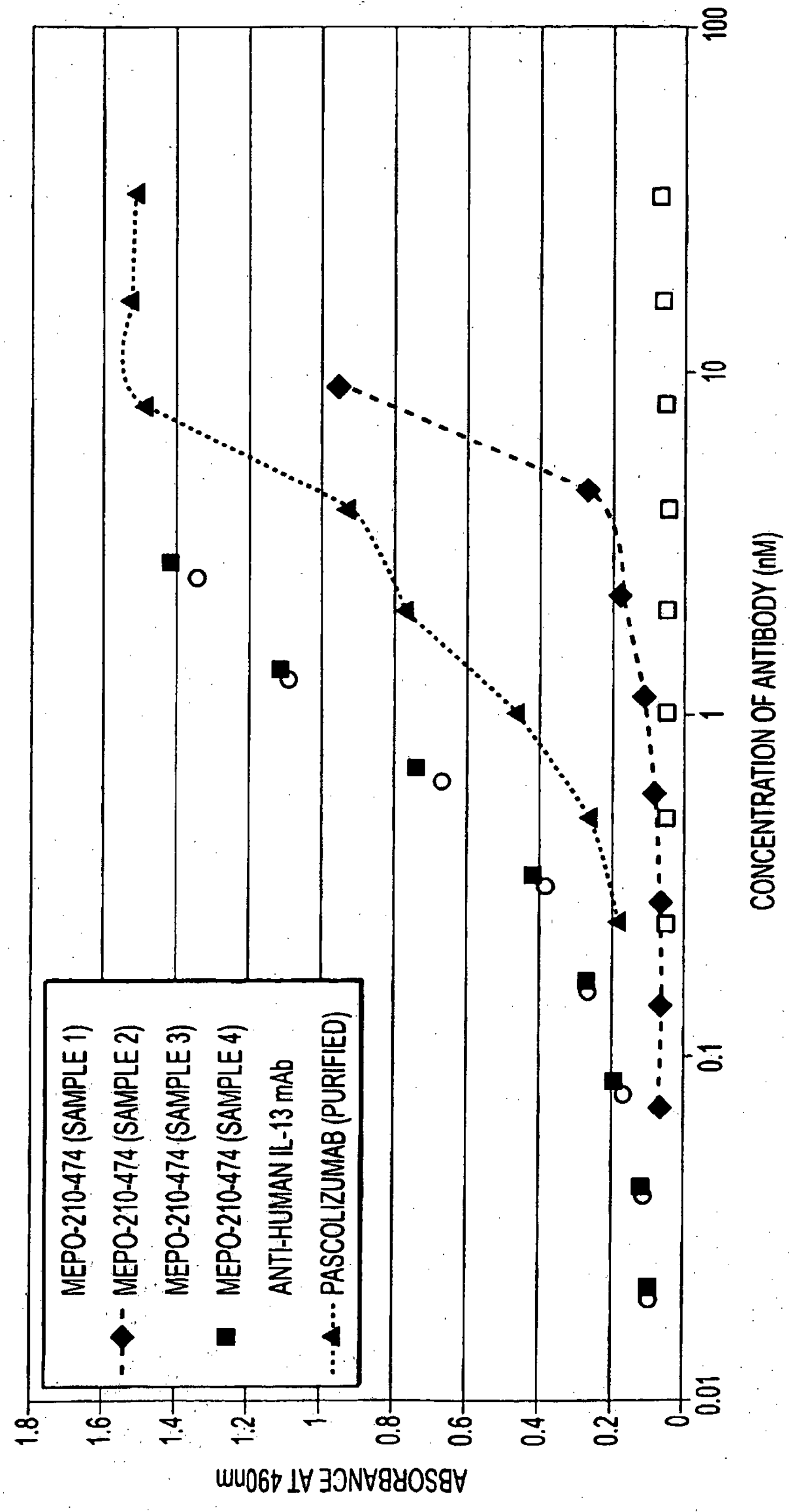
MEPO-210-474 (SAMPLE 1)
MEPO-210-474 (SAMPLE 2)
MEPO-210-474 (SAMPLE 3)
MEPO-210-474 (SAMPLE 4)
ANTI-HUMAN IL-13 mAb
PASCOLIZUMAB (PURIFIED)
ABSORBANCE AT 490nm
1.8
1.6
1.4
1.2
1
0.8
0.6
0.4
0.2
0
0.01
0.1
1
10
100
CONCENTRATION OF ANTIBODY (nM)

FIG. 41

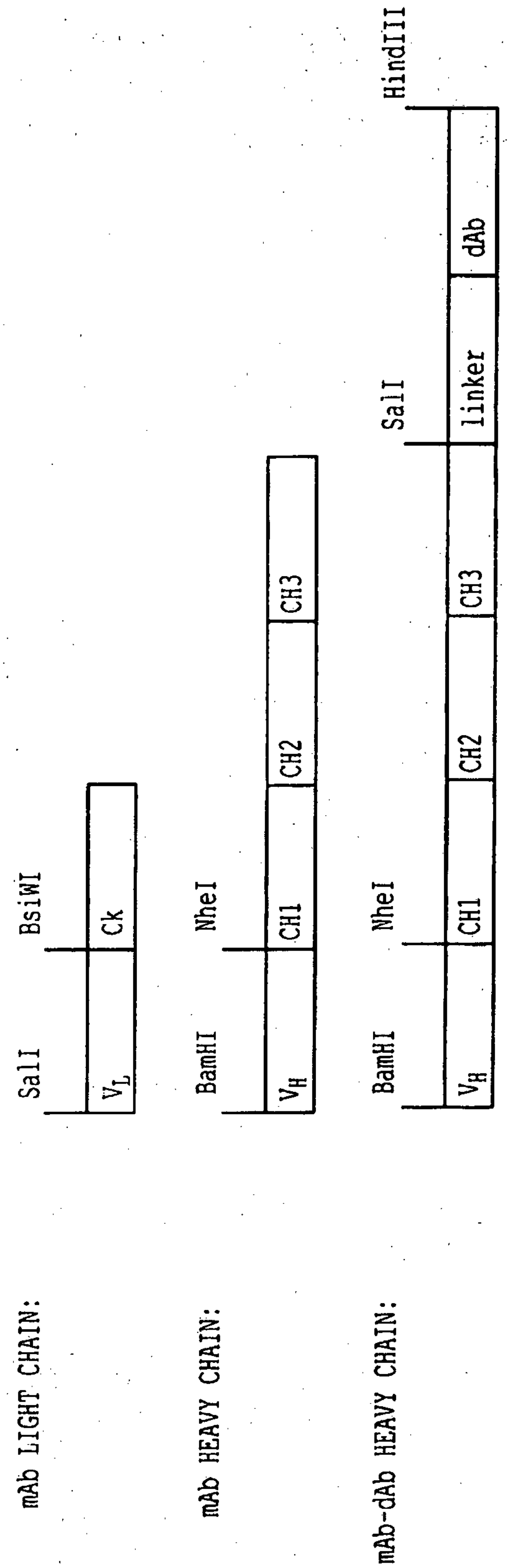


CLONING OF THE ANTI-TNF/ANTI-EGFR mAb-dAb (EXAMPLE 10)

FIG. 42

SDS-PAGE ANALYSIS OF THE ANTI-TNF/ANTI-EGFR mAb-dAb (EXAMPLE 10)

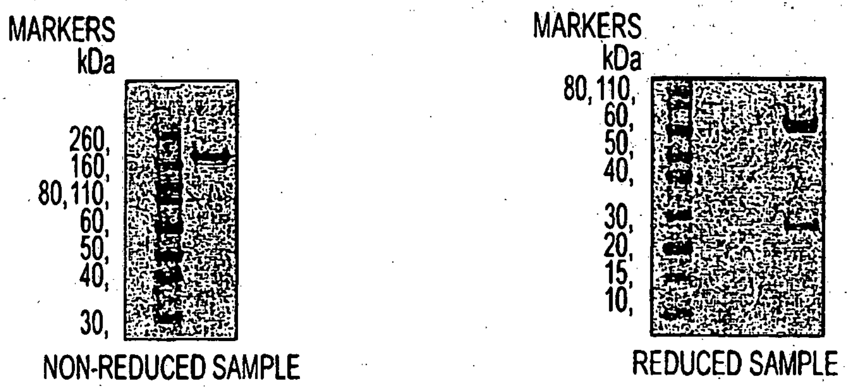


*FIG. 43*

SEC PROFILE OF THE ANTI-TNF/ANTI-EGFR mAb-dAb (EXAMPLE 10)

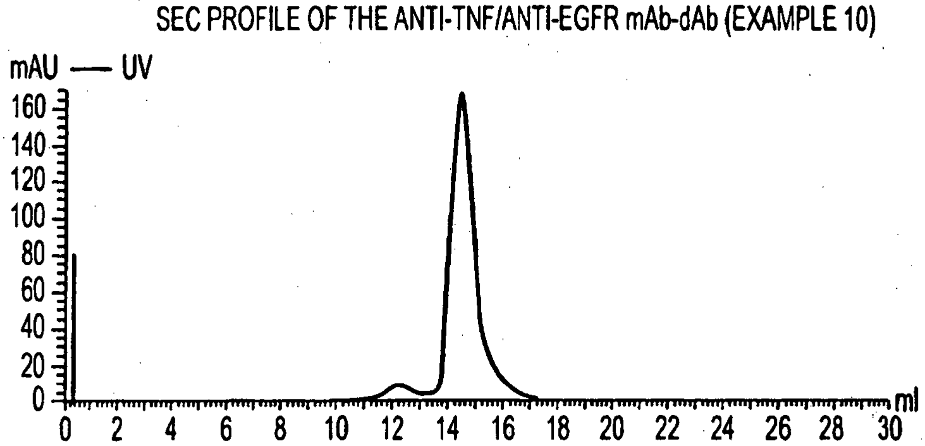


*FIG. 44*

ANTI-EGFR ACTIVITY OF EXAMPLE 10

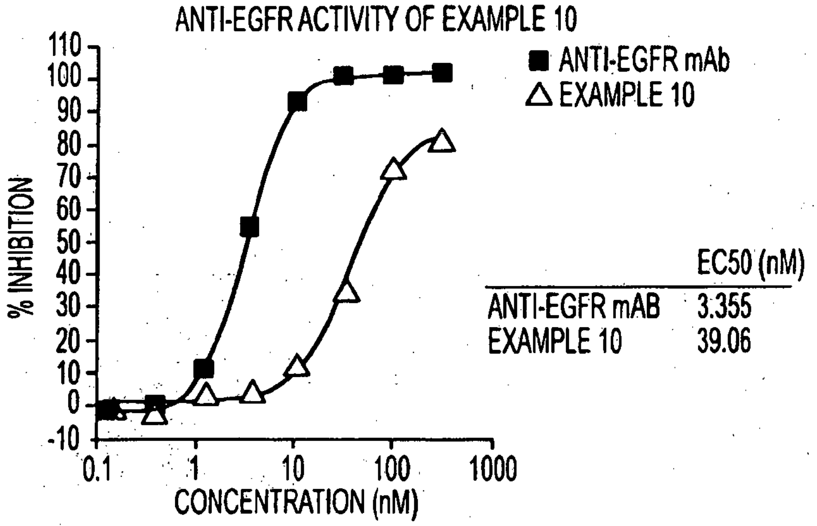


*FIG. 45*

ANTI-TNF ACTIVITY OF EXAMPLE 10

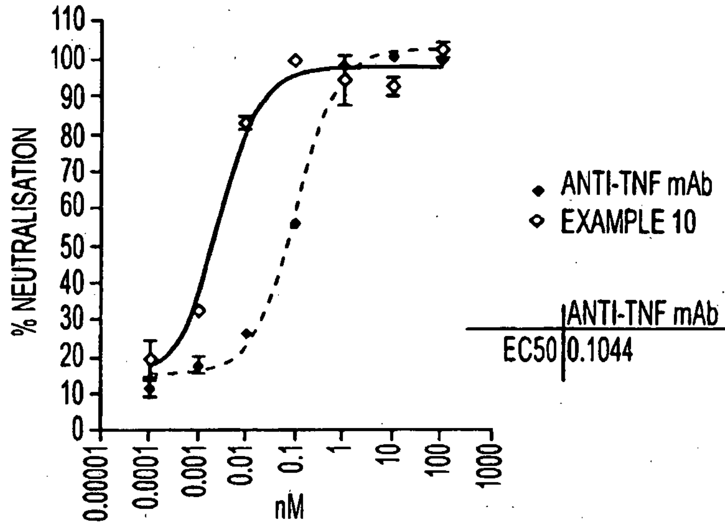


| | ANTI-TNF mAb | EXAMPLE 10 |
|---|---|---|
| EC50 | 0.1044 | 0.002862 |

*FIG. 46*

SDS-PAGE ANALYSIS OF THE ANTI-TNF/ANTI-VEGF mAb-dAb (EXAMPLE 11)

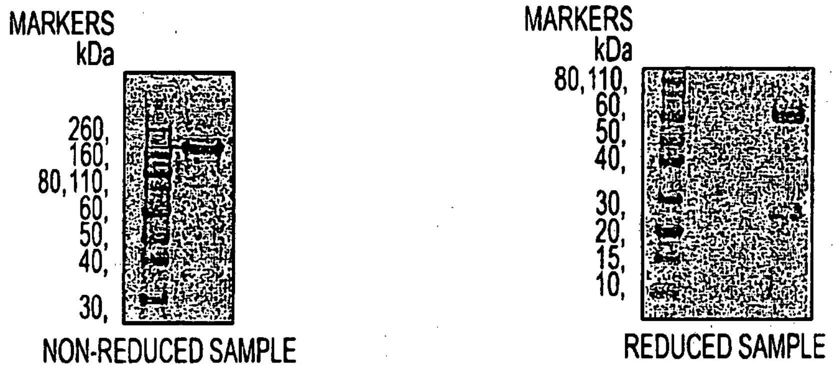


*FIG. 47*

SEC PROFILE OF THE ANTI-TNF/ANTI-VEGF mAb-dAb (EXAMPLE 11)

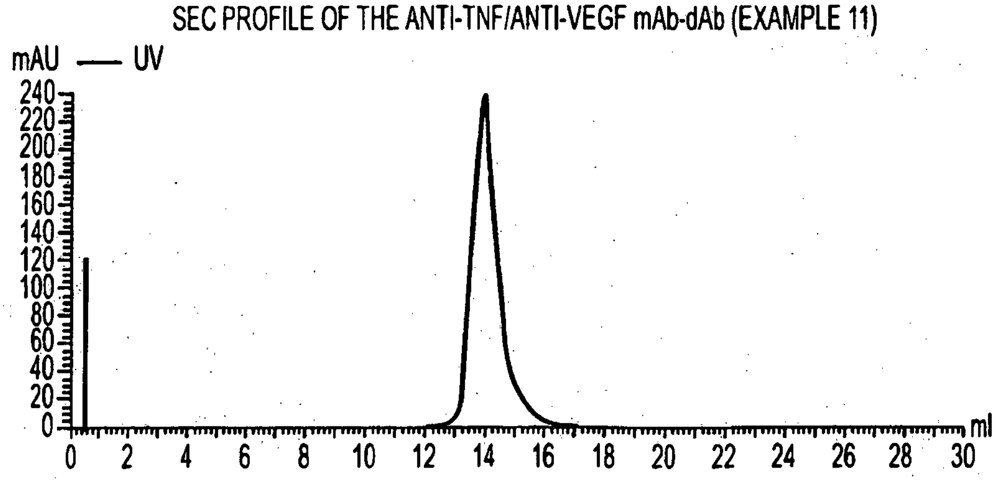


*FIG. 48*

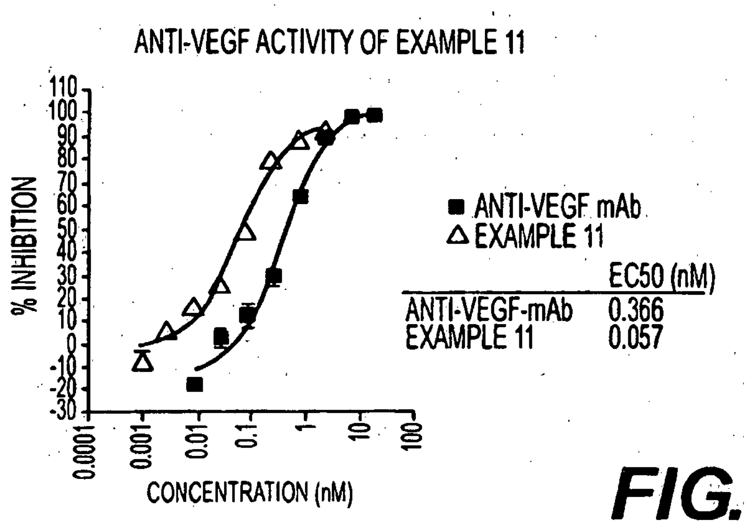


*FIG. 49*

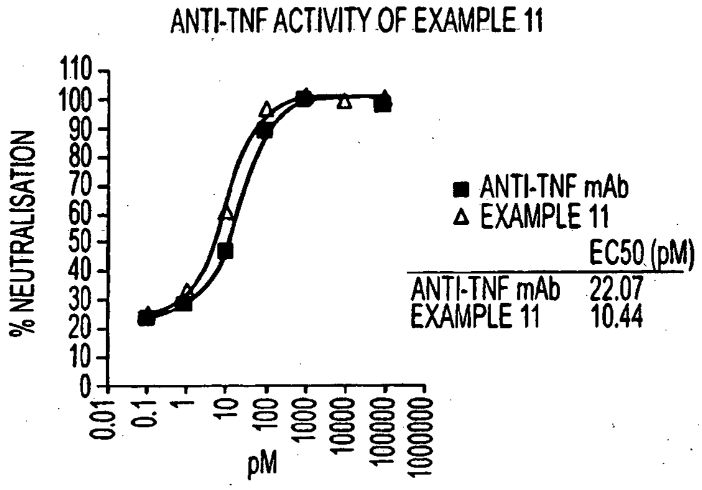


*FIG. 50*

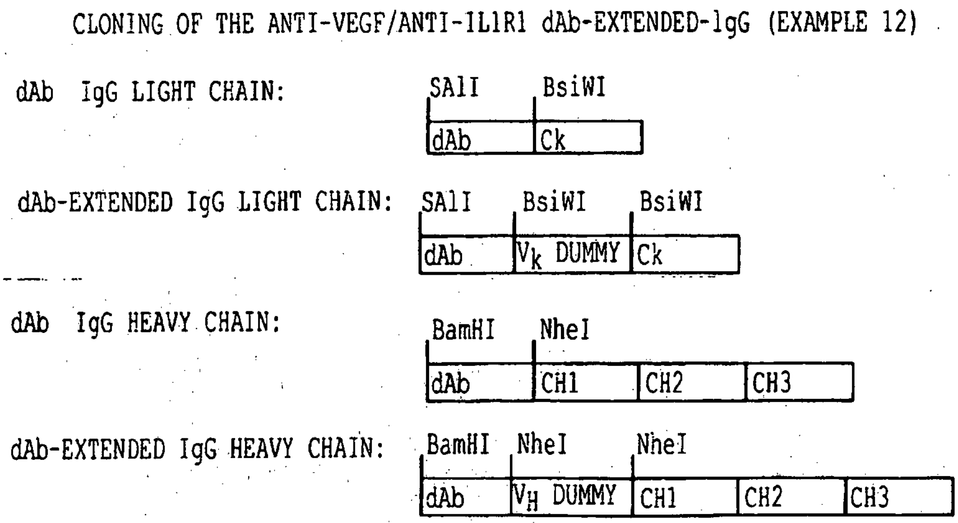


*FIG. 51*

SDS-PAGE ANALYSIS OF THE ANTI-TNF/ANTI-VEGF dAb-EXTENDED IgG A (EXAMPLE 12)

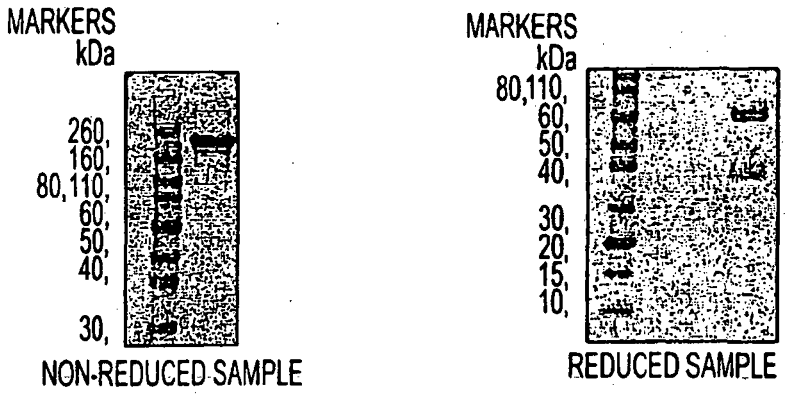


*FIG. 52*

SDS-PAGE ANALYSIS OF THE ANTI-TNF/ANTI-VEGF dAb-EXTENDED IgG B (EXAMPLE 12)

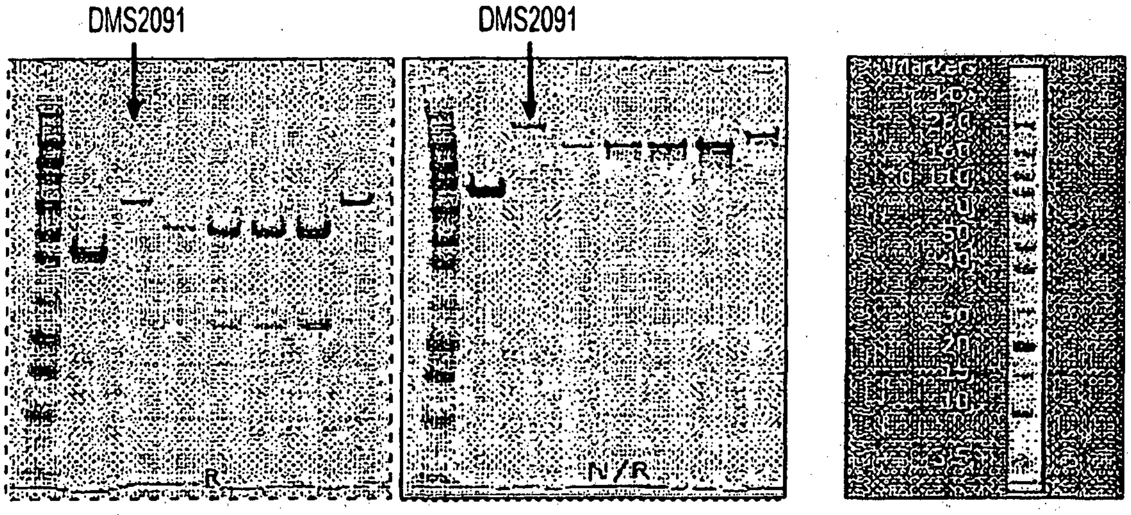


N/R=NON-REDUCED; R=REDUCED

*FIG. 53*

SEC PROFILE OF THE ANTI-TNF/ANTI-VEGF dAb-EXTENDED IgG A (EXAMPLE 12)

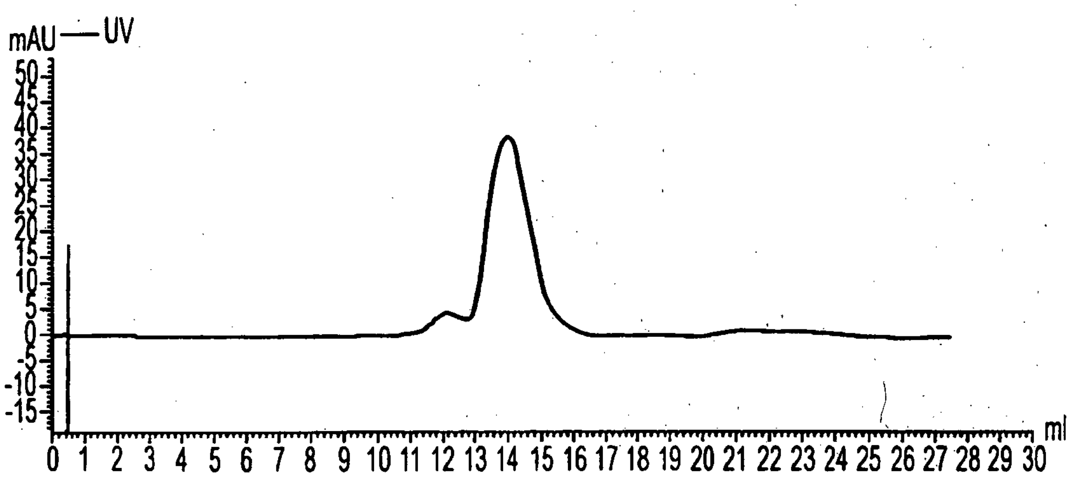


*FIG. 54*

SEC PROFILE OF THE ANTI-TNF/ANTI-VEGF dAb-EXTENDED IgG B (EXAMPLE 12)

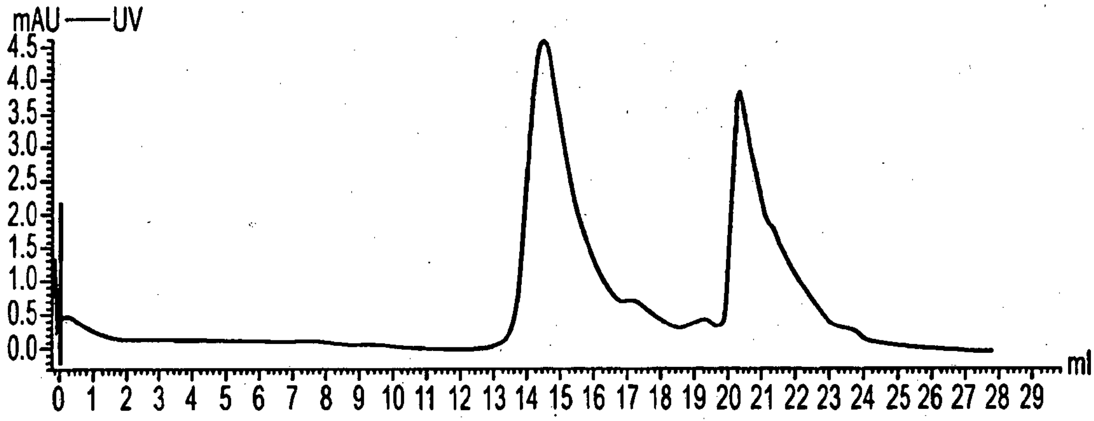


*FIG. 55*

ANTI-VEGF ACTIVITY OF EXAMPLE 12 (DMS2091)

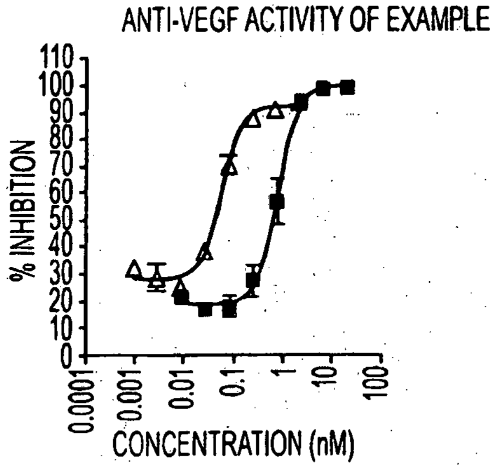


| | EC50 (nM) |
|---|---|
| ANTI-VEGF-mAb | 0.766 |
| EXAMPLE 12 | 0.055 |

*FIG. 56*

ANTI-VEGF ACTIVITY OF EXAMPLE 12 (DMS2090)

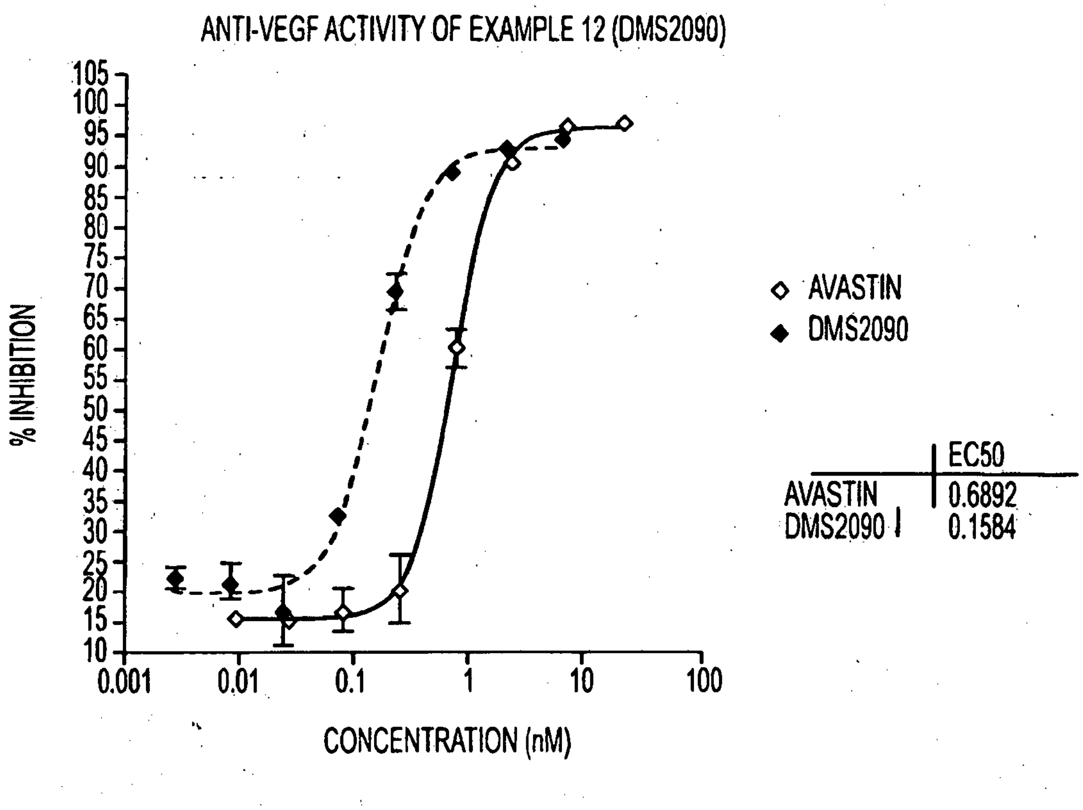


*FIG. 57*

ANTI-IL1R1 ACTIVITY OF EXAMPLE 12 (DMS2090)

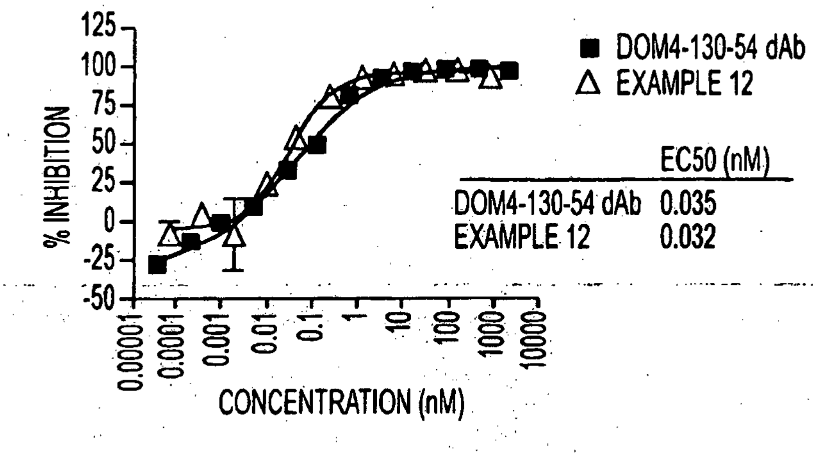


*FIG. 58*

ANTI-IL1R1 ACTIVITY OF EXAMPLE 12 (DMS2091)

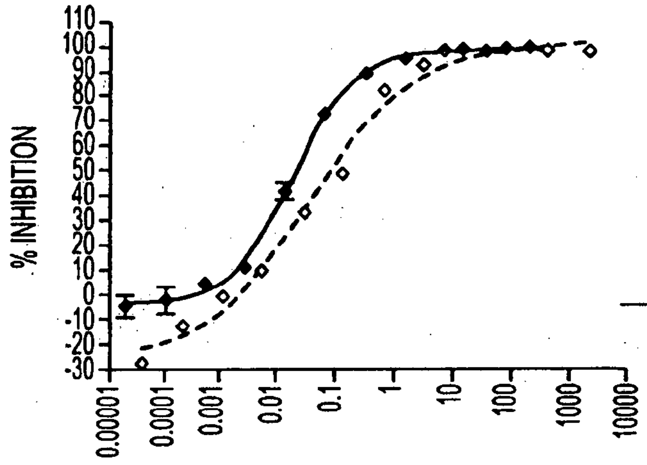


| | DOM4-130-54 | DMS2091 |
|---|---|---|
| EC50 | 0.03502 | 0.01747 |

*FIG. 59*

CLONING OF THE ANTI-TNF/ANTI-VEGF/ANTI-EGFR mAb-dAb (EXAMPLE 13)

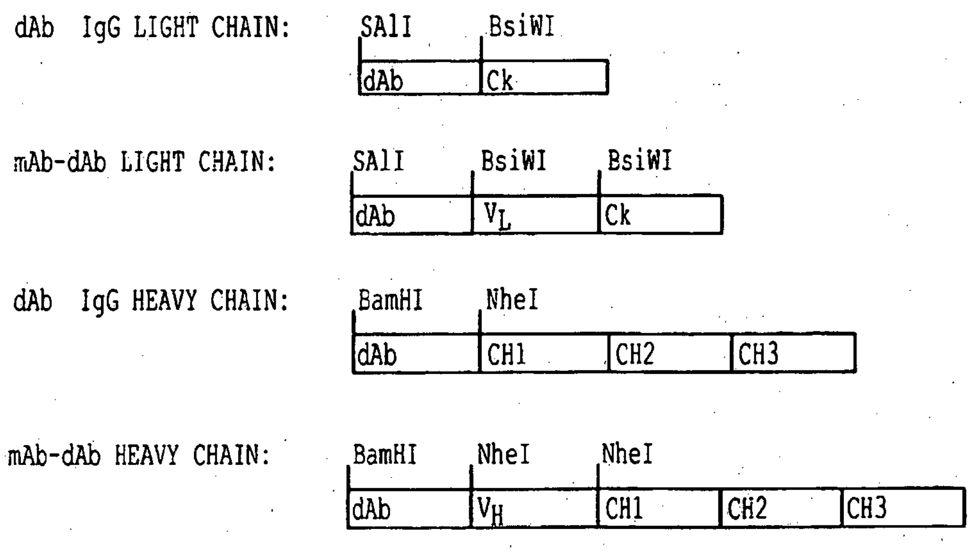


*FIG. 60*

SDS-PAGE ANALYSIS OF THE ANTI-TNF/ANTI-VEGF/ANTI-EGFR mAb-dAb (EXAMPLE 13)

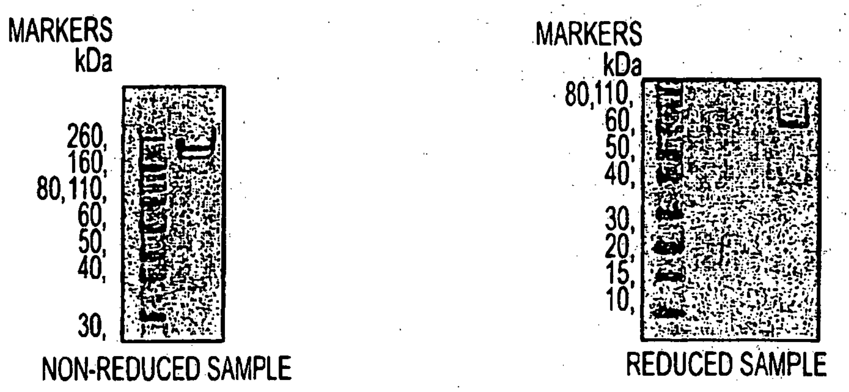


*FIG. 61*

ANTI-VEGF ACTIVITY OF EXAMPLE 13

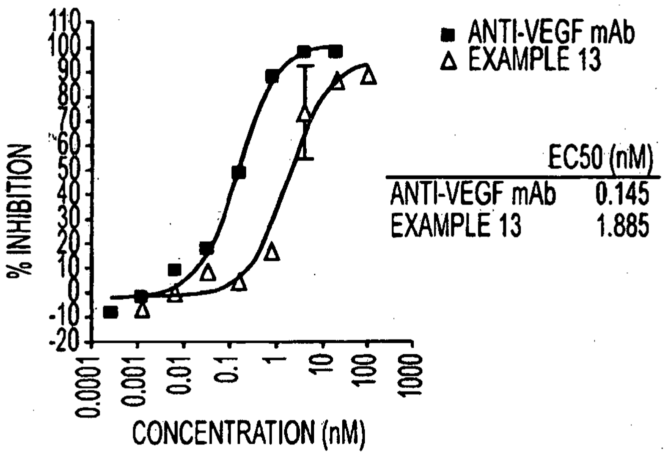


*FIG. 62*

ANTI-TNF ACTIVITY OF EXAMPLE 13

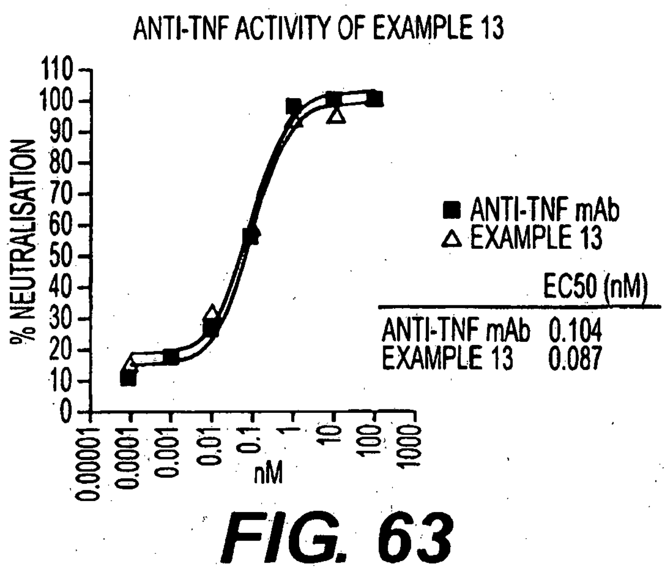


*FIG. 63*

ANTI-EGFR ACTIVITY OF EXAMPLE 13

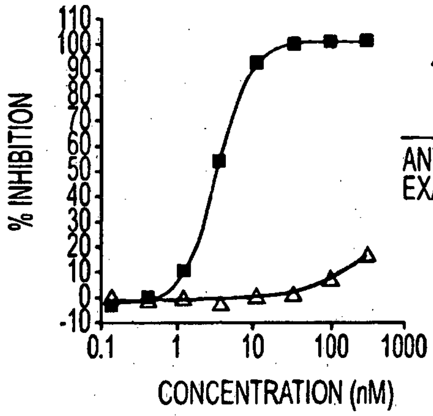


| | EC50 (nM) |
|---|---|
| ANTI-EGFR mAb | 3.355 |
| EXAMPLE 13 | - |

*FIG. 64*

SEC ANALYSIS OF PURIFIED BISPECIFIC ANTIBODIES, BPC1603 (A), BPC1604 (B), BPC1605 (C), BPC1606 (D)

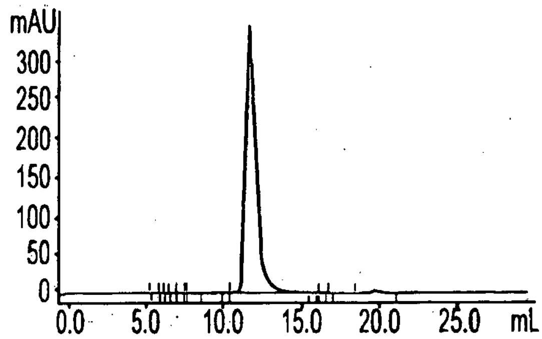


*FIG. 65A*

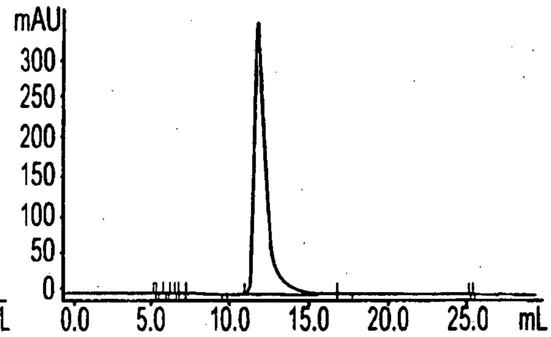


*FIG. 65B*

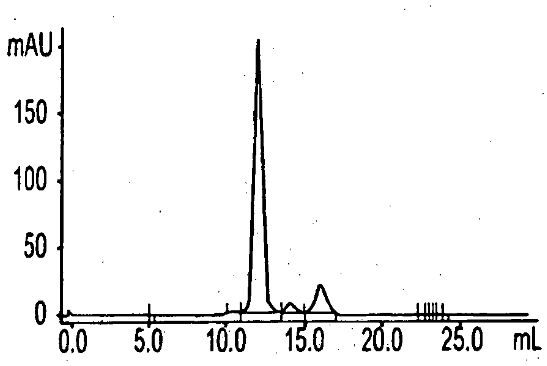


*FIG. 65C*

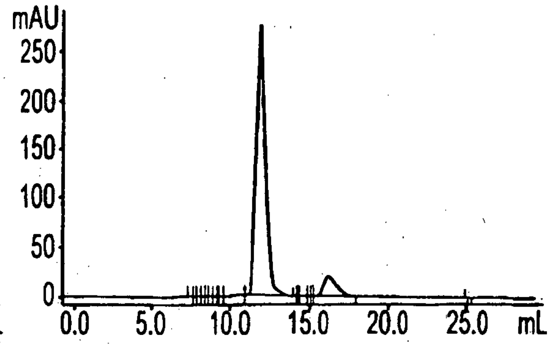


*FIG. 65D*

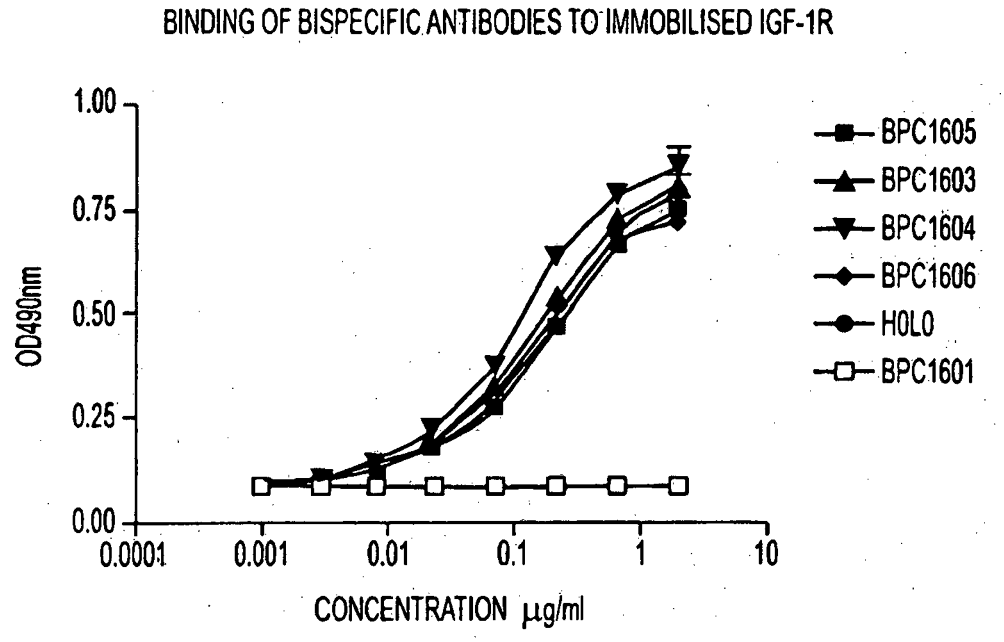
BINDING OF BISPECIFIC ANTIBODIES TO IMMOBILISED IGF-1R
1.00
0.75
0.50
0.25
0.00
OD490nm
0.0001
0.001
0.01
0.1
1
10
CONCENTRATION µg/ml
BPC1605
BPC1603
BPC1604
BPC1606
H0L0
BPC1601

*FIG. 66*

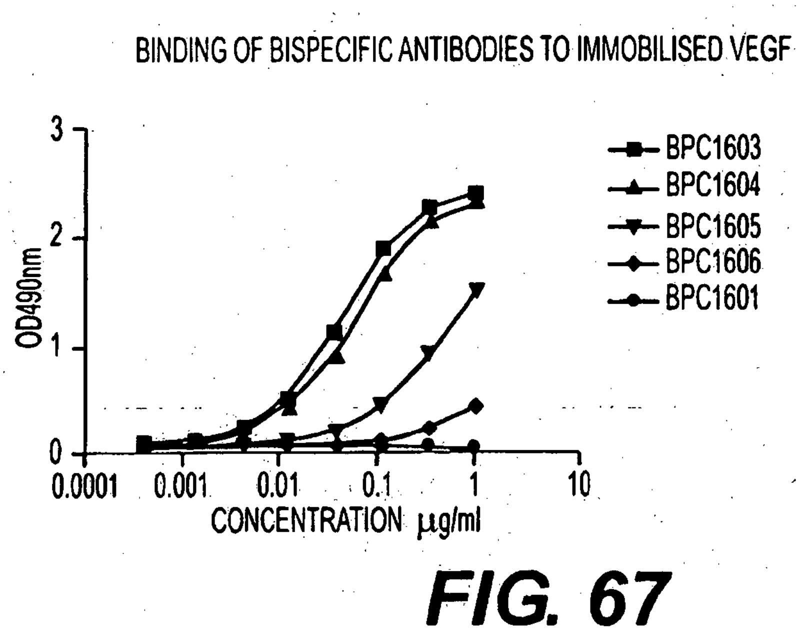
BINDING OF BISPECIFIC ANTIBODIES TO IMMOBILISED VEGF
3
2
1
0
OD490nm
0.0001
0.001
0.01
0.1
1
10
CONCENTRATION µg/ml
BPC1603
BPC1604
BPC1605
BPC1606
BPC1601

*FIG. 67*

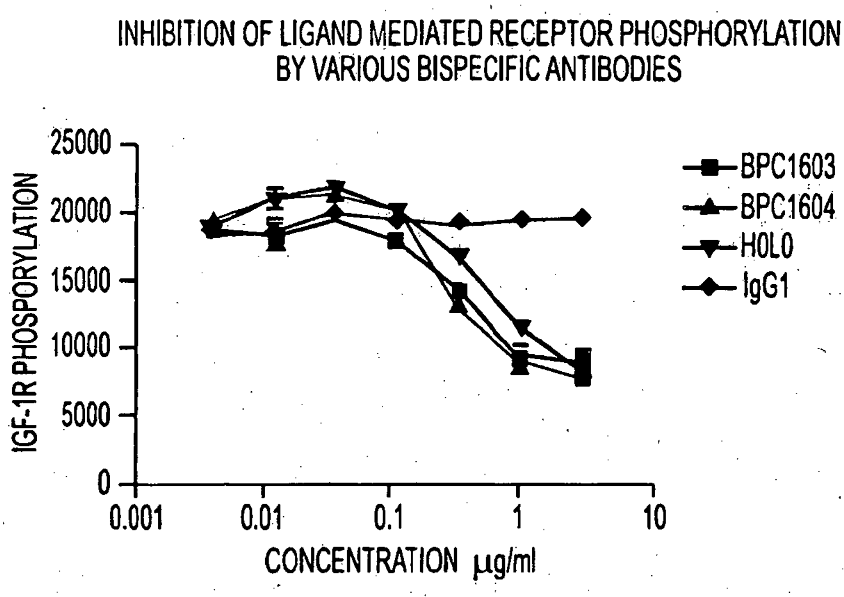
INHIBITION OF LIGAND MEDIATED RECEPTOR PHOSPHORYLATION
BY VARIOUS BISPECIFIC ANTIBODIES
IGF-1R PHOSPORYLATION
25000
20000
15000
10000
5000
0
0.001
0.01
0.1
1
10
CONCENTRATION µg/ml
BPC1603
BPC1604
H0L0
IgG1

*FIG. 68*

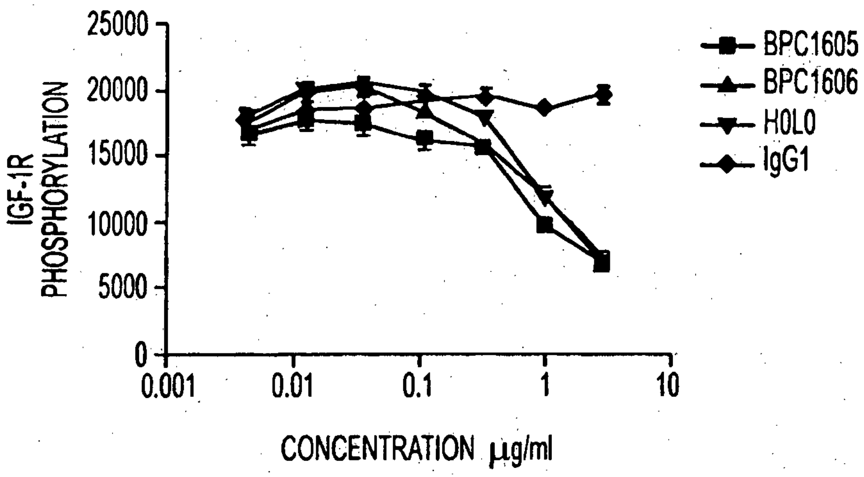
IGF-1R PHOSPHORYLATION
25000
20000
15000
10000
5000
0
0.001
0.01
0.1
1
10
CONCENTRATION µg/ml
BPC1605
BPC1606
H0L0
IgG1

*FIG. 69*

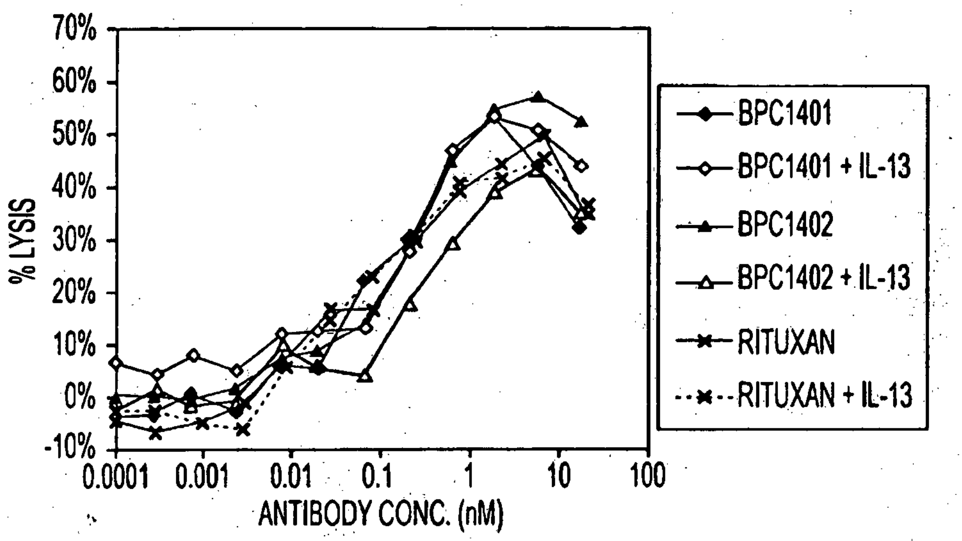
70%
60%
50%
40%
30%
20%
10%
0%
-10%
% LYSIS
0.0001
0.001
0.01
0.1
1
10
100
ANTIBODY CONC. (nM)
BPC1401
BPC1401 + IL-13
BPC1402
BPC1402 + IL-13
RITUXAN
RITUXAN + IL-13

*FIG. 70*

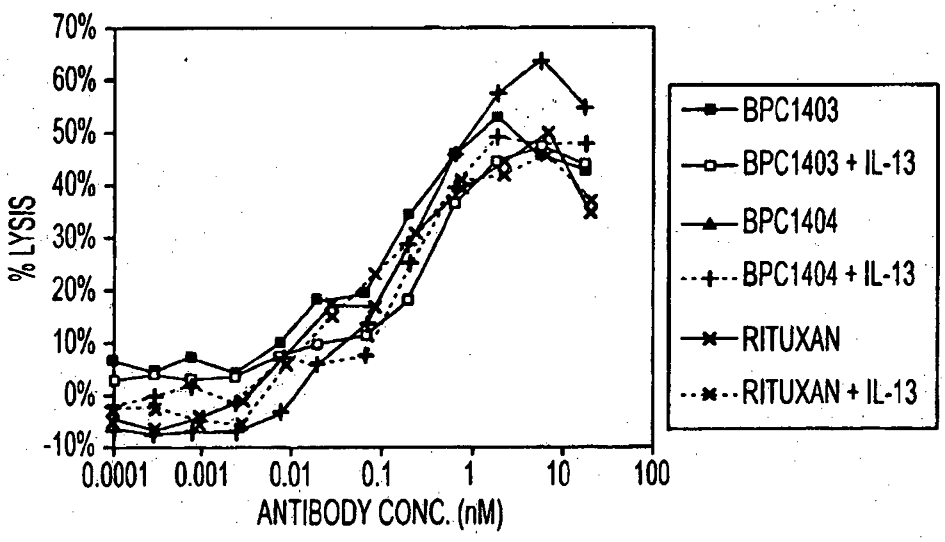
70%
60%
50%
40%
30%
20%
10%
0%
-10%
% LYSIS
0.0001
0.001
0.01
0.1
1
10
100
ANTIBODY CONC. (nM)
BPC1403
BPC1403 + IL-13
BPC1404
BPC1404 + IL-13
RITUXAN
RITUXAN + IL-13

*FIG. 71*

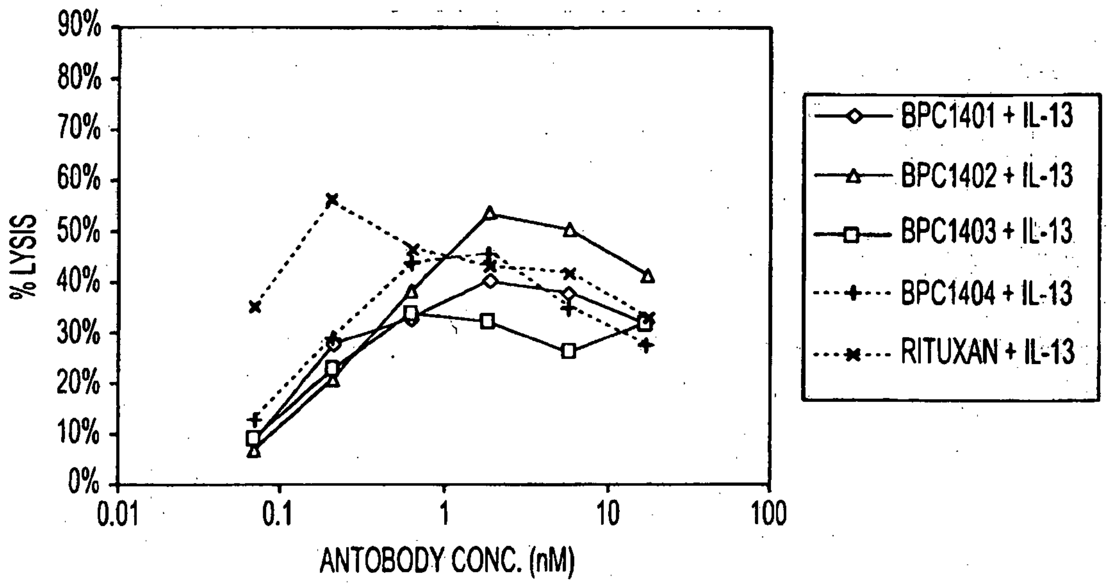
90%
80%
70%
60%
50%
40%
30%
20%
10%
0%
% LYSIS
0.01
0.1
1
10
100
ANTOBODY CONC. (nM)
BPC1401 + IL-13
BPC1402 + IL-13
BPC1403 + IL-13
BPC1404 + IL-13
RITUXAN + IL-13

*FIG. 72*

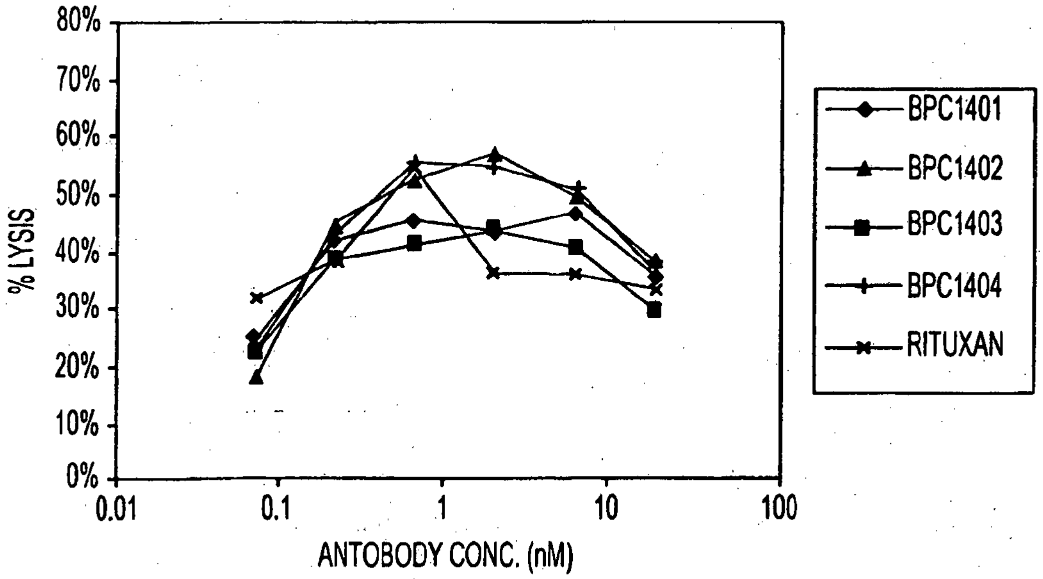
80%
70%
60%
50%
40%
30%
20%
10%
0%
% LYSIS
0.01
0.1
1
10
100
ANTOBODY CONC. (nM)
BPC1401
BPC1402
BPC1403
BPC1404
RITUXAN

*FIG. 73*

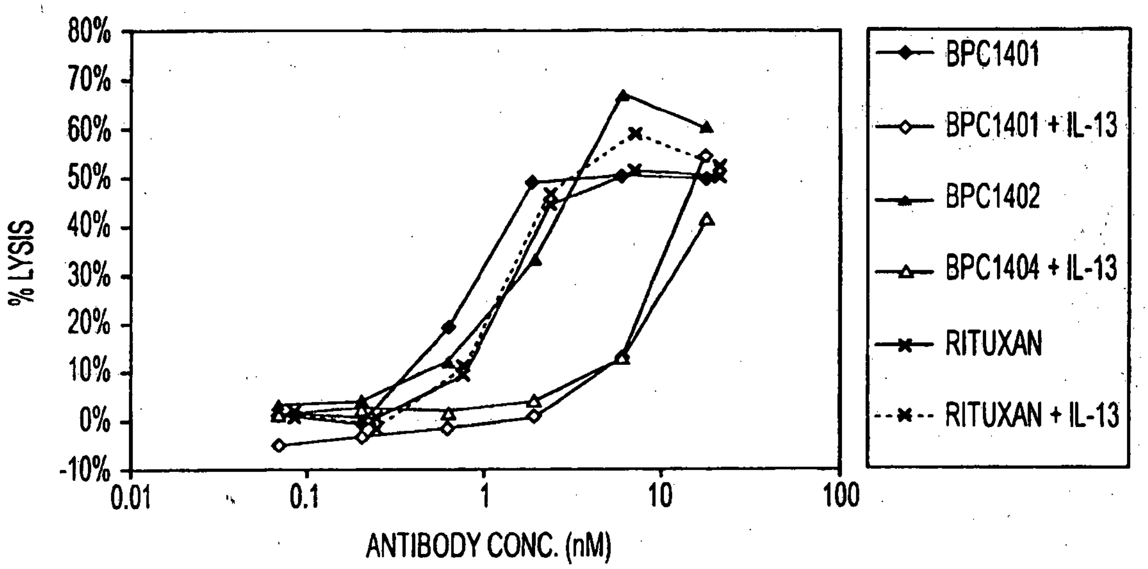
80%
70%
60%
50%
40%
30%
20%
10%
0%
-10%
% LYSIS
0.01
0.1
1
10
100
ANTIBODY CONC. (nM)
BPC1401
BPC1401 + IL-13
BPC1402
BPC1404 + IL-13
RITUXAN
RITUXAN + IL-13

*FIG. 74*

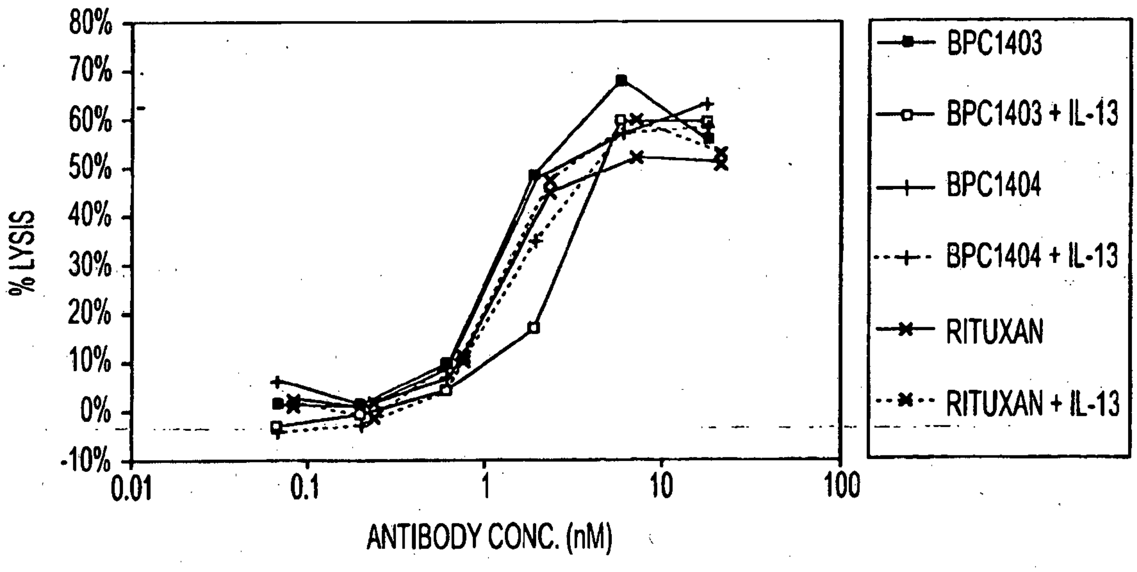
80%
70%
60%
50%
40%
30%
20%
10%
0%
-10%
% LYSIS
0.01
0.1
1
10
100
ANTIBODY CONC. (nM)
BPC1403
BPC1403 + IL-13
BPC1404
BPC1404 + IL-13
RITUXAN
RITUXAN + IL-13

*FIG. 75*

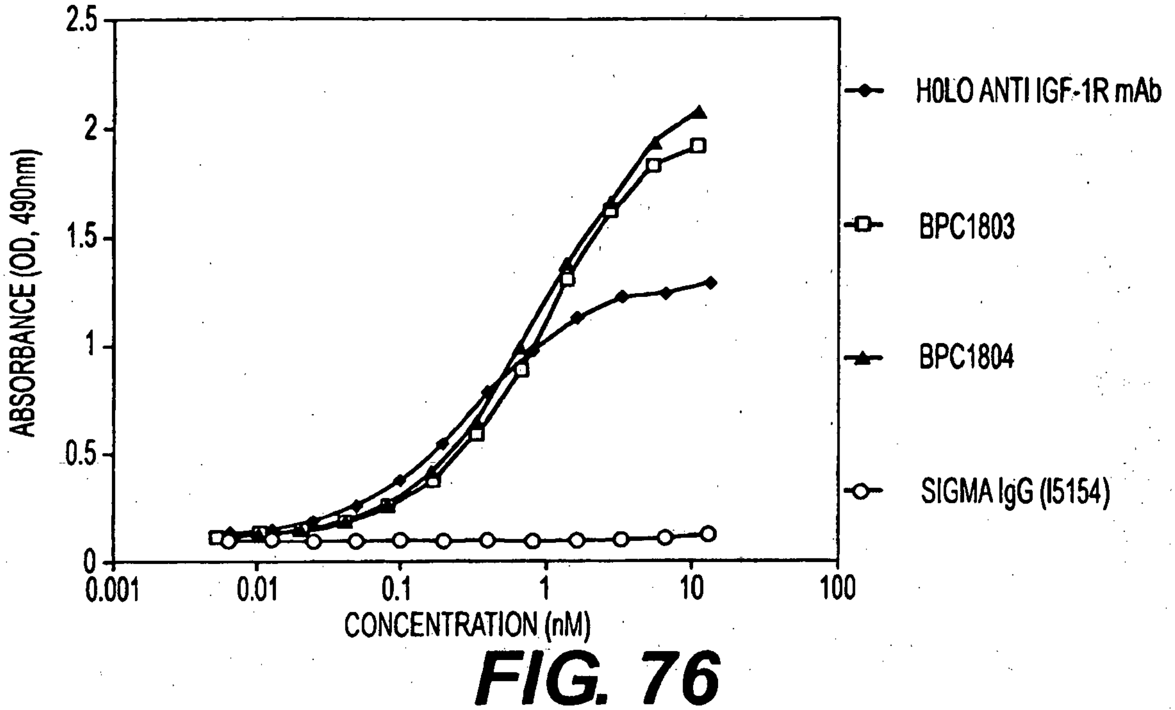
2.5
2
1.5
1
0.5
0
0.001
0.01
0.1
1
10
100
ABSORBANCE (OD, 490nm)
CONCENTRATION (nM)
H0L0 ANTI IGF-1R mAb
BPC1803
BPC1804
SIGMA IgG (I5154)

FIG. 76

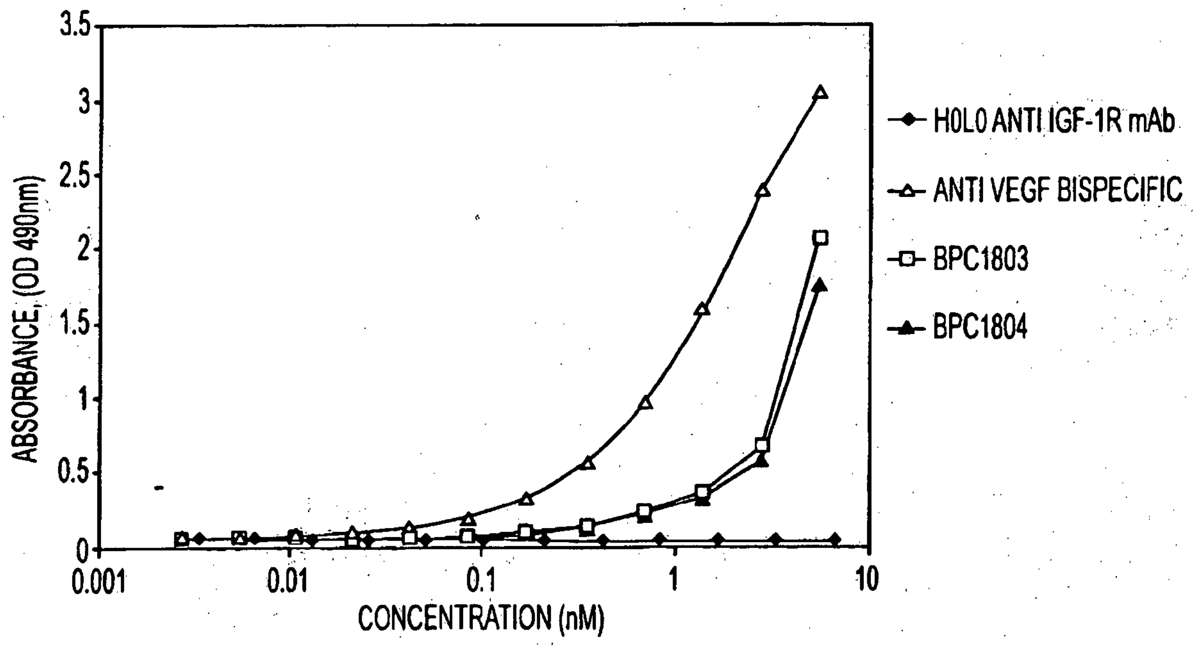
3.5
3
2.5
2
1.5
1
0.5
0
0.001
0.01
0.1
1
10
ABSORBANCE, (OD 490nm)
CONCENTRATION (nM)
H0L0 ANTI IGF-1R mAb
ANTI VEGF BISPECIFIC
BPC1803
BPC1804

FIG. 77

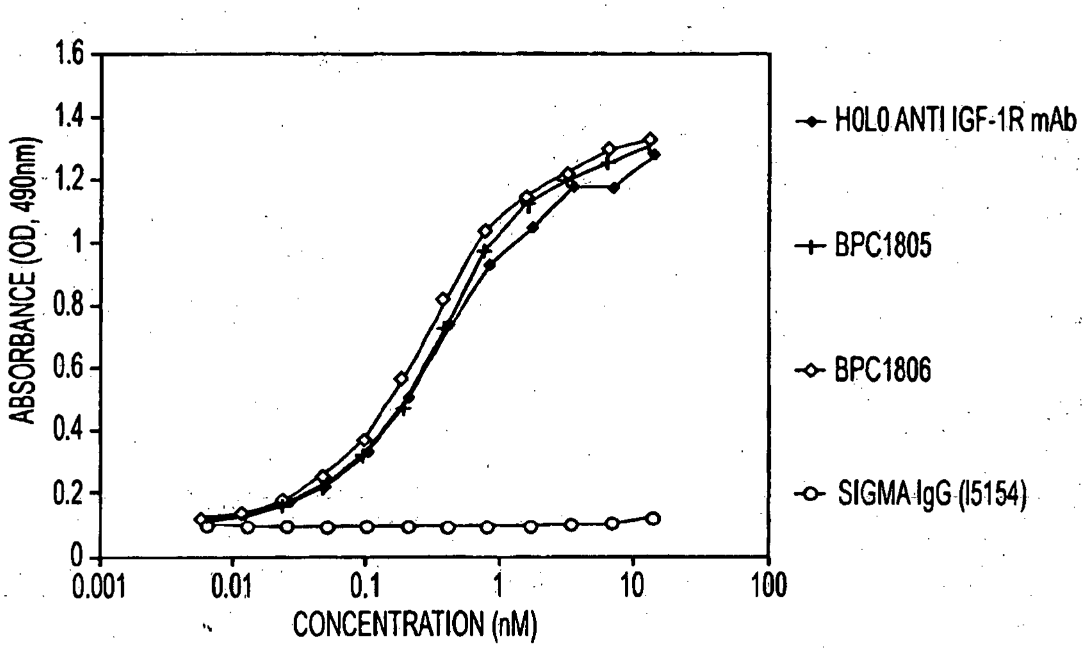
1.6
1.4
1.2
1
0.8
0.6
0.4
0.2
0
ABSORBANCE (OD, 490nm)
0.001
0.01
0.1
1
10
100
CONCENTRATION (nM)
H0L0 ANTI IGF-1R mAb
BPC1805
BPC1806
SIGMA IgG (I5154)

*FIG. 78*

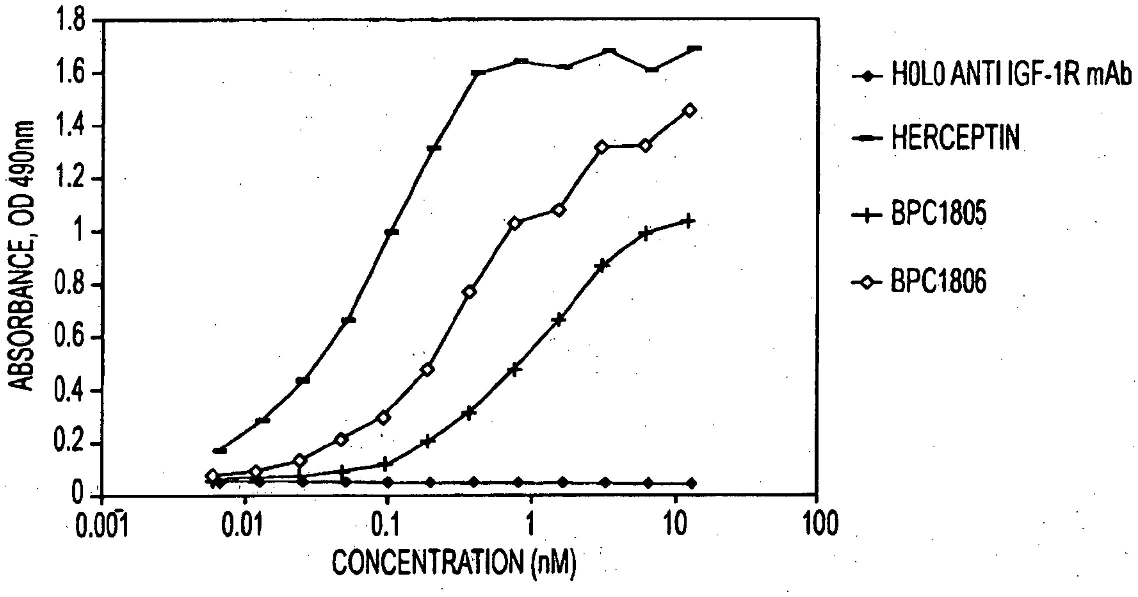
1.8
1.6
1.4
1.2
1
0.8
0.6
0.4
0.2
0
ABSORBANCE, OD 490nm
0.001
0.01
0.1
1
10
100
CONCENTRATION (nM)
H0L0 ANTI IGF-1R mAb
HERCEPTIN
BPC1805
BPC1806

*FIG. 79*

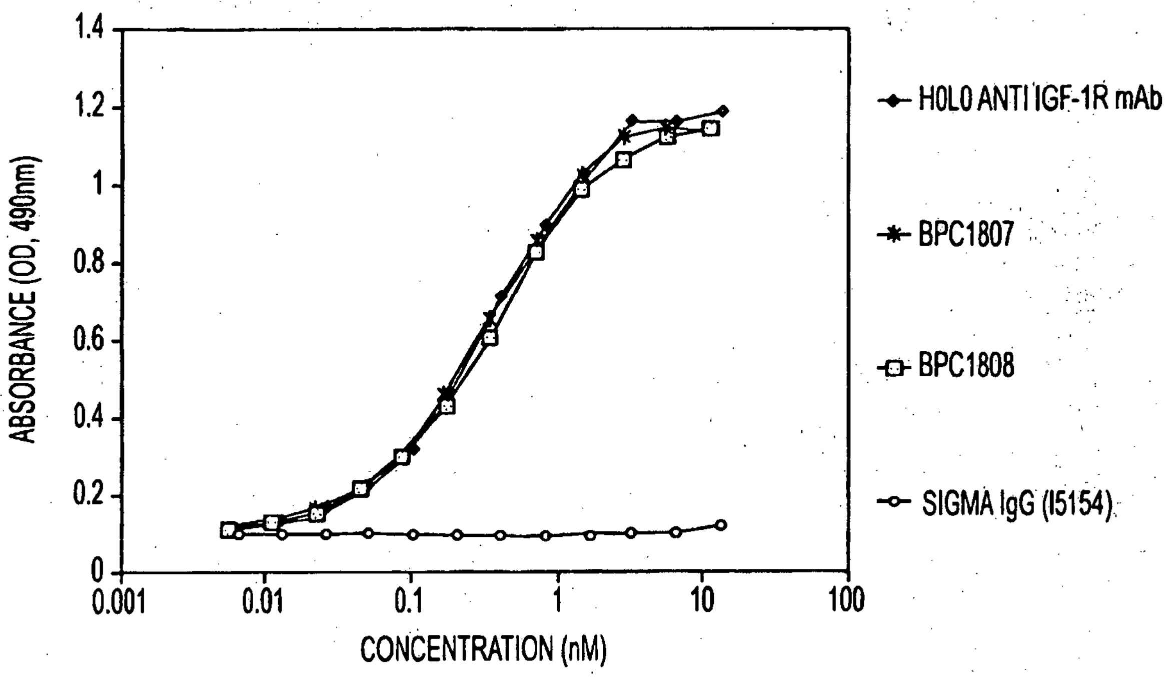
1.4
1.2
1
0.8
0.6
0.4
0.2
0
ABSORBANCE (OD, 490nm)
0.001
0.01
0.1
1
10
100
CONCENTRATION (nM)
H0L0 ANTI IGF-1R mAb
BPC1807
BPC1808
SIGMA IgG (I5154)

FIG. 80

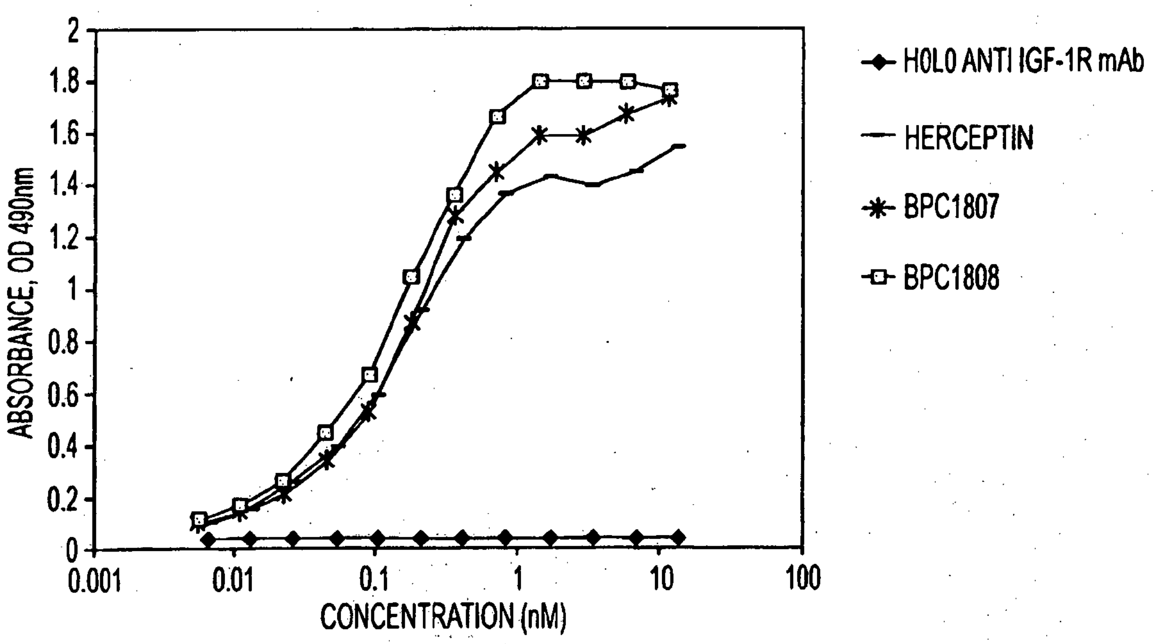
2
1.8
1.6
1.4
1.2
1
0.8
0.6
0.4
0.2
0
ABSORBANCE, OD 490nm
0.001
0.01
0.1
1
10
100
CONCENTRATION (nM)
H0L0 ANTI IGF-1R mAb
HERCEPTIN
BPC1807
BPC1808

FIG. 81

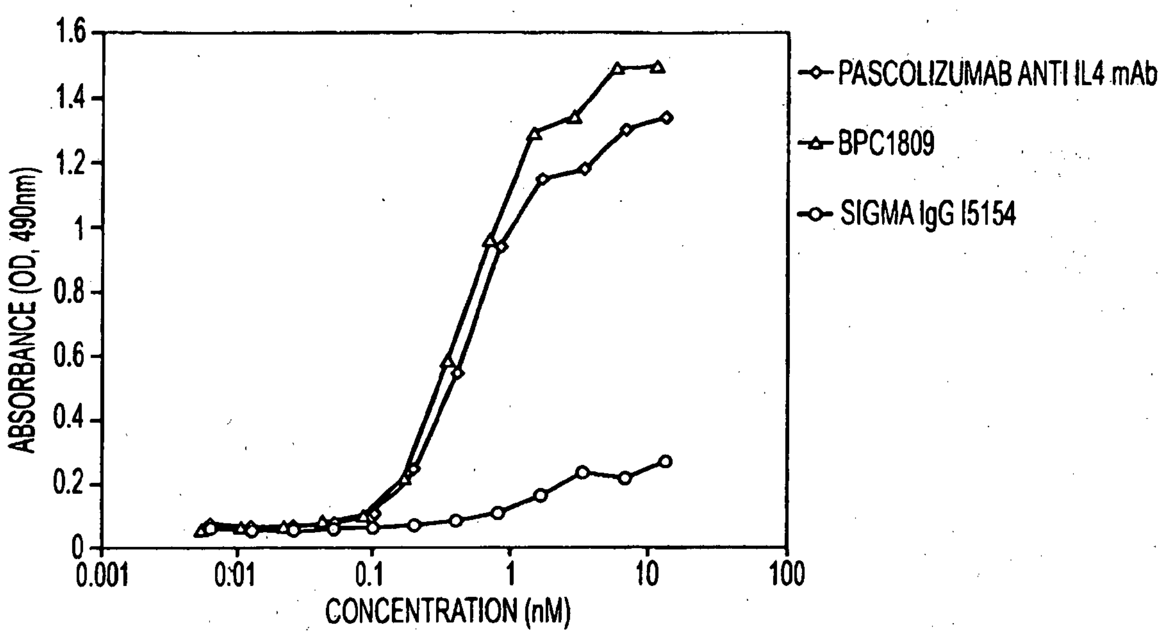
ABSORBANCE (OD, 490nm)
1.6
1.4
1.2
1
0.8
0.6
0.4
0.2
0
0.001
0.01
0.1
1
10
100
CONCENTRATION (nM)
PASCOLIZUMAB ANTI IL4 mAb
BPC1809
SIGMA IgG I5154

FIG. 82

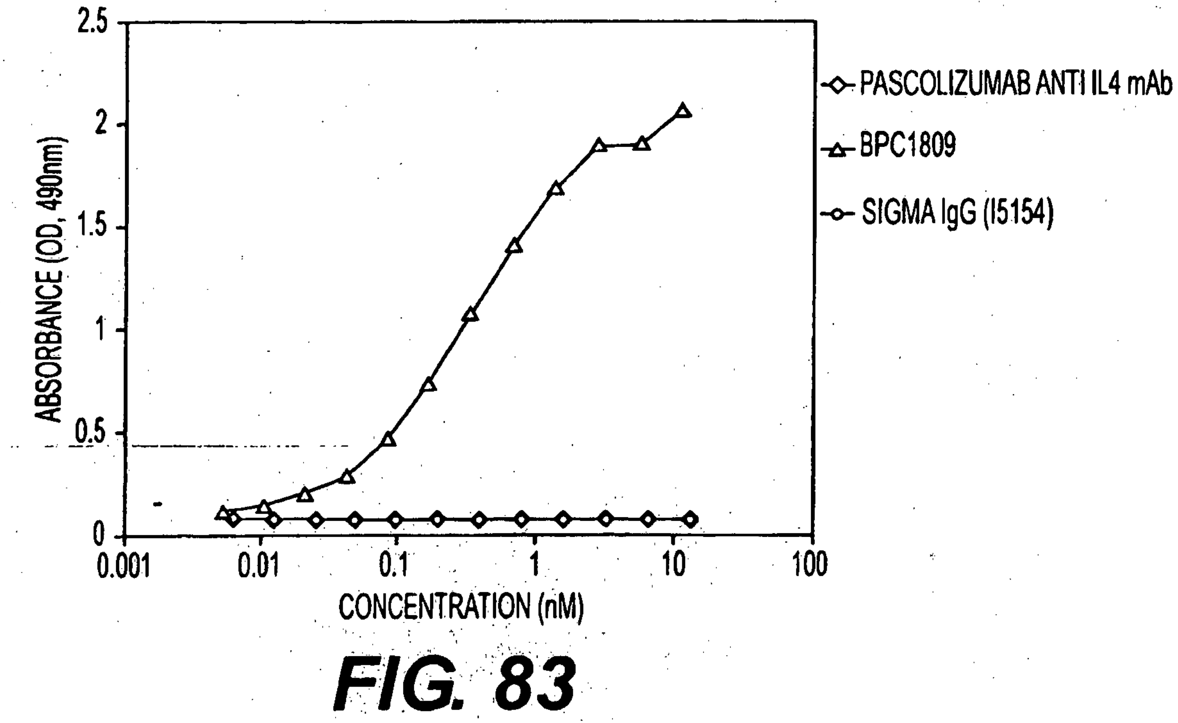
ABSORBANCE (OD, 490nm)
2.5
2
1.5
1
0.5
0
0.001
0.01
0.1
1
10
100
CONCENTRATION (nM)
PASCOLIZUMAB ANTI IL4 mAb
BPC1809
SIGMA IgG (I5154)

FIG. 83

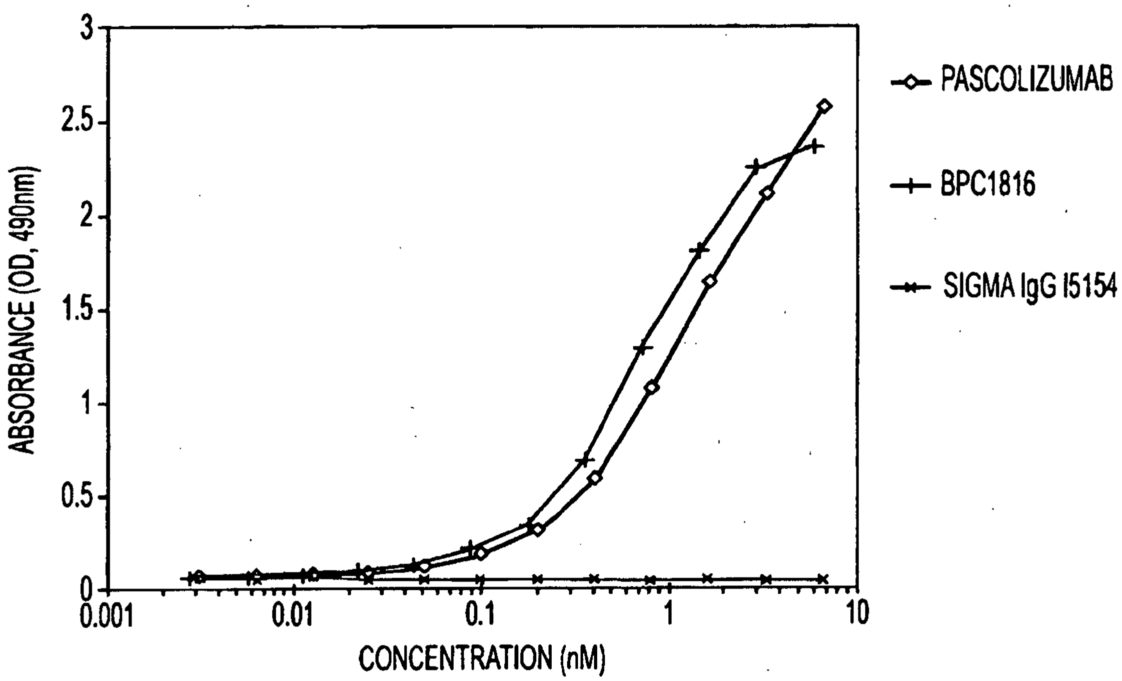
3
2.5
2
1.5
1
0.5
0
0.001
0.01
0.1
1
10
ABSORBANCE (OD, 490nm)
CONCENTRATION (nM)
PASCOLIZUMAB
BPC1816
SIGMA IgG I5154

FIG. 84

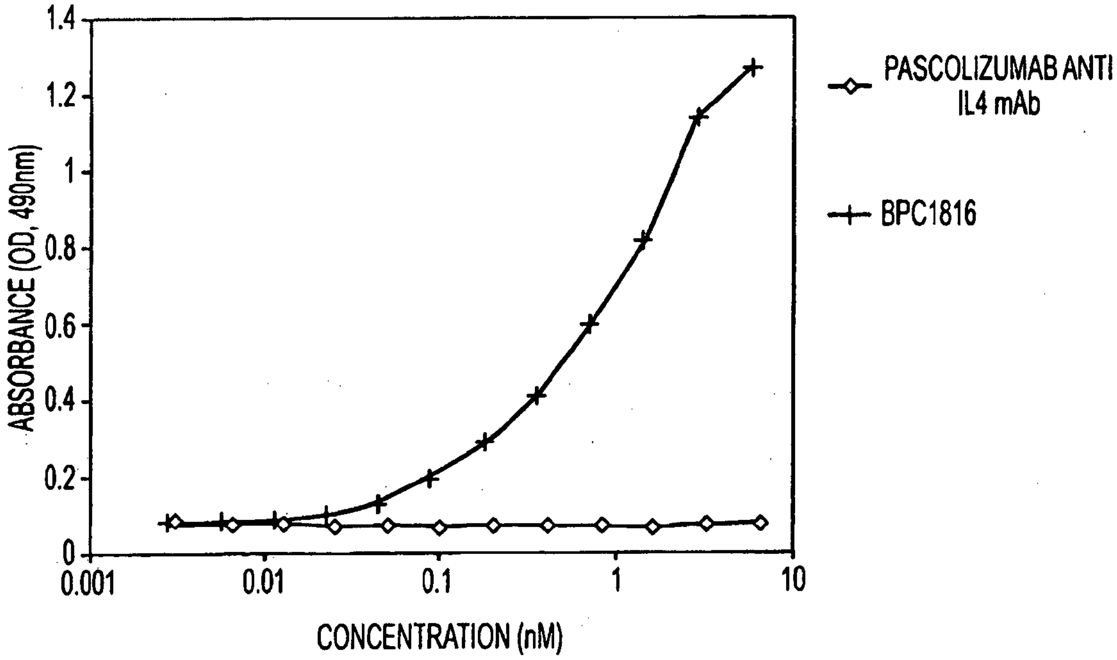
1.4
1.2
1
0.8
0.6
0.4
0.2
0
0.001
0.01
0.1
1
10
ABSORBANCE (OD, 490nm)
CONCENTRATION (nM)
PASCOLIZUMAB ANTI IL4 mAb
BPC1816

FIG. 85

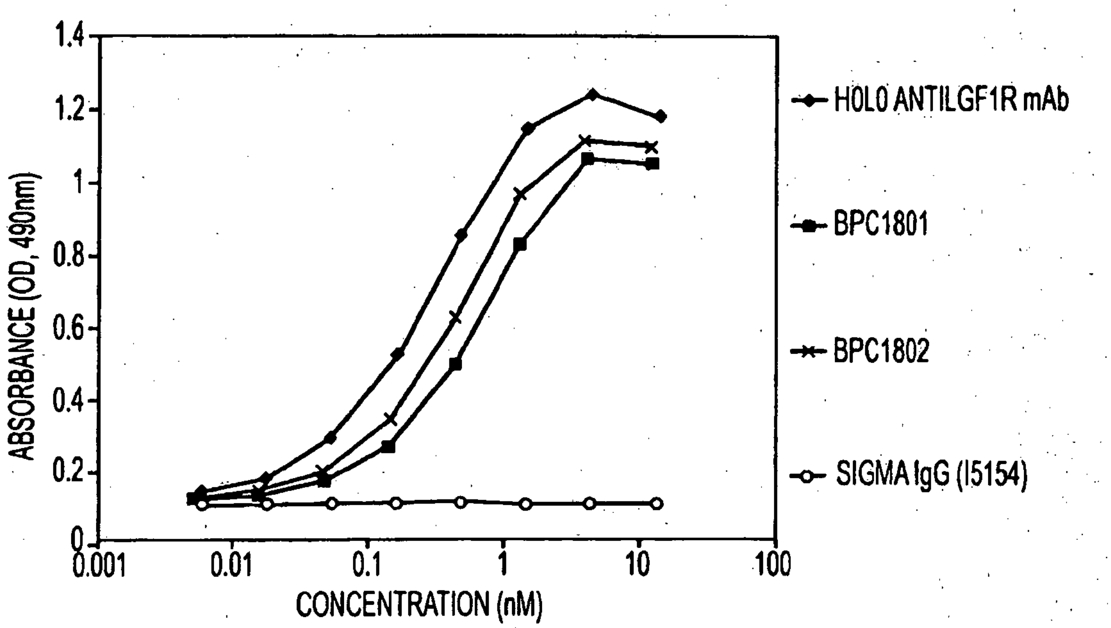
1.4
1.2
1
0.8
0.6
0.4
0.2
0
ABSORBANCE (OD, 490nm)
0.001
0.01
0.1
1
10
100
CONCENTRATION (nM)
HOLO ANTILGF1R mAb
BPC1801
BPC1802
SIGMA IgG (I5154)

FIG. 86

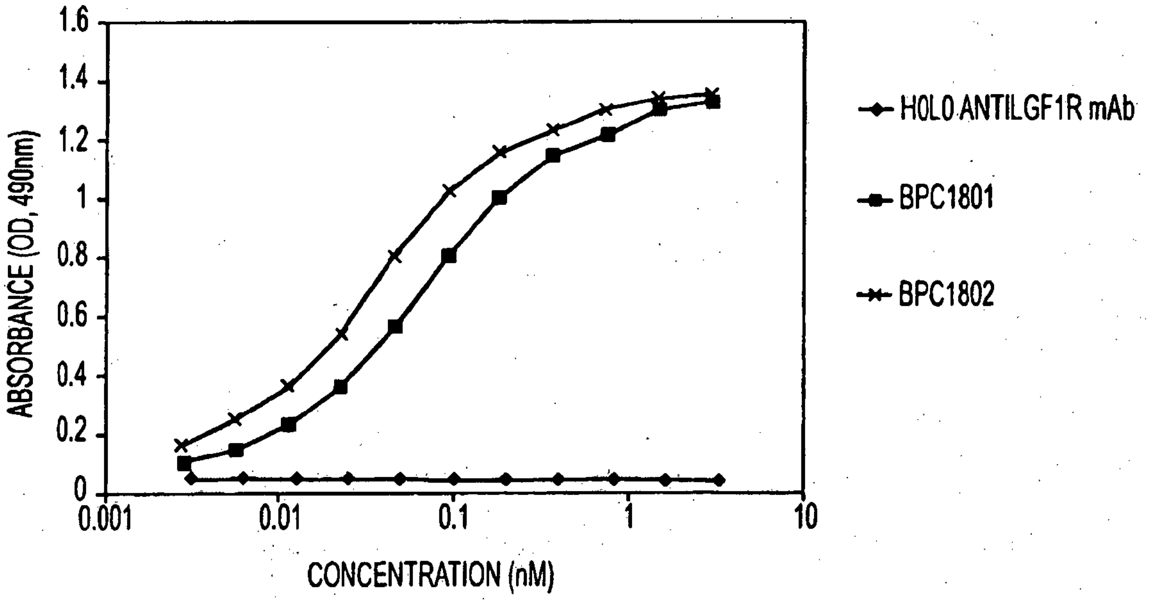
1.6
1.4
1.2
1
0.8
0.6
0.4
0.2
0
ABSORBANCE (OD, 490nm)
0.001
0.01
0.1
1
10
CONCENTRATION (nM)
HOLO ANTILGF1R mAb
BPC1801
BPC1802

FIG. 87

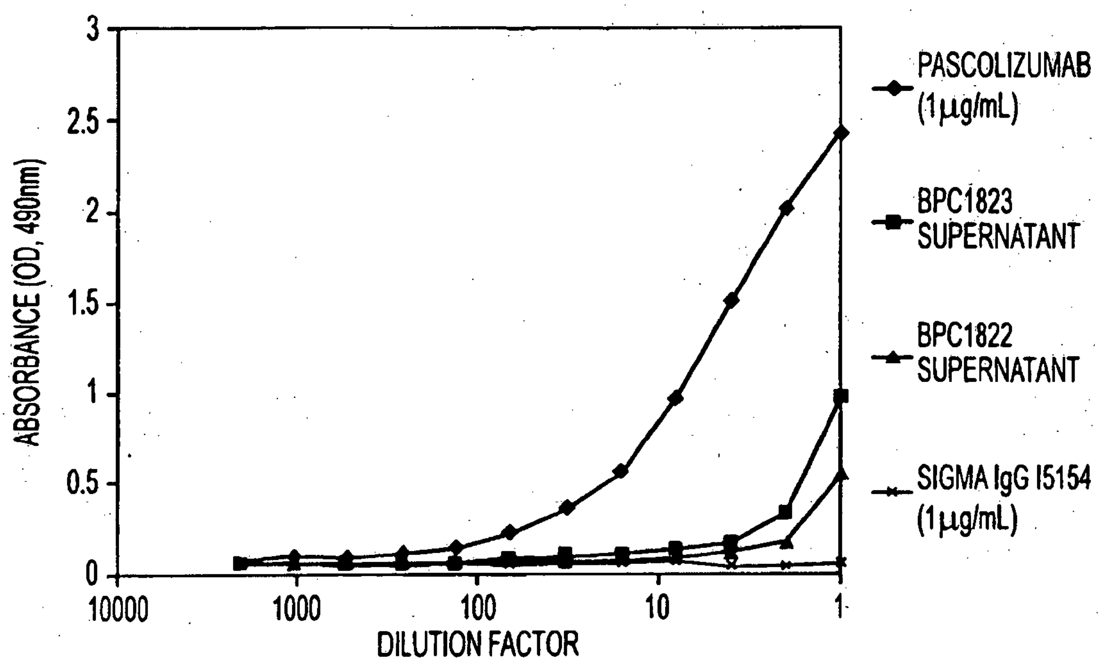


*FIG. 88a*

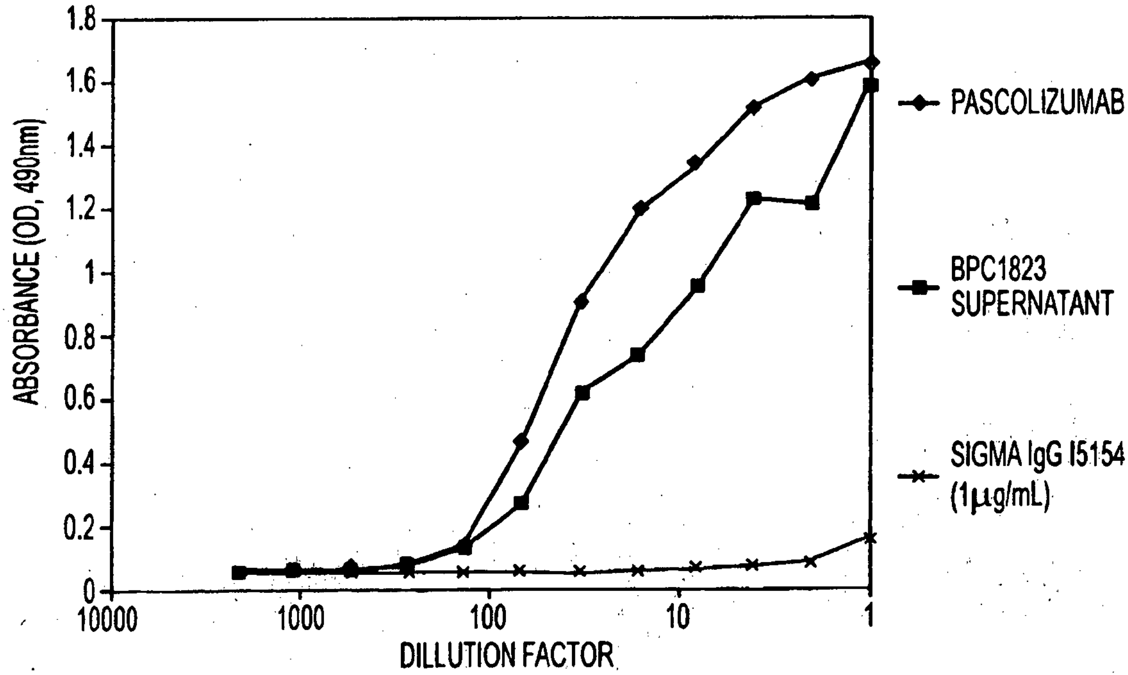


*FIG. 88b*

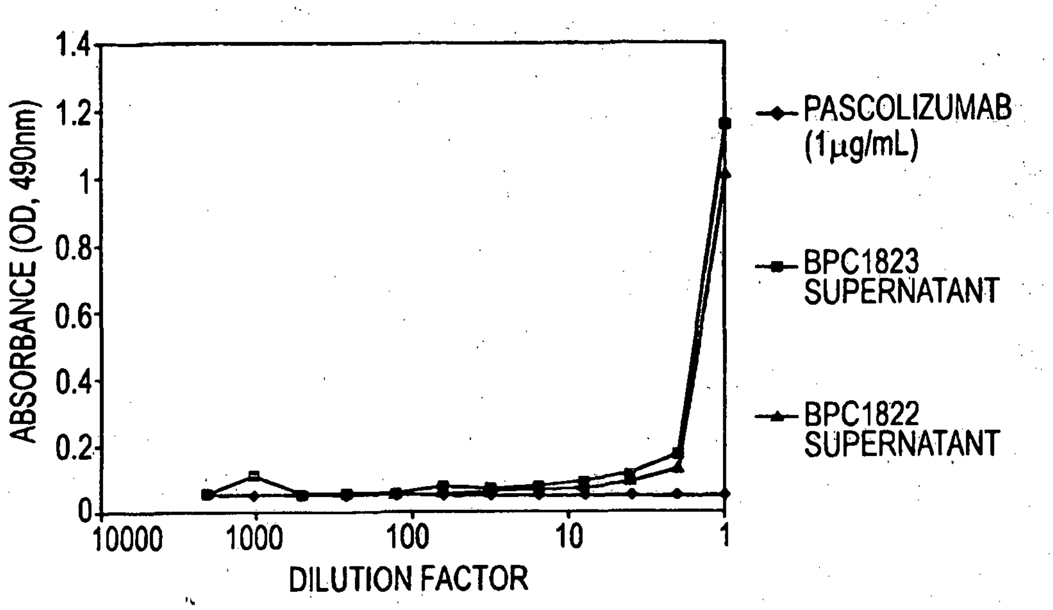


FIG. 89a

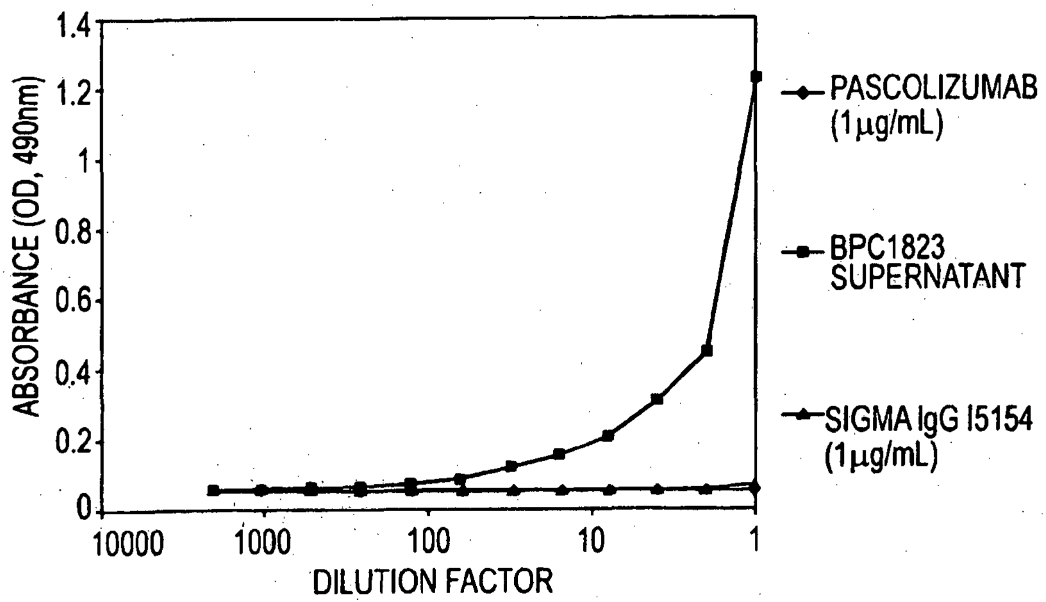


FIG. 89b

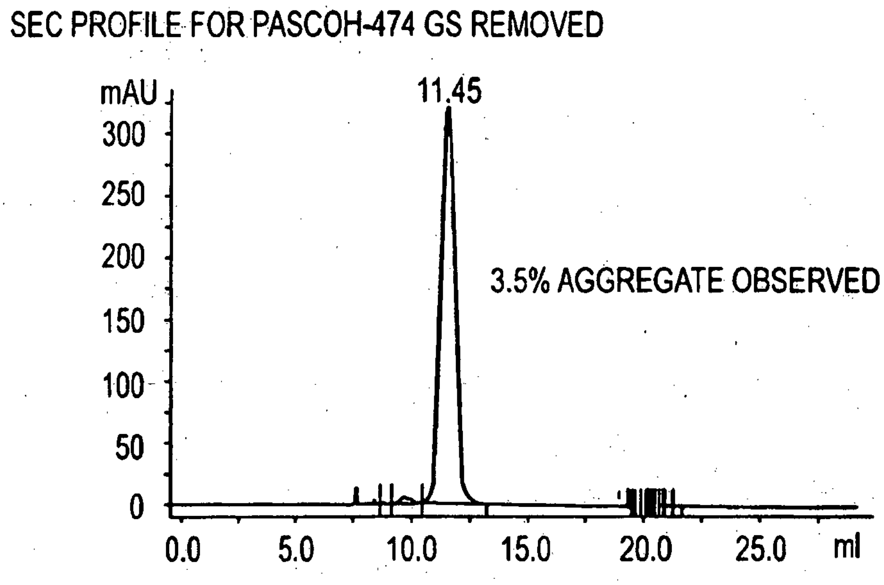
SEC PROFILE FOR PASCOH-474 GS REMOVED
mAU
300
250
200
150
100
50
0
11.45
3.5% AGGREGATE OBSERVED
0.0
5.0
10.0
15.0
20.0
25.0
ml

*FIG. 90*

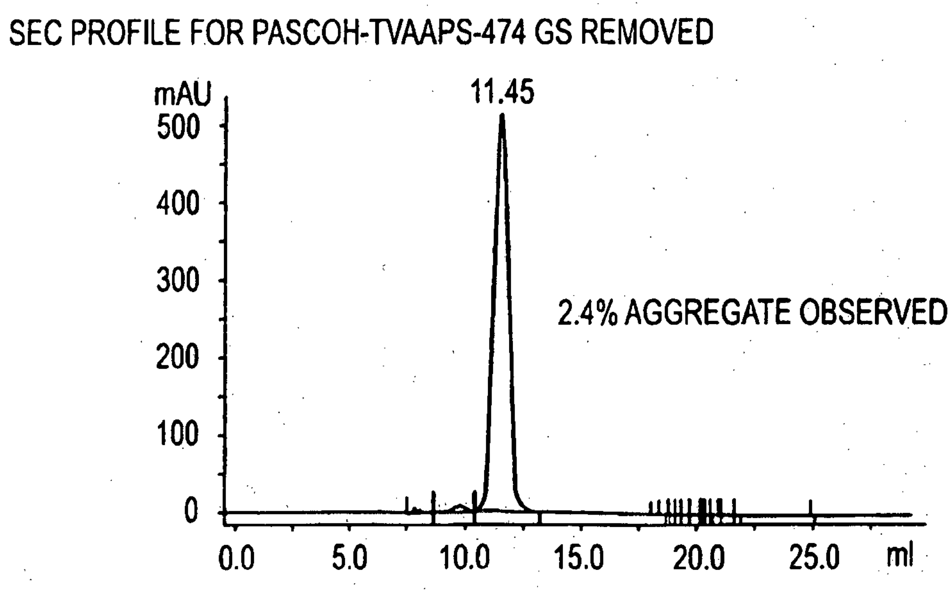
SEC PROFILE FOR PASCOH-TVAAPS-474 GS REMOVED
mAU
500
400
300
200
100
0
11.45
2.4% AGGREGATE OBSERVED
0.0
5.0
10.0
15.0
20.0
25.0
ml

*FIG. 91*

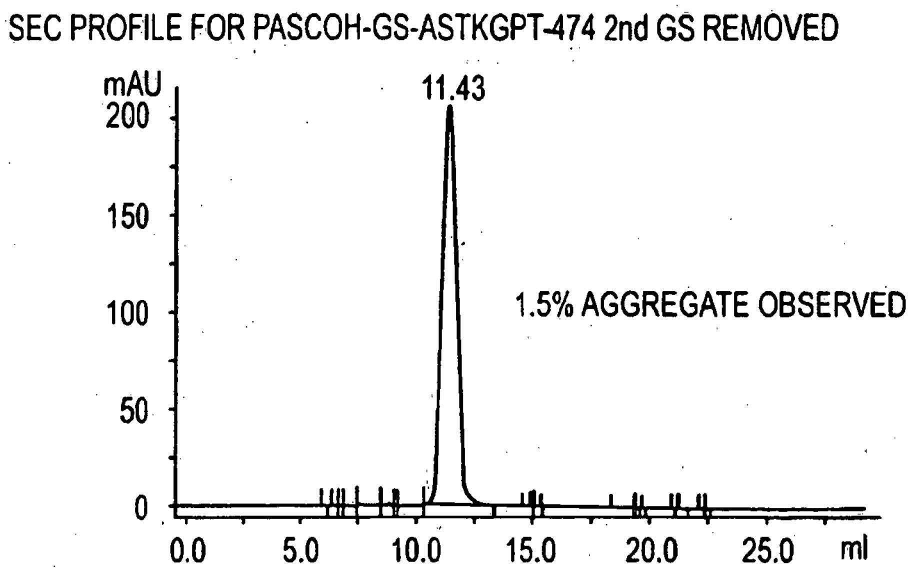
SEC PROFILE FOR PASCOH-GS-ASTKGPT-474 2nd GS REMOVED
mAU
200
150
100
50
0
11.43
1.5% AGGREGATE OBSERVED
0.0
5.0
10.0
15.0
20.0
25.0
ml

FIG. 92

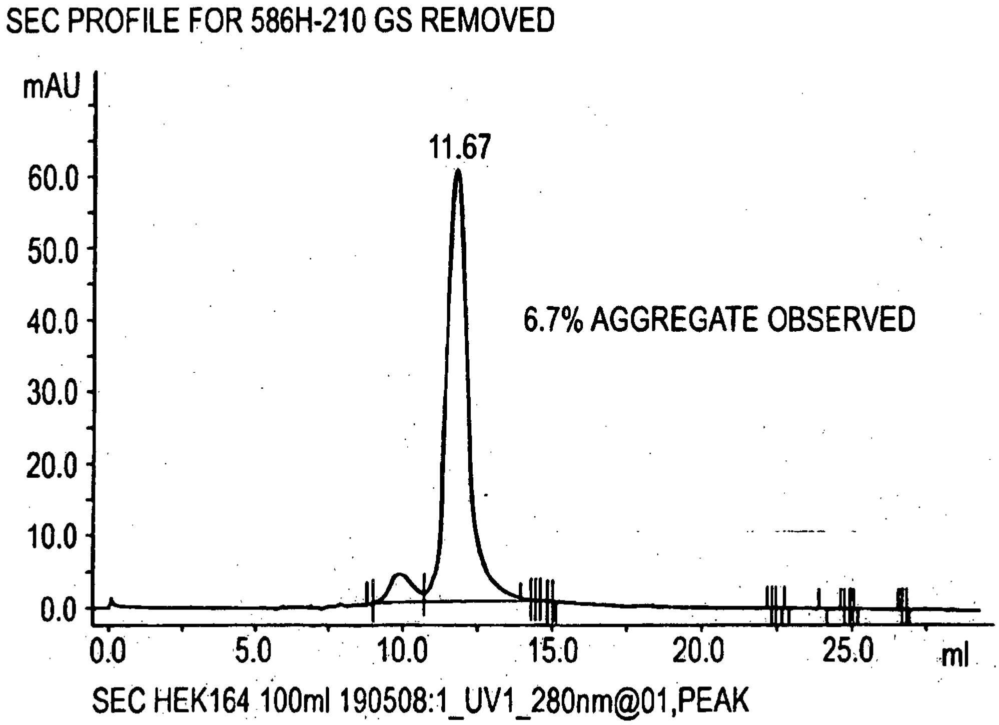
SEC PROFILE FOR 586H-210 GS REMOVED
mAU
60.0
50.0
40.0
30.0
20.0
10.0
0.0
11.67
6.7% AGGREGATE OBSERVED
0.0
5.0
10.0
15.0
20.0
25.0
ml
SEC HEK164 100ml 190508:1_UV1_280nm@01,PEAK

FIG. 93

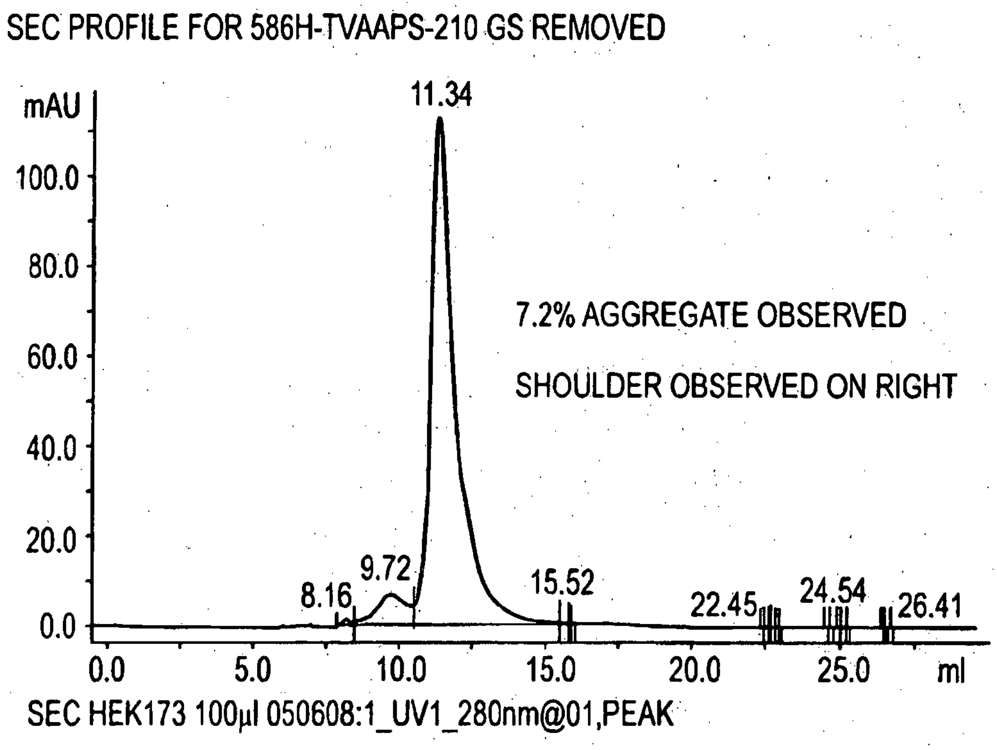
SEC PROFILE FOR 586H-TVAAPS-210 GS REMOVED
mAU
100.0
80.0
60.0
40.0
20.0
0.0
11.34
7.2% AGGREGATE OBSERVED
SHOULDER OBSERVED ON RIGHT
9.72
8.16
15.52
22.45
24.54
26.41
0.0
5.0
10.0
15.0
20.0
25.0
ml
SEC HEK173 100µl 050608:1_UV1_280nm@01,PEAK

*FIG. 94*

SDS PAGE FOR PASCOH-474 GS REMOVED (LANE B) AND PASCOH-TVAAPS-474 GS REMOVED (LANE A)

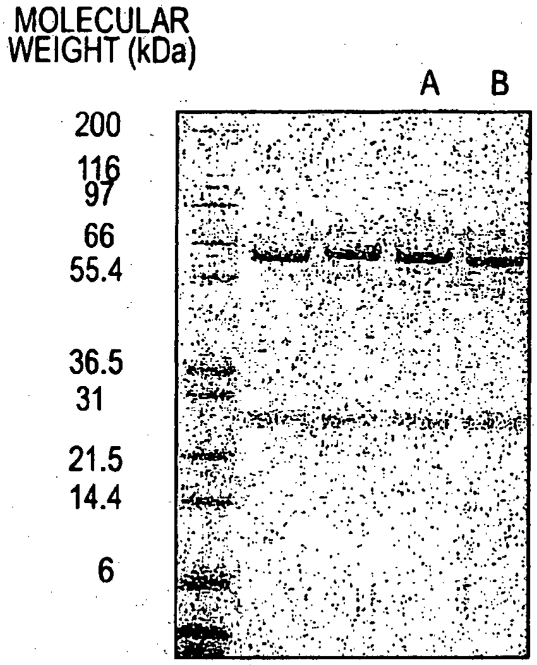


FIG. 95

SDS PAGE FOR PASCOH-GS-ASTKGPT-474 2nd GS REMOVED [A = NON-REDUCING CONDITIONS, B = REDUCING CONDITIONS]

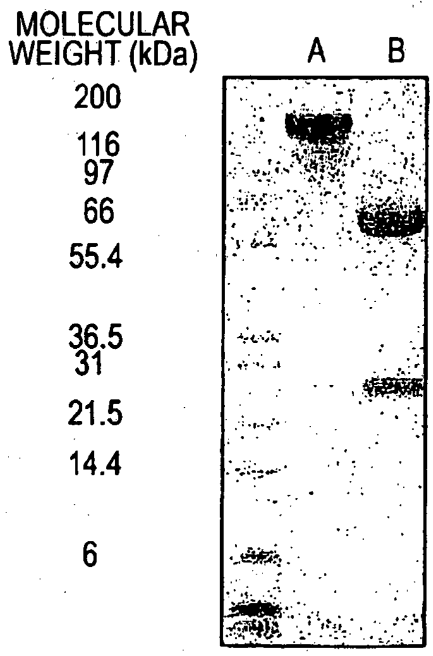


FIG. 96

SDS PAGE FOR 586H-210 GS REMOVED (LANE A)

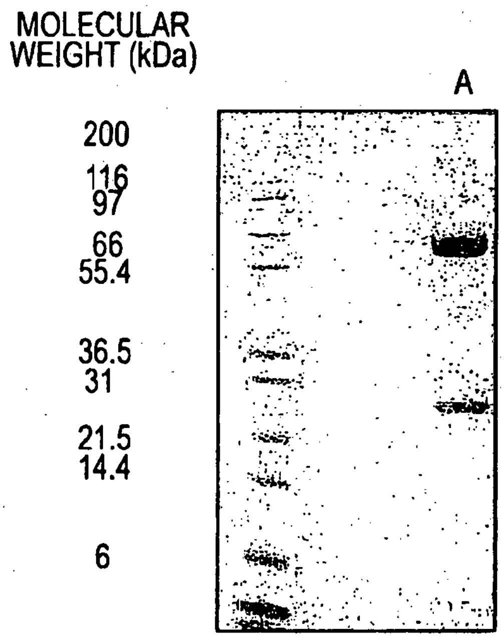


FIG. 97

SDS PAGE FOR 586H-TVAAPS-210 GS REMOVED (LANE A)

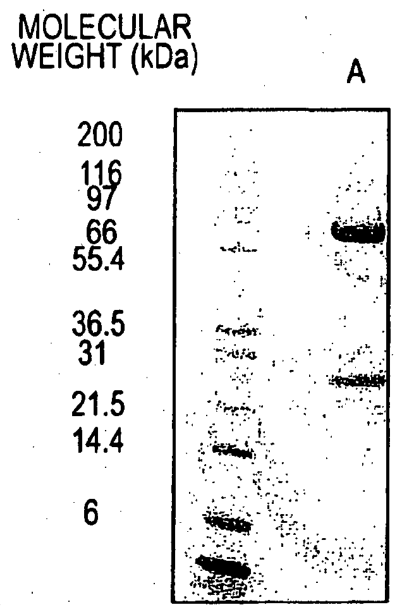


FIG. 98

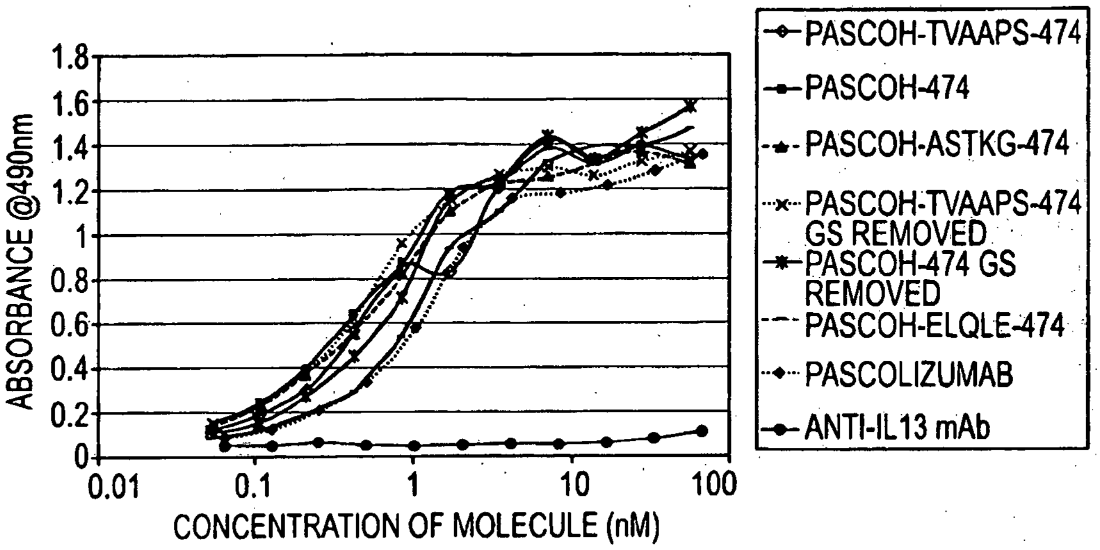


*FIG. 99*

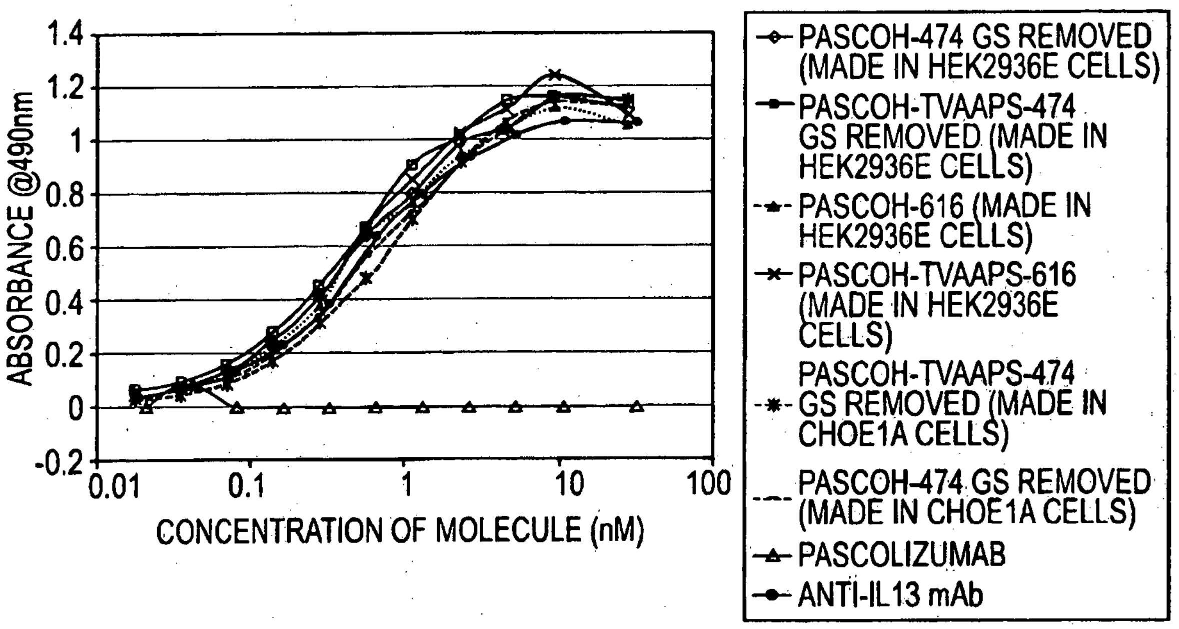


*FIG. 100*

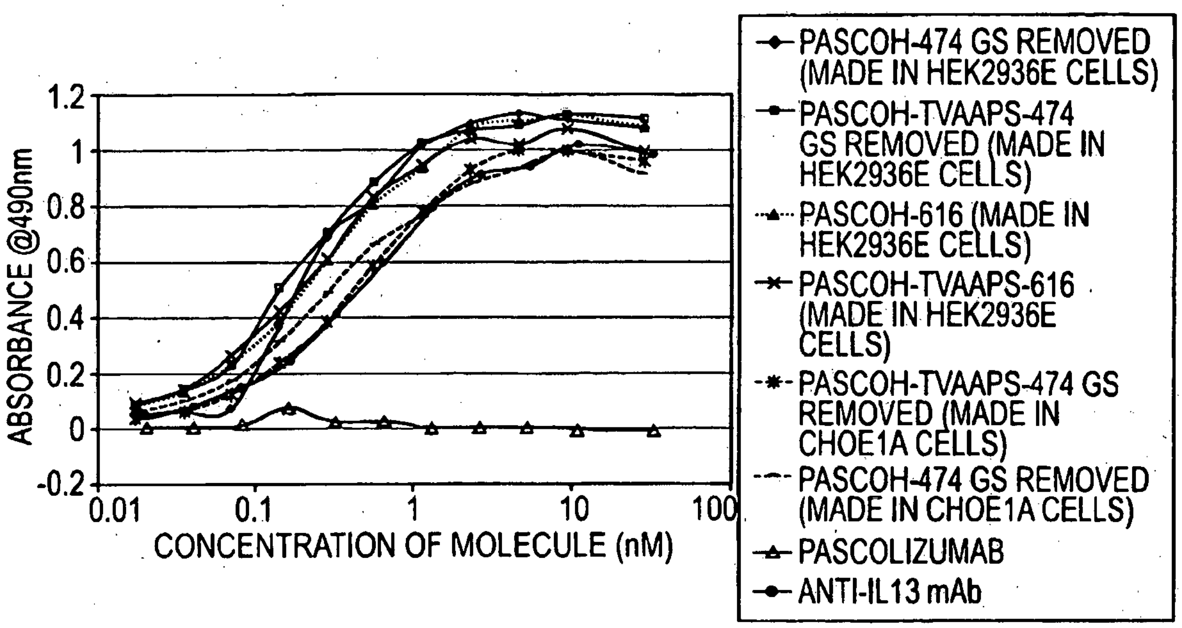


*FIG. 101*

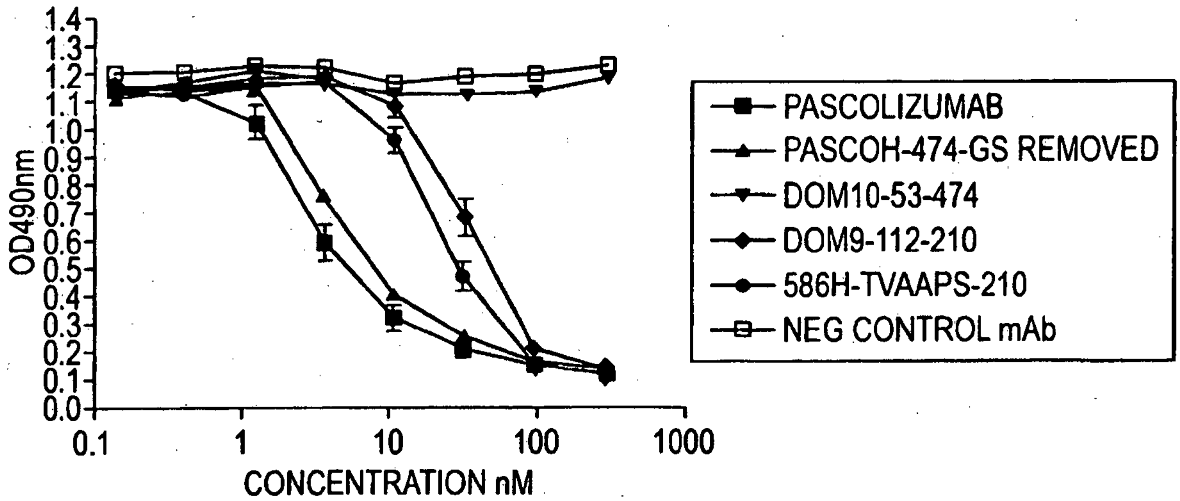


*FIG. 102*

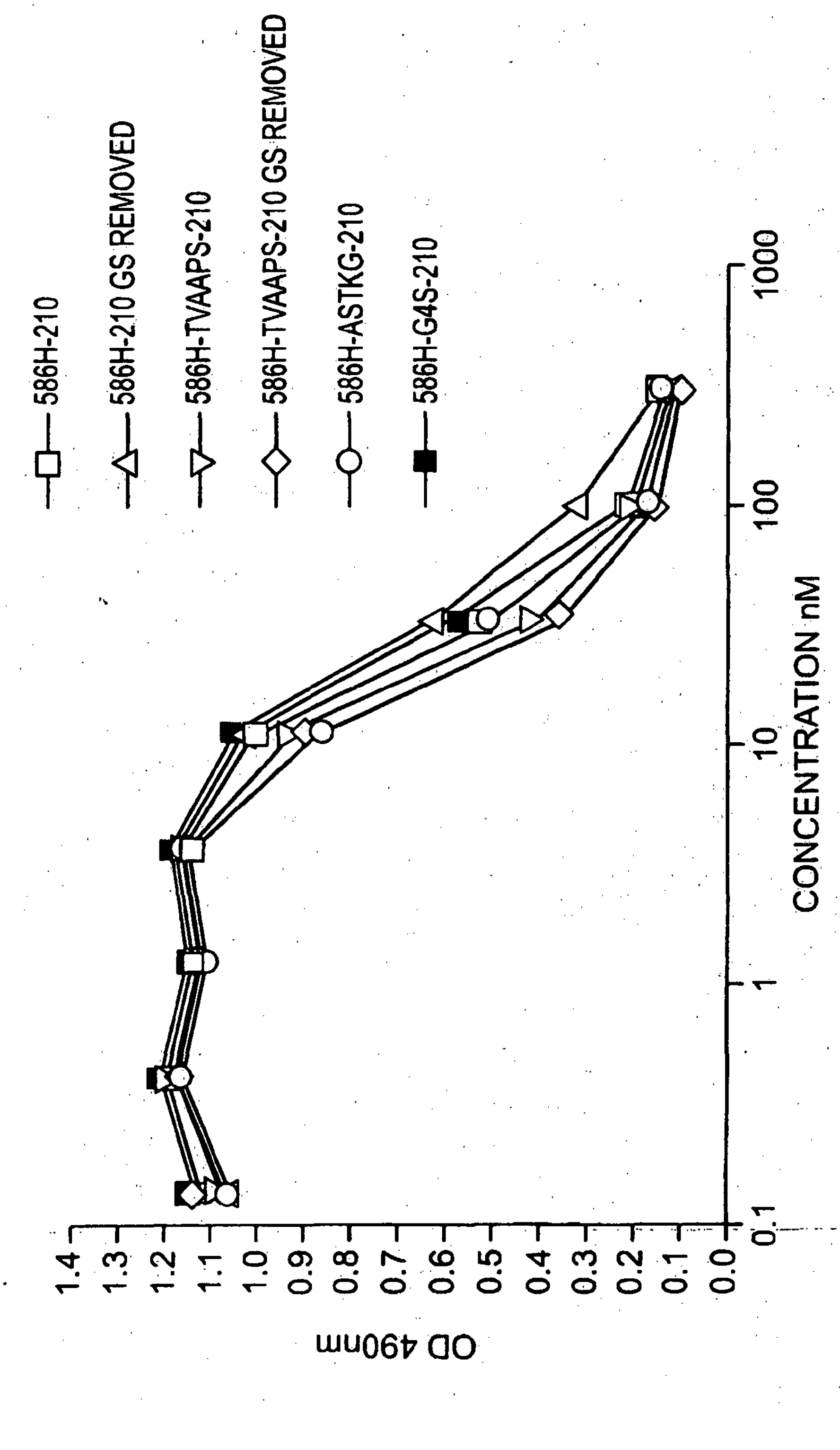
586H-210
586H-210 GS REMOVED
586H-TVAAPS-210
586H-TVAAPS-210 GS REMOVED
586H-ASTKG-210
586H-G4S-210
OD 490nm
1.4
1.3
1.2
1.1
1.0
0.9
0.8
0.7
0.6
0.5
0.4
0.3
0.2
0.1
0.0
0.1
1
10
100
1000
CONCENTRATION nM

*FIG. 103*

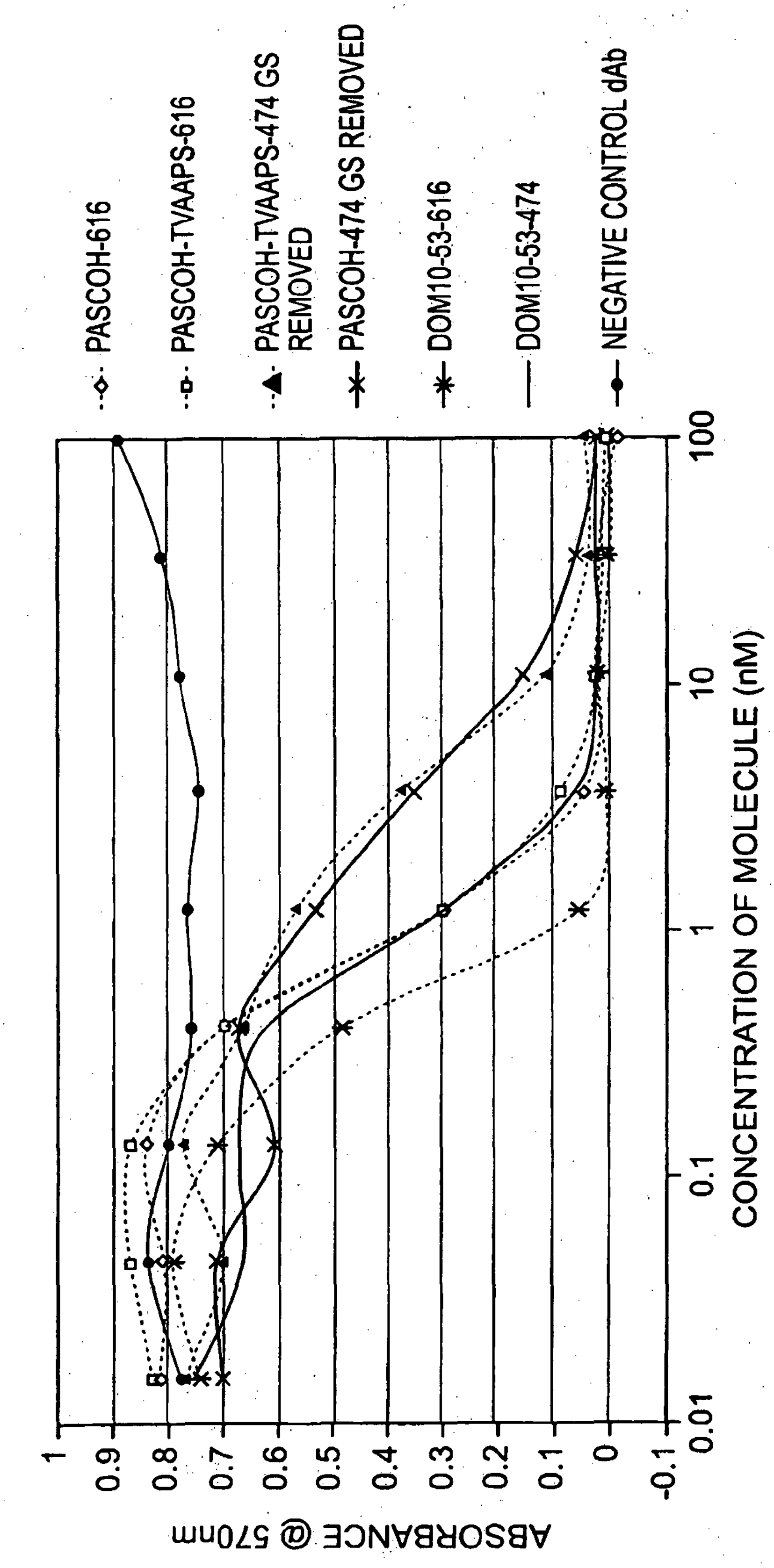
PASCOH-616
PASCOH-TVAAPS-616
PASCOH-TVAAPS-474 GS REMOVED
PASCOH-474 GS REMOVED
DOM10-53-616
DOM10-53-474
NEGATIVE CONTROL dAb
ABSORBANCE @ 570nm
1
0.9
0.8
0.7
0.6
0.5
0.4
0.3
0.2
0.1
0
-0.1
0.01
0.1
1
10
100
CONCENTRATION OF MOLECULE (nM)

FIG. 104

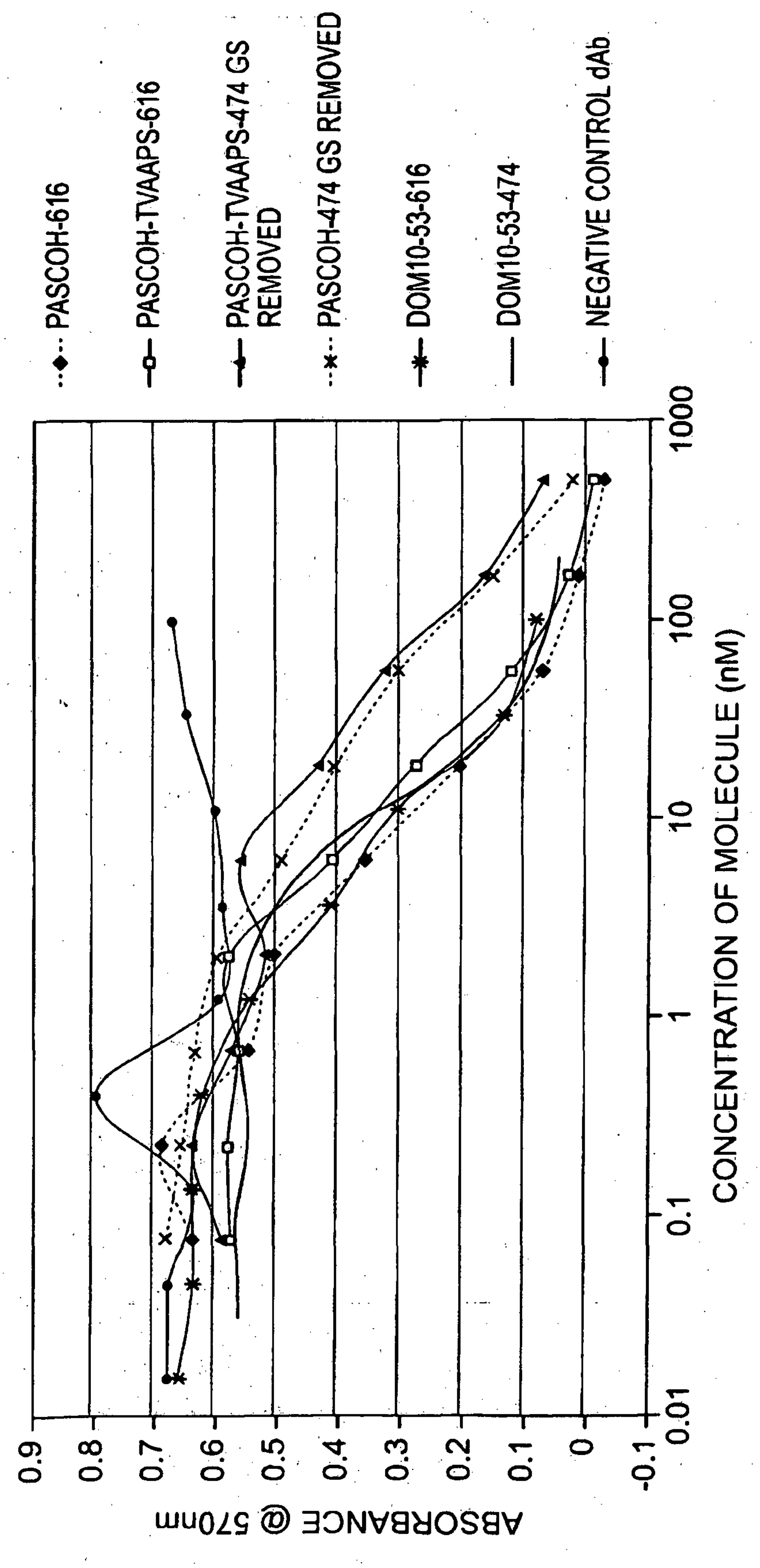
ABSORBANCE @ 570nm
-0.1
0
0.1
0.2
0.3
0.4
0.5
0.6
0.7
0.8
0.9
CONCENTRATION OF MOLECULE (nM)
0.01
0.1
1
10
100
1000
PASCOH-616
PASCOH-TVAAPS-616
PASCOH-TVAAPS-474 GS REMOVED
PASCOH-474 GS REMOVED
DOM10-53-616
DOM10-53-474
NEGATIVE CONTROL dAb

FIG. 105

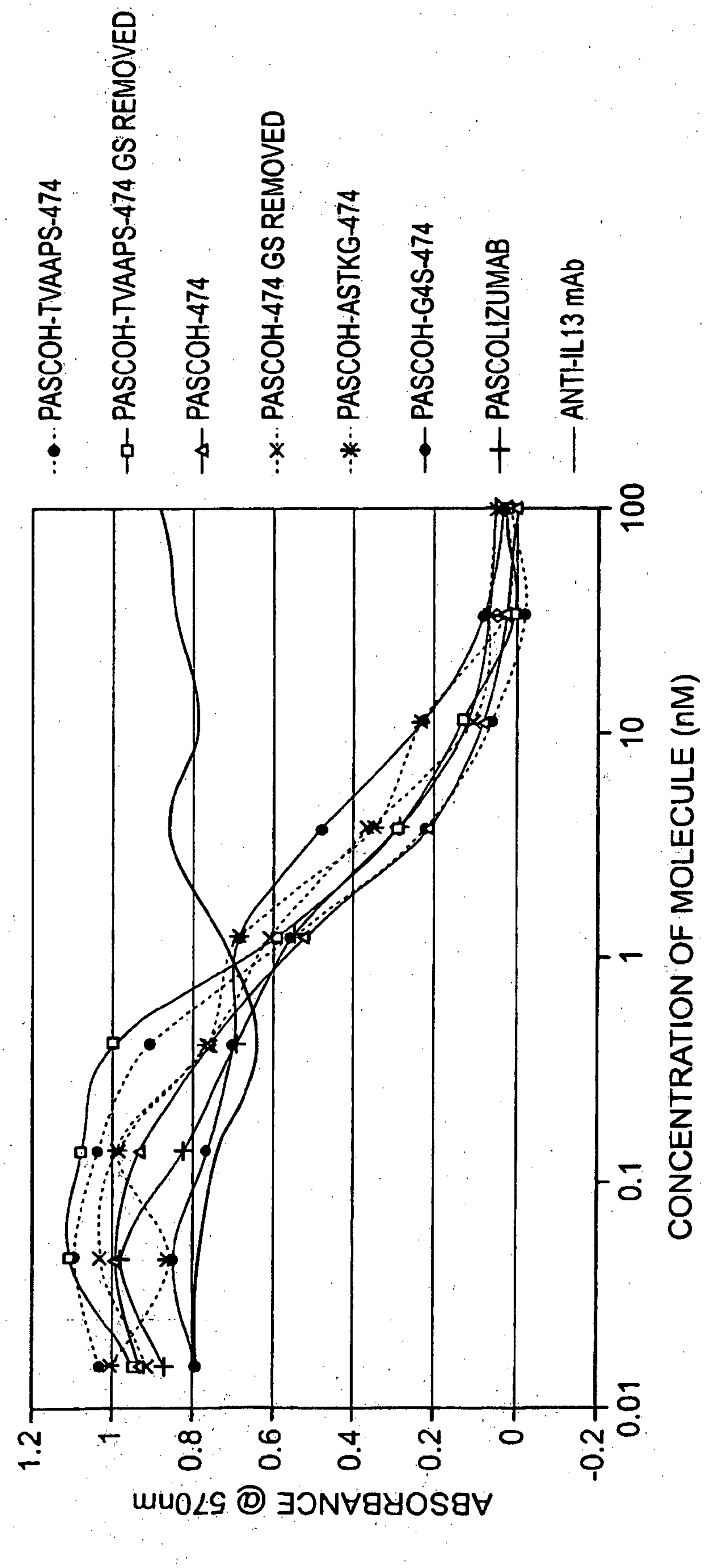
PASCOH-TVAAPS-474
PASCOH-TVAAPS-474 GS REMOVED
PASCOH-474
PASCOH-474 GS REMOVED
PASCOH-ASTKG-474
PASCOH-G4S-474
PASCOLIZUMAB
ANTI-IL13 mAb
ABSORBANCE @ 570nm
1.2
1
0.8
0.6
0.4
0.2
0
-0.2
0.01
0.1
1
10
100
CONCENTRATION OF MOLECULE (nM)

*FIG. 106*

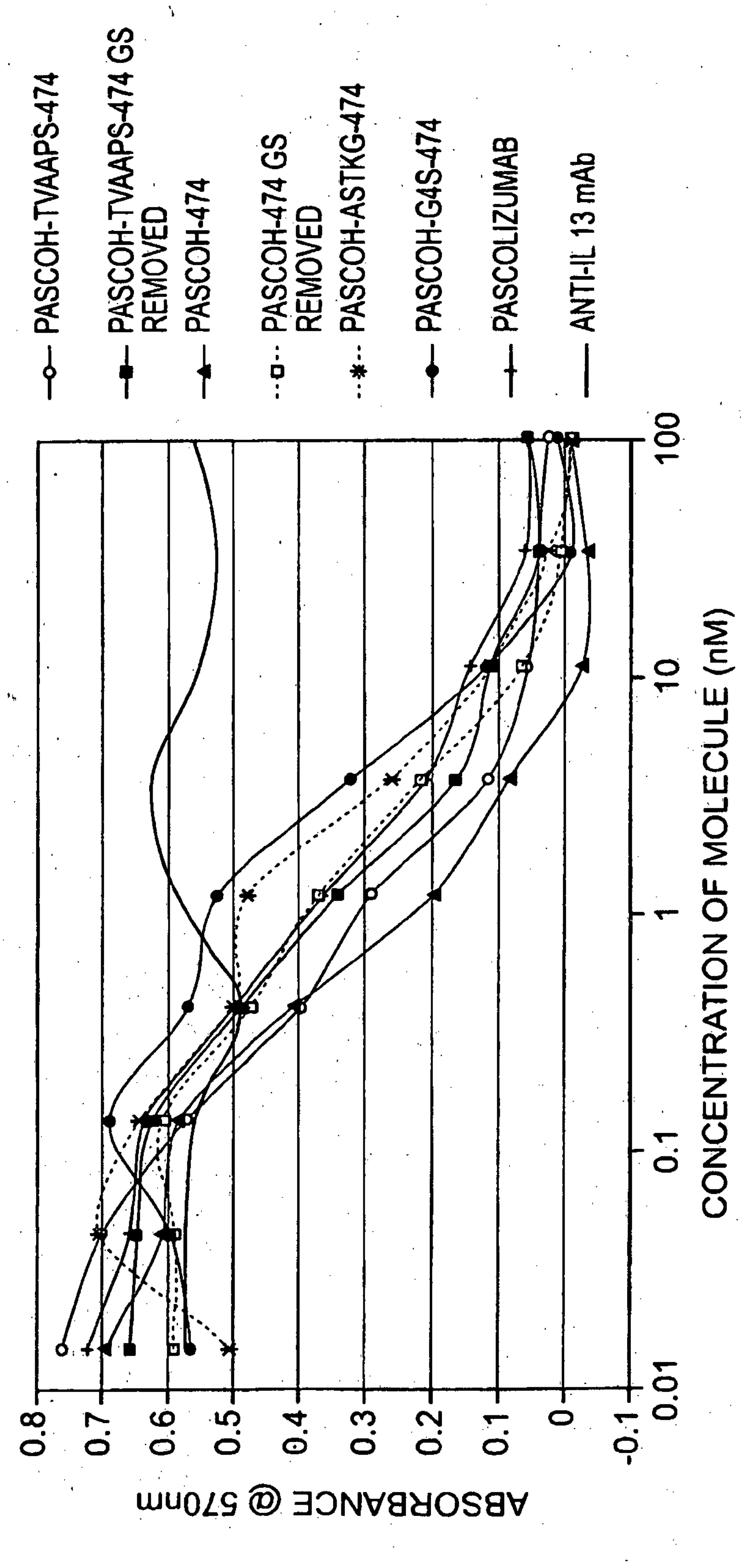
PASCOH-TVAAPS-474
PASCOH-TVAAPS-474 GS REMOVED
PASCOH-474
PASCOH-474 GS REMOVED
PASCOH-ASTKG-474
PASCOH-G4S-474
PASCOLIZUMAB
ANTI-IL 13 mAb
ABSORBANCE @ 570nm
0.8
0.7
0.6
0.5
0.4
0.3
0.2
0.1
0
-0.1
0.01
0.1
1
10
100
CONCENTRATION OF MOLECULE (nM)

FIG. 107

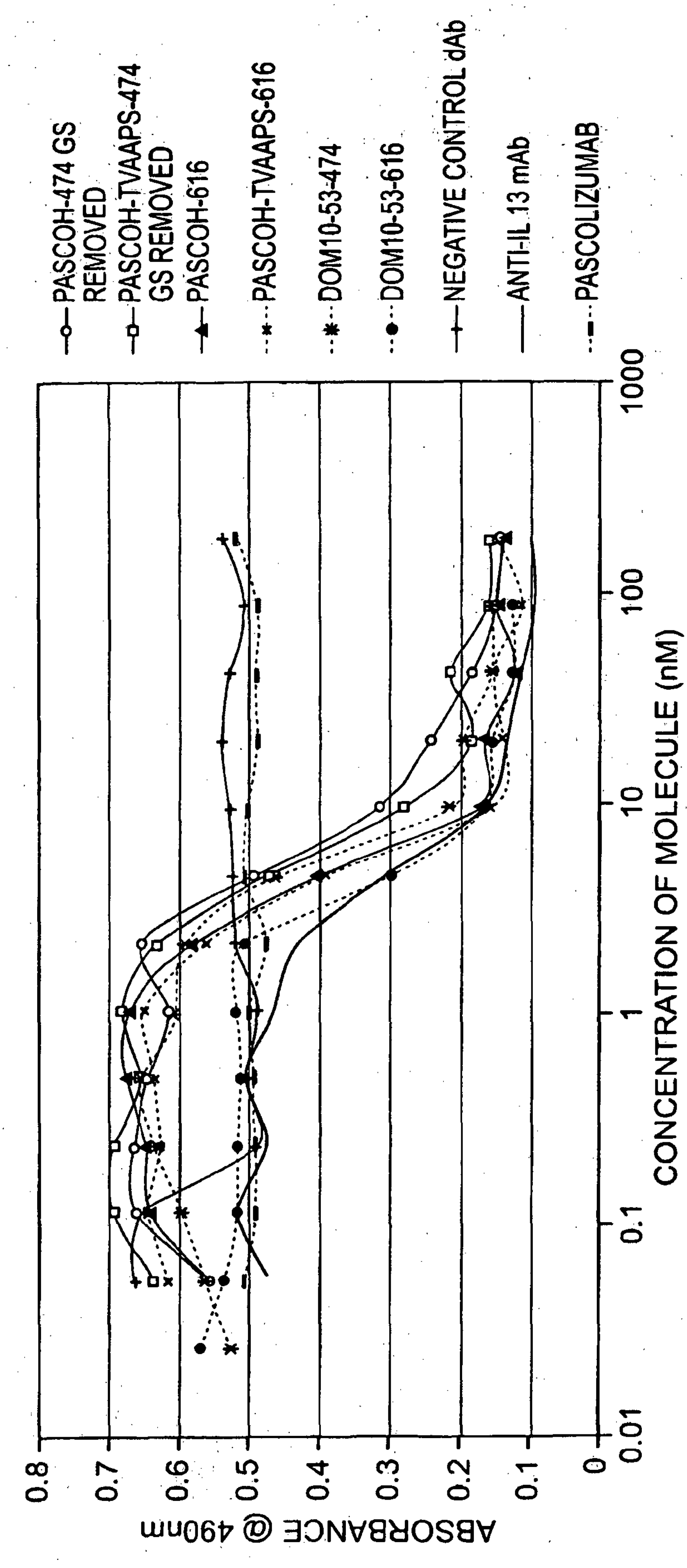
ABSORBANCE @ 490nm
0.8
0.7
0.6
0.5
0.4
0.3
0.2
0.1
0
0.01
0.1
1
10
100
1000
CONCENTRATION OF MOLECULE (nM)
PASCOH-474 GS REMOVED
PASCOH-TVAAPS-474 GS REMOVED
PASCOH-616
PASCOH-TVAAPS-616
DOM10-53-474
DOM10-53-616
NEGATIVE CONTROL dAb
ANTI-IL 13 mAb
PASCOLIZUMAB

*FIG. 108*

SEC PROFILE FOR PASCOH-616

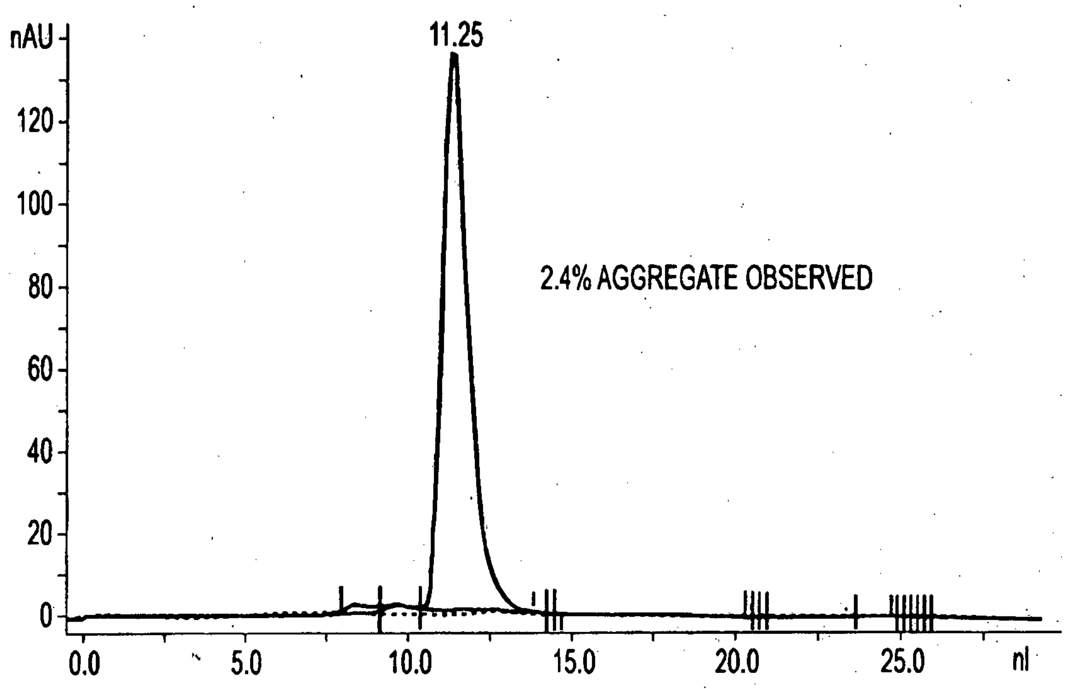


FIG. 109

SEC PROFILE FOR PASCOH-TVAAPS_616

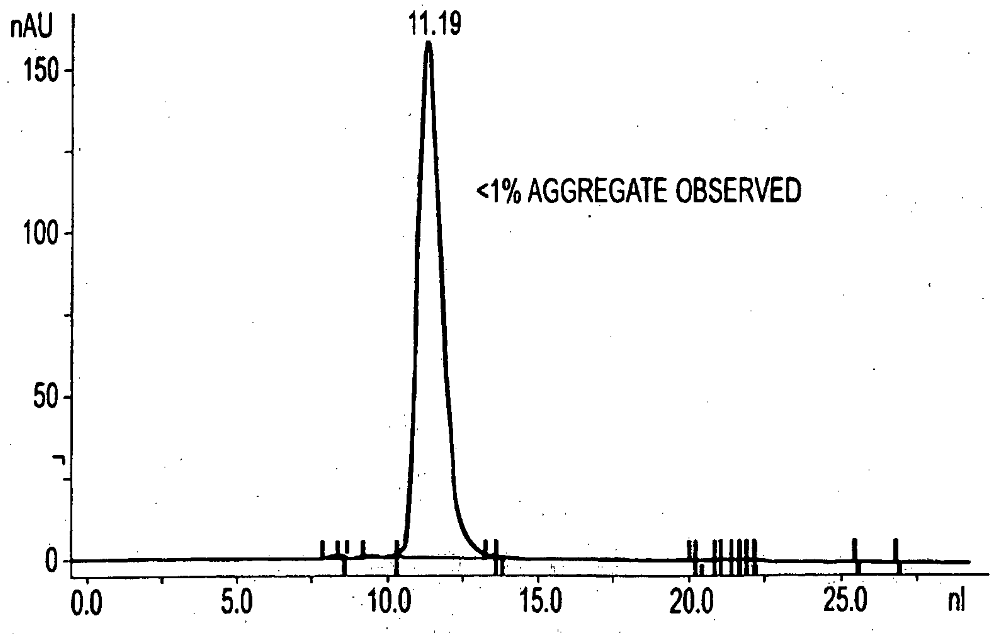


FIG. 110

SDS PAGE FOR PASCOH-616
[E1 = NON-REDUCING CONDITIONS, E2 = REDUCING CONDITIONS]

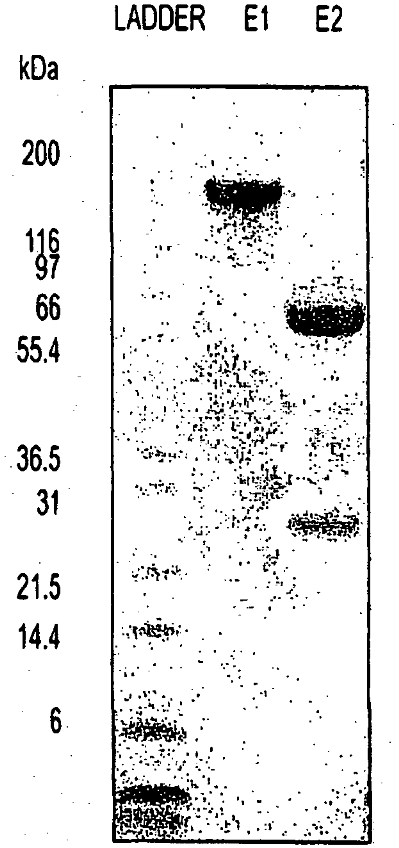


FIG. 111

SDS PAGE FOR PASCOH-TVAAPS-616
[A = NON-REDUCING CONDITIONS, B = REDUCING CONDITIONS]

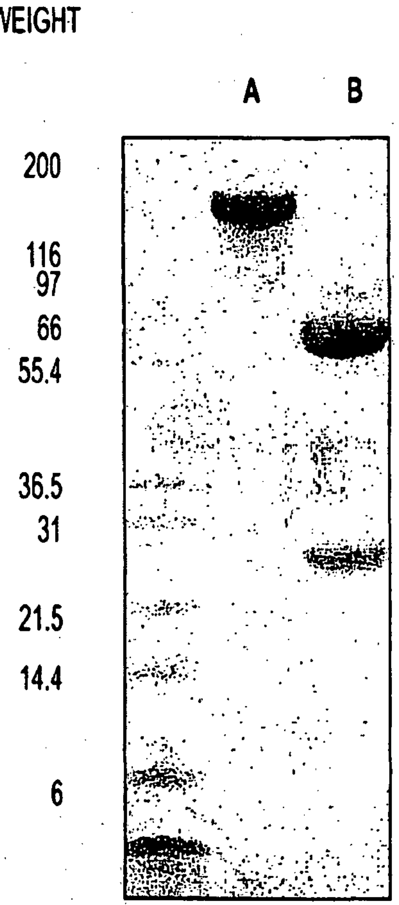


FIG. 112

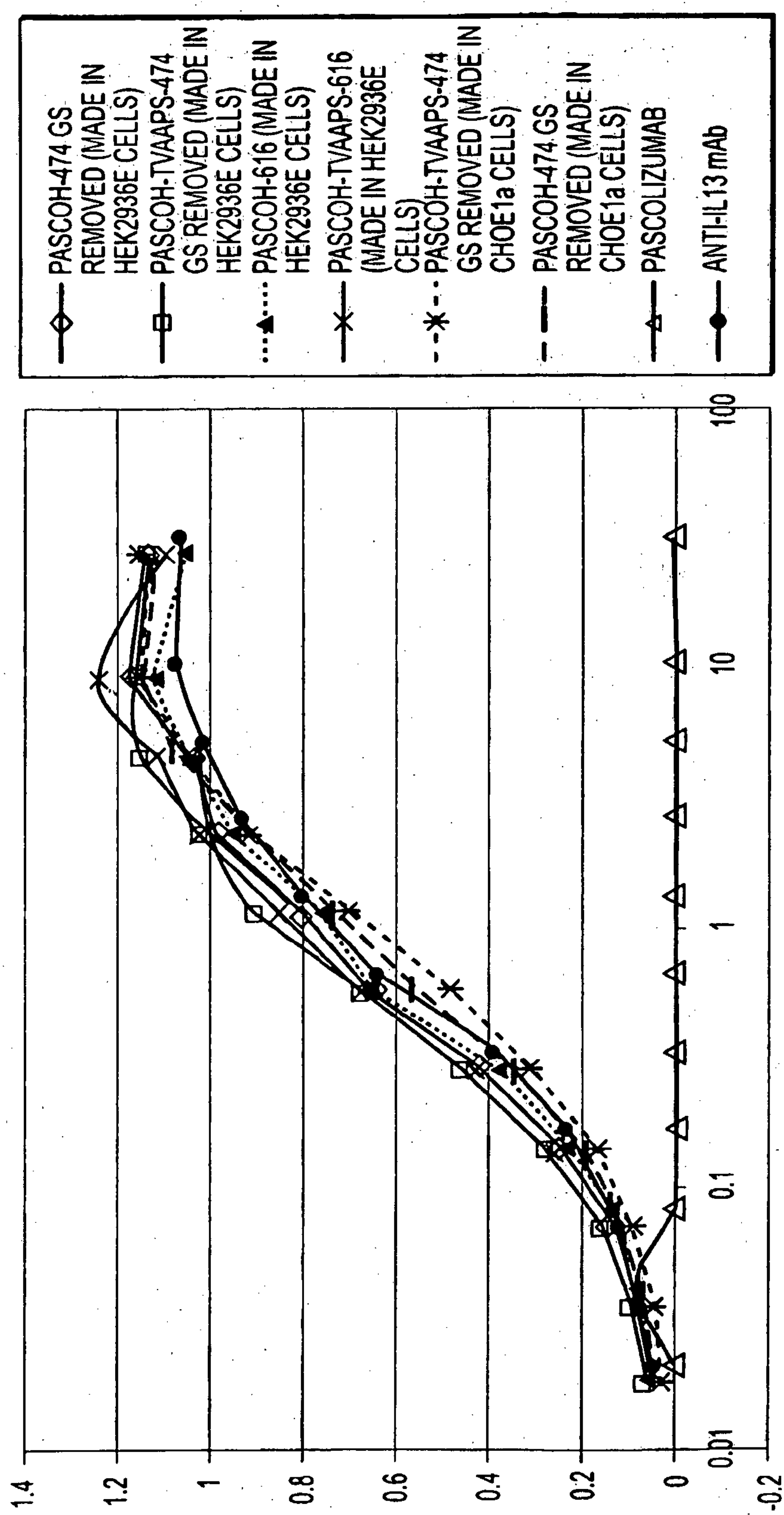
PASCOH-474 GS REMOVED (MADE IN HEK2936E CELLS)
PASCOH-TVAAPS-474 GS REMOVED (MADE IN HEK2936E CELLS)
PASCOH-616 (MADE IN HEK2936E CELLS)
PASCOH-TVAAPS-616 (MADE IN HEK2936E CELLS)
PASCOH-TVAAPS-474 GS REMOVED (MADE IN CHOE1a CELLS)
PASCOH-474 GS REMOVED (MADE IN CHOE1a CELLS)
PASCOLIZUMAB
ANTI-IL13 mAb
ABSORBANCE @490nm
1.4
1.2
1
0.8
0.6
0.4
0.2
0
-0.2
0.01
0.1
1
10
100
CONCENTRATION OF MOLECULE (nM)

FIG. 113

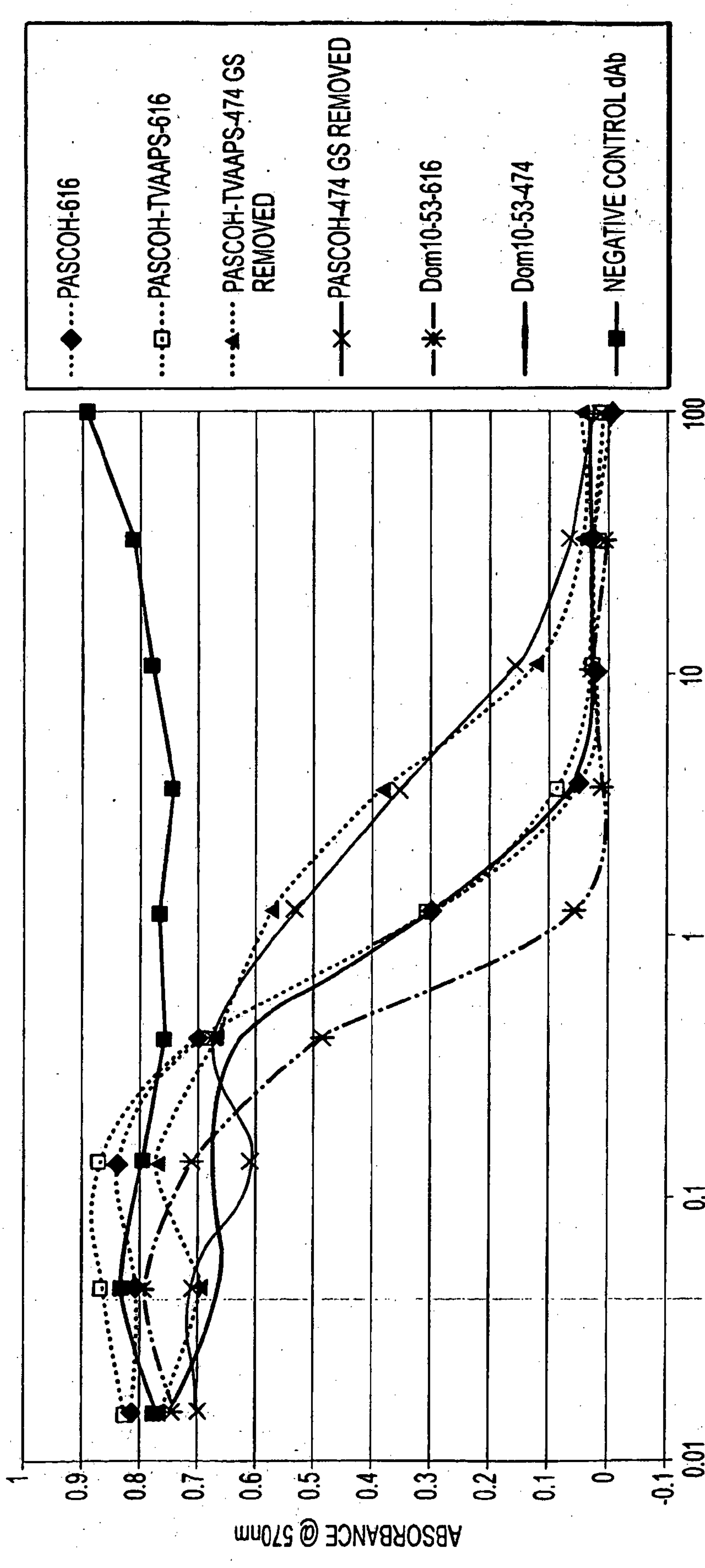
PASCOH-616
PASCOH-TVAAPS-616
PASCOH-TVAAPS-474 GS REMOVED
PASCOH-474 GS REMOVED
Dom10-53-616
Dom10-53-474
NEGATIVE CONTROL dAb
ABSORBANCE @ 570nm
1
0.9
0.8
0.7
0.6
0.5
0.4
0.3
0.2
0.1
0
-0.1
0.01
0.1
1
10
100
CONCENTRATION OF MOLECULE (nM)

FIG. 114

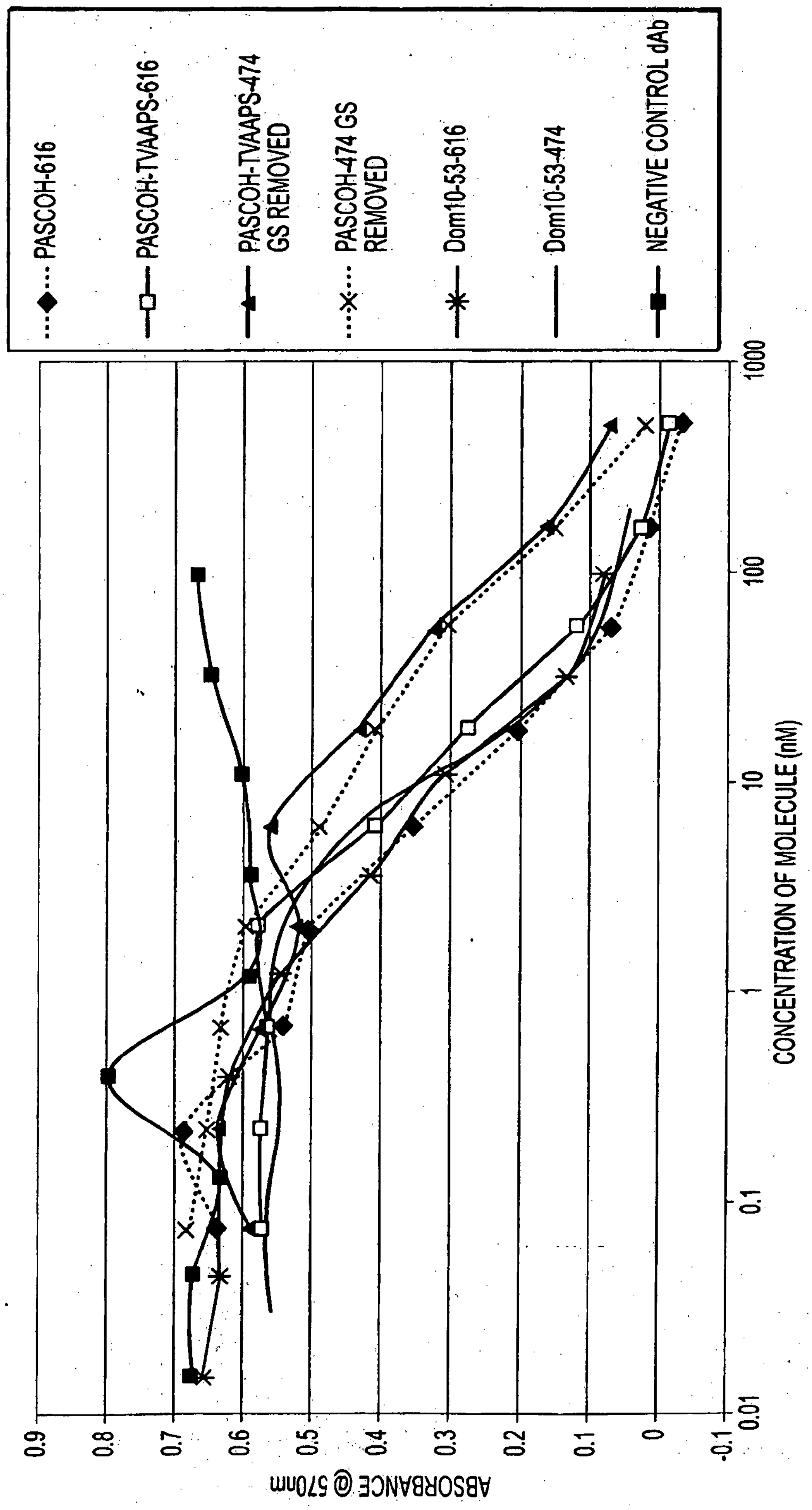
PASCOH-616
PASCOH-TVAAPS-616
PASCOH-TVAAPS-474 GS REMOVED
PASCOH-474 GS REMOVED
Dom10-53-616
Dom10-53-474
NEGATIVE CONTROL dAb
ABSORBANCE @ 570nm
CONCENTRATION OF MOLECULE (nM)
0.9
0.8
0.7
0.6
0.5
0.4
0.3
0.2
0.1
0
-0.1
0.01
0.1
1
10
100
1000

FIG. 114a

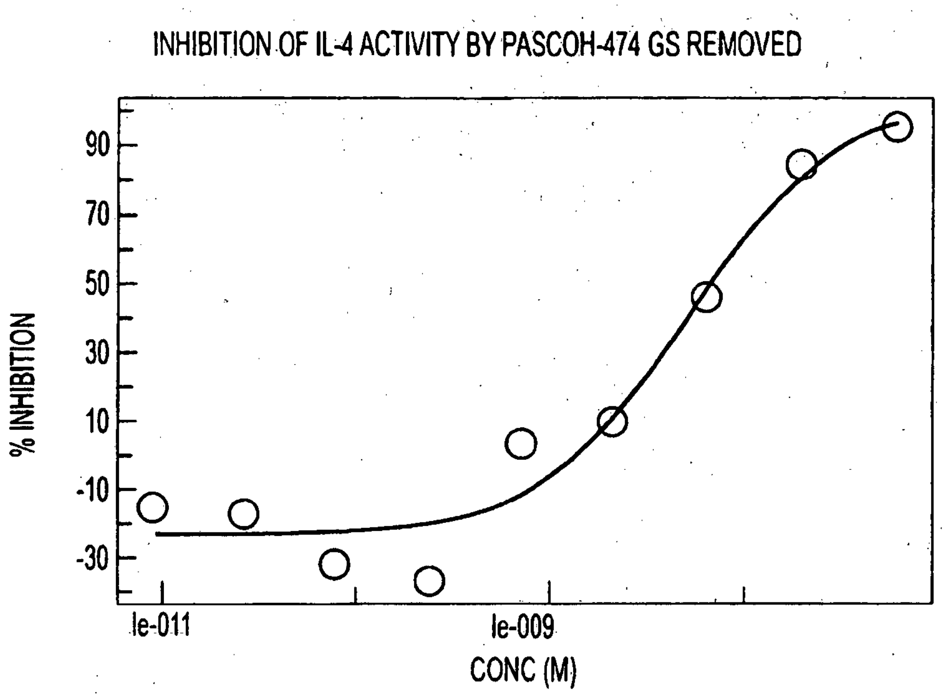


FIG. 115

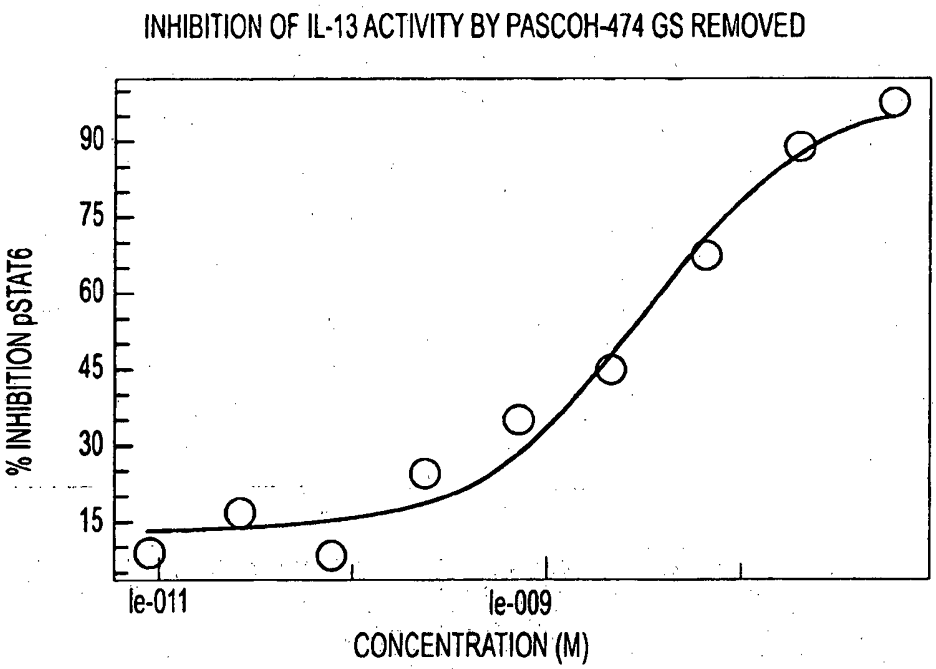


FIG. 116

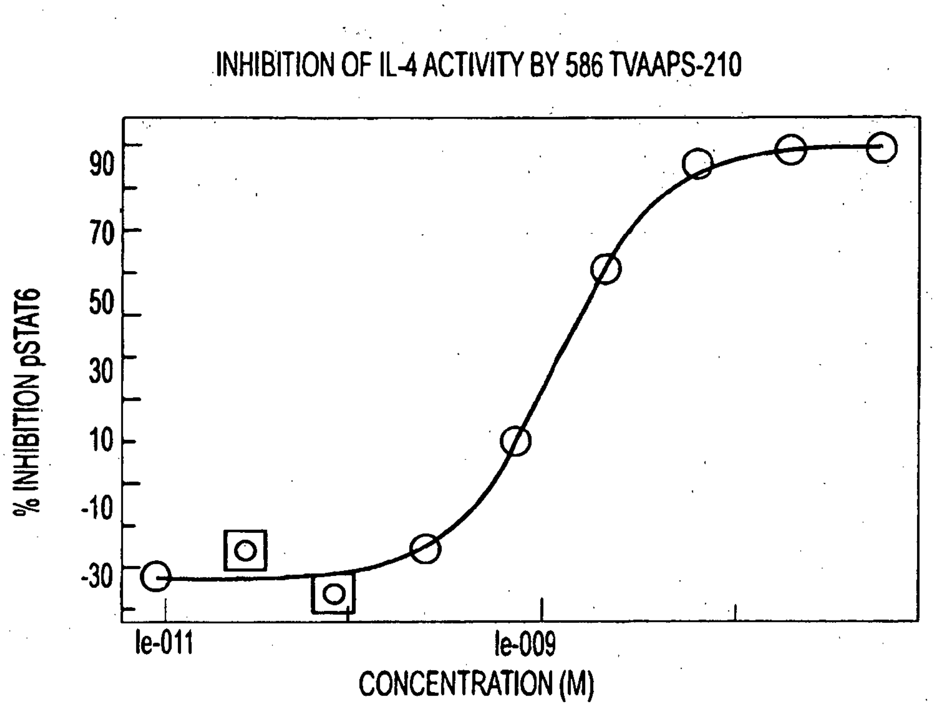


FIG. 117

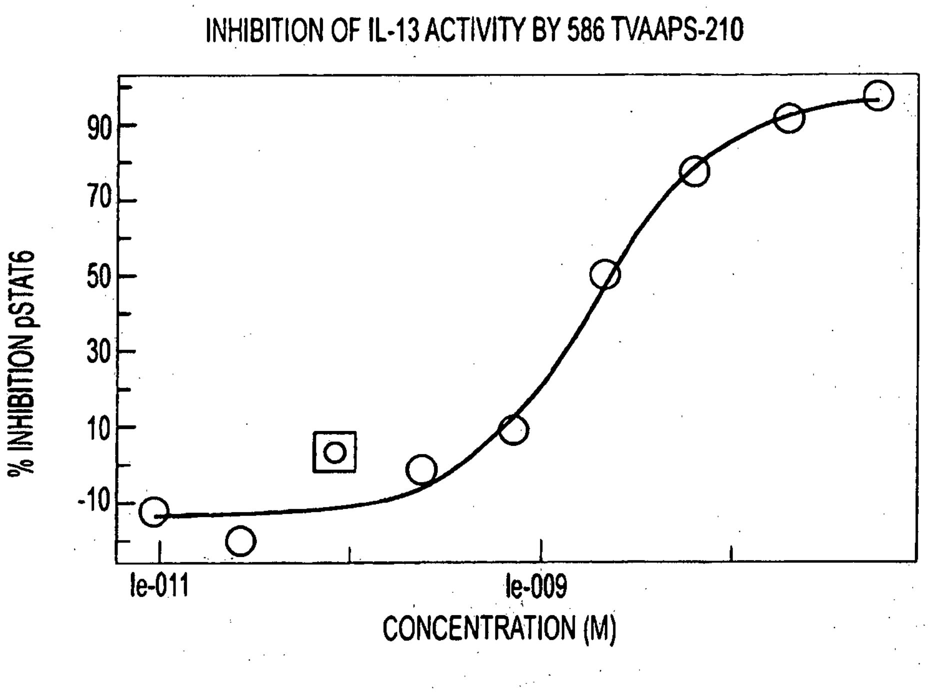


FIG. 118

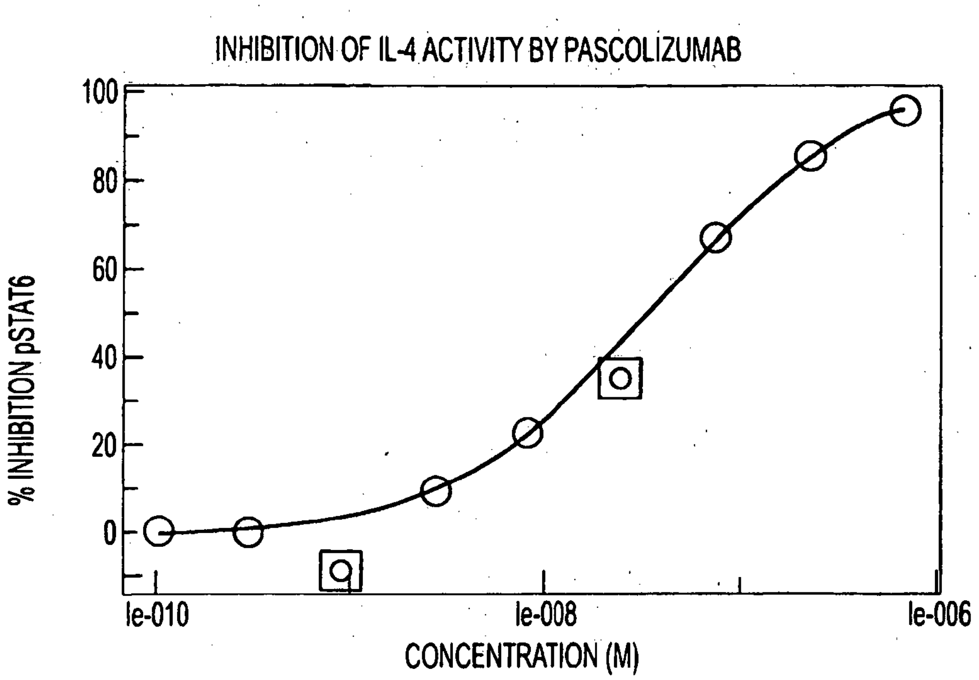


*FIG. 119*

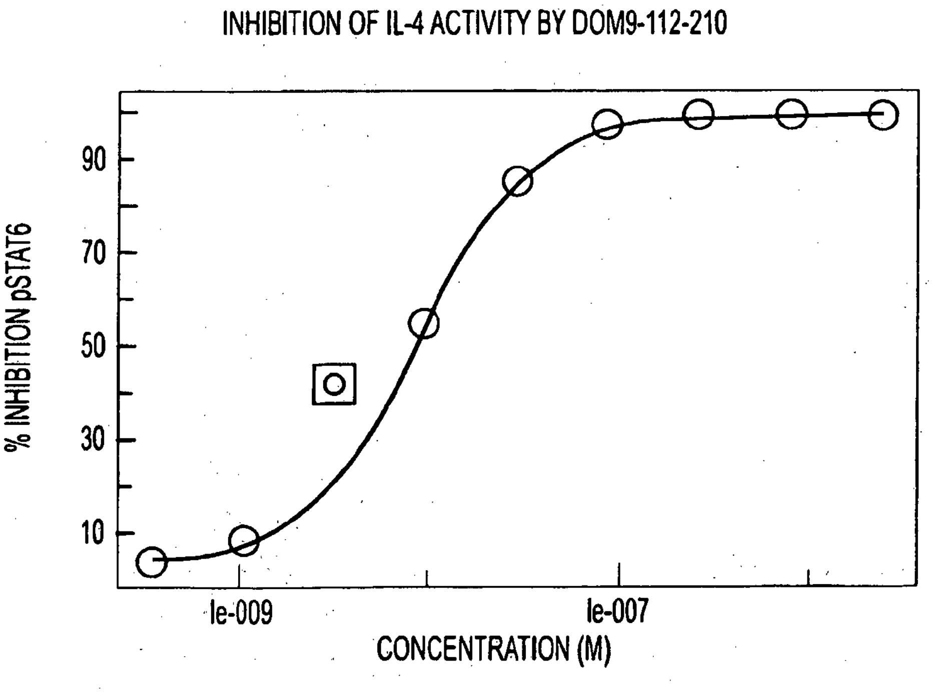


*FIG. 120*

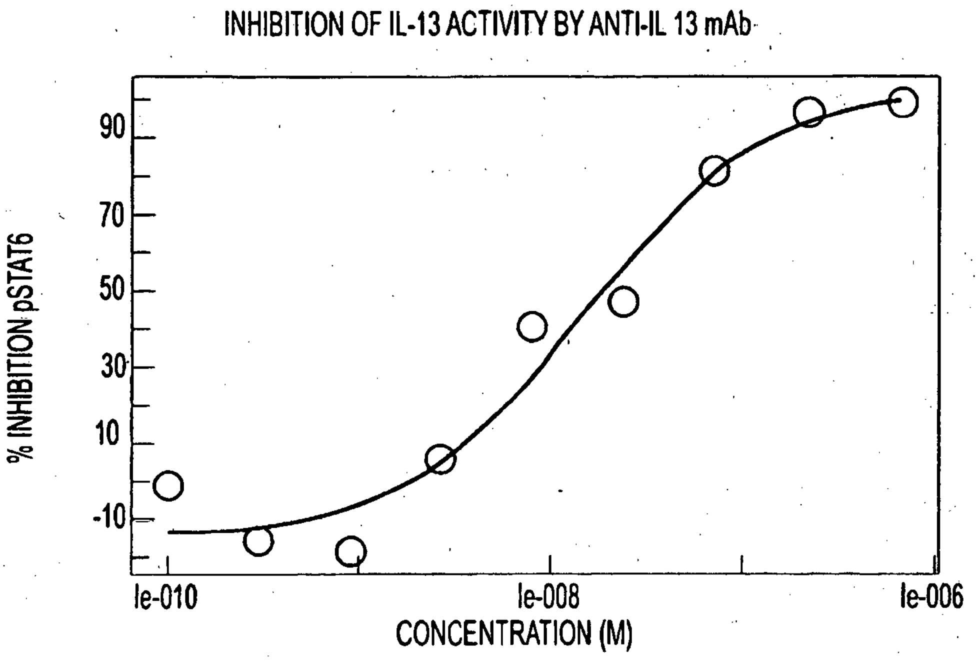


FIG. 121

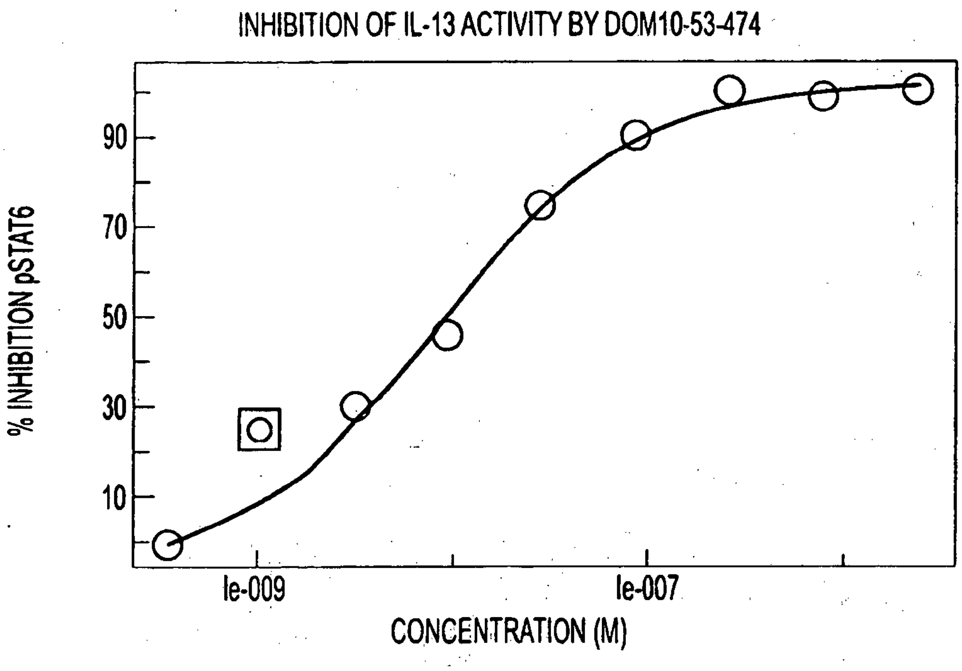


FIG. 122

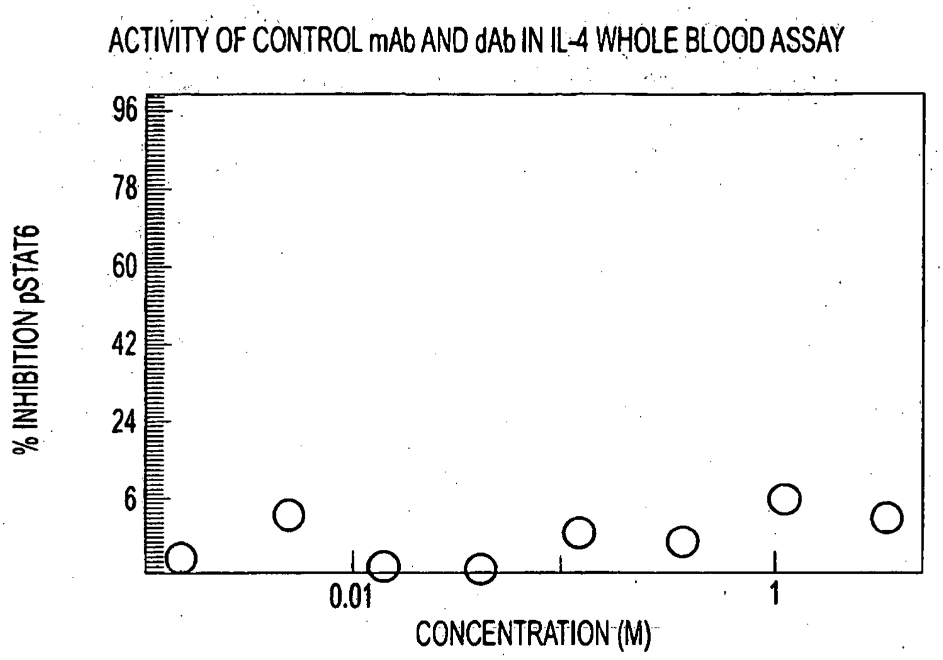


FIG. 123

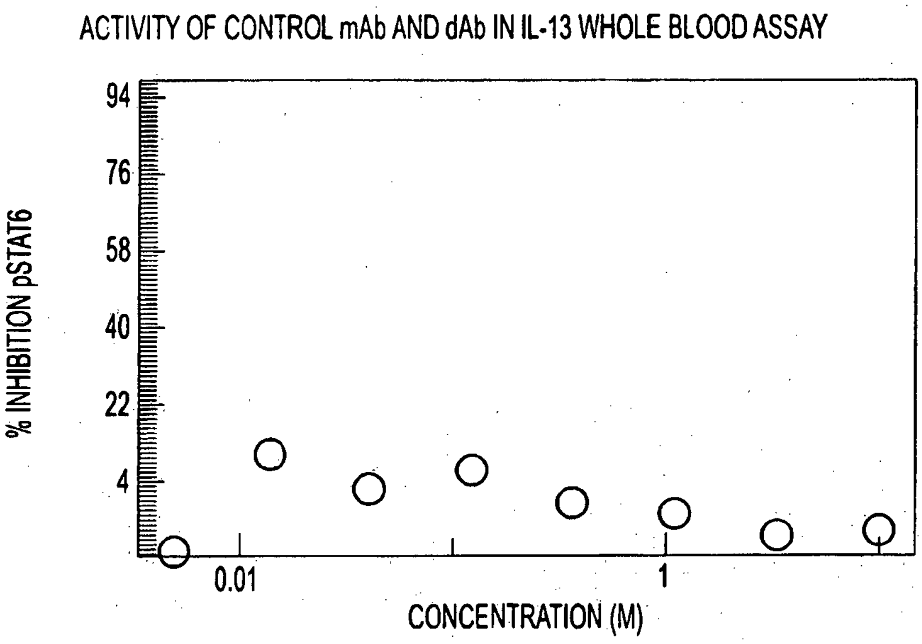


FIG. 124

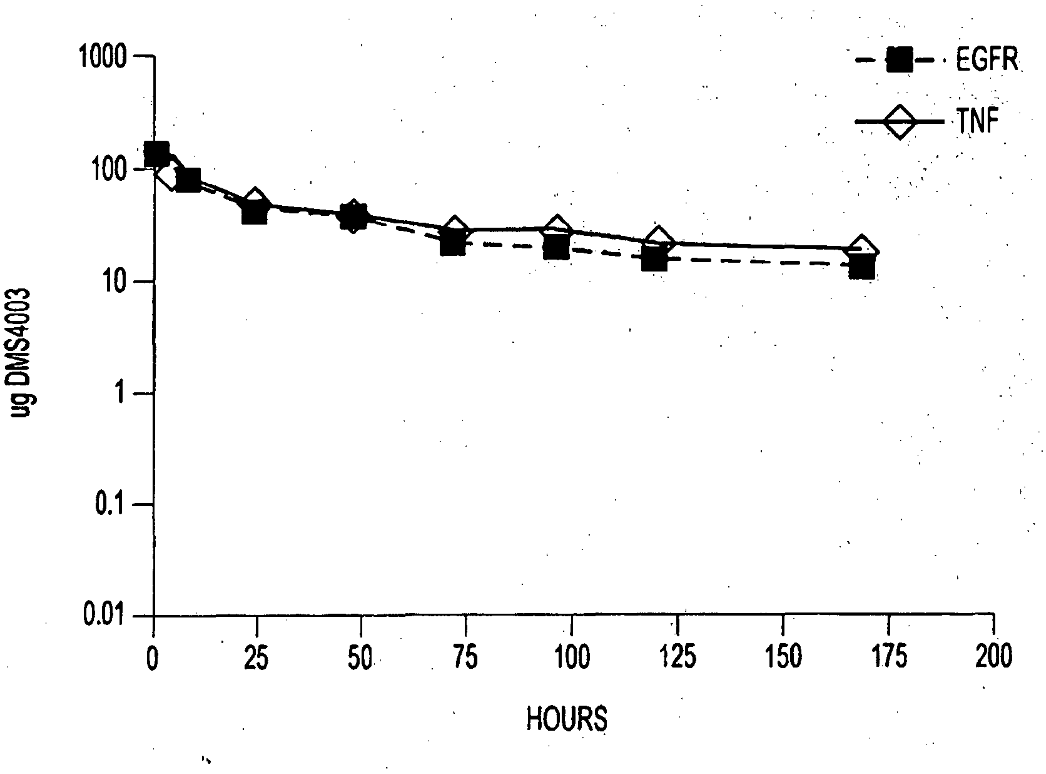


*FIG. 125*

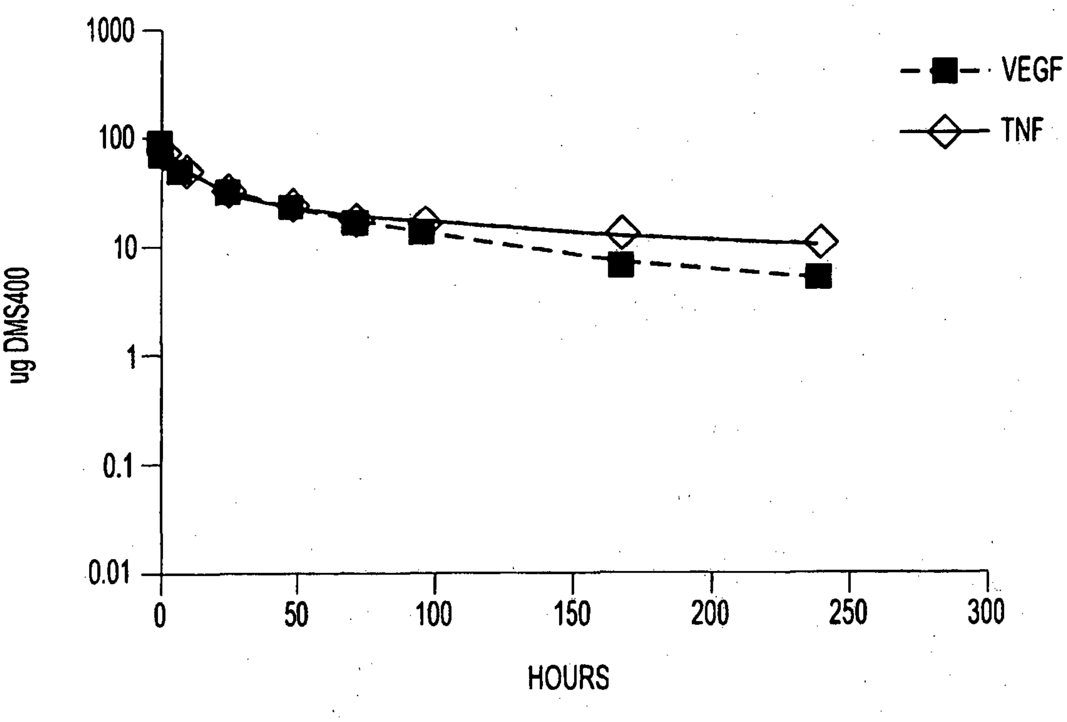


*FIG. 126*

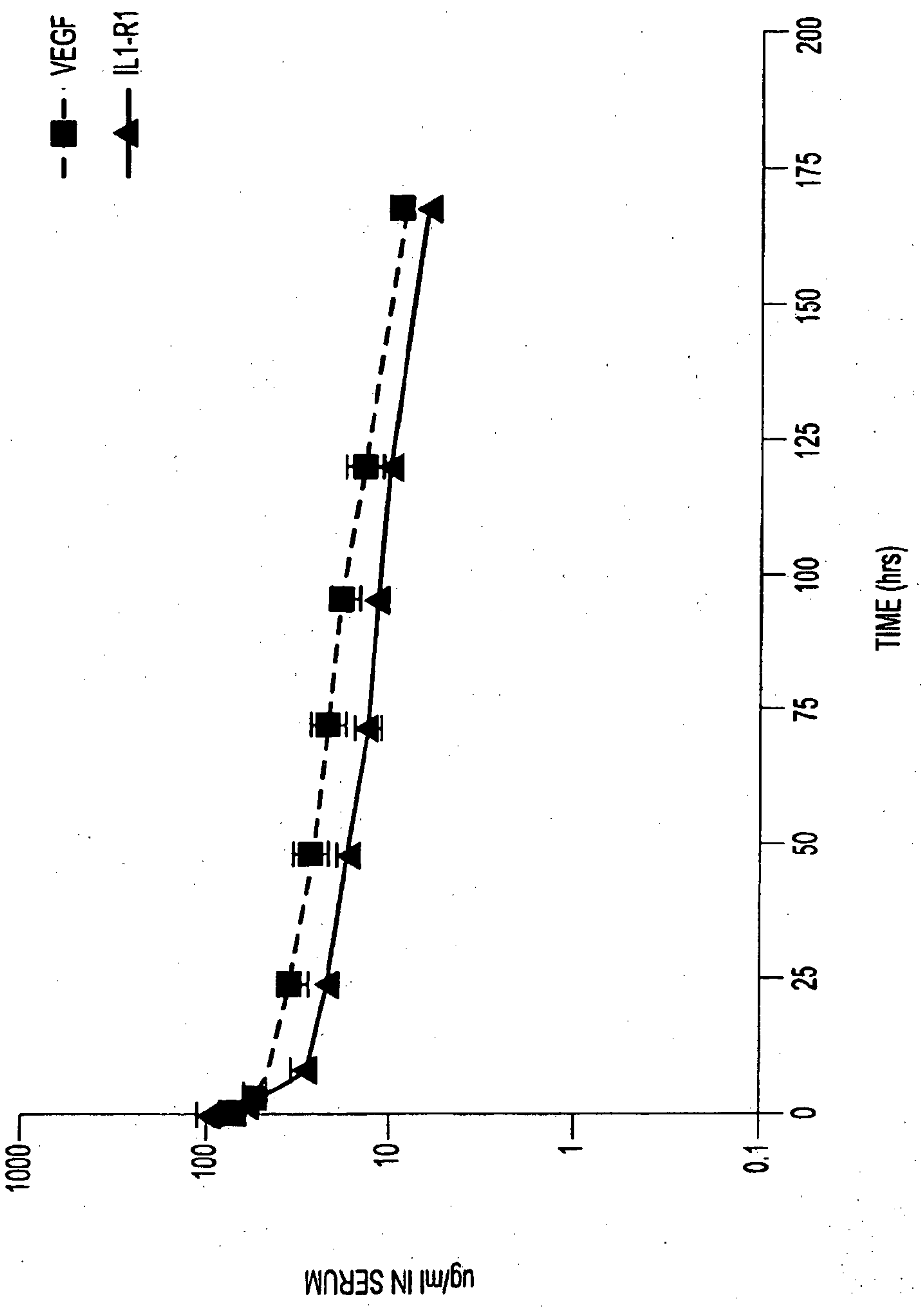
VEGF
IL1-R1
ug/ml IN SERUM
1000
100
10
1
0.1
0
25
50
75
100
125
150
175
200
TIME (hrs)

FIG. 127

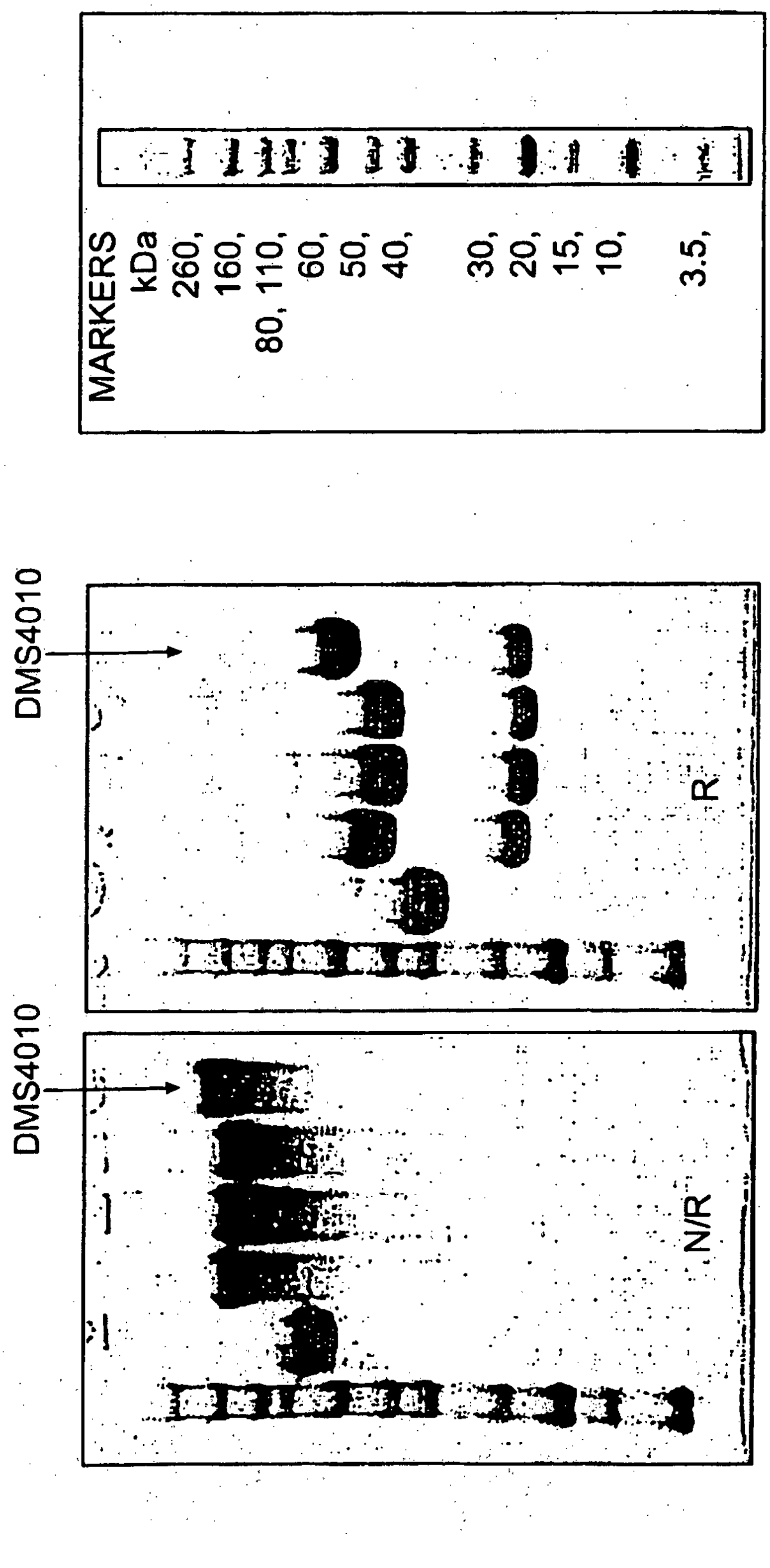


N/R=NON-REDUCED; R=REDUCED

FIG. 128

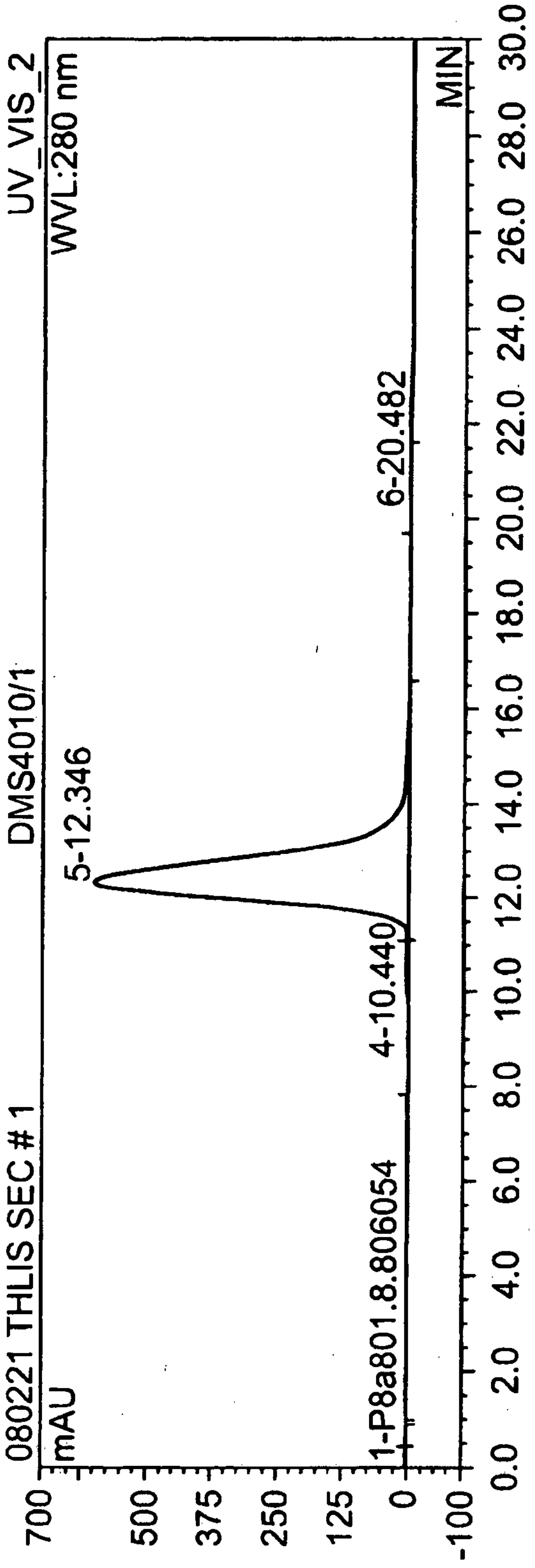
080221 THLIS SEC # 1
DMS4010/1
UV_VIS_2
WVL:280 nm
mAU
MIN
5-12.346
4-10.440
6-20.482
1-P8a801.8.806054
700
500
375
250
125
0
-100
0.0
2.0
4.0
6.0
8.0
10.0
12.0
14.0
16.0
18.0
20.0
22.0
24.0
26.0
28.0
30.0

*FIG. 129*

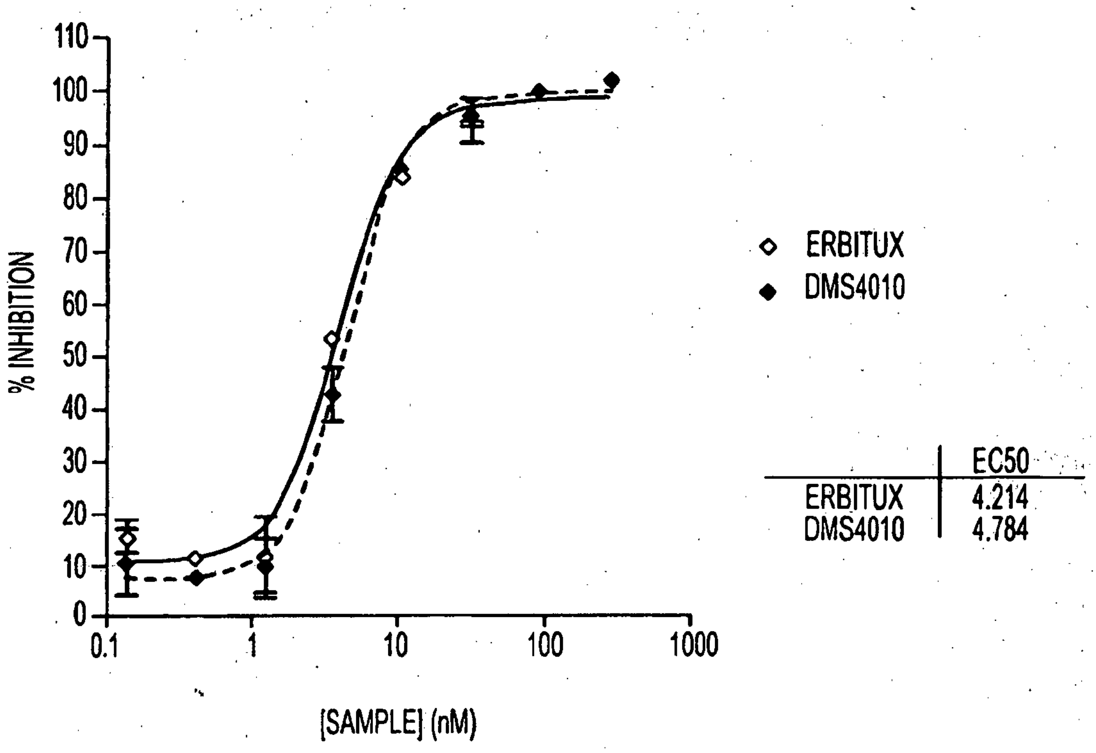


FIG. 130

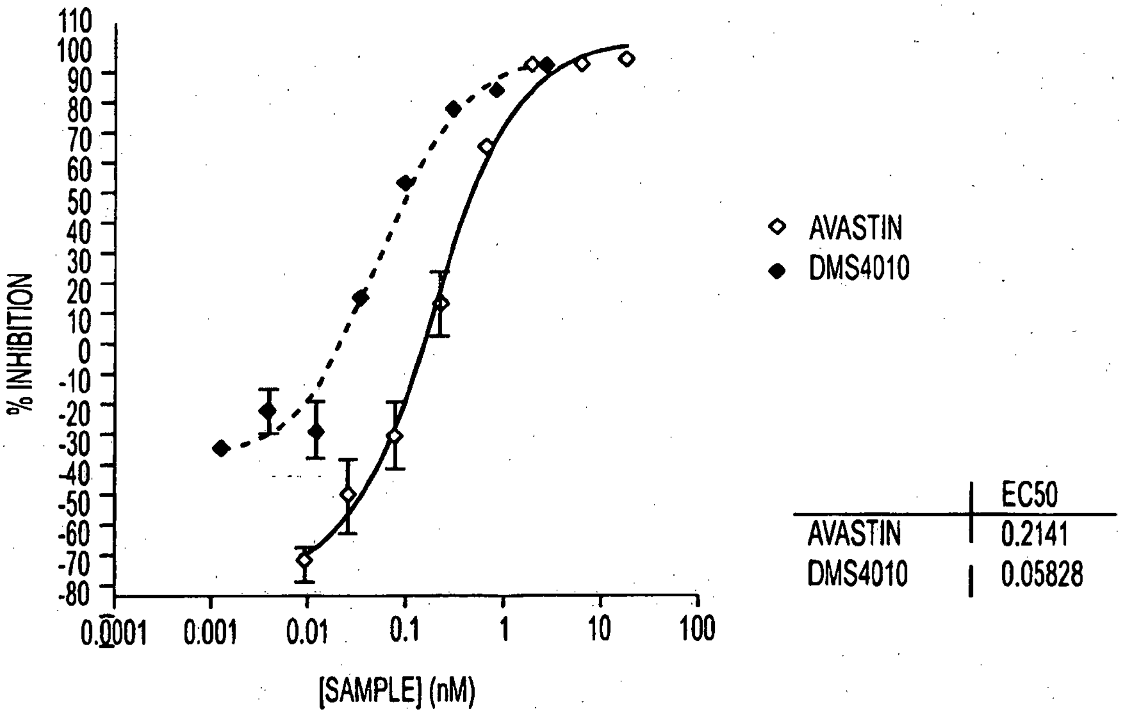


FIG. 131

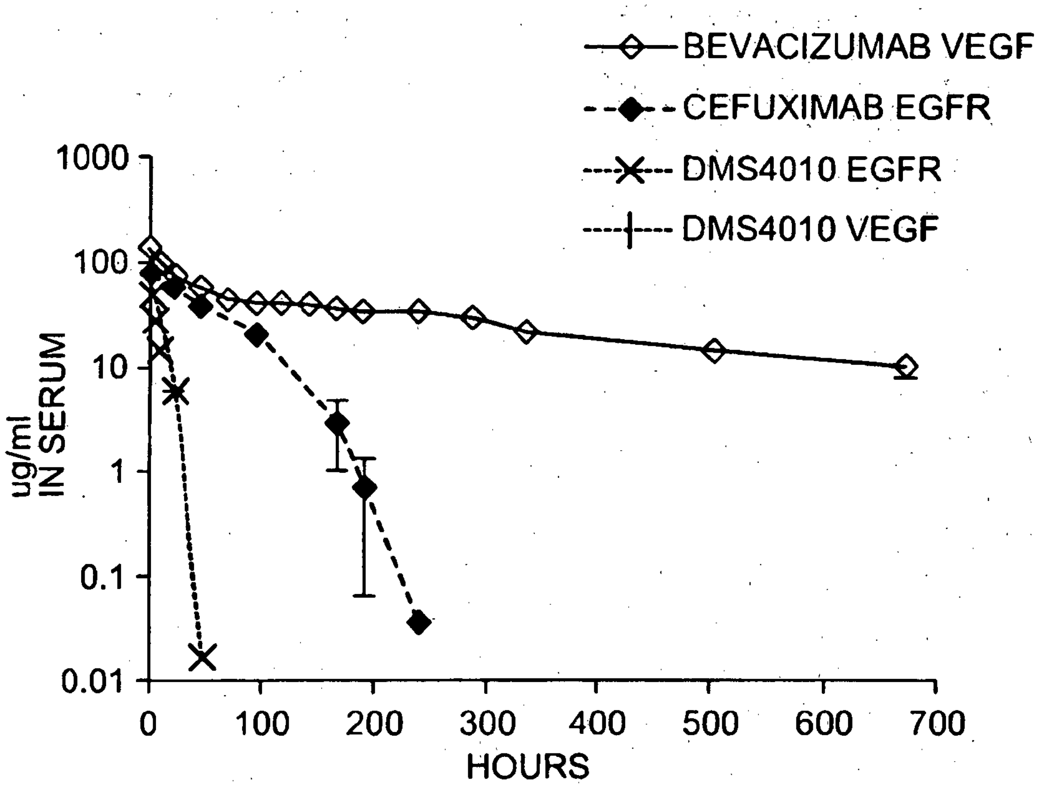


*FIG. 132*

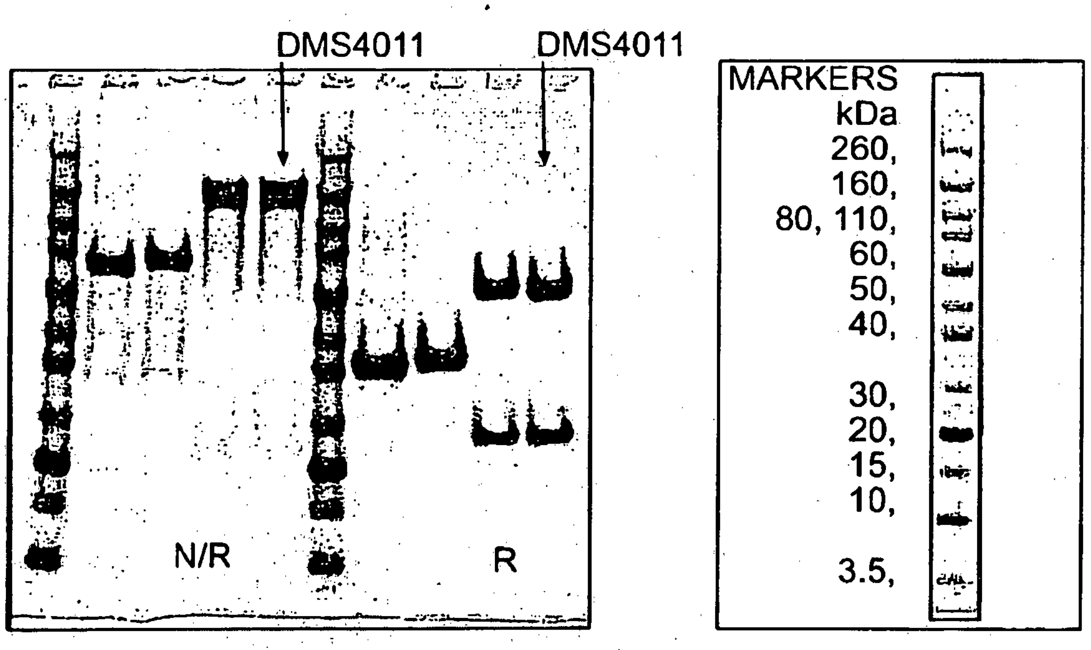


N/R=NON-REDUCED; R=REDUCED

*FIG. 133*

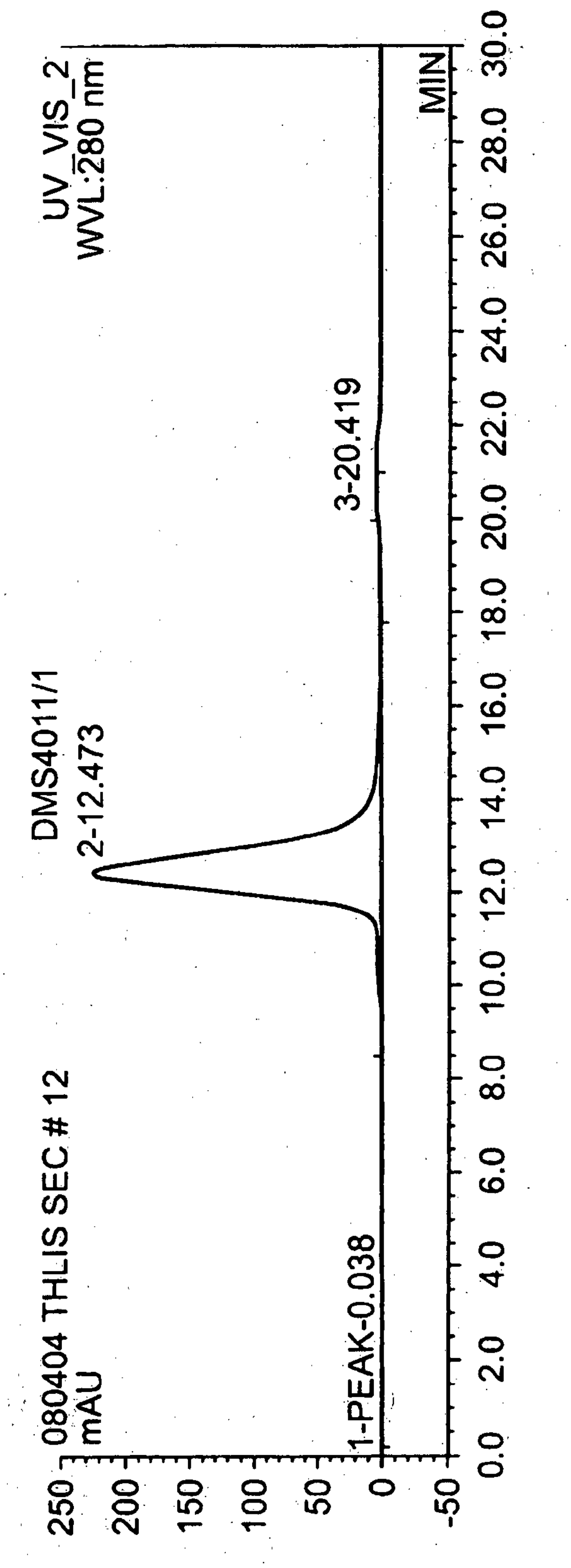
080404 THLIS SEC # 12
mAU
UV_VIS_2
WVL:280 nm
DMS4011/1
2-12.473
3-20.419
1-PEAK-0.038
MIN
250
200
150
100
50
0
-50
0.0
2.0
4.0
6.0
8.0
10.0
12.0
14.0
16.0
18.0
20.0
22.0
24.0
26.0
28.0
30.0

*FIG. 134*

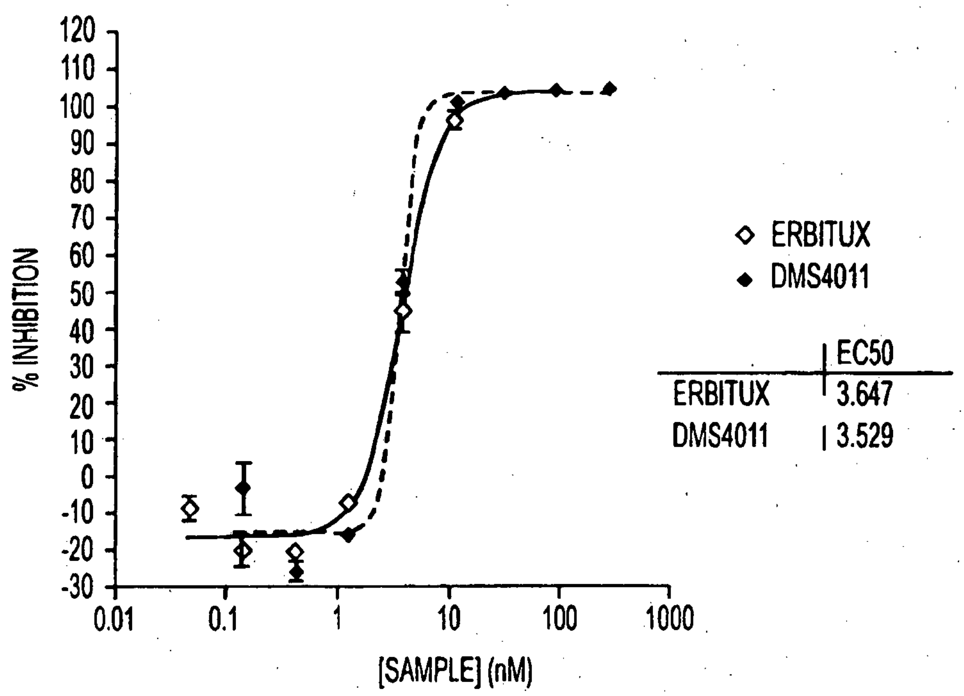


*FIG. 135*

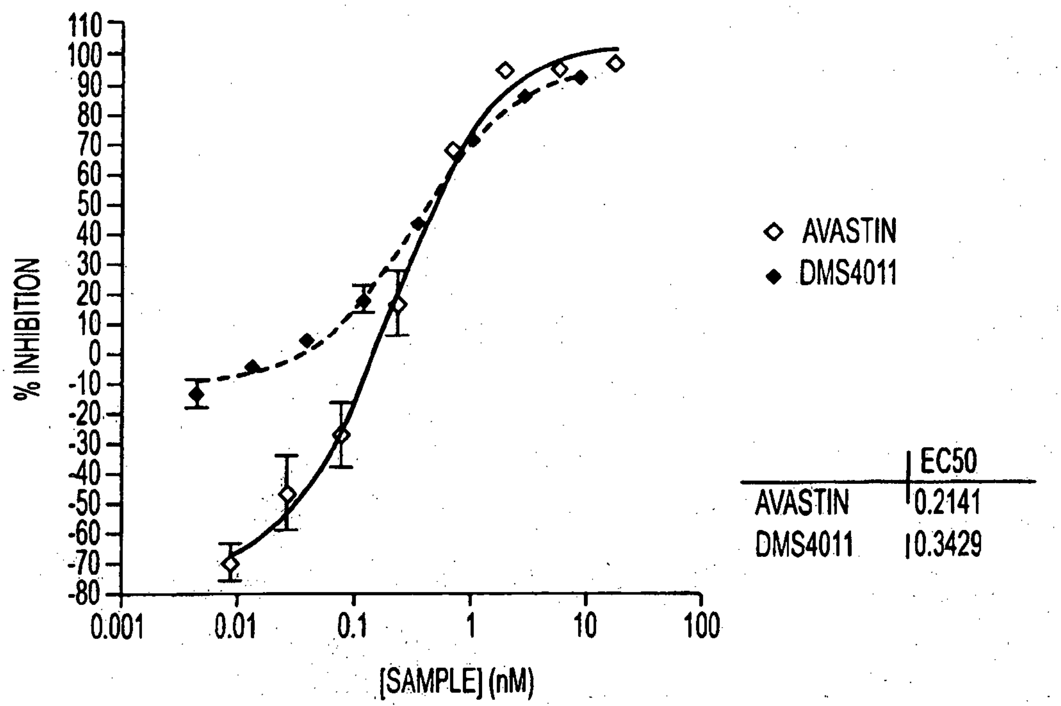


*FIG. 136*

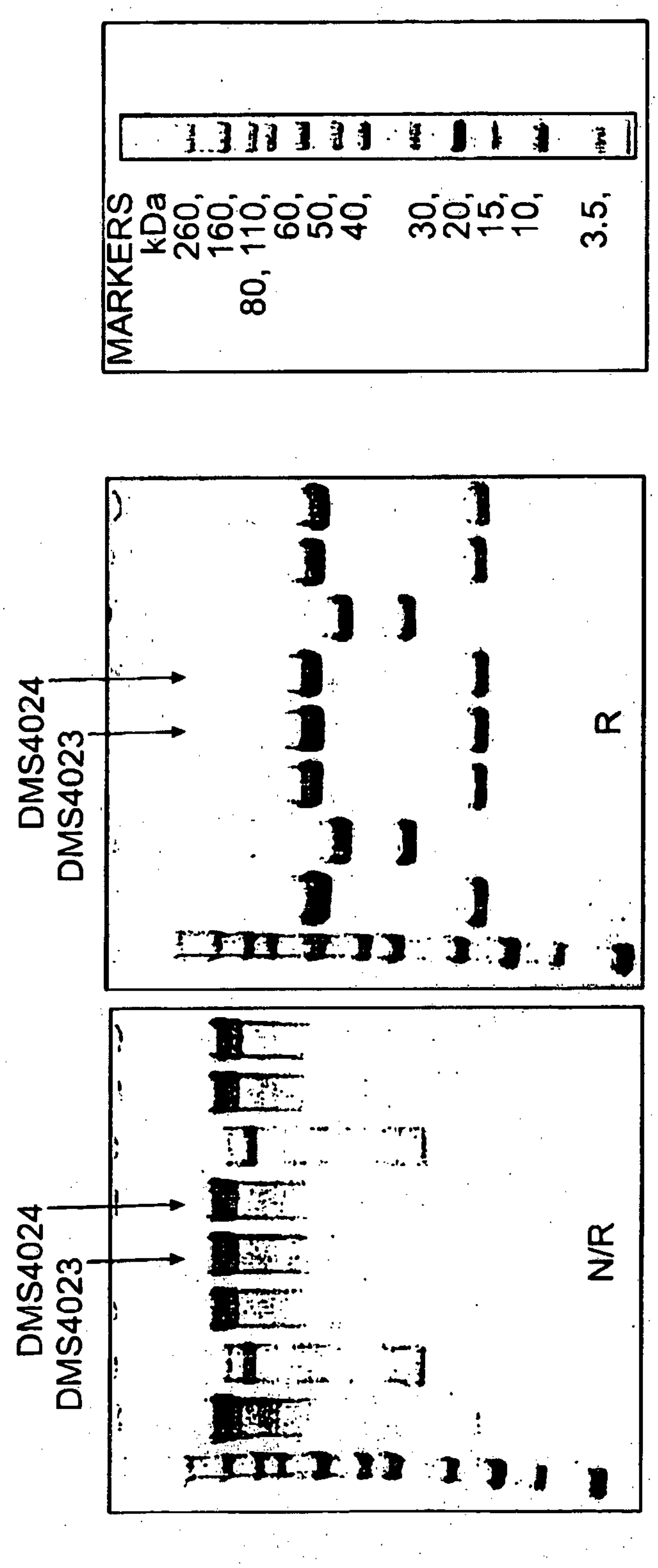


N/R=NON-REDUCED; R=REDUCED

*FIG. 137*

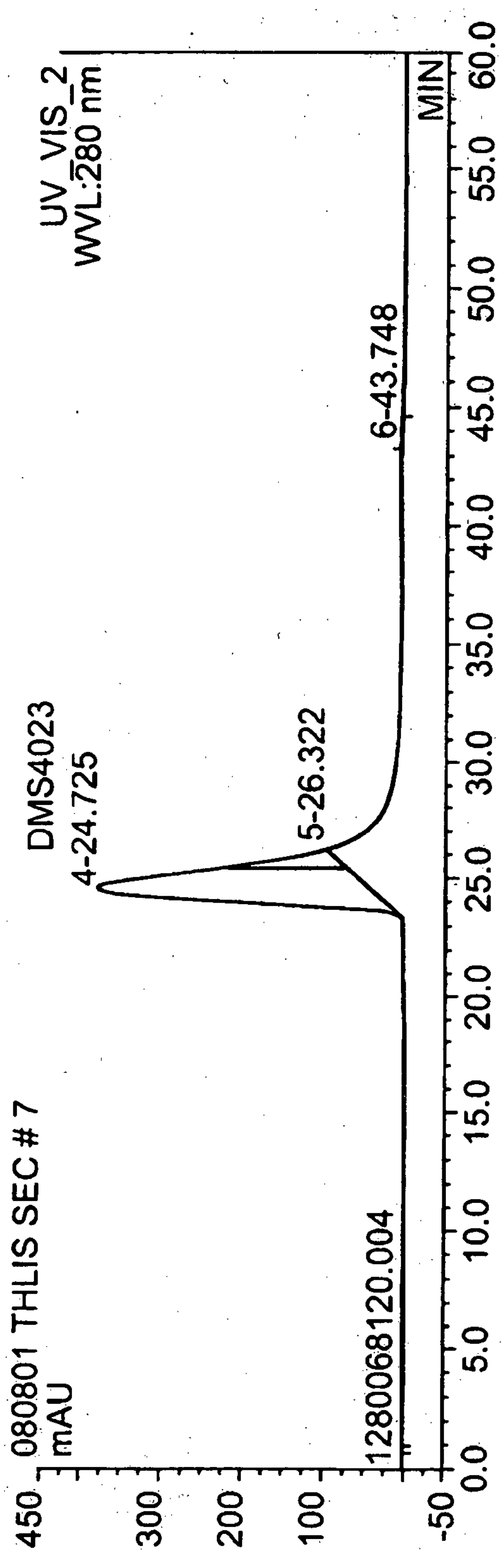
080801 THLIS SEC # 7
mAU
UV_VIS_2
WVL:280 nm
DMS4023
4-24.725
5-26.322
6-43.748
1280068120.004
MIN
450
300
200
100
-50
0.0
5.0
10.0
15.0
20.0
25.0
30.0
35.0
40.0
45.0
50.0
55.0
60.0

*FIG. 138*

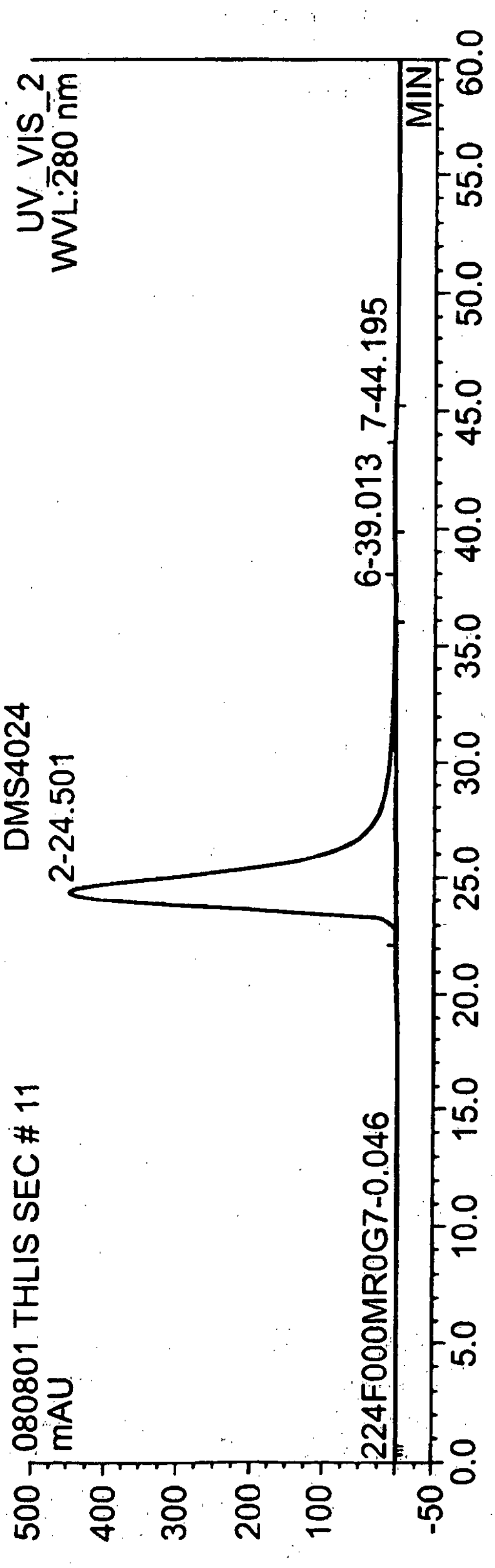
080801 THLIS SEC # 11
mAU
UV_VIS_2
WVL:280 nm
DMS4024
2-24.501
6-39.013
7-44.195
224F000MR0G7-0.046
MIN
500
400
300
200
100
-50
0.0
5.0
10.0
15.0
20.0
25.0
30.0
35.0
40.0
45.0
50.0
55.0
60.0

FIG. 139

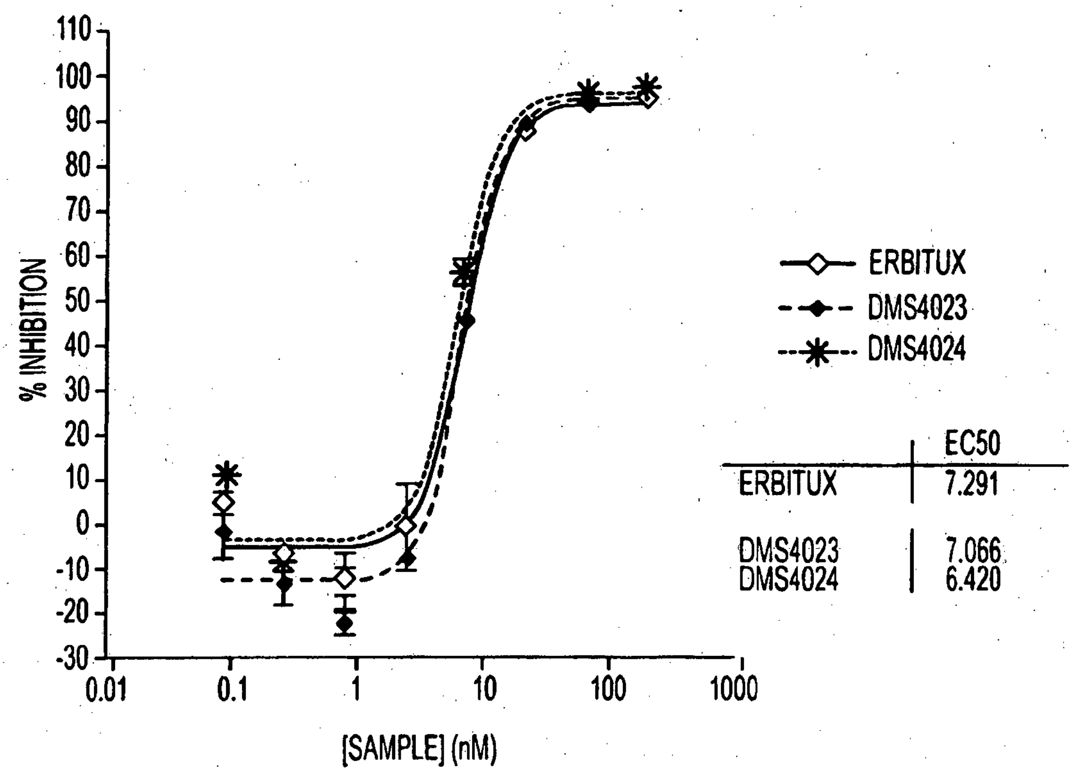


FIG. 140

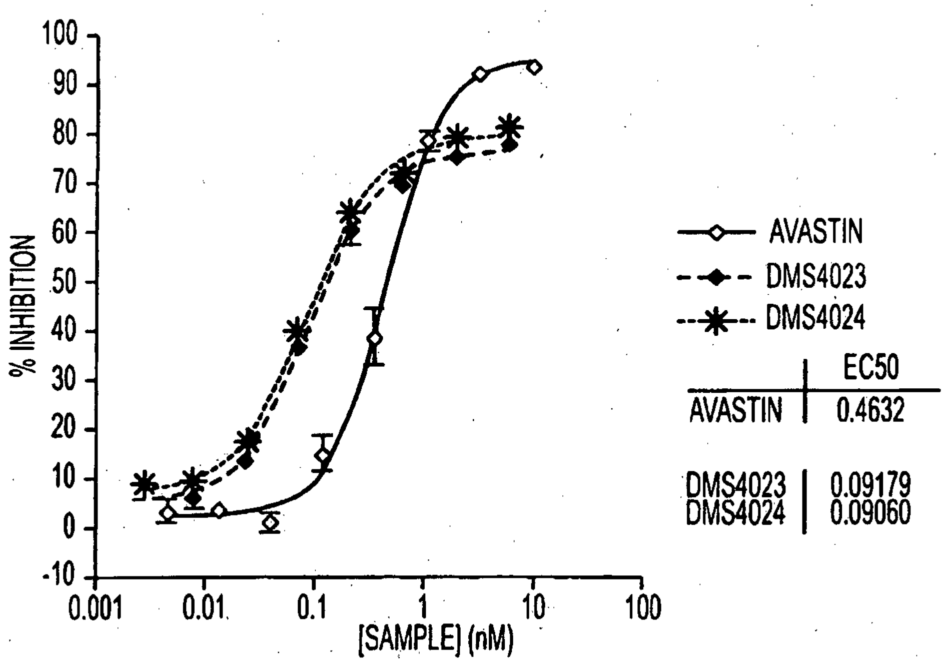


FIG. 141

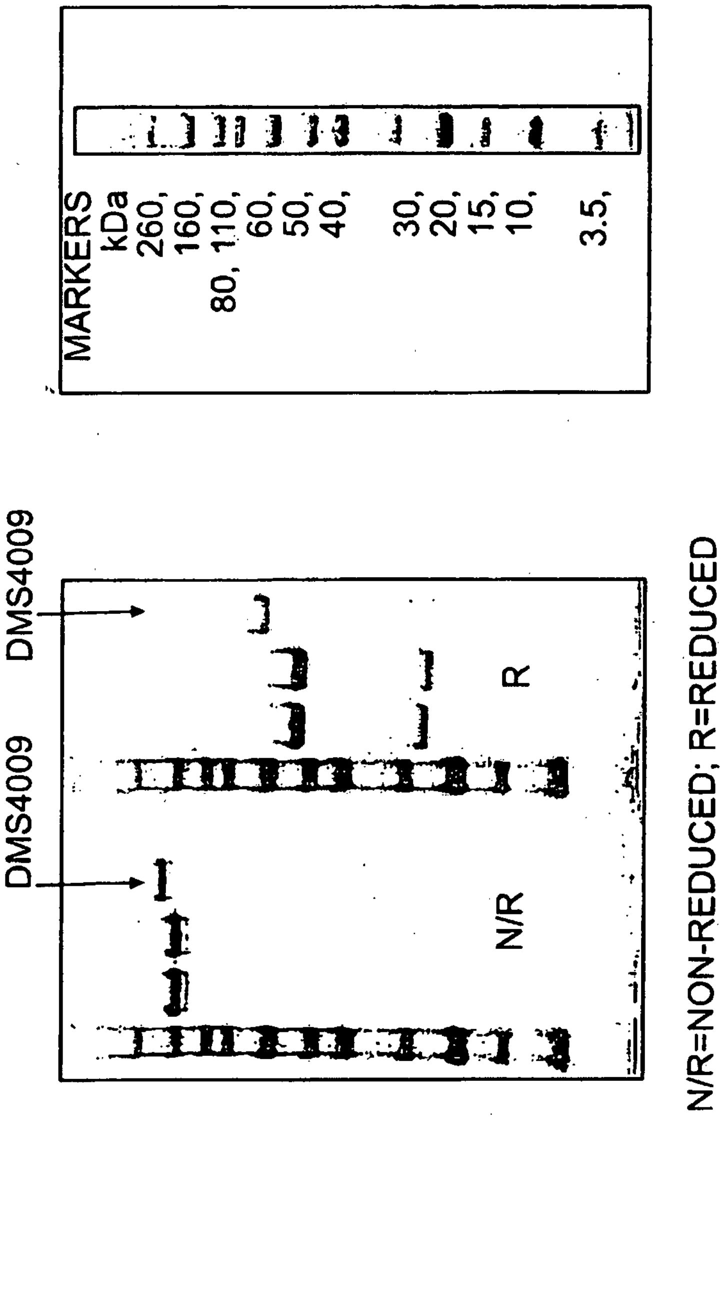
DMS4009
DMS4009
N/R
R
N/R=NON-REDUCED; R=REDUCED
MARKERS
kDa
260,
160,
80, 110,
60,
50,
40,
30,
20,
15,
10,
3.5,

*FIG. 142*

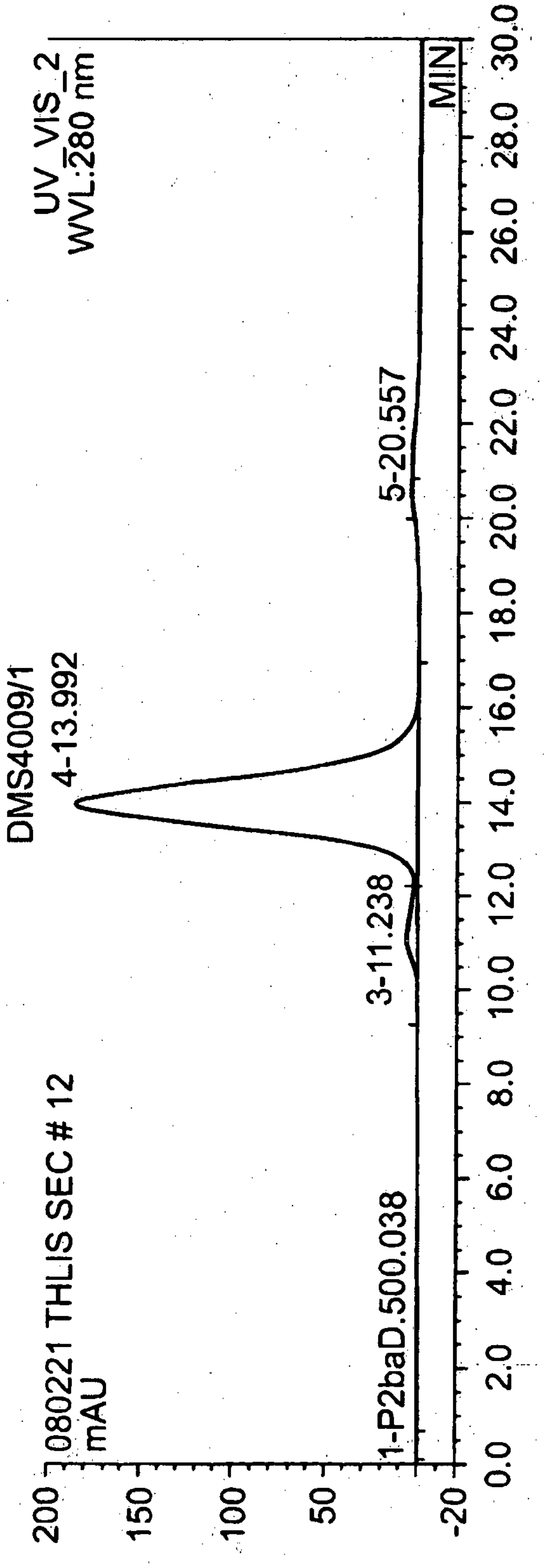
080221 THLIS SEC # 12
mAU
UV_VIS_2
WVL:280 nm
DMS4009/1
4-13.992
3-11.238
5-20.557
1-P2baD.500.038
MIN
200
150
100
50
-20
0.0
2.0
4.0
6.0
8.0
10.0
12.0
14.0
16.0
18.0
20.0
22.0
24.0
26.0
28.0
30.0

FIG. 143

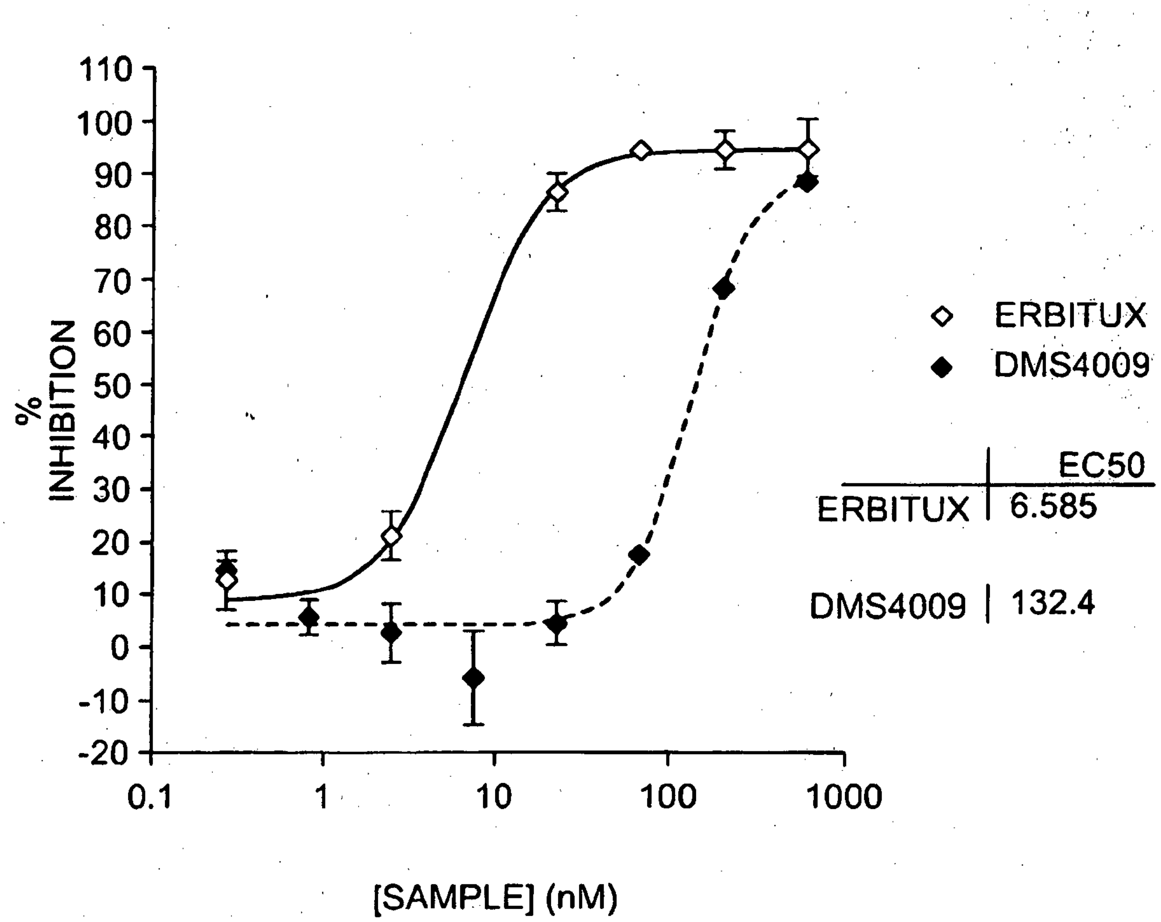
110
100
90
80
70
60
50
40
30
20
10
0
-10
-20
% INHIBITION
0.1
1
10
100
1000
[SAMPLE] (nM)
ERBITUX
DMS4009
EC50
ERBITUX 6.585
DMS4009 132.4

*FIG. 144*

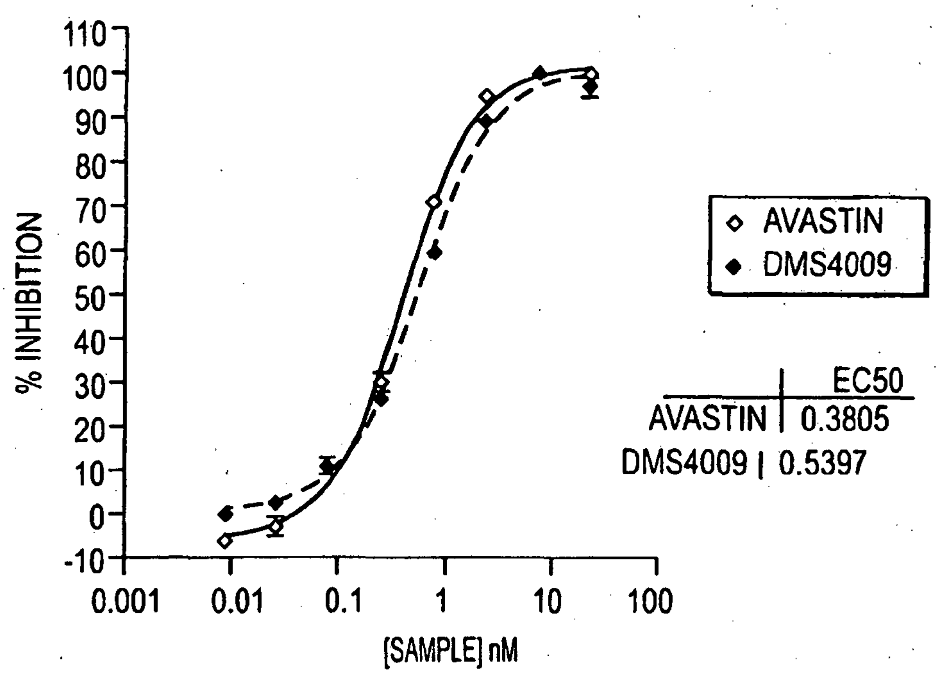


*FIG. 145*

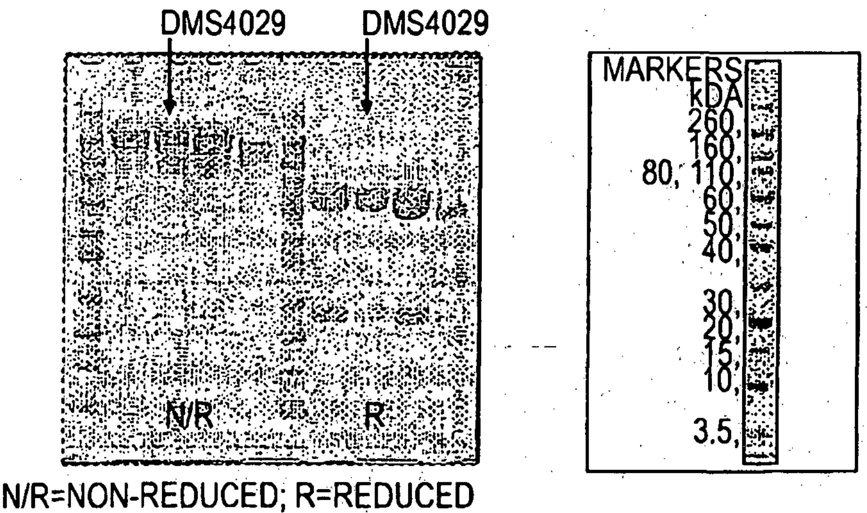


N/R=NON-REDUCED; R=REDUCED

*FIG. 146*

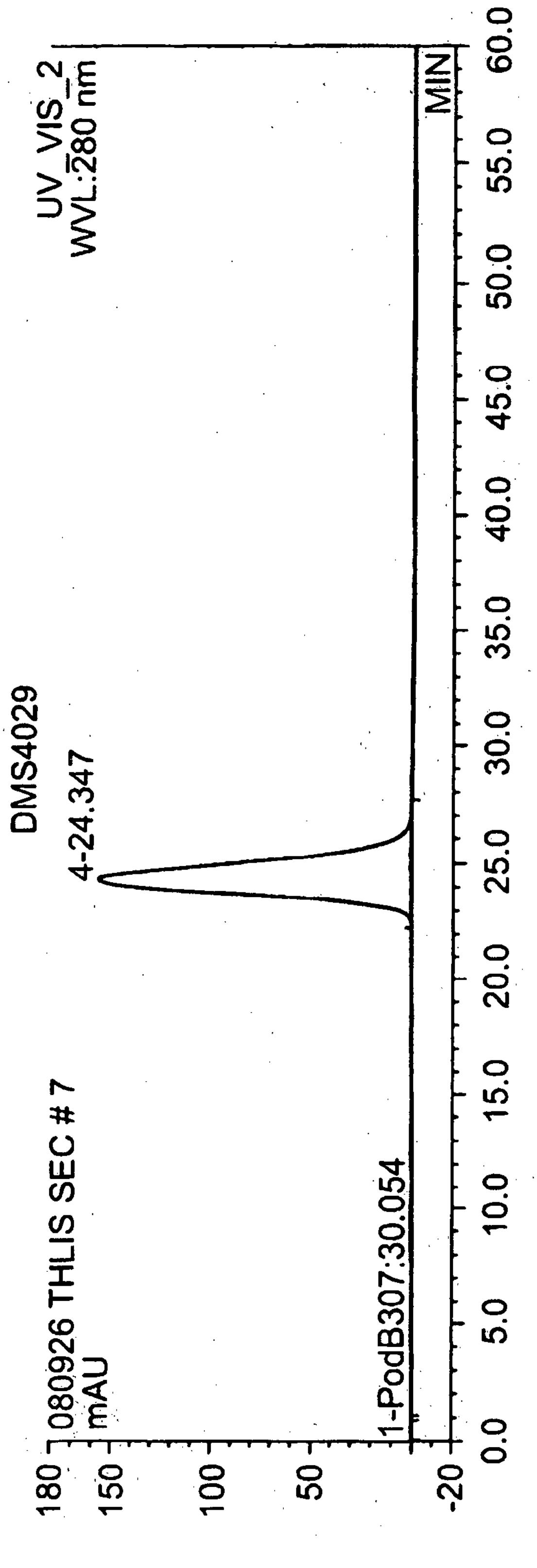
080926 THLIS SEC # 7
mAU
UV_VIS_2
WVL:280 nm
DMS4029
4-24.347
1-PodB307:30.054
180
150
100
50
-20
0.0
5.0
10.0
15.0
20.0
25.0
30.0
35.0
40.0
45.0
50.0
55.0
60.0
MIN

*FIG. 147*

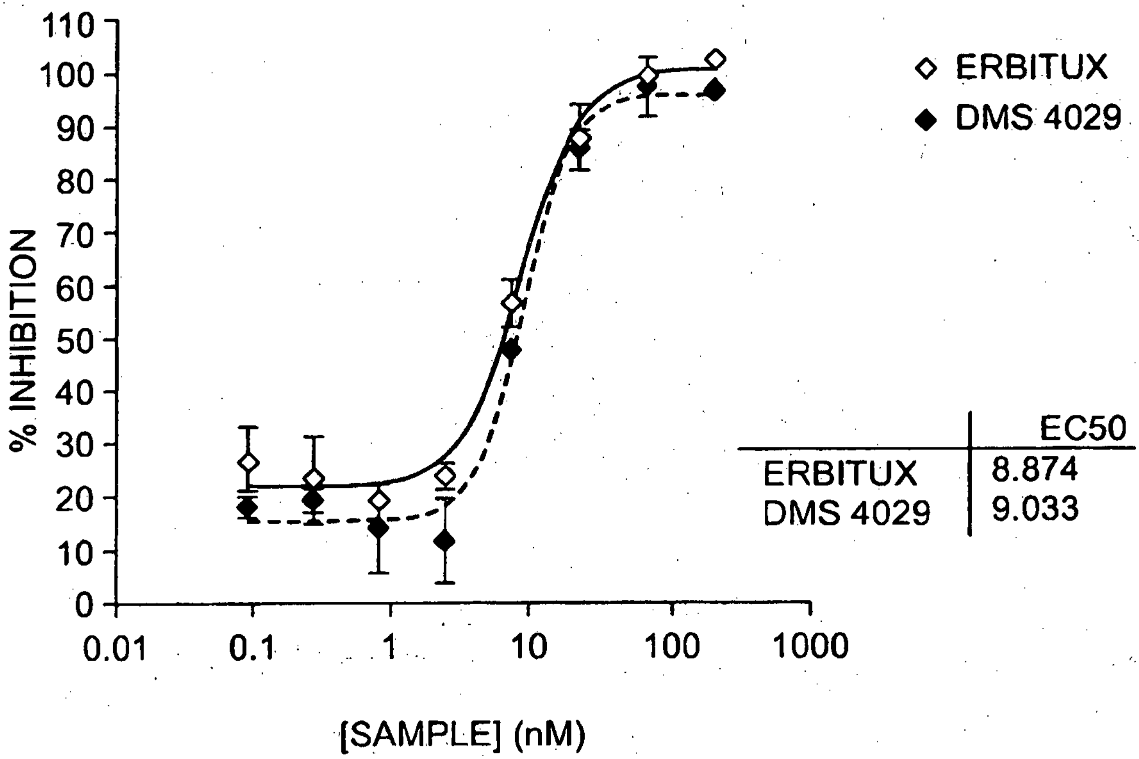
110
100
90
80
70
60
50
40
30
20
10
0
% INHIBITION
0.01
0.1
1
10
100
1000
[SAMPLE] (nM)
ERBITUX
DMS 4029
EC50
ERBITUX 8.874
DMS 4029 9.033

FIG. 148

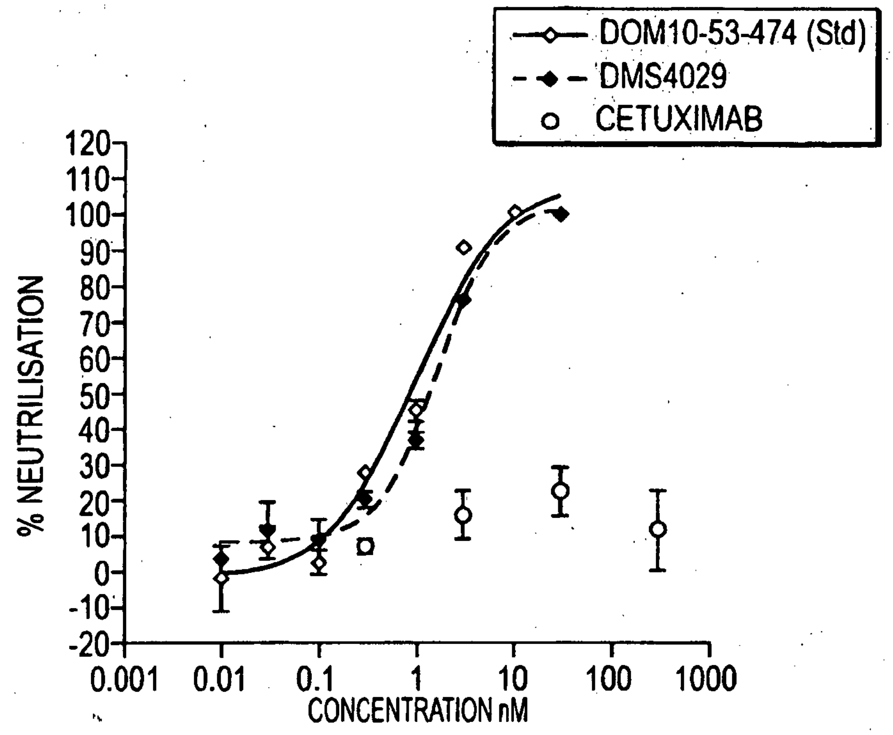


| | DOM10-53-474 (Std) | DMS4029 |
|---|---|---|
| EC50 | 0.9960 | 1.654 |

FIG. 149

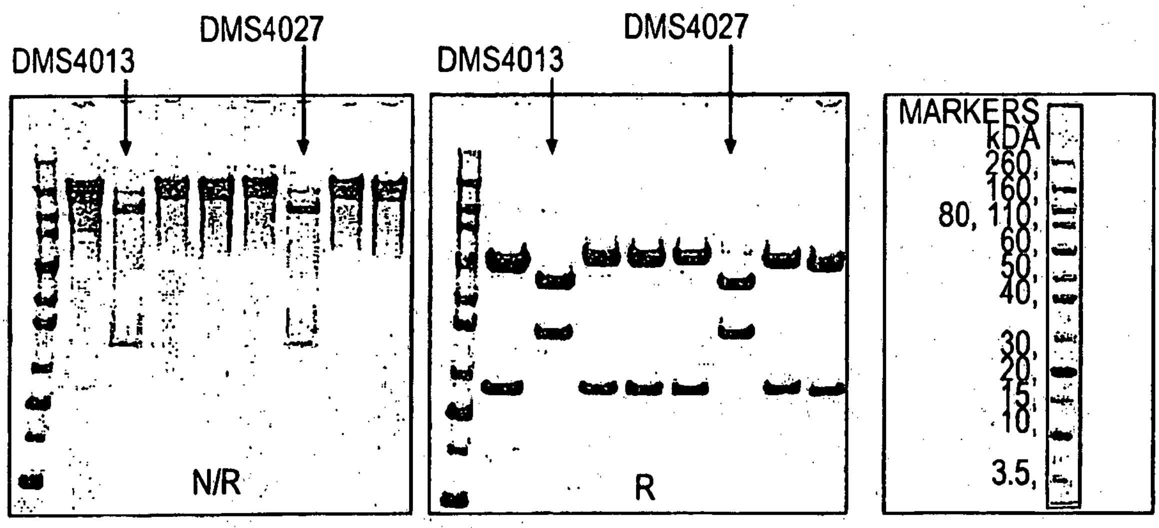


N/R=NON-REDUCED; R=REDUCED

FIG. 150

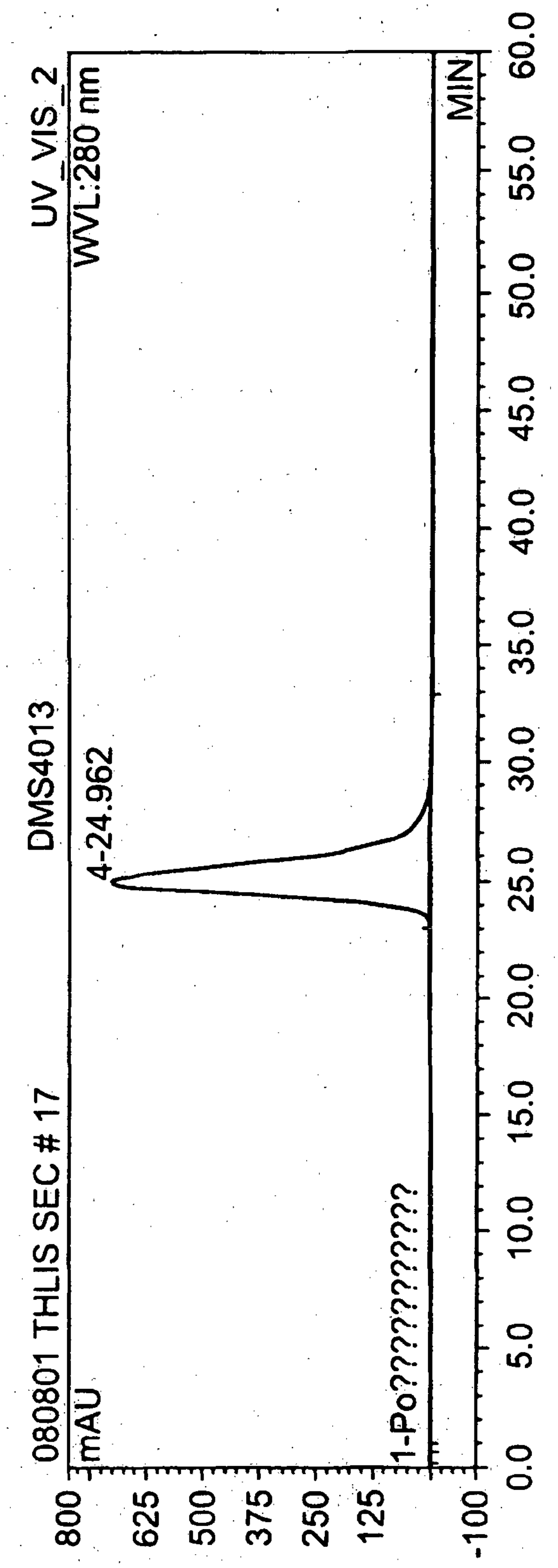
080801 THLIS SEC # 17
DMS4013
UV_VIS_2
WVL:280 nm
mAU
4-24.962
1-Po??????????????
MIN
800
625
500
375
250
125
-100
0.0
5.0
10.0
15.0
20.0
25.0
30.0
35.0
40.0
45.0
50.0
55.0
60.0

*FIG. 151*

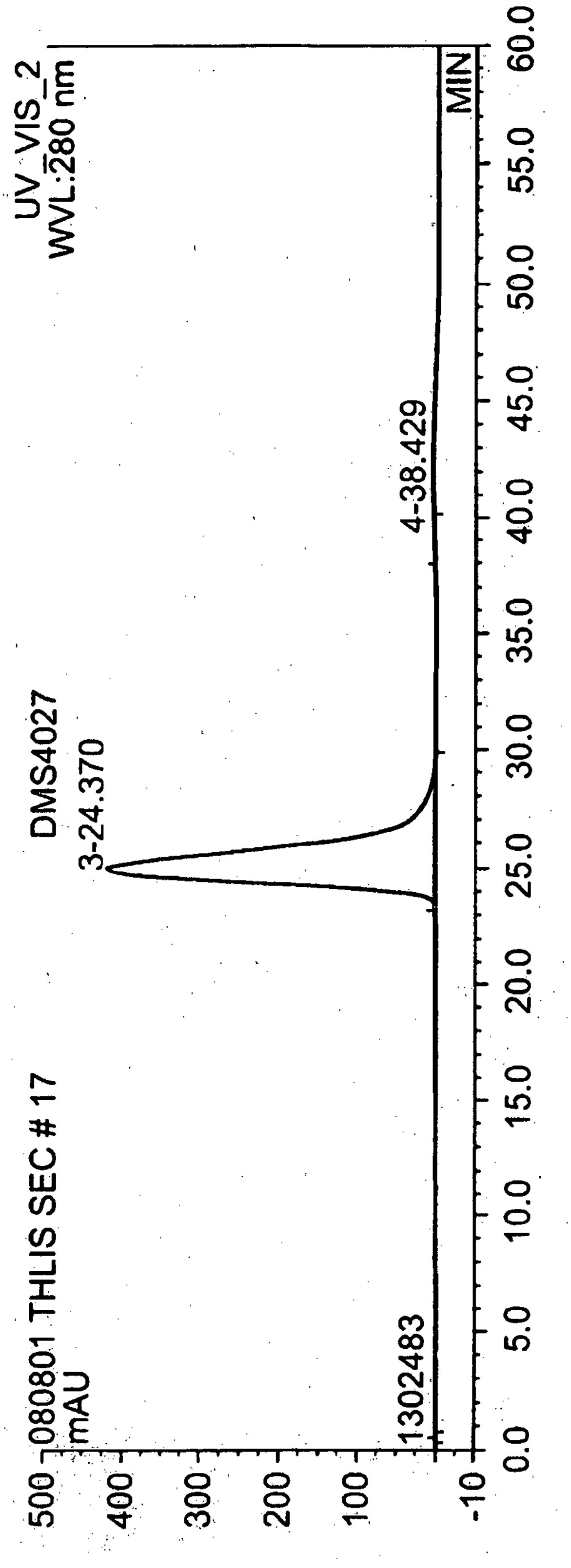
080801 THLIS SEC # 17
mAU
UV_VIS_2
WVL:280 nm
DMS4027
3-24.370
4-38.429
1302483
MIN
500
400
300
200
100
-10
0.0
5.0
10.0
15.0
20.0
25.0
30.0
35.0
40.0
45.0
50.0
55.0
60.0

FIG. 152

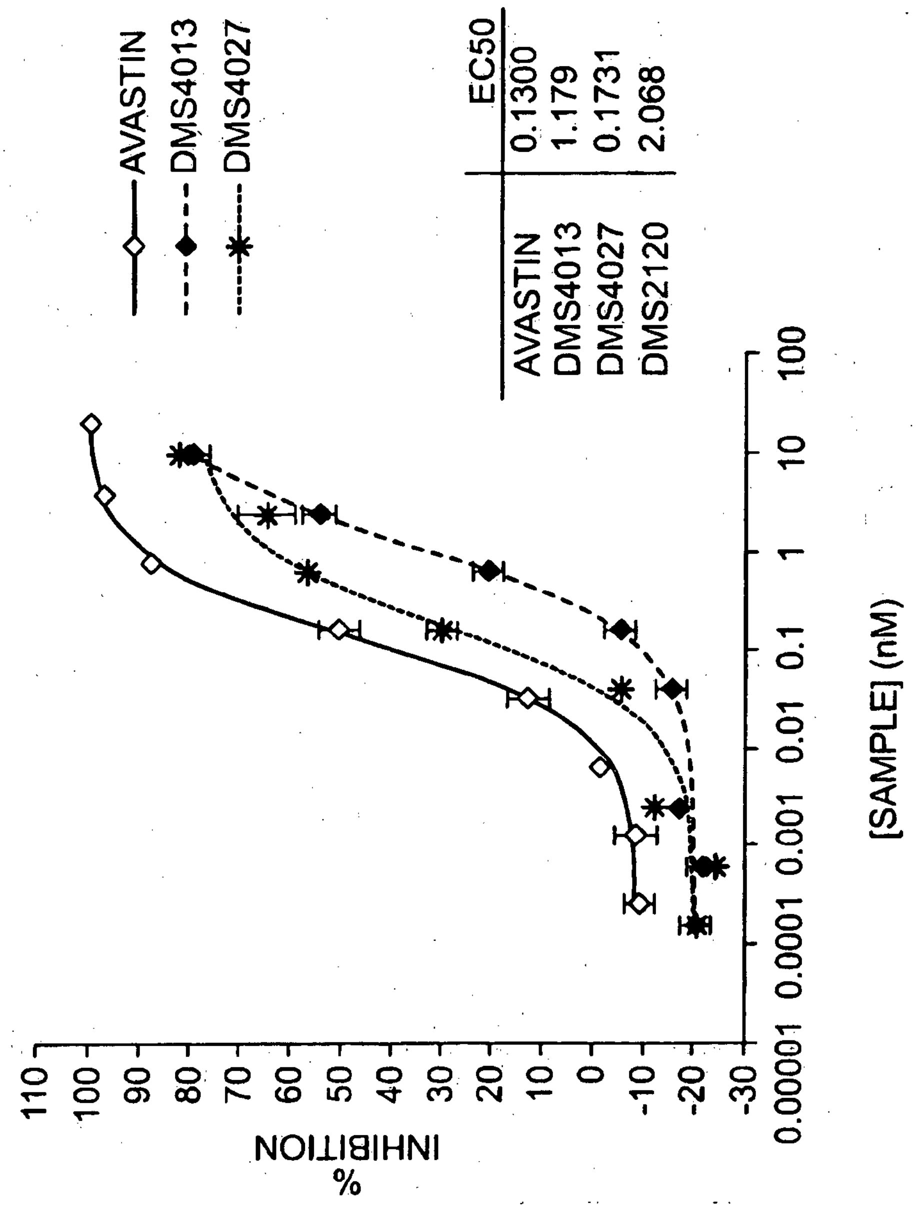
AVASTIN
DMS4013
DMS4027
EC50
AVASTIN 0.1300
DMS4013 1.179
DMS4027 0.1731
DMS2120 2.068
% INHIBITION
110
100
90
80
70
60
50
40
30
20
10
0
-10
-20
-30
0.00001
0.0001
0.001
0.01
0.1
1
10
100
[SAMPLE] (nM)

FIG. 153

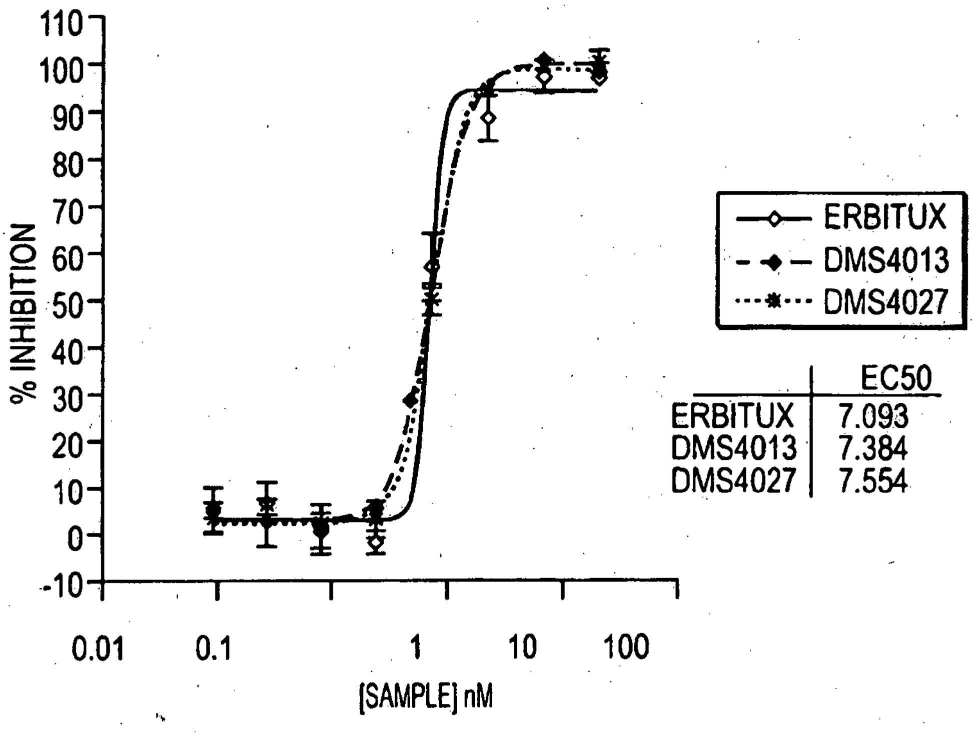


FIG. 154

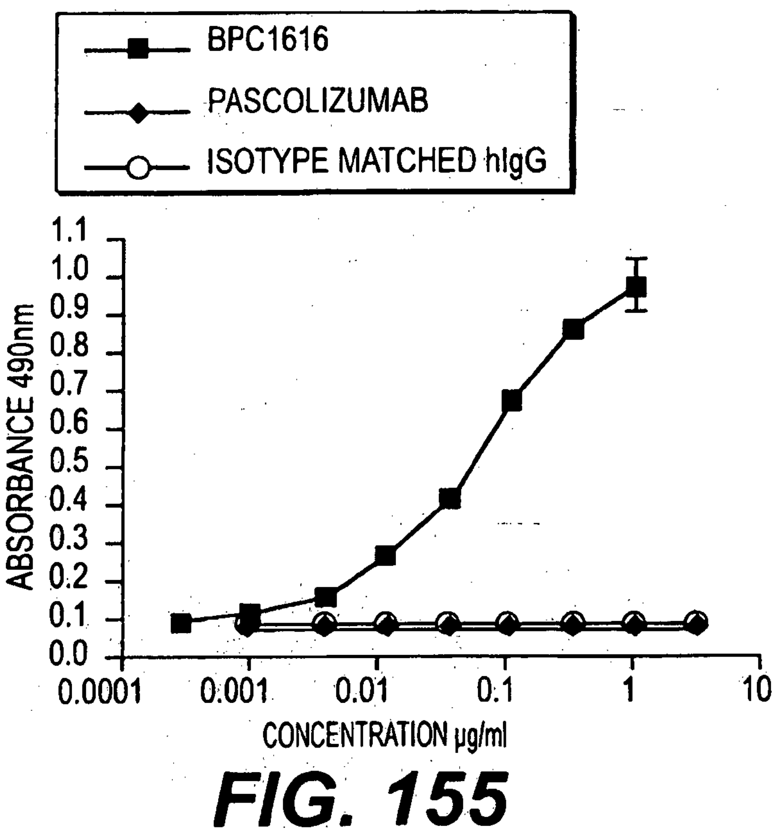


FIG. 155

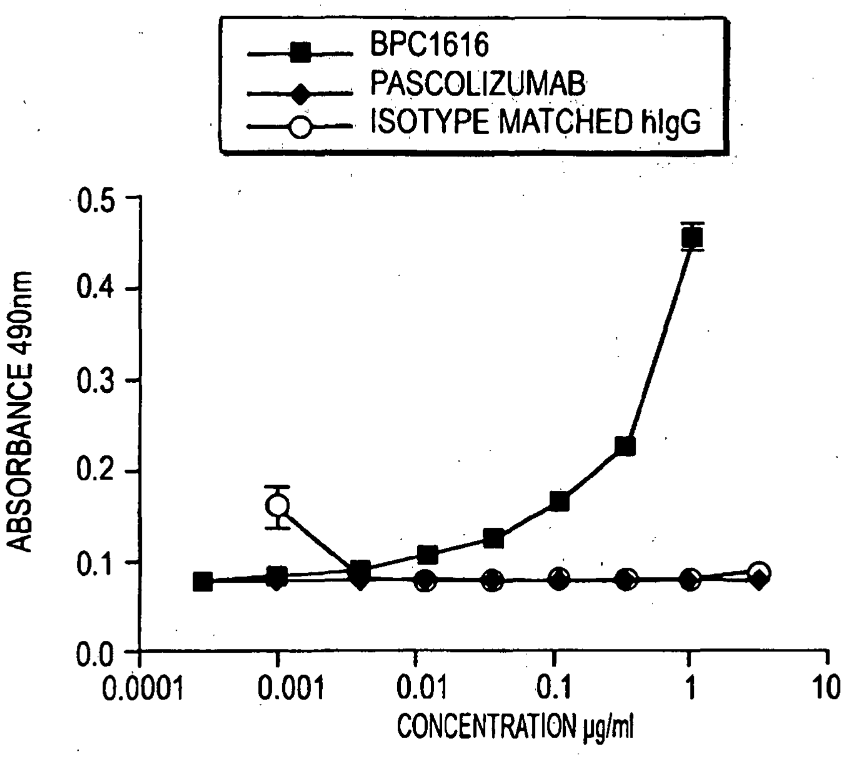


FIG. 156

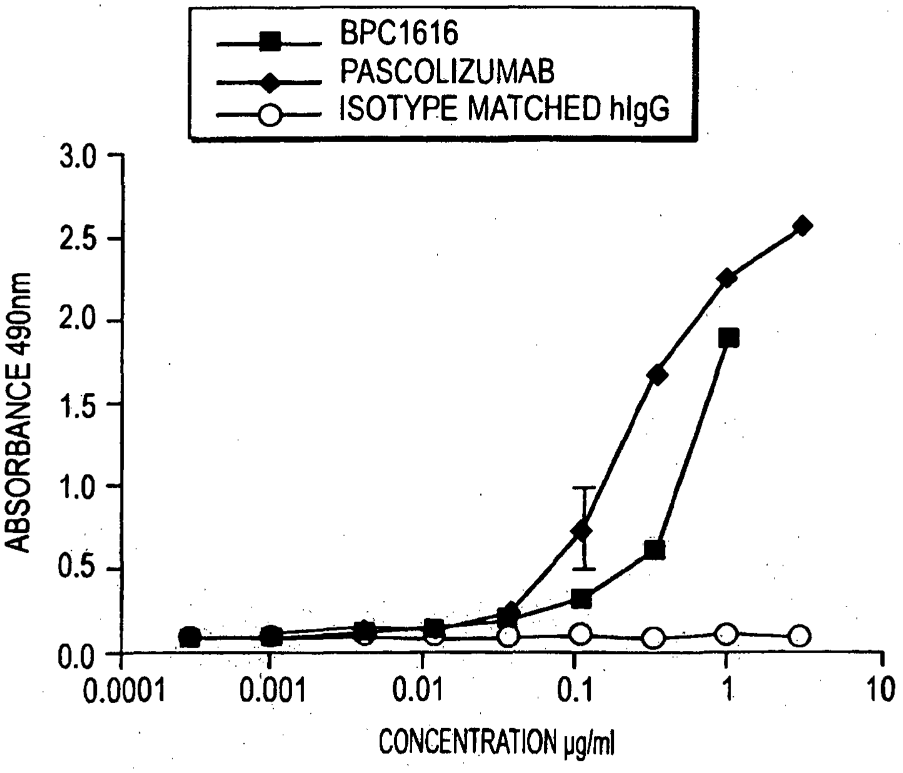


FIG. 157

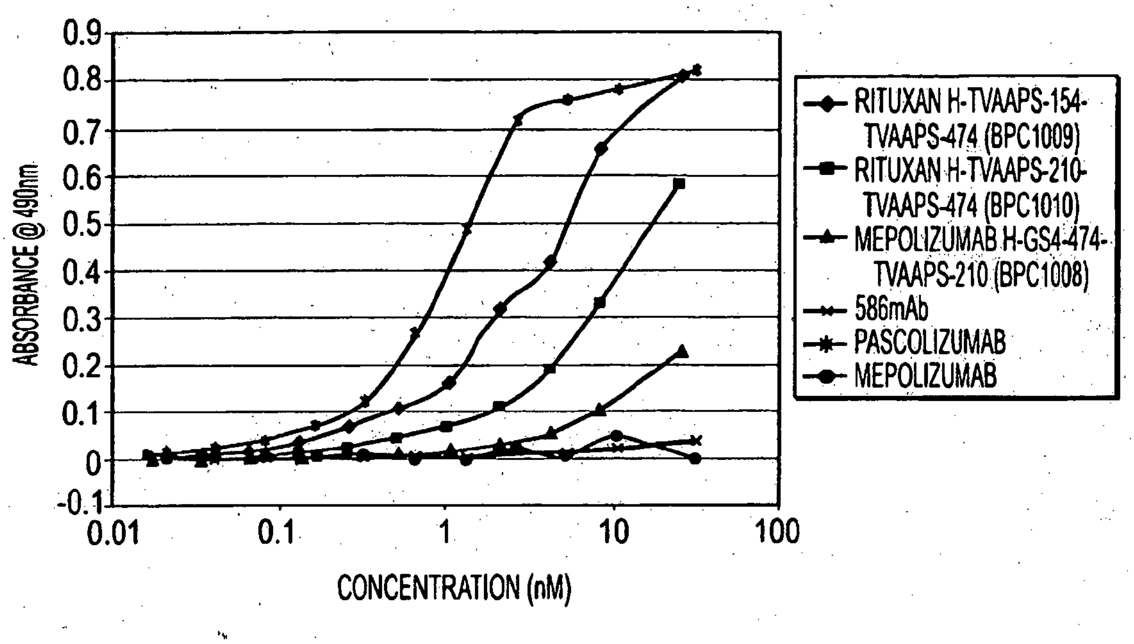


*FIG. 158*

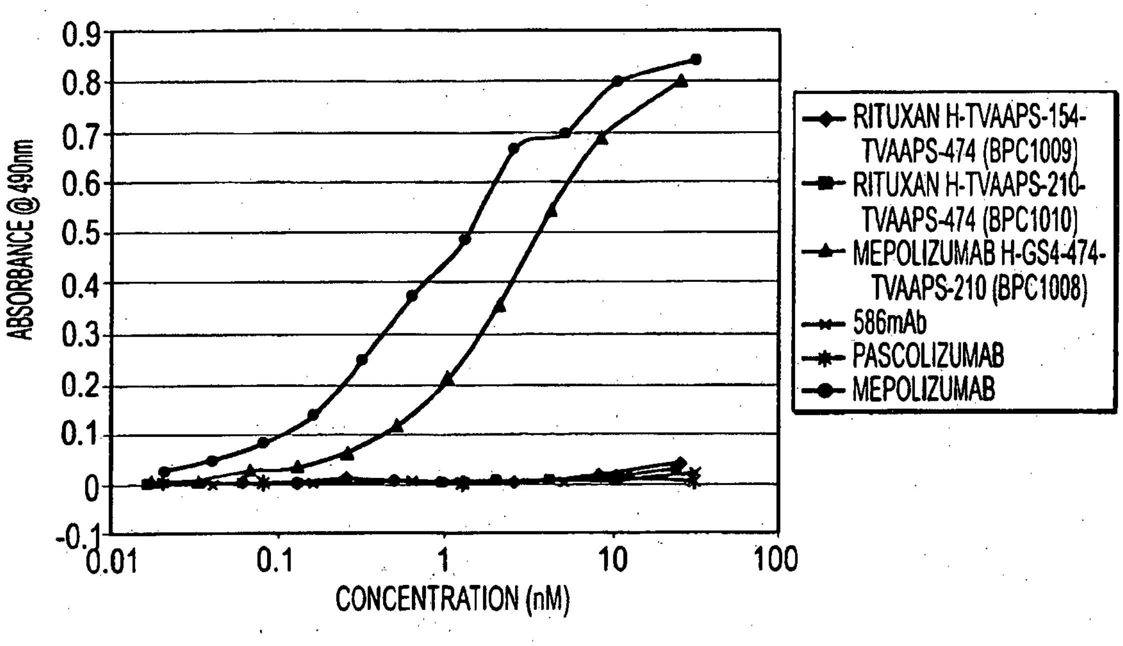


*FIG. 159*

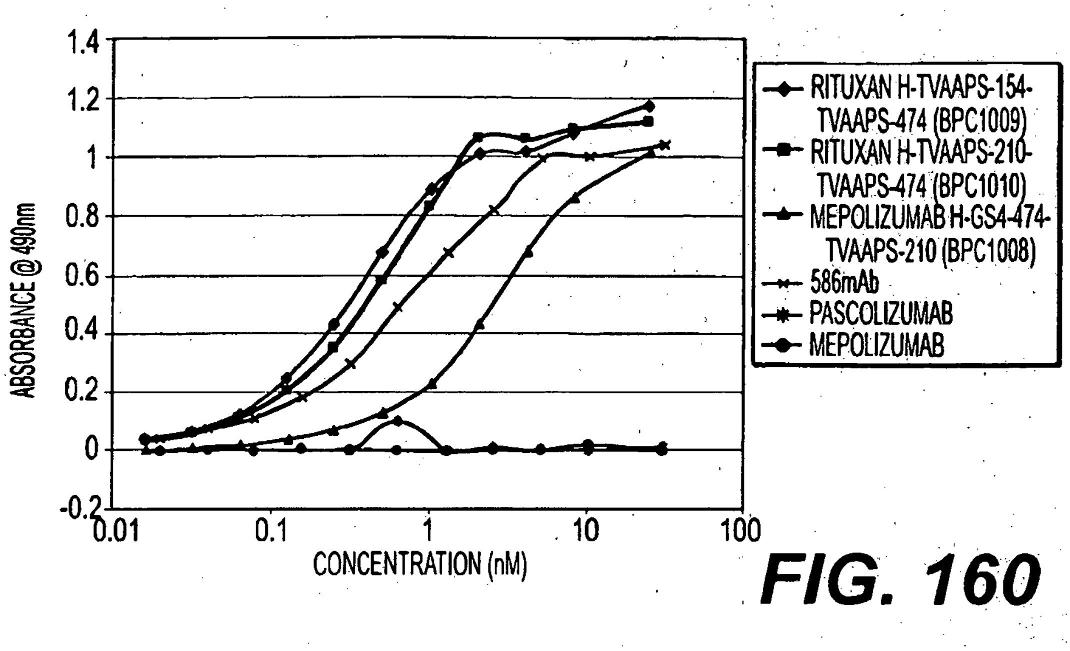


*FIG. 160*

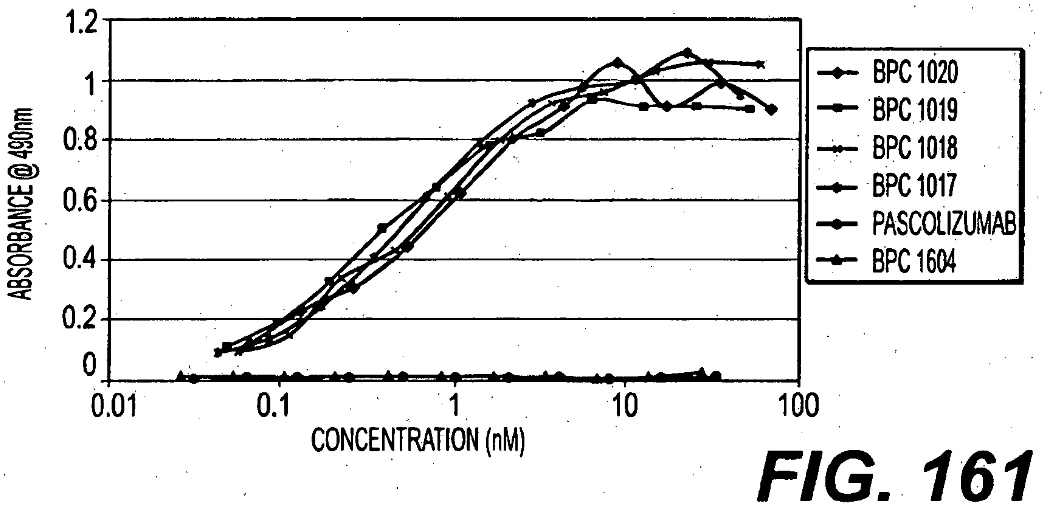


*FIG. 161*

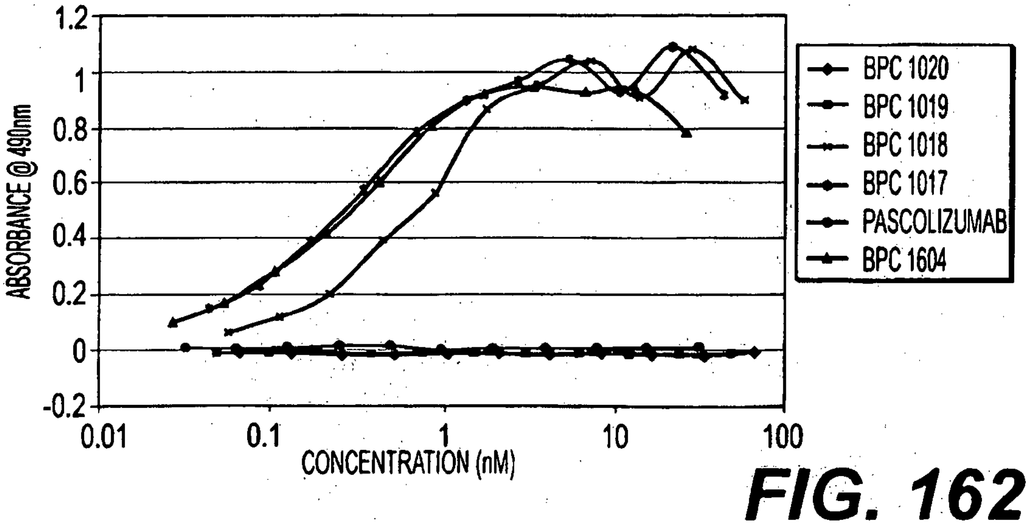


*FIG. 162*

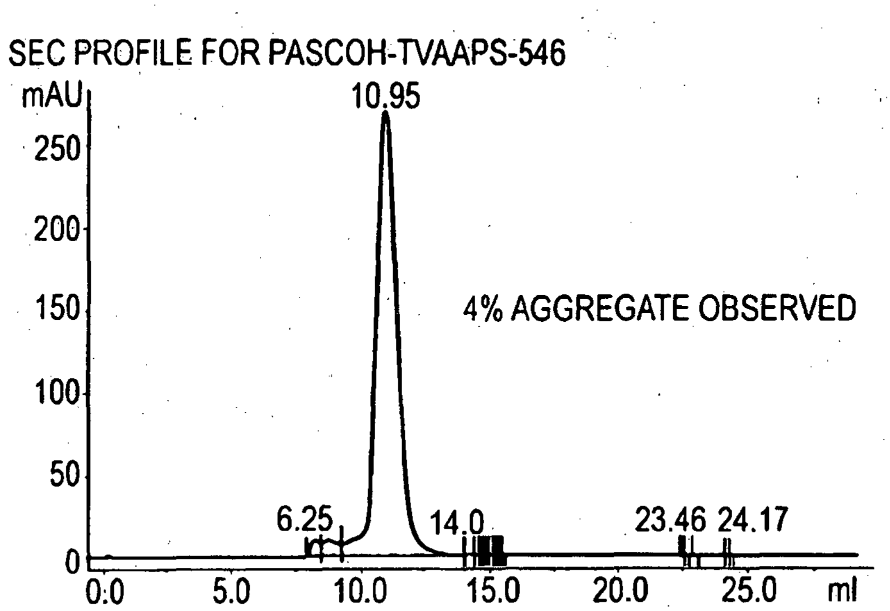


FIG. 163

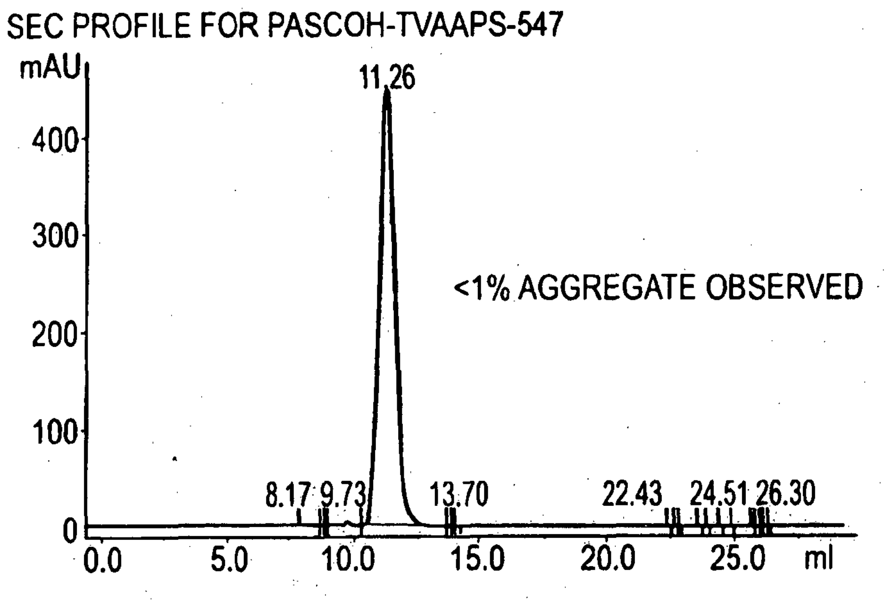


FIG. 164

SDS PAGE FOR PASCOH-TVAAPS-546 [A= NON-REDUCING CONDITIONS, B = REDUCING CONDITIONS]

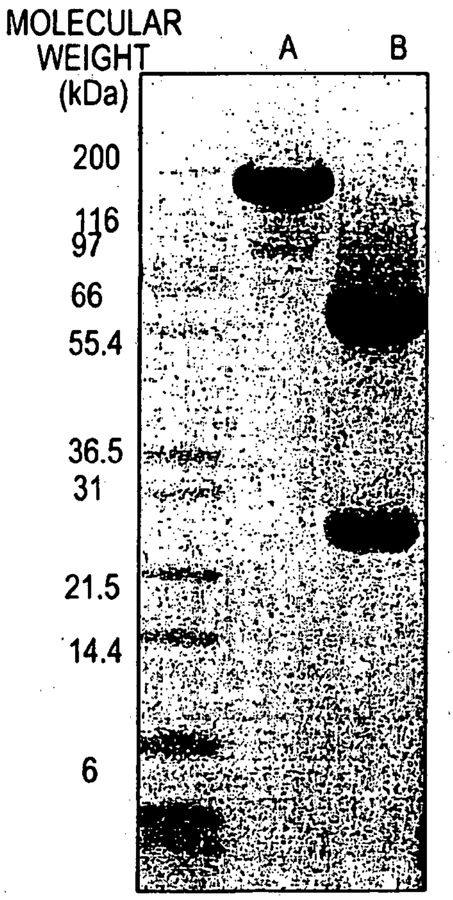


FIG. 165

SDS PAGE FRO PASCOH-TVAAPS-567 [A= NON-REDUCING CONDITIONS, B = REDUCING CONDITIONS]

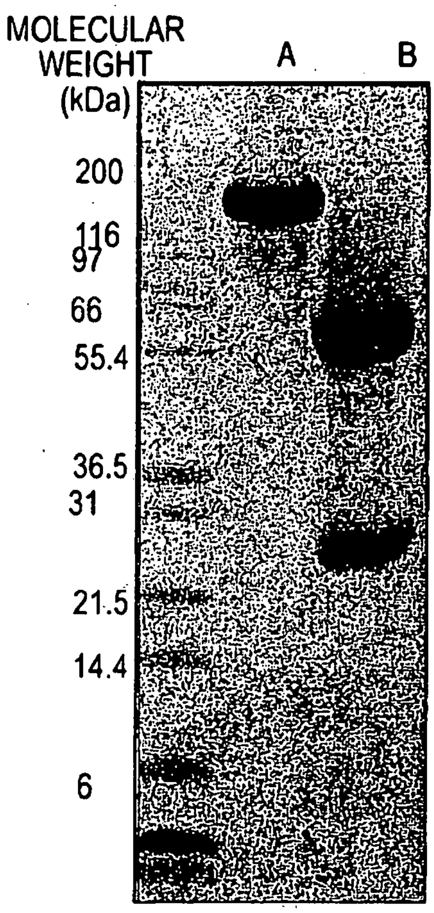


FIG. 166

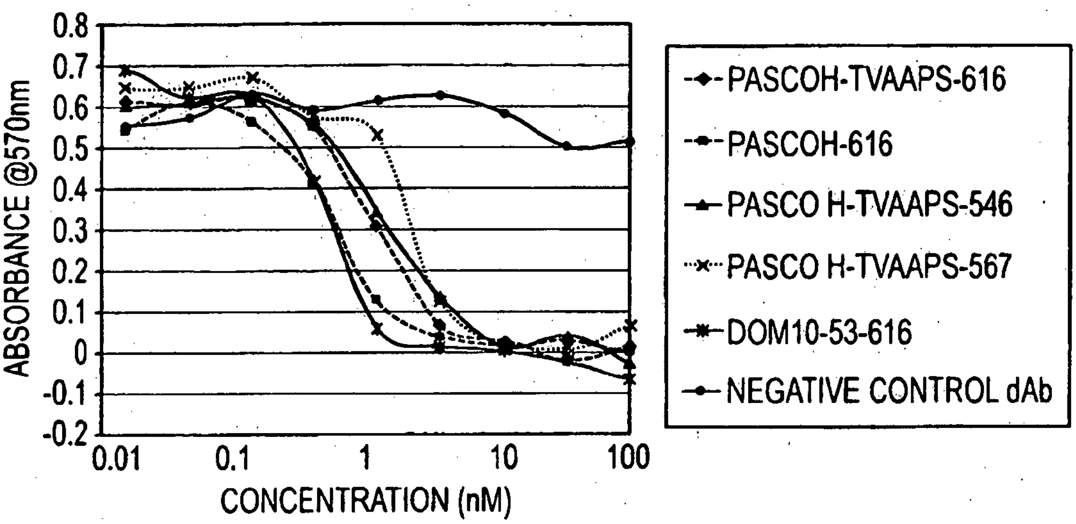


*FIG. 167*

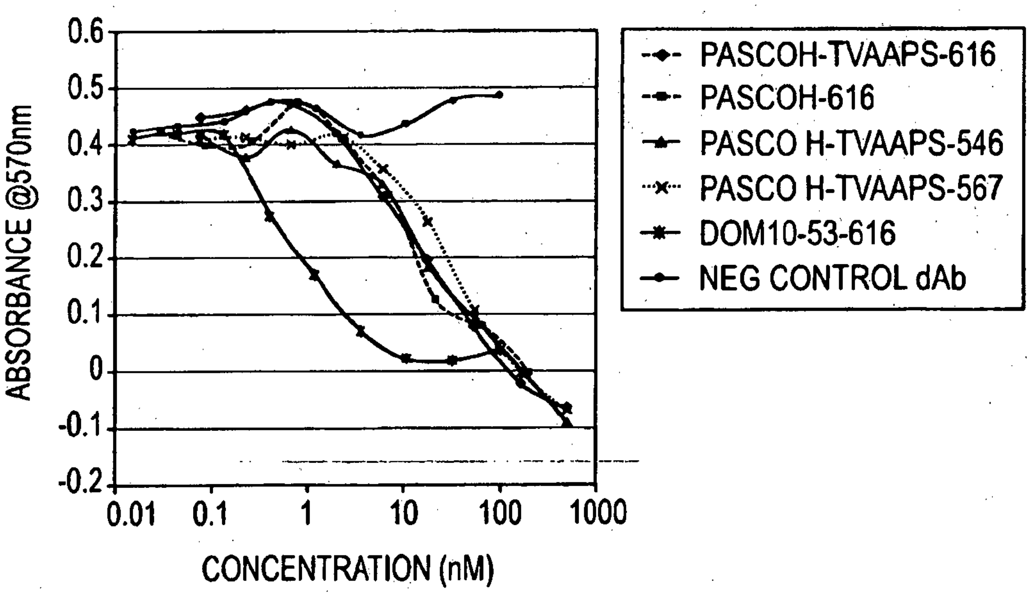


*FIG. 168*

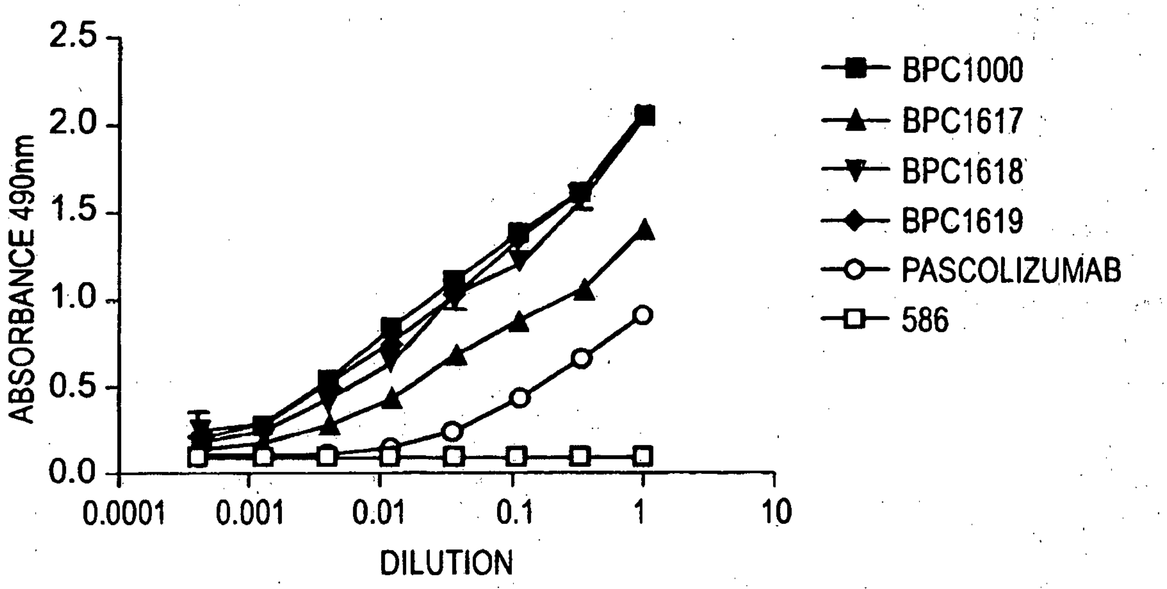


*FIG. 169*

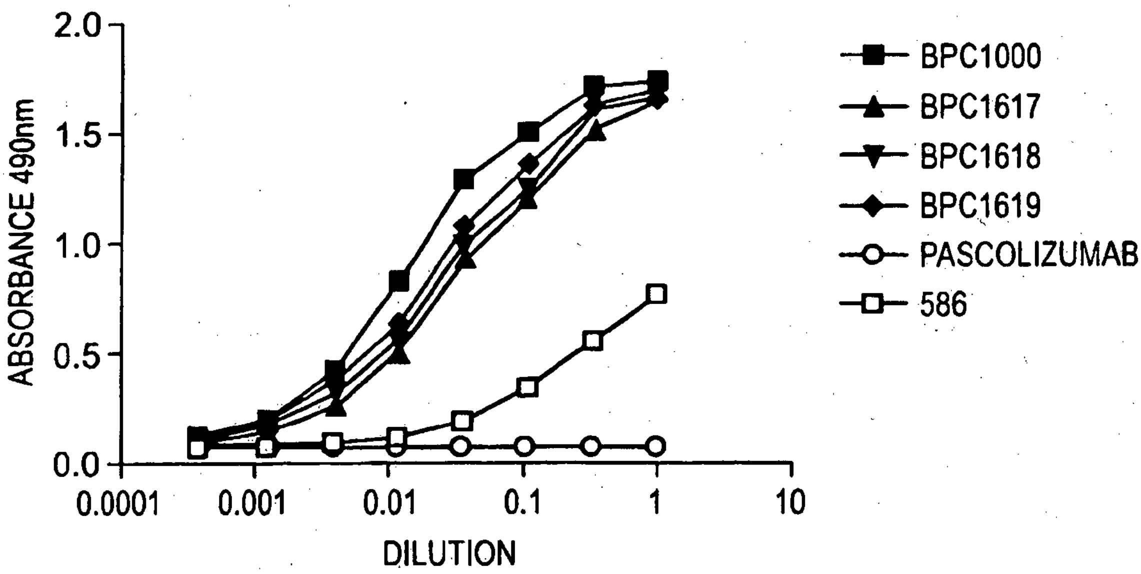


*FIG. 170*

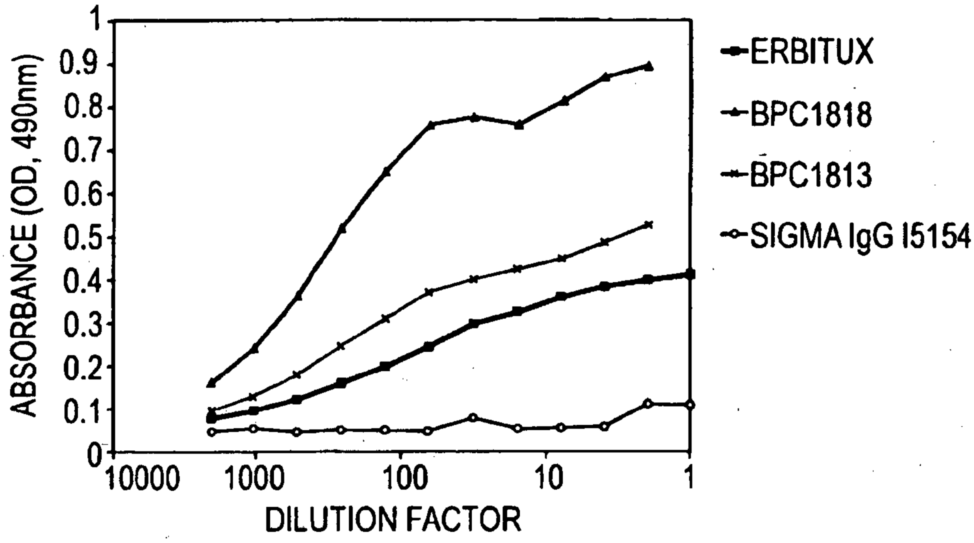


*FIG. 171*

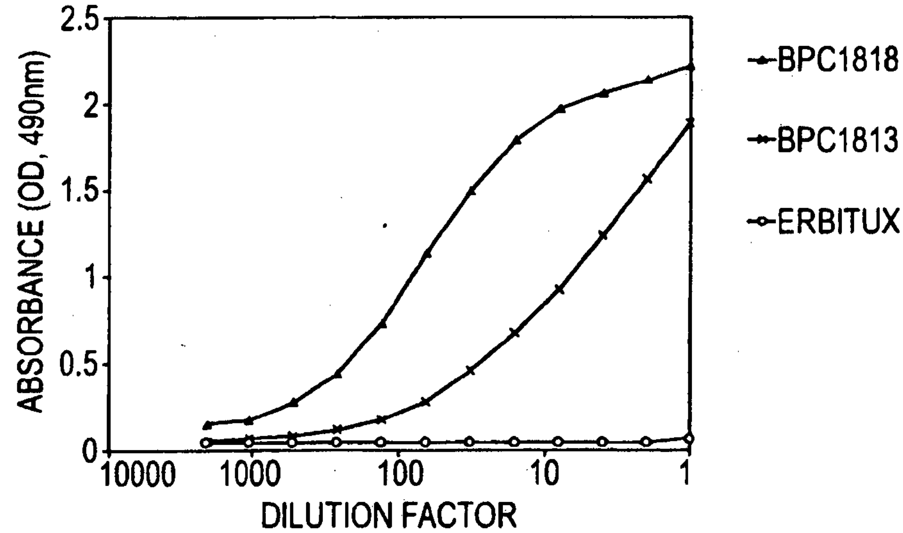


*FIG. 172*

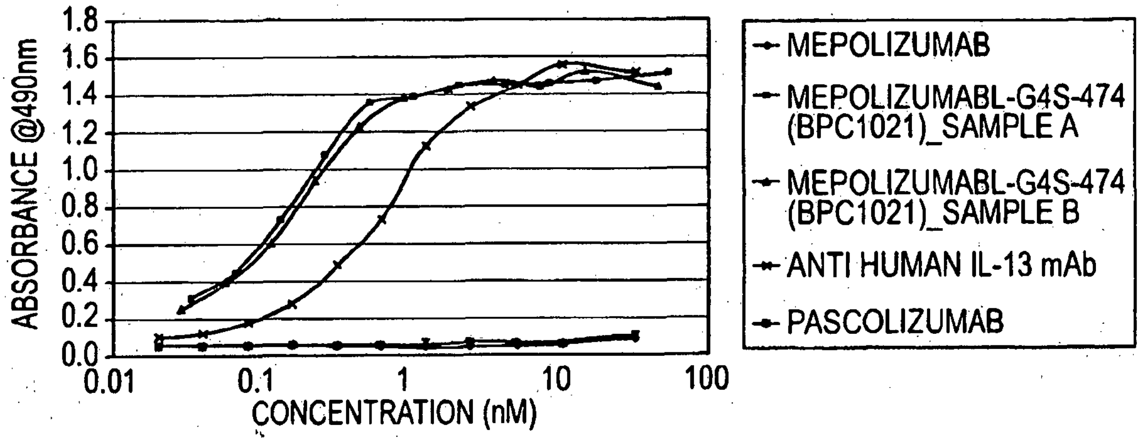


*FIG. 173*

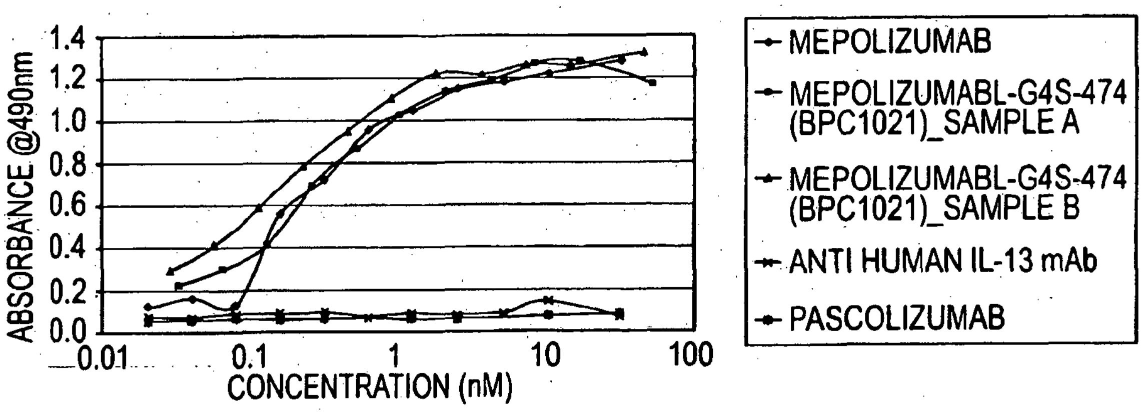


*FIG. 174*

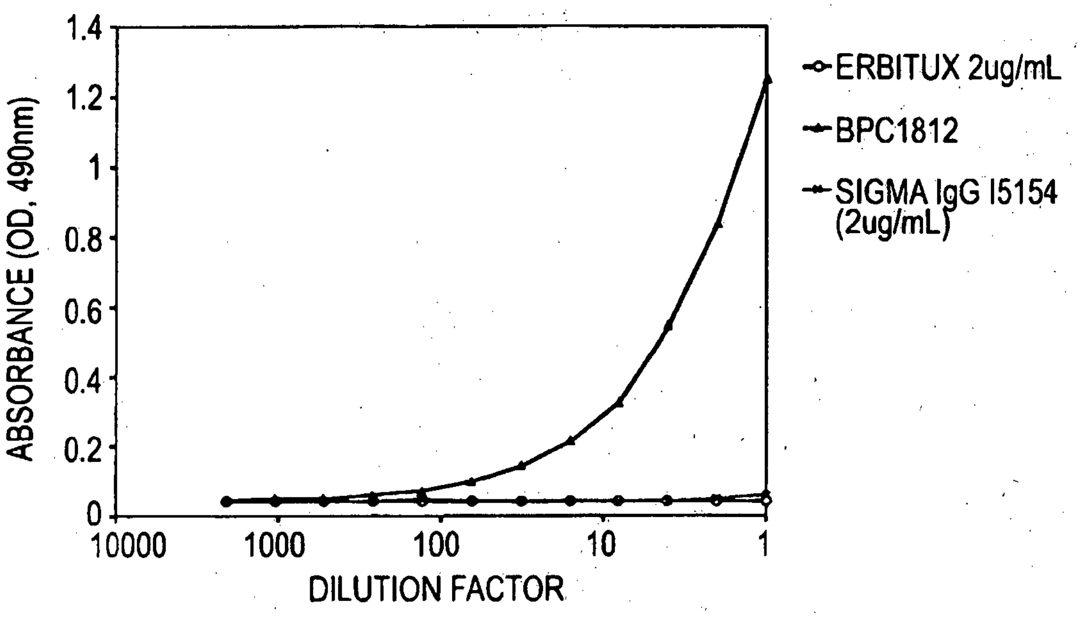


*FIG. 175*

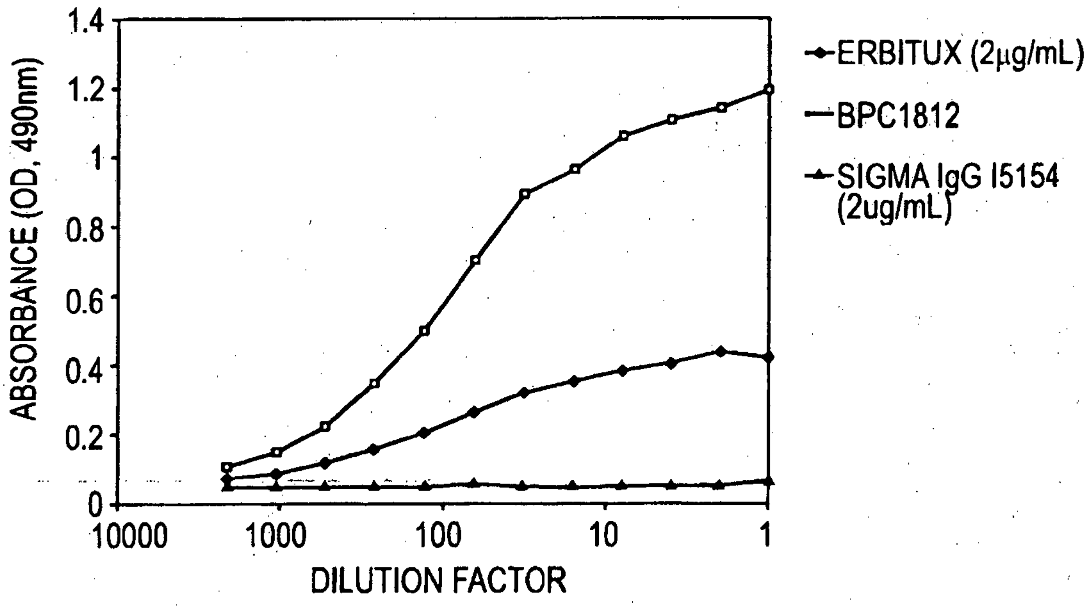


*FIG. 176*

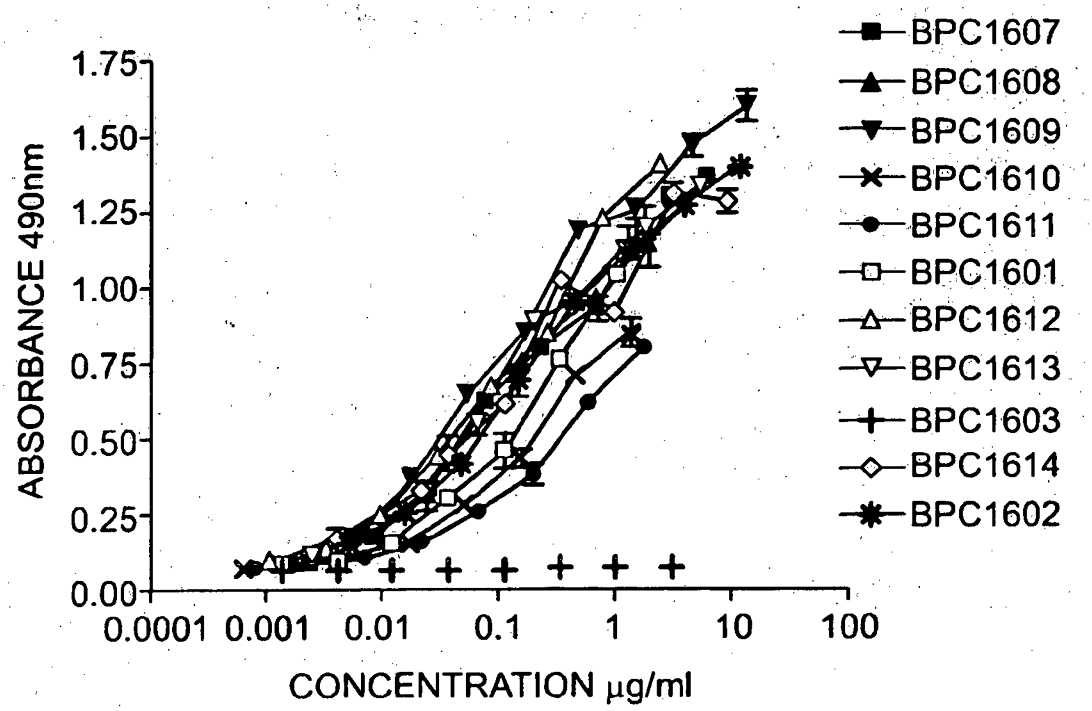
1.75
1.50
1.25
1.00
0.75
0.50
0.25
0.00
ABSORBANCE 490nm
0.0001
0.001
0.01
0.1
1
10
100
CONCENTRATION µg/ml
BPC1607
BPC1608
BPC1609
BPC1610
BPC1611
BPC1601
BPC1612
BPC1613
BPC1603
BPC1614
BPC1602

FIG. 177

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9308829 A
- WO 9404690 A
- WO 2007095338 A
- WO 2007066106 A
- US 6492123 B
- WO 2007085814 A
- WO 0029004 A
- US 20050043519 A
- US 7250297 B1
- US 20070224633 A
- EP 1641818 A1
- US 20040132028 A1
- US 20080139791 A
- WO 2005056764 A
- US 6818418 B1
- WO 2008098796 A
- EP 0307434 A
- WO 02092771 A
- WO 2005081854 A
- WO 2007059782 A
- WO 2006015371 A
- WO 9958679 A
- WO 9616990 A
- US 4873316 A
- US 6555313 B1, Griffiths
- US 5733743 A, Johnson
- US 5969108 A, McCafferty
- US 5702892 A, Mulligan-Kehoe
- WO 9920749 A, Tomlinson
- WO 0157065 A
- WO 9958655 A
- WO 9708320 A, Morphosys
- WO 9902671 A
- WO 0040712 A
- US 6297053 B
- WO 2005003156 A1
- WO 2007024715 A
- US 60991449 B
- US 61027858 B
- US 61046572 B
- US 61081191 B
- US 61084431 B


### Non-patent literature cited in the description


- **MILLSTEIN et al.** *Nature,* 1983, vol. 305, 537-539
- **TRAUNECKER et al.** *EMBO,* 1991, vol. 10, 3655-3659
- **SURESH et al.** *Methods in Enzymology,* 1986, vol. 121, 210
- **SHEN JUQUN et al.** *Journal of Immunological Methods,* 2007, vol. 318, 65-74
- *Mol. Immunol.,* 2006, vol. 44, 656-665
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- Handbook of Therapeutic Antibodies. 2007
- *Protein Science,* 2006, vol. 15, 14-27
- **REITER et al.** *J Mol Biol,* 1999, vol. 290, 685-698
- **EWERT et al.** *J Mol Biol,* 2003, vol. 325, 531-553
- **JESPERS et al.** *J Mol Biol,* 2004, vol. 337, 893-903
- **JESPERS et al.** *Nat Biotechnol,* 2004, vol. 22, 1161-1165
- **MARTIN et al.** *Protein Eng.,* 1997, vol. 10, 607-614
- **SEPULVADA et al.** *J Mol Biol,* 2003, vol. 333, 355-365
- **EPP et al.** *Biochemistry,* 1975, vol. 14, 4943-4952
- **HUAN et al.** *Biochemistry,* 1994, vol. 33, 14848-14857
- **HUANG et al.** *Mol immunol,* 1997, vol. 34, 1291-1301
- **JAMES et al.** *J Mol Biol.,* 2007, vol. 367, 603-8
- **PLÜCKTHUN, A.** *Immunol. Rev.,* 1992, vol. 130, 151-188

- **MILLER et al.** Genetic Engineering. Plenum Press, 1986, vol. 8, 277-298
- Remington's Pharmaceutical Science. Mack Publishing Company
- **LASMAR U ; PARKINS D.** The formulation of Biopharmaceutical products. *Pharma. Sci.Tech.today,* 03 April 2000, vol. 3, 129-137
- **WANG, W.** Instability, stabilisation and formulation of liquid protein pharmaceuticals. *Int. J. Pharm,* 1999, vol. 185, 129-188
- Stability of Protein Pharmaceuticals Part A and B. Plenum Press, 1992
- **AKERS,M.J.** Excipient-Drug interactions in Parenteral Formulations. *J.Pharm Sci,* 2002, vol. 91, 2283-2300
- **IMAMURA, K et al.** Effects of types of sugar on stabilization of Protein in the dried state. *J Pharm Sci,* 2003, vol. 92, 266-274
- **IZUTSU, KKOJIMA, S.** Excipient crystalinity and its protein-structure-stabilizing effect during freeze-drying. *J Pharm. Pharmacol,* 2002, vol. 54, 1033-1039
- **JOHNSON, R.** Mannitol-sucrose mixtures-versatile formulations for protein lyophilization. *J. Pharm. Sci,* 2002, vol. 91, 914-922
- **HA ; E WANG W ; WANG Y.J.** Peroxide formation in polysorbate 80 and protein stability. *J. Pharm Sci,* 2002, vol. 91, 2252-2264
- **CHOTHIA ; LESK.** *J. Mol. Biol.,* 1987, vol. 196, 910-917
- **TATUSOVA, T. A.** *FEMS Microbiol Lett,* 1999, vol. 174, 187-188
- **WINTER, G. et al.** *Annu. Rev. Immunol.,* 1994, vol. 12, 433-455
- **SOUMILLION, P. et al.** *Appl. Biochem. Biotechnol.,* 1994, vol. 47 (2-3), 175-189
- **CASTAGNOLI, L. et al.** *Comb. Chem. High Throughput Screen,* 2001, vol. 4 (2), 121-133
- **DENG et al.** *J. Biol. Chem.,* 1994, vol. 269, 9533
- **LOW et al.** *J. Mol. Biol.,* 1996, vol. 260, 359
- **CRAMERI et al.** *Nature Med.,* 1996, vol. 2, 100
- **RIECHMANN et al.** *Bio/Technology,* 1995, vol. 13, 475
- **LANDT, O et al.** *Gene,* 1990, vol. 96, 125-128
- **HORTON, R.M. et al.** *Gene,* 1989, vol. 77, 61-68
- **GOLDMAN, L.A. et al.** *Biotechniques,* 1996, vol. 21, 1013-1015
- **MIZUSHIMA, S. et al.** *Nucleic Acids Res.,* 1990, vol. 18, 5322
- **TAWFIK ; GRIFFITHS.** *Nature Biotechnol,* 1998, vol. 16 (7), 652-6
- **WINTER et al.** *Ann. Rev. Immunology,* 1994, vol. 12, 433-55
- **TOMLINSON et al.** *J. Mol. Biol.,* 1992, vol. 227, 776
- **STEMMER.** *Nature,* 1994, vol. 370, 389-391
- **CHOTHIA ; LESK.** *J. Mol. Biol.,* 1987, vol. 196, 901
- **CHOTHIA et al.** *Nature,* 1989, vol. 342, 877
- **CHOTHIA et al.** *J. Mol. Biol.,* 1992, vol. 227, 799
- **TOMLINSON et al.** *EMBO J.,* 1995, vol. 14, 4628
- **WILLIAMS et al.** *J. Mol. Biol.,* 1996, vol. 264, 220
- **MARTIN et al.** *J. Mol. Biol.,* 1996, vol. 263, 800
- **SHIRAI et al.** *FEBS Letters,* 1996, vol. 399, 1
- **HOOD et al.** Quant. Biol. Cold Spring Harbor Symp, 1967, vol. 48, 133
- **KABAT et al.** Sequences of proteins of immunological interest. U.S. Department of Health and Human Services, 1991
- **HAWKINS et al.** *J. Mol. Biol.,* 1992, vol. 226, 889
- **BARBAS et al.** *Proc. Natl. Acad. Sci. USA,* 1992, vol. 89, 4457
- **HOOGENBOOM ; WINTER.** *J. Mol. Biol.,* 1992, vol. 227, 381
- **NISSIM et al.** *EMBO J.,* 1994, vol. 13, 692
- **GRIFFITHS et al.** *EMBO J.,* 1994, vol. 13, 3245
- **DE KRUIF et al.** *J. Mol. Biol.,* 1995, vol. 248, 97
- **TOMLINSON et al.** *J. Mol. Biol.,* 1996, vol. 256, 813
- **E. MEYERS ; W. MILLER.** *Comput. Appl. Biósci.,* 1988, vol. 4, 11-17
- **NEEDLEMAN ; WUNSCH.** *J. Mol. Biol.,* 1970, vol. 48, 444-453
- **ZIMM.** *J. Chem. Phys.,* 1948, vol. 16, 1093-1099
- **BOYD et al.** *J. Imm. Meth.,* 1995, vol. 184, 29-38
- **ZAHND, C. et al.** *J. Mol. Biol.,* 2007, vol. 369, 1015-1028